# EUROPEAN PATENT APPLICATION

(11) **EP 4 563 571 A1**
(43) Date of publication of application: **04.06.2025**
(21) Application number: 23845640.4
(22) Date of filing: 27.07.2023
(51) Int. Cl.: C07D 213/75, C07D 471/04, C07D 401/12, C07D 401/14, A61K 31/4406, A61K 31/437, A61P 35/00

(54) **PRMT5 INHIBITORS**

(30) Priority: 28.07.2022 CN 202210898107; 02.12.2022 CN 202211541013; 28.04.2023 CN 202310484698; 20.07.2023 CN 202310897087
(71) Applicant: Beijing Earthwise Technology Co., Ltd., Beijing 100080 (CN)
(72) Inventor: YANG, Xu, Beijing 100080 (CN); MENG, Qinghua, Beijing 100080 (CN); LIU, Lei, Beijing 100080 (CN); WANG, Huting, Beijing 100080 (CN); SHI, Lei, Beijing 100080 (CN); DU, Haolin, Beijing 100080 (CN); WANG, Jianhao, Beijing 100080 (CN); KONG, Desheng, Beijing 100080 (CN)
(74) Representative: Cabinet Becker et Associés
(86) International application number: PCT/CN2023/109524
(87) International publication number: WO 2024/022433

(57) **Abstract**

Provided is a class of PRMT5 inhibitors represented by formula (X) or pharmaceutically acceptable salts, isotopic variants, tautomers, stereoisomers, prodrugs, polymorphs, hydrates or solvates thereof. Further provided are a preparation method for the compounds, pharmaceutical compositions comprising the compounds, and use of the compounds in the prevention and treatment of cancers.

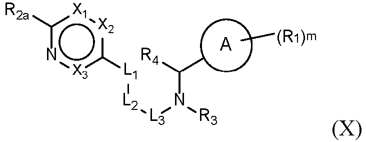

## Description

The present disclosure claims the priority of Chinese application CN202210898107.7 filed on July 28, 2022, Chinese application CN202211541013.0 filed on December 2, 2022, Chinese application CN202310484698.8 filed on April 28, 2023, and Chinese application CN202310897087.6 filed on July 20, 2023, which are incorporated herein by reference in their entirety.

### TECHNICAL FIELD

The present disclosure relates to a class of novel PRMT5 inhibitors, or a pharmaceutically acceptable salt, an isotopic variant, a tautomer, a stereoisomer, a prodrug, a polymorph, a hydrate or a solvate thereof. The present disclosure also relates to a method of preparing the compound, a pharmaceutical composition comprising the compound, and use of the compound in the prevention and treatment of a PRMT5-mediated disease such as cancer.

### BACKGROUND

Protein arginine methyltransferase 5 (PRMT5) belongs to type II PRMT and is a type of S-adenosyl methionine (SAM)-dependent methyltransferase. It is mainly responsible for symmetrically transferring the methyl group of SAM to a nitrogen atom of a guanidinium group at the end of an arginine residue of histones or other proteins. In vivo, PRMT5 forms a complex with MEP50 (methylosome protein 50) to recognize the substrates and locate them. At the same time, the complex morphology is also required for the activity of PRMT5 to catalyze the methyl transfer of histone 2A and histone 4. PRMT5 is a universal transcriptional repressor that can regulate the process of gene transcription and protein modification. At the same time, PRMT5 plays an important role in the proliferation, differentiation, and apoptosis of tumor cells and is a potential target for tumor therapy. However, PRMT5 is also a gene required for normal cells. PRMT5 knockout and siRNA knockdown studies have shown that inhibiting the activity of PRMT5 in normal tissues may cause many toxic side effects such as thrombocytopenia, infertility, skeletal muscle loss, and myocardial hypertrophy.

In 2016, multiple independent research teams reported that MTAP (methylthioadenosine phosphorylase)-deficient tumor cells had an increased dependence on PRMT5 activity. The deficiency of MTAP increases the level of intracellular MTA (methylthioadenosine), which is also a natural PRMT5 inhibitor at the same time. Therefore, MTAP-deficient tumor cells have an increased dependence on PRMT5 activity, which provides the possibility of targeting MTAP null tumor cells, that is, targeting the PRMT5/MTA complex, while leaving MTAP WT cells unaffected.

The mechanisms of action of PRMT5 inhibitors under development include SAM competitive inhibitors, substrate competitive inhibitors, SAM and substrate dual competitive inhibitors, and MTA-synergistic inhibitors. Non-MTA-synergistic PRMT5 inhibitors indiscriminately inhibit the activity of PRMT5, and dose-limiting toxic side effects such as thrombocytopenia, anemia, and neutropenia have been found. Among the inhibitors, MTA-synergistic inhibitors target the PRMT5/MTA complex, and are expected to eliminate the effect on MTAP WT cells while inhibiting MTAP-deficient tumor cells, thereby improving the therapeutic window.

Therefore, the development of PRMT5 inhibitors targeting the PRMT5/MTA complex will have important clinical application value, and there is a demand for such inhibitors in the art.

### SUMMARY

The present disclosure uses the PRMT5/MTA complex as a target and develops a class of new small molecule inhibitors that can be used to treat various cancers.

The compounds of the present disclosure target the PRMT5/MTA complex, and are MTA-synergistic PRMT5 inhibitors. They have excellent PRMT5/MTA inhibitory activity, and have obvious selectivity for the PRMT5/SAM complex. At the same time, the compounds of the present disclosure have good ADMET (absorption-distribution-metabolism-excretion-toxicity) properties.

In one aspect, the present disclosure provides a compound of formula (X), or a pharmaceutically acceptable salt, an isotopic variant, a tautomer, a stereoisomer, a prodrug, a polymorph, a hydrate or a solvate thereof: 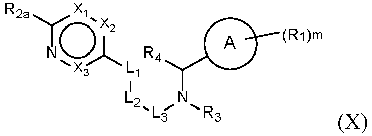 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)(=NRₐ)Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
R₄ is selected from H, D, C₁₋₆ alkyl and C₁₋₆ haloalkyl;
or one R₁ is connected to R₄ to form -(CH₂)_{q1}-X-(CH₂)_{q2}-; wherein X is O, S, NH or CH₂, q1=0, 1 or 2, q2=1, 2 or 3;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, R₄, L, R, R₁ₛ, R₂ₛ, R₃ₛ, R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.

In another aspect, the present disclosure provides a compound of formula (I), or a pharmaceutically acceptable salt, an isotopic variant, a tautomer, a stereoisomer, a prodrug, a polymorph, a hydrate or a solvate thereof: 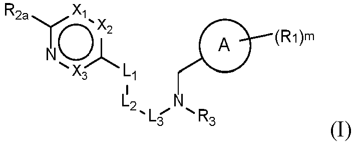 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, L, R, R₁ₛ, R₂ₛ, R₃ₛ, R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.

In another aspect, the present disclosure provides a pharmaceutical composition, comprising a compound of the present disclosure, and optionally a pharmaceutically acceptable excipient, such as a carrier, adjuvant or vehicle.

In another aspect, the present disclosure provides a pharmaceutical composition comprising a compound of the present disclosure and a pharmaceutically acceptable excipient, which further comprises other therapeutic agents, such as those selected from the following targeted drugs/cell activity modulators, including CDK4/6 inhibitors, MAT2A inhibitors, MAPK1/MAPK3 inhibitors, Type I PRMT inhibitors, EGFR inhibitors, SHP2 inhibitors, pan-KRAS inhibitors, KRASG12C inhibitors, RAF inhibitors, MEK inhibitors, ERK inhibitors, Bcl-2 inhibitors, SOS1 inhibitors, PARP inhibitors, MALT1 inhibitors, MALT2 inhibitors, BTK inhibitors, PI3K inhibitors, AKT inhibitors, FGFR inhibitors, DNA methyltransferase (DNMT) inhibitors, EZH1/2 inhibitors, EZH2 inhibitors, Menin-MLL inhibitors, IDH1 inhibitors, IDH2 inhibitors, IDH1/2 inhibitors, chemotherapy drugs, radiotherapy, STING agonists and immune checkpoint inhibitors/modulators.

In another aspect, the present disclosure provides use of the compound of the present disclosure in the manufacture of a medicament for the treatment and/or prevention of a PRMT5-mediated disease.

In another aspect, the present disclosure provides a method of treating and/or preventing a PRMT5-mediated disease in a subject, comprising administering a compound of the present disclosure or a pharmaceutical composition of the present disclosure to the subject.

In another aspect, the present disclosure provides a compound of the present disclosure or a pharmaceutical composition of the present disclosure, for use in the treatment and/or prevention of a PRMT5-mediated disease.

In a specific embodiment, the present disclosure is used in the treatment and/or prevention of a cancer. In another specific embodiment, the present disclosure is used in the treatment and/or prevention of the following cancers: heart: sarcoma (angiosarcoma, fibrosarcoma, rhabdomyosarcoma, liposarcoma), myxoma, rhabdomyoma, fibroma, lipoma, and teratoma; lung: bronchogenic carcinoma (squamous cell carcinoma, small cell undifferentiated carcinoma, large cell undifferentiated carcinoma, adenocarcinoma), alveolar (bronchiolar) carcinoma, bronchial adenoma, sarcoma, lymphoma, chondroma, hamartoma, mesothelioma; gastrointestinal tract: esophagus (squamous cell carcinoma, adenocarcinoma, leiomyosarcoma, lymphoma), stomach (cancer, lymphoma, leiomyosarcoma), pancreas (ductal adenocarcinoma, insulinoma, glucagonoma, gastrinoma, carcinoid tumor, hemangioma), small intestine (adenocarcinoma, lymphoma, carcinoid tumor, Kaposi's sarcoma, leiomyoma, hemangioma, lipoma, neurofibroma, fibroma), large intestine (adenocarcinoma, tubular adenoma, villous adenoma, hamartoma, leiomyoma); genitourinary tract: kidney (adenocarcinoma, Wilms tumor (nephroblastoma), lymphoma, leukemia), bladder and urethra (squamous cell carcinoma, transitional cell carcinoma, adenocarcinoma), prostate (adenocarcinoma, sarcoma), testicle (seminoma, teratoma, embryonal carcinoma, teratocarcinoma, choriocarcinoma, sarcoma, stromal cell carcinoma, fibroma, fibroadenoma, adenomatoid tumor, lipoma); liver: hepatoma (hepatocellular carcinoma), cholangiocarcinoma, hepatoblastoma, angiosarcoma, hepatocellular adenoma, hemangioma; biliary tract: gallbladder cancer, ampullary cancer, cholangiocarcinoma; bone: osteosarcoma, fibrosarcoma, malignant fibrous histiocytoma, chondrosarcoma, Ewing sarcoma, malignant lymphoma (reticulum cell sarcoma), multiple myeloma, malignant giant cell tumor, chordoma, osteocartilaginous exostoses, benign enchondroma, chondroblastoma, fibrochondroma, chondromyxoid fibroma, osteoid osteoma, and giant cell tumor; nervous system: skull (osteoma, hemangioma, granuloma, xanthoma, osteitis deformans), meninges (meningioma, meningeal sarcoma, glioma), brain (astrocytoma, medulloblastoma, glioma, ependymoma, germinoma (pineal tumor), glioblastoma multiforme, oligodendroglioma, schwannoma, retinoblastoma, congenital tumor), spinal neurofibroma, meningioma, glioma, sarcoma; gynaecology: uterus (endometrial cancer), cervix (cervical cancer, preneoplastic cervical dysplasia, etc.), ovary (ovarian cancer, serous cystadenocarcinoma, mucinous cystadenocarcinoma, unclassified cancer), granulosa cell tumor, Sertoli-Leydig cell tumor, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, sarcoma botryoides (embryonal rhabdomyosarcoma)), fallopian tube (cancer); hematology: blood (myeloid leukemia (acute and chronic), acute lymphoblastic leukemia, chronic lymphocytic leukemia, diffuse large B-cell lymphoma, mantle cell lymphoma (MCL), follicular lymphoma, myeloproliferative disorder, multiple myeloma, myelodysplastic syndrome), Hodgkin disease, non-Hodgkin lymphoma (malignant lymphoma); skin: malignant melanoma, basal cell carcinoma, squamous cell carcinoma, Kaposi sarcoma, dysplastic nevus, lipoma, hemangioma, dermatofibroma, keloma, psoriasis; and adrenal gland: neuroblastoma.

In another specific embodiment, the PRMT5-mediated disease of the present disclosure is selected from the following cancers: malignant peripheral nerve sheath tumor, mesothelioma, glioblastoma multiforme, pancreatic cancer, cholangiocarcinoma, prostate cancer, breast cancer, brain cancer, skin cancer, cervical cancer, bladder cancer, astrocytoma, colorectal cancer, endometrial cancer, esophageal cancer, gastric cancer, thymoma, head and neck cancer, hepatocellular carcinoma, laryngeal cancer, squamous cell carcinoma of the lung, lung adenocarcinoma, oral cancer, ovarian cancer, kidney cancer, and thyroid cancer and sarcoma.

In another specific embodiment, the PRMT5-mediated disease of the present disclosure is selected from MTAP-related cancers, such as hepatocellular carcinoma, breast cancer, skin cancer, bladder cancer, liver cancer, pancreatic cancer and head and neck cancer.

### Definition

### Chemical definitions

Definitions of specific functional groups and chemical terms are described in more detail below.

When a range of values is listed, it is intended to encompass each value and sub-range within the range. For example, "C₁₋₆ alkyl" is intended to include C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅ and C₅₋₆ alkyl.

"C₁₋₆ alkyl" refers to a radical of a straight or branched, saturated hydrocarbon group having 1 to 6 carbon atoms. In some embodiments, C₁₋₄ alkyl and C₁₋₂ alkyl are alternative. Examples of C₁₋₆ alkyl include methyl (C₁), ethyl (C₂), n-propyl (C₃), iso-propyl (C₃), n-butyl (C₄), tert-butyl (C₄), sec-butyl (C₄), iso-butyl (C₄), n-pentyl (C₅), 3-pentyl (C₅), pentyl (C₅), neopentyl (C₅), 3-methyl-2-butyl (C₅), tert-pentyl (C₅) and n-hexyl (C₆). The term "C₁₋₆ alkyl" also includes heteroalkyl, wherein one or more (e.g., 1, 2, 3 or 4) carbon atoms are subsituted with heteroatoms (e.g., oxygen, sulfur, nitrogen, boron, silicon, phosphorus). Alkyl groups can be optionally substituted with one or more substituents, for example, with 1 to 5 substituents, 1 to 3 substituents or 1 substituent. Conventional abbreviations of alkyl include Me (-CH₃), Et (-CH₂CH₃), iPr (-CH(CH₃)₂), nPr (-CH₂CH₂CH₃), n-Bu (-CH₂CH₂CH₂CH₃) or i-Bu (-CH₂CH(CH₃)₂).

"C₂₋₆ alkenyl" refers to a radical of a straight or branched hydrocarbon group having 2 to 6 carbon atoms and at least one carbon-carbon double bond. In some embodiments, C₂₋₄ alkenyl is alternative. Examples of C₂₋₆ alkenyl include vinyl (C₂), 1-propenyl (C₃), 2-propenyl (C₃), 1-butenyl (C₄), 2-butenyl (C₄), butadienyl (C₄), pentenyl (C₅), pentadienyl (C₅), hexenyl (C₆), etc. The term "C₂₋₆ alkenyl" also includes heteroalkenyl, wherein one or more (e.g., 1, 2, 3 or 4) carbon atoms are replaced by heteroatoms (e.g., oxygen, sulfur, nitrogen, boron, silicon, phosphorus). The alkenyl groups can be optionally substituted with one or more substituents, for example, with 1 to 5 substituents, 1 to 3 substituents or 1 substituent.

"C₂₋₆ alkynyl" refers to a radical of a straight or branched hydrocarbon group having 2 to 6 carbon atoms, at least one carbon-carbon triple bond and optionally one or more carbon-carbon double bonds. In some embodiments, C₂₋₄ alkynyl is alternative. Examples of C₂₋₆ alkynyl include, but are not limited to, ethynyl (C₂), 1-propynyl (C₃), 2-propynyl (C₃), 1-butynyl (C₄), 2-butynyl (C₄), pentynyl (C₅), hexynyl (C₆), etc. The term "C₂₋₆ alkynyl" also includes heteroalkynyl, wherein one or more (e.g., 1, 2, 3 or 4) carbon atoms are replaced by heteroatoms (e.g., oxygen, sulfur, nitrogen, boron, silicon, phosphorus). The alkynyl groups can be substituted with one or more substituents, for example, with 1 to 5 substituents, 1 to 3 substituents or 1 substituent.

"C₁₋₆ alkylene" refers to a divalent group formed by removing another hydrogen of the C₁₋₆ alkyl, and can be substituted or unsubstituted. In some embodiments, C₁₋₄ alkylene, C₂₋₄ alkylene and C₁₋₃ alkylene are alternative. The unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), etc. Examples of substituted alkylene groups, such as those substituted with one or more alkyl (methyl) groups, include, but are not limited to, substituted methylene (-CH(CH₃)-, -C(CH₃)₂-), substituted ethylene (-CH(CH₃)CH₂-, -CH₂CH(CH₃)-, -C(CH₃)₂CH₂-, -CH₂C(CH₃)₂-), substituted propylene (-CH(CH₃)CH₂CH₂-, -CH₂CH(CH₃)CH₂-, -CH₂CH₂CH(CH₃)-, -C(CH₃)₂CH₂CH₂-, -CH₂C(CH₃)₂CH₂-, -CH₂CH₂C(CH₃)₂-), etc.

"C₂₋₆ alkenylene" refers to a C₂₋₆ alkenyl group wherein another hydrogen is removed to provide a divalent radical of alkenylene, and which may be substituted or unsubstituted. In some embodiments, C₂₋₄ alkenylene is yet alternative. Exemplary unsubstituted alkenylene groups include, but are not limited to, vinylene (-CH=CH-) and propenylene (e.g., -CH=CHCH₂-, -CH₂-CH=CH-). Exemplary substituted alkenylene groups, e.g., substituted with one or more alkyl (methyl) groups, include but are not limited to, substituted ethylene (-C(CH₃)=CH-, -CH=C(CH₃)-), substituted propenylene (e.g., -C(CH₃)=CHCH₂-, -CH=C(CH₃)CH₂-, -CH=CHCH(CH₃)-, -CH=CHC(CH₃)₂-, -CH(CH₃)-CH=CH-, -C(CH₃)₂-CH=CH-, -CH₂-C(CH₃)=CH-, -CH₂-CH=C(CH₃)-), and the like.

"C₂₋₆ alkynylene" refers to a C₂₋₆ alkynyl group wherein another hydrogen is removed to provide a divalent radical of alkynylene, and which may be substituted or unsubstituted. In some embodiments, C₂₋₄ alkynylene is yet alternative. Exemplary alkynylene groups include, but are not limited to, ethynylene (-C≡C-), substituted or unsubstituted propynylene (-C≡CCH₂-), and the like.

"C₀₋₆ alkylene" refers to a chemical bond and the above "C₁₋₆ alkylene", and "C₀₋₄ alkylene" refers to a chemical bond and the above "C₁₋₄ alkylene".

"Halo" or "halogen" refers to fluorine (F), chlorine (Cl), bromine (Br) and iodine (I).

Thus, "C₁₋₆ haloalkyl" refers to the above "C₁₋₆ alkyl", which is substituted with one or more halogen. In some embodiments, C₁₋₄ haloalkyl is yet alternative, and still alternatively C₁₋₂ haloalkyl. Exemplary haloalkyl groups include, but are not limited to, -CF₃, -CH₂F, -CHF₂, -CHFCH₂F, -CH₂CHF₂, -CF₂CF₃, -CCl₃, -CH₂Cl, -CHCl₂, 2,2,2-trifluoro-1,1-dimethyl-ethyl, and the like. The haloalkyl can be substituted at any available point of attachment, for example, with 1 to 5 substituents, 1 to 3 substituents or 1 substituent.

"C₃₋₁₀ cycloalkyl" refers to a radical of a non-aromatic cyclic hydrocarbon group having from 3 to 10 ring carbon atoms and zero heteroatoms, optionally containing 1, 2 or 3 double or triple bonds. In some embodiments, C₅₋₁₀ cycloalkyl, C₃₋₇ cycloalkyl and C₃₋₆ cycloalkyl are yet alternative, and still alternatively C₅₋₇ cycloalkyl and C₅₋₆ cycloalkyl. The cycloalkyl also includes a ring system in which the cycloalkyl described herein is fused with one or more aryl or heteroaryl groups, wherein the point of attachment is on the cycloalkyl ring, and in such case, the number of carbon atoms continues to represent the number of carbon atoms in the cycloalkyl system. The cycloalkyl also includes the above cycloalkyl rings, wherein the substituents on any non-adjacent carbon atoms are connected to form a bridged ring, together forming a polycycloalkane sharing two or more carbon atoms. The cycloalkyl also includes the above cycloalkyl rings, wherein the substituents on the same carbon atom are connected to form a ring, together forming a polycycloalkane sharing one carbon atom. Exemplary cycloalkyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), cycloheptyl (C₇), cycloheptenyl (C₇), cycloheptadienyl (C₇), cycloheptatrienyl (C₇), etc. The cycloalkyl groups can be substituted with one or more substituents, for example, with 1 to 5 substituents, 1 to 3 substituents or 1 substituent.

"C₃₋₁₀ cycloalkylene" refers to a divalent group formed by removing another hydrogen of the C₃₋₁₀ cycloalkyl, and can be substituted or unsubstituted. In some embodiments, C₃₋₆ cycloalkylene and C₃₋₄ cycloalkylene are yet alternative, and still alternatively cyclopropylene.

"3- to 10-membered heterocyclyl" refers to a saturated or unsaturated radical of 3- to 10-membered non-aromatic ring system having ring carbon atoms and 1 to 5 ring heteroatoms, wherein each of the heteroatoms is independently selected from nitrogen, oxygen, sulfur, boron, phosphorus and silicon, and optionally containing 1, 2 or 3 double or triple bonds. In the heterocyclyl containing one or more nitrogen atoms, the point of attachment can be a carbon or nitrogen atom as long as the valence permits. In some embodiments, 5- to 10-membered heterocyclyl is alternative, which is a radical of 5- to 10-membered non-aromatic ring system having ring carbon atoms and 1 to 5 ring heteroatoms; in some embodiments, 3- to 7-membered heterocyclyl is alternative, which is a radical of 3- to 7-membered non-aromatic ring system having ring carbon atoms and 1 to 4 ring heteroatoms; 5- to 7-membered heterocyclyl is alternative, which is a radical of 5- to 7-membered non-aromatic ring system having ring carbon atoms and 1 to 3 ring heteroatoms; 3- to 6-membered heterocyclyl is alternative, which is a radical of 3- to 6-membered non-aromatic ring system having ring carbon atoms and 1 to 3 ring heteroatoms; 4- to 6-membered heterocyclyl is alternative, which is a radical of 4- to 6-membered non-aromatic ring system having ring carbon atoms and 1 to 3 ring heteroatoms; 5- to 6-membered heterocyclyl is yet alternative, which is a radical of 5- to 6-membered non-aromatic ring system having ring carbon atoms and 1 to 3 ring heteroatoms. The heterocyclyl also includes a ring system wherein the heterocyclyl described above is fused with one or more cycloalkyl groups, wherein the point of attachment is on the heterocyclyl ring, or the heterocyclyl described above is fused with one or more aryl or heteroaryl groups, wherein the point of attachment is on the heterocyclyl ring; and in such cases, the number of ring members continues to represent the number of ring members in the heterocyclyl ring system. The heterocyclyl also includes the above heterocyclyl rings, wherein the substituents on any non-adjacent carbon or nitrogen atoms are connected to form a bridged ring, together forming a polycyclic heteroalkane sharing two or more carbon or nitrogen atoms. The heterocyclyl also includes the above heterocyclyl rings, wherein the substituents on the same carbon atom are connected to form a ring, together forming a polycyclic heteroalkane sharing one carbon atom. Exemplary 3-membered heterocyclyl groups containing one heteroatom include, but are not limited to, aziridinyl, oxiranyl and thiorenyl. Exemplary 4-membered heterocyclyl groups containing one heteroatom include, but are not limited to, azetidinyl, oxetanyl and thietanyl. Exemplary 5-membered heterocyclyl groups containing one heteroatom include, but are not limited to, tetrahydrofuranyl, dihydrofuranyl, tetrahydrothiophenyl, dihydrothienyl, pyrrolidinyl, dihydropyrrolyl and pyrrolyl-2,5-dione. Exemplary 5-membered heterocyclyl groups containing two heteroatoms include, but are not limited to, pyrazolidinyl, dioxolanyl, oxasulfuranyl, disulfuranyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidyl, tetrahydropyranyl, dihydropyridyl and thianyl. Exemplary 6-membered heterocyclyl groups containing two heteroatoms include, but are not limited to, piperazinyl, morpholinyl, dithianyl and dioxanyl. Exemplary 6-membered heterocyclyl groups containing three heteroatoms include, but are not limited to, triazinanyl. Exemplary 7-membered heterocycly groups containing one heteroatom include, but are not limited to, azepanyl, oxepanyl and thiepanyl. Exemplary 5-membered heterocyclyl groups fused with a C₆ aryl (also referred as 5,6-bicyclic heterocyclyl herein) include, but are not limited to, indolinyl, isoindolinyl, dihydrobenzofuranyl, dihydrobenzothiophenyl, benzoxazolinonyl, etc. Exemplary 6-membered heterocyclyl groups fused with a C₆ aryl (also referred as 6,6-bicyclic heterocyclyl herein) include, but are not limited to, tetrahydroquinolinyl, tetrahydroisoquinolinyl, etc. The heterocyclyl also includes a bridged ring or a spiral ring formed by the above heterocyclyl and a cycloalkyl, heterocyclyl, aryl or heteroaryl group sharing one or two atoms, and the shared atom can be a carbon or nitrogen atom as long as the valence permits. The heterocyclyl also includes the above heterocyclyl and heterocyclyl groups which can be optionally substituted with one or more substituents, for example, with 1 to 5 substituents, 1 to 3 substituents or 1 substituent.

"C₆₋₁₀ aryl" refers to a radical of monocyclic or polycyclic (e.g., bicyclic) 4n+2 aromatic ring system having 6 to 10 ring carbon atoms and zero heteroatoms (e.g., having 6 or 10 shared π electrons in a cyclic array). In some embodiments, the aryl group has six ring carbon atoms ("C₆ aryl"; for example, phenyl). In some embodiments, the aryl group has ten ring carbon atoms ("C₁₀ aryl"; for example, naphthyl, e.g., 1-naphthyl and 2-naphthyl). The aryl group also includes a ring system in which the aryl ring described above is fused with one or more cycloalkyl or heterocyclyl groups, and the point of attachment is on the aryl ring, in which case the number of carbon atoms continues to represent the number of carbon atoms in the aryl ring system. The aryl can be substituted with one or more substituents, for example, with 1 to 5 substituents, 1 to 3 substituents or 1 substituent.

"5- to 14-membered heteroaryl" refers to a radical of 5- to 14-membered monocyclic or bicyclic 4n+2 aromatic ring system (e.g., having 6, 10 or 14 shared π electrons in a cyclic array) having ring carbon atoms and 1 to 4 ring heteroatoms, wherein each heteroatom is independently selected from nitrogen, oxygen and sulfur. In the heteroaryl group containing one or more nitrogen atoms, the point of attachment can be a carbon or nitrogen atom as long as the valence permits. Heteroaryl bicyclic systems may include one or more heteroatoms in one or two rings. Heteroaryl also includes ring systems wherein the heteroaryl ring described above is fused with one or more cycloalkyl or heterocyclyl groups, and the point of attachment is on the heteroaryl ring. In such case, the number the carbon atoms continues to represent the number of carbon atoms in the heteroaryl ring system. In some embodiments, 5- to 10-membered heteroaryl groups are alternative, which are radicals of 5- to 10-membered monocyclic or bicyclic 4n+2 aromatic ring systems having ring carbon atoms and 1 to 4 ring heteroatoms. In other embodiments, 5- to 6-membered heteroaryl groups are yet alternative, which are radicals of 5- to 6-membered monocyclic or bicyclic 4n+2 aromatic ring systems having ring carbon atoms and 1 to 4 ring heteroatoms. Exemplary 5-membered heteroaryl groups containing one heteroatom include, but are not limited to, pyrrolyl, furyl and thienyl. Exemplary 5-membered heteroaryl groups containing two heteroatoms include, but are not limited to, imidazolyl, pyrazolyl, oxazolyl, isoxazolyl, thiazolyl, and isothiazolyl. Exemplary 5-membered heteroaryl groups containing three heteroatoms include, but are not limited to, triazolyl, oxadiazolyl (such as, 1,2,4-oxadiazoly), and thiadiazolyl. Exemplary 5-membered heteroaryl groups containing four heteroatoms include, but are not limited to, tetrazolyl. Exemplary 6-membered heteroaryl groups containing one heteroatom include, but are not limited to, pyridyl and pyridonyl. Exemplary 6-membered heteroaryl groups containing two heteroatoms include, but are not limited to, pyridazinyl, pyrimidinyl, and pyrazinyl. Exemplary 6-membered heteroaryl groups containing three or four heteroatoms include, but are not limited to, triazinyl and tetrazinyl, respectively. Exemplary 7-membered heteroaryl groups containing one heteroatom include, but are not limited to, azepinyl, oxepinyl, and thiepinyl. Exemplary 5,6-bicyclic heteroaryl groups include, but are not limited to, indolyl, isoindolyl, indazolyl, benzotriazolyl, benzothiophenyl, isobenzothiophenyl, benzofuranyl, benzoisofuranyl, benzimidazolyl, benzoxazolyl, benzoisoxazolyl, benzoxadiazolyl, benzothiazolyl, benzoisothiazolyl, benzothiadiazolyl, indolizinyl and purinyl. Exemplary 6,6-bicyclic heteroaryl groups include, but are not limited to, naphthyridinyl, pteridinyl, quinolyl, isoquinolyl, cinnolinyl, quinoxalinyl, phthalazinyl and quinazolinyl. The heteroaryl can be substituted with one or more substituents, for example, with 1 to 5 substituents, 1 to 3 substituents or 1 substituent.

The divalent groups formed by removing another hydrogen from the alkyl, alkenyl, alkynyl, cycloalkyl, heterocyclyl, aryl and heteroaryl groups defined above are collectively referred to as "-ylene" or "-ylidene". Ring-forming groups such as cycloalkyl, heterocyclyl, aryl and heteroaryl are collectively referred to as "cyclyl groups".

Alkyl, alkenyl, alkynyl, cycloalkyl, heterocyclyl, aryl and heteroaryl as defined herein are optionally substituted groups.

Exemplary substituents on carbon atoms include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, -OCO₂R^{aa}, -C(=O)N(R^{bb})₂, -OC(=O)N(R^{bb})₂, -NR^{bb}C(=O)R^{aa}, -NR^{bb}CO₂R^{aa}, -NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, -C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, -NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, -Si(R^{aa})₃, -OSi(R^{aa})₃, -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, -OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, -OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, -OP(R^{cc})₃, -B(R^{aa})₂, -B(OR^{cc})₂, -BR^{aa}(OR^{cc}), alkyl, haloalkyl, alkenyl, alkynyl, cycloalkyl, heterocyclyl, aryl and heteroaryl, wherein each of the alkyl, alkenyl, alkynyl, cycloalkyl, heterocyclyl, aryl and heteroaryl is independently substituted with 0, 1, 2, 3, 4 or 5 R^{dd} groups;
or two geminal hydrogen on a carbon atom are replaced with =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb} or =NOR^{cc} groups;
each of the R^{aa} is independently selected from alkyl, haloalkyl, alkenyl, alkynyl, cycloalkyl, heterocyclyl, aryl and heteroaryl, or two of the R^{aa} groups are combined to form a heterocyclyl or heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{bb} is independently selected from hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{bb} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{cc} is independently selected from hydrogen, alkyl, haloalkyl, alkenyl, alkynyl, cycloalkyl, heterocyclyl, aryl and heteroaryl, or two R^{cc} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups;
each of the R^{dd} is independently selected from halogen, -CN, -NO₂, -N₃, -SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, -SSR^{ee}, -C(=O)R^{ee}, -CO₂H, -CO₂R^{ee}, -OC(=O)R^{ee}, -OCO₂R^{ee}, -C(=O)N(R^{ff})₂, -OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, -OC(=NR^{ff})R^{ee}, -OC(=NR^{ff})OR^{ee}, -C(=NR^{ff})N(R^{ff})₂, -OC(=NR^{ff})N(R^{ff})₂, -NR^{ff}C(=NR^{ff})N(R^{ff})₂, -NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, -P(=O)(R^{ee})₂, -OP(=O)(R^{ee})₂, -OP(=O)(OR^{ee})₂, alkyl, haloalkyl, alkenyl, alkynyl, cycloalkyl, heterocyclyl, aryl, and heteroaryl, wherein, each of the alkyl, alkenyl, alkynyl, cycloalkyl, heterocyclyl, aryl and heteroaryl is independently substituted with 0, 1, 2, 3, 4 or 5 R^{gg} groups, or two geminal R^{dd} substituents can be combined to form=O or =S;
each of the R^{ee} is independently selected from alkyl, haloalkyl, alkenyl, alkynyl, cycloalkyl, aryl, heterocyclyl, and heteroaryl, wherein, each of the alkyl, alkenyl, alkynyl, cycloalkyl, heterocyclyl, aryl and heteroaryl is independently substituted with 0, 1, 2, 3, 4 or 5 R^{gg} groups;
each of the R^{ff} is independently selected from hydrogen, alkyl, haloalkyl, alkenyl, alkynyl, cycloalkyl, heterocyclyl, aryl and heteroaryl, or two R^{ff} groups are combined to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{gg} groups;
each of the R^{gg} is independently halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl), -OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, -OC(NH)NH(C₁₋₆ alkyl), -OC(NH)NH₂, -NHC(NH)N(C₁₋₆ alkyl)₂, -NHC(=NH)NH₂, -NHSO₂(C₁₋₆ alkyl), -SO₂N(C₁₋₆ alkyl)₂, -sO₂NH(C₁₋₆ alkyl), -SO₂NH₂, -SO₂C₁₋₆ alkyl, -SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃, -C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, -OP(=O)(OC₁₋₆ alkyl)₂, C₁₋₆ alkyl, C₁₋₆ haloalkyl, C₂-C₆ alkenyl, C₂-C₆ alkynyl, C₃-C₇ cycloalkyl, C₆-C₁₀ aryl, C₃-C₇ heterocyclyl, or C₅-C₁₀ heteroaryl; or two geminal R^{gg} substituents may combine to form =O or =S; wherein, X⁻ is a counter-ion.

Exemplary substituents on nitrogen atoms include, but are not limited to, hydrogen, -OH, -OR^{aa}, -N(R^{cc})₂, -CN, -C(=O)R^{aa}, -C(=O)N(R^{cc})₂, -CO₂R^{aa}, -SO₂R^{aa}, -C(=NR^{bb})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})2, -C(=O)SR^{cc}, -C(=S)SR^{cc}, -P(=O)₂R^{aa}, -P(=O)(R^{aa})₂, -P(=O)₂N(R^{cc})₂, -P(=O)(NR^{cc})₂, alkyl, haloalkyl, alkenyl, alkynyl, cycloalkyl, heterocyclyl, aryl and heteroaryl, or two R^{cc} groups attached to a nitrogen atom combine to form a heterocyclyl or a heteroaryl ring, wherein each of the alkyl, alkenyl, alkynyl, cycloalkyl, heterocyclyl, aryl and heteroaryl is independently substituted with 0, 1, 2, 3, 4 or 5 R^{dd} groups, and wherein R^{aa}, R^{bb}, R^{cc} and R^{dd} are as described herein.

### Other definitions

The term "cancer" includes, but is not limited to, the following cancers: heart: sarcoma (angiosarcoma, fibrosarcoma, rhabdomyosarcoma, liposarcoma), myxoma, rhabdomyoma, fibroma, lipoma, and teratoma; lung: bronchogenic carcinoma (squamous cell carcinoma, small cell undifferentiated carcinoma, large cell undifferentiated carcinoma, adenocarcinoma), alveolar (bronchiolar) carcinoma, bronchial adenoma, sarcoma, lymphoma, chondroma, hamartoma, mesothelioma; gastrointestinal tract: esophagus (squamous cell carcinoma, adenocarcinoma, leiomyosarcoma, lymphoma), stomach (cancer, lymphoma, leiomyosarcoma), pancreas (ductal adenocarcinoma, insulinoma, glucagonoma, gastrinoma, carcinoid tumor, hemangioma), small intestine (adenocarcinoma, lymphoma, carcinoid tumor, Kaposi's sarcoma, leiomyoma, hemangioma, lipoma, neurofibroma, fibroma), large intestine (adenocarcinoma, tubular adenoma, villous adenoma, hamartoma, leiomyoma); genitourinary tract: kidney (adenocarcinoma, Wilms tumor (nephroblastoma), lymphoma, leukemia), bladder and urethra (squamous cell carcinoma, transitional cell carcinoma, adenocarcinoma), prostate (adenocarcinoma, sarcoma), testicle (seminoma, teratoma, embryonal carcinoma, teratocarcinoma, choriocarcinoma, sarcoma, stromal cell carcinoma, fibroma, fibroadenoma, adenomatoid tumor, lipoma); liver: hepatoma (hepatocellular carcinoma), cholangiocarcinoma, hepatoblastoma, angiosarcoma, hepatocellular adenoma, hemangioma; biliary tract: gallbladder cancer, ampullary cancer, cholangiocarcinoma; bone: osteosarcoma, fibrosarcoma, malignant fibrous histiocytoma, chondrosarcoma, Ewing sarcoma, malignant lymphoma (reticulum cell sarcoma), multiple myeloma, malignant giant cell tumor, chordoma, osteocartilaginous exostoses, benign enchondroma, chondroblastoma, fibrochondroma, chondromyxoid fibroma, osteoid osteoma, and giant cell tumor; nervous system: skull (osteoma, hemangioma, granuloma, xanthoma, osteitis deformans), meninges (meningioma, meningeal sarcoma, glioma), brain (astrocytoma, medulloblastoma, glioma, ependymoma, germinoma (pineal tumor), glioblastoma multiforme, oligodendroglioma, schwannoma, retinoblastoma, congenital tumor), spinal neurofibroma, meningioma, glioma, sarcoma; gynaecology: uterus (endometrial cancer), cervix (cervical cancer, preneoplastic cervical dysplasia, etc.), ovary (ovarian cancer, serous cystadenocarcinoma, mucinous cystadenocarcinoma, unclassified cancer), granulosa cell tumor, Sertoli-Leydig cell tumor, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, sarcoma botryoides (embryonal rhabdomyosarcoma)), fallopian tube (cancer); hematology: blood (myeloid leukemia (acute and chronic), acute lymphoblastic leukemia, chronic lymphocytic leukemia, diffuse large B-cell lymphoma, mantle cell lymphoma (MCL), follicular lymphoma, myeloproliferative disorder, multiple myeloma, myelodysplastic syndrome), Hodgkin disease, non-Hodgkin lymphoma (malignant lymphoma); skin: malignant melanoma, basal cell carcinoma, squamous cell carcinoma, Kaposi sarcoma, dysplastic nevus, lipoma, hemangioma, dermatofibroma, keloma, psoriasis; and adrenal gland: neuroblastoma.

In one embodiment, the term "cancer" includes, but is not limited to, the following cancers: malignant peripheral nerve sheath tumor, mesothelioma, glioblastoma multiforme, pancreatic cancer, cholangiocarcinoma, prostate cancer, breast cancer, brain cancer, skin cancer, cervical cancer, bladder cancer, astrocytoma, colorectal cancer, endometrial cancer, esophageal cancer, gastric cancer, thymoma, head and neck cancer, hepatocellular carcinoma, laryngeal cancer, squamous cell carcinoma of the lung, lung adenocarcinoma, oral cancer, ovarian cancer, kidney cancer, and thyroid cancer and sarcoma.

In one embodiment, the term "cancer" includes, but is not limited to, the following cancers: hepatocellular carcinoma, breast cancer, skin cancer, bladder cancer, liver cancer, pancreatic cancer and head and neck cancer.

The term "treating" as used herein relates to reversing, alleviating or inhibiting the progression or prevention of the disorders or conditions to which the term applies, or of one or more symptoms of such disorders or conditions. The noun "treatment" as used herein relates to the action of treating, which is a verb, and the latter is as just defined.

The term "pharmaceutically acceptable salt" as used herein refers to those carboxylate and amino acid addition salts of the compounds of the present disclosure, which are suitable for the contact with patients' tissues within a reliable medical judgment, and do not produce inappropriate toxicity, irritation, allergy, etc. They are commensurate with a reasonable benefit/risk ratio, and are effective for their intended use. The term includes, if possible, the zwitterionic form of the compounds of the disclosure.

The pharmaceutically acceptable base addition salts are formed with metals or amines, such as alkali metal and alkaline earth metal hydroxides or organic amines. Examples of the metals used as cations include sodium, potassium, magnesium, calcium, etc. Examples of suitable amines are N,N'-dibenzylethylenediamine, chloroprocaine, choline, diethanolamine, ethylenediamine, N-methylglucamine and procaine.

The base addition salt of the acidic compound can be prepared by contacting the free acid form with a sufficient amount of the required base to form a salt in a conventional manner. The free acid can be regenerated by contacting the salt form with an acid in a conventional manner and then isolating the free acid. The free acid forms are somewhat different from their respective salt forms in their physical properties, such as solubility in polar solvents. But for the purposes of the present disclosure, the salts are still equivalent to their respective free acids.

The salts can be prepared from the inorganic acids, which include sulfates, pyrosulfates, bisulfates, sulfites, bisulfites, nitrates, phosphates, monohydrogen phosphates, dihydrogen phosphates, metaphosphates, pyrophosphates, chlorides, bromides and iodides. Examples of the acids include hydrochloric acid, nitric acid, sulfuric acid, hydrobromic acid, hydroiodic acid, phosphoric acid, etc. The representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthalate, methanesulfonate, glucoheptanate, lactobionate, lauryl sulfonate, isethionate, etc. The salts can also be prepared from the organic acids, which include aliphatic monocarboxylic and dicarboxylic acids, phenyl-substituted alkanoic acids, hydroxyalkanoic acids, alkanedioic acid, aromatic acids, aliphatic and aromatic sulfonic acids, etc. The representative salts include acetate, propionate, octanoate, isobutyrate, oxalate, malonate, succinate, suberate, sebacate, fumarate, maleate, mandelate, benzoate, chlorobenzoate, methyl benzoate, dinitrobenzoate, naphthoate, besylate, tosylate, phenylacetate, citrate, lactate, maleate, tartrate, methanesulfonate, etc. The pharmaceutically acceptable salts can include cations based on alkali metals and alkaline earth metals, such as sodium, lithium, potassium, calcium, magnesium, etc., as well as non-toxic ammonium, quaternary ammonium, and amine cations including, but not limited to, ammonium, tetramethylammonium, tetraethylammonium, methylamine, dimethylamine, trimethylamine, triethylamine, ethylamine, etc. Salts of amino acids are also included, such as arginine salts, gluconates, galacturonates, etc. (for example, see Berge S. M. et al., "Pharmaceutical Salts," J. Pharm. Sci., 1977; 66: 1- 19 for reference).

"Subjects" to which administration is contemplated include, but are not limited to, humans (e.g., males or females of any age group, e.g., paediatric subjects (e.g., infants, children, adolescents) or adult subjects (e.g., young adults, middle-aged adults or older adults)) and/or non-human animals, such as mammals, e.g., primates (e.g., cynomolgus monkeys, rhesus monkeys), cattle, pigs, horses, sheep, goats, rodents, cats and/or dogs. In some embodiments, the subject is a human. In some embodiments, the subject is a non-human animal. The terms "human", "patient" and "subject" can be used interchangeably herein.

"Disease," "disorder," and "condition" can be used interchangeably herein.

Unless indicated, otherwise the term "treatment" as used herein includes the effect on a subject who is suffering from a particular disease, disorder, or condition, which reduces the severity of the disease, disorder, or condition, or delays or slows the progression of the disease, disorder or condition ("therapeutic treatment"). The term also includes the effect that occurs before the subject begins to suffer from a specific disease, disorder or condition ("prophylactic treatment").

Generally, the "effective amount" of a compound refers to an amount sufficient to elicit a target biological response. As understood by those skilled in the art, the effective amount of the compound of the disclosure can vary depending on the following factors, such as the desired biological endpoint, the pharmacokinetics of the compound, the diseases being treated, the mode of administration, and the age, health status and symptoms of the subjects. The effective amount includes therapeutically effective amount and prophylactically effective amount.

Unless indicated, otherwise the "therapeutically effective amount" of the compound as used herein is an amount sufficient to provide therapeutic benefits in the course of treating a disease, disorder or condition, or to delay or minimize one or more symptoms associated with the disease, disorder or condition. The therapeutically effective amount of a compound refers to the amount of the therapeutic agent that, when used alone or in combination with other therapies, provides a therapeutic benefit in the treatment of a disease, disorder or condition. The term "therapeutically effective amount" can include an amount that improves the overall treatment, reduces or avoids the symptoms or causes of the disease or condition, or enhances the therapeutic effect of other therapeutic agents.

Unless indicated, otherwise the "prophylactically effective amount" of the compound as used herein is an amount sufficient to prevent a disease, disorder or condition, or an amount sufficient to prevent one or more symptoms associated with a disease, disorder or condition, or an amount sufficient to prevent the recurrence of a disease, disorder or condition. The prophylactically effective amount of a compound refers to the amount of a therapeutic agent that, when used alone or in combination with other agents, provides a prophylactic benefit in the prevention of a disease, disorder or condition. The term "prophylactically effective amount" can include an amount that improves the overall prevention, or an amount that enhances the prophylactic effect of other preventive agents.

"Combination" and related terms refer to the simultaneous or sequential administration of the compounds of the present disclosure and other therapeutic agents. For example, the compounds of the present disclosure can be administered simultaneously or sequentially in separate unit dosage with other therapeutic agents, or simultaneously in a single unit dosage with other therapeutic agents.

### BRIEF DESCRIPTION OF DRAWINGS

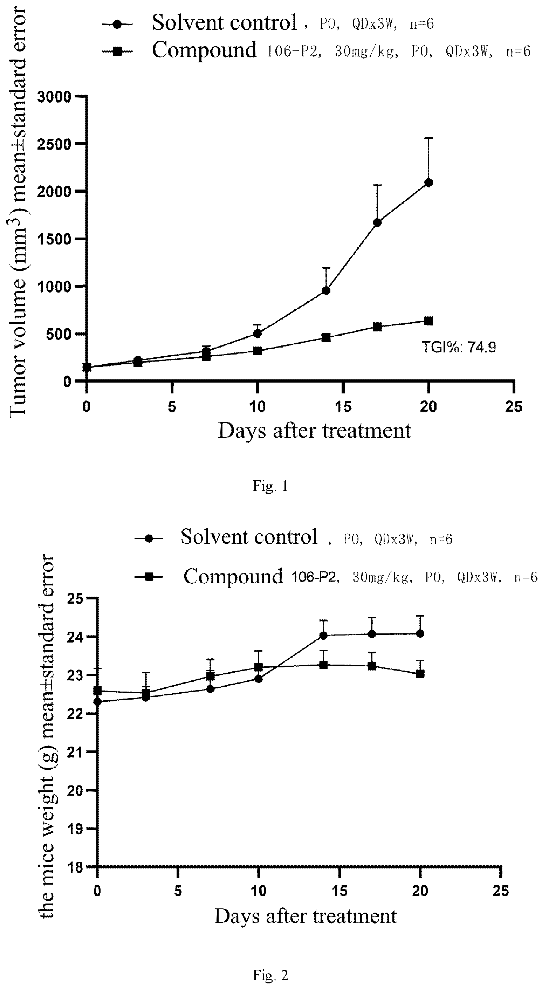

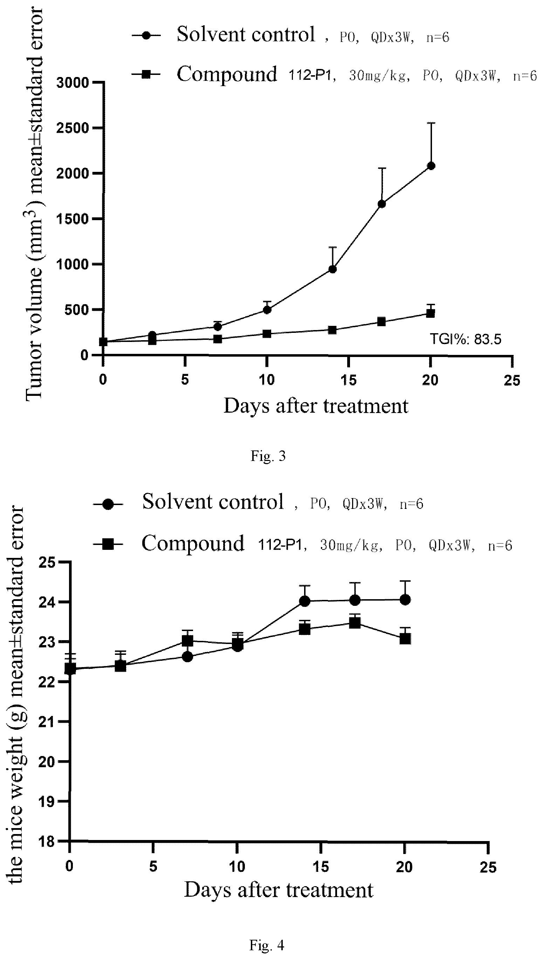

Fig. 1 to Fig. 4 show the results of in vivo pharmacodynamic studies in the LU99 CDX model.

### DETAILED DESCRIPTION

As used herein, the "compound disclosed herein" or "compound of the present disclosure" refers to the following compounds of formula (I), formula (II), etc., or a pharmaceutically acceptable salt, an isotopic variant, a tautomer, a stereoisomer, a prodrug, a polymorph, a hydrate or a solvate thereof.

In the present disclosure, compounds are named using standard nomenclature. For compounds having an asymmetric center, it should be understood, unless otherwise stated, that all optical isomers and mixtures thereof are included. Furthermore, unless otherwise specified, all isomer compounds and carbon-carbon double bonds included in the present disclosure may occur in the form of Z and E. Compounds which exist in different tautomeric forms, one of which is not limited to any particular tautomer, but is intended to cover all tautomeric forms.

In one embodiment, the present disclosure relates to a compound of formula (X), or a pharmaceutically acceptable salt, an isotopic variant, a tautomer, a stereoisomer, a prodrug, a polymorph, a hydrate or a solvate thereof: 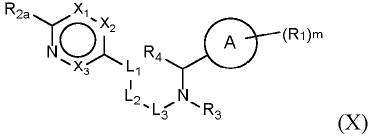 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)(=NRₐ)Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
R₄ is selected from H, D, C₁₋₆ alkyl and C₁₋₆ haloalkyl;
or one R₁ is connected to R₄ to form -(CRₛₛRₛₛ)_{q1}-X-(CRₛₛRₛₛ)_{q2}- (alternatively -(CH₂)_{q1}-X-(CH₂)_{q2}-); wherein X is O, S, NH or CH₂, q1=0, 1 or 2, q2=1, 2 or 3, Rₛₛ is independently selected from H, D, C₁₋₆ alkyl and C₁₋₆ haloalkyl, or two Rₛₛ and the carbon atom to which they are attached are taken together to form C₃₋₁₀ cycloalkyl;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, CN, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; or two R₁ₛ and the atom(s) to which they are attached are taken together to form C₃₋₁₀ cycloalkyl or 3- to 10-membered heterocyclyl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
or, R₃ₛ₁, R₃ₛ₂ and the carbon atom(s) to which they are attached are taken together to form C₃₋₁₀ cycloalkyl;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, R₄, L, R, R₁ₛ, R₂ₛ, R₃ₛ, R₃ₛ₁, R₃ₛ₂, Rₐ, R_{b} and R_{c} is optionally deuterated, up to fully deuterated;
with the proviso that when L₂ is CRₓR_{y} and L₃ is -S(O)- or -S(O)₂-, R_{y} and R_{2d} are combined to form vinylene.

In another embodiment, the present disclosure relates to a compound of formula (I), or a pharmaceutically acceptable salt, an isotopic variant, a tautomer, a stereoisomer, a prodrug, a polymorph, a hydrate or a solvate thereof: 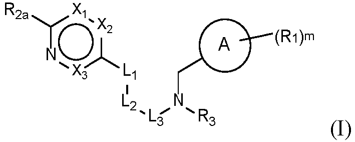 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, L, R, R₁ₛ, R₂ₛ, R₃ₛ, R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.
Ring A

In one embodiment, ring A is C₆₋₁₀ aryl, alternatively phenyl; in another embodiment, ring A is 5- to 10-membered heteroaryl, alternatively 5- to 10-membered heteroaryl, such as phenyl, pyridyl, pyridazinyl, pyrimidinyl or pyrazinyl, alternatively pyridyl or pyridazinyl.

### L₁, L₂, and L₃

In one embodiment, L₁ is NH; in another embodiment, L₁ is CHRₓ; in another embodiment, L₁ is NH and L₂ is CRₓR_{y}, -L₁-L₂- is alternatively 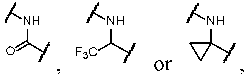 -L₁-L₂- is alternatively 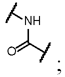 in another embodiment, L₁ is CHRₓ and L₂ is CRₓR_{y}, -L₁-L₂- is alternatively 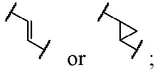 in another embodiment, L₃ is -C(O)-; in another embodiment, L₃ is -S(O)-; in another embodiment, L₃ is -S(O)₂-.

### Rₓ and R_{y}

In one embodiment, Rₓ and R_{y} are H; in another embodiment, Rₓ and R_{y} are D; in another embodiment, Rₓ and R_{y} are C₁₋₆ alkyl; in another embodiment, Rₓ and R_{y} are C₁₋₆ haloalkyl, alternatively CF₃; in another embodiment, CRₓR_{y} are taken together to form C=O; in another embodiment, CRₓR_{y} are taken together to form C=S; in another embodiment, CRₓR_{y} are taken together to form C₃₋₆ cycloalkylene, alternatively cyclopropylene; in another embodiment, L₁ is CHRₓ and L₂ is CRₓR_{y}, and the two Rₓ groups are combined to form a chemical bond; in another embodiment, L₁ is CHRₓ and L₂ is CRₓR_{y}, and the two Rₓ groups are combined to form C₁₋₄ alkylene, alternatively cyclopropylene.

### R₁

In one embodiment, R₁ is H; in another embodiment, R₁ is D; in another embodiment, R₁ is halogen; in another embodiment, R₁ is CN; in another embodiment, R₁ is NO₂; in another embodiment, R₁ is -L-ORₐ; in another embodiment, R₁ is -L-SRₐ; in another embodiment, R₁ is -L-NR_{b}R_{c}; in another embodiment, R₁ is SF₅; in another embodiment, R₁ is C(O)Rₐ; in another embodiment, R₁ is C(O)ORₐ; in another embodiment, R₁ is C(O)NR_{b}R_{c}; in another embodiment, R₁ is OC(O)Rₐ; in another embodiment, R₁ is NR_{b}C(O)R_{c}; in another embodiment, R₁ is S(O)Rₐ; in another embodiment, R₁ is S(O)₂Rₐ; in another embodiment, R₁ is S(O)(=NRₐ)Rₐ; in another embodiment, R₁ is S(O)ORₐ; in another embodiment, R₁ is S(O)₂ORₐ; in another embodiment, R₁ is S(O)NR_{b}R_{c}; in another embodiment, R₁ is S(O)₂NR_{b}R_{c}; in another embodiment, R₁ is P(O)(Rₐ)₂; in another embodiment, R₁ is C₁₋₆ alkyl; in another embodiment, R₁ is C₁₋₆ haloalkyl; in another embodiment, R₁ is C₂₋₆ alkenyl; in another embodiment, R₁ is C₂₋₆ alkynyl; in another embodiment, R₁ is -L-C₃₋₇ cycloalkyl; in another embodiment, R₁ is -L-3- to 7-membered heterocyclyl; in another embodiment, R₁ is -L-C₆₋₁₀ aryl; in another embodiment, R₁ is -L-5- to 10-membered heteroaryl; in another embodiment, R₁ is optionally substituted with 1, 2 or 3 R₁ₛ.

In a more specific embodiment, R₁ is selected from H, D, halogen, CN, NO₂, -L-ORₐ, -L-SRₐ, -L-NR_{b}R_{c}, SF₅, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl; in another more specific embodiment, R₁ is selected from H, D, halogen, CN, NO₂, -L-ORₐ, -L-SRₐ, -L-NR_{b}R_{c}, SF₅, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl; in another more specific embodiment, R₁ is selected from H, D, halogen, CN, ORₐ, SRₐ, NR_{b}R_{c}, SF₅, C₁₋₆ alkyl, C₁₋₆ haloalkyl, C₃₋₇ cycloalkyl and 3- to 7-membered heterocyclyl; in another more specific embodiment, R₁ is selected from H, D, halogen, CN, ORₐ, SRₐ, NR_{b}R_{c}, SF₅, C₁₋₆ alkyl, C₁₋₆ haloalkyl, C₅₋₇ cycloalkyl and 5- to 7-membered heterocyclyl; in another more specific embodiment, R₁ is selected from H, D, halogen, CN, ORₐ, NR_{b}R_{c}, C₁₋₆ alkyl, C₁₋₆ haloalkyl, C₃₋₆ cycloalkyl and 4- to 6-membered heterocyclyl; in another more specific embodiment, R₁ is selected from H, D, halogen, CN, ORₐ, NR_{b}R_{c}, C₁₋₆ alkyl, C₁₋₆ haloalkyl and 5- to 6-membered heterocyclyl; in another more specific embodiment, R₁ is selected from H, D, CN, ORₐ, C₁₋₆ alkyl, C₁₋₆ haloalkyl, C₃₋₆ cycloalkyl and 5- to 6-membered heterocyclyl; in another more specific embodiment, R₁ is selected from H, D, CN, ORₐ, C₁₋₆ alkyl, C₁₋₆ haloalkyl and 5- to 6-membered heterocyclyl; in another more specific embodiment, R₁ is selected from CF₃, CN, OCF₃, OCH₂CH₃, 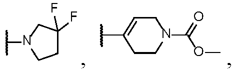 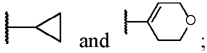 in another more specific embodiment, R₁ is selected from CF₃, CN, OCF₃, OCH₂CH₃, 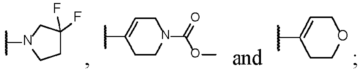 in another more specific embodiment, R₁ is CF₃.

### m

In one embodiment, m=0; in another embodiment, m=1; in another embodiment, m=2; in another embodiment, m=3; in another embodiment, m=4.

### R₄

In one embodiment, R₄ is H; in another embodiment, R₄ is D; in another embodiment, R₄ is C₁₋₆ alkyl; in another embodiment, R₄ is C₁₋₆ haloalkyl; in another embodiment, R₁ and R₄ are connected to form -(CRₛₛRₛₛ)_{q1}-X-(CRₛₛRₛₛ)_{q2}-, wherein X is O, S, NH or CH₂, q1=0, 1 or 2, q2=1, 2 or 3, Rₛₛ is independently selected from H, D, C₁₋₆ alkyl and C₁₋₆ haloalkyl, or two Rₛₛ and the carbon atom to which they are attached are taken together to form C₃₋₁₀ cycloalkyl, such as C₃₋₆ cycloalkyl; in another embodiment, R₁ and R₄ are connected to form -(CH₂)_{q1}-X-(CH₂)_{q2}-, wherein X is O, S, NH or CH₂, q1=0, 1 or 2, q2=1, 2 or 3; in another embodiment, R₁ and R₄ are connected to form -(CH₂)_{q1}-X-(CH₂)_{q2}-, wherein X is O, S, NH or CH₂, q1=0 or 1, q2=1 or 2; in another embodiment, R₁ and R₄ are connected to form -(CH₂)_{q1}-X-(CH₂)_{q2}-, wherein X is O, S or NH₂, q1=0 or 1, q2=1 or 2.

### X₁, X₂ and X₃

In one embodiment, X₁ is C atom; in another embodiment, X₁ is N atom; in another embodiment, X₁ is CR_{2b}; in another embodiment, X₁ is NR_{2b}; in one embodiment, X₂ is C atom; in another embodiment, X₂ is N atom; in another embodiment, X₂ is CR_{2c}; in another embodiment, X₂ is NR_{2c}; in one embodiment, X₃ is C atom; in another embodiment, X₃ is N atom; in another embodiment, X₃ is CR_{2d}; in another embodiment, X₃ is NR_{2d}.

In a more specific embodiment, 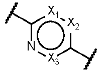 is selected from 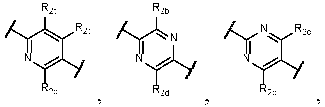 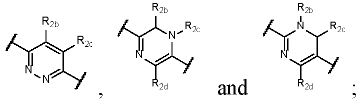 in another more specific embodiment, 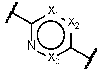 is 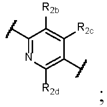 in another more specific embodiment, 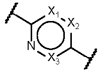 is 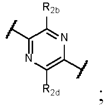 in another more specific embodiment, 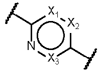 is 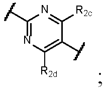 in another more specific embodiment, 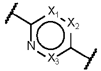 is 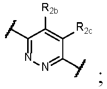 in another more specific embodiment, 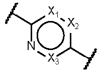 is 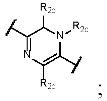 in another more specific embodiment, 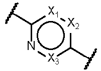 is

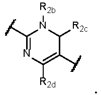

In a more specific embodiment, X₁, X₂ and their substituents are taken together to form C₅₋₁₀ cycloalkyl; in another more specific embodiment, X₁, X₂ and their substituents are taken together to form 5- to 10-membered heterocyclyl; in another more specific embodiment, X₁, X₂ and their substituents are taken together to form C₆₋₁₀ aryl; in another more specific embodiment, X₁, X₂ and their substituents are taken together to form 5- to 10-membered heteroaryl; in another more specific embodiment, X₁, X₂ and their substituents are taken together to form C₅₋₇ cycloalkyl, 5- to 7-membered heterocyclyl, phenyl or 5- to 6-membered heteroaryl; in another more specific embodiment, X₁, X₂ and their substituents are taken together to form 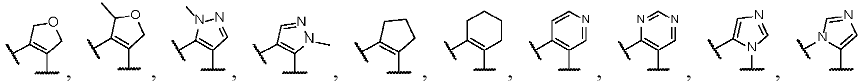 or 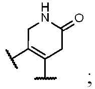 alternatively form 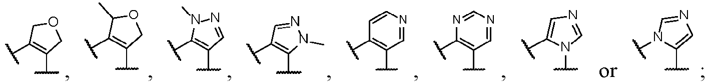 alternatively form 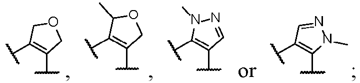 alternatively form

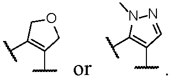

### R₂ₐ

In one embodiment, R₂ₐ is H; in another embodiment, R₂ₐ is D; in another embodiment, R₂ₐ is CN; in another embodiment, R₂ₐ is ORₐ; in another embodiment, R₂ₐ is NR_{b}R_{c}; in another embodiment, R₂ₐ is C(O)ORₐ; in another embodiment, R₂ₐ is C(O)NR_{b}R_{c}.

In a more specific embodiment, R₂ₐ is selected from H, D, CN, ORₐ and NR_{b}R_{c}; in another more specific embodiment, R₂ₐ is selected from ORₐ and NR_{b}R_{c}; in another more specific embodiment, R₂ₐ is selected from OMe and NH₂, alternatively NH₂.

### R_{2b}

In one embodiment, R_{2b} is H; in another embodiment, R_{2b} is D; in another embodiment, R_{2b} is halogen; in another embodiment, R_{2b} is C(O)ORₐ; in another embodiment, R_{2b} is C(O)NR_{b}R_{c}; in another embodiment, R_{2b} is C₁₋₆ alkyl; in another embodiment, R_{2b} is C₁₋₆ haloalkyl; in another embodiment, R_{2b} is C₃₋₁₀ cycloalkyl; in another embodiment, R_{2b} is 3- to 10-membered heterocyclyl.

In a more specific embodiment, R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl; in another more specific embodiment, R_{2b} is selected from H, D, halogen, C(O)NR_{b}R_{c}, C₁₋₆ alkyl, C₁₋₆ haloalkyl and 4- to 6-membered heterocyclyl; in another more specific embodiment, R_{2b} is selected from H, D, halogen, C(O)NR_{b}R_{c}, C₁₋₆ alkyl and C₁₋₆ haloalkyl; in another more specific embodiment, R_{2b} is selected from H, D, halogen, C(O)NR_{b}R_{c} and C₁₋₆ alkyl; in another more specific embodiment, R_{2b} is selected from H, F, Cl, Br, Me, CH₂CH₃, C(O)NH₂ and 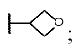 in another more specific embodiment, R_{2b} is selected from H, F, Cl, Br, Me, CH₂CH₃ and C(O)NH₂, alternatively selected from Me and C(O)NH₂.

### R_{2c}

In one embodiment, R_{2c} is H; in another embodiment, R_{2c} is D; in another embodiment, R_{2c} is halogen; in another embodiment, R_{2c} is C₁₋₆ alkyl; in another embodiment, R_{2c} is C₁₋₆ haloalkyl.

In a more specific embodiment, R_{2c} is selected from H, halogen and Me; alternatively selected from H and Me.

### R_{2d}

In one embodiment, R_{2d} is H; in another embodiment, R_{2d} is D; in another embodiment, R_{2d} is halogen; in another embodiment, R₂₄ is CN; in another embodiment, R_{2d} is NO₂; in another embodiment, R_{2d} is ORₐ; in another embodiment, R_{2d} is NR_{b}R_{c}; in another embodiment, R_{2d} is C₁₋₆ alkyl; in another embodiment, R_{2d} is C₁₋₆ haloalkyl; in another embodiment, R_{2d} is C₅₋₁₀ cycloalkyl; in another embodiment, R_{2d} is 5- to 10-membered heterocyclyl; in another embodiment, R_{2d} is C₆₋₁₀ aryl; in another embodiment, R₂ₐ is 5- to 10-membered heteroaryl.

In a more specific embodiment, R_{2d} is selected from H, D, C₁₋₆ alkyl and C₁₋₆ haloalkyl; in another more specific embodiment, R_{2d} is selected from H and D.

### R₃

In one embodiment, R₃ is -C₁₋₆ alkylene-ORₐ; in another embodiment, R₃ is -C₁₋₆ alkylene-NR_{b}R_{c}; in another embodiment, R₃ is C₁₋₆ alkyl; in another embodiment, R₃ is C₁₋₆ haloalkyl; in another embodiment, R₃ is C₂₋₆ alkenyl; in another embodiment, R₃ is C₂₋₆ alkynyl; in another embodiment, R₃ is -L-C₃₋₁₀ cycloalkyl; in another embodiment, R₃ is -L-3- to 10-membered heterocyclyl; in another embodiment, R₃ is -L-C₆₋₁₀ aryl; in another embodiment, R₃ is -L-5- to 10-membered heteroaryl; in another embodiment, R₃ is optionally substituted with 1, 2 or 3 R₃ₛ₁ and one R₃ₛ₂.

In a more specific embodiment, R₃ is selected from C₁₋₆ alkyl, C₁₋₆ haloalkyl, -L-C₃₋₁₀ cycloalkyl, -L-3- to 10-membered heterocyclyl, -L-C₆₋₁₀ aryl and -L-5- to 10-membered heteroaryl; in another more specific embodiment, R₃ is selected from C₁₋₆ alkyl, C₁₋₆ haloalkyl, C₅₋₆ cycloalkyl, 5- to 6-membered heterocyclyl, phenyl and 5- to 6-membered heteroaryl; in another more specific embodiment, R₃ is selected from Me, Et, iPr, cyclopropyl, cyclobutyl,

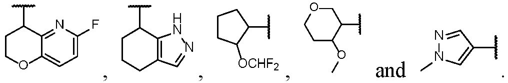

### L

In one embodiment, L is a chemical bond; in another embodiment, L is C₁₋₆ alkylene; in another embodiment, L is C₂₋₆ alkenylene; in another embodiment, L is C₂₋₆ alkynylene; in another embodiment, L is optionally substituted with 1, 2 or 3 R.

### R₁ₛ

In one embodiment, R₁ₛ is H; in another embodiment, R₁ₛ is D; in another embodiment, R₁ₛ is halogen; in another embodiment, R₁ₛ is CN; in another embodiment, R₁ₛ is =O; in another embodiment, R₁ₛ is C(O)ORₐ; in another embodiment, R₁ₛ is C(O)NR_{b}R_{c}; in another embodiment, R₁ₛ is C₁₋₆ alkyl; in another embodiment, R₁ₛ is C₁₋₆ haloalkyl; in another embodiment, R₁ₛ is C₂₋₆ alkenyl; in another embodiment, R₁ₛ is C₂₋₆ alkynyl; in another embodiment, R₁ₛ is C₃₋₁₀ cycloalkyl; in another embodiment, R₁ₛ is 3- to 10-membered heterocyclyl; in another embodiment, R₁ₛ is C₆₋₁₀ aryl; in another embodiment, R₁ₛ is 5- to 10-membered heteroaryl; in another embodiment, two R₁ₛ and the atom(s) to which they are attached are taken together to form C₃₋₁₀ cycloalkyl; in another embodiment, two R₁ₛ and the atom(s) to which they are attached are taken together to form 3- to 10-membered heterocyclyl.

In one embodiment, R₂ₛ is H; in another embodiment, R₂ₛ is D; in another embodiment, R₂ₛ is halogen; in another embodiment, R₂ₛ is =O; in another embodiment, R₂ₛ is C(O)ORₐ; in another embodiment, R₂ₛ is C(O)NR_{b}R_{c}; in another embodiment, R₂ₛ is C₁₋₆ alkyl; in another embodiment, R₂ₛ is C₁₋₆ haloalkyl; in another embodiment, R₂ₛ is C₂₋₆ alkenyl; in another embodiment, R₂ₛ is C₂₋₆ alkynyl; in another embodiment, R₂ₛ is C₃₋₁₀ cycloalkyl; in another embodiment, R₂ₛ is 3- to 10-membered heterocyclyl; in another embodiment, R₂ₛ is C₆₋₁₀ aryl; in another embodiment, R₂ₛ is 5- to 10-membered heteroaryl.

In a more specific embodiment, R₁ₛ is selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; in another more specific embodiment, R₁ₛ is selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl; in another more specific embodiment, R₁ₛ is selected from H, D, halogen, C(O)ORₐ, C(O)NR_{b}R_{c}, C₁₋₆ alkyl and C₁₋₆ haloalkyl; in another more specific embodiment, R₁ₛ is selected from H, D, halogen, C(O)ORₐ, C₁₋₆ alkyl and C₁₋₆ haloalkyl; in another more specific embodiment, R₁ₛ is selected from halogen and C(O)OCH₃.

In a more specific embodiment, R₂ₛ is selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; in another more specific embodiment, R₂ₛ is selected from H, D, halogen, =O, C₁₋₆ alkyl and C₁₋₆ haloalkyl; in another more specific embodiment, R₂ₛ is selected from H, D, =O, C₁₋₆ alkyl and C₁₋₆ haloalkyl; in another more specific embodiment, R₂ₛ is selected from H, D, =O and Me.

### R₃ₛ₁

In one embodiment, R₃ₛ₁ is H; in another embodiment, R₃ₛ₁ is D; in another embodiment, R₃ₛ₁ is halogen; in another embodiment, R₃ₛ₁ is C₁₋₆ alkyl; in another embodiment, R₃ₛ₁ is C₁₋₆ haloalkyl; in another embodiment, R₃ₛ₁ is C₂₋₆ alkenyl; in another embodiment, R₃ₛ₁ is C₂₋₆ alkynyl; in another embodiment, R₃ₛ₁ is C₃₋₁₀ cycloalkyl; in another embodiment, R₃ₛ₁ is 3- to 10-membered heterocyclyl; in another embodiment, R₃ₛ₁ is C₆₋₁₀ aryl; in another embodiment, R₃ₛ₁ is 5- to 10-membered heteroaryl.

In a more specific embodiment, R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; in another more specific embodiment, R₃ₛ₁ is selected from H, D, halogen, C₁₋₆ alkyl, C₁₋₆ haloalkyl, C₃₋₆ cycloalkyl and 3- to 6-membered heterocyclyl; in another more specific embodiment, R₃ₛ₁ is selected from H, D, C₁₋₆ alkyl, C₁₋₆ haloalkyl, C₃₋₆ cycloalkyl and 3- to 6-membered heterocyclyl; in another more specific embodiment, R₃ₛ₁ is selected from H, D, C₁₋₆ alkyl, C₁₋₆ haloalkyl and C₃₋₆ cycloalkyl; in another more specific embodiment, R₃ₛ₁ is selected from H, Me and cyclopropyl; alternatively selected from H and Me.

### R₃ₛ₂

In one embodiment, R₃ₛ₂ is H; in another embodiment, R₃ₛ₂ is D; in another embodiment, R₃ₛ₂ is -L-ORₐ; in another embodiment, R₃ₛ₂ is -L-NR_{b}R_{c}; in another embodiment, R₃ₛ₂ is C₁₋₆ alkyl; in another embodiment, R₃ₛ₂ is C₁₋₆ haloalkyl; in another embodiment, R₃ₛ₂ is C₃₋₁₀ cycloalkyl; in another embodiment, R₃ₛ₂ is 5- to 10-membered heterocyclyl; in another embodiment, R₃ₛ₂ is C₆₋₁₀ aryl; in another embodiment, R₃ₛ₂ is 5- to 10-membered heteroaryl; in another embodiment, R₃ₛ₂ is optionally substituted with 1, 2 or 3 R₃ₛ.

In a more specific embodiment, R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl; in another more specific embodiment, R₃ₛ₂ is selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl; in another more specific embodiment, R₃ₛ₂ is selected from H, D, -L-ORₐ, C₁₋₆ alkyl, C₁₋₆ haloalkyl, C₅₋₇ cycloalkyl, phenyl and 5- to 10-membered heteroaryl; in another more specific embodiment, R₃ₛ₂ is selected from H, D, Me, CF₃, CH₂OCH₃, cyclopropyl, 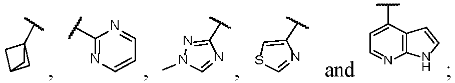 alternatively selected from C₅₋₇ cycloalkyl, 5- to 7-membered heterocyclyl, C₆₋₁₀ aryl and 5- to 10-membered heteroaryl; alternatively C₅₋₇ cycloalkyl, such as cyclopropyl; alternatively Et.

In another more specific embodiment, R₃ₛ₁, R₃ₛ₂ and the carbon atom(s) to which they are attached are taken together to form C₃₋₁₀ cycloalkyl, such as C₃₋₆ cycloalkyl.

### R

In one embodiment, R is H; in another embodiment, R is D; in another embodiment, R is halogen; in another embodiment, R is C₁₋₆ alkyl; in another embodiment, R is C₁₋₆ haloalkyl.

### R₃ₛ

In one embodiment, R₃ₛ is H; in another embodiment, R₃ₛ is D; in another embodiment, R₃ₛ is halogen; in another embodiment, R₃ₛ is C₁₋₆ alkyl; in another embodiment, R₃ₛ is C₁₋₆ haloalkyl.

### Rₐ, R_{b} and R_{c}

In one embodiment, Rₐ, R_{b} and R_{c} are independently H; in another embodiment, Rₐ, R_{b} and R_{c} are independently D; in another embodiment, Rₐ, R_{b} and R_{c} are independently C₁₋₆ alkyl; in another embodiment, Rₐ, R_{b} and R_{c} are independently C₁₋₆ haloalkyl; in another embodiment, Rₐ, R_{b} and R_{c} are independently C₃₋₁₀ cycloalkyl; in another embodiment, Rₐ, R_{b} and R_{c} are independently 3- to 10-membered heterocyclyl; in another embodiment, Rₐ, R_{b} and R_{c} are independently C₆₋₁₀ aryl; in another embodiment, Rₐ, R_{b} and R_{c} are independently 5- to 10-membered heteroaryl; in another embodiment, R_{b}, R_{c} and the atom(s) to which they are attached are taken together to form 5- to 10-membered heterocyclyl, alternatively 5- to 6-membered heterocyclyl.

Any technical solution in any one of the above specific embodiments, or any combination thereof, may be combined with any technical solution in other specific embodiments or any combination thereof. For example, any technical solution of ring A or any combination thereof may be combined with any technical solution of L₁, L₂, L₃, Rₓ, R_{y}, R₁, m, X₁, X₂, X₃, R₂ₐ, R_{2b}, R_{2c}, R_{2d}, R₃, L, R, R₁ₛ, R₂ₛ, R₃ₛ₁ R₃ₛ₁, R₃ₛ₂, Rₐ, R_{b} and R_{c}, etc., or any combination thereof. The present disclosure is intended to include all combination of such technical solutions, which are not exhaustively listed here to save space.

In a more specific embodiment, the present disclosure provides a compound of formula (X), or a pharmaceutically acceptable salt, an isotopic variant, a tautomer, a stereoisomer, a prodrug, a polymorph, a hydrate or a solvate thereof: 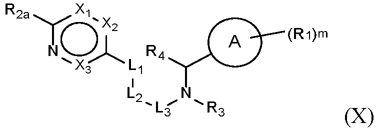 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)(=NRₐ)Rₐ, S(O)ORₐ, S(O)₂0Rₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
R₄ is selected from H, D, C₁₋₆ alkyl and C₁₋₆ haloalkyl;
or one R₁ is connected to R₄ to form -(CH₂)_{q1}-X-(CH₂)_{q2}-; wherein X is O, S, NH or CH₂, q1=0, 1 or 2, q2=1, 2 or 3;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, R₄, L, R, R₁ₛ, R₂ₛ, R₃ₛ₁ R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.

In a more specific embodiment, the present disclosure provides a compound of formula (I), or a pharmaceutically acceptable salt, an isotopic variant, a tautomer, a stereoisomer, a prodrug, a polymorph, a hydrate or a solvate thereof: 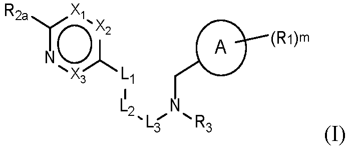 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and one R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, L, R, R₁ₛ, R₂ₛ, R₃ₛ₁ R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, ring A is phenyl or 5- to 6-membered heteroaryl; alternatively phenyl, pyridyl, pyridazinyl, pyrimidinyl or pyrazinyl.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, L₁ is NH and L₂ is CRₓR_{y}; -L₁-L₂- is alternatively 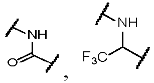 or 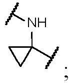 -L₁-L₂- is alternatively

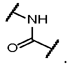

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, L₁ is CHRₓ and L₂ is CRₓR_{y}; -L₁-L₂- is alternatively

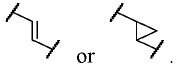

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, L₃ is -C(O)-.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₁ is selected from H, D, halogen, CN, NO₂, -L-ORₐ, -L-SRₐ, -L-NR_{b}R_{c}, SF₅, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl; alternatively selected from H, D, halogen, CN, NO₂, -L-ORₐ, -L-SRₐ, -L-NR_{b}R_{c}, SF₅, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl; alternatively selected from H, D, halogen, CN, ORₐ, SRₐ, NR_{b}R_{c}, SF₅, C₁₋₆ alkyl, C₁₋₆ haloalkyl, C₃₋₇ cycloalkyl and 3- to 7-membered heterocyclyl; alternatively selected from H, D, halogen, CN, ORₐ, SRₐ, NR_{b}R_{c}, SF₅, C₁₋₆ alkyl, C₁₋₆ haloalkyl, C₅₋₇ cycloalkyl and 5- to 7-membered heterocyclyl; alternatively selected from H, D, halogen, CN, ORₐ, NR_{b}R_{c}, C₁₋₆ alkyl, C₁₋₆ haloalkyl, C₃₋₆ cycloalkyl and 4- to 6-membered heterocyclyl; alternatively selected from H, D, halogen, CN, ORₐ, NR_{b}R_{c}, C₁₋₆ alkyl, C₁₋₆ haloalkyl and 5- to 6-membered heterocyclyl; alternatively selected from H, D, CN, ORₐ, C₁₋₆ alkyl, C₁₋₆ haloalkyl, C₃₋₆ cycloalkyl and 5- to 6-membered heterocyclyl; alternatively selected from H, D, CN, ORₐ, C₁₋₆ alkyl, C₁₋₆ haloalkyl and 5- to 6-membered heterocyclyl; alternatively selected from CF₃, CN, OCF₃, OCH₂CH₃, 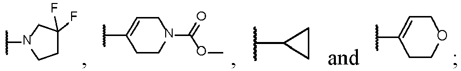 alternatively selected from CF₃, CN, OCF₃, OCH₂CH₃, 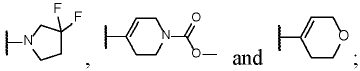 alternatively CF₃.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₁ and R₄ are connected to form -(CH₂)_{q1}-X-(CH₂)_{q2}-; wherein X is O, S, NH or CH₂, q1=0 or 1, q2=1 or 2; alternatively, R₁ and R₄ are connected to form -(CH₂)_{q1}-X-(CH₂)_{q2}-; wherein X is O, S or NH, q1=0 or 1, q2=1 or 2.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, 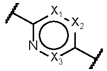 is selected from 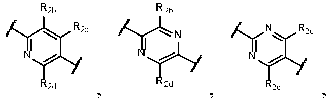 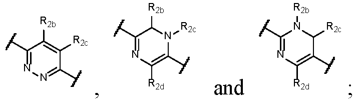 alternatively

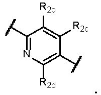

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₂ₐ is selected from H, D, CN, ORₐ and NR_{b}R_{c}; alternatively selected from ORₐ and NR_{b}R_{c}; alternatively selected from OMe and NH₂; alternatively NH₂.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl; alternatively selected from H, D, halogen, C(O)NR_{b}R_{c}, C₁₋₆ alkyl, C₁₋₆ haloalkyl and 4- to 6-membered heterocyclyl; alternatively selected from H, D, halogen, C(O)NR_{b}R_{c}, C₁₋₆ alkyl and C₁₋₆ haloalkyl; alternatively selected from H, D, halogen, C(O)NR_{b}R_{c} and C₁₋₆ alkyl; alternatively selected from H, F, Cl, Br, Me, CH₂CH₃, C(O)NH₂ and 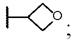 alternatively selected from H, F, Cl, Br, Me, CH₂CH₃ and C(O)NH₂; alternatively selected from Me and C(O)NH₂.

In a more specific embodiment, the present disclosure provides the above compound of formula (I), or a pharmaceutically acceptable salt, an isotopic variant, a tautomer, a stereoisomer, a prodrug, a polymorph, a hydrate or a solvate thereof, wherein, R_{2c} is selected from H and Me.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, X₁, X₂ and their substituents are taken together to form C₅₋₁₀ cycloalkyl, 5-to 10-membered heterocyclyl, C₆₋₁₀ aryl or 5- to 10-membered heteroaryl; alternatively form C₅₋₇ cycloalkyl, 5-to 7-membered heterocyclyl, phenyl or 5- to 6-membered heteroaryl; alternatively form 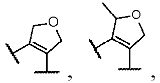 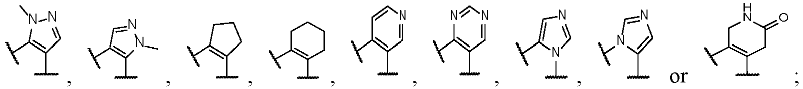 alternatively form 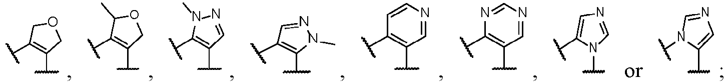 alternatively form 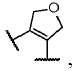 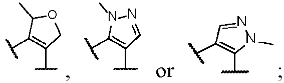 alternatively form

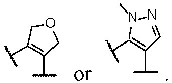

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R_{2d} is selected from H, D, C₁₋₆ alkyl and C₁₋₆ haloalkyl; alternatively selected from H and D.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₃ is selected from C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl; alternatively selected from C₁₋₆ alkyl, C₁₋₆ haloalkyl, -L-C₃₋₁₀ cycloalkyl, -L-3- to 10-membered heterocyclyl, -L-C₆₋₁₀ aryl and -L-5- to 10-membered heteroaryl; alternatively selected from C₁₋₆ alkyl, C₁₋₆ haloalkyl, C₅₋₆ cycloalkyl, 5- to 6-membered heterocyclyl, phenyl and 5- to 6-membered heteroaryl; alternatively selected from Me, Et, iPr, cyclopropyl, cyclobutyl,

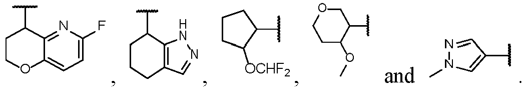

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₁ₛ is selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; alternatively selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl; alternatively selected from H, D, halogen, C(O)ORₐ, C(O)NR_{b}R_{c}, C₁₋₆ alkyl and C₁₋₆ haloalkyl; alternatively selected from H, D, halogen, C(O)ORₐ, C₁₋₆ alkyl and C₁₋₆ haloalkyl, alternatively selected from halogen and C(O)OCH₃.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₂ₛ is selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; alternatively selected from H, D, halogen, =O, C₁₋₆ alkyl and C₁₋₆ haloalkyl; alternatively selected from H, D, =O, C₁₋₆ alkyl and C₁₋₆ haloalkyl; alternatively selected from H, D, =O and Me.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; alternatively selected from H, D, halogen, C₁₋₆ alkyl, C₁₋₆ haloalkyl, C₃₋₆ cycloalkyl and 3- to 6-membered heterocyclyl; alternatively selected from H, D, C₁₋₆ alkyl, C₁₋₆ haloalkyl, C₃₋₆ cycloalkyl and 3- to 6-membered heterocyclyl; alternatively selected from H, D, C₁₋₆ alkyl, C₁₋₆ haloalkyl and C₃₋₆ cycloalkyl; alternatively selected from H, Me and cyclopropyl; alternatively selected from H and Me.

In a more specific embodiment, the present disclosure provides the above compound, or a pharmaceutically acceptable salt, an isotopic variant, a tautomer, a stereoisomer, a prodrug, a polymorph, a hydrate or a solvate thereof, wherein, R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl; alternatively selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl; alternatively selected from H, D, -L-ORₐ, C₁₋₆ alkyl, C₁₋₆ haloalkyl, C₅₋₇ cycloalkyl, phenyl and 5- to 10-membered heteroaryl; alternatively selected from H, D, Me, CF₃, CH₂OCH₃, cyclopropyl, 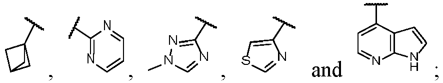 alternatively selected from C₅₋₇ cycloalkyl, 5- to 7-membered heterocyclyl, C₆₋₁₀ aryl and 5- to 10-membered heteroaryl; alternatively C₅₋₇ cycloalkyl, such as cyclopropyl; alternatively Et.

In a more specific embodiment, the present disclosure provides the above compound, or a pharmaceutically acceptable salt, an isotopic variant, a tautomer, a stereoisomer, a prodrug, a polymorph, a hydrate or a solvate thereof, and the compound has the following structural formula: 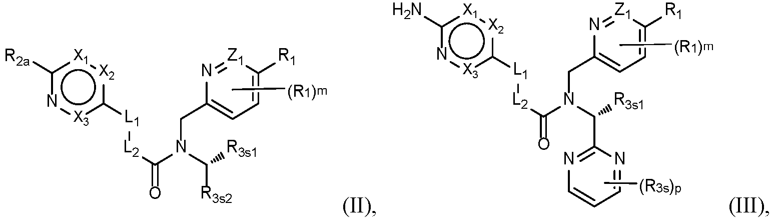 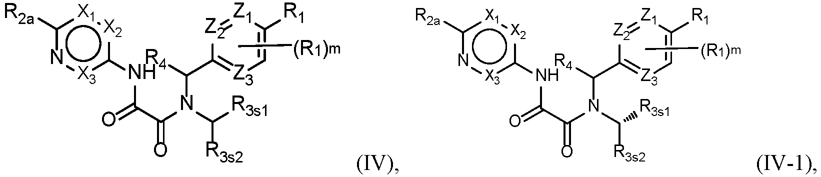 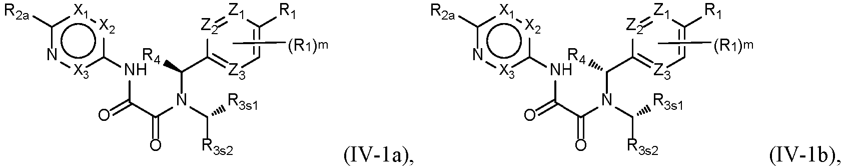 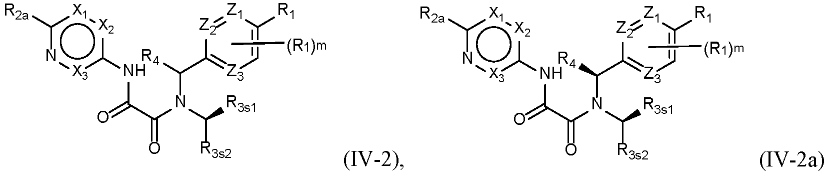 or 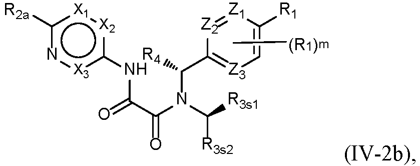 wherein each group is as defined above.

In a more specific embodiment, the present disclosure provides the above compound, or a pharmaceutically acceptable salt, an isotopic variant, a tautomer, a stereoisomer, a prodrug, a polymorph, a hydrate or a solvate thereof, and the compound is a compound of formula (II), wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
Z₁ is CH or N;
R₁ is H, D, halogen, CN, ORₐ, SRₐ, NR_{b}R_{c}, SF₅, C₁₋₆ alkyl, C₁₋₆ haloalkyl, C₅₋₇ cycloalkyl or 5- to 7-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, ORₐ and NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₁ₛ is selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl;
R₂ₛ is selected from H, D, halogen, =O, C₁₋₆ alkyl and C₁₋₆ haloalkyl;
R₃ₛ₁ is selected from H, D, halogen, C₁₋₆ alkyl, C₁₋₆ haloalkyl, C₃₋₆ cycloalkyl and 3- to 6-membered heterocyclyl;
R₃ₛ₂ is selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl, which is optionally substituted with 1, 2 or 3 R₃ₛ;
L is a chemical bond or C₁₋₆ alkylene, which is optionally substituted with 1, 2 or 3 R;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the groups in the above L₁, L₂, Rₓ, R_{y}, Z₁, R₁, R₂ₐ, R_{2b}, R_{2c}, R_{2d}, R₁ₛ, R₂ₛ, R₃ₛ₁, R₃ₛ₂, L, R, R₃ₛ, Rₐ, R_{b} and R_{c} is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₄ cycloalkylene;
or when L₁ is CHRₓ and L₂ is CRₓR_{y}, the two Rₓ groups may combine to form a chemical bond or C₁₋₃ alkylene;
Z₁ is CH or N;
R₁ is selected from H, D, halogen, CN, ORₐ, NR_{b}R_{c}, C₁₋₆ alkyl, C₁₋₆ haloalkyl and 5- to 6-membered heterocyclyl, wherein the heterocyclyl is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is NH₂;
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H, D, C₁₋₆ alkyl and C₁₋₆ haloalkyl;
R₁ₛ is selected from H, D, halogen, C(O)ORₐ, C(O)NR_{b}R_{c}, C₁₋₆ alkyl and C₁₋₆ haloalkyl;
R₂ₛ is selected from H, D, =O, C₁₋₆ alkyl and C₁₋₆ haloalkyl;
R₃ₛ₁ is selected from H, D, C₁₋₆ alkyl, C₁₋₆ haloalkyl, C₃₋₆ cycloalkyl and 3- to 6-membered heterocyclyl;
R₃ₛ₂ is selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl, which is optionally substituted with 1, 2 or 3 R₃ₛ;
L is a chemical bond or C₁₋₃ alkylene, which is optionally substituted with 1, 2 or 3 R;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl.
In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₃ alkyl or C₁₋₃ haloalkyl;
or CRₓR_{y} are taken together to form C=O or cyclopropylene;
or when L₁ is CHRₓ and L₂ is CRₓR_{y}, the two Rₓ groups may combine to form a chemical bond or C₁₋₂ alkylene;
Z₁ is CHor N;
R₁ is selected from H, D, CN, ORₐ, C₁₋₆ alkyl, C₁₋₆ haloalkyl and 5- to 6-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is NH₂;
R_{2b} is selected from H, D, halogen, C(O)NR_{b}R_{c}, C₁₋₆ alkyl, C₁₋₆ haloalkyl and 4- to 6-membered heterocyclyl;
R_{2c} is selected from H, D, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ; alternatively form 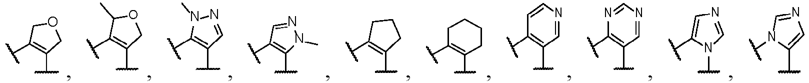 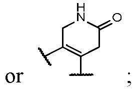
R_{2d} is selected from H and D;
R₁ₛ is selected from H, D, halogen, C(O)ORₐ, C₁₋₆ alkyl and C₁₋₆ haloalkyl, alternatively halogen or C(O)OCH₃;
R₂ₛ is selected from H, D, =O and Me;
R₃ₛ₁ is selected from H, D, C₁₋₆ alkyl, C₁₋₆ haloalkyl and C₃₋₆ cycloalkyl;
R₃ₛ₂ is selected from H, D, -L-ORₐ, C₁₋₆ alkyl, C₁₋₆ haloalkyl, C₅₋₇ cycloalkyl, phenyl and 5- to 10-membered heteroaryl, which is optionally substituted with 1, 2 or 3 R₃ₛ; alternatively, R₃ₛ₂ is selected from H, D, Me, CF₃, CH₂OCH₃, cyclopropyl, 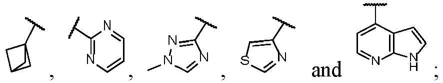
L is a chemical bond or C₁₋₃ alkylene, alternatively methylene, which is optionally substituted with 1, 2 or 3 R;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3 or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl and C₁₋₆ haloalkyl;
Z₁ is CH;
L is a chemical bond, C₁₋₆ alkylene, C₂₋₆ alkenylene or C₂₋₆ alkynylene;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, C₁₋₆ alkyl and C₁₋₆ haloalkyl;
Z₁ is CH;
L is a chemical bond or C₁₋₆ alkylene;
R₁ₛ and R₂ₛ are independently selected from H, D, halogen, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl and C₁₋₆ haloalkyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, -L-ORₐ, -L-SRₐ, -L-NR_{b}R_{c}, C(O)Rₐ, C(O)ORₐ, C(O)NR_{b}R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from H, D, CN, ORₐ and NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen and CN;
Z₁ is CH;
L is a chemical bond, C₁₋₆ alkylene, C₂₋₆ alkenylene or C₂₋₆ alkynylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from ORₐ and NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H, D, halogen, C₁₋₄ alkyl and C₁₋₄ haloalkyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from ORₐ and NR_{b}R_{c};
R_{2b} is selected from C(O)ORₐ, C(O)NR_{b}R_{c} and C₁₋₆ alkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is Me;
R₃ₛ₂ is Et;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 6-membered heteroaryl, which is optionally substituted with 1, 2 or 3 R₁ₛ;
m = 0, 1, 2, 3, or 4;
X₃ is C atom, which is substituted with R_{2d};
R₂ₐ is selected from ORₐ and NR_{b}R_{c};
X₁, X₂ and their substituents are taken together to form 5- to 6-membered heteroaryl, which is optionally substituted with 1, 2 or 3 R₂ₛ;
R_{2d} is selected from H, D, halogen and CN;
Z₁ is CH;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H and D;
R₃ₛ₂ is selected from C₃₋₆ cycloalkyl, 5- to 6-membered heterocyclyl, phenyl and 5- to 6-membered heteroaryl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (II), or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 6-membered heteroaryl, which is optionally substituted with 1, 2 or 3 R₁ₛ;
m = 0, 1, 2, 3, or 4;
X₃ is C atom, which is substituted with R_{2d};
R₂ₐ is selected from ORₐ and NR_{b}R_{c};
X₁, X₂ and their substituents are taken together to form 5- to 6-membered heteroaryl, which is optionally substituted with 1, 2 or 3 R₂ₛ;
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from C₁₋₄ alkyl and C₁₋₄ haloalkyl;
R₃ₛ₂ is selected from -L-ORₐ, -L-NR_{b}R_{c}, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (III), or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene;
Z₁ is CH or N;
R₁ is selected from H, D, halogen, CN, ORₐ, SRₐ, NR_{b}R_{c}, SF₅, C₁₋₆ alkyl, C₁₋₆ haloalkyl, C₅₋₇ cycloalkyl and 5- to 7-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl;
R₂ₛ is selected from H, D, halogen, =O, C₁₋₆ alkyl and C₁₋₆ haloalkyl;
R₃ₛ₁ is selected from H, D, halogen, C₁₋₆ alkyl, C₁₋₆ haloalkyl, C₃₋₆ cycloalkyl and 3- to 6-membered heterocyclyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the groups in the above L₁, L₂, Rₓ, R_{y}, Z₁, R₁, R_{2b}, R_{2c}, R₁ₛ, R₂ₛ, R₃ₛ₁, R₃ₛ, Rₐ, R_{b} and R_{c} is optionally deuterated, up to fully deuterated.

In a more specific embodiment, the present disclosure provides the above compound of formula (III), or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₄ cycloalkylene;
or when L₁ is CHRₓ and L₂ is CRₓR_{y}, the two Rₓ groups may combine to form a chemical bond or C₁₋₃ alkylene;
Z₁ is CH or N;
R₁ is selected from H, D, halogen, CN, ORₐ, NR_{b}R_{c}, C₁₋₆ alkyl, C₁₋₆ haloalkyl and 5- to 6-membered heterocyclyl, wherein the heterocyclyl is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C(O)NR_{b}R_{c}, C₁₋₆ alkyl and C₁₋₆ haloalkyl;
R₂ₛ is selected from H, D, =O, C₁₋₆ alkyl and C₁₋₆ haloalkyl;
R₃ₛ₁ is selected from H, D, C₁₋₆ alkyl, C₁₋₆ haloalkyl, C₃₋₆ cycloalkyl and 3- to 6-membered heterocyclyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl.

In a more specific embodiment, the present disclosure provides the above compound of formula (III), or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₃ alkyl or C₁₋₃ haloalkyl;
or CRₓR_{y} are taken together to form C=O or cyclopropylene;
or when L₁ is CHRₓ and L₂ is CRₓR_{y}, the two Rₓ groups may combine to form a chemical bond or C₁₋₂ alkylene;
Z₁ is CH or N;
R₁ is selected from H, D, CN, ORₐ, C₁₋₆ alkyl, C₁₋₆ haloalkyl and 5- to 6-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)NR_{b}R_{c}, C₁₋₆ alkyl, C₁₋₆ haloalkyl and 4- to 6-membered heterocyclyl;
R_{2c} is selected from H, D, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C₁₋₆ alkyl and C₁₋₆ haloalkyl, alternatively halogen or C(O)OCH₃;
R₂ₛ is selected from H, D, =O and Me;
R₃ₛ₁ is selected from H, D, C₁₋₆ alkyl, C₁₋₆ haloalkyl and C₃₋₆ cycloalkyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl.

In a more specific embodiment, the present disclosure provides the above compound of formula (III), or a pharmaceutically acceptable salt, an isotopic variant, a tautomer, a stereoisomer, a prodrug, a polymorph, a hydrate or a solvate thereof, wherein,
L₁-L₂ is: 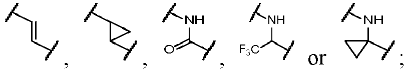
Z₁ is CH or N;
R₁ is CF₃, CN, OCF₃, OCH₂CH₃, 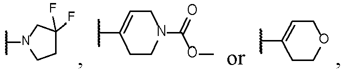 alternatively CF₃;
m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is H, F, Cl, Br, Me, CH₂CH₃, C(O)NH₂ or 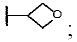
R_{2c} is H or Me;
or X₁, X₂ and their substituents are taken together to form: 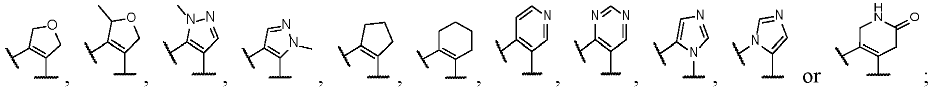
R_{2d} is H;
R₃ₛ₁ is H, Me or cyclopropyl;
R₃ₛ is H;
p=0.

In a more specific embodiment, the present disclosure provides the above compound of formula (IV), (IV-1), (IV-1a), (IV-1b), (IV-2), (IV-2a) or (IV-2b), or a pharmaceutically acceptable salt, an isotopic variant, a tautomer, a stereoisomer, a prodrug, a polymorph, a hydrate or a solvate thereof, wherein,
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
Z₂ is CR₁ or N;
Z₃ is CR₁ or N;
when Z₂ is CR₁, the R₁ and R₄ are connected to form -(CH₂)_{q1}-X-(CH₂)_{q2}-; wherein X is O, S, NH or CH₂, q1=0, 1 or 2, q2=1, 2 or 3;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, CN, =O, ORₐ, NR_{b}R_{c}, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; or two R₁ₛ and the atom(s) to which they are attached are taken together to form C₃₋₁₀ cycloalkyl or 3- to 10-membered heterocyclyl; alternatively R₁ₛ and R₂ₛ are independently selected from H, D, halogen, CN, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (IV), (IV-1), (IV-1a), (IV-1b), (IV-2), (IV-2a) or (IV-2b), or a pharmaceutically acceptable salt, an isotopic variant, a tautomer, a stereoisomer, a prodrug, a polymorph, a hydrate or a solvate thereof, wherein,
R₁ is selected from H, D, halogen, CN, NO₂, ORₐ, SRₐ, NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, C₃₋₇ cycloalkyl, 3- to 7-membered heterocyclyl, C₆₋₁₀ aryl and 5- to 10-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₆ cycloalkyl, 5- to 6-membered heterocyclyl, phenyl or 5- to 6-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl and C₁₋₆ haloalkyl;
Z₁ is CH;
Z₂ is CR₁, and the R₁ and R₄ are connected to form -(CH₂)_{q1}-X-(CH₂)_{q2}-; wherein X is O, S, NH or CH₂, q1=0 or 1, q2=1 or 2;
Z₃ is CR₁ or N;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the above groups is optionally deuterated, up to fully deuterated.

In a more specific embodiment, the present disclosure provides the above compound of formula (IV), (IV-1), (IV-1a), (IV-1b), (IV-2), (IV-2a) or (IV-2b), or a pharmaceutically acceptable salt, an isotopic variant, a tautomer, a stereoisomer, a prodrug, a polymorph, a hydrate or a solvate thereof, wherein,
R₁ is selected from H, D, halogen, CN, NO₂, ORₐ, SRₐ, NR_{b}R_{c}, SF₅, C(O)Rₐ, S(O)Rₐ, S(O)₂Rₐ, C₁₋₆ alkyl, C₁₋₆ haloalkyl, C₂₋₆ alkenyl, C₂₋₆ alkynyl, C₄₋₆ cycloalkyl, 5- to 6-membered heterocyclyl, phenyl and 5- to 6-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₆ cycloalkyl, 5- to 6-membered heterocyclyl, phenyl or 5- to 6-membered heteroaryl, which is optionally substituted with 1, 2 or 3 R₂ₛ;
R_{2d} is selected from H, D, halogen, CN, NO₂, C₁₋₆ alkyl and C₁₋₆ haloalkyl;
Z₁ is CH;
Z₂ is CR₁, and the R₁ and R₄ are connected to form -(CH₂)_{q1}-X-(CH₂)_{q2}-; wherein X is O, S or NH, q1=0 or 1, q2=1 or 2;
Z₃ is CR₁ or N;
L is a chemical bond or C₁₋₆ alkylene;
R₁ₛ and R₂ₛ are independently selected from H, D, halogen, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from H, D, -L-ORₐ, -L-NR_{b}R_{c}, C₁₋₆ alkyl, C₁₋₆ haloalkyl, C₃₋₆ cycloalkyl, 5- to 6-membered heterocyclyl, phenyl and 5- to 6-membered heteroaryl, which is optionally substituted with 1, 2 or 3 R₃ₛ;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

In a most specific embodiment, the present disclosure provides the above compound of formula (X), or a pharmaceutically acceptable salt, an isotopic variant, a tautomer, a stereoisomer, a prodrug, a polymorph, a hydrate or a solvate thereof, wherein, the compound is selected from:

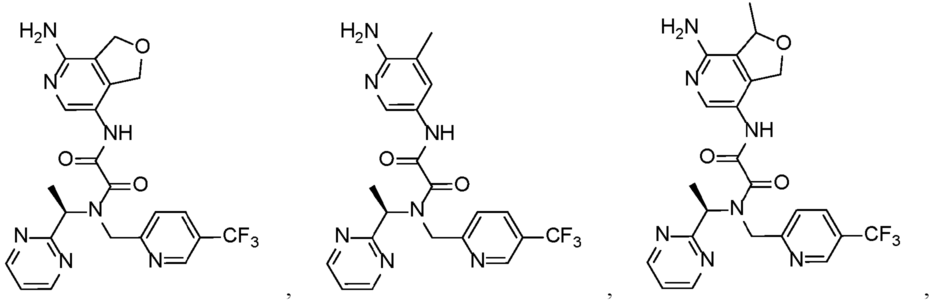

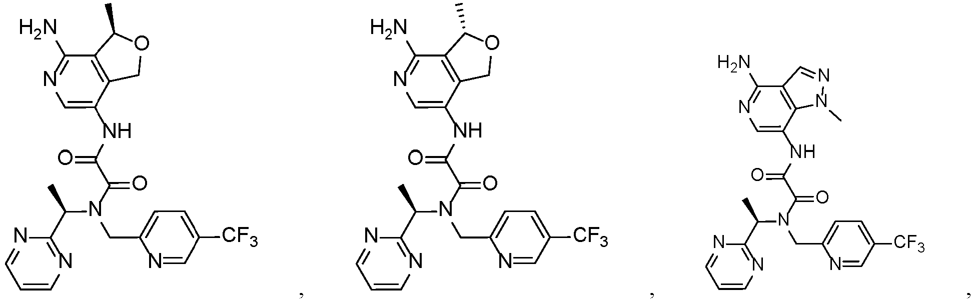

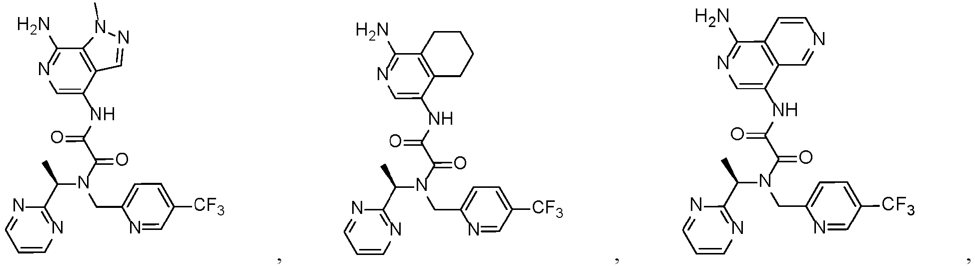

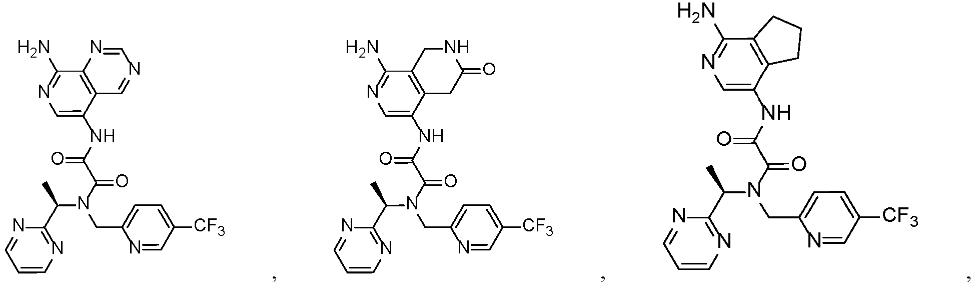

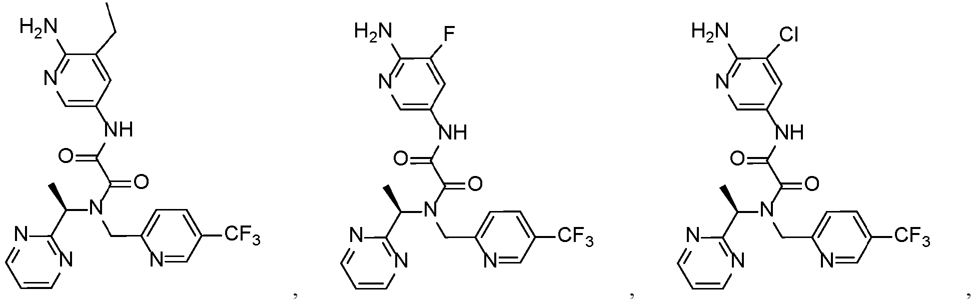

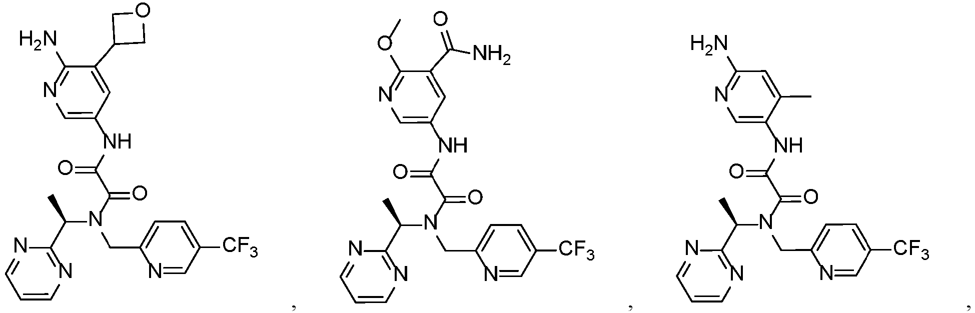

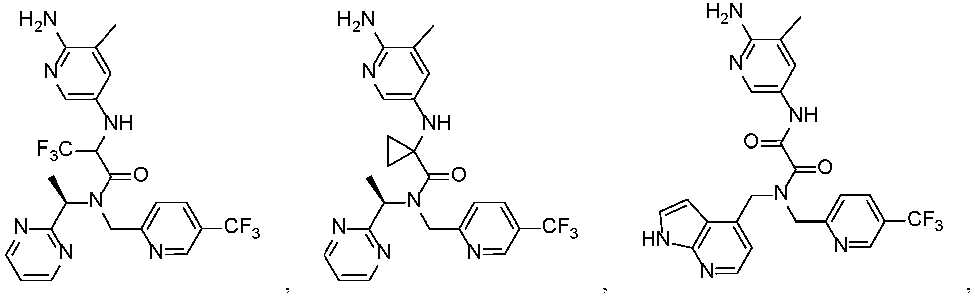

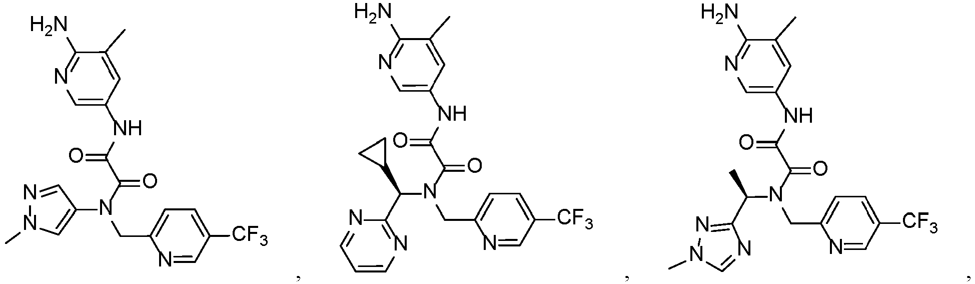

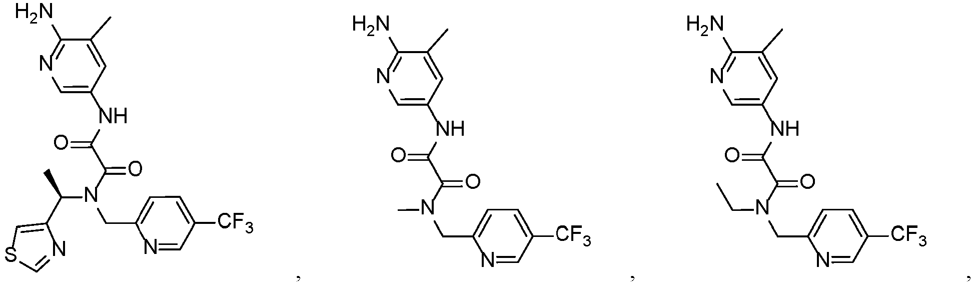

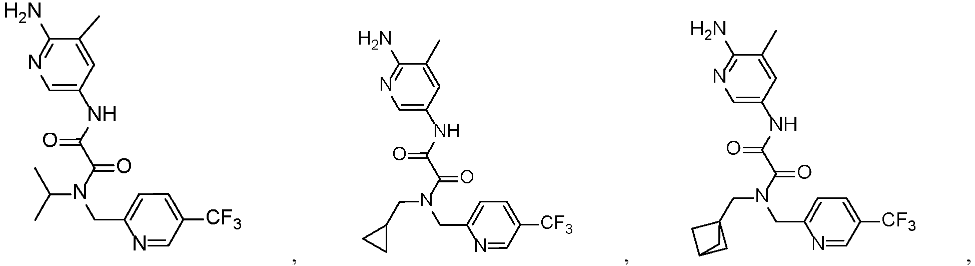

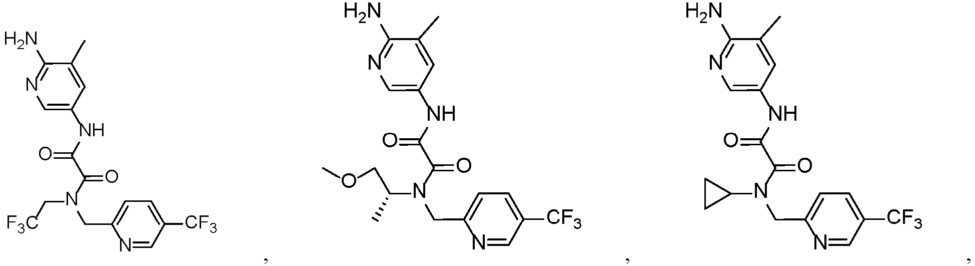

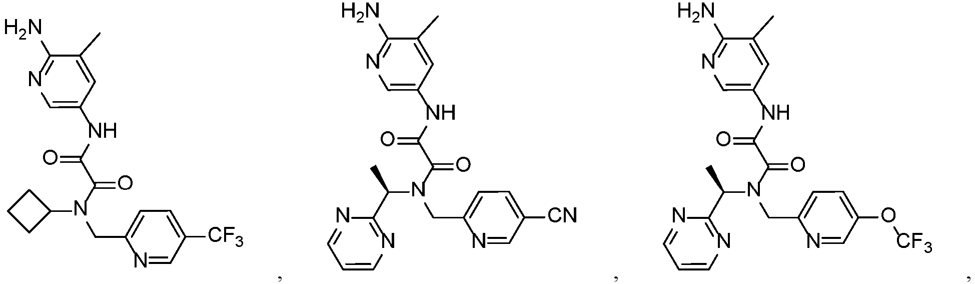

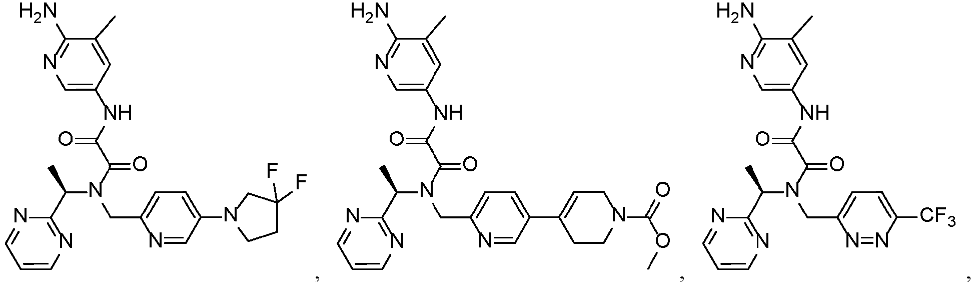

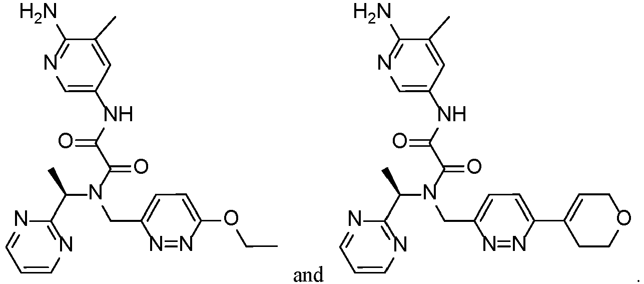

In a most specific embodiment, the present disclosure provides the above compound of formula (X), or a pharmaceutically acceptable salt, an isotopic variant, a tautomer, a stereoisomer, a prodrug, a polymorph, a hydrate or a solvate thereof, wherein, the compound is selected from:

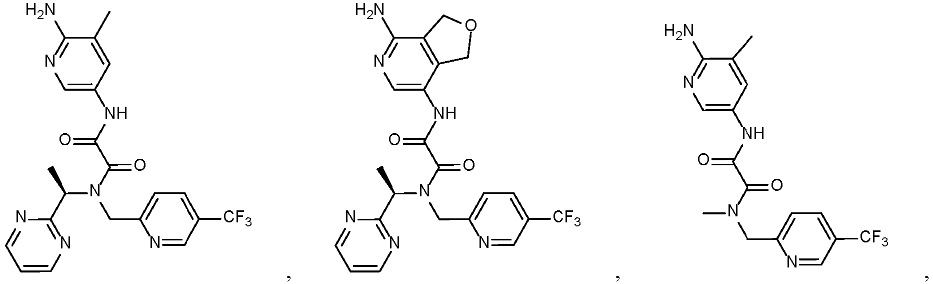

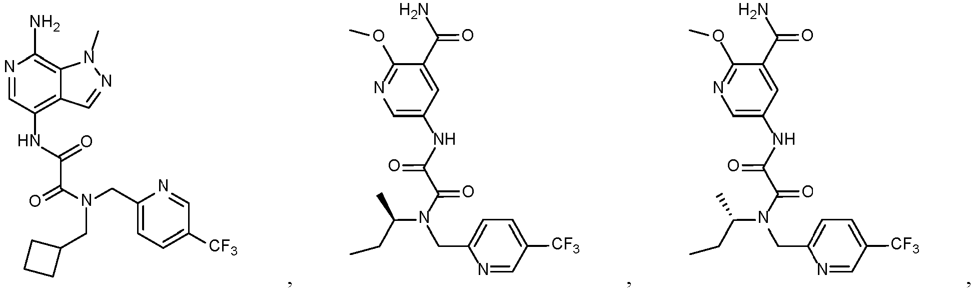

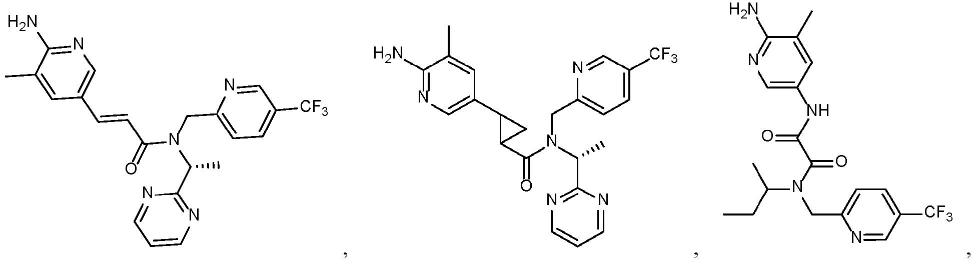

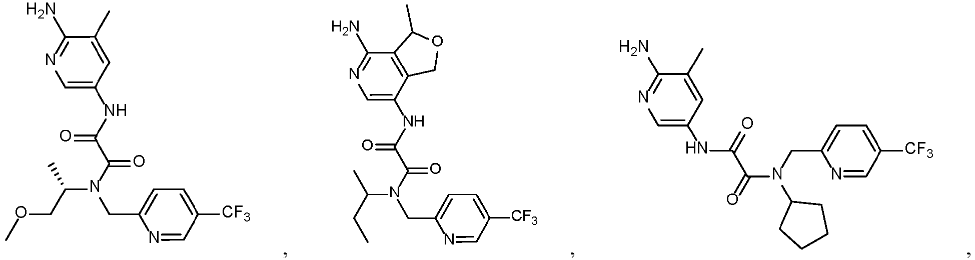

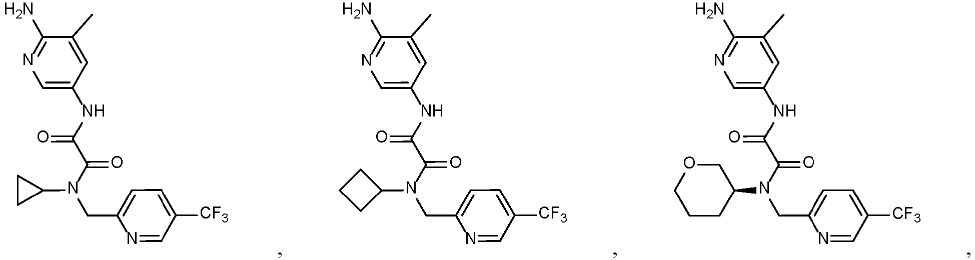

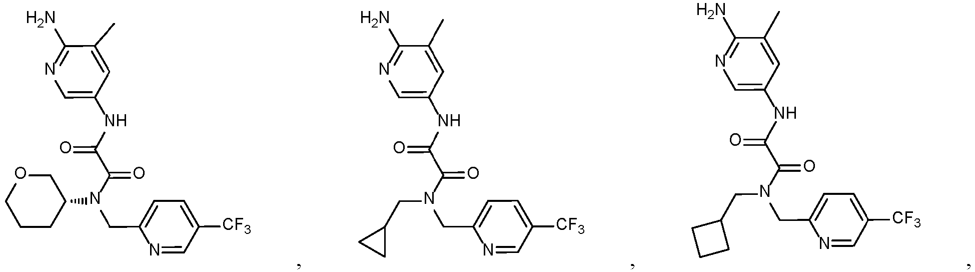

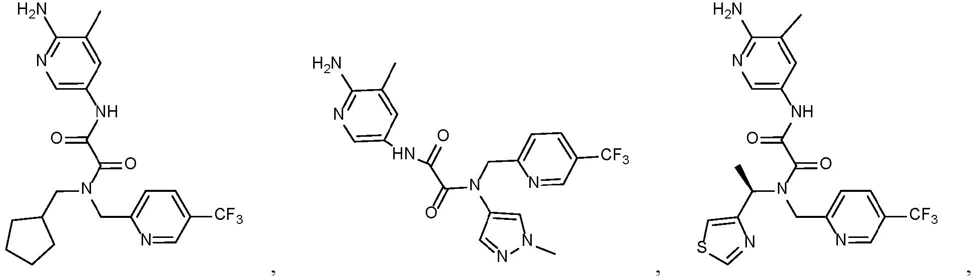

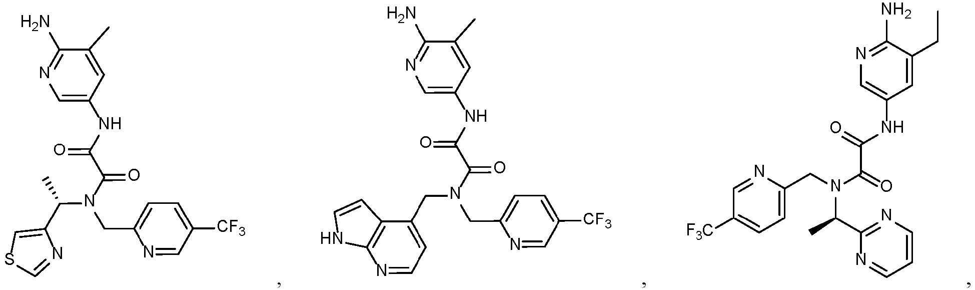

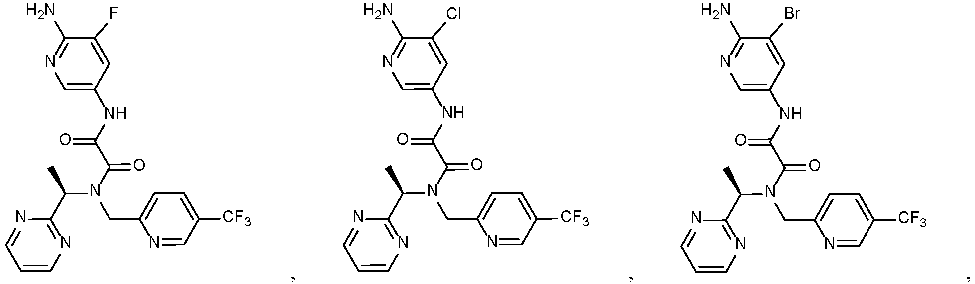

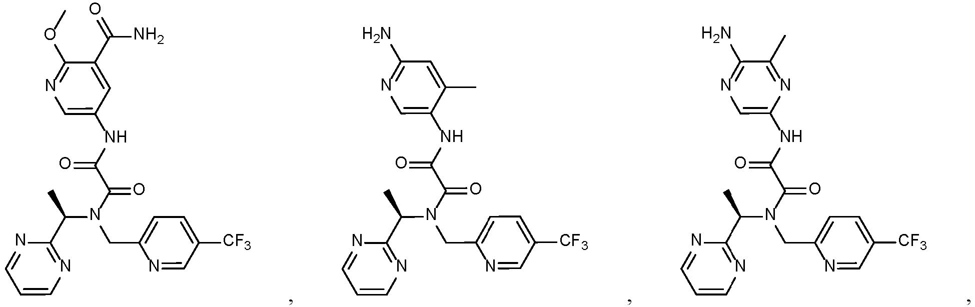

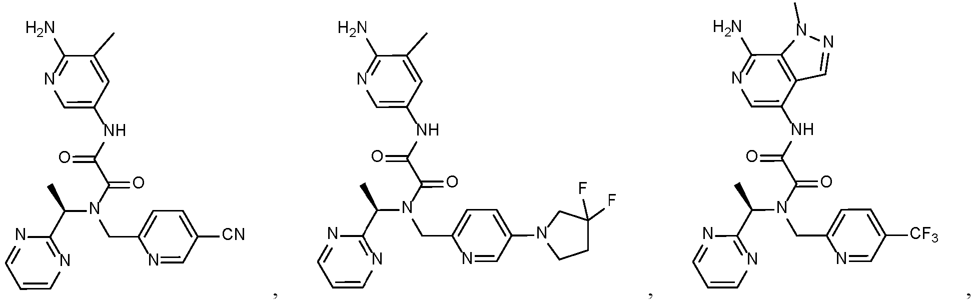

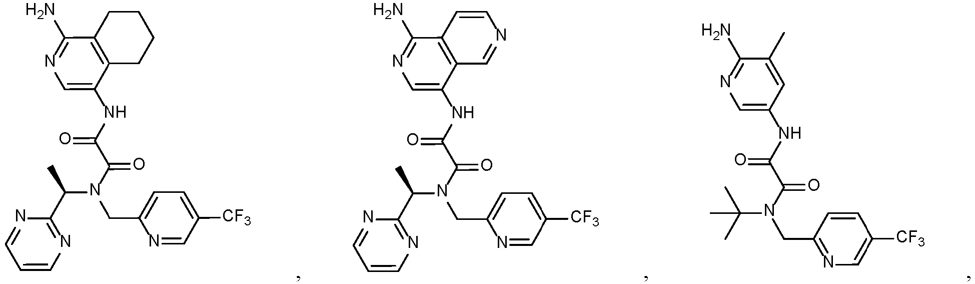

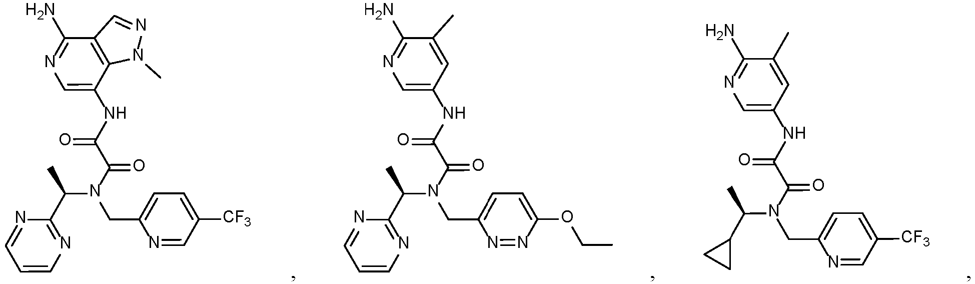

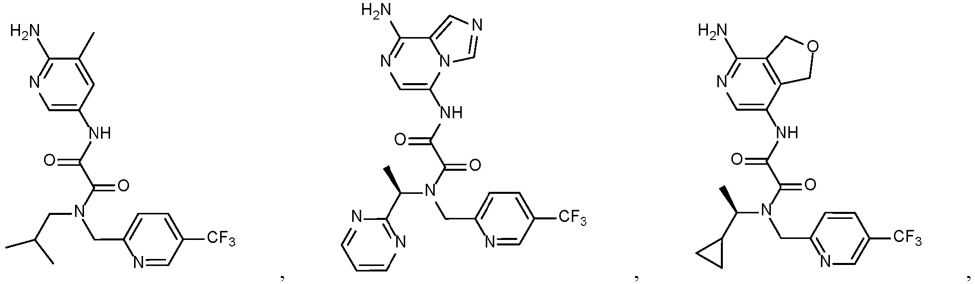

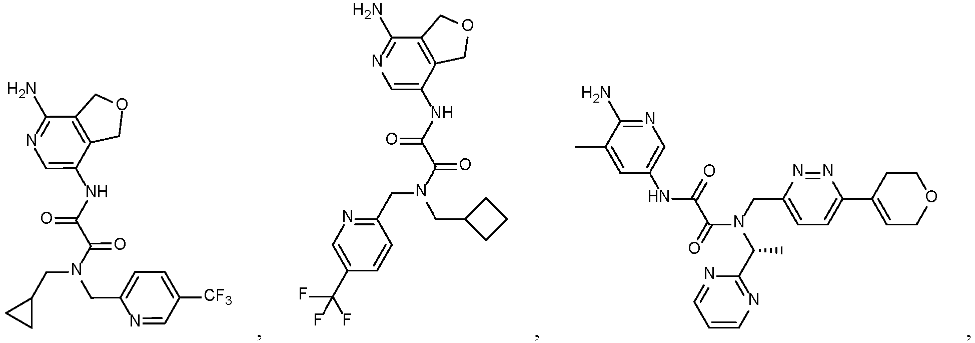

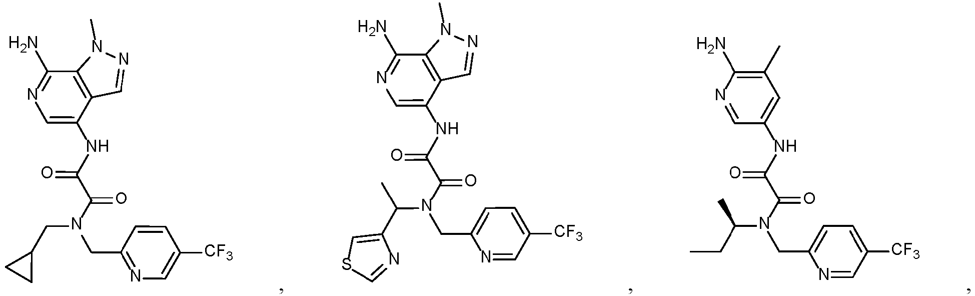

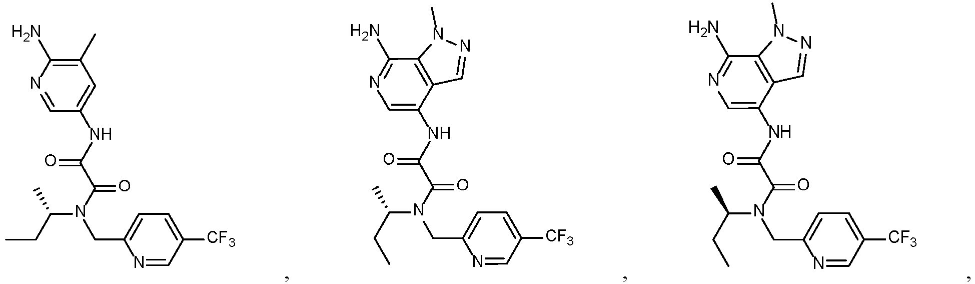

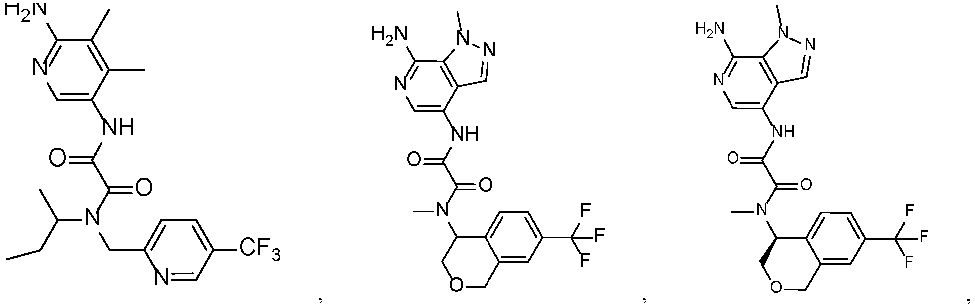

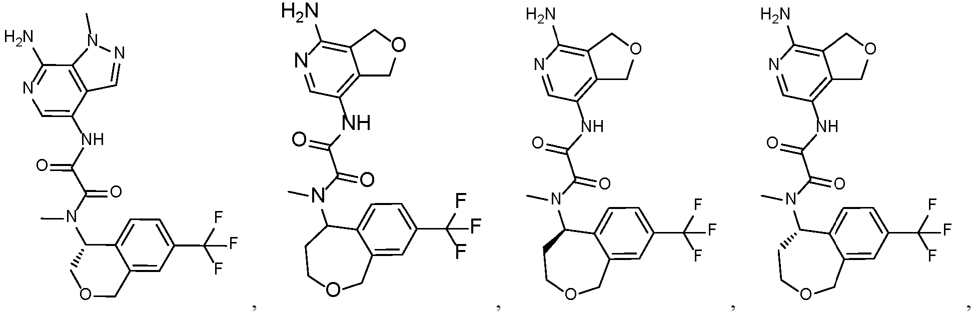

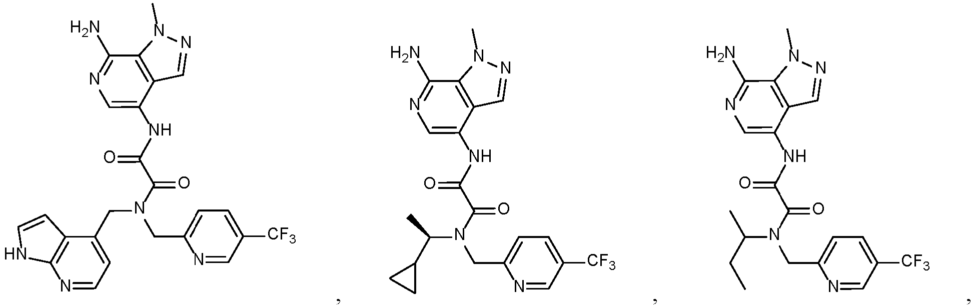

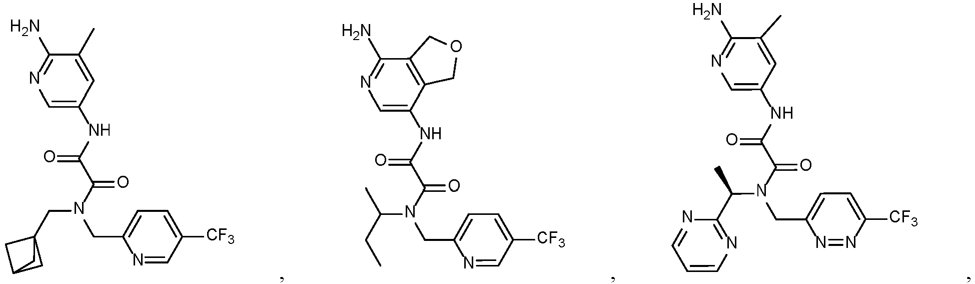

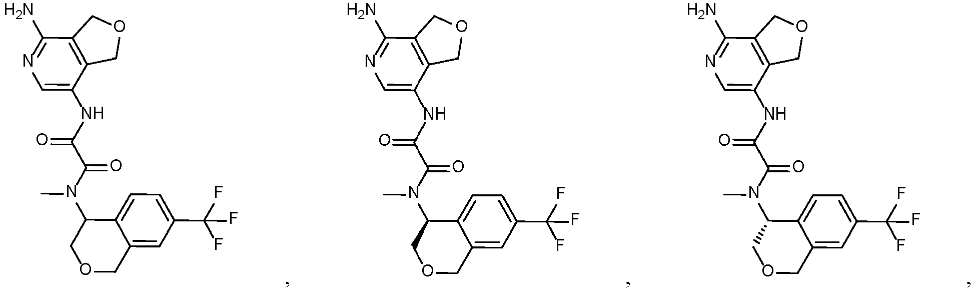

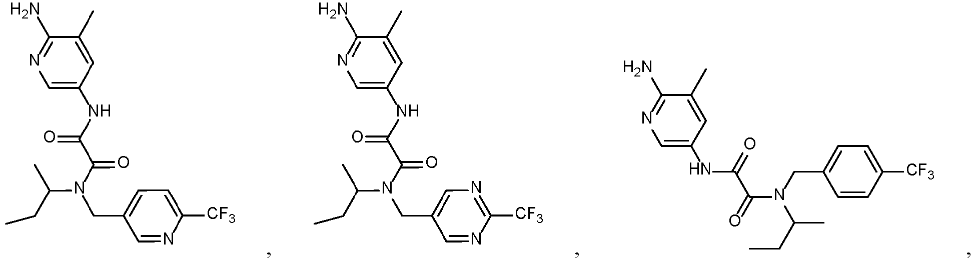

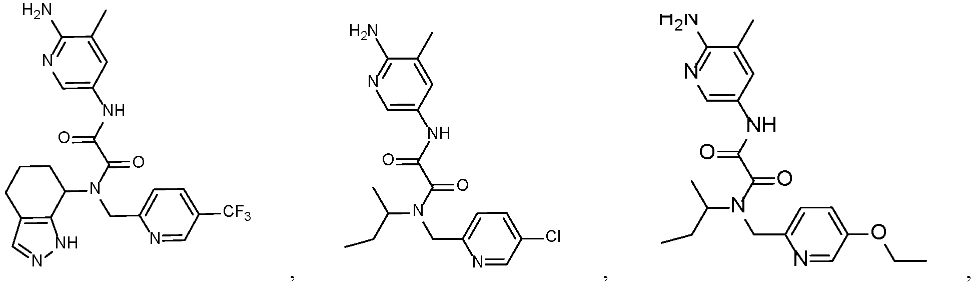

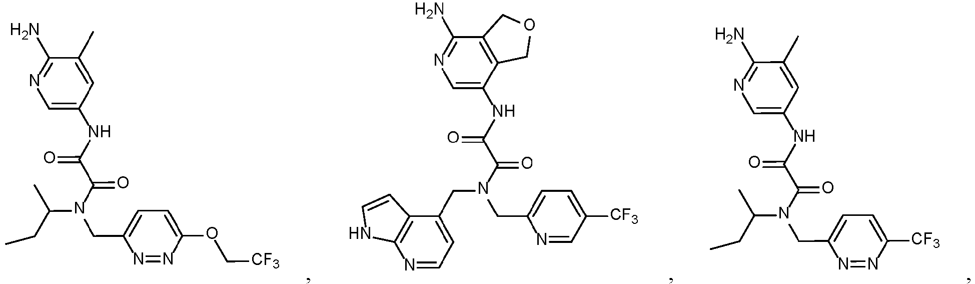

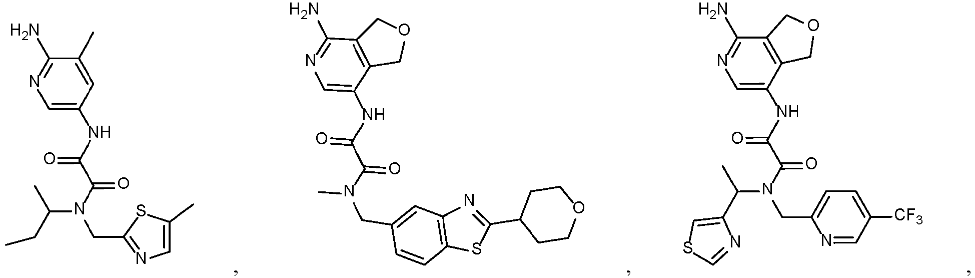

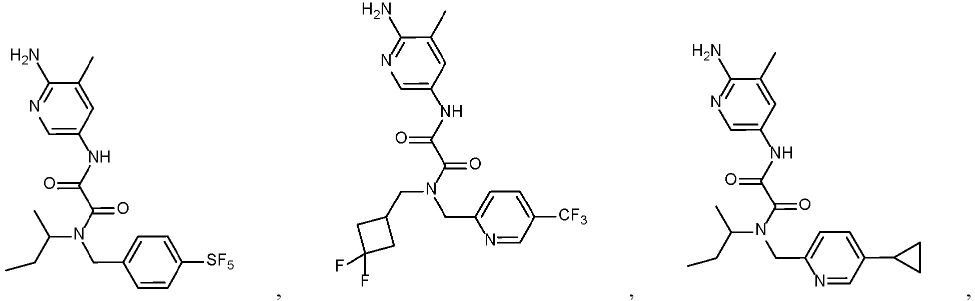

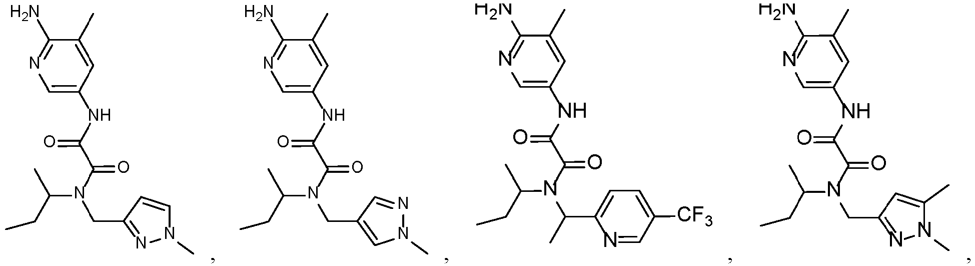

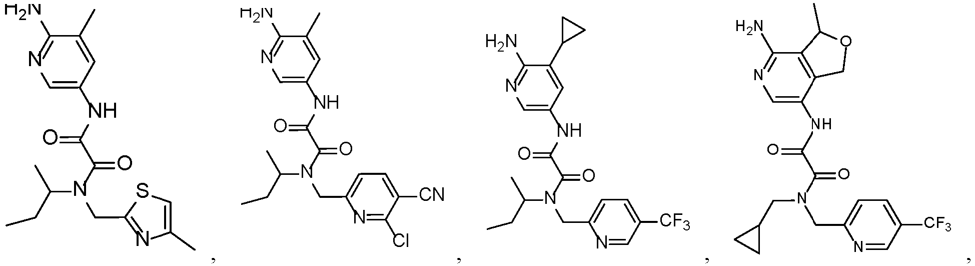

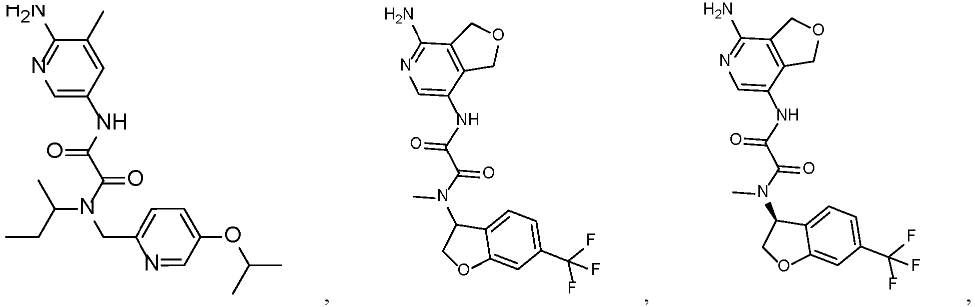

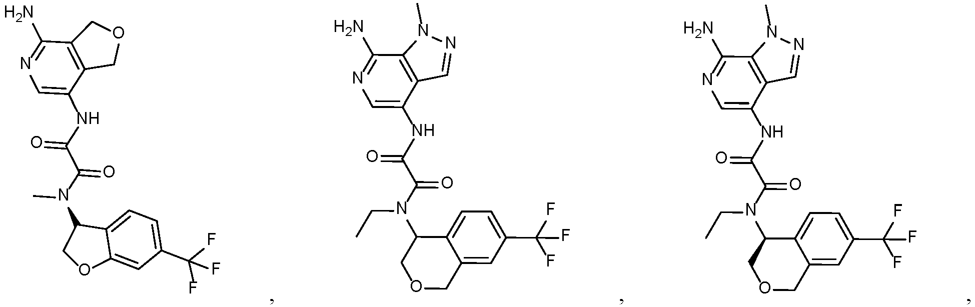

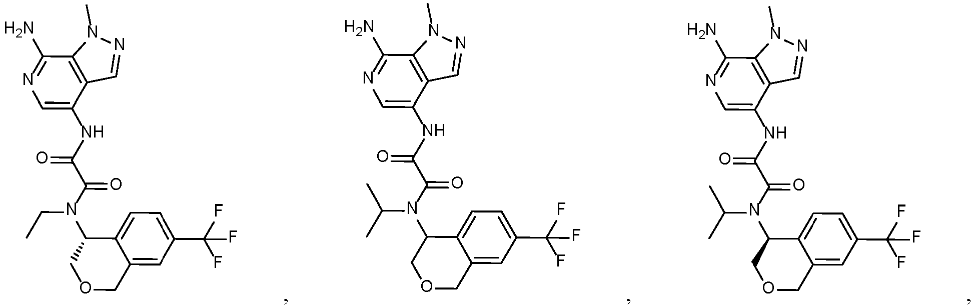

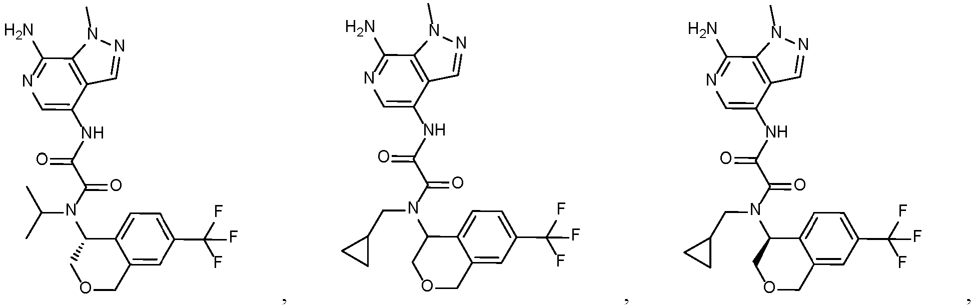

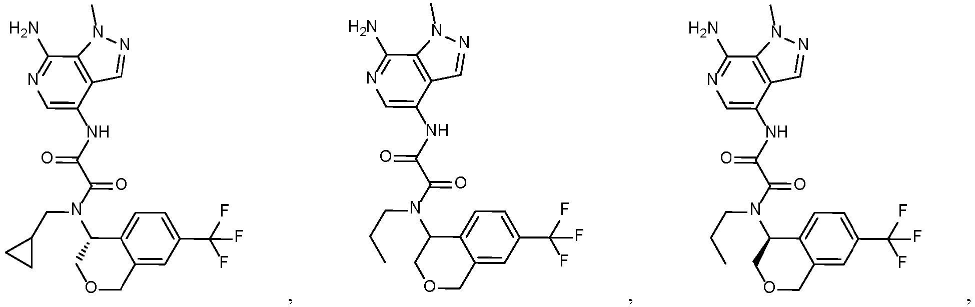

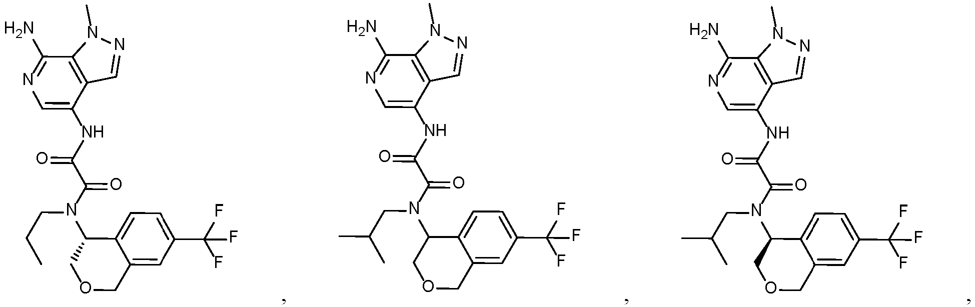

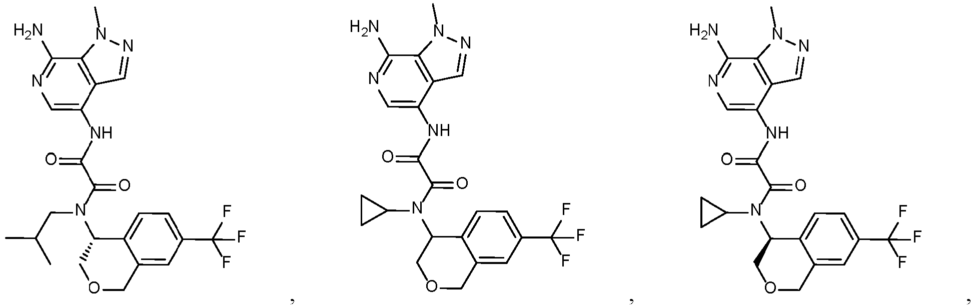

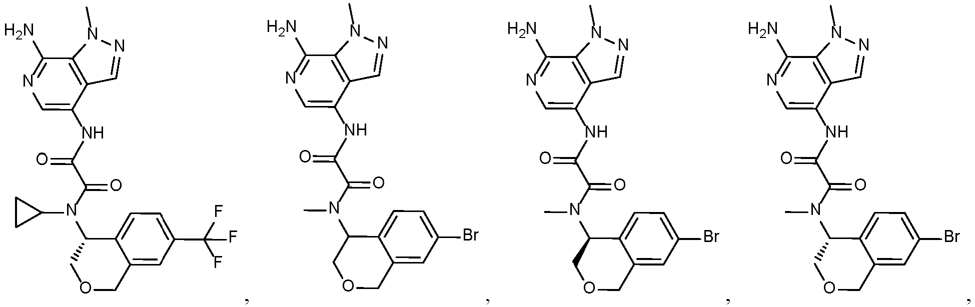

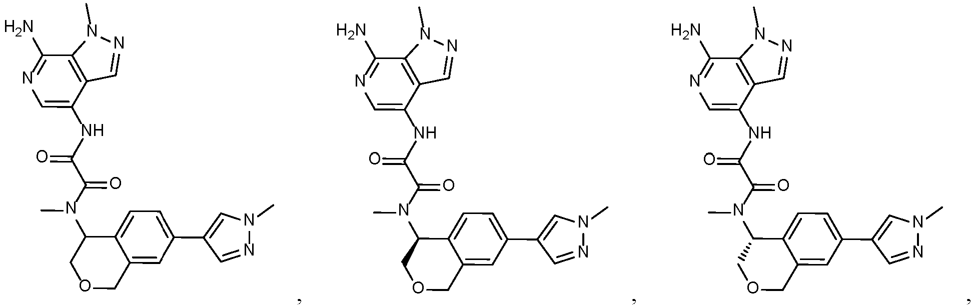

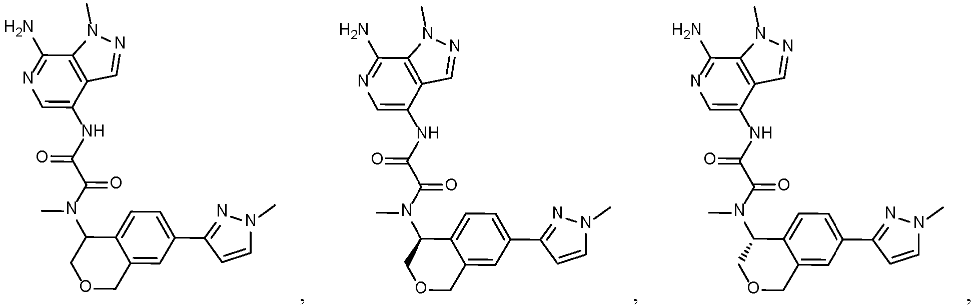

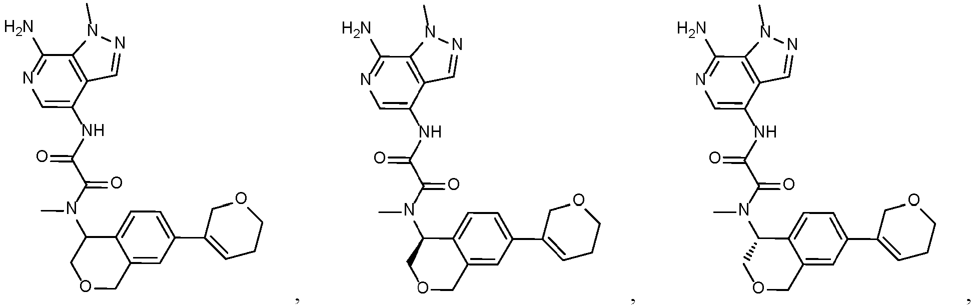

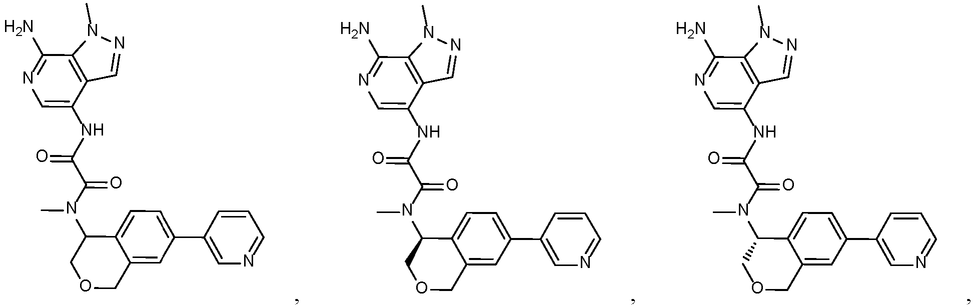

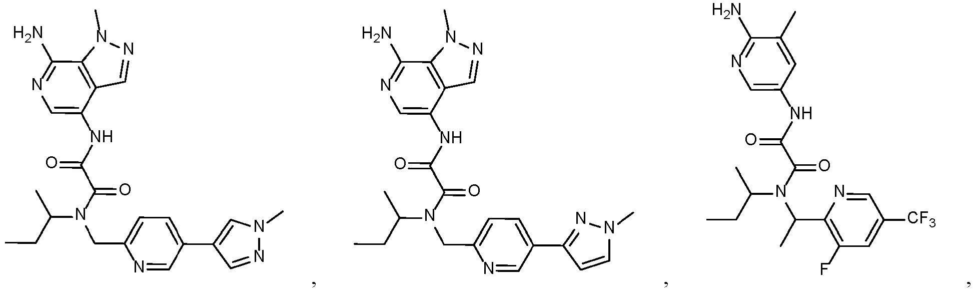

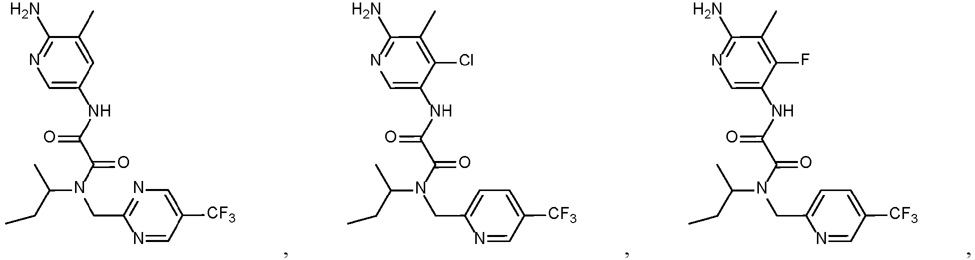

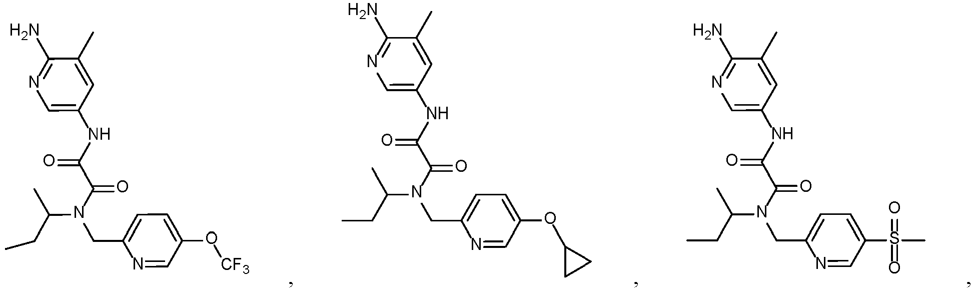

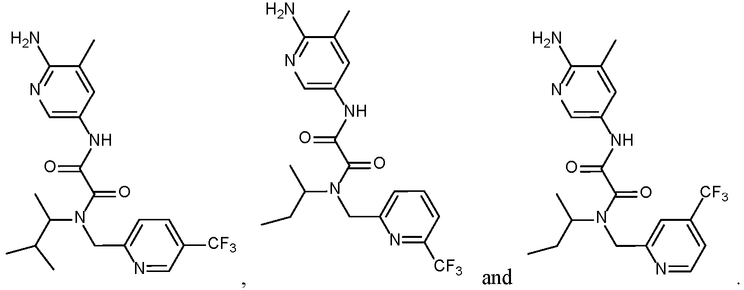

In a most specific embodiment, the present disclosure provides the above compound of formula (X), or a pharmaceutically acceptable salt, an isotopic variant, a tautomer, a stereoisomer, a prodrug, a polymorph, a hydrate or a solvate thereof, wherein, the compound is selected from: 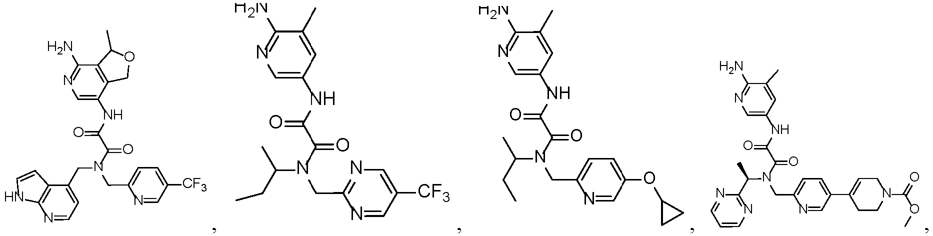 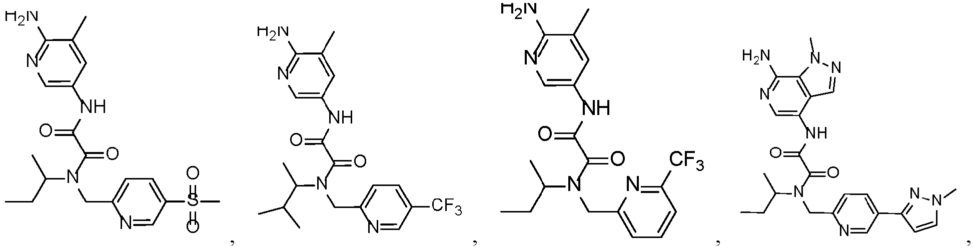 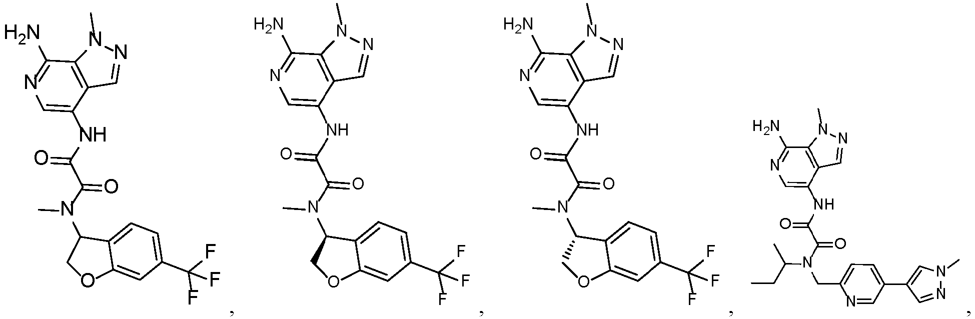 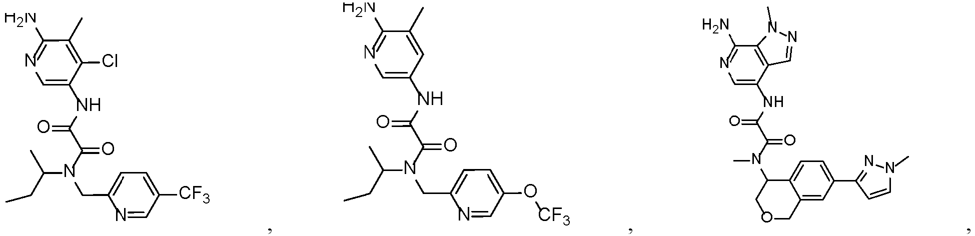 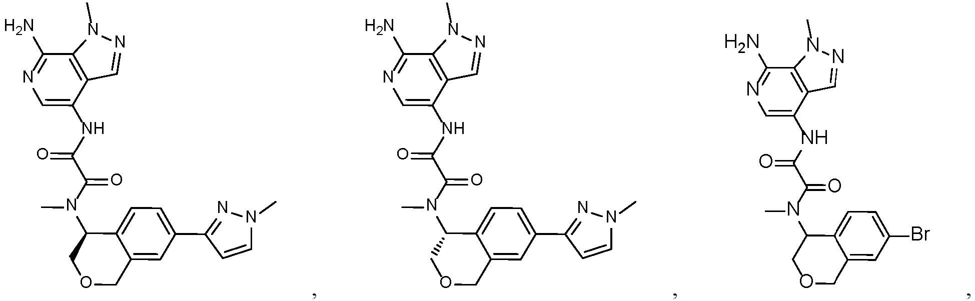 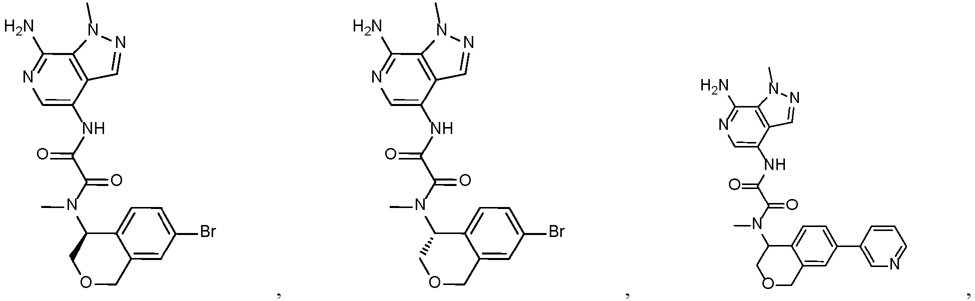 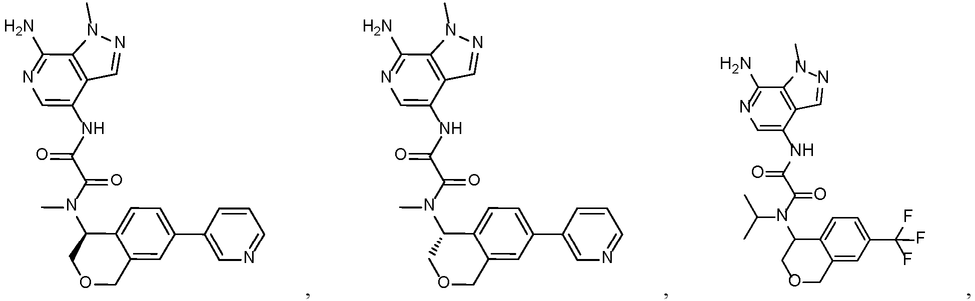 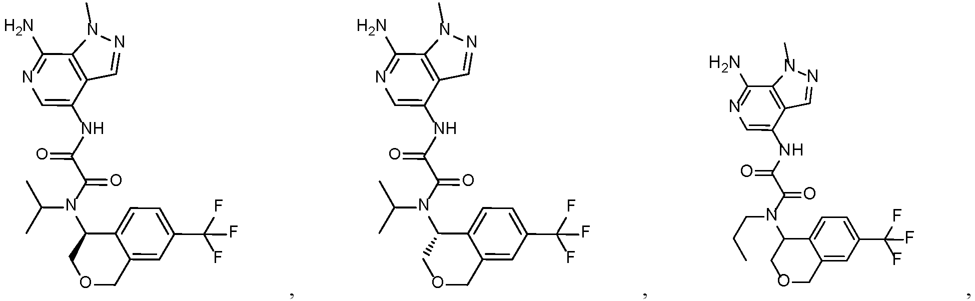 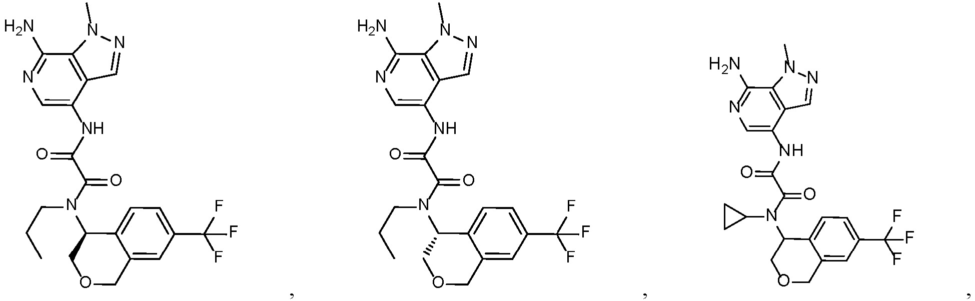 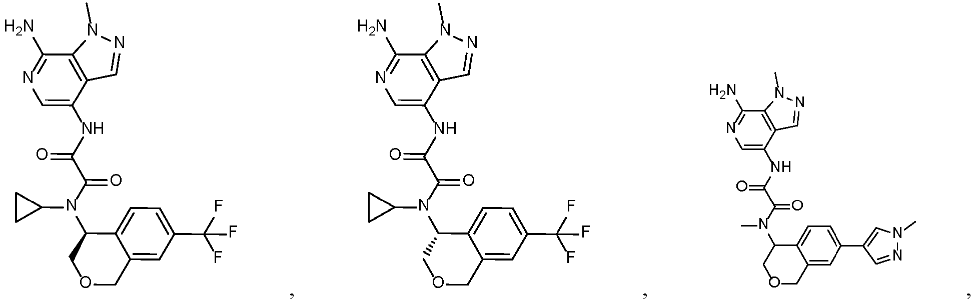 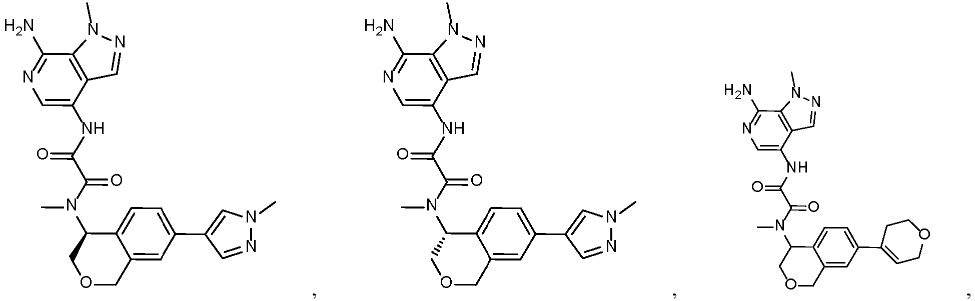 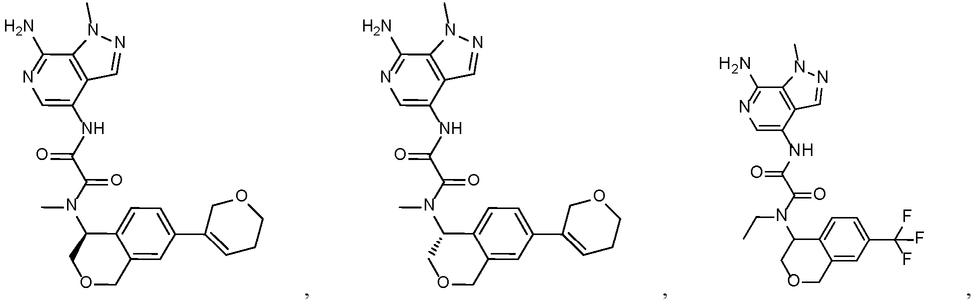 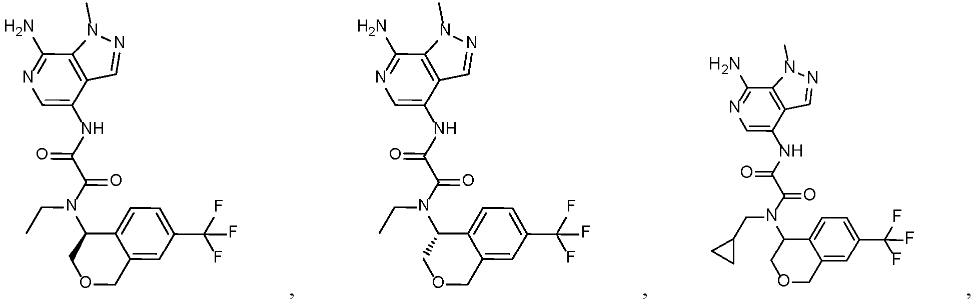 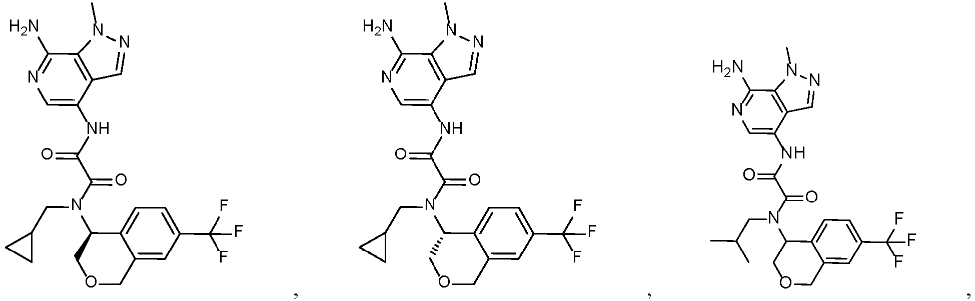 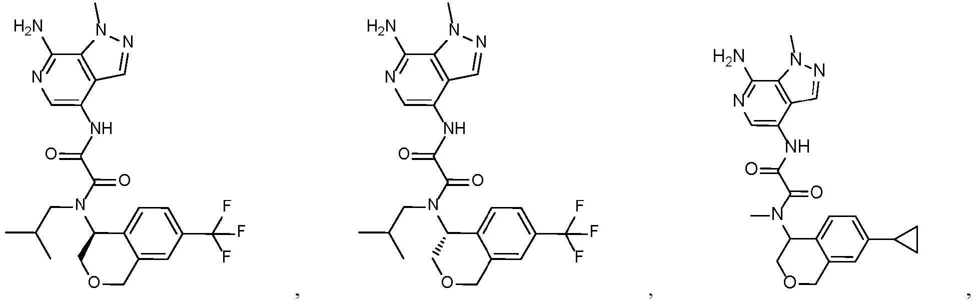 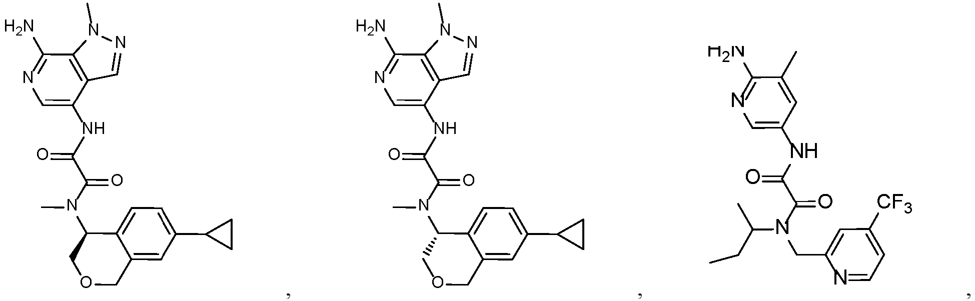 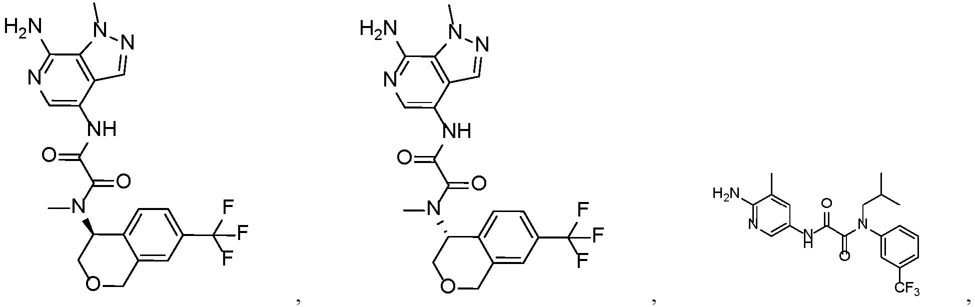 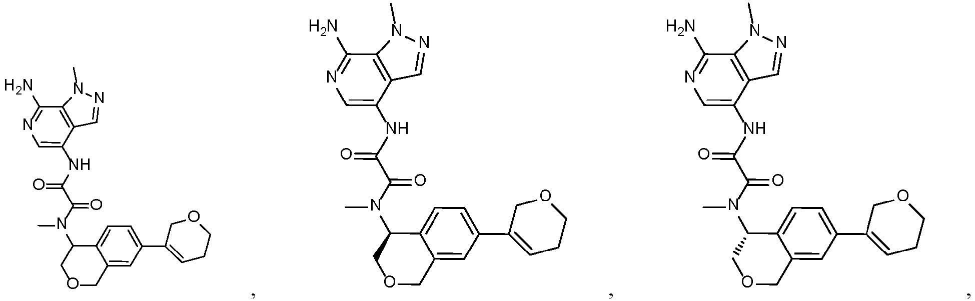 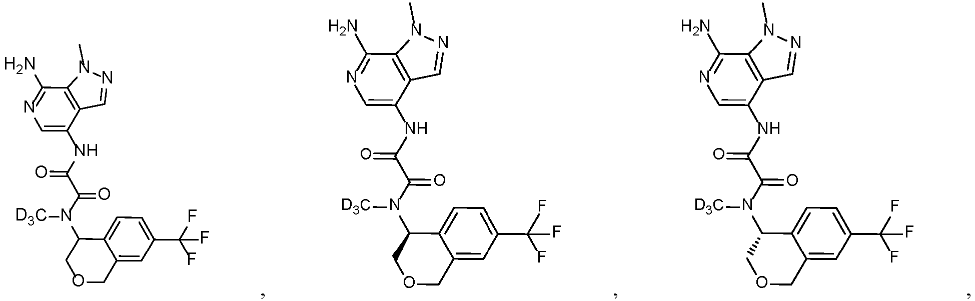 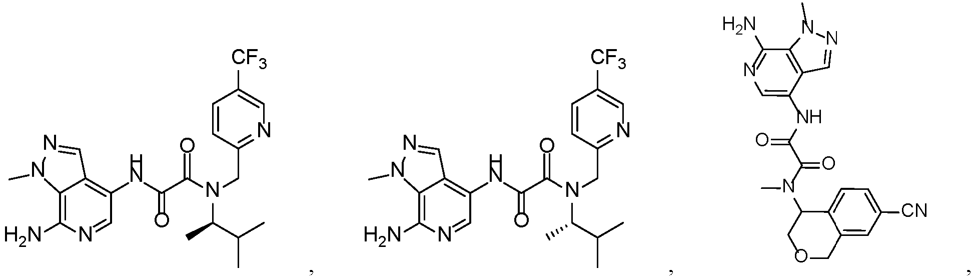 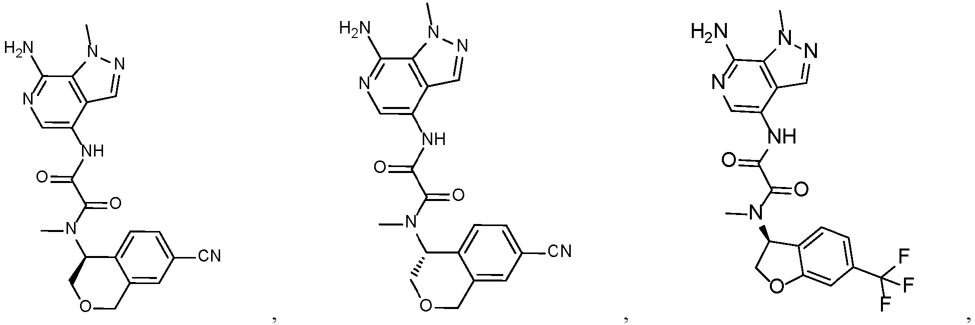 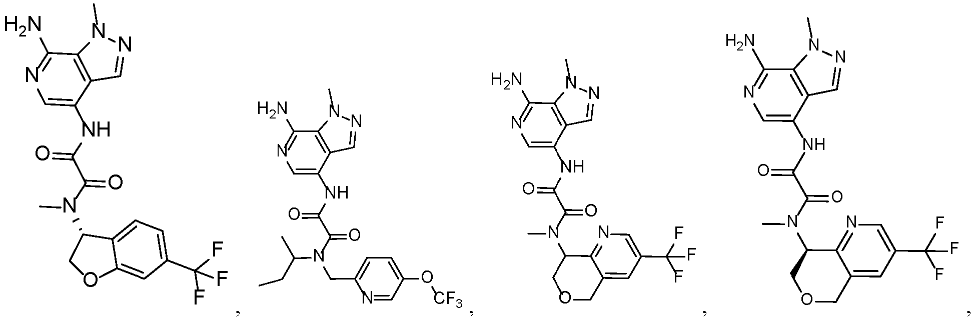 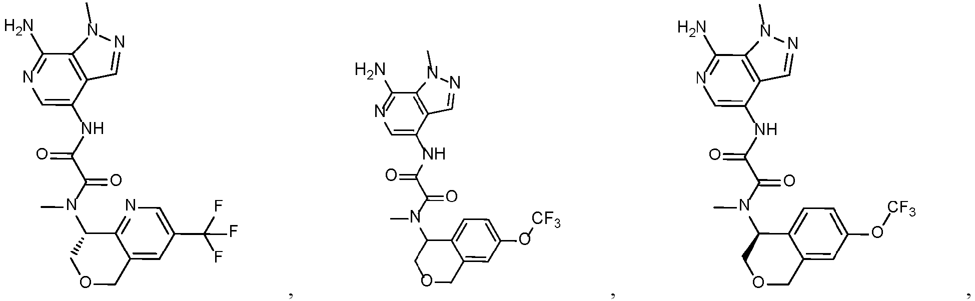 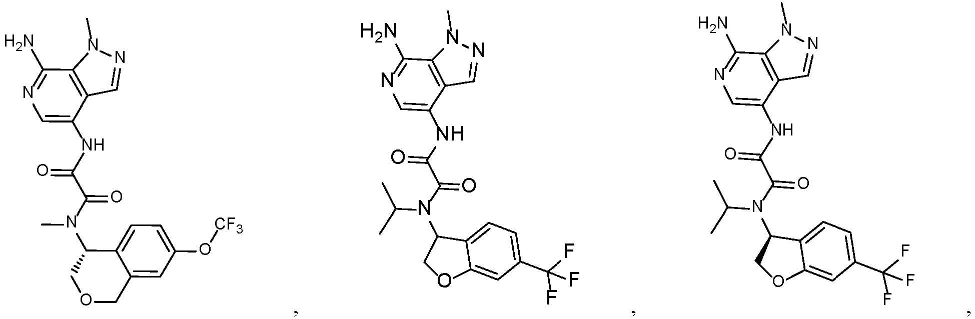 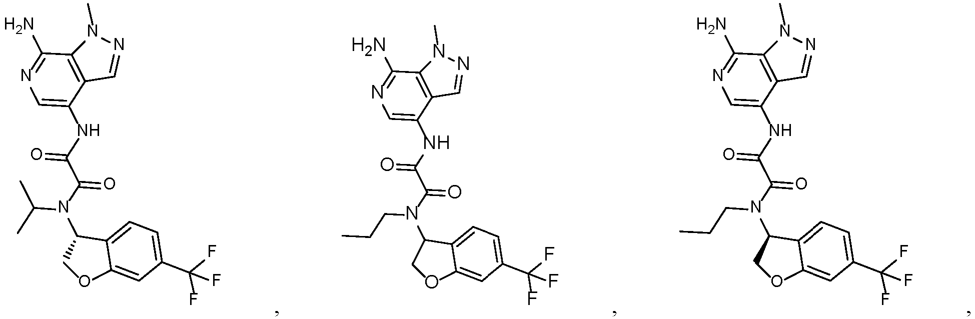 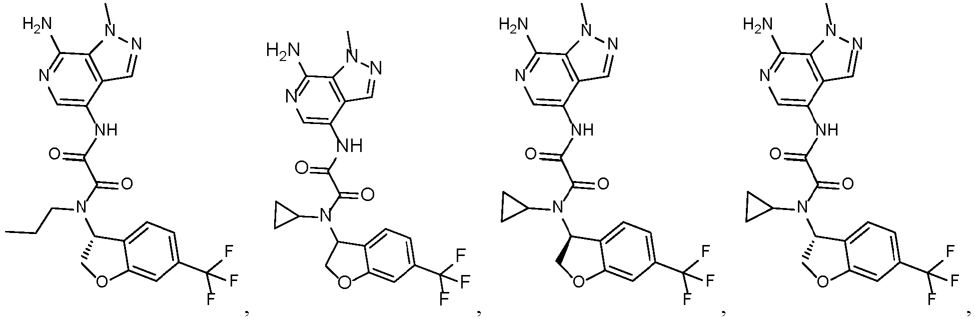 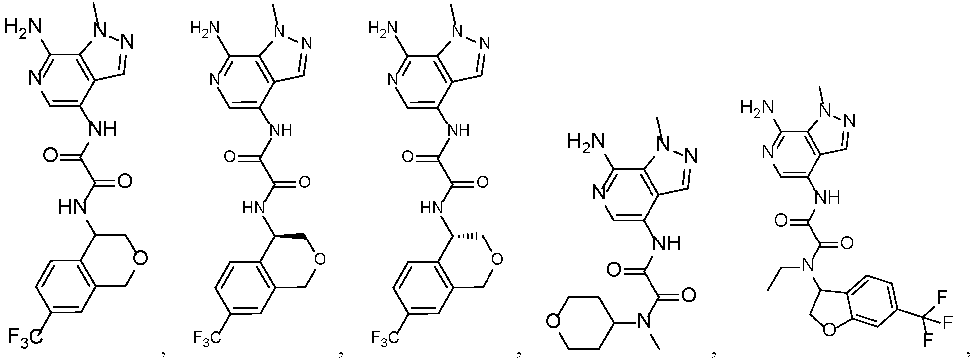 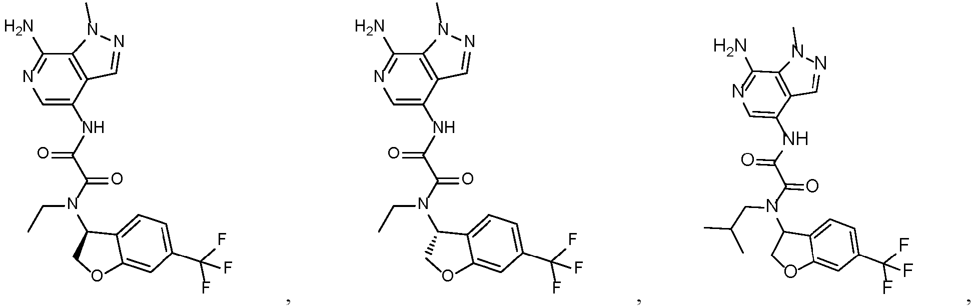 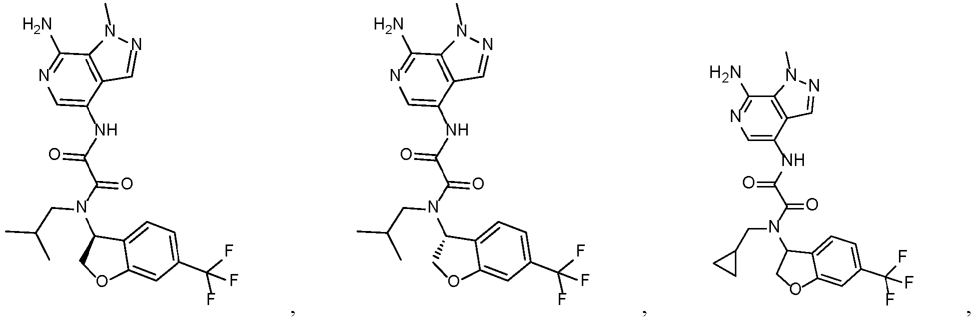 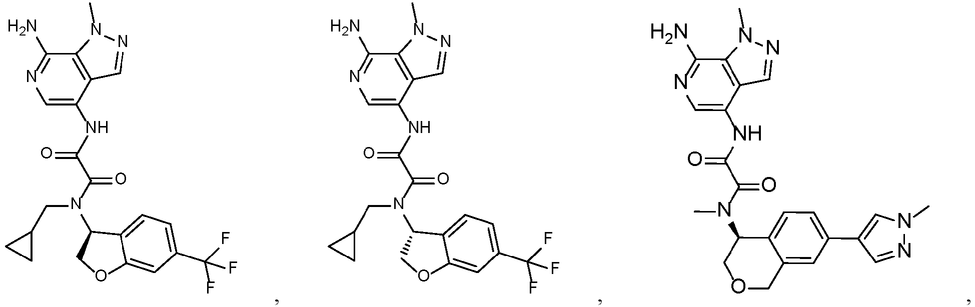 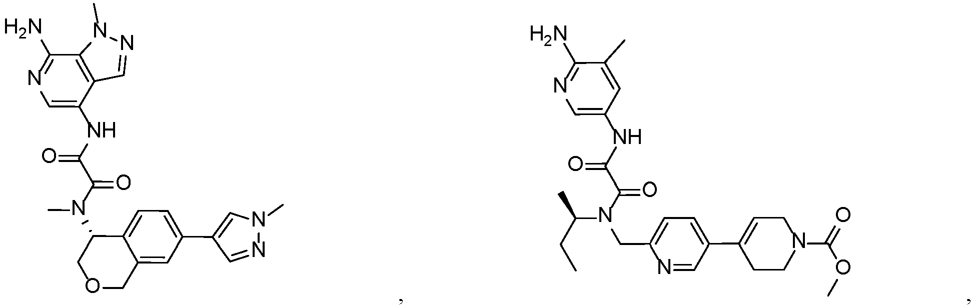 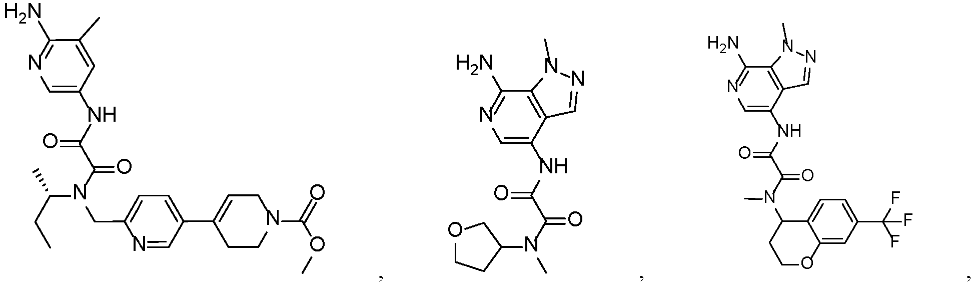 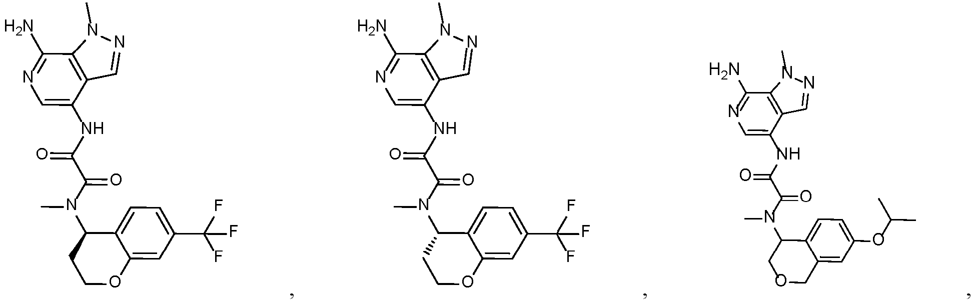 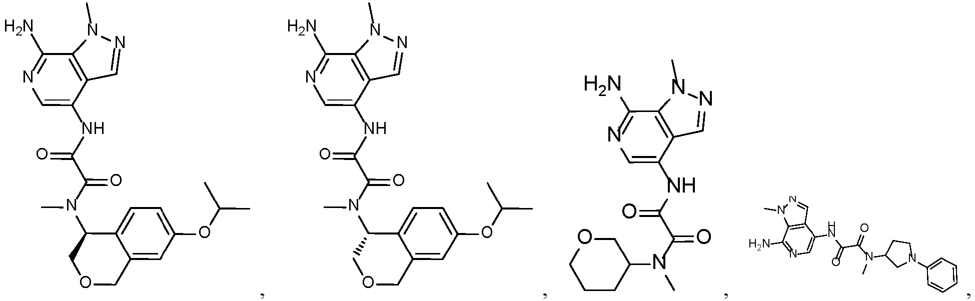 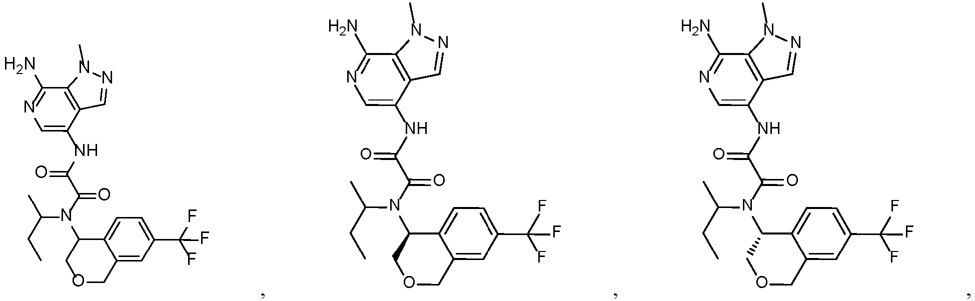 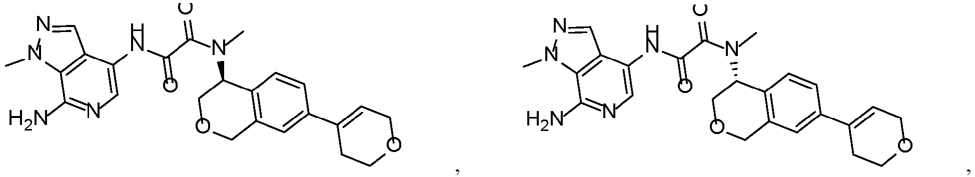 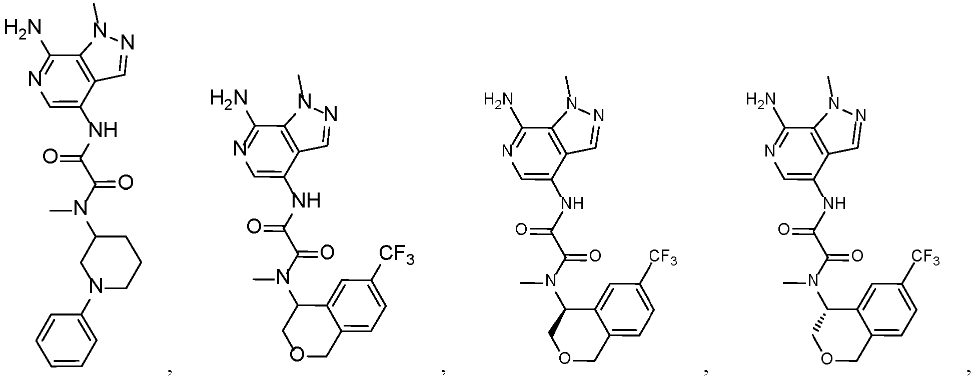 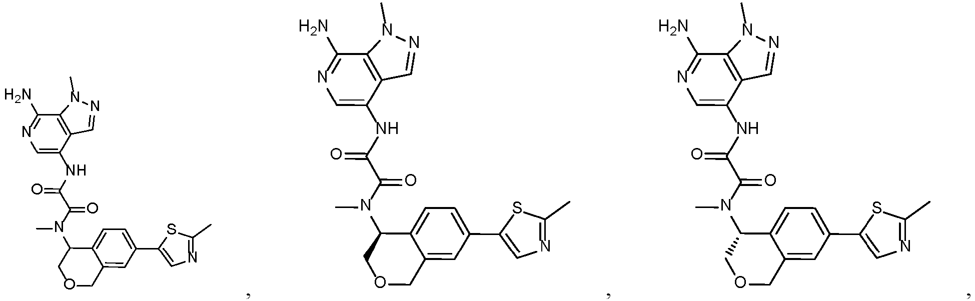 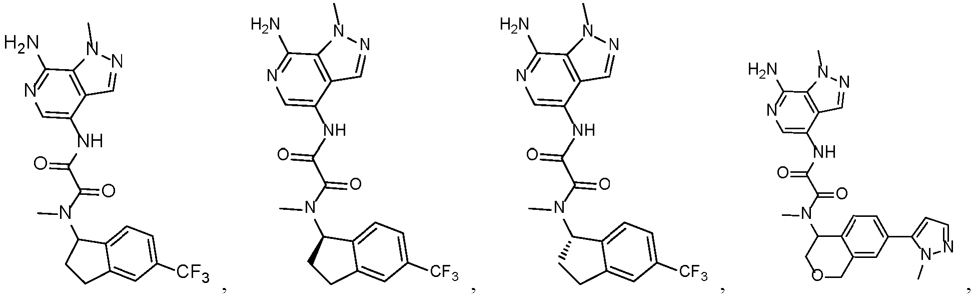 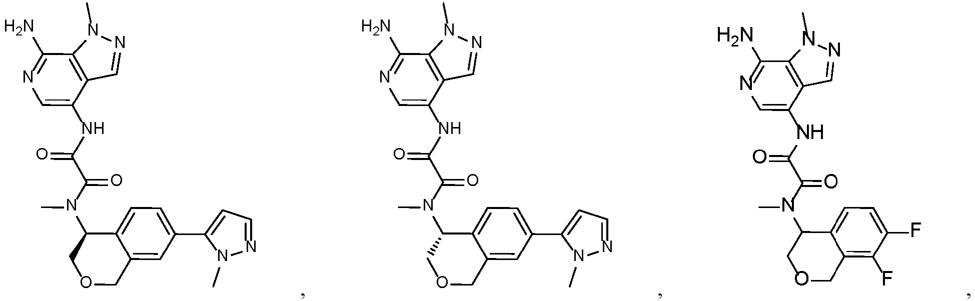 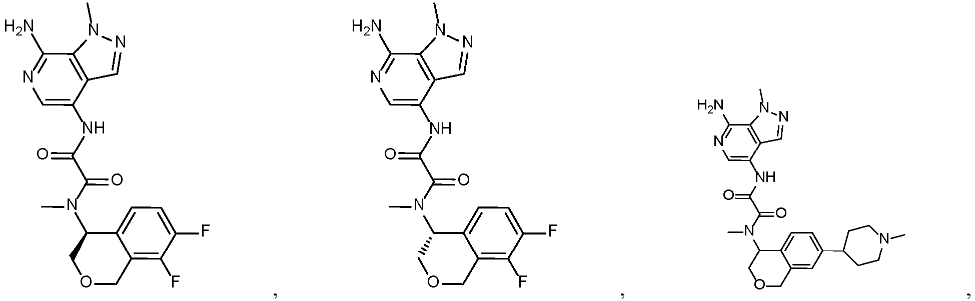 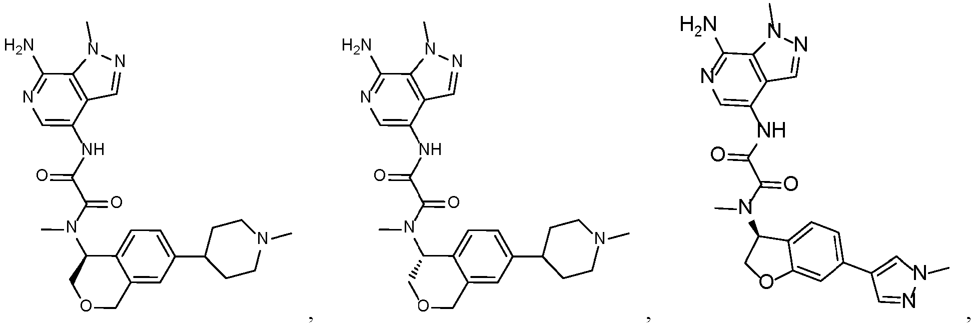 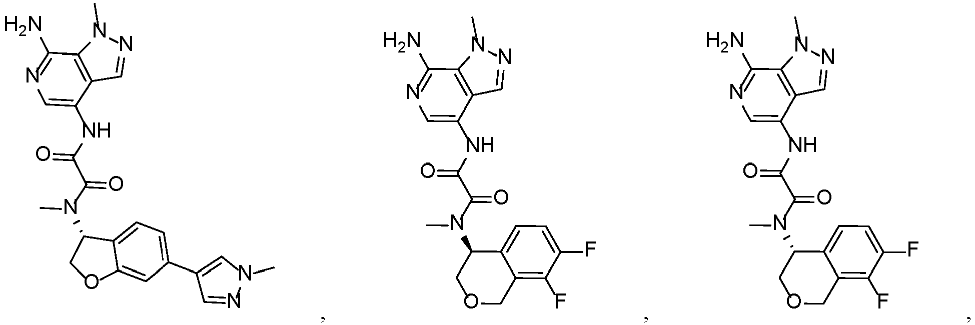 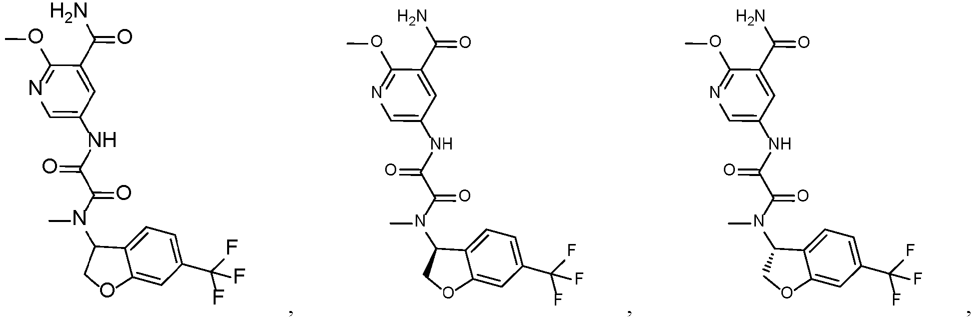 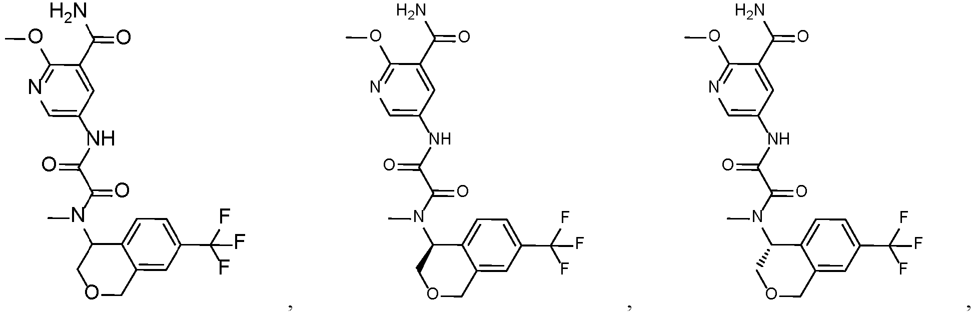 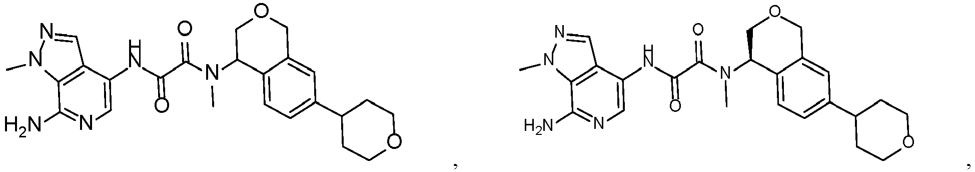 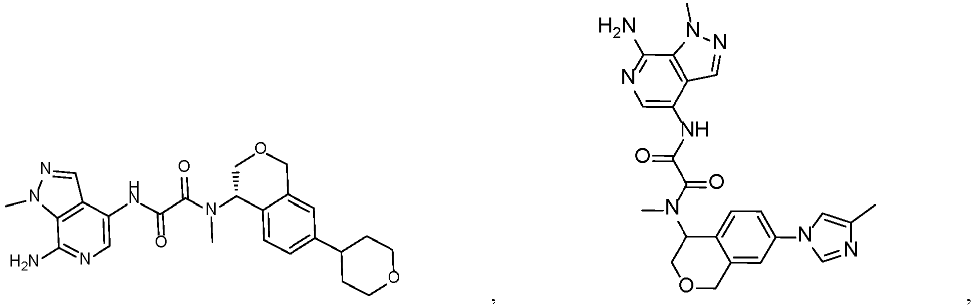 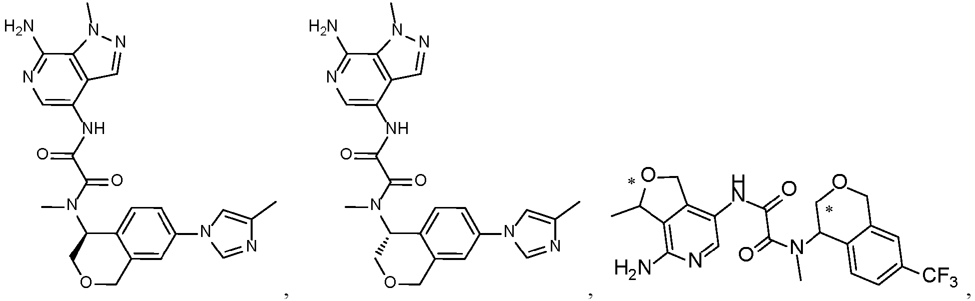 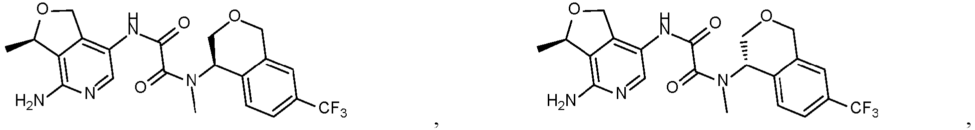 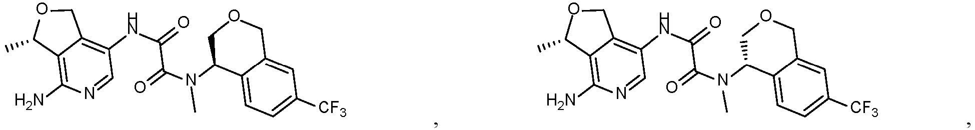 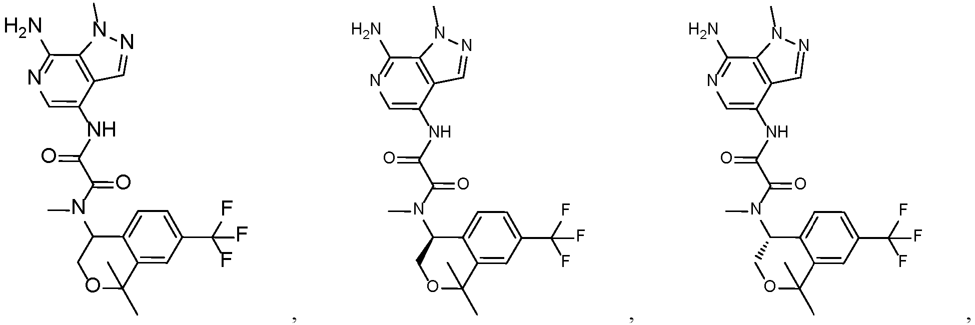 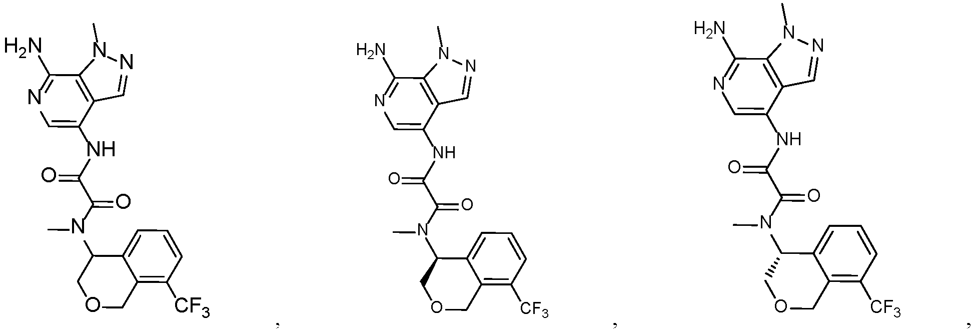 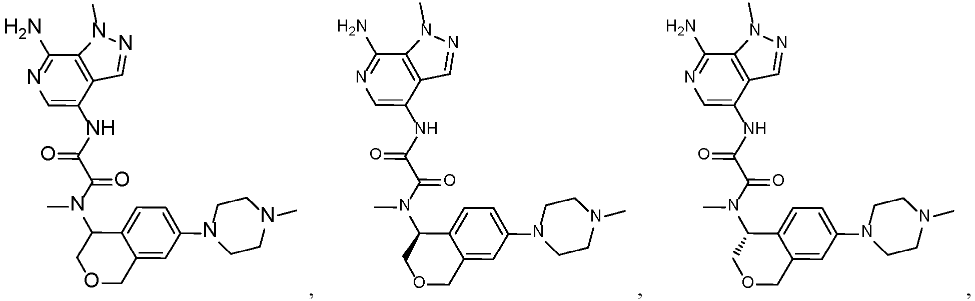 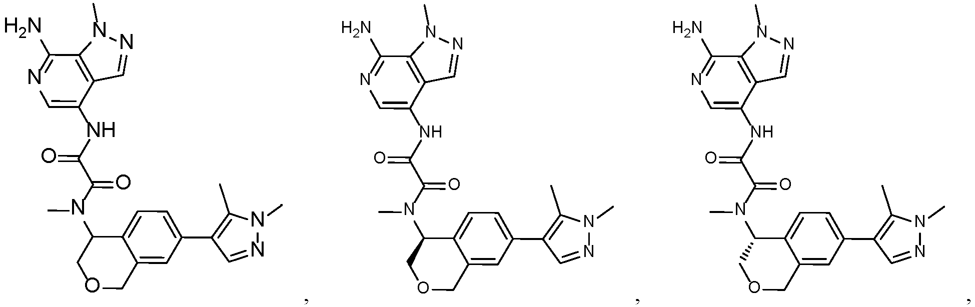 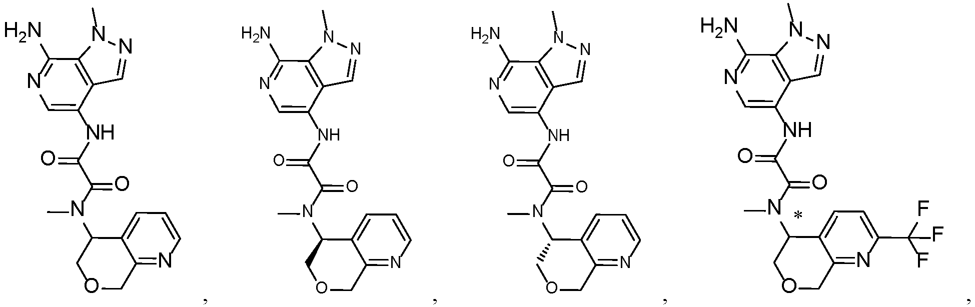 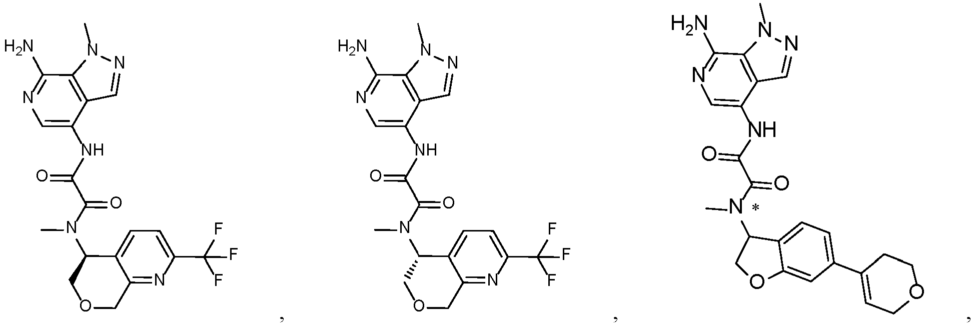 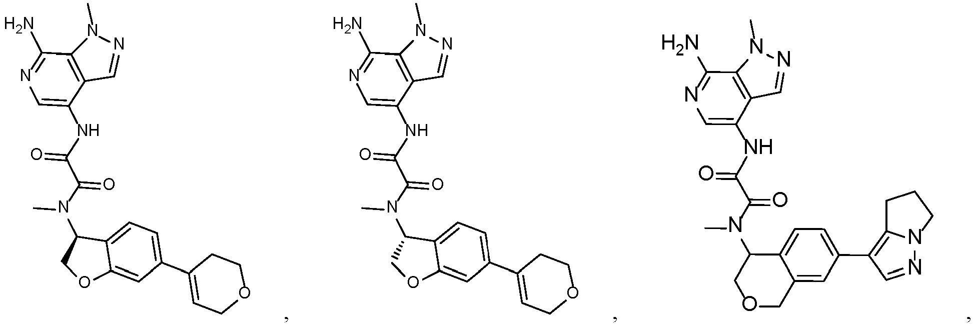 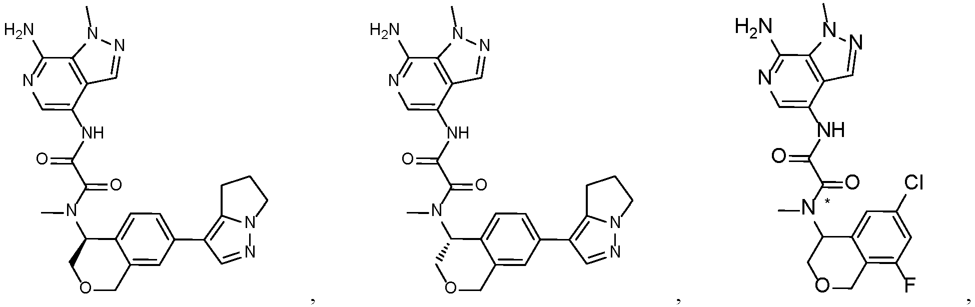 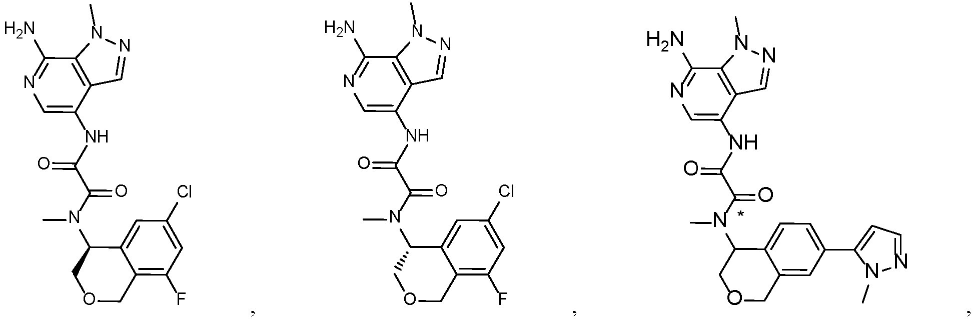 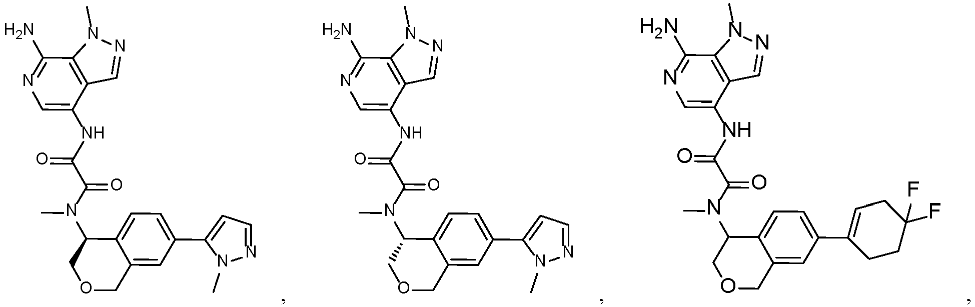 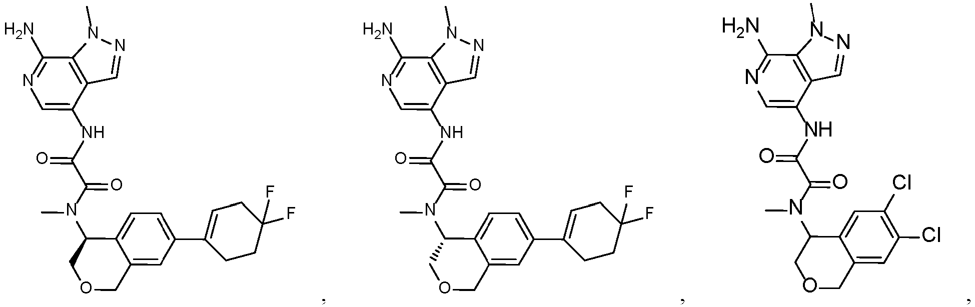 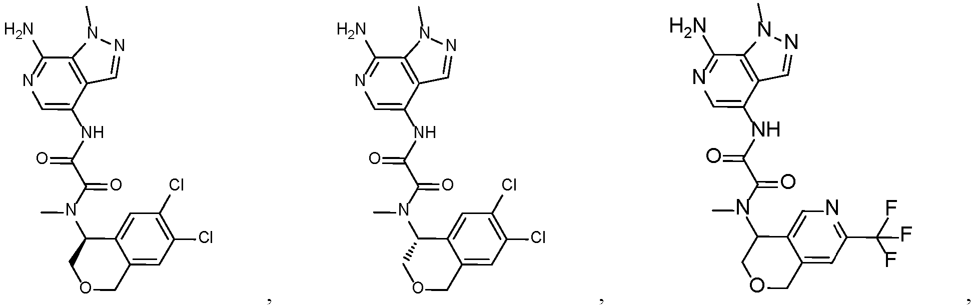 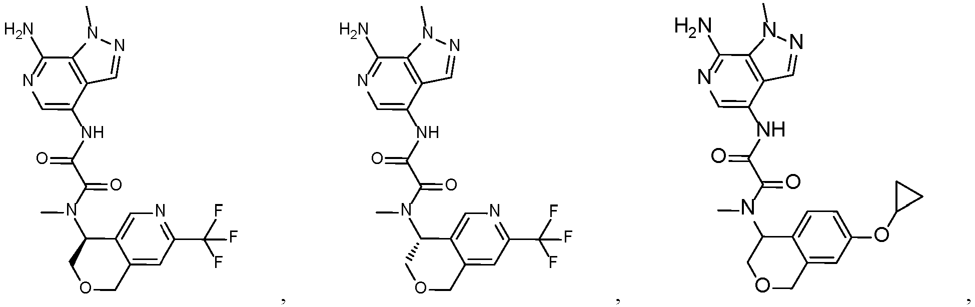 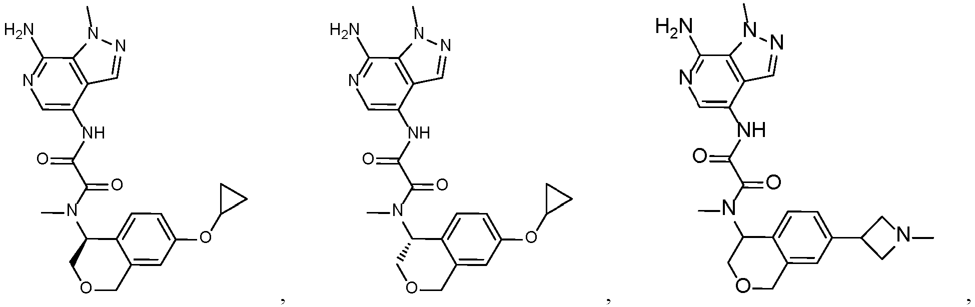 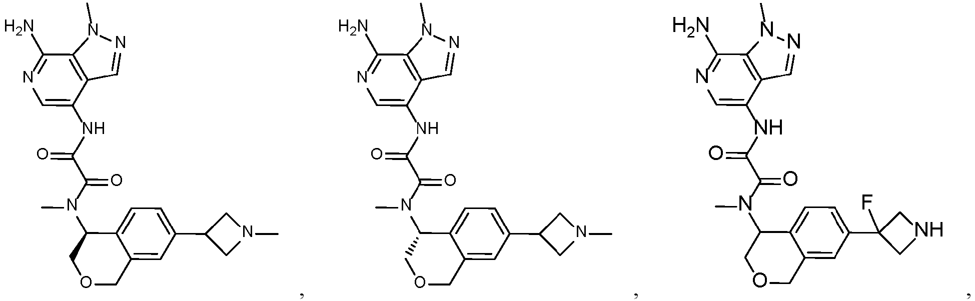 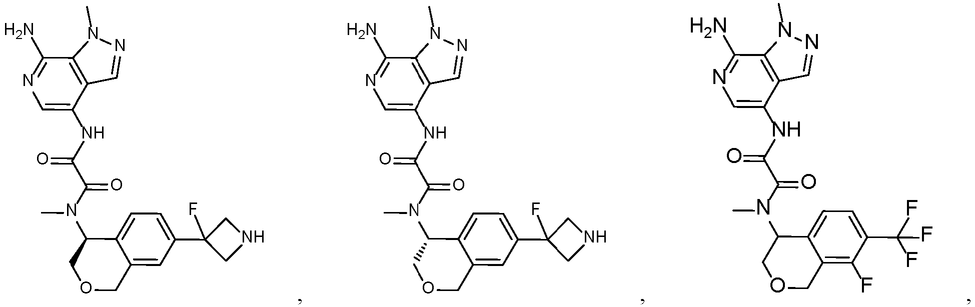 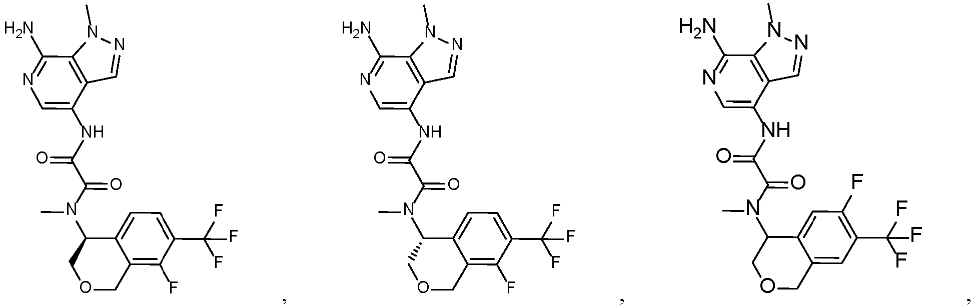 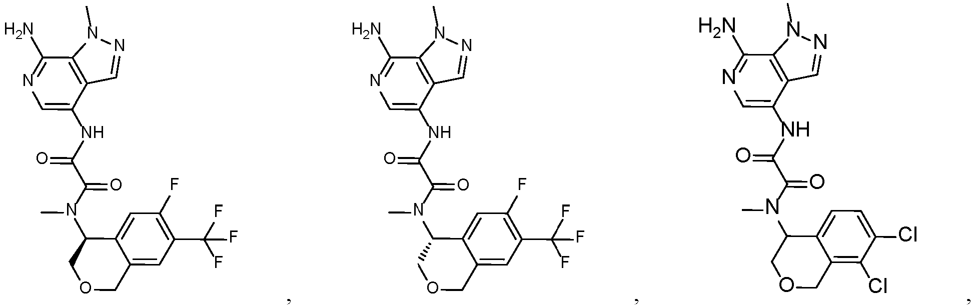 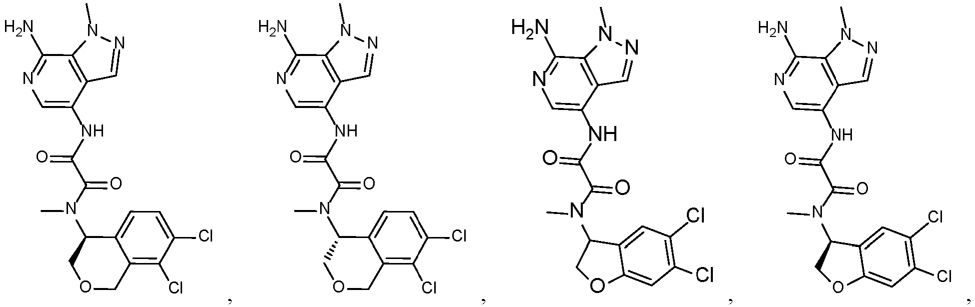 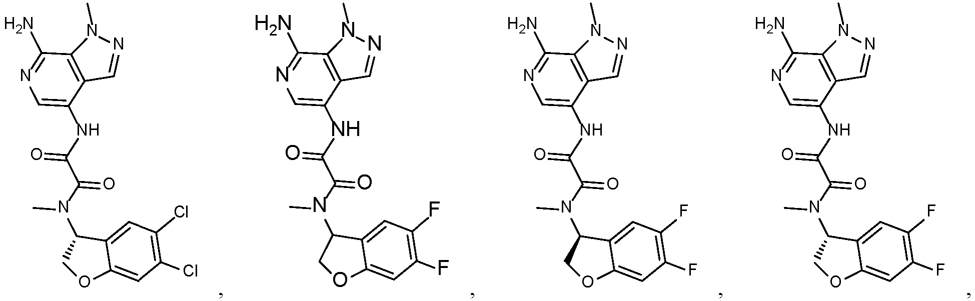 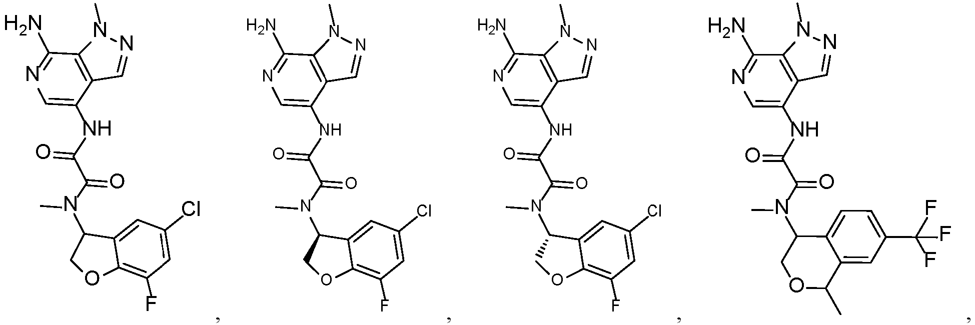 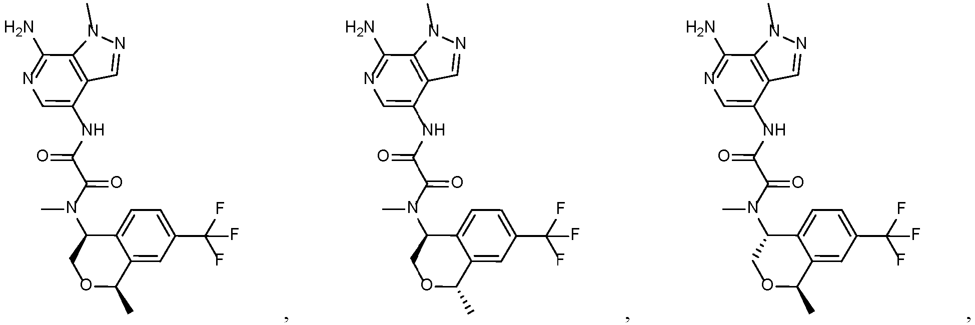 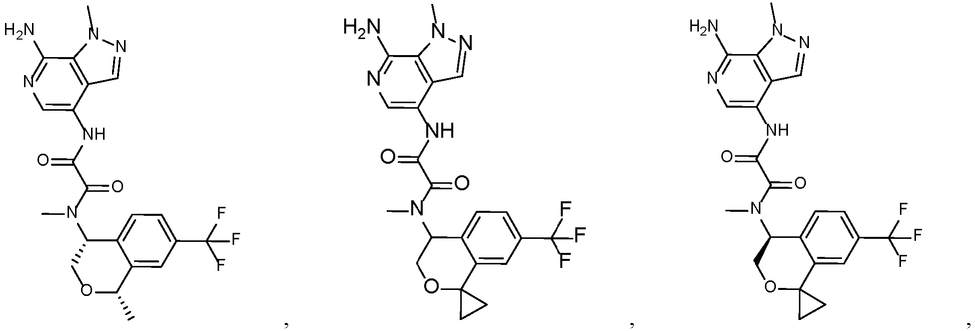 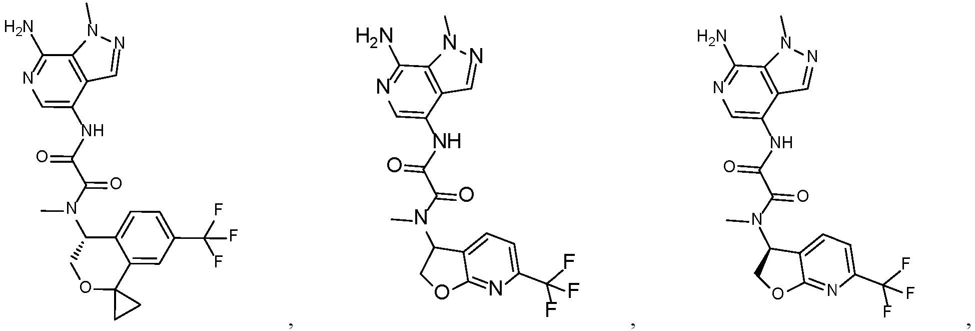 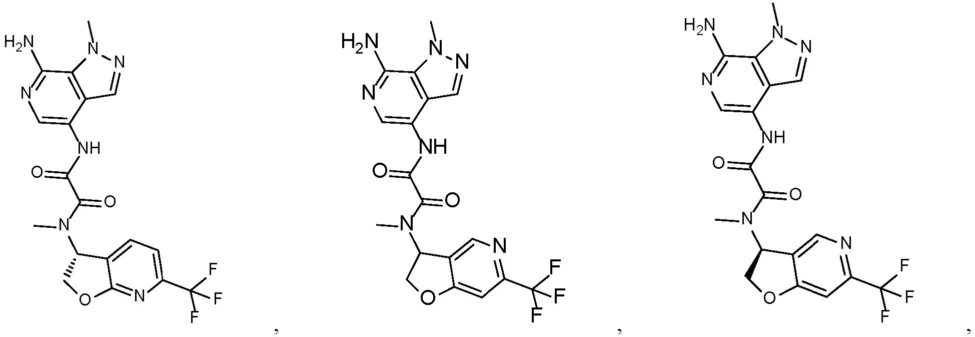 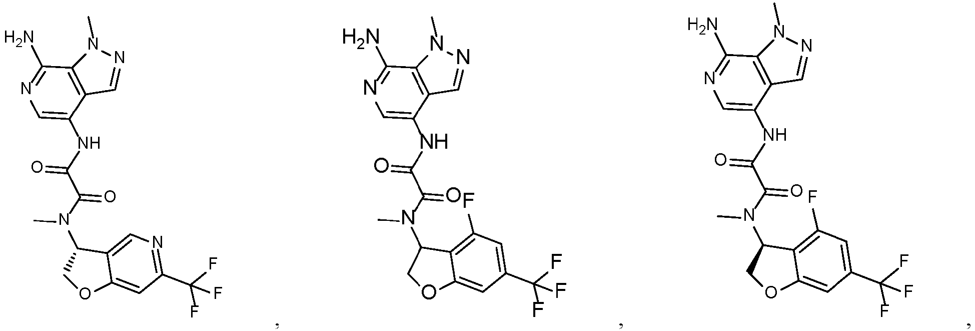 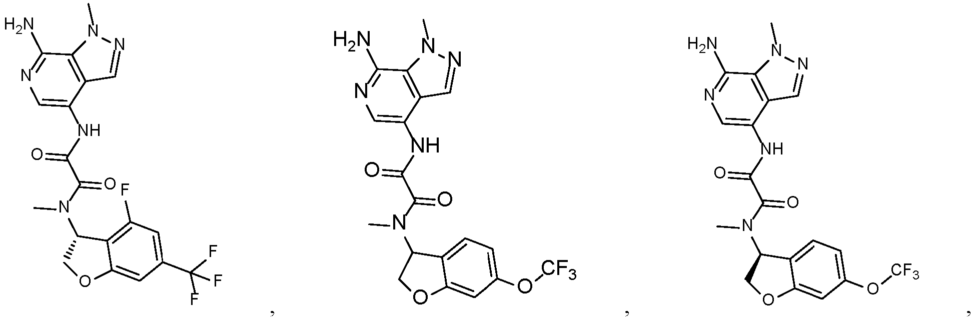 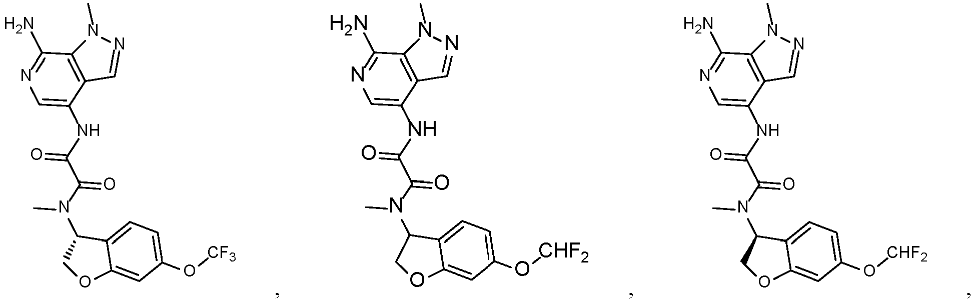 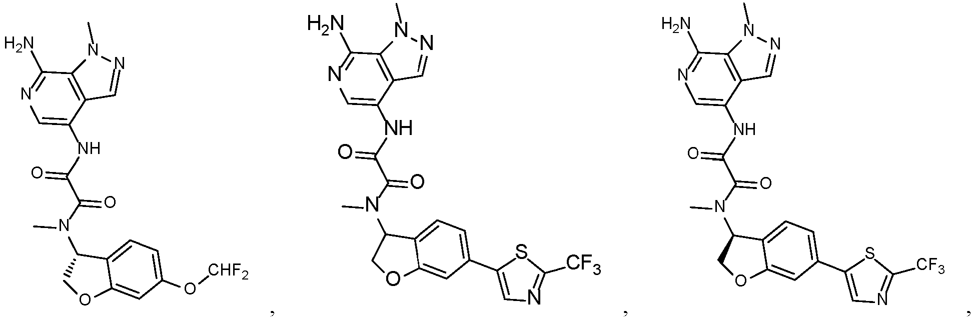 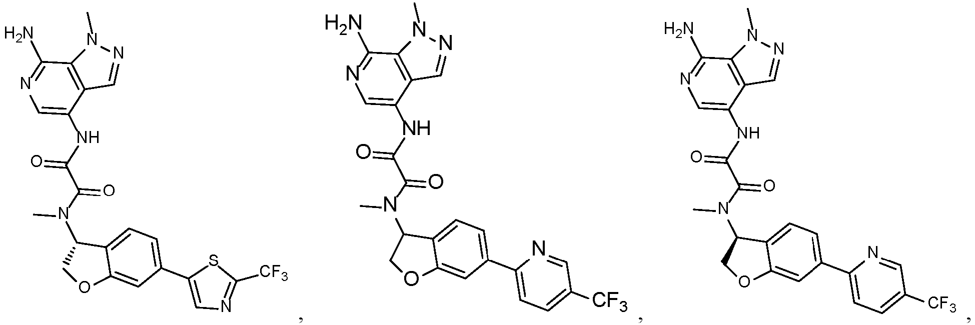 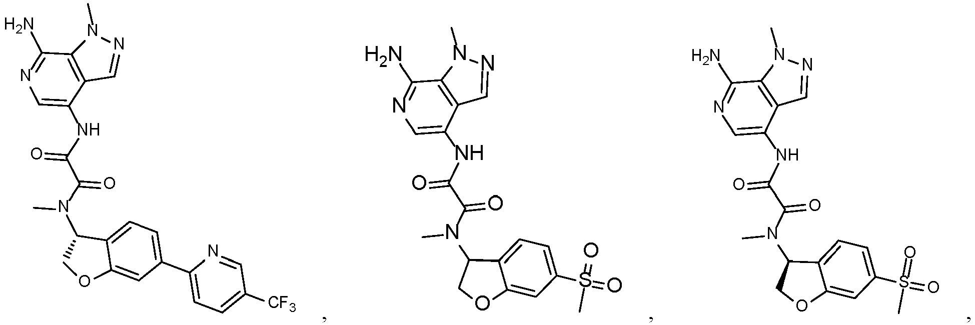 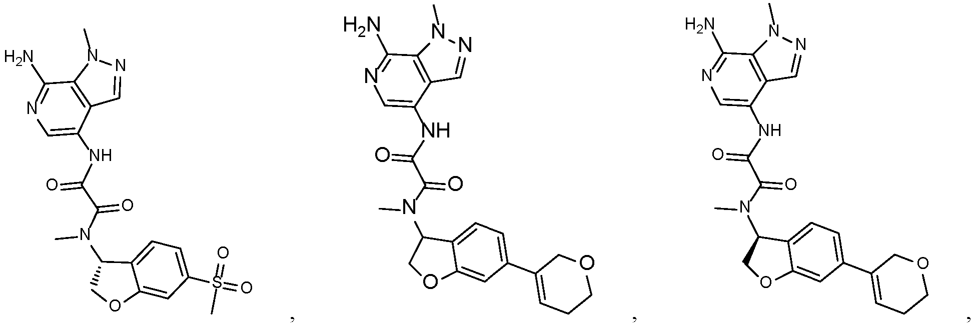 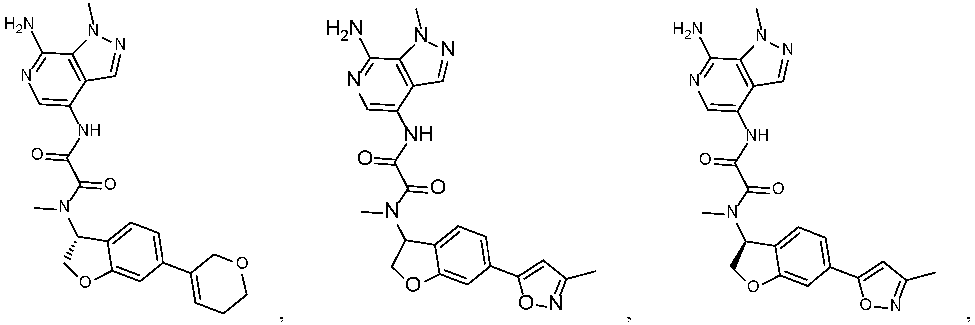 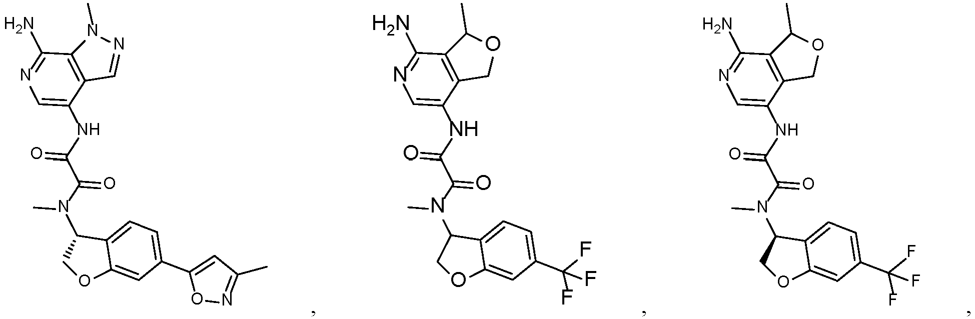 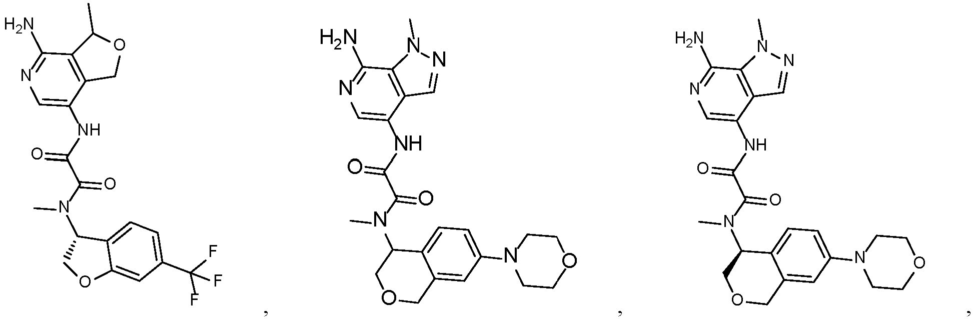 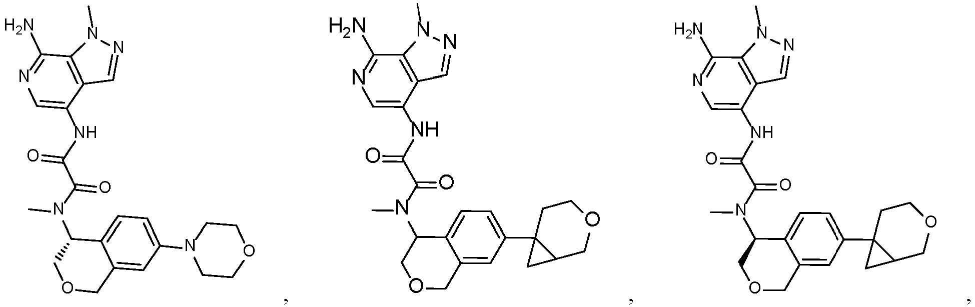 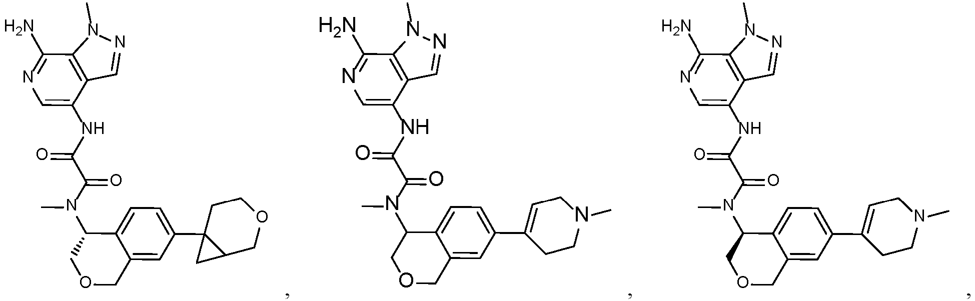 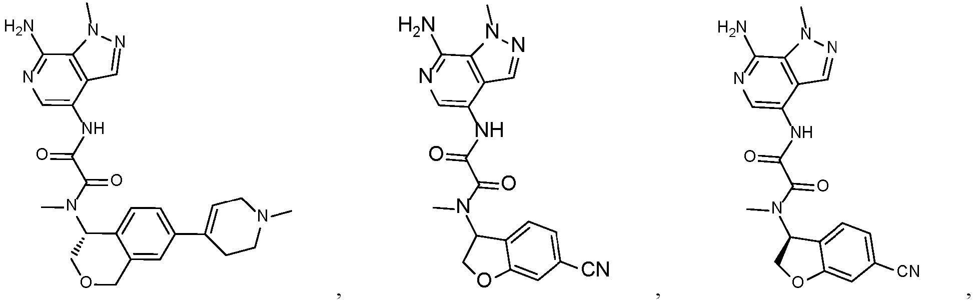 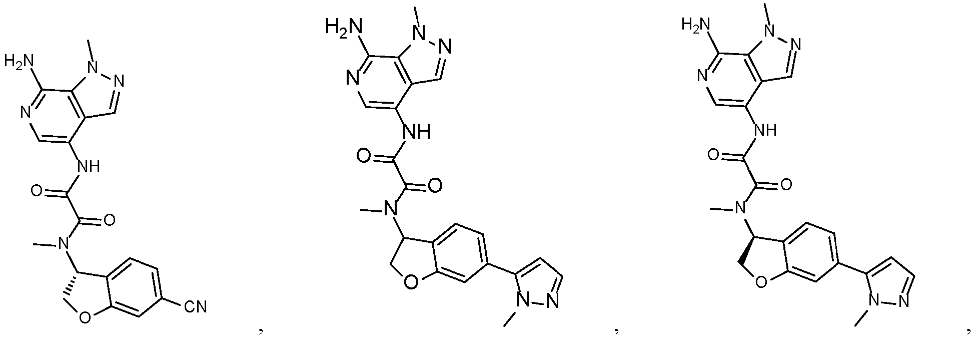 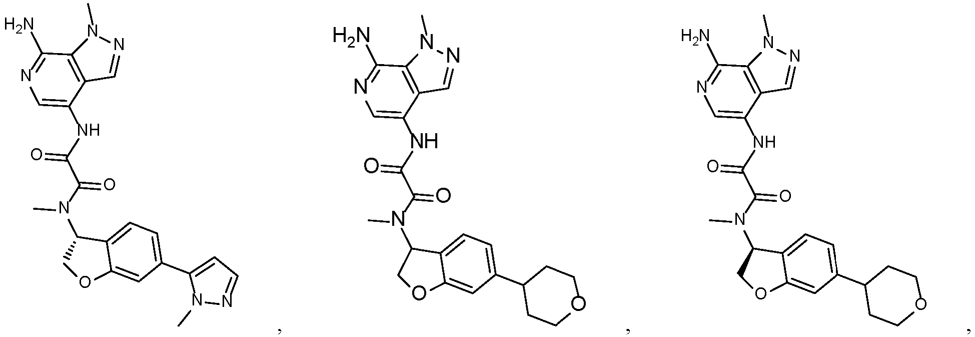 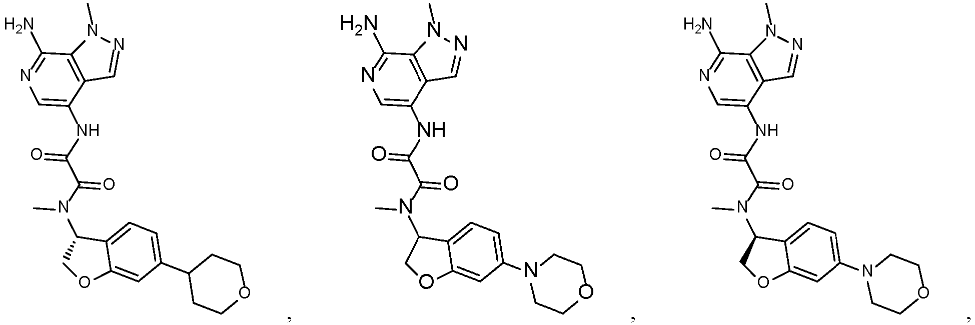 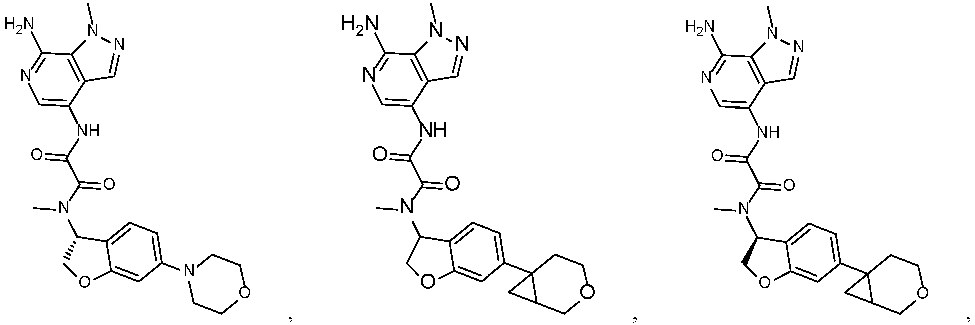 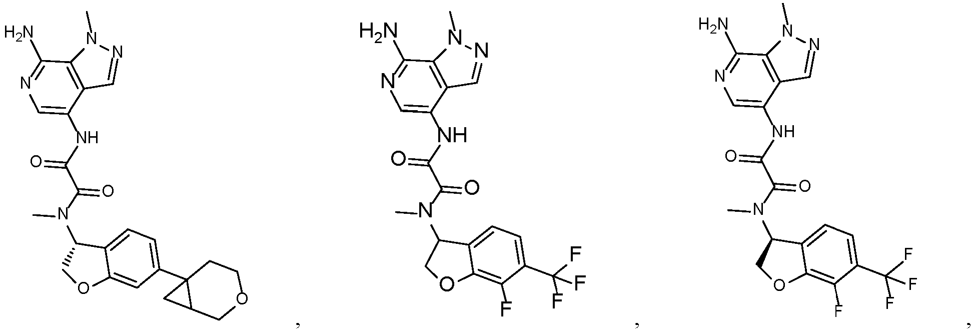 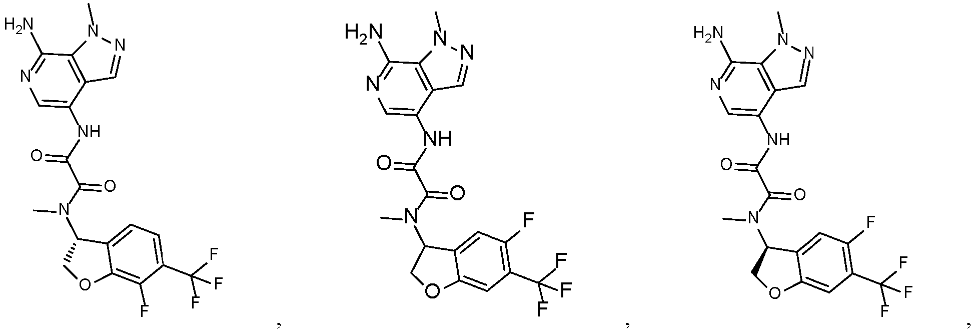 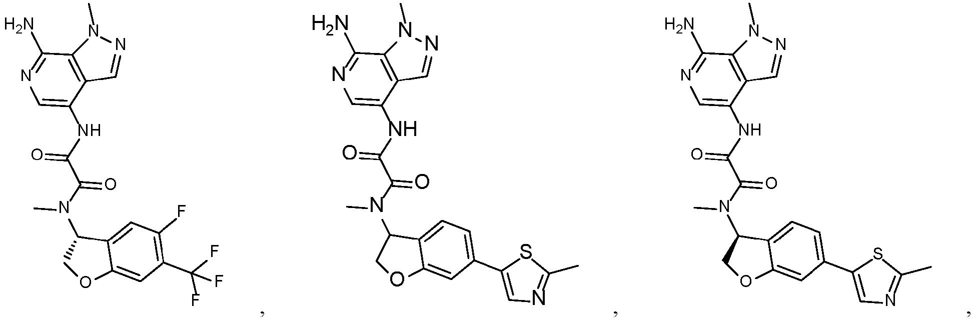 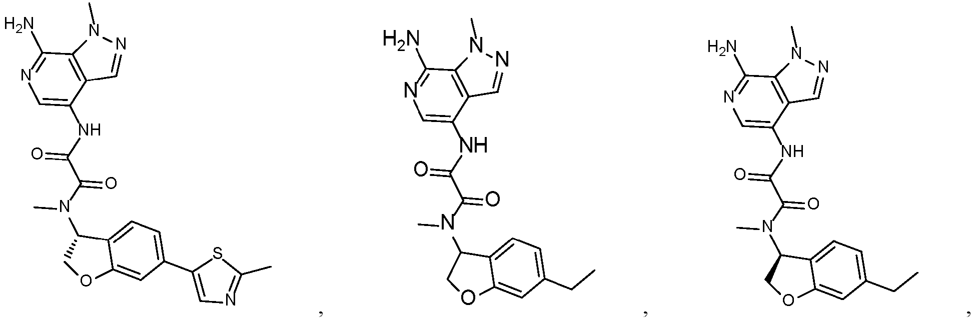 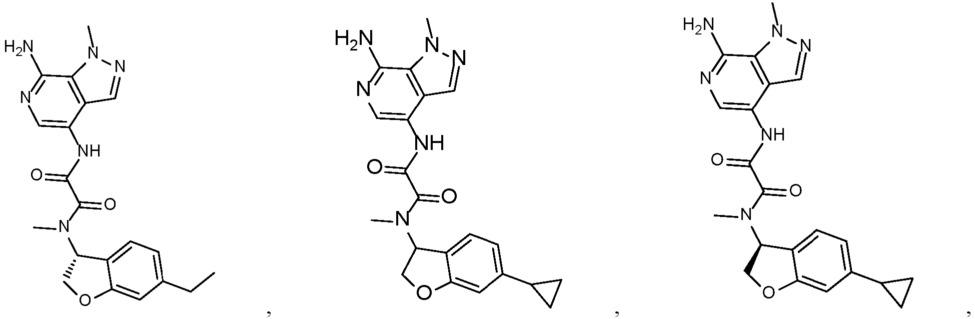 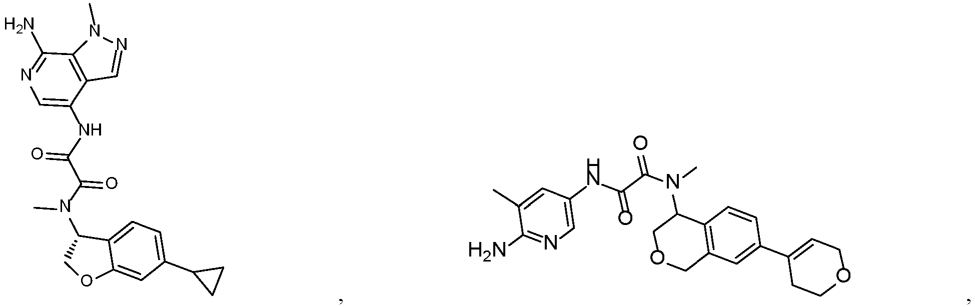 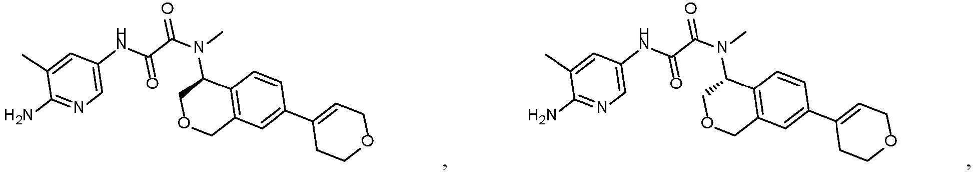 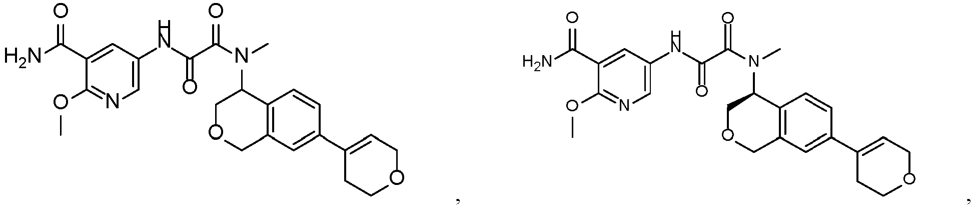 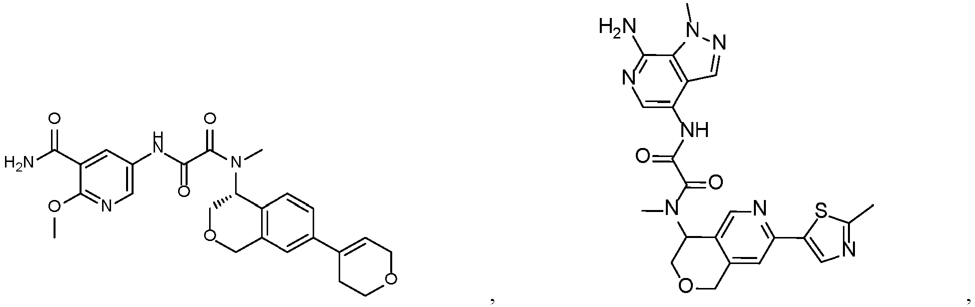 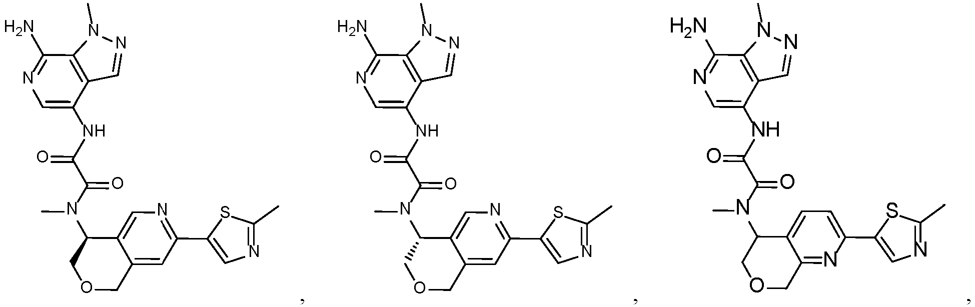 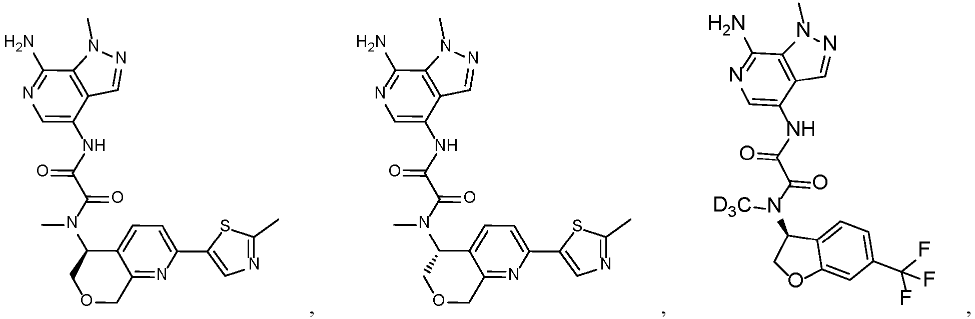 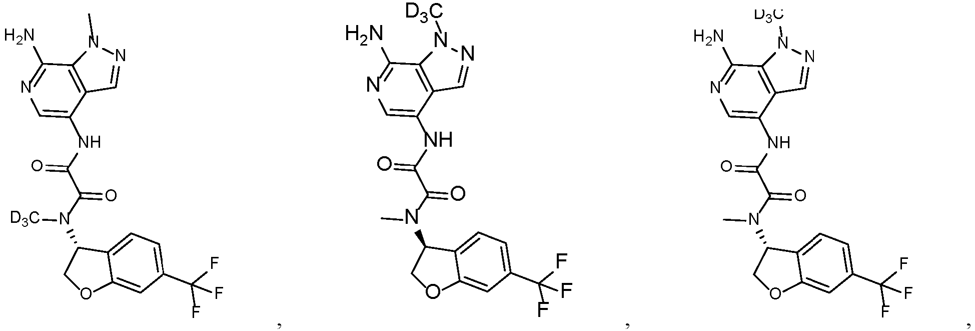 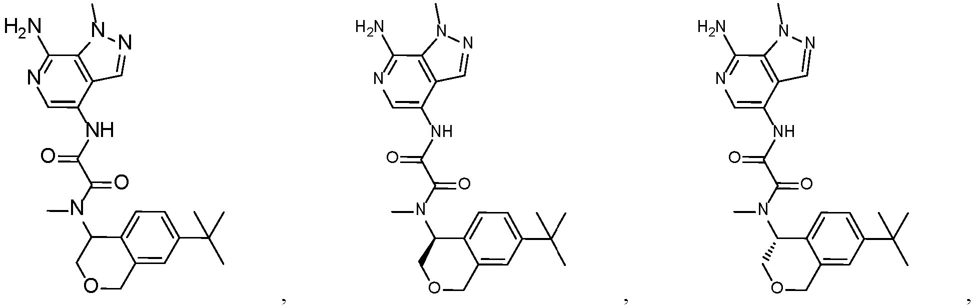 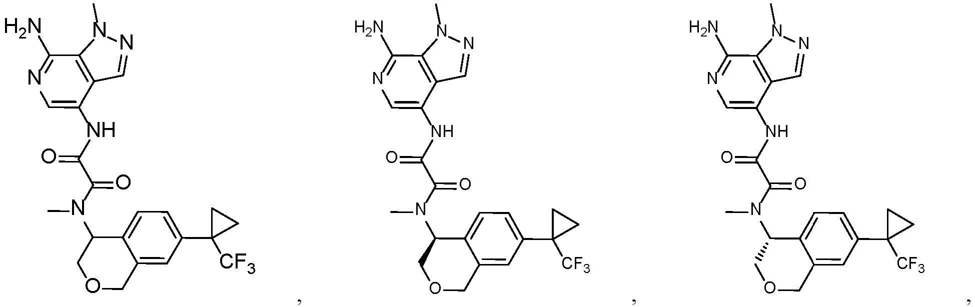 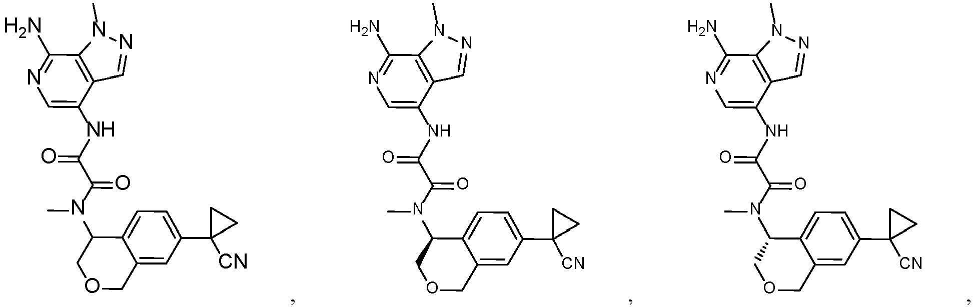 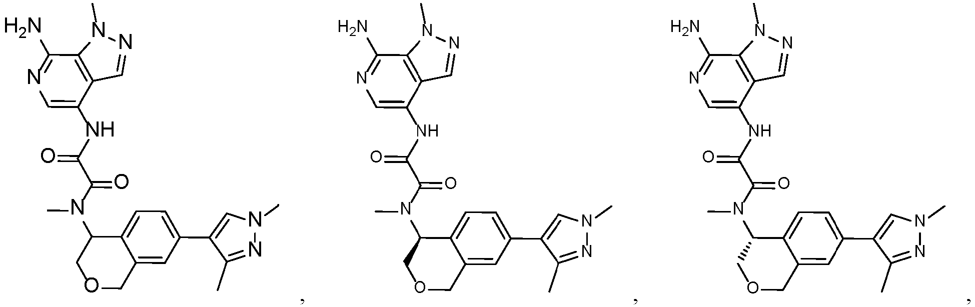 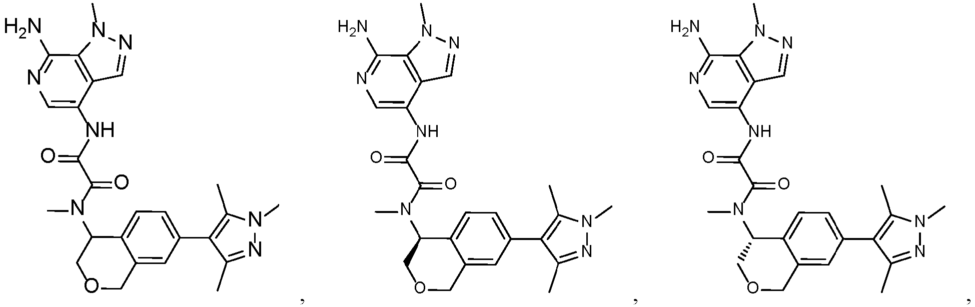 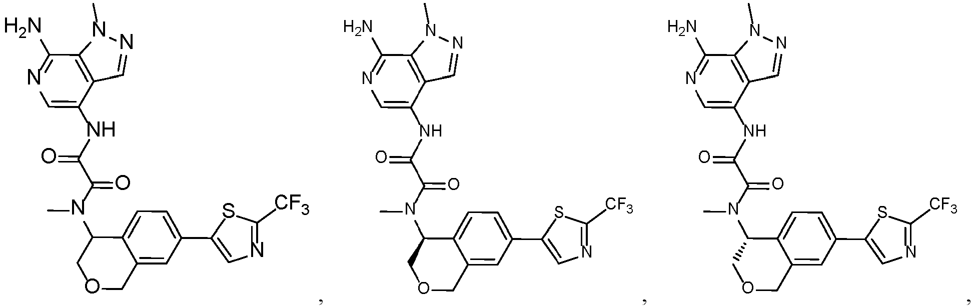 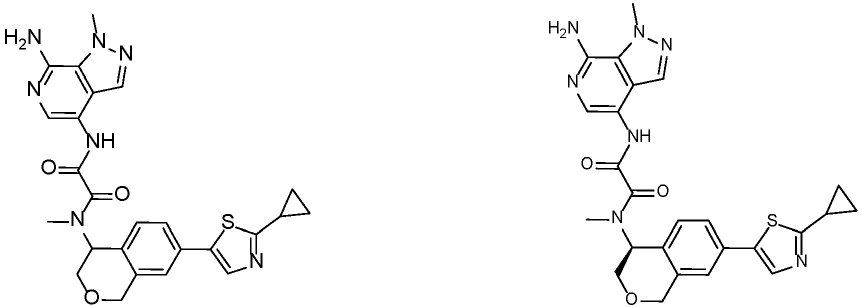 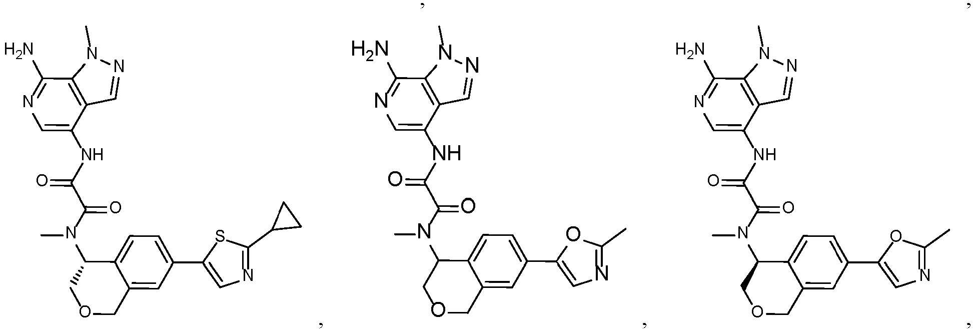 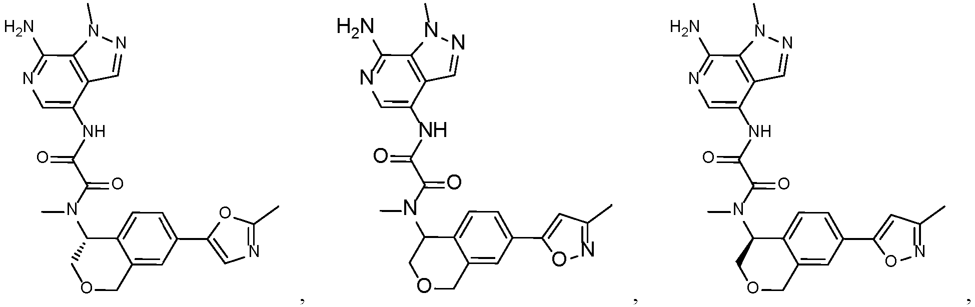 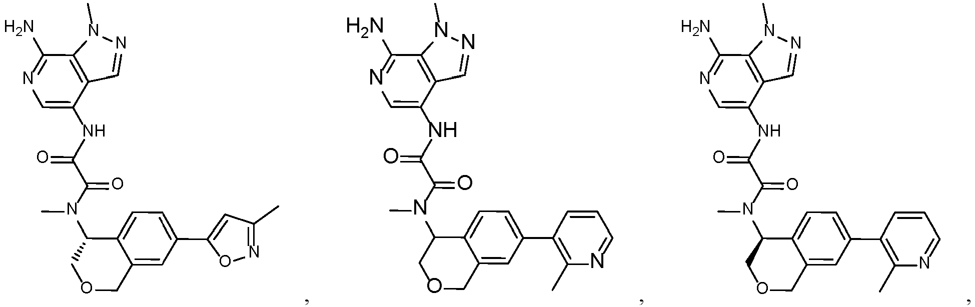 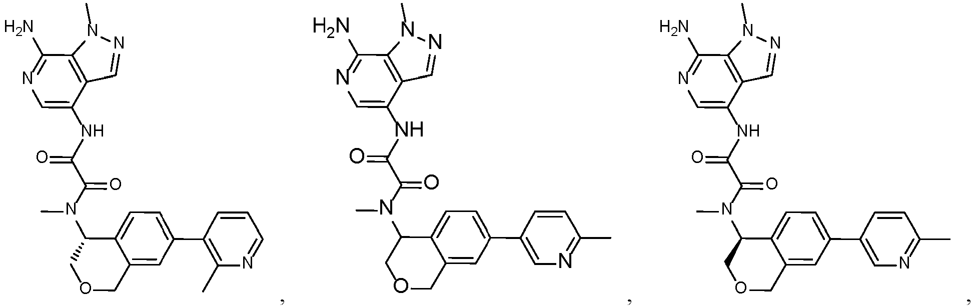 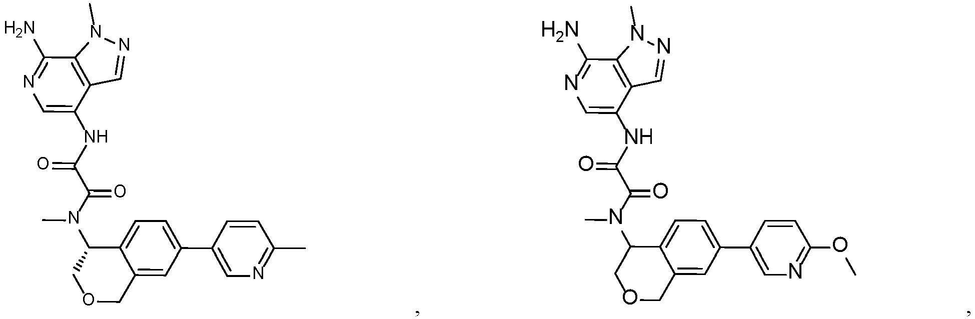 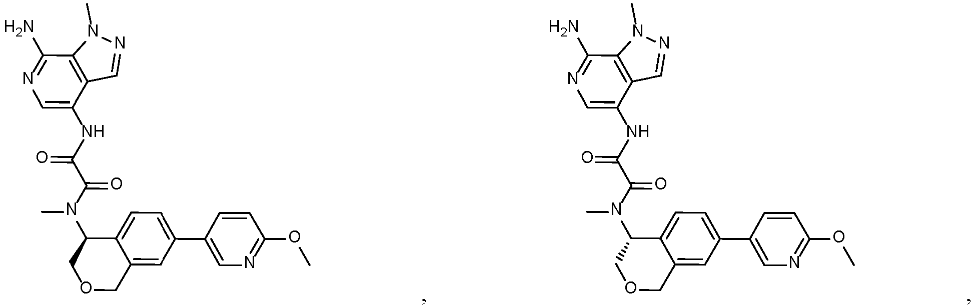 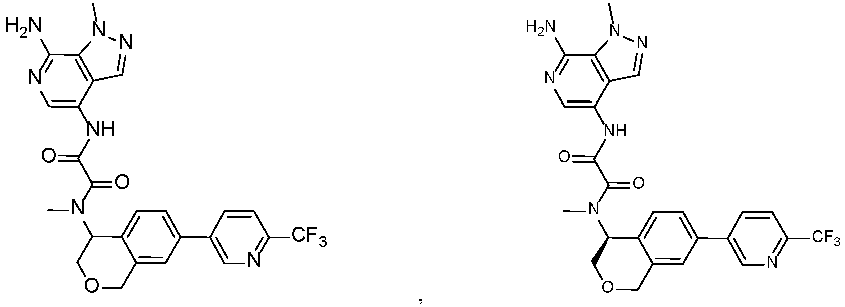 and

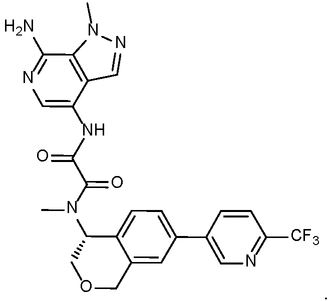

In a most specific embodiment, the present disclosure provides the above compound of formula (X), or a pharmaceutically acceptable salt, an isotopic variant, a tautomer, a stereoisomer, a prodrug, a polymorph, a hydrate or a solvate thereof, wherein, the compound is selected from: 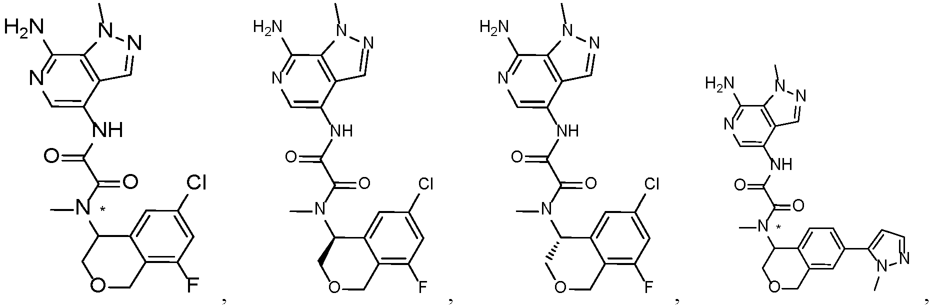 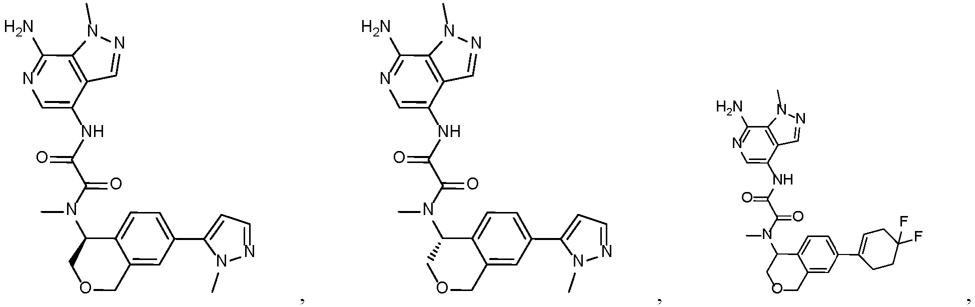 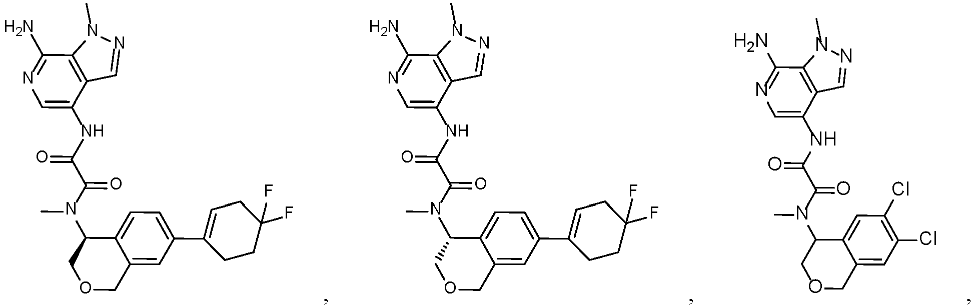 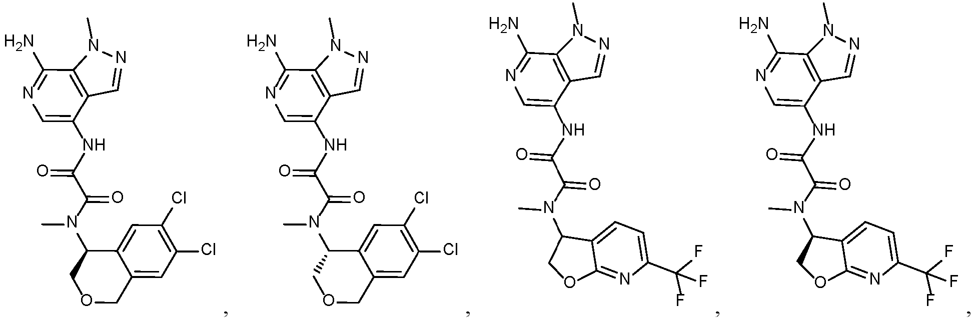 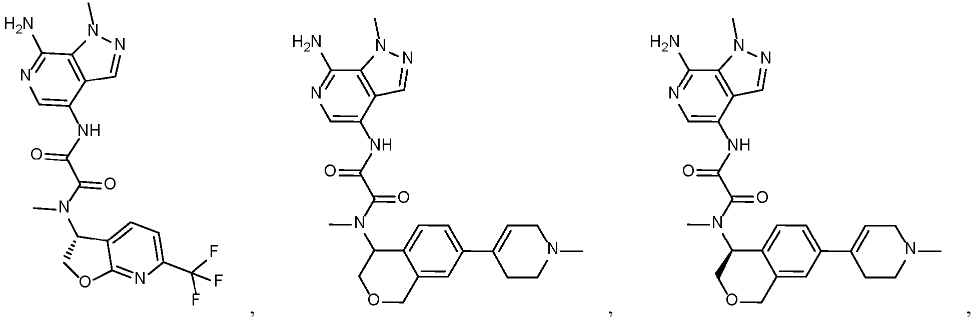 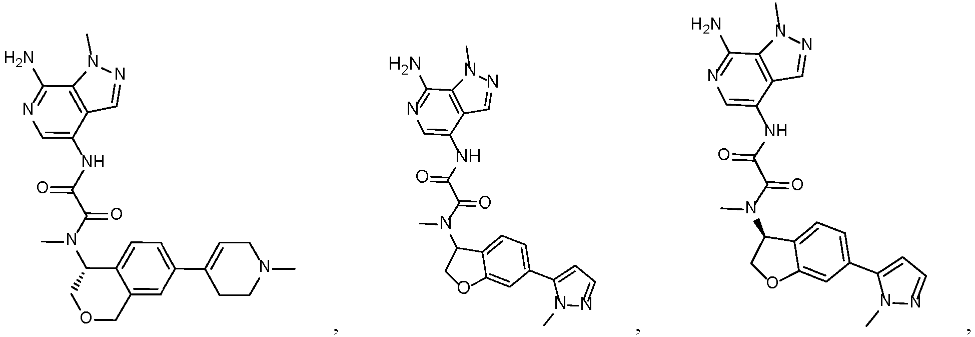 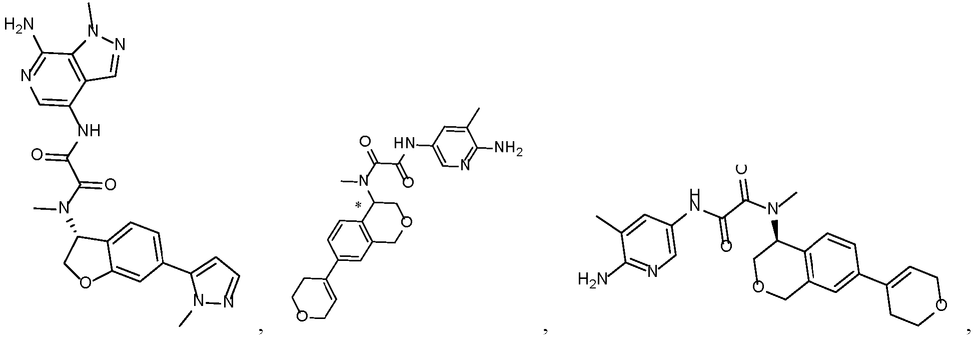 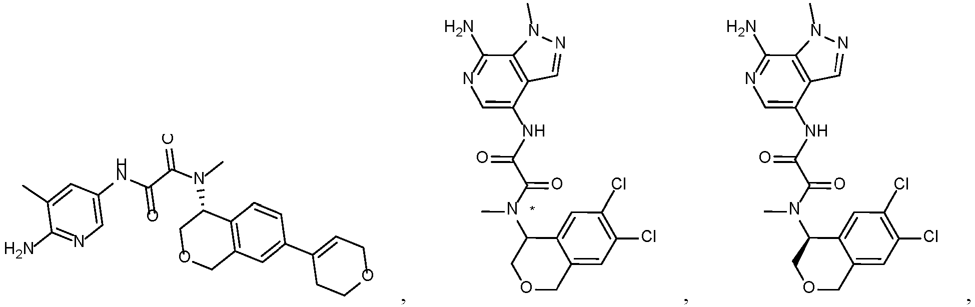 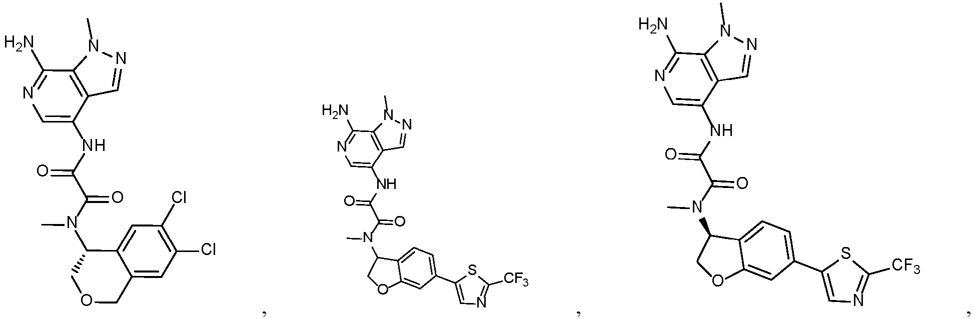 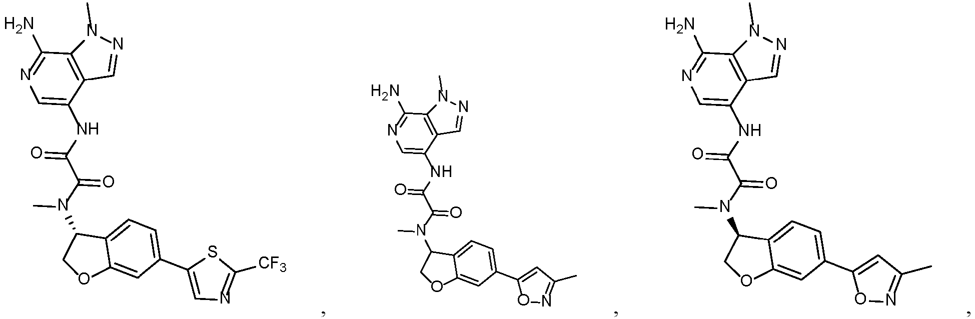 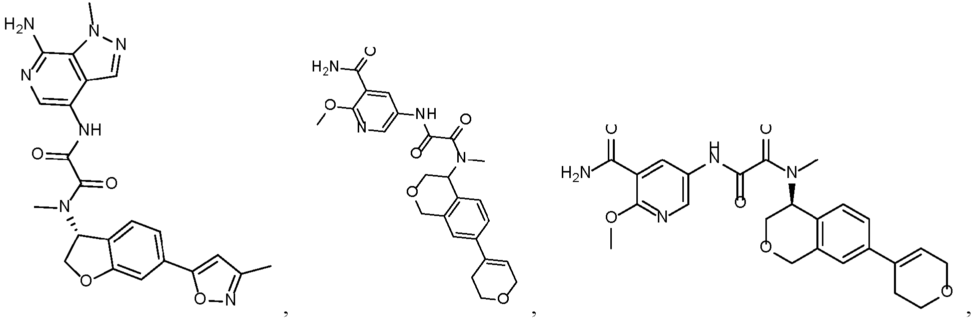 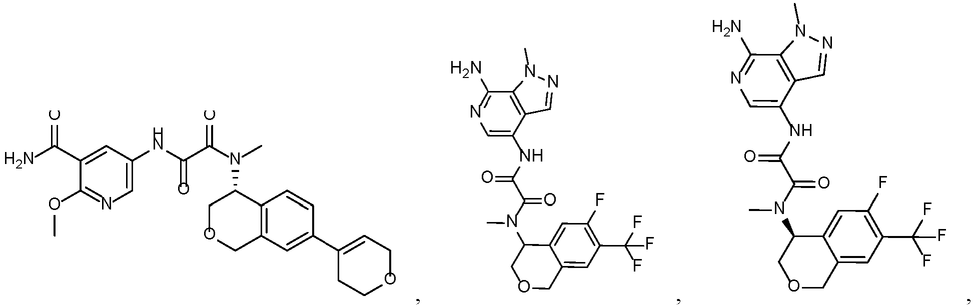 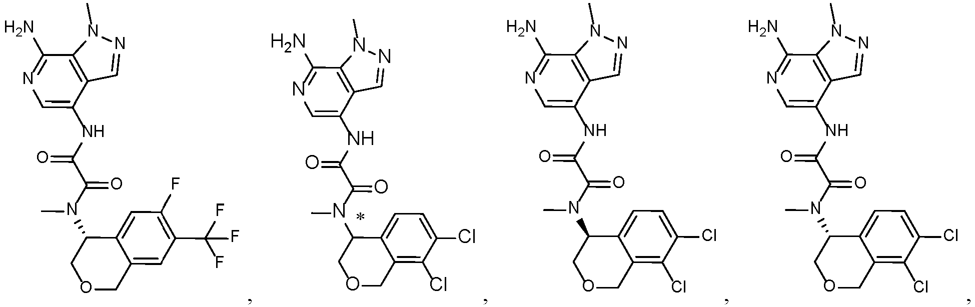 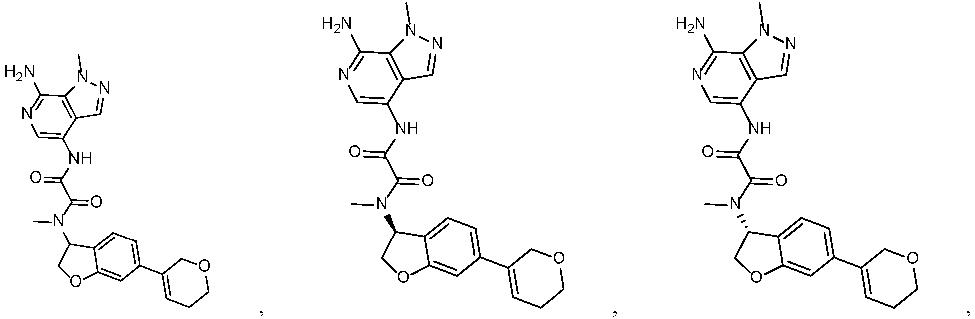 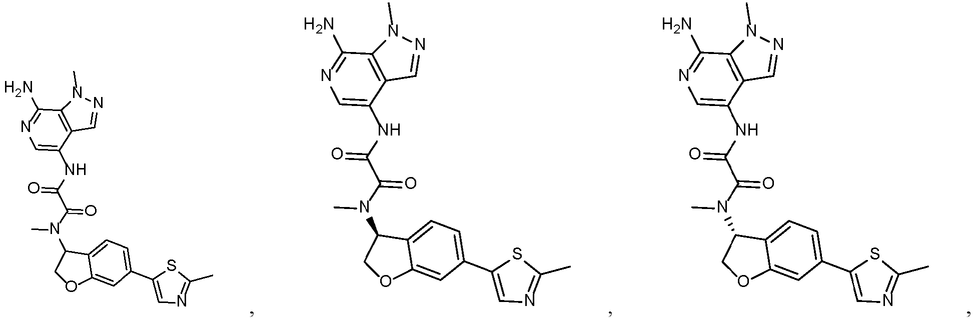 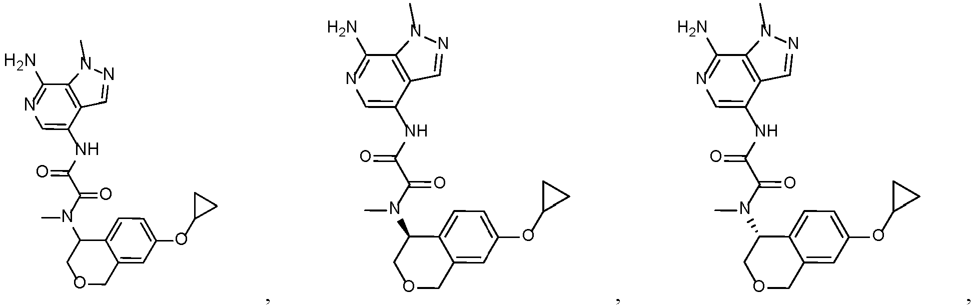 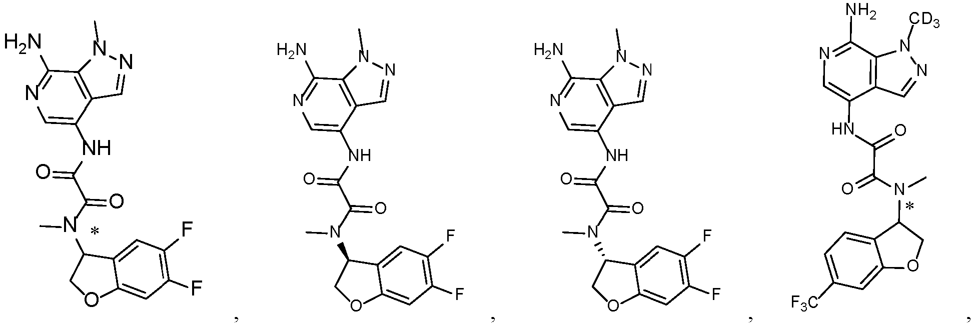 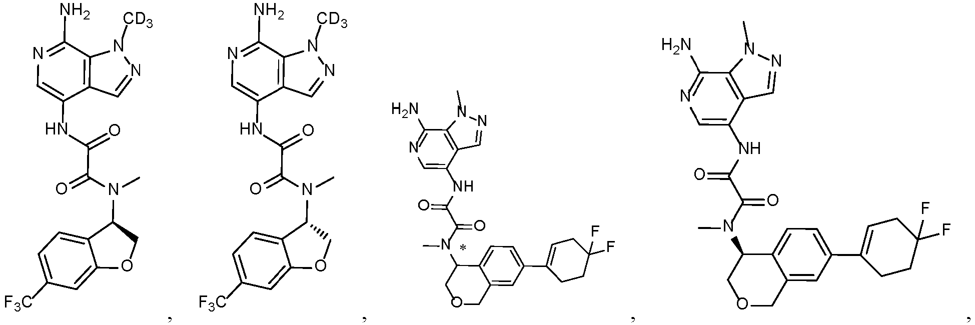 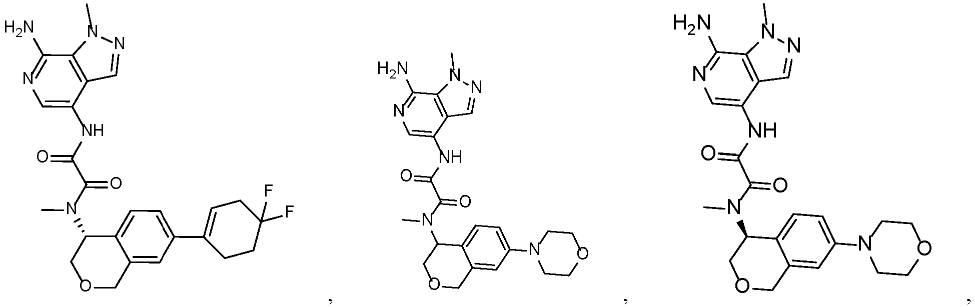 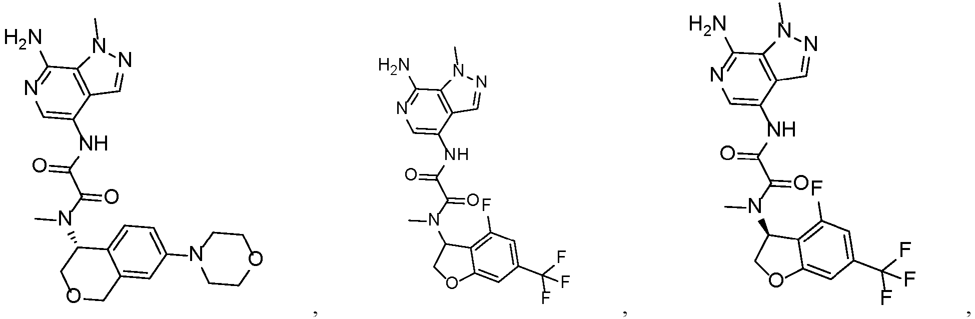 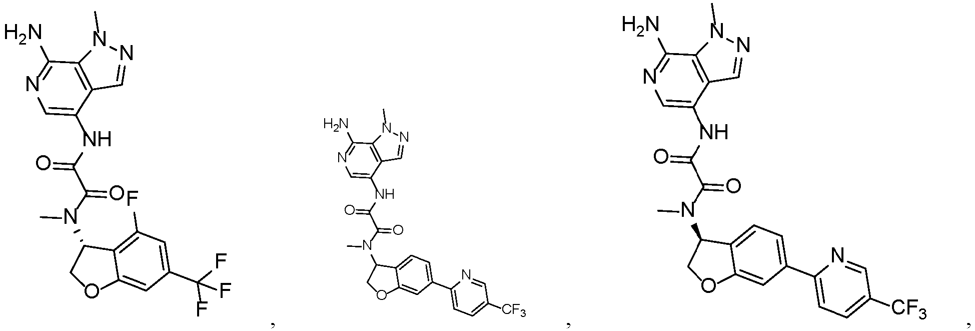 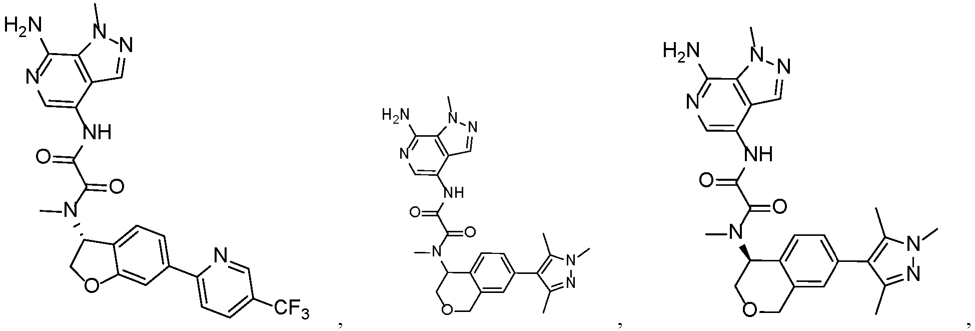 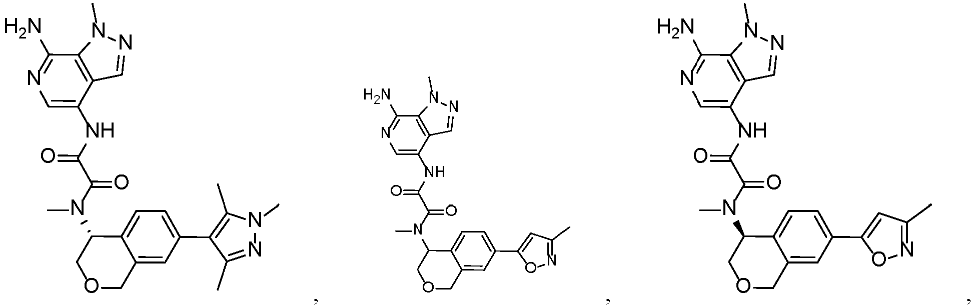 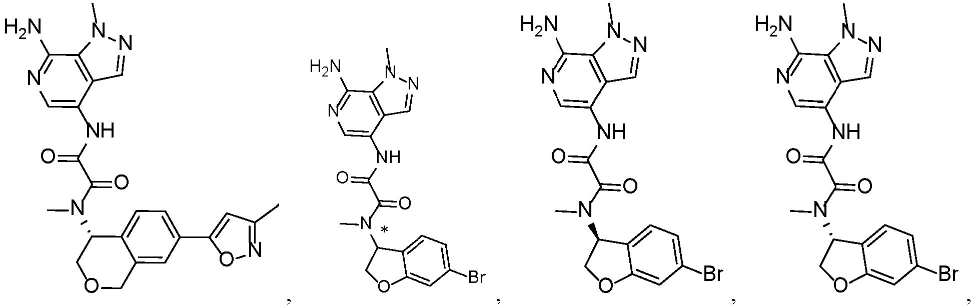 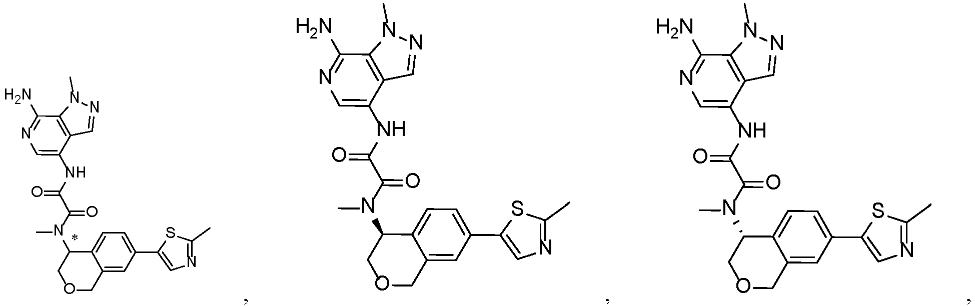 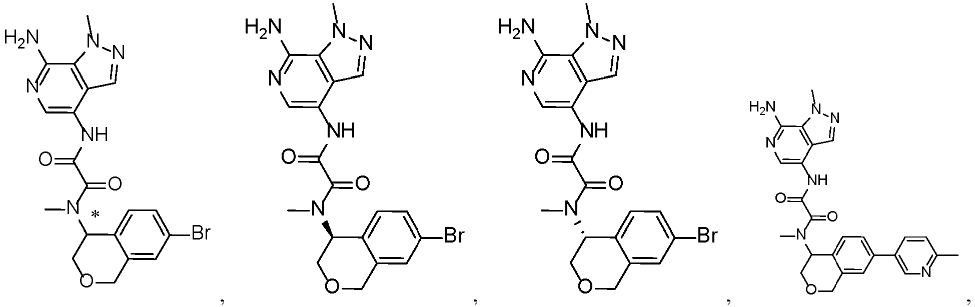 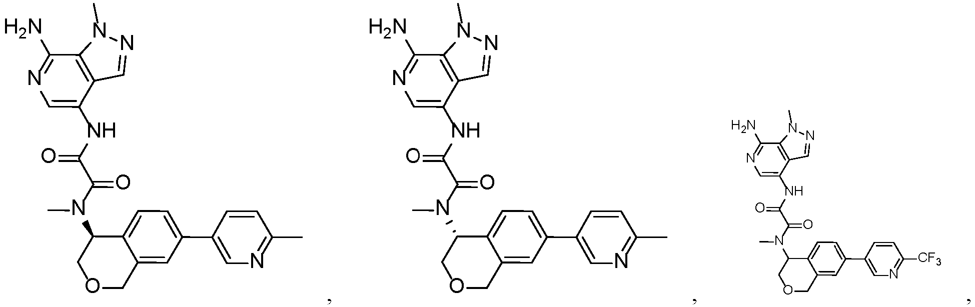 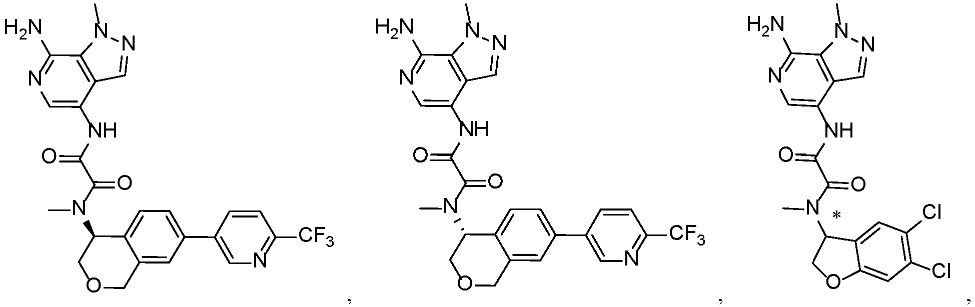 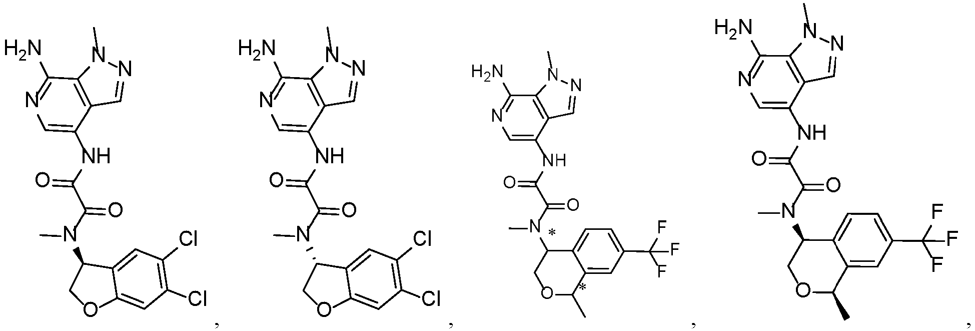 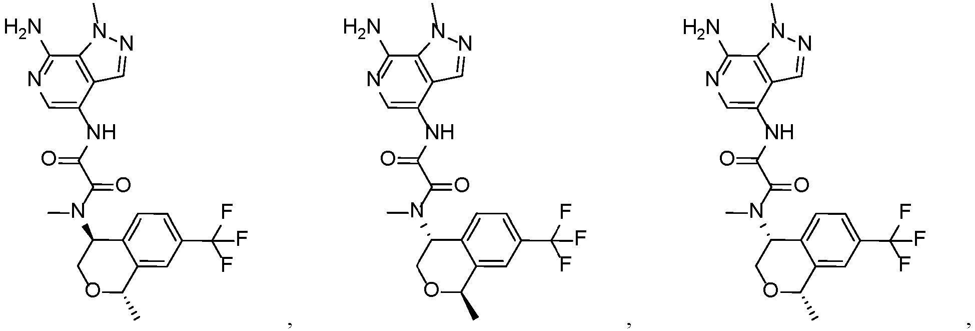 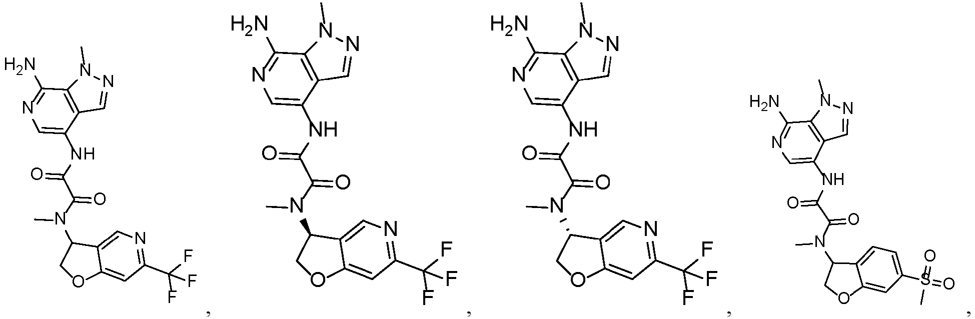 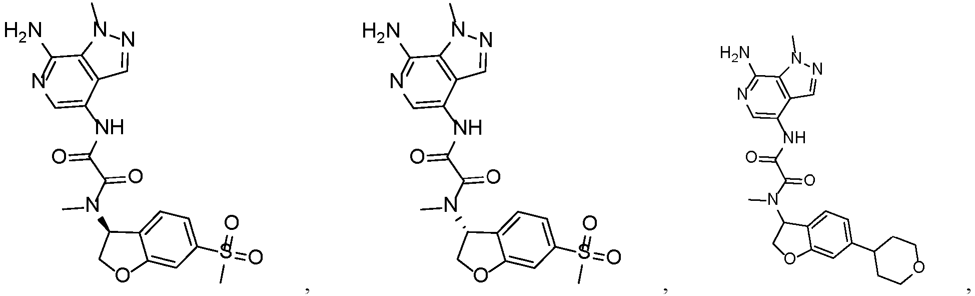 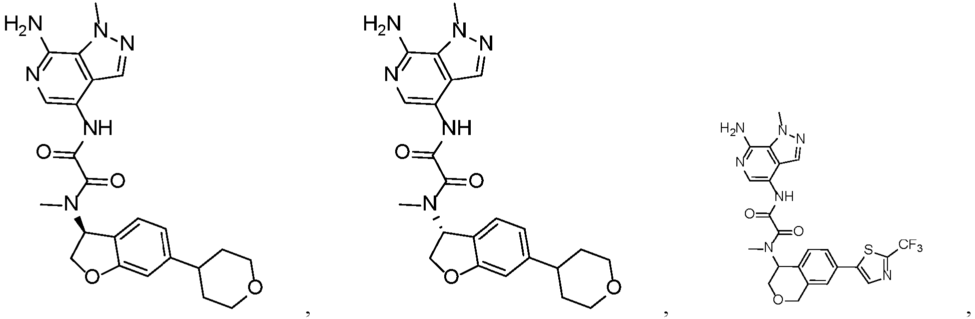 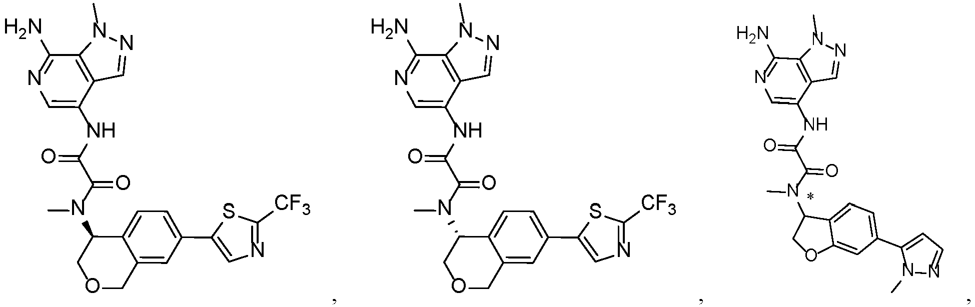 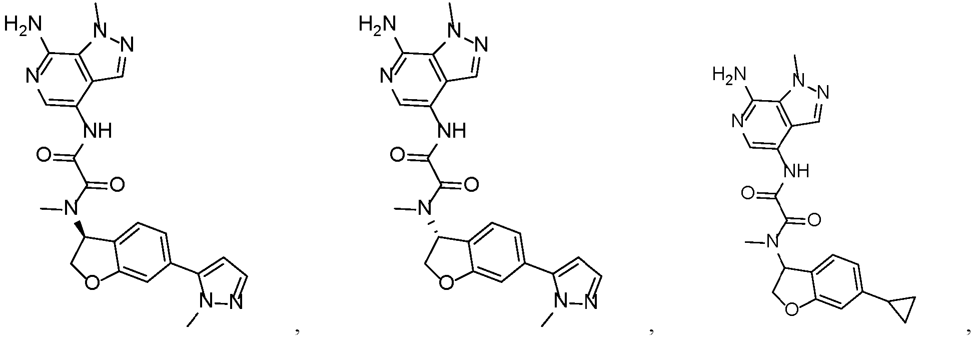 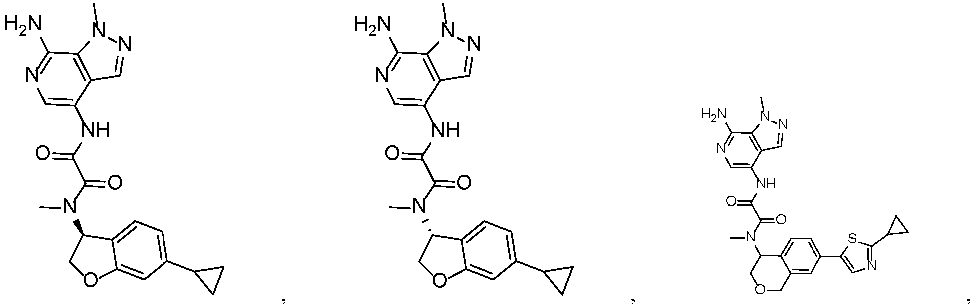 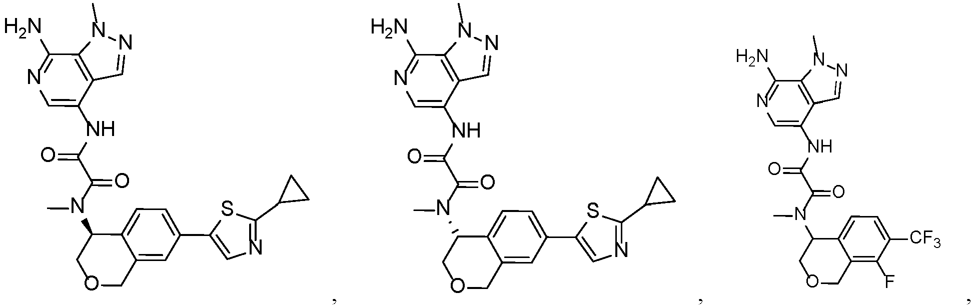 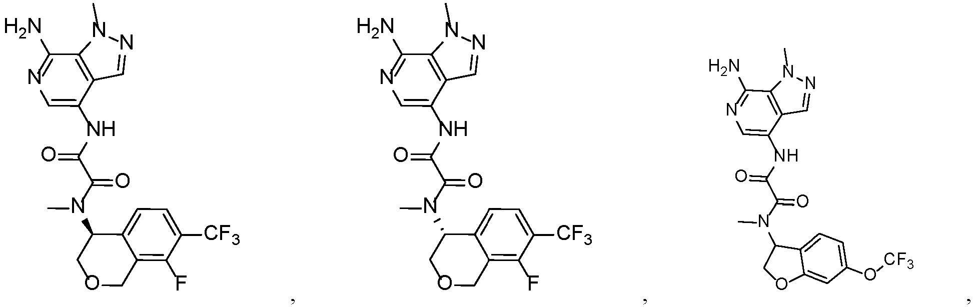 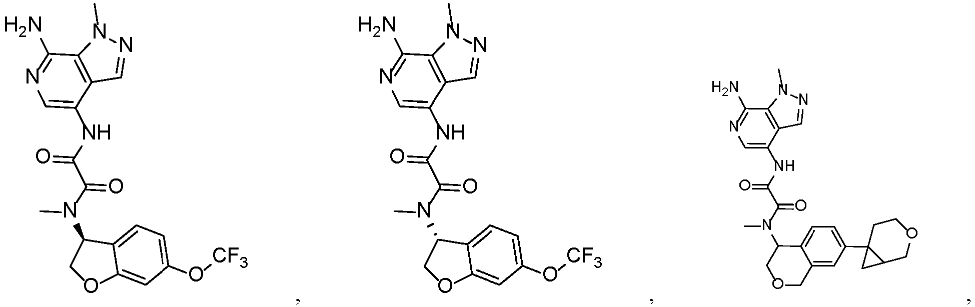 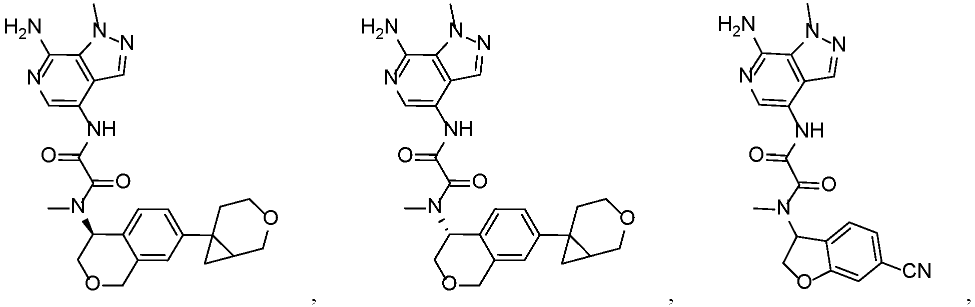 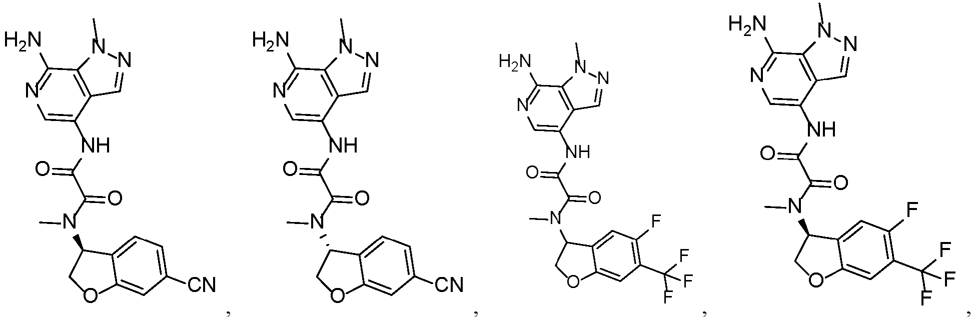 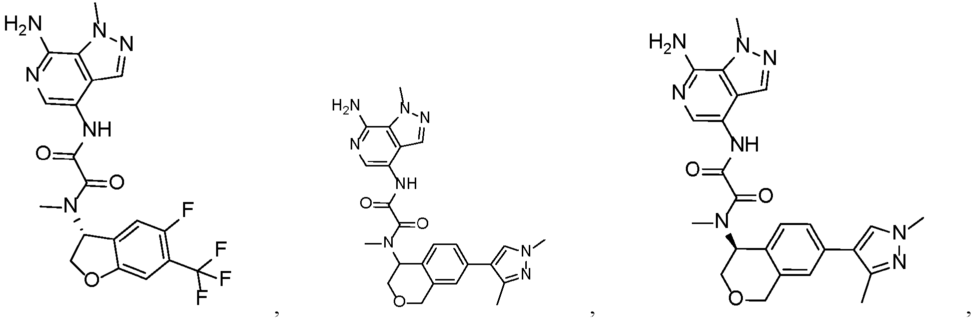 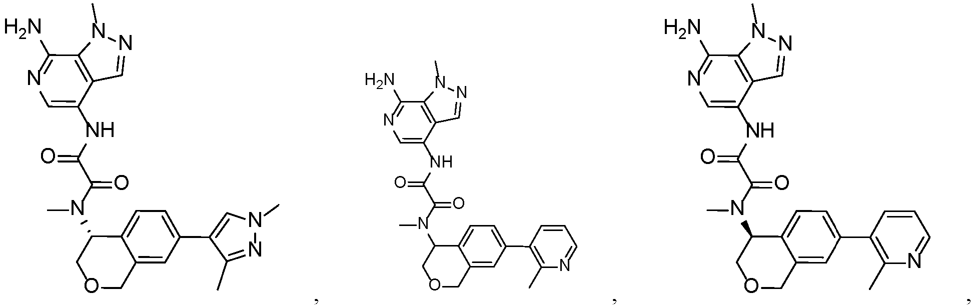 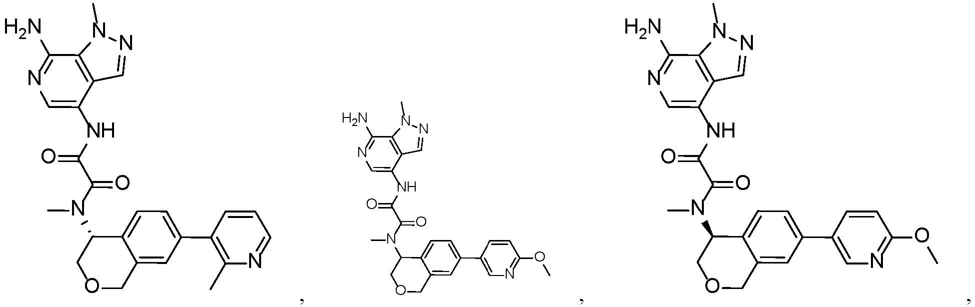 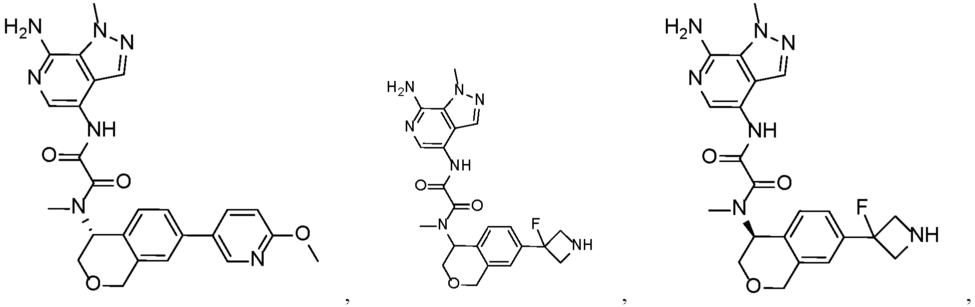 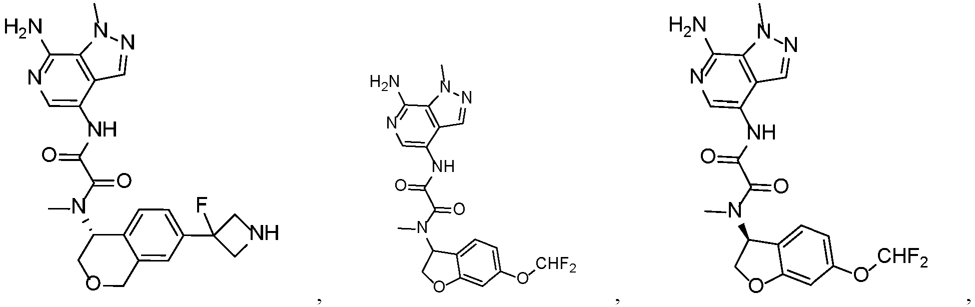 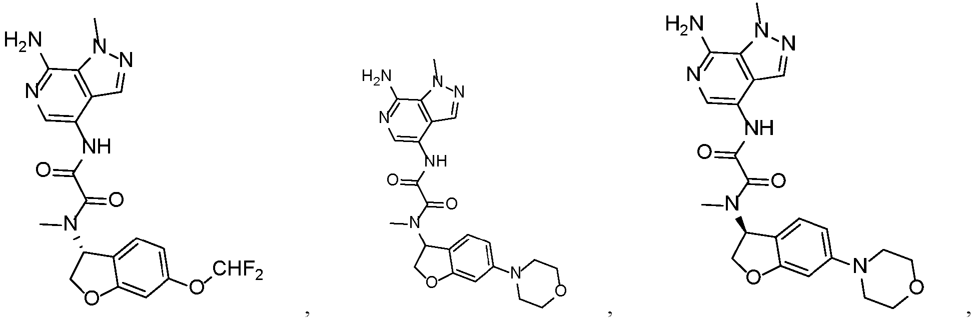 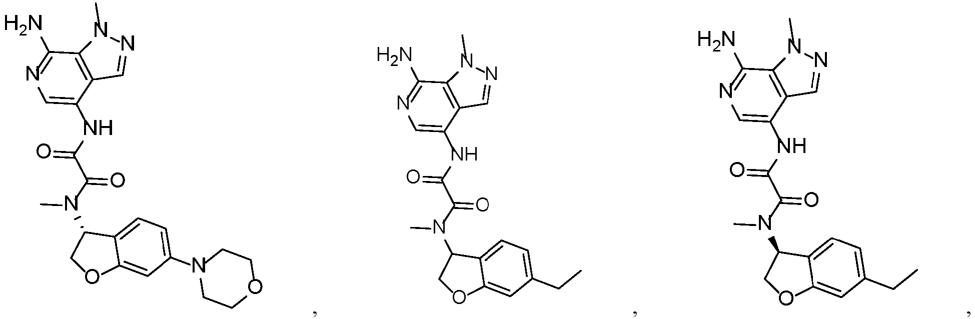 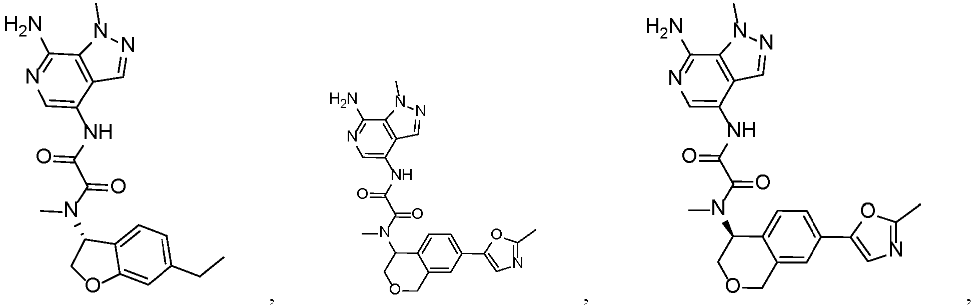 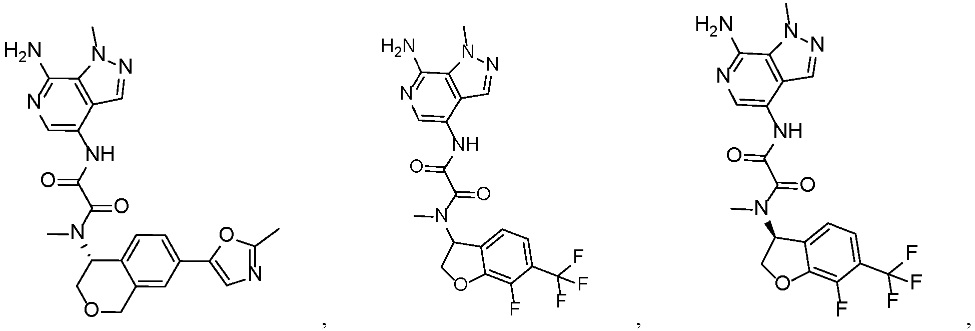 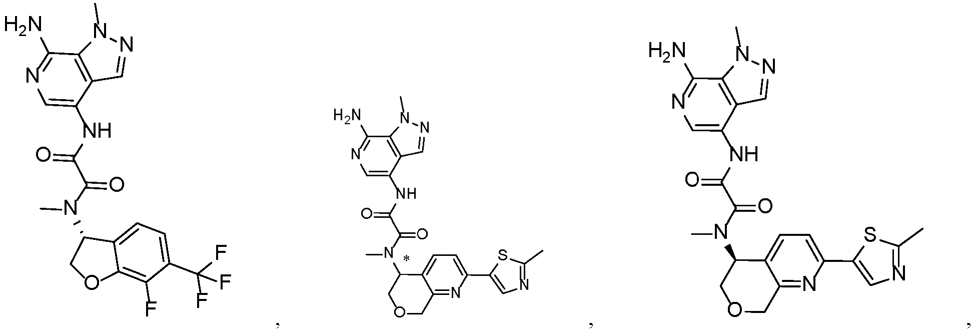 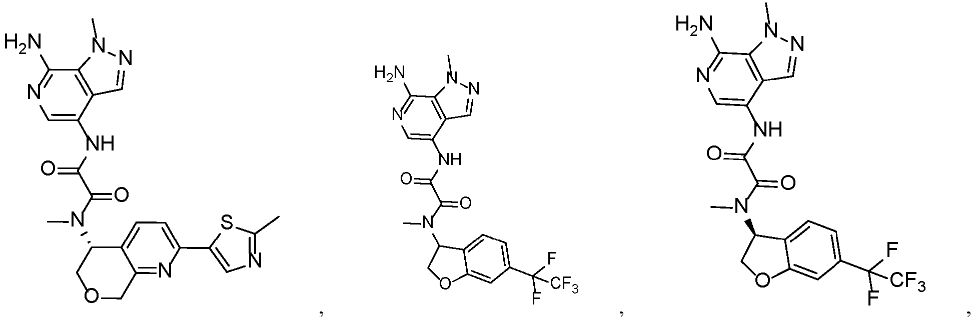 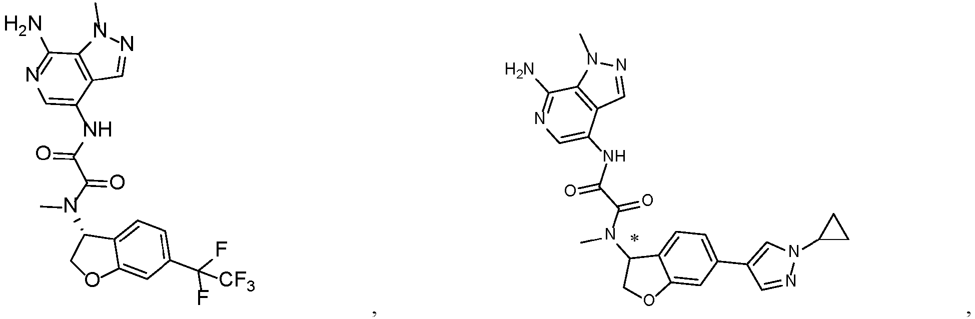 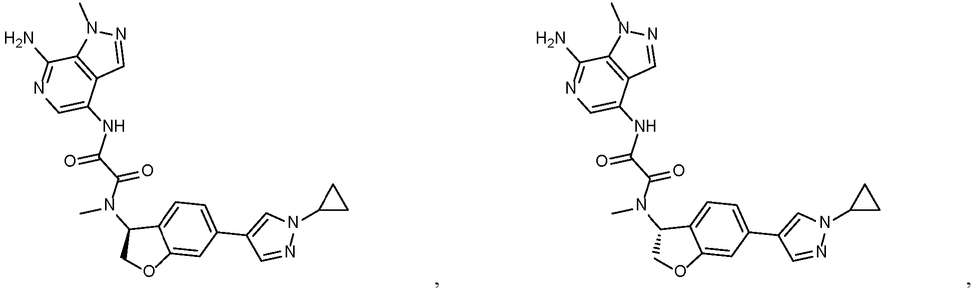 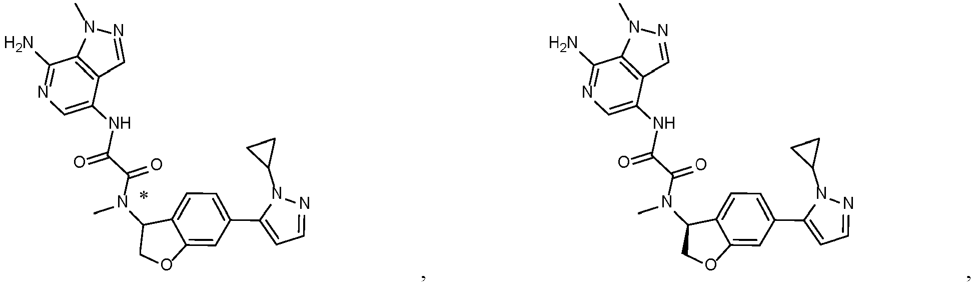 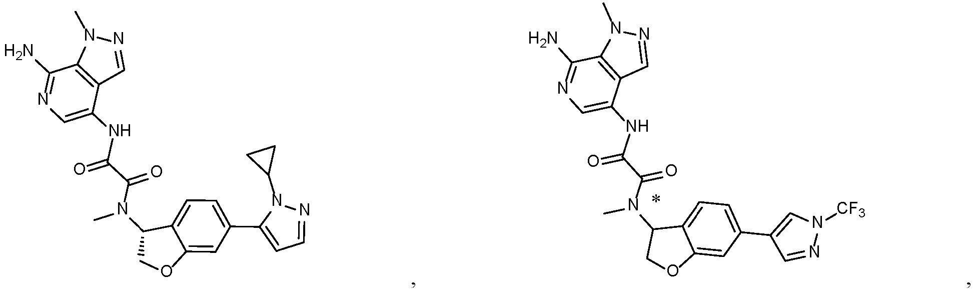 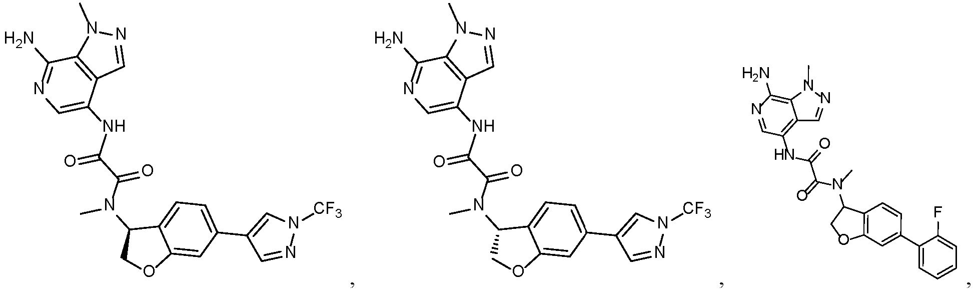 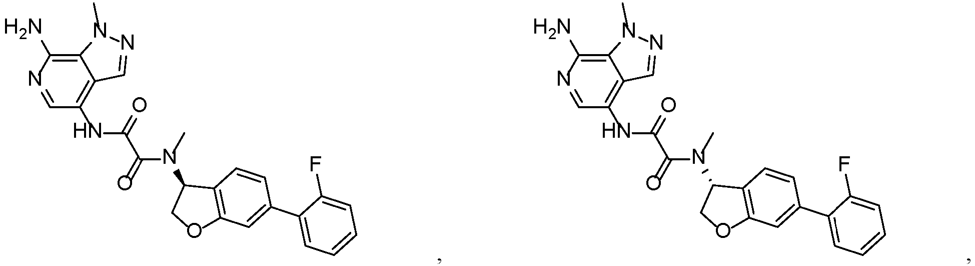 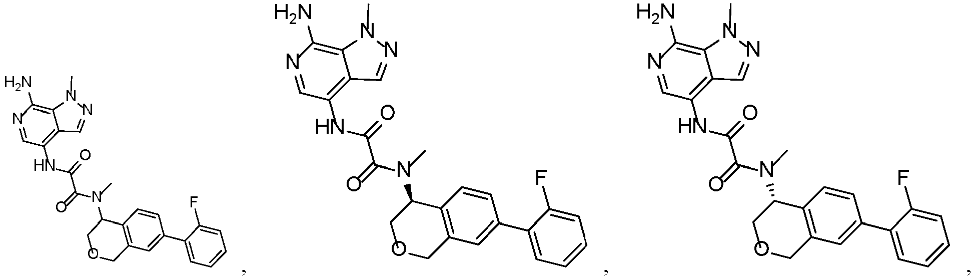 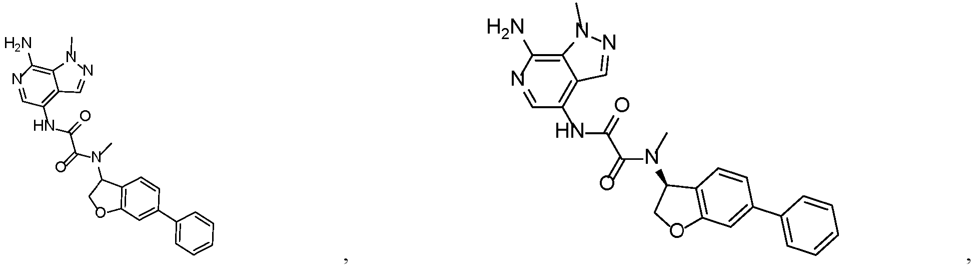 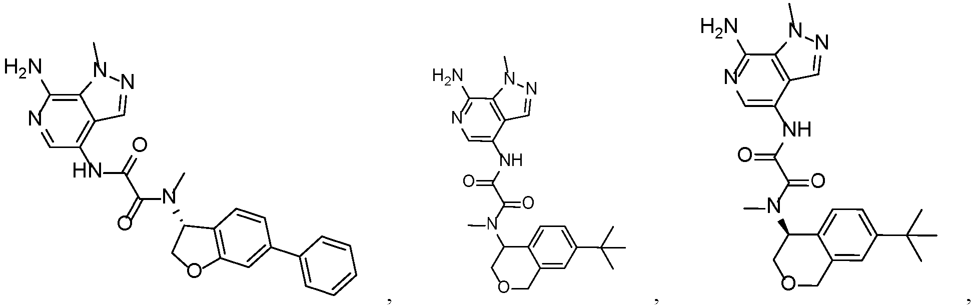 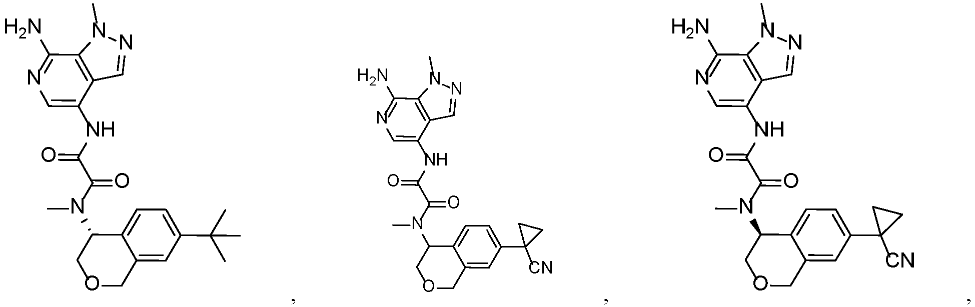 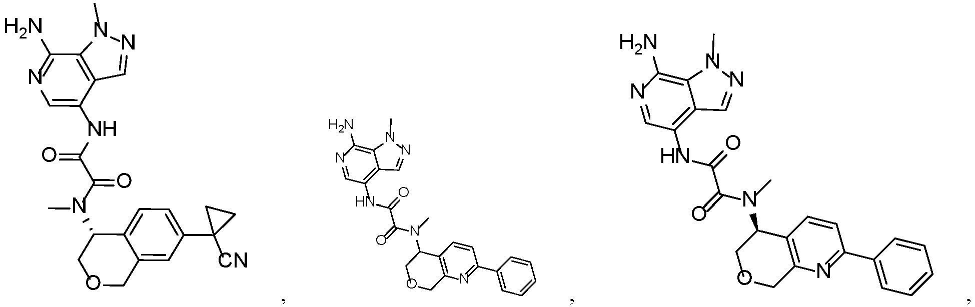 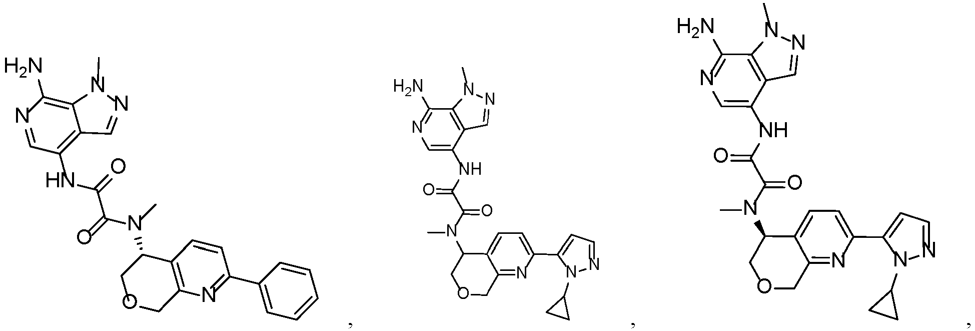 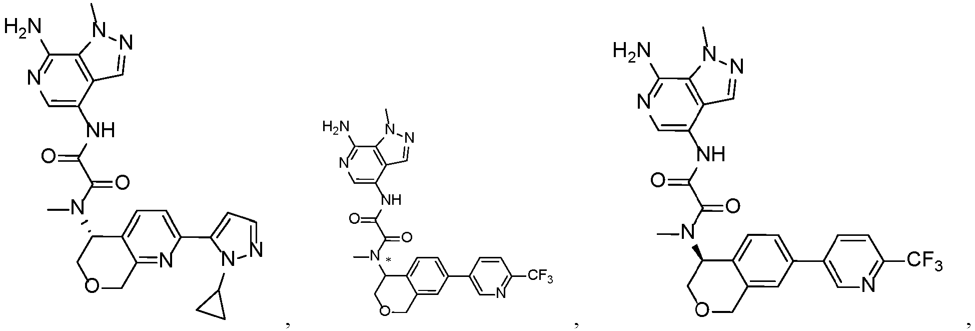 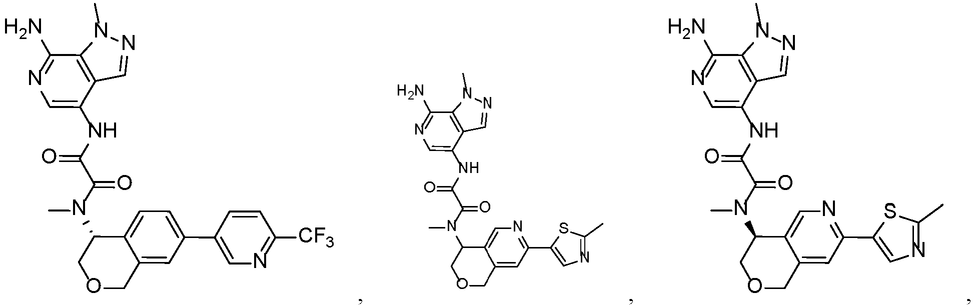 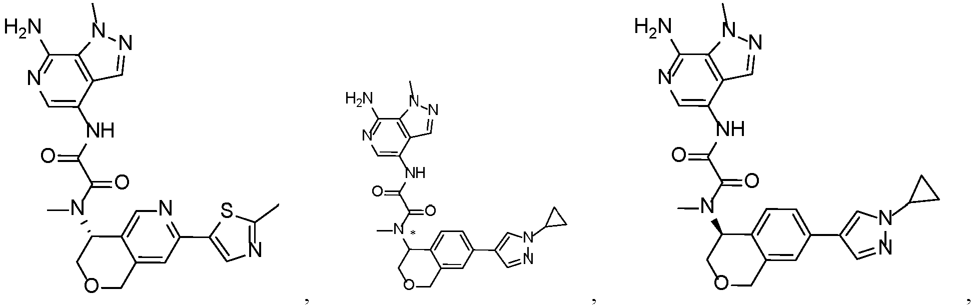 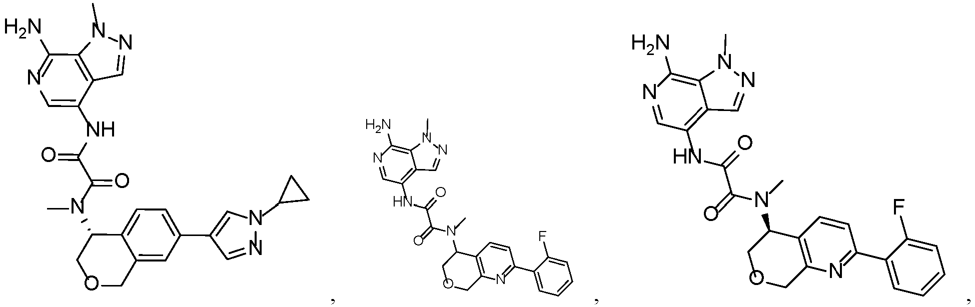 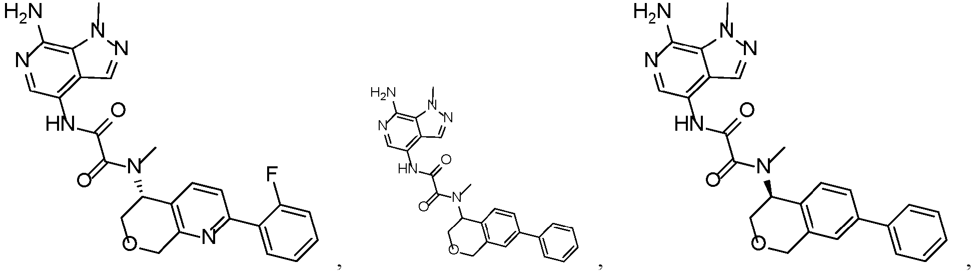 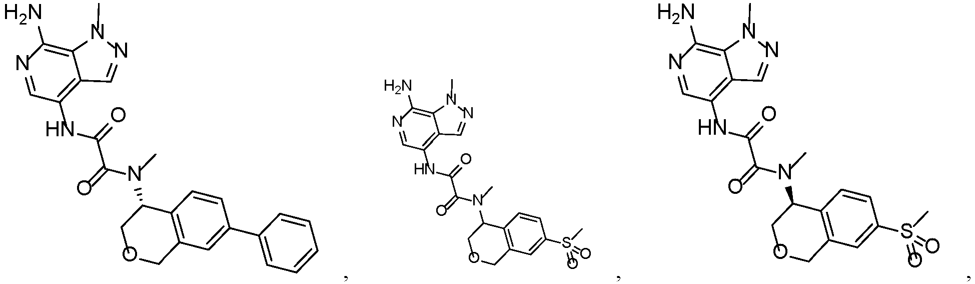 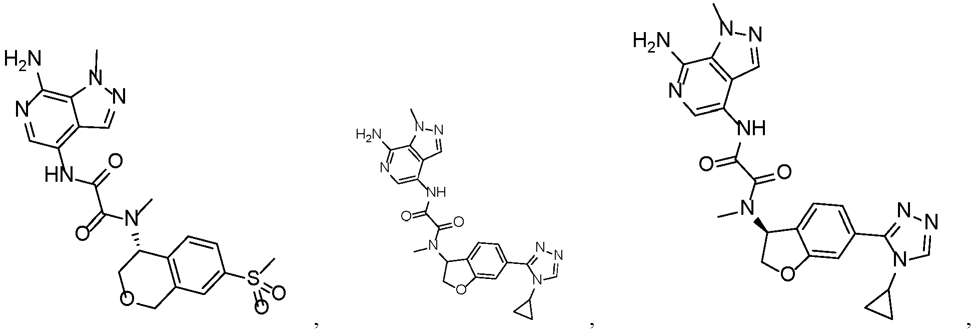 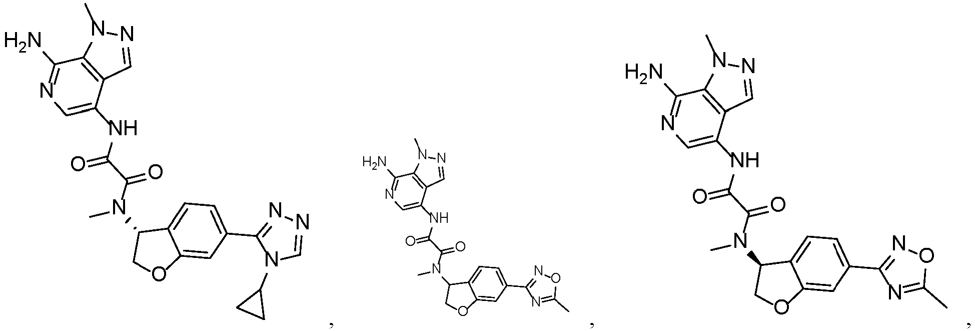 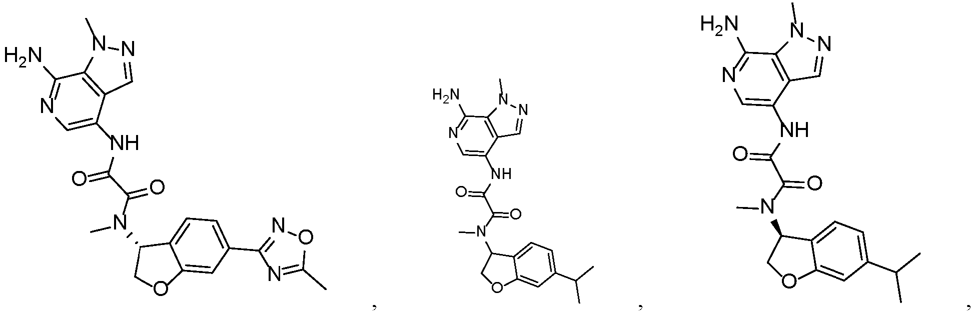 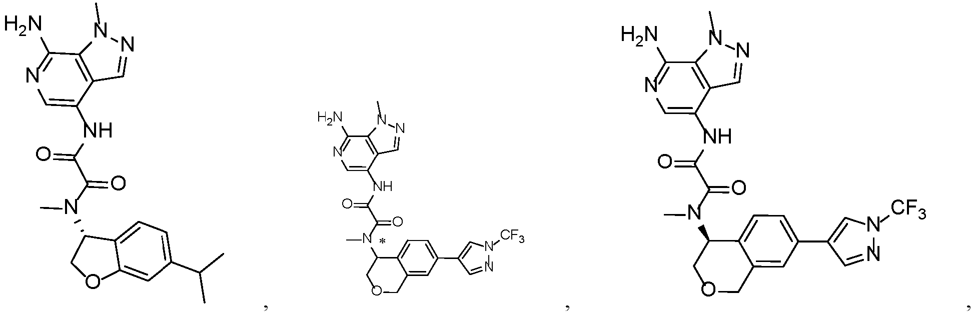 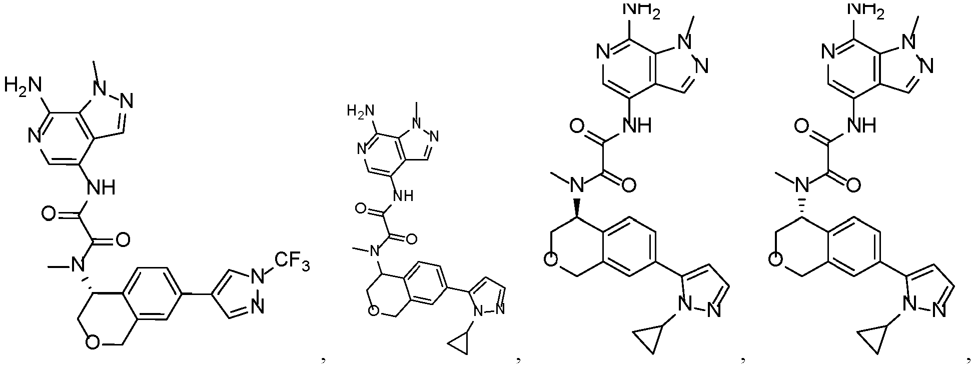 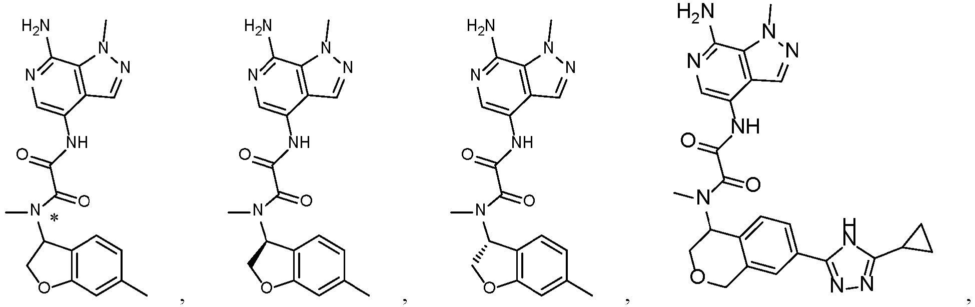 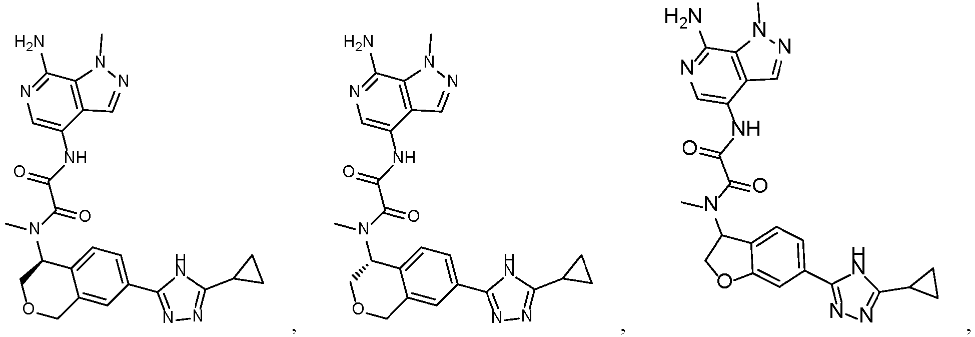 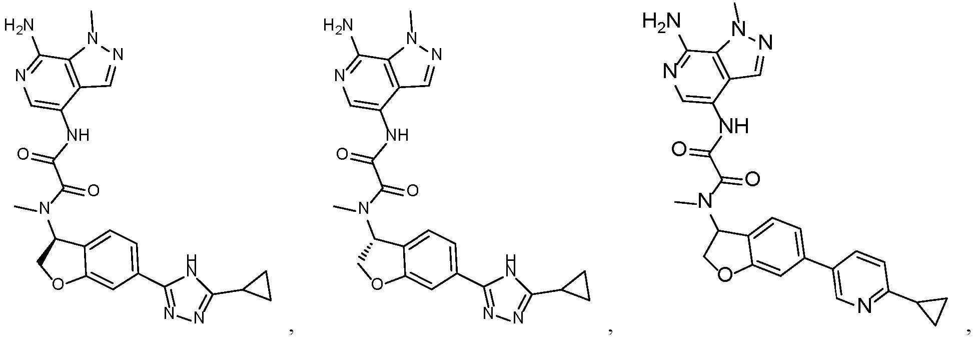 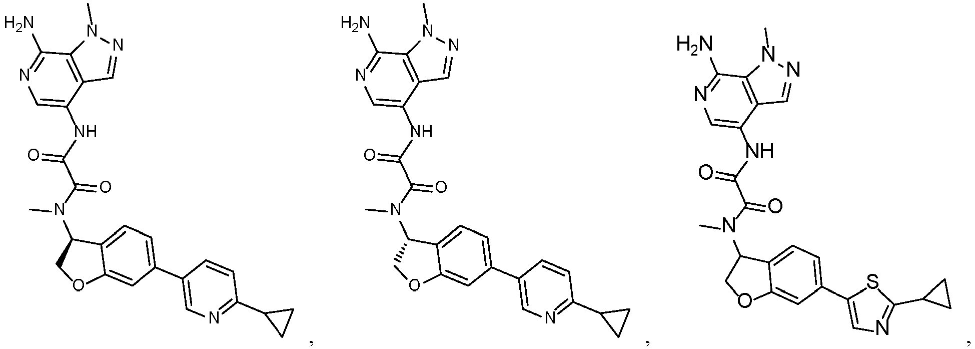 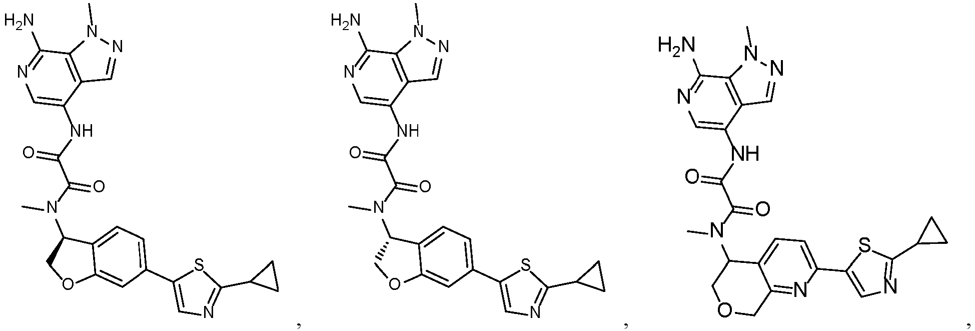 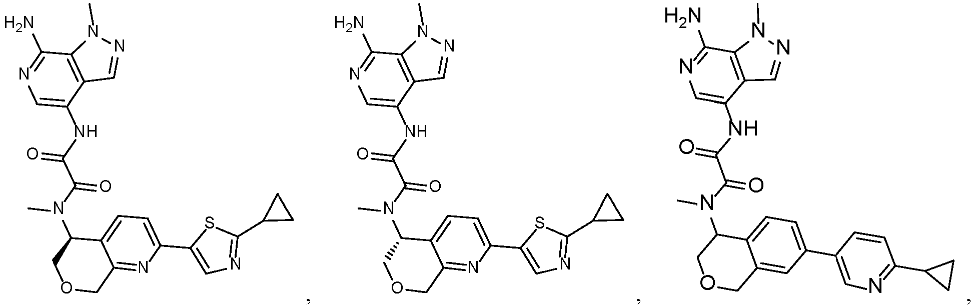 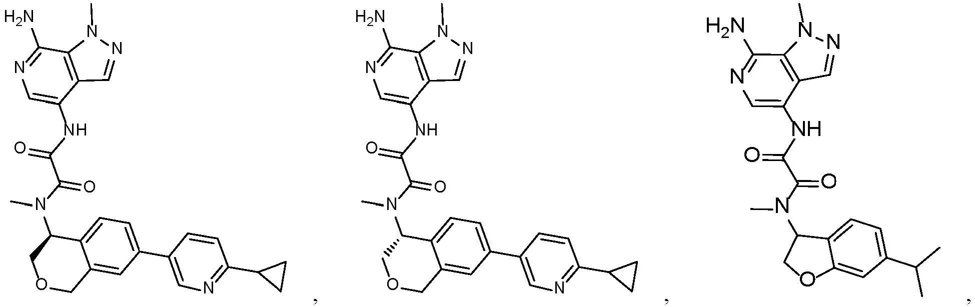 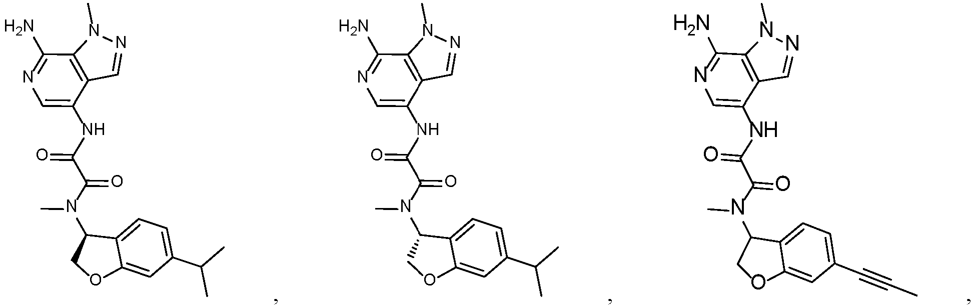 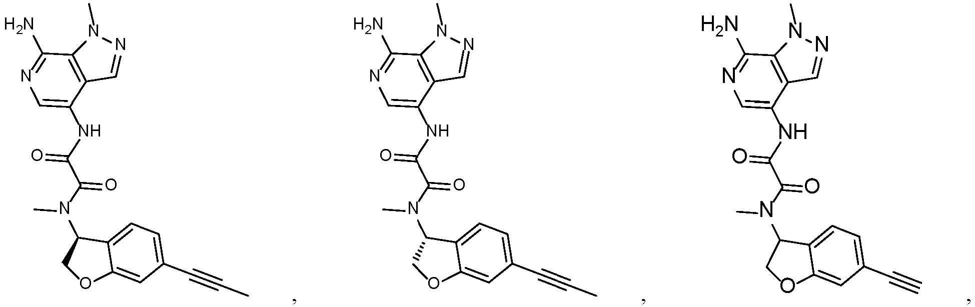 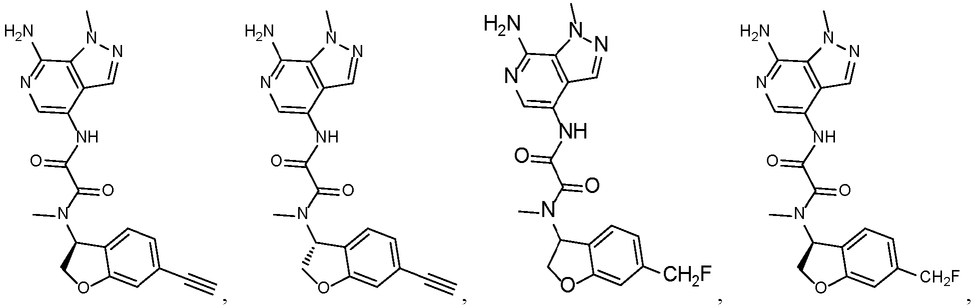 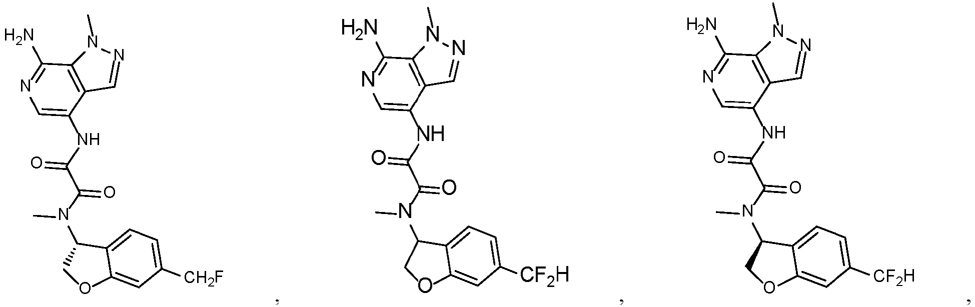 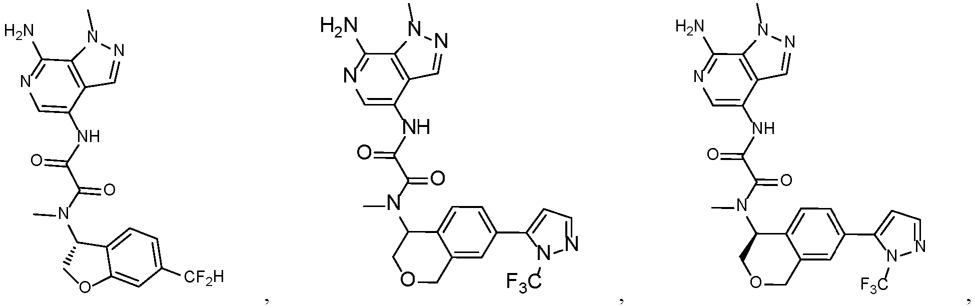 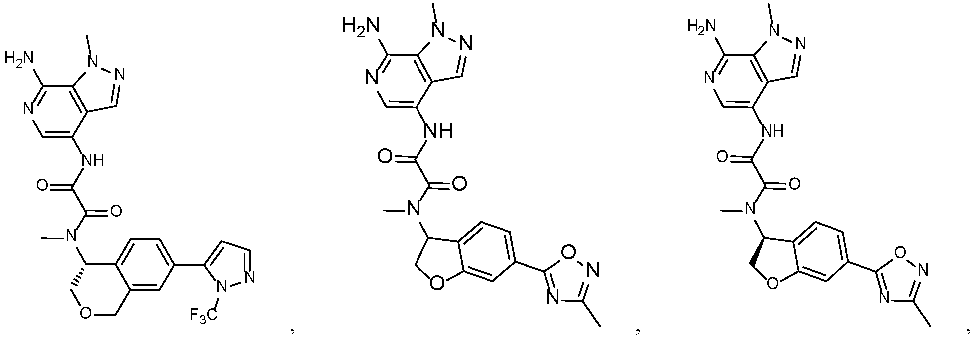 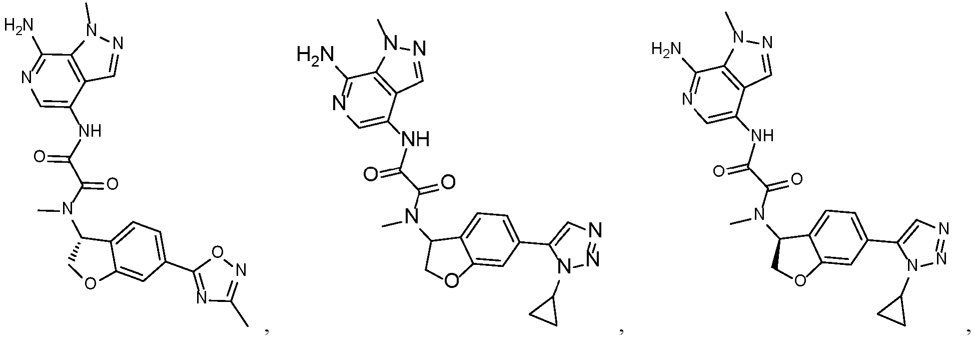 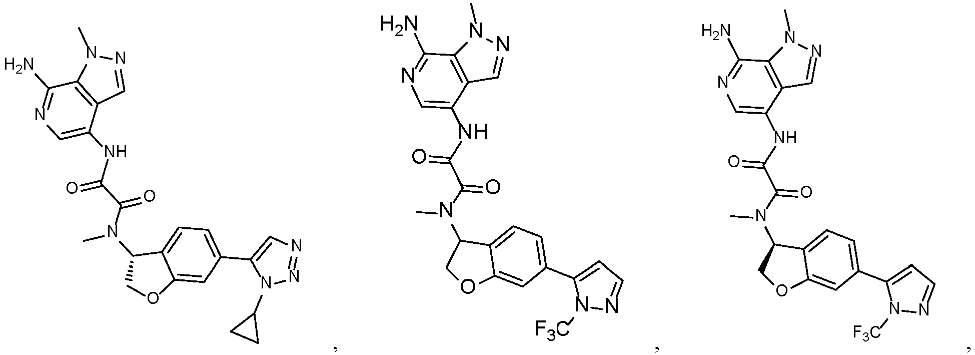 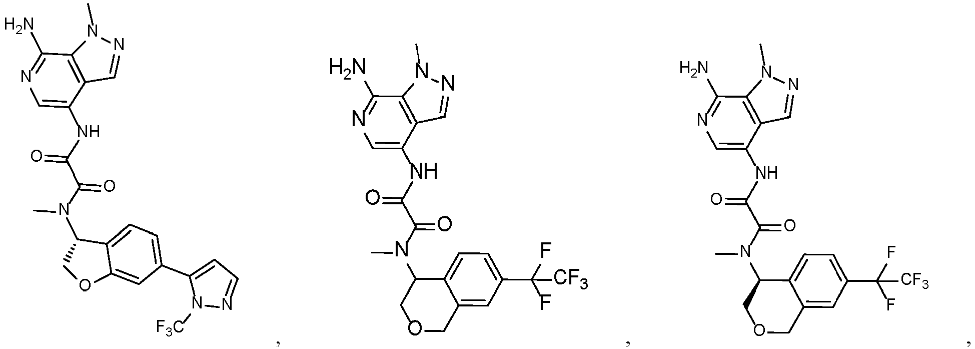 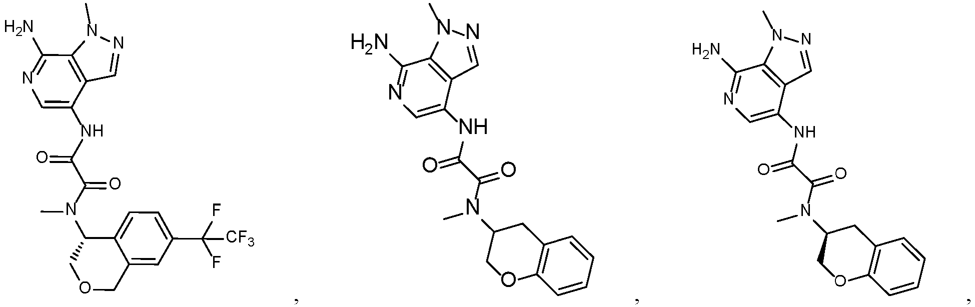 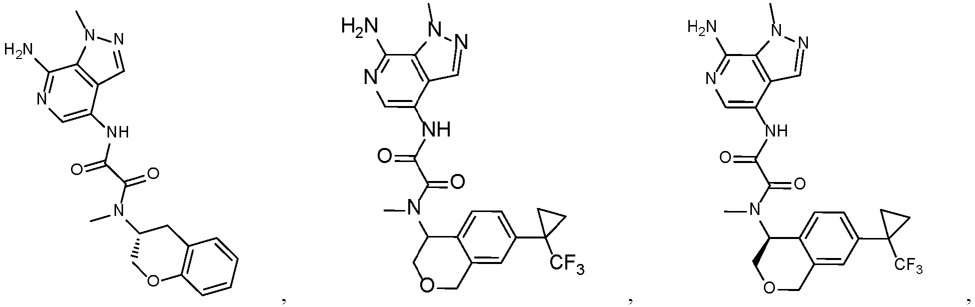 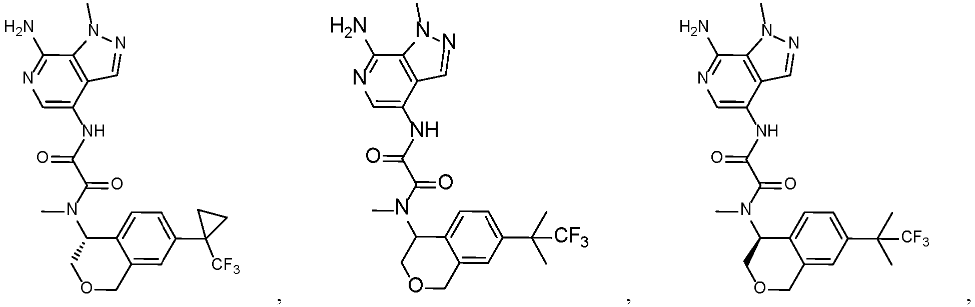 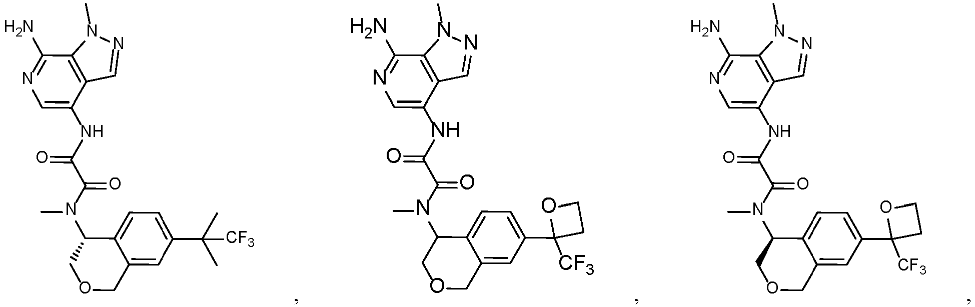 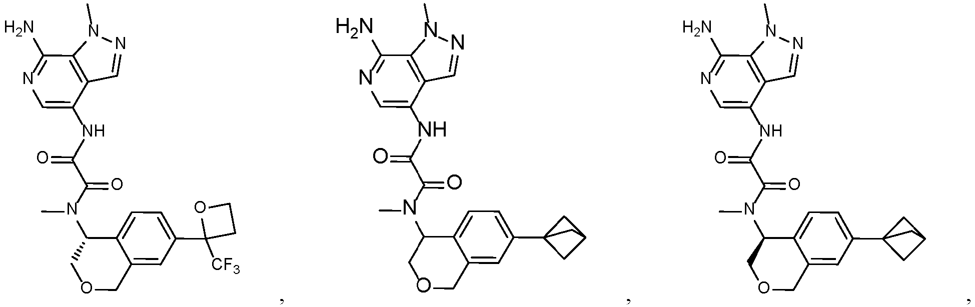 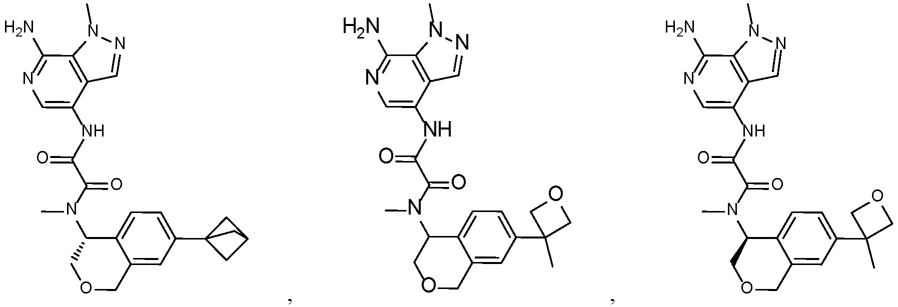 and

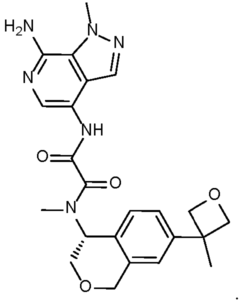

The compounds of the present disclosure may include one or more asymmetric centers, and thus may exist in a variety of stereoisomeric forms, for example, enantiomers and/or diastereomers. For example, the compounds of the present disclosure may be in the form of an individual enantiomer, diastereomer or geometric isomer (e.g., cis- and trans-isomers), or may be in the form of a mixture of stereoisomers, including racemic mixture and a mixture enriched in one or more stereoisomers. The isomers can be separated from the mixture by the methods known to those skilled in the art, including chiral high pressure liquid chromatography (HPLC) and the formation and crystallization of chiral salts; or alternative isomers can be prepared by asymmetric synthesis.

The compounds of the present disclosure may exist in tautomeric forms. Tautomers are functional group isomers produced by the rapid movement of an atom in a molecule between two positions. The tautomers are special functional group isomers. A pair of tautomers can be converted into each other, but usually a relatively stable isomer is the main form of existence. The most important examples are the enol and keto tautomers.

It will be understood by those skilled in the art that the organic compounds can form complexes with solvents in which they are reacted or from which they are precipitated or crystallized. These complexes are known as "solvates." When the solvent is water, the complex is known as "hydrate." The present disclosure encompasses all solvates of the compounds of the present disclosure.

The term "solvate" refers to forms of a compound or a salt thereof, which are associated with a solvent, usually by a solvolysis reaction. This physical association may include hydrogen bonding. Conventional solvents include water, methanol, ethanol, acetic acid, DMSO, THF, diethyl ether, etc. The compounds described herein can be prepared, for example, in crystalline form, and can be solvated. Suitable solvates include pharmaceutically acceptable solvates and further include both stoichiometric solvates and non-stoichiometric solvates. In some cases, the solvates will be capable of isolation, for example, when one or more solvent molecules are incorporated into the crystal lattice of a crystalline solid. "Solvate" includes both solution-phase and isolatable solvates. Representative solvates include hydrates, ethanolates and methanolates.

The term "hydrate" refers to a compound that is associated with water. Generally, the number of water molecules contained in a hydrate of a compound is in a definite ratio to the number of the compound molecules in the hydrate. Therefore, hydrates of a compound can be represented, for example, by a general formula R·x H₂O, wherein R is the compound, and x is a number greater than 0. Given compounds can form more than one type of hydrates, including, for example, monohydrates (x is 1), lower hydrates (x is a number greater than 0 and smaller than 1, for example, hemihydrates (R·0.5 H₂O)) and polyhydrates (x is a number greater than 1, for example, dihydrates (R·2 H₂O) and hexahydrates (R·6 H₂O)).

Compounds of the present disclosure may be in an amorphous or a crystalline form (polymorph). Furthermore, the compounds of the present disclosure may exist in one or more crystalline forms. Therefore, the present disclosure includes all amorphous or crystalline forms of the compounds of the present disclosure within its scope. The term "polymorph" refers to a crystalline form of a compound (or a salt, hydrate or solvate thereof) in a particular crystal packing arrangement. All polymorphs have the same elemental composition. Different crystalline forms generally have different X-ray diffraction patterns, infrared spectra, melting points, density, hardness, crystal shapes, optical and electrical properties, stability, and solubility. Recrystallization solvents, rate of crystallization, storage temperatures, and other factors may cause one crystalline form to dominate. Various polymorphs of a compound can be prepared by crystallization under different conditions.

The present disclosure also comprises compounds that are labeled with isotopes (isotopic variants), which are equivalent to those described in formula (I), but one or more atoms are replaced by atoms having an atom mass or mass number that are different from that of atoms that are common in nature. Examples of isotopes which may be introduced into the compounds of the disclosure include isotopes of hydrogen, carbon, nitrogen, oxygen, phosphorus, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms, prodrugs thereof and pharmaceutically acceptable salts of said compounds or prodrugs all are within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life in vivo or decreasing the dosage requirements, and thus may be alternative in some cases. Isotope-labeled compounds of formula (A) of the present disclosure and prodrugs thereof can be prepared generally by using readily available isotope-labeled reagents to replace non-isotope-labeled reagents in the following schemes and/or the procedures disclosed in the examples and preparation examples.

In addition, prodrugs are also included within the context of the present disclosure. The term "prodrug" as used herein refers to a compound that is converted into an active form that has medical effects in vivo by, for example, hydrolysis in blood. Pharmaceutically acceptable prodrugs are described in T. Higuchi and V. Stella, Prodrugs as Novel Delivery Systems, A.C.S. Symposium Series, Vol. 14, Edward B. Roche, ed., Bioreversible Carriers in Drug Design, American Pharmaceutical Association and Pergamon Press, 1987, and D. Fleisher, S. Ramon and H. Barbra "Improved oral drug delivery: solubility limitations overcome by the use of prodrugs", Advanced Drug Delivery Reviews (1996) 19(2) 115-130, each of which is incorporated herein by reference.

The prodrugs are any covalently bonded compounds of the present disclosure, which release the parent compound in vivo when the prodrug is administered to a patient. Prodrugs are typically prepared by modifying functional groups in such a way that the modifications can be cleaved either by routine manipulation or decompose in vivo to yield the parent compound. Prodrugs include, for example, compounds of the present disclosure wherein the hydroxyl, amino or sulfhydryl groups are bonded to any group that, when administered to a patient, cleaves to form the hydroxyl, amino or sulfhydryl groups. Thus, representative examples of prodrugs include (but are not limited to) the acetate/acetamide, formate/formamide and benzoate/benzamide derivatives of the hydroxyl, sulfhydryl or amino functional groups of the compounds of formula (A). Furthermore, in the case of carboxylic acid (-COOH), esters such as methyl esters and ethyl esters, etc. can be employed. The ester itself may be active in their own and/or hydrolyzable under in vivo conditions in the human body. Suitable pharmaceutically acceptable in vivo hydrolysable ester groups include those groups that can readily break down in the human body to release the parent acids or salts thereof.

The present disclosure also provides a pharmaceutical formulation comprising a therapeutically effective amount of a compound of formula (A), or therapeutically acceptable salts thereof, and pharmaceutically acceptable carriers, diluents or excipients thereof. All of these forms belong to the present disclosure.

### Pharmaceutical compositions and kits

In another aspect, the present disclosure provides a pharmaceutical composition comprising a compound of the present disclosure (also referred to as the "active ingredient") and a pharmaceutically acceptable excipient. In certain embodiments, the pharmaceutical composition comprises an effective amount of the compound of the present disclosure. In certain embodiments, the pharmaceutical composition comprises a therapeutically effective amount of the compound of the present disclosure. In certain embodiments, the pharmaceutical composition comprises a prophylactically effective amount of the compound of the present disclosure.

A pharmaceutically acceptable excipient for use in the present disclosure refers to a non-toxic carrier, adjuvant or vehicle which does not destroy the pharmacological activity of the compound formulated together. Pharmaceutically acceptable carriers, adjuvants, or vehicles that may be used in the compositions of the present disclosure include, but are not limited to, ion exchangers, alumina, aluminum stearate, lecithin, serum proteins (e.g., human serum albumin), buffer substances (such as phosphate), glycine, sorbic acid, potassium sorbate, a mixture of partial glycerides of saturated plant fatty acids, water, salt or electrolyte (such as protamine sulfate), disodium hydrogen phosphate, potassium hydrogen phosphate, sodium chloride, zinc salt, silica gel, magnesium trisilicate, polyvinyl pyrrolidone, cellulose-based materials, polyethylene glycol, sodium carboxymethyl cellulose, polyacrylate, wax, polyethylene-polyoxypropylene block polymers, polyethylene glycol and lanolin.

Suitable formulations for administering the compounds of the present disclosure will be apparent to those of ordinary skill in the art and include, for example, tablets, pills, capsules, suppositories, lozenges, troches, solutions (particularly solutions for injection (subcutaneous, intravenous, intramuscular) and infusion (injection)), elixirs, syrups, cachets, emulsions, inhalants and dispersible powders. The content of one or more pharmaceutically active compounds should range from 0.1 to 90 wt%, alternatively from 0.5 to 50 wt%, of the composition as a whole, i.e., an amount sufficient to achieve the dosage range specified below. If necessary, the specified dosage can be administered several times a day.

The present disclosure also includes kits (e.g., pharmaceutical packs). Kits provided may include a compound disclosed herein, other therapeutic agents, and a first and a second containers (e.g., vials, ampoules, bottles, syringes, and/or dispersible packages or other suitable containers) containing the compound disclosed herein or other therapeutic agents. In some embodiments, kits provided can also optionally include a third container containing a pharmaceutically acceptable excipient for diluting or suspending the compound disclosed herein and/or other therapeutic agent. In some embodiments, the compound disclosed herein provided in the first container and the other therapeutic agents provided in the second container is combined to form a unit dosage form.

### Administration

The pharmaceutical composition provided by the present disclosure can be administered by a variety of routes including, but not limited to, oral administration, parenteral administration, inhalation administration, topical administration, rectal administration, nasal administration, oral administration, vaginal administration, administration by implant or other means of administration. For example, parenteral administration as used herein includes subcutaneous administration, intradermal administration, intravenous administration, intramuscular administration, intra-articular administration, intraarterial administration, intrasynovial administration, intrasternal administration, intracerebroventricular administration, intralesional administration, and intracranial injection or infusion techniques.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the disorder disclosed herein, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, for example, over 3 months, 6 months, 1 year, 2 years, 3 years, 5 years, etc, or may be continued indefinitely, for example, for the rest of the subject's life. In certain embodiments, the chronic administration is intended to provide a constant level of the compound in the blood, e.g., within the therapeutic window over the extended period of time.

The pharmaceutical compostions of the present disclosure may be further delivered using a variety of dosing methods. For example, in certain embodiments, the pharmaceutical composition may be given as a bolus, e.g., in order to raise the concentration of the compound in the blood to an effective level. The placement of the bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or alternatively from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with alternative doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, alternatively from about 0.1 to about 20% by weight, alternatively from about 0.1 to about 10% by weight, and still alternatively from about 0.5 to about 15% by weight.

Injection dose levels range from about 0.1 mg/kg/hour to at least 10 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 2 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as a ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania, which is incorporated herein by reference.

The compounds of the present disclosure can also be administered in sustained release forms or from sustained release drug delivery systems. A description of representative sustained release materials can be found in Remington's Pharmaceutical Sciences.

The present disclosure also relates to the pharmaceutically acceptable formulations of a compound of the present disclosure. In one embodiment, the formulation comprises water. In another embodiment, the formulation comprises a cyclodextrin derivative. The most common cyclodextrins are α-, β- and γ-cyclodextrins consisting of 6, 7 and 8 α-1,4-linked glucose units, respectively, optionally comprising one or more substituents on the linked sugar moieties, which include, but are not limited to, methylated, hydroxyalkylated, acylated, and sulfoalkylether substitution. In certain embodiments, the cyclodextrin is a sulfoalkyl ether β-cyclodextrin, for example, sulfobutyl ether β-cyclodextrin, also known as Captisol. See, e.g., U.S. 5,376,645. In certain embodiments, the formulation comprises hexapropyl-β-cyclodextrin (e.g., 10-50% in water).

### Indications

For MTAP-deficient tumors, the development of MTA-synergistic PRMT5 inhibitors can provide therapeutic benefits for a large number of tumor patients. The compounds of the present disclosure exert therapeutic effects by negatively regulating the activity of MTA-bound PRMT5 in tumor cells, especially in MTAP-deficient cells or various MTAP-related tumor cells.

In some embodiments, the MTA-synergistic PRMT5 inhibitors of the present disclosure can treat a variety of cancers, including but not limited to the tumor types such as malignant peripheral nerve sheath tumor, mesothelioma, glioblastoma multiforme, pancreatic cancer, cholangiocarcinoma, prostate cancer, breast cancer, brain cancer, skin cancer, cervical cancer, bladder cancer, astrocytoma, colorectal cancer, endometrial cancer, esophageal cancer, gastric cancer, thymoma, head and neck cancer, hepatocellular carcinoma, laryngeal cancer, squamous cell carcinoma of the lung, lung adenocarcinoma, oral cancer, ovarian cancer, kidney cancer, and thyroid cancer and sarcoma.

More specifically, these compounds are useful in treating: heart: sarcoma (angiosarcoma, fibrosarcoma, rhabdomyosarcoma, liposarcoma, etc.), myxoma, rhabdomyoma, fibroma, lipoma, and teratoma; lung: bronchogenic carcinoma (squamous cell carcinoma, small cell undifferentiated carcinoma, large cell undifferentiated carcinoma, adenocarcinoma, etc.), alveolar (bronchiolar) carcinoma, bronchial adenoma, sarcoma, lymphoma, chondroma, hamartoma, mesothelioma; gastrointestinal tract: esophagus (squamous cell carcinoma, adenocarcinoma, leiomyosarcoma, lymphoma, etc.), stomach (cancer, lymphoma, leiomyosarcoma, etc.), pancreas (ductal adenocarcinoma, insulinoma, glucagonoma, gastrinoma, carcinoid tumor, hemangioma, etc.), small intestine (adenocarcinoma, lymphoma, carcinoid tumor, Kaposi's sarcoma, leiomyoma, hemangioma, lipoma, neurofibroma, fibroma, etc.), large intestine (adenocarcinoma, tubular adenoma, villous adenoma, hamartoma, leiomyoma, etc.); genitourinary tract: kidney (adenocarcinoma, nephroblastoma, etc.), bladder and urethra (squamous cell carcinoma, transitional cell carcinoma, adenocarcinoma, etc.), prostate (adenocarcinoma, sarcoma, etc.), testicle (seminoma, teratoma, embryonal carcinoma, teratocarcinoma, choriocarcinoma, sarcoma, stromal cell carcinoma, fibroma, fibroadenoma, adenomatoid tumor, lipoma, etc.); liver: hepatoma, cholangiocarcinoma, hepatoblastoma, angiosarcoma, hepatocellular adenoma, hemangioma; biliary tract: gallbladder cancer, ampullary cancer, cholangiocarcinoma, etc.; bone: osteosarcoma, fibrosarcoma, malignant fibrous histiocytoma, chondrosarcoma, Ewing sarcoma, malignant lymphoma (reticulum cell sarcoma, etc.), multiple myeloma, malignant giant cell tumor, chordoma, osteochondroma (osteocartilaginous exostosis), benign enchondroma, chondroblastoma, chondromyxoid fibroma, osteoid osteoma, and giant cell tumor of bone; nervous system: skull (osteoma, hemangioma, granuloma, xanthoma, osteitis deformans, etc.), meninges (meningioma, meningeal sarcoma, glioma, etc.), brain (astrocytoma, medulloblastoma, glioma, ependymoma, germinoma (pineal tumor, etc.), glioblastoma multiforme, oligodendroglioma, schwannoma, retinoblastoma, congenital tumor, etc.), spinal neurofibroma, meningioma, glioma, sarcoma, etc.; gynaecology: uterus (endometrial cancer, etc.), cervix (cervical cancer, preneoplastic cervical dysplasia, etc.), ovary (ovarian cancer, serous cystadenocarcinoma, mucinous cystadenocarcinoma, unclassified cancer, etc.), granulosa cell tumor, Sertoli-Leydig cell tumor, dysgerminoma, malignant teratoma, etc.), vulva (squamous cell carcinoma, intraepithelial carcinoma, adenocarcinoma, fibrosarcoma, melanoma, etc.), vagina (clear cell carcinoma, squamous cell carcinoma, sarcoma botryoides (embryonal rhabdomyosarcoma, etc.), fallopian tube cancer, etc.; hematology: blood (myeloid leukemia (acute and chronic), acute lymphoblastic leukemia, chronic lymphocytic leukemia, diffuse large B-cell lymphoma, mantle cell lymphoma (MCL), follicular lymphoma, myeloproliferative disorder, multiple myeloma, myelodysplastic syndrome, etc.), Hodgkin disease, non-Hodgkin lymphoma (malignant lymphoma), etc.; skin: malignant melanoma, basal cell carcinoma, squamous cell carcinoma, Kaposi sarcoma, dysplastic nevus, lipoma, hemangioma, dermatofibroma, keloma, psoriasis, etc.; adrenal gland: neuroblastoma, etc.

### Combination therapy

The MTA-synergistic PRMT5 inhibitors of the present disclosure may be combined with other drugs to treat cancers, wherein other drugs include at least one of targeted drugs/cell activity regulators, including CDK4/6 inhibitors, MAT2A inhibitors, MAPK1/MAPK3 inhibitors, Type I PRMT inhibitors, EGFR inhibitors, SHP2 inhibitors, pan-KRAS inhibitors, KRASG12C inhibitors, RAF inhibitors, MEK inhibitors, ERK inhibitors, Bcl-2 inhibitors, SOS1 inhibitors, PARP inhibitors, MALT1 inhibitors, MALT2 inhibitors, BTK inhibitors, PI3K inhibitors, AKT inhibitors, FGFR inhibitors, DNA methyltransferase (DNMT) inhibitors, EZH1/2 inhibitors, EZH2 inhibitors, Menin-MLL inhibitors, IDH1 inhibitors, IDH2 inhibitors, IDH1/2 inhibitors, chemotherapy drugs, radiotherapy, STING agonists and immune checkpoint inhibitors/regulators, etc.

### Examples

The materials or reagents used herein are commercially available or are prepared by synthetic methods commonly known in the art.

### 1. Synthesis of example 1

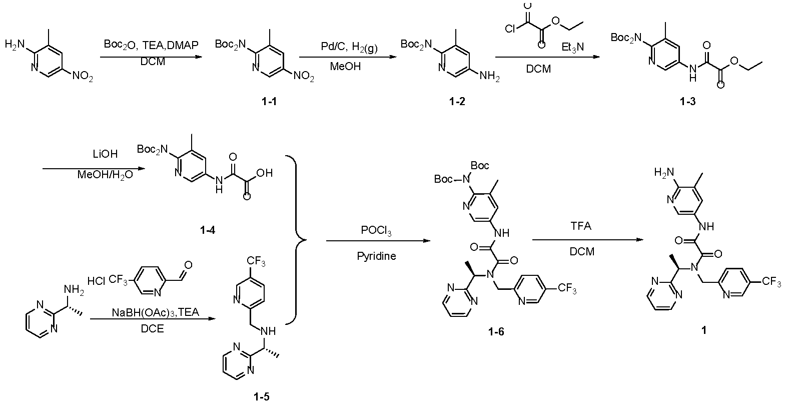

### 1.1 Synthesis of intermediate 1-1

Di-tert-butyl dicarbonate (18 g), triethylamine (13 g), and 4-dimethylaminopyridine (0.8 g) were added to a solution of 2-amino-3-methyl-5-nitropyridine (5 g) in dichloromethane (50 mL), and the mixture was stirred at room temperature for 3 hours. The mixture was extracted with ethyl acetate (100 * 3 mL). The organic phase was washed with saturated sodium chloride solution (100 mL), dried over anhydrous sodium sulfate and filtered. The filtrate was concentrated under reduced pressure. The residue was purified by column chromatography (silica, petroleum ether/ethyl acetate = 5/1) to give a white solid **1-1** (10 g, yield 82%). LC-MS: M+Na⁺ = 376.1.

### 1.2 Synthesis of intermediate 1-2

10% palladium on carbon (2 g) was added to a solution of compound **1-1** (10 g) in methanol (100 mL), and the mixture was stirred at room temperature for 2 hours under hydrogen. The mixture was filtered, and the filtrate was concentrated under reduced pressure to give a light yellow solid **1-2** (9.2 g). LC-MS: M+H⁺ = 324.2.

### 1.3 Synthesis of intermediate 1-3

Ethyl 2-chloro-2-oxoacetate (830 mg) and triethylamine (620 mg) were added to a solution of **1-2** (2 g) in dichloromethane (20 mL) at 0 °C under nitrogen. The mixture was stirred at room temperature for 2 hours. Water (20 mL) was added, and the mixture was extracted with dichloromethane (10 * 3 mL). The organic phase was washed with saturated sodium chloride solution (20 mL), dried over anhydrous sodium sulfate, and filtered. The filtrate was concentrated under reduced pressure. The residue was purified by column chromatography (silica, petroleum ether/ethyl acetate = 2/1) to give a white solid **1-3** (2.5 g, yield 80%). LC-MS: M+H⁺ = 424.0.

### 1.4 Synthesis of intermediate 1-4

Lithium hydroxide (240 mg) was added to a solution of **1-3** (3 g) in methanol (10 mL) and water (10 mL), and the mixture was stirred at room temperature for 2 hours. The mixture was concentrated under reduced pressure, and diluted with added water (20 mL). The pH was adjusted to 6 with 1N hydrochloric acid solution, and the mixture was filtered. The filter cake was dried to give a white solid **1-4** (1 g). LC-MS: M+H⁺ = 396.2.

### 1.5 Synthesis of intermediate 1-5

Triethylamine (1080 mg) was added to a solution of (R)-1-(pyrimidin-2-yl)ethanamine hydrochloride (2 g) in 1,2-dichloroethane (100 mL). The mixture was stirred at room temperature for 1 hour. 5-(trifluoromethyl)pyridine-2-carboxaldehyde (1.8 g) was added, and the mixture was stirred at room temperature for 2 hours. Sodium triacetoxyborohydride (3.2 g) was added, and the mixture was stirred at room temperature for 1 hour. Dichloromethane (50 mL) was added, and the mixture was washed with saturated aqueous solution of sodium bicarbonate (50 mL), water (50 mL) and saturated brine (50 mL) successively. The organic phase was dried over anhydrous sodium sulfate and filtered. The filtrate was concentrated under reduced pressure. The residue was purified by column chromatography (silica, dichloromethane/methanol = 100/0 - 100/3) to give a light yellow oil **1-5** (1.8 g, yield 64%). LC-MS: M+H⁺ = 283.1.

### 1.6 Synthesis of intermediate 1-6

Phosphorus oxychloride (38 mg) was added dropwise to a solution of **1-4** (50 mg) and **1-5** (36 mg) in pyridine (2 mL) at 0 °C under nitrogen. The mixture was stirred at 0 °C for 1 hour. The mixture was concentrated under reduced pressure, and an aqueous solution of sodium bicarbonate (10 mL) and water (20 mL) were added to the residue. The mixture was extracted with ethyl acetate (10 * 3 mL). The organic phase was washed with saturated sodium chloride solution (10 mL), dried over anhydrous sodium sulfate and filtered. The filtrate was concentrated under reduced pressure. The residue was purified by column chromatography (silica, dichloromethane/methanol = 10/1) to give a yellow solid **1-6** (20 mg, yield 32%). LC-MS: M+H⁺ = 660.0.

### 1.7 Synthesis of compound 1

Trifluoroacetic acid (2 mL) was added to a solution of **1-6** (20 mg) in dichloromethane (6 mL), and the mixture was reacted at room temperature for 1 hour. The mixture was concentrated under reduced pressure, and the residue was purified by high performance liquid chromatography (column: Gemini 5u C18 150 x 21.2 mm; mobile phase: [H₂O(NH₃)-ACN]; B%: 2%-10%, 6 min) to give a white solid **1** (5 mg, yield 20 %). LC-MS: M+H⁺ = 460.2. ¹H NMR (400 MHz, DMSO-*d₆*) δ ppm 10.52 - 10.35 (m, 1H), 8.82 - 8.73 (m, 2H), 8.14 - 7.32 (m, 6H), 5.73 - 5.60 (m, 3H), 5.20 - 4.57 (m, 2H), 2.03 - 1.96 (m, 3H), 1.66 - 1.52 (m, 3H).

### 2. Synthesis of example 2

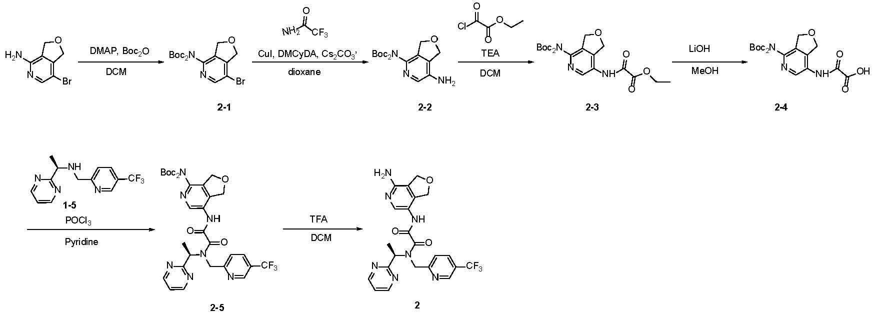

### 2.1 Synthesis of intermediate 2-1

Di-tert-butyl dicarbonate (565 mg) was added to a solution of 4-dimethylaminopyridine (6.8 mg), triethylamine (112.9 mg) and 7-bromo-1,3-dihydrofuro[3,4-c]pyridin-4-amine (120 mg) in dichloromethane (5 mL). The mixture was stirred at room temperature for 2 hours. The organic phase was washed with saturated sodium chloride solution (1 mL), dried over anhydrous sodium sulfate and filtered. The filtrate was concentrated under reduced pressure. The residue was purified by column chromatography (silica, petroleum ether/ethyl acetate = *5*/*1* - 2/1) to give a white solid **2-1** (160 mg, yield 65.6%). LC-MS: M+Na⁺ = 437.1, 439.1.

### 2.2 Synthesis of compound 2-2

Copper(I) iodide (18.1 mg), cesium carbonate (200 mg), and trans-(1R,2R)-N,N'-dimethyl-1,2-cyclohexanediamine (110.2 mg) were added to a solution of trifluoroacetamide (71.7 mg) and **2-1** (100 mg) in dioxane (5 mL). The mixture was stirred at 110 °C for 16 hours under nitrogen, and cooled to room temperature. Water (10 mL) was added, and the mixture was extracted with ethyl acetate (10 mL*3). The organic phase was washed with saturated sodium chloride solution (5 mL), dried over anhydrous sodium sulfate and filtered. The filtrate was concentrated under reduced pressure. The residue was purified by column chromatography (silica, dichloromethane/ethyl acetate = *5*/*1* - 1/1) to give a white solid **2-2** (34 mg, yield 38.7%). LC-MS: M+H⁺ = 352.0.

### 2.3 Synthesis of compound 2-3

Ethyl 2-chloro-2-oxoacetate (139.8 mg) was added to a solution of triethylamine (121.2 mg) and **2-2** (240 mg) in dichloromethane (5 mL) in an ice bath. The mixture was stirred at room temperature for 1 hour. The organic phase was washed with saturated sodium chloride solution (2 mL), dried over anhydrous sodium sulfate and filtered. The filtrate was concentrated under reduced pressure. The residue was purified by column chromatography (silica, petroleum ether/ethyl acetate = *5*/*1* - 2/1) to give a white solid **2-3** (230 mg, yield 74.6%). LC-MS: M+H⁺ = 451.9.

### 2.4 Synthesis of compound 2-4

Lithium hydroxide (38.2 mg) was added to a solution of **2-3** (230 mg) in methanol (5 mL) and water (1 mL). The mixture was stirred at room temperature for 1 hour, and the pH was adjusted to 6 with 1N dilute hydrochloric acid. The mixture was extracted with ethyl acetate (10 mL*3). The organic phase was washed with saturated sodium chloride solution (5 mL), dried over anhydrous sodium sulfate and filtered. The filtrate was concentrated under reduced pressure and freeze-dried to give a white solid **2-4** (170 mg, yield 71.9%). LC-MS: M+H⁺ = 424.2.

### 2.5 Synthesis of compound 2-5

Phosphorus oxychloride (36.2 mg) was added to a solution of **1-5** (40 mg) and **2-4** (50 mg) in pyridine (2 mL) in an ice bath. The mixture was stirred at room temperature for 1 hour. Saturated aqueous solution of sodium bicarbonate (1 mL) was added, and the mixture was extracted with ethyl acetate (5 mL*3). The organic phase was washed with saturated sodium chloride solution (1 mL), dried over anhydrous sodium sulfate and filtered. The filtrate was concentrated under reduced pressure. The residue was purified by column chromatography (silica, petroleum ether/ethyl acetate = 3/1 - 1/1) to give a brown solid **2-5** (22 mg, yield 30.6%). LC-MS: M+H⁺ = 688.2.

### 2.6 Synthesis of compound 2

Trifluoroacetic acid (0.5 mL) was added to a solution of **2-5** (22 mg) in dichloromethane (1.5 mL) in an ice bath. The mixture was stirred at room temperature for 1 hour, and concentrated under reduced pressure. The residue was purified by high performance liquid chromatography (column: Xbridge-C18 150 x 19 mm, Sum; mobile phase: ACN--H₂O (0.05%NH₃); B%: 5%-10%, 20 min) to give a white solid 2 (3.6 mg, yield 23.2%). LC-MS: M+H⁺ = 488.0. ¹H NMR (400 MHz, CD₃OD) δ ppm 8.69 - 8.62 (m, 2H), 8.59 (d, *J* = 4.8 Hz, 1H), 7.91 (d, *J* = 8.4 Hz, 1H), 7.79 - 7.63 (m, 1H), 7.52 - 7.39 (m, 1H), 7.24 -7.15 (m, 1H), 5.78 - 5.66 (m, 1H), 5.23 - 5.04 (m, 1H), 4.94 - 4.92 (m, 1H), 4.86 - 4.79 (m, 3H), 4.71 - 4.63 (m, 1H), 1.64-1.49 (m, 3H).

### 3. Synthesis of example 3

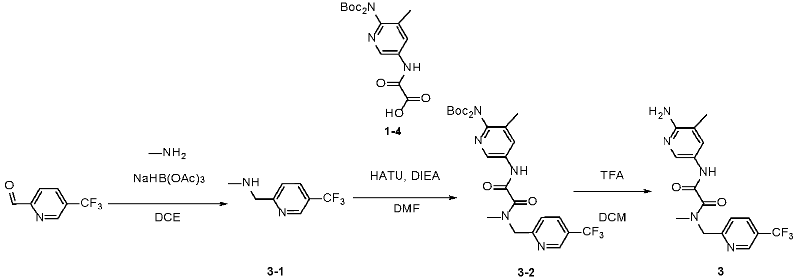

### 3.1 Synthesis of intermediate 3-1

5-(Trifluoromethyl)pyridine-2-carboxaldehyde (200.0 mg) and acetic acid (102.0 mg) were added to a solution of methylamine (70.9 mg) in 1,2-dichloroethane (5 mL), and the mixture was stirred at room temperature for 2 hours. Sodium triacetoxyborohydride (484.2 mg) was added, and the mixture was stirred at room temperature for 0.5 hours. Saturated sodium carbonate solution (10 mL) was added to quench the reaction, and the mixture was extracted with dichloromethane (15 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a white solid **3-1** (150.0 mg, yield 72.0%). LC-MS m/z (ESI): 191.1 [M+H]⁺.

### 3.2 Synthesis of intermediate 3-2

O-(7-azabenzotriazol-1-yl)-N,N,N',N'-tetramethyluronium hexafluorophosphate (120.2 mg) and N,N-diisopropylethylamine (40.8 mg) were added to a solution of **1-4** (62.2 mg) and **3-1** (36.0 mg) in N,N-dimethylformamide (5 mL), and the mixture was stirred at room temperature for 5 hours. Water (10 mL) was added, and the mixture was extracted with dichloromethane (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1) to give a black oil **3-2** (21.0 mg, yield 22.3%). LC-MS m/z (ESI): 568.0 [M+H]⁺.

### 3.3 Synthesis of compound 3

Trifluoroacetic acid (2 mL) was added to a solution of **3-2** (21.0 mg) in dichloromethane (2 mL) in an ice bath. The mixture was stirred at room temperature for 1 hour, and concentrated under reduced pressure. The residue was purified by high performance liquid chromatography (column: Xbridge-C18 150 × 19 mm, Sum; mobile phase: ACN--H₂O (0.1% FA); B%: 5%-10%, 20 min) to give the formate of **3** (2.3 mg, yield 16.9 %) as a white solid. LC-MS m/z (ESI): 368.1 [M+H]⁺. ¹H NMR (400 MHz, DMSO-*d₆*) δ 8.86 (s, 1H), 8.14 - 8.01 (m, 2H), 7.62 - 7.55 (m, 2H), 4.84 (s, 1H), 4.59 (s, 1H), 3.28 - 3.05 (m, 3H), 2.15 - 2.11 (m, 3H).

### 4. Synthesis of example 4

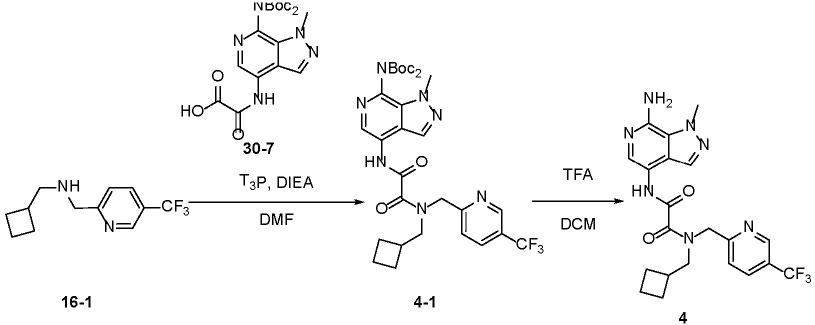

### 4.1 Synthesis of intermediate 4-1

**30-7** (30.0 mg), propylphosphonic anhydride (45.0 mg), and N,N-diisopropylethylamine (15.0 mg) were added to a solution of **16-1** (35.0 mg) in N,N-dimethylformamide (1 mL). The mixture was stirred at room temperature for 12 hours. Water (5 mL) was added to the reaction solution, and the mixture was extracted with ethyl acetate (5 mL × 3). The organic phase was washed with saturated sodium chloride solution (1 mL), dried over anhydrous sodium sulfate and filtered. The filtrate was concentrated under reduced pressure. The residue was purified by silica gel column chromatography (eluent: petroleum ether/ethyl acetate = 2/1 - 1/1) to give a light yellow solid **4-1** (30.0 mg, yield 65.8%). LC-MS m/z (ESI): 662.3 [M+H]⁺.

### 4.2 Synthesis of compound 4

Trifluoroacetic acid (0.5 mL) was added to a solution of **4-1** (30.0 mg)in dichloromethane (1 mL) in an ice bath. The mixture was stirred at room temperature for 2 hours, and the pH was adjusted to 8 with saturated sodium carbonate solution. The mixture was extracted with dichloromethane (5 mL × 3), and the organic phase was concentrated under reduced pressure. The residue was purified by high performance liquid chromatography (column: Xbridge-C18 150 x 19 mm, 5um; mobile phase: ACN--H₂O (0.05% NH₃); B%: 5%-10%, 20 min) to give a white solid 4 (6.40 mg, yield 30.6%). LC-MS m/z (ESI): 462.1 [M+H]⁺. ¹H NMR (400 MHz, DMSO-*d₆*) δ ppm 10.68 (s, 1H), 8.96 - 8.91 (m, 1H), 8.29 - 8.20 (m, 1H), 7.92 -7.84 (m, 1H), 7.66 (d, *J =* 4Hz, 1H), 7.53 (d, *J =* 8Hz, 1H), 6.24 - 6.18 (m, 2H), 4.91 - 4.75 (m, 2H), 4.28 - 4.23 (m, 3H), 3.57 - 3.45 (m, 2H), 2.67 - 2.57 (m, 1H), 1.97 - 1.84 (m, 2H), 1.83 - 1.66 (m, 4H).

### 5. Synthesis of example 5

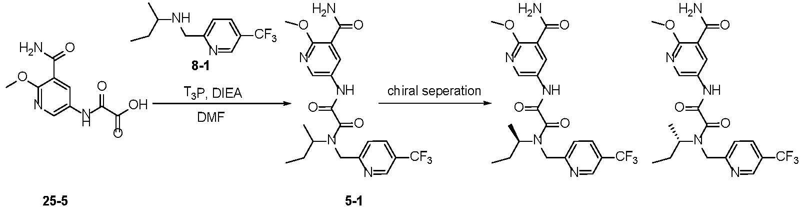

### 5.1 Synthesis of intermediate 5-1

Propylphosphonic anhydride (260.0 mg) and N,N-diisopropylethylamine (84.1 mg) were added to a solution of **25-5** (100.0 mg) and **8-1** (77.0 mg) in N,N-dimethylformamide (3 mL) in an ice bath. The mixture was stirred at room temperature for 1 hour, and saturated ammonium chloride solution (10 mL) was added to the reaction solution. The mixture was extracted with ethyl acetate (10 mL × 3). The organic phase was washed with saturated sodium chloride solution (5 mL), dried over anhydrous sodium sulfate and filtered. The filtrate was concentrated under reduced pressure. The residue was purified by high performance liquid chromatography (column: Xbridge-C18 150 × 19 mm, 5um; mobile phase: ACN--H₂O (0.1% FA); B%: 5%-60%, 20 min) to give a white solid **5-1** (20.8 mg, yield 10.8%). LC-MS m/z (ESI): 454.0 [M+H]⁺.

### 5.2 Synthesis of compound 5

Compound **5-1** (20.8 mg) was purified by SFC (column: CHIRALPAK AD-H 250 × 20 mm, Sum; mobile phase: 40% IPA (0.2% NH₄OH)) to give a white solid **5-P1** (5.8 mg, yield 36.8%). LC-MS m/z (ESI): 454.1 [M+H]⁺. ¹H NMR (400 MHz, DMSO-*d*₆) δ ppm 11.04 - 10.79 (m, 1H), 8.92 - 8.83 (m, 1H), 8.58 - 8.49 (m, 1H), 8.40 - 8.37 (m, 1H), 8.24 - 8.14 (m, 1H), 7.78 - 7.69 (m, 2H), 7.59 - 7.57 (m, 1H), 4.75 - 4.58 (m, 2H), 3.99 - 3.92 (m, 4H), 1.67 - 1.43 (m, 2H), 1.19 - 1.03 (m, 3H), 0.85 - 0.78 (m, 3H);

A white solid **5-P2** (4.5 mg, yield 42.1%) was obtained. LC-MS m/z (ESI): 454.1 [M+H]⁺. ¹H NMR (400 MHz, DMSO-*d*₆) δ ppm 11.04 - 10.80 (m, 1H), 8.93 - 8.83 (m, 1H), 8.58 - 8.49 (m, 1H), 8.40 - 8.15 (m, 2H), 7.79 - 7.57 (m, 3H), 4.75 - 4.57 (m, 2H), 4.01 - 3.92 (m, 4H), 1.68 - 1.45 (m, 2H), 1.19 - 1.04 (m, 3H), 0.84 - 0.78 (m, 3H).

### 6. Synthesis of example 6

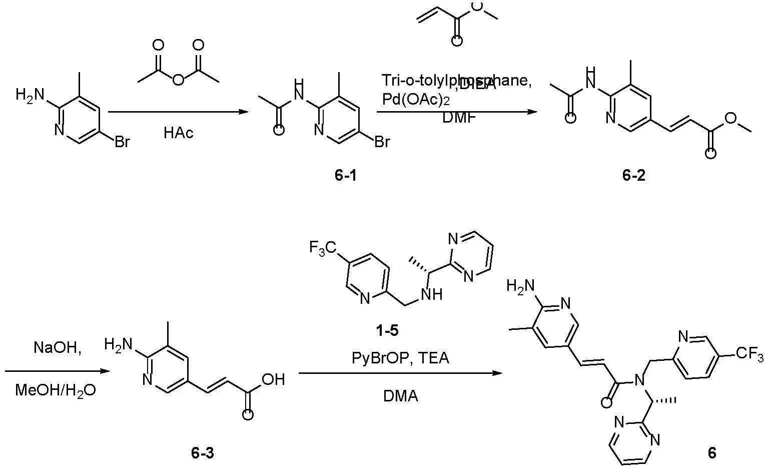

### 6.1 Synthesis of intermediate 6-1

Acetic anhydride (5.4 g) was added to a solution of compound 2-amino-5-bromo-3-methylpyridine (5.0 g) in acetic acid (40 mL), and the mixture was stirred at 120 °C for 4 hours. The mixture was cooled to 50 °C and concentrated under reduced pressure. The residue was dissolved in ethyl acetate (100 mL), and the mixture was washed with saturated sodium bicarbonate solution (100 mL) and saturated sodium chloride solution (100 mL) successively. The organic phase was dried over anhydrous sodium sulfate and filtered. The filtrate was concentrated under reduced pressure. The residue was purified by silica gel column chromatography (eluent: petroleum ether/ethyl acetate = 5/1) to give a white solid **6-1** (4.5 g, yield 73%). LC-MS m/z (ESI): 229.0, 231.1 [M+H]⁺.

### 6.2 Synthesis of intermediate 6-2

Methyl acrylate (1.1 g), N,N-diisopropylethylamine (2.2 g), palladium(II) acetate (0.2 g), and tri(o-tolyl)phosphine (0.5 g) were added to a solution of compound **6-1** (2.0 g) in N,N-dimethylformamide (40 mL), and the mixture was stirred at 100 °C for 16 hours. The mixture was cooled to room temperature, and the reaction solution was slowly poured into iced water (200 mL). The mixture was extracted with ethyl acetate (100 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: dichloromethane/methanol = 50/1) to give a light yellow solid 6-2 (1.6 g, yield 80%). LC-MS m/z (ESI): 235.0 [M+H]⁺.

### 6.3 Synthesis of intermediate 6-3

Sodium hydroxide (70.0 mg) was added to a solution of **6-2** (0.2 g) in methanol (5 mL) and water (5 mL) at 0 °C, and the mixture was stirred at 70 °C for 5 hours. The mixture was cooled to room temperature and concentrated under reduced pressure. The pH of the residue was adjusted to 6 with 1 M hydrochloric acid. The mixture was extracted with ethyl acetate (20 mL × 3). The organic phase was washed with saturated sodium chloride solution (20 mL), dried over anhydrous sodium sulfate and filtered. The filtrate was concentrated under reduced pressure to give a white solid **6-3** (100.0 mg), and the crude product was directly used in the next step reaction. LC-MS m/z (ESI): 179.0 [M+H]⁺.

### 6.4 Synthesis of compound 6

**6-3** (38.0 mg), bromotripyrrolidinophosphonium hexafluorophosphate (103.0 mg), and triethylamine (46.0 mg) were added to a solution of **1-5** (50.0 mg) in N,N-dimethylacetamide (2 mL). The mixture was stirred at room temperature for 16 hours and vacuum filtered. The filtrate was purified by high performance liquid chromatography (column: Gemini 5u C18 150 x 21.2 mm; mobile phase: [water(FA)-ACN]; B%: 2%-10%, 10 min) to give the formate of **6** (10 mg, yield 12%) as a white solid. LC-MS m/z (ESI): 443.2 [M+H]⁺. ¹H NMR (400 MHz, DMSO-*d*₆) δ ppm 8.89 - 8.71 (m, 3H), 8.15 - 8.05 (m, 1H), 8.00 - 7.73 (m, 2H), 7.54 - 7.31 (m, 3H), 7.25 - 6.74 (m, 1H), 6.25 - 6.22 (m, 2H), 5.59 - 5.88 (m, 1H), 5.19 - 4.88 (m, 1H), 4.85 - 4.42 (m, 1H), 2.06 - 1.98 (m, 3H), 1.63 - 1.42 (m, 3H).

### 7. Synthesis of example 7

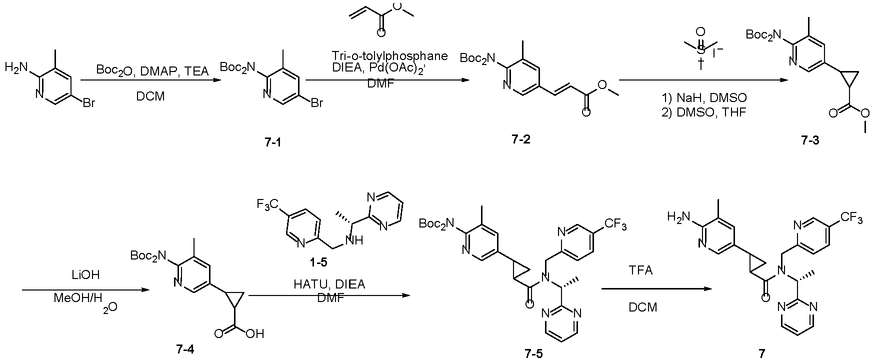

### 7.1 Synthesis of intermediate 7-1

Di-tert-butyl dicarbonate (14.6 g), triethylamine (10.8 g), and 4-dimethylaminopyridine (0.7 g) were added to a solution of 2-amino-5-bromo-3-methylpyridine (5.0 g) in dichloromethane (50 mL), and the mixture was stirred at room temperature for 2 hours. Water (100 mL) was added, and the mixture was extracted with ethyl acetate (100 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: petroleum ether/ethyl acetate = 5/1) to give a white solid **7-1** (9.3 g, yield 85.5%). LC-MS m/z (ESI): 796.9 [2M+H]⁺.

### 7.2 Synthesis of intermediate 7-2

Methyl acrylate (0.7 g), N,N-diisopropylethylamine (1.3 g), and palladium(II) acetate (0.1 g) were added to a solution of compound **7-1** (2.0 g) in N,N-dimethylformamide (20 mL). The mixture was stirred at 100 °C for 16 hours under nitrogen. The mixture was cooled to room temperature, and the reaction solution was slowly added to iced water (100 mL). The mixture was extracted with ethyl acetate (50 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: petroleum ether/ethyl acetate = 3/1) to give a white solid **7-2** (1.4 g, yield 63.6%). LC-MS m/z (ESI): 393.1 [M+H]⁺.

### 7.3 Synthesis of intermediate 7-3

Sodium hydride (1.5 g, 60%) was added to a solution of trimethylsulfoxonium iodide (1.3 g) in dimethylsulfoxide (5 mL) at 0 °C under nitrogen, and the mixture was stirred for 1 hour. Compound **7-2** (1.4 g) was added, and the mixture was slowly warmed to room temperature and stirred for 16 hours. 1 M hydrochloric acid (20 mL) was added to adjust the pH to 6. The mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1) to give a white solid **7-3** (400.0 mg, yield 52.1%). LC-MS m/z (ESI): 393.1 [M+H]⁺.

### 7.4 Synthesis of intermediate 7-4

Lithium hydroxide (14.1 mg) was added to a mixed solution of compound 7-3 (90.0 mg) in methanol (5 mL) and water (5 mL), and the mixture was stirred at 50 °C for 2 hours. The mixture was cooled to room temperature, and the pH was adjusted to 6 with 1 M hydrochloric acid. The mixture was extracted with ethyl acetate (20 mL × 3). The organic phase was washed with saturated sodium chloride solution (20 mL), dried over anhydrous sodium sulfate and filtered. The filtrate was concentrated under reduced pressure to give a white solid 7-4 (50 mg, yield 34.4%), and the crude product was directly used in the next step reaction. LC-MS m/z (ESI): 293.1 [M+H]⁺.

### 7.5 Synthesis of intermediate 7-5

**7-4** (50.0 mg), O-(7-azabenzotriazol-1-yl)-N,N,N',N'-tetramethyluronium hexafluorophosphate (101.0 mg), and N,N-diisopropylethylamine (46.5 mg) were added to a solution of **1-5** (45.0 mg) in N,N-dimethylformamide (2 mL), and the mixture was stirred at room temperature for 2 hours. Iced water (20 mL) was added, and the mixture was extracted with ethyl acetate (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 10/1) to give a yellow solid **7-5** (20.0 mg, yield 32.3%). LC-MS m/z (ESI): 557.2 [M+H]⁺.

### 7.6 Synthesis of compound 7

Trifluoroacetic acid (2 mL) was added to a solution of **7-5** (20.0 mg) in dichloromethane (6 mL), and the mixture was reacted at room temperature for 1 hour. The mixture was concentrated under reduced pressure. The residue was purified by high performance liquid chromatography (column: Gemini 5u C18 150 x 21.2 mm; mobile phase: [water(TFA)-ACN]; B%: 2%-10%, 8 min) to give the trifluoroacetate of **7** (8.0 mg, yield 57.4%) as a white solid. LC-MS m/z (ESI): 457.0 [M+H]⁺. ¹H NMR (400 MHz, MeOD-*d*₄) δ ppm 8.76 (d, *J =* 4.8 Hz, 1H), 8.68 (d, *J =* 4.8 Hz, 2H), 8.01 - 7.91 (m, 1H), 7.66 - 7.57 (m, 1H), 7.42 - 7.41 (m, 1H), 7.38 - 7.26 (m, 2H), 6.05 - 5.83 (m, 1H), 5.25 - 4.69 (m, 2H), 2.57 - 2.28 (m, 1H), 2.26 - 1.98 (m, 4H), 1.71 - 1.60 (m, 4H), 1.41 - 1.16 (m, 1H).

### 8. Synthesis of example 8

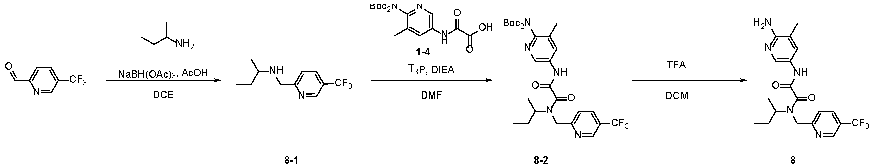

### 8.1 Synthesis of intermediate 8-1

5-(Trifluoromethyl)pyridine-2-carboxaldehyde (200.0 mg) and acetic acid (137.0 mg) were added to a solution of 2-aminobutane (166.0 mg) in 1,2-dichloroethane (5 mL), and the mixture was stirred at room temperature for 2 hours. Sodium triacetoxyborohydride (485.0 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **8-1** (200.0 mg, yield 75.5%). LC-MS m/z (ESI): 233.1 [M+H]⁺.

### 8.2 Synthesis of intermediate 8-2

Compound **8-1** (90.0 mg), propylphosphonic anhydride (50% ethyl acetate solution, 244.0 mg) and N,N-diisopropylethylamine (66.0 mg) were added to a solution of compound **1-4** (50.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow solid **8-2** (50.0 mg, yield 64.9%). LC-MS m/z (ESI): 610.0 [M+H]⁺.

### 8.3 Synthesis of compound 8

Trifluoroacetic acid (2 mL) was added to a solution of compound **8-2** (50.0 mg) in dichloromethane (4 mL) in an ice bath, and the mixture was warmed to room temperature and stirred for 2 hours. The reaction solution was concentrated under pressure, and water (10 mL) was added. The pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: ACN--H₂O (0.1% FA); B%: 2%-30%, 12 min) to give the formate of **8** (6.0 mg, yield 17.8%) as a white solid. LC-MS m/z (ESI): 410.0 [M+H]⁺. ¹H NMR (400 MHz, MeOD-*d*₄) δ ppm 8.82 (s, 1H), 8.12 - 7.96 (m, 2H), 7.67 - 7.46 (m, 2H), 5.14 - 4.62 (m, 2H), 4.51 - 4.13 (m, 1H), 2.17 - 2.11 (m, 3H), 1.74 - 1.54 (m, 2H), 1.26 - 1.19 (m, 3H), 0.93 - 0.88 (m, 3H).

### 9. Synthesis of example 9

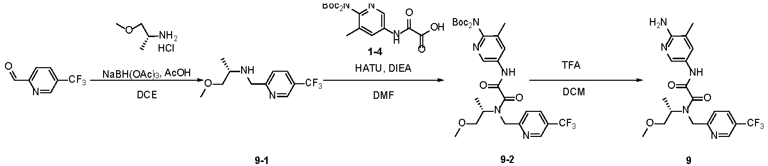

### 9.1 Synthesis of intermediate 9-1

5-(Trifluoromethyl)pyridine-2-carboxaldehyde (200.0 mg) and acetic acid (137.0 mg) were added to a solution of (R)-1-methoxy-2-propylamine hydrochloride (166.0 mg) in 1,2-dichloroethane (5 mL), and the mixture was stirred at room temperature for 2 hours. Sodium triacetoxyborohydride (485.0 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **9-1** (20.0 mg, yield 7.1%). LC-MS m/z (ESI): 249.1 [M+H]⁺.

### 9.2 Synthesis of intermediate 9-2

Compound **1-4** (50.0 mg), O-(7-azabenzotriazol-1-yl)-N,N,N',N'-tetramethyluronium hexafluorophosphate (50.0 mg) and N,N-diisopropylethylamine (20.0 mg) were added to a solution of compound **9-1** (20.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow solid **9-2** (15.0 mg, yield 30.2%). LC-MS m/z (ESI): 626.3 [M+H]⁺.

### 9.3 Synthesis of compound 9

Trifluoroacetic acid (0.5 mL) was added to a solution of compound **9-2** (15.0 mg) in dichloromethane (2 mL) in an ice bath, and the mixture was warmed to room temperature and stirred for 2 hours. The reaction solution was concentrated under pressure, and water (10 mL) was added. The pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: ACN--H₂O (0.1% FA); B%: 2%-40%, 12 min) to give the formate of **9** (3.0 mg, yield 29.4%) as a white solid. LC-MS m/z (ESI): 426.2 [M+H]⁺. ¹H NMR (400 MHz, MeOD-*d*₄) δ ppm 8.71 (s, 1H), 8.02 - 7.84 (m, 2H), 7.62 - 7.51 (m, 2H), 4.95 (s, 1H), 4.69 (d, *J* = 8.0 Hz, 1H), 4.53 - 4.43 (m, 1H), 3.36 - 3.25 (m, 2H), 3.09 (d, *J* = 3.6 Hz, 3H), 2.03 (d, *J =* 224.8 Hz, 3H), 1.18 - 1.07 (m, 3H).

### 10. Synthesis of example 10

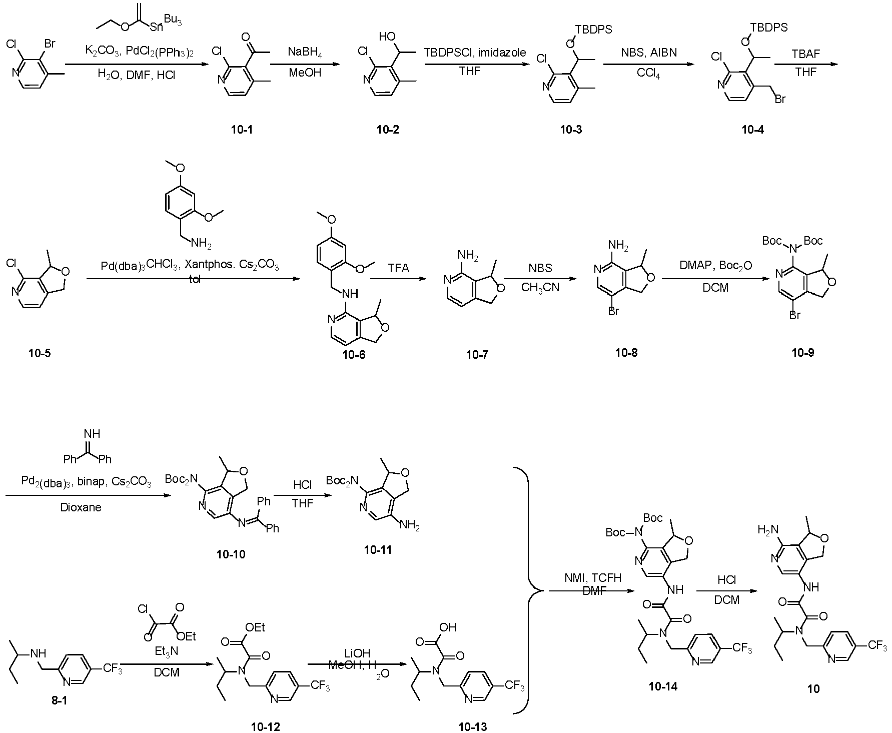

### 10.1 Synthesis of intermediate 10-1

Tributyl(1-ethoxyvinyl)tin (13.2 g) was added to a solution of 3-bromo-2-chloro-4-methylpyridine (5.0 g), potassium carbonate (6.7 g) and bis(triphenylphosphine)palladium(II) dichloride (850.0 mg) in a mixed solvent of tetrahydrofuran and water (50 mL, V/V = 3:1). The mixture was stirred at 90 °C for 16 hours under nitrogen. The reaction solution was cooled to room temperature, and saturated potassium fluoride solution (100 mL) and ethyl acetate (100 mL) were added to the reaction solution. The mixture was filtered. The organic phase was dried over anhydrous sodium sulfate and filtered. The filtrate was concentrated under reduced pressure. The residue was dissolved with methanol (20 mL), and hydrochloric acid (6 M, 10 mL) was added to the above solution. The mixture was stirred at room temperature for 16 hours under nitrogen. The reaction solution was concentrated under reduced pressure, and saturated sodium bicarbonate solution (100 mL) was added. The mixture was extracted with ethyl acetate (100 mL × 3). The organic phases were combined, washed with saturated sodium chloride solution (300 mL), dried over anhydrous sodium sulfate and filtered. The filtrate was concentrated under reduced pressure. The residue was purified by silica gel column chromatography (eluent: petroleum ether/ethyl acetate = 10/1 - 20/1) to give a yellow liquid **10-1** (2.2 g, yield 53.7%). LC-MS m/z (ESI): 170.0 [M+H]⁺.

### 10.2 Synthesis of intermediate 10-2

Sodium borohydride (980.0 mg) was added to a solution of **10-1** (2.2 g) in methanol (20 mL). The mixture was stirred at 25 °C for 1 hour under nitrogen. Water (50 mL) was added to the reaction solution, and the mixture was extracted with ethyl acetate (50 mL × 3). The organic phases were combined, washed with saturated sodium chloride solution (50 mL), dried over anhydrous sodium sulfate and filtered. The filtrate was concentrated under reduced pressure. The residue was purified by silica gel column chromatography (eluent: dichloromethane/ethyl acetate = 10/1 - 5/1) to give a colorless liquid **10-2** (2.0 g, yield 80.8%). LC-MS m/z (ESI): 172.1 [M+H]⁺.

### 10.3 Synthesis of intermediate 10-3

tert-Butyl(chloro)diphenylsilane (3.9 g) was added to a solution of **10-2** (2.0 g) and imidazole (1.2 g) in dichloromethane (20 mL). The mixture was stirred at 25 °C for 16 hours under nitrogen. Water (100 mL) was added to the reaction solution, and the mixture was extracted with dichloromethane (30 mL × 3). The organic phases were combined, washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = 100/1 - 5/1) to give a colorless oil 79-3 (4.5 g, yield 93.2%). LC-MS m/z (ESI): 410.0 [M+H]⁺.

### 10.4 Synthesis of intermediate 10-4

N-bromosuccinimide (2.4 g) was added to a solution of **10-3** (5.0 g) and azobisisobutyronitrile (0.1 g) in carbon tetrachloride (50 mL). The mixture was stirred at 90 °C for 2 hours under nitrogen. The reaction solution was cooled to room temperature, and water (100 mL) was added to the reaction solution. The mixture was extracted with dichloromethane (100 mL × 3). The organic phases were combined, washed with saturated sodium chloride solution (100 mL), dried over anhydrous sodium sulfate and filtered. The filtrate was concentrated under reduced pressure. The residue was purified by silica gel column chromatography (eluent: petroleum ether/ethyl acetate = 100/1 - 10/1) to give a colorless oil **10-4** (4.5 g, yield 59.8%). LC-MS m/z (ESI): 488.2 [M+H]⁺.

### 10.5 Synthesis of intermediate 10-5

A solution of tetrabutylammonium fluoride (1 M, 18.4 mL) in tetrahydrofuran was added to a solution of **10-4** (4.5 g) in tetrahydrofuran (50 mL). The mixture was stirred at 25 °C for 1 hour under nitrogen. Water (100 mL) was added to the reaction solution, and the mixture was extracted with ethyl acetate (10 mL × 3). The organic phases were combined, washed with saturated sodium chloride solution (100 mL), dried over anhydrous sodium sulfate and filtered. The filtrate was concentrated under reduced pressure. The residue was purified by silica gel column chromatography (eluent: petroleum ether/ethyl acetate = 100/1 - 5/1) to give a colorless oil **10-5** (600.0 mg, yield 38.0%). LC-MS m/z (ESI): 170.1 [M+H]⁺.

### 10.6 Synthesis of intermediate 10-6

2,2'-Bis(diphenylphosphino)-1,1'-binaphthyl (418.5 mg), tris(dibenzylideneacetone)dipalladium(0) (193.2 mg) and sodium tert-butoxide (645.9 mg) were added to a solution of **10-5** (570.0 mg) and 2,4-dimethoxybenzylamine (842.9 mg) in toluene (1 mL). The mixture was stirred at 100 °C for 16 hours under nitrogen. The reaction solution was cooled to room temperature, and water (50 mL) was added to the reaction solution. The mixture was extracted with ethyl acetate (50 mL × 3). The organic phases were combined, washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 100/1 - 5/1) to give a white solid **10-6** (410 mg, yield 40.6%). LC-MS m/z (ESI): 301.1 [M+H]⁺.

### 10.7 Synthesis of intermediate 10-7

**10-6** (410 mg) was added to a solution of trifluoroacetic acid (3 mL). The mixture was stirred at 25 °C for 1 hour under nitrogen. Saturated sodium bicarbonate solution (100 mL) was added to the reaction solution, and the mixture was extracted with ethyl acetate (50 mL × 3). The organic phases were combined, washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = *5*/*1* - 1/10) to give a white solid **10-7** (150.0 mg, yield 73.2%). LC-MS m/z (ESI): 151.0 [M+H]⁺.

### 10.8 Synthesis of intermediate 10-8

N-bromosuccinimide (213.3 mg) was added to a solution of **10-7** (150.0 mg) in acetonitrile (5 mL). The mixture was stirred at 25 °C for 2 hours under nitrogen. Water (20 mL) was added to the reaction solution, and the mixture was extracted with ethyl acetate (20 mL × 3). The organic phases were combined, washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = *5*/*1* - 1/1) to give a white solid **10-8** (130.0 mg, yield 56.8%). LC-MS m/z (ESI): 229.0 [M+H]⁺.

### 10.9 Synthesis of intermediate 10-9

Di-tert-butyl dicarbonate (309.6 mg) was added to a solution of **10-8** (130.0 mg), 4-dimethylaminopyridine (6.9 mg) and triethylamine (172.3 mg) in dichloromethane (5 mL). Water (20 mL) was added to the reaction solution, and the mixture was extracted with ethyl acetate (20 mL × 3). The organic phases were combined, washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 10/1 - 5/1) to give a white solid **10-9** (150.0 mg, yield 61.3%). LC-MS m/z (ESI): 451 [M+Na]⁺.

### 10.10 Synthesis of intermediate 10-10

Tris(dibenzylideneacetone)dipalladium(0) (66.2 mg), 2,2'-bis(diphenylphosphino)-1,1'-binaphthyl (67.5 mg) and cesium carbonate (176.5 mg) were added to a solution of benzophenone imine (98.2 mg) and **10-9** (150.0 mg) in dioxane (5 mL). The mixture was stirred at 100 °C for 8 hours under nitrogen. The reaction solution was cooled to room temperature, and water (30 mL) was added to the reaction solution. The mixture was extracted with ethyl acetate (20 mL × 3). The organic phases were combined, washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 10/1 - 1/1) to give a white solid **10-10** (60.0 mg, yield 32.2%). LC-MS m/z (ESI): 530.1 [M+H]⁺.

### 10.11 Synthesis of intermediate 10-11

A solution of HCl in dioxane (0.6 mL, 4 M) was added to a solution of **10-10** (60.0 mg) in tetrahydrofuran (5 mL) at 0 °C. The mixture was stirred at 0 °C for 1 hour under nitrogen. Saturated sodium carbonate aqueous solution (20 mL) was added to the reaction solution, and the mixture was extracted with ethyl acetate (10 mL × 3). The organic phases were combined, washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate *= 5*/*1* - 1/2) to give a white solid **10-11** (25.0 mg, yield 60.4%). LC-MS m/z (ESI): 366.2 [M+H]⁺.

### 10.12 Synthesis of intermediate 10-12

Ethyl 2-chloro-2-oxoacetate (70.6 mg) was added to a solution of **10-11** (100.0 mg) and triethylamine (87.1 mg) in dichloromethane (3 mL). The mixture was stirred at 25 °C for 1 hour under nitrogen. Water (50 mL) was added to the reaction solution, and the mixture was extracted with dichloromethane (30 mL × 3). The organic phases were combined, washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 100/1 - 2/1) to give a brown oil **10-12** (140 mg, yield 97.8%). LC-MS m/z (ESI): 333.2 [M+H]⁺.

### 10.13 Synthesis of intermediate 10-13

Lithium hydroxide monohydrate (94.7 mg) was added to a solution of **10-12** (150.0 mg) in a mixed solvent of methanol and water (6 mL, V/V = 5:1)*.* The mixture was stirred at 25 °C for 1 hour under nitrogen. Water (10 mL) was added to the reaction solution, and 1 M hydrochloric acid was added dropwise until the pH of the reaction solution was 5 to 6. The mixture was extracted with ethyl acetate (50 mL × 3). The organic phases were combined, washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 100/1 - 5/1) to give a brown solid **10-13** (80.0 mg, yield 58.2%). LC-MS m/z (ESI): 305.2 [M+H]⁺.

### 10.14 Synthesis of intermediate 10-14

Chloro-N,N,N',N'-tetramethylformamidinium hexafluorophosphate (22.9 mg) was added to a solution of **10-13** (33.1 mg), **10-11** (20.0 mg) and methylimidazole (8.9 mg) in N,N-dimethylformamide (1 mL). The mixture was stirred at 25 °C for 1 hour under nitrogen. Water (10 mL) was added to the reaction solution, and the mixture was extracted with ethyl acetate (10 mL × 3). The organic phases were combined, washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = *5*/*1* - 1/1) to give a white solid **10-14** (10.0 mg, yield 28.1%). LC-MS m/z (ESI): 652.2 [M+H]⁺.

### 10.15 Synthesis of compound 10

A solution of HCl in dioxane (0.2 mL, 4 M) was added to a solution of **10-14** (10 mg) in dichloromethane (1 mL). The mixture was stirred at 25 °C for 3 hours under nitrogen. The reaction solution was concentrated under reduced pressure. The residue was purified by thin layer chromatography (eluent: dichloromethane/methanol = 10/1) to give a white solid **10** (5.4 mg, yield 78.4%). LC-MS m/z (ESI): 452.2 [M+H]⁺. ¹H NMR (400 MHz, DMSO-*d*₆, 80 °C) δ 10.50-10.25 (m, 1H), 8.87 (s, 1H), 8.15 (d, *J =* 8.0 Hz, 1H), 7.89 (s, 1H), 7.70 - 7.60 (m, 1H), 5.72 (s, 2H), 5.25-5.20 (m, 1H), 5.05 - 4.93 (m, 1H), 4.85 (m, 1H), 4.72 (m, 1H), 4.62 - 4.52 (m, 1H), 4.10 - 3.87 (m, 1H), 1.67 - 1.48 (m, 2H), 1.39 (d, *J =* 6.4 Hz, 2H), 1.34 - 1.31 (m, 1H), 1.20 (d, *J* = 6.5 Hz, 2H), 1.07 (d, *J* = 6.8 Hz, 1H), 0.84 (t, *J* = 7.4 Hz, 3H).

### 11. Synthesis of example 11

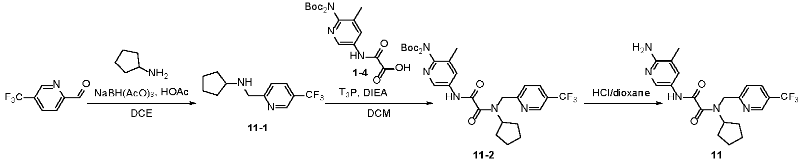

### 11.1 Synthesis of intermediate 11-1

Cyclopentylamine (486.3 mg) was added to a solution of 5-(trifluoromethyl)pyridine-2-carboxaldehyde (500.0 mg) and acetic acid (343.0 mg) in 1,2-chloroethane (10 mL). The mixture was stirred at room temperature for 1 hour, and then sodium triacetoxyborohydride (1210.0 mg) was added. The mixture was stirred at room temperature for 1 hour. Saturated sodium bicarbonate solution (20 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 10/1 - 8/1) to give a yellow oil **11-1** (540.0 mg, yield 69.7%). LC-MS m/z (ESI): 245.1 [M+H]⁺.

### 11.2 Synthesis of intermediate 11-2

Propylphosphonic anhydride (483.0 mg, 50% ethyl acetate solution) was added to a solution of N,N-diisopropylethylamine (130.7 mg), **11-1** (148.3 mg) and **1-4** (200.0 mg) in dichloromethane (8 mL). The mixture was stirred at room temperature for 2 hours. Iced water (20 mL) was added, and the mixture was extracted with dichloromethane (20 mL × 3). The organic phase was washed with saturated sodium chloride solution (15 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **11-2** (150.0 mg, yield 43.0%). LC-MS m/z (ESI): 622.3 [M+H]⁺.

### 11.3 Synthesis of compound 11

A solution of HCl in 1,4-dioxane (4 mL, 4 M) was added to **11-2** (140.0 mg) in an ice bath. The mixture was stirred at room temperature for 2 hours, and concentrated under reduced pressure. The residue was purified by high performance liquid chromatography (column: Xbridge-C18 150 × 19 mm, Sum; mobile phase: ACN--H₂O (0.1% FA); B%: 10%-40%, 20 min) to give the formate of **11** (15.1 mg, yield 15.8%) as a white solid. LC-MS m/z (ESI): 422.0 [M+H]⁺. ¹H NMR (400 MHz, DMSO-*d*₆) δ ppm 10.52 - 10.30 (m, 1H), 8.93 - 8.88 (m, 1H), 8.28 - 8.05 (m, 2H), 7.87 - 7.30 (m, 2H), 5.67 - 5.60 (m, 2H), 4.88 - 4.66 (m, 2H), 4.38 - 4.34 (m, 1H), 2.06 - 1.95 (m, 3H), 1.84 - 1.73 (m, 2H), 1.63 - 1.49 (m, 6H).

### 12. Synthesis of example 12

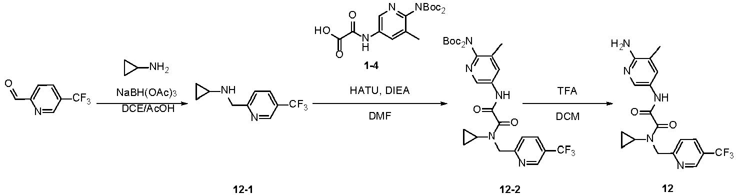

### 12.1 Synthesis of intermediate 12-1

Cyclopropylamine (131.0 mg) was added to a solution of 5-(trifluoromethyl)pyridine-2-carboxaldehyde (200.0 mg) in 1,2-chloroethane (2 mL) and acetic acid (1 mL). The mixture was stirred at room temperature for 1 hour under nitrogen, and then sodium triacetoxyborohydride (485.0 mg) was added. The mixture was stirred at room temperature for 1 hour. Saturated sodium bicarbonate solution (10 mL) was added to the reaction solution, and the mixture was extracted with dichloromethane (30 mL × 3). The organic phase was washed with saturated sodium chloride solution (5 mL), dried over anhydrous sodium sulfate and filtered. The filtrate was concentrated under reduced pressure to give a light yellow liquid crude product **12-1** (10.0 mg). LC-MS m/z (ESI): 217.1 [M+H]⁺.

### 12.2 Synthesis of intermediate 12-2

**1-4** (20.0 mg), O-(7-azabenzotriazol-1-yl)-N,N,N',N'-tetramethyluronium hexafluorophosphate (35.0 mg), and N,N-diisopropylethylamine (10.0 mg) were added to a solution of **12-1** (10.0 mg) in N,N-dimethylformamide (1 mL). The mixture was stirred at room temperature for 2 hours under nitrogen. Water (1 mL) was added to the reaction solution, and the mixture was extracted with dichloromethane (10 mL × 3). The organic phase was washed with saturated sodium chloride solution (1 mL), dried over anhydrous sodium sulfate and filtered. The filtrate was concentrated under reduced pressure to give a light yellow liquid crude product **12-2** (15.0 mg). LC-MS m/z (ESI): 594.0 [M+H]⁺.

### 12.3 Synthesis of compound 12

Trifluoroacetic acid (0.5 mL) was added to a solution of **12-2** (15.0 mg) in dichloromethane (1 mL) in an ice bath. The mixture was stirred at room temperature for 1 hour, and the pH was adjusted to 8 with saturated sodium carbonate solution. The mixture was extracted with dichloromethane (5 mL × 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **12** (3.47 mg, yield 34.9%) as a yellow solid. LC-MS m/z (ESI): 394.1 [M+H]⁺. ¹H NMR (400 MHz, CD₃OD) δ ppm 8.87 (s, 1H), 8.14 - 8.13 (m, 1H), 8.10 - 8.09 (m, 1H), 7.63 - 7.61 (m, 2H), 4.87 (s, 2H), 3.13 - 3.03 (m, 1H), 2.18 (s, 3H), 0.91 - 0.83 (m, 4H).

### 13. Synthesis of example 13

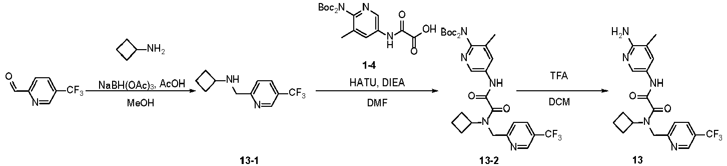

### 13.1 Synthesis of intermediate 13-1

5-(Trifluoromethyl)pyridine-2-carboxaldehyde (200.0 mg) and acetic acid (137.0 mg) were added to a solution of cyclobutylamine (162.0 mg) in 1,2-dichloroethane (5 mL), and the mixture was stirred at room temperature for 2 hours. Sodium triacetoxyborohydride (485.0 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **13-1** (250.0 mg, yield 95.0%). LC-MS m/z (ESI): 230.9 [M+H]⁺.

### 13.2 Synthesis of intermediate 13-2

Compound **13-1** (100.0 mg), O-(7-azabenzotriazol-1-yl)-N,N,N',N'-tetramethyluronium hexafluorophosphate (216.0 mg) and N,N-diisopropylethylamine (98.0 mg) were added to a solution of compound **1-4** (150.0 mg) in N,N-dimethylformamide (5 mL). The mixture was stirred at room temperature for 2 hours. Water (50 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid **13-2** (40.0 mg, yield 17.4%). LC-MS m/z (ESI): 608.2 [M+H]⁺.

### 13.3 Synthesis of compound 13

Trifluoroacetic acid (2 mL) was added to a solution of compound **13-2** (40.0 mg) in dichloromethane (4 mL) in an ice bath. The mixture was warmed to room temperature and stirred for 2 hours. The reaction solution was concentrated under reduced pressure, and water (10 mL) was added. The pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by silica gel column chromatography (eluent: dichloromethane/methanol = 20/1) to give a light yellow solid **13** (10.0 mg, yield 37.3%). LC-MS m/z (ESI): 408.0 [M+H]⁺. ¹H NMR (400 MHz, MeOD-*d*₄) δ ppm 8.84 - 8.82 (m, 1H), 8.11 - 7.95 (m, 2H), 7.64 - 7.47 (m, 2H), 5.10 - 4.93 (m, 2H), 4.75 - 4.65 (m, 1H), 2.27 - 2.06 (m, 7H), 1.72 - 1.63 (m, 2H).

### 14. Synthesis of example 14-P1 and example 14-P2

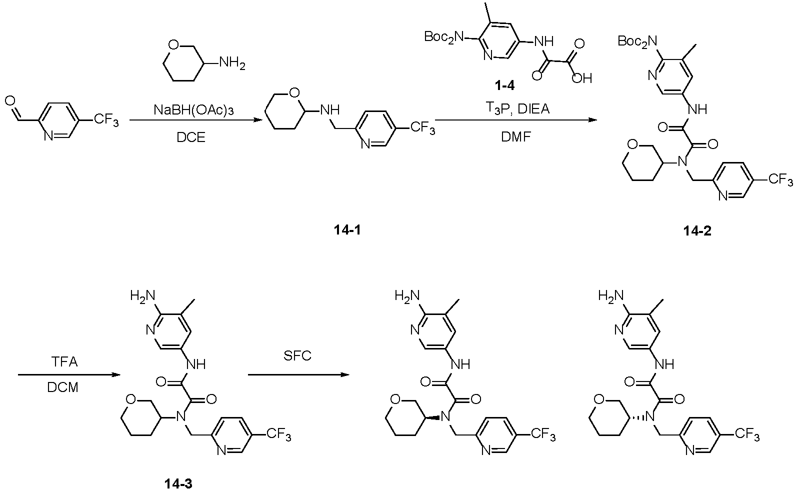

### 14.1 Synthesis of intermediate 14-1

5-(Trifluoromethyl)pyridine-2-carboxaldehyde (200.0 mg) and acetic acid (102.0 mg) were added to a solution of 3-aminotetrahydropyran (229.7 mg) in1,2-dichloroethane (5 mL), and the mixture was stirred at room temperature for 2 hours. Sodium triacetoxyborohydride (418.6 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 30/1) to give a yellow oil **14-1** (210.0 mg, yield 67.9%). LC-MS m/z (ESI): 261.0 [M+H]⁺.

### 14.2 Synthesis of intermediate 14-2

Propylphosphonic anhydride (216.0 mg) and N,N-diisopropylethylamine (98.0 mg) were added to a solution of compound **14-1** (100.0 mg) and **1-4** (150.0 mg) in N,N-dimethylformamide (5 mL), and the mixture was stirred at room temperature for 2 hours. Water (50 mL) was added, and the mixture was extracted with ethyl acetate (30 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 30/1) to give a yellow solid **14-2** (210.0 mg, yield 85.6%). LC-MS m/z (ESI): 638.1 [M+H]⁺.

### 14.3 Synthesis of compound 14-P1 and compound 14-P2

Trifluoroacetic acid (2 mL) was added to a solution of compound **14-2** (210.0 mg) in dichloromethane (6 mL) in an ice bath, and the mixture was warmed to room temperature and stirred for 2 hours. The reaction solution was concentrated under reduced pressure, and water (30 mL) was added. The pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: [water(FA)-ACN]; B%: 2%-10%, 16min) to give the formate of **14-3** (82.0 mg, yield 67.3%) as a white solid. The product was separated by SFC (column: CHIRALPAK IC 250mm × 20 mm, 5 µm; mobile phase: ETOH (NH₄OH 0.2%); B%: 40% EtOH, 15 min) to give a white solid **14-P1** (30.0 mg). LC-MS m/z (ESI): 438.0 [M+H]⁺. ¹H NMR (400 MHz, MeOD-*d*₄) δ ppm 8.83 (s, 1H), 8.13 - 7.90 (m, 2H), 7.68 - 7.40 (m, 2H), 5.11 - 4.83 (m, 2H), 4.43 - 4.12 (m, 2H), 4.03 - 3.75 (m, 2H), 3.45 - 3.41 (m, 1H), 2.16 - 2.09 (m, 3H), 2.04 - 1.79 (m, 2H), 1.75 - 1.65 (m, 2H);

A white solid **14-P2** (37.0 mg) was obtained. LC-MS m/z (ESI): 438.0 [M+H]⁺. ¹H NMR (400 MHz, MeOD-*d*₄) δ ppm 8.83 (s, 1H), 8.11 - 7.90 (m, 2H), 7.68 - 7.39 (m, 2H), 5.12 - 4.81 (m, 2H), 4.43 - 4.09 (m, 2H), 4.03 - 3.74 (m, 2H), 3.50 - 3.39 (m, 1H), 2.18 - 2.06 (m, 3H), 2.03 - 1.80 (m, 2H), 1.75 - 1.65 (m, 2H).

### 15. Synthesis of example 15

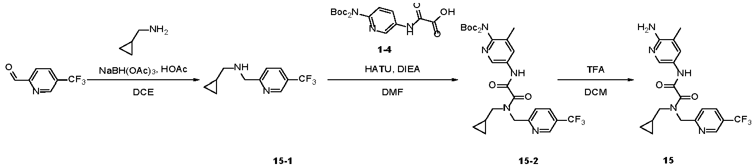

### 15.1 Synthesis of intermediate 15-1

5-(Trifluoromethyl)pyridine-2-carboxaldehyde (100.0 mg) and acetic acid (65.0 mg) were added to a solution of cyclopropylmethylamine (41.0 mg) in anhydrous dichloroethane (2 mL), and the mixture was stirred at room temperature for 1 hour. Sodium triacetoxyborohydride (363.0 mg) was added, and the mixture was stirred at room temperature for 3 hours. Saturated sodium bicarbonate solution (30 mL) was added, and the mixture was extracted with dichloromethane (10 mL × 3). The organic phase was dried over anhydrous sodium sulfate and filtered. The filtrate was concentrated under reduced pressure. The residue was purified by silica gel column chromatography (eluent: petroleum ether/ethyl acetate = 1/1) to give a yellow oil 15-1 (20.0 mg, yield 15.2%). LC-MS m/z (ESI): 231.1 [M+H]⁺.

### 15.2 Synthesis of intermediate 15-2

**1-4** (34.0 mg), O-(7-azabenzotriazol-1-yl)-N,N,N',N'-tetramethyluronium hexafluorophosphate (66.0 mg), and N,N-diisopropylethylamine (23.0 mg) were added to a solution of **15-1** in N,N-dimethylformamide (2 mL), and the mixture was stirred at room temperature for 3 hours. Iced water (20 mL) was added, and the mixture was extracted with ethyl acetate (10 mL × 3). The organic phase was washed with saturated sodium chloride solution (20 mL), dried over anhydrous sodium sulfate and filtered. The filtrate was concentrated under reduced pressure to give compound **15-2** (20.0 mg), and the crude product was directly used in the next step reaction. LC-MS m/z (ESI): 608.3 [M+H]⁺.

### 15.3 Synthesis of compound 15

Trifluoroacetic acid (1 mL) was added to a solution of **15-2** (20.0 mg) in dichloromethane (3 mL), and the mixture was stirred at room temperature for 1 hour. The mixture was concentrated under reduced pressure, and the residue was purified by thin layer chromatography (eluent: petroleum ether/ethyl acetate = 1/1) to give a white solid **15** (4.4 mg, yield 32.8%). LC-MS m/z (ESI): 408.2 [M+H]⁺.¹H NMR (400 MHz, MeOD-d4) δ ppm 8.61 (s, 1H), 8.00 - 7.92 (m, 2H), 7.54 - 7.44 (m, 2H), 5.04 - 4.85 (m, 2H), 3.41 - 3.28 (m, 2H), 2.09 - 2.03 (m, 3H), 1.00 - 0.82 (m, 1H), 0.26 - 0.21 (m, 2H), 0.13 - 0.11 (m, 2H).

### 16. Synthesis of example 16

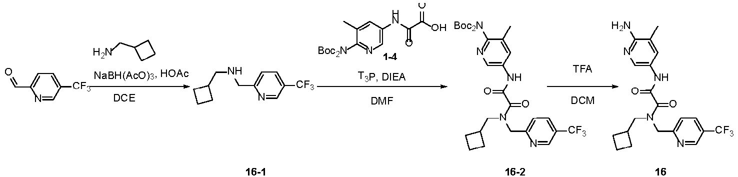

### 16.1 Synthesis of intermediate 16-1

Cyclobutylmethylamine (300.0 mg) was added to a solution of 5-(trifluoromethyl)pyridine-2-carboxaldehyde (300.0 mg) in 1,2-chloroethane (2 mL) and acetic acid (1 mL). The mixture was stirred at room temperature for 1 hour under nitrogen. Then, sodium triacetoxyborohydride (727.0 mg) was added, and the mixture was stirred at room temperature for 1 hour. Saturated sodium bicarbonate solution (10 mL) was added to the reaction solution, and the mixture was extracted with dichloromethane (10 mL × 3). The organic phase was washed with saturated sodium chloride solution (10 mL), dried over anhydrous sodium sulfate and filtered. The filtrate was concentrated under reduced pressure to give a light yellow oil **16-1** (300.0 mg, yield 71.7%). LC-MS m/z (ESI): 245.0 [M+H]⁺.

### 16.2 Synthesis of intermediate 16-2

**1-4** (453.0 mg), propylphosphonic anhydride (782.1 mg), and N,N-diisopropylethylamine (318.0 mg) were added to a solution of **16-1** (280.0 mg) in N,N-dimethylformamide (3 mL). The mixture was stirred at room temperature for 12 hours. Water (5 mL) was added to the reaction solution, and the mixture was extracted with ethyl acetate (5 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 2/1) to give a white solid **16-2** (240.0 mg, yield 33.7%). LC-MS m/z (ESI): 622.0 [M+H]⁺.

### 16.3 Synthesis of compound 16

Trifluoroacetic acid (1 mL) was added to a solution of **16-2** (240.0 mg) in dichloromethane (2 mL) in an ice bath. The mixture was stirred at room temperature for 2 hours, and the pH was adjusted to 8 with saturated sodium carbonate solution. The mixture was extracted with dichloromethane (5 mL × 3), and the organic phase was concentrated under reduced pressure. The residue was purified by high performance liquid chromatography (column: Xbridge-C18 150 x 19 mm, Sum; mobile phase: ACN--H₂O (0.1% FA); B%: 5%-10%, 20 min) to give the formate of **16** (145.6 mg, yield 89.5%) as a white solid. LC-MS m/z (ESI): 422.0 [M+H]⁺. ¹H NMR (400 MHz, DMSO-*d*₆) δ ppm 10.54 - 10.39 (m, 1H), 8.95 - 8.90 (m, 1H), 8.25 - 8.21 (m, 1H), 8.06 - 7.95 (m, 1H), 7.62 - 7.42 (m, 2H), 5.86 - 5.66 (m, 2H), 4.91 - 4.73 (m, 2H), 3.57 - 3.34 (m, 2H), 2.61 - 2.56 (m, 1H), 2.05 - 1.99 (m, 3H), 1.95 - 1.63 (m, 6H).

### 17. Synthesis of example 17

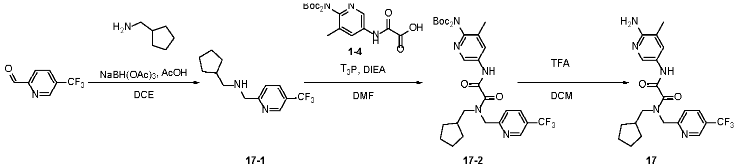

### 17.1 Synthesis of intermediate 17-1

5-(Trifluoromethyl)pyridine-2-carboxaldehyde (200.0 mg) and acetic acid (137.0 mg) were added to a solution of cyclopentylmethylamine (225.0 mg) in 1,2-dichloroethane (5 mL), and the mixture was stirred at room temperature for 2 hours. Sodium triacetoxyborohydride (485.0 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **17-1** (200.0 mg, yield 67.8%). LC-MS m/z (ESI): 259.2 [M+H]⁺.

### 17.2 Synthesis of intermediate 17-2

Compound **17-1** (100.0 mg), propylphosphonic anhydride (244.0 mg, 50% ethyl acetate solution) and N,N-diisopropylethylamine (66.0 mg) were added to a solution of compound **1-4** (50.0 mg) in N,N-dimethylformamide (3 mL), and the mixture was stirred at room temperature for 2 hours. Water (30 mL) was added, and the mixture was extracted with ethyl acetate (20mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow solid **17-2** (55.0 mg, yield 68.4%). LC-MS m/z (ESI): 636.1 [M+H]⁺.

### 17.3 Synthesis of compound 17

Trifluoroacetic acid (2 mL) was added to a solution of compound **17-2** (40.0 mg) in dichloromethane (4 mL) in an ice bath, and the mixture was warmed to room temperature and stirred for 2 hours. Water (10 mL) was added, and the pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: ACN--H₂O (0.1% FA); B%: 2%-40%, 12 min) to give the formate of **17** (18.0 mg, yield 27.5%) as a white solid. LC-MS m/z (ESI): 436.1 [M+H]⁺. ¹H NMR (400 MHz, MeOD-*d*₄) δ ppm 8.84 (s, 1H), 8.13 - 8.0 (m, 2H), 7.63 - 7.52 (m, 2H), 5.08 - 4.86 (m, 2H), 3.62 - 3.43 (m, 2H), 2.31 - 2.29 (m, 1H), 2.16 - 2.11 (m, 3H), 1.75 - 1.55 (m, 6H), 1.29 - 1.25 (m, 2H).

### 18. Synthesis of example 18

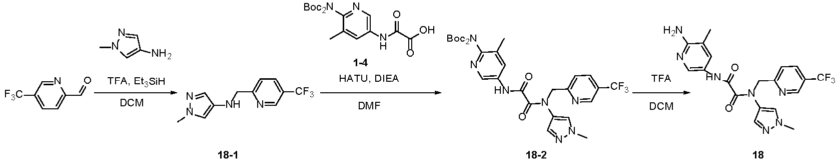

### 18.1 Synthesis of intermediate 18-1

1-Methyl-1H-pyrazol-4-amine (270.0 mg) was added to a solution of 5-(trifluoromethyl)pyridine-2-carboxaldehyde (200.0 mg) in dichloromethane (2 mL) and trifluoroacetic acid (1 mL). The mixture was stirred at room temperature for 1 hour under nitrogen, and then triethylsilane (1 mL) was added. The mixture was stirred at room temperature for 2 hours. Saturated sodium bicarbonate solution (10 mL) was added, and the mixture was extracted with dichloromethane (10 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 1/1) to give a yellow liquid **18-1** (240.0 mg, yield 82.0%). LC-MS m/z (ESI): 257.2 [M+H]⁺.

### 18.2 Synthesis of intermediate 18-2

**1-4** (53.0 mg), O-(7-azabenzotriazol-1-yl)-N,N,N',N'-tetramethyluronium hexafluorophosphate (120.0 mg), and N,N-diisopropylethylamine (40.0 mg) were added to a solution of **18-1** (40.0 mg) in N,N-dimethylformamide (1 mL), and the mixture was stirred at room temperature for 2 hours. Water (5 mL) was added, and the mixture was extracted with dichloromethane (5 mL × 3). The organic phase was washed with saturated sodium chloride solution (1 mL), dried over anhydrous sodium sulfate and filtered. The filtrate was concentrated under reduced pressure to give a light yellow oil **18-2** (50.0 mg). The crude product was directly used in the next step reaction without purification. LC-MS m/z (ESI): 634.0 [M+H]⁺.

### 18.3 Synthesis of compound 18

Trifluoroacetic acid (0.5 mL) was added to a solution of **18-2** (50.0 mg) in dichloromethane (1 mL) in an ice bath, and the mixture was stirred at room temperature for 1 hour. The mixture was concentrated under reduced pressure, and the residue was purified by high performance liquid chromatography (column: Xbridge-C18 150 x 19 mm, Sum; mobile phase: ACN--H₂O (0.1% FA); B%: 5%-10%, 20 min) to give the formate of **18** (9.4 mg, yield 27.5%) as a yellow solid. LC-MS m/z (ESI): 434.0 [M+H]⁺. ¹H NMR (400 MHz, DMSO-*d₆*) δ ppm 10.77 - 10.59 (m, 1H), 8.94 - 8.90 (m, 1H), 8.26 - 8.23 (m, 1H), 8.06 - 8.04 (m, 1H), 7.78 (s, 1H), 7.64 - 7.57 (m, 2H), 7.41 - 7.38 (m, 1H), 6.21 (s, 2H), 5.29 - 5.02 (m, 2H), 3.79 - 3.71 (m, 3H), 2.05 (s, 3H).

### 19. Synthesis of example 19-P1 and example 19-P2

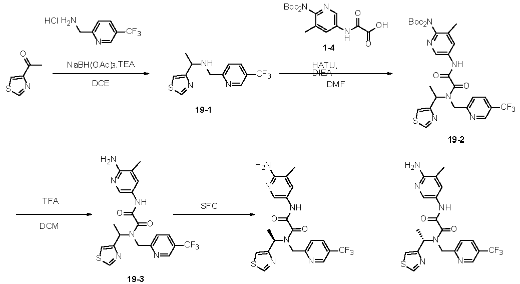

### 19.1 Synthesis of intermediate 19-1

Triethylamine (239.0 mg) was added to a solution of (5-(trifluoromethyl)pyridin-2-yl)methanamine hydrochloride (334.0 mg) in 1,2-dichloroethane (5 mL), and the mixture was stirred at room temperature for 3 hours. 4-Acetylthiazole (200.0 mg) was added, and the mixture was stirred at room temperature for 2 hours. Sodium triacetoxyborohydride (667.0 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **19-1** (150.0 mg, yield 33.2%). LC-MS m/z (ESI): 288.0 [M+H]⁺.

### 19.2 Synthesis of intermediate 19-2

Compound **19-1** (130.0 mg), O-(7-azabenzotriazol-1-yl)-N,N,N',N'-tetramethyluronium hexafluorophosphate (258.0 mg) and N,N-diisopropylethylamine (117.0 mg) were added to a solution of compound **1-4** (180.0 mg) in N,N-dimethylformamide (5 mL), and the mixture was stirred at room temperature for 2 hours. Water (50 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid 19-2 (75.0 mg, yield 24.9%). LC-MS m/z (ESI): 665.2 [M+H]⁺.

### 19.3 Synthesis of compound 19-P1 and compound 19-P2

Trifluoroacetic acid (2 mL) was added to a solution of compound **19-2** (75 mg) in dichloromethane (4 mL) in an ice bath, and the mixture was warmed to room temperature and stirred for 2 hours. The reaction solution was concentrated under pressure. Water (10 mL) was added, and the pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by silica gel column chromatography (eluent: dichloromethane/methanol = 20/1) to give a light yellow solid **19-3** (30.0 mg, yield 57.3%). Compound **19-3** was purified by high performance liquid chromatography (column: SFC Thar prep 80, CHIRALPAK AD-H 250mm x 20 mm, 5um; mobile phase: 40% IPA (NH₄OH 0.2%) to give a white solid **19-P1** (12.0 mg, yield 40%). LC-MS m/z (ESI): 464.9 [M+H]⁺. ¹H NMR (400 MHz, MeOD-*d*₄) δ ppm 8.88 - 8.87 (m, 1H), 8.70 - 8.68 (m, 1H), 8.14 - 7.89 (m, 2H), 7.68 - 7.60 (m, 1H), 7.54 - 7.36 (m, 2H), 6.09 - 5.81 (m, 1H), 5.01 - 4.60 (m, 2H), 2.16 - 2.09 (m, 3H), 1.75 - 1.61 (m, 3H);

A white solid **19-P2** (11.8 mg, yield 39.3%) was obtained. LC-MS m/z (ESI): 465.0 [M+H]⁺. ¹H NMR (400 MHz, MeOD-*d*₄) δ ppm 8.88 - 8.87 (m, 1H), 8.70 - 8.68 (m, 1H), 8.14 - 7.89 (m, 2H), 7.68 - 7.60 (m, 1H), 7.55 - 7.36 (m, 2H), 6.09 - 5.81 (m, 1H), 5.01 - 4.60 (m, 2H), 2.16 - 2.10 (m, 3H), 1.75 - 1.61 (m, 3H).

### 20. Synthesis of example 20

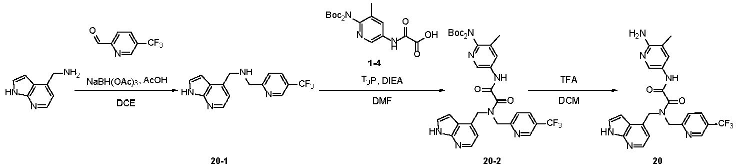

### 20.1 Synthesis of intermediate 20-1

5-(Trifluoromethyl)pyridine-2-carboxaldehyde (200.0 mg) was added to a solution of 4-aminomethyl-7-azaindole (167.0 mg) and acetic acid (136.0 mg) in 1,2-dichloroethane (5 mL), and the mixture was stirred at room temperature for 2 hours. Sodium triacetoxyborohydride (481.0 mg) was added, and the mixture was stirred for 30 minutes. Water (20 mL) was added, and the pH was adjusted to weak alkalinity with saturated sodium carbonate solution. The mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a yellow oil **20-1** (250.0 mg, yield 71.4%). LC-MS m/z (ESI): 307.1 [M+H]⁺.

### 20.2 Synthesis of intermediate 20-2

Compound **20-1** (36.5 mg), propylphosphonic anhydride (121.0 mg, 50% ethyl acetate solution) and N,N-diisopropylethylamine (33.0 mg) were added to a solution of compound **1-4** (50.0 mg) in N,N-dimethylformamide (2 mL), and the mixture was stirred at room temperature for 16 hours. Water (20 mL) was added, and the mixture was extracted with ethyl acetate (20 mL × 3). The organic phase was washed with saturated sodium chloride solution, dried over anhydrous sodium sulfate and filtered. The filtrate was concentrated under reduced pressure. The residue was purified by silica gel column chromatography (eluent: petroleum ether/ethyl acetate = *5*/*1* - 2/1) to give a yellow solid **20-2** (40.0 mg, yield 46.3%). LC-MS m/z (ESI): 684.0 [M+H]⁺.

### 20.3 Synthesis of compound 20

Trifluoroacetic acid (2 mL) was added to a solution of compound **20-2** (40.0 mg) in dichloromethane (4 mL) in an ice bath, and the mixture was warmed to room temperature and stirred for 2 hours. The reaction solution was concentrated under pressure. Water (10 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **20** (10.0 mg, yield 35.3%). LC-MS m/z (ESI): 484.0 [M+H]⁺. ¹H NMR (400 MHz, MeOD-*d*₄) δ ppm 8.76 (s, 1H), 8.16 - 8.11 (m, 1H), 8.05 - 7.93 (m, 2H), 7.58 - 7.47 (m, 2H), 7.39 - 7.38(m, 1H), 7.13 - 7.03 (m, 1H), 6.58 - 6.57 (m, 1H), 5.32 - 5.06(m, 2H), 5.04 - 4.8 (m, 2H), 2.12 - 2.16 (m, 3H).

### 21. Synthesis of example 21

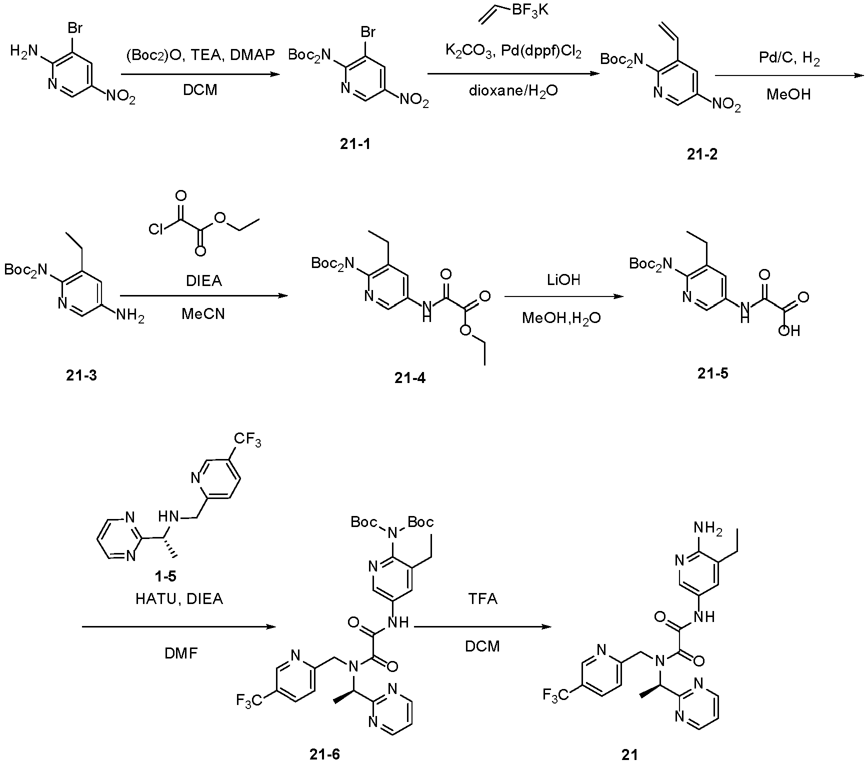

### 21.1 Synthesis of intermediate 21-1

Di-tert-butyl dicarbonate (12.5 g), 4-dimethylaminopyridine (1.4 g), and triethylamine (4.7 g) were added to a solution of 3-bromo-5-nitropyridin-2-amine (5.0 g) in dichloromethane (50 mL), and the mixture was stirred at room temperature for 12 hours. The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = *5*/*1* - 1/1) to give a light yellow solid **21-1** (8.0 g, yield 83.3%). LC-MS m/z (ESI): 440.1, 442.1 [M+Na]⁺.

### 21.2 Synthesis of intermediate 21-2

Potassium vinyltrifluoroborate (540.0 mg), potassium carbonate (1.3 g), [1,1'-bis(diphenylphosphino)ferrocene]dichloropalladium(II) (150.0 mg), and **21-1** (1.0 g) were added to a mixed solution of 1,4-dioxane (12 mL) and water (4 mL), and the mixture was stirred at 100 °C for 12 hours under nitrogen. The mixture was cooled to room temperature, and water (30 mL) was added. The mixture was extracted with dichloromethane (30 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 3/1 - 1/1) to give a light yellow solid **21-2** (500.0 mg, yield 57.1%). LC-MS m/z (ESI): 753.0 [2M+Na]⁺.

### 21.3 Synthesis of intermediate 21-3

Palladium on carbon (500.0 mg) was added to a solution of **21-2** (500.0 mg) in methanol (10 mL) under nitrogen. The system was purged with introduced hydrogen three times. The mixture was stirred at room temperature for 2 hours under hydrogen. The mixture was vacuum filtered, and the filtrate was concentrated under reduced pressure. The residue was purified by silica gel column chromatography (eluent: petroleum ether/ethyl acetate = 2/1 - 1/1) to give a light yellow solid **21-3** (400.0 mg, yield 86.6%). LC-MS m/z (ESI): 338.1 [M+H]⁺.

### 21.4 Synthesis of intermediate 21-4

Ethyl 2-chloro-2-oxoacetate (410.0 mg) was added to a solution of **21-3** (1.0 g) and N,N-diisopropylethylamine (390.0 mg) in dichloromethane (14 mL) in an ice bath, and the mixture was stirred at room temperature for 3 hours. Water (10 mL) was added, and the mixture was extracted with dichloromethane (15 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = *5*/*1* - 1/1) to give a light yellow solid **21-4** (1.0 g, yield 76.9%). LC-MS m/z (ESI): 438.1 [M+H]⁺.

### 21.5 Synthesis of intermediate 21-5

Lithium hydroxide (110.0 mg) was added to a solution of **21-4** (1.0 g) in methanol (9 mL) and water (3 mL). The mixture was stirred at room temperature for 2 hours. 2 M hydrochloric acid was added to adjust the pH to 6, and the mixture was vacuum filtered to give a light yellow solid **21-5** (800.0 mg, yield 85.5%). LC-MS m/z (ESI): 410.0 [M+H]⁺.

### 21.6 Synthesis of intermediate 21-6

O-(7-azabenzotriazol-1-yl)-N,N,N',N'-tetramethyluronium hexafluorophosphate (134.2 mg) and N,N-diisopropylethylamine (45.6 mg) were added to a solution of **21-5** (87.2 mg) and **1-5** (50.0 mg) in N,N-dimethylformamide (5 mL), and the mixture was stirred at room temperature for 5 hours. Water (10 mL) was added, and the mixture was extracted with dichloromethane (10mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1) to give a yellow oil **21-6** (40.0 mg, 40.7%). LC-MS m/z (ESI): 674.0 [M+H]⁺.

### 21.7 Synthesis of compound 21

Trifluoroacetic acid (2 mL) was added to a solution of **21-6** (40.0 mg) in dichloromethane (2 mL) in an ice bath. The mixture was stirred at room temperature for 1 hour, and concentrated under reduced pressure. The residue was purified by high performance liquid chromatography (column: Xbridge-C18 150 × 19 mm, Sum; mobile phase: ACN--H₂O (0.1% FA); B%: 5%-10%, 20 min) to give the formate of **21** (2.0 mg, yield 7.1%) as a white solid. LC-MS m/z (ESI): 474.2 [M+H]⁺. ¹H NMR (400 MHz, DMSO-*d*₆) δ 8.71 - 8.68 (m, 3H), 8.02 - 7.95 (m, 2H), 7.65 - 7.63 (m, 1H), 7.54 - 7.46 (m, 1H), 7.31 - 7.28 (m, 1H), 5.88 - 5.80 (m, 1H), 4.59 (s, 2H), 2.51 - 2.44 (m, 2H), 1.75 - 1.61 (m, 3H), 1.26 - 1.17 (m, 3H).

### 22. Synthesis of example 22

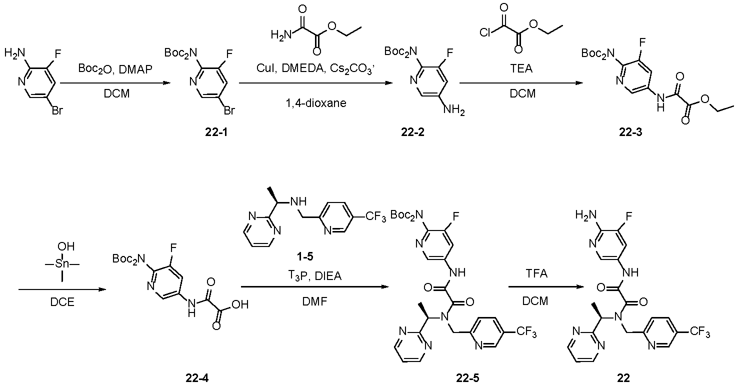

### 22.1 Synthesis of intermediate 22-1

Di-tert-butyl dicarbonate (14.3 g), triethylamine (10.6 g) and 4-dimethylaminopyridine (0.6 g) were added to a solution of 5-bromo-3-fluoropyridin-2-amine (5.0 g) in dichloromethane (50 mL), and the mixture was stirred at room temperature for 2 hours. Water (30 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 20/1) to give a white solid **22-1** (8.8 g, yield 95.3%). LC-MS m/z (ESI): 804.9 [2M+Na]⁺.

### 22.2 Synthesis of intermediate 22-2

Ethyl oxamate (134.1 mg), copper(I) iodide (15.0 mg), cesium carbonate (497.0 mg) and N,N'-dimethylethylenediamine (19.4 mg) were added to a solution of compound **22-1** (300 mg) in 1,4-dioxane (5 mL), and the mixture was stirred at 100 °C for 2 hours under nitrogen. The mixture was cooled to room temperature, and water (50 mL) was added. The mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 30/1) to give a yellow solid **22-2** (200.0 mg, yield 49.4%). LC-MS m/z (ESI): 677.3 [2M+Na]⁺.

### 22.3 Synthesis of intermediate 22-3

Ethyl 2-chloro-2-oxoacetate (82.9 mg) was added to a solution of triethylamine (61.4 mg) and compound 22-2 (200.0 mg) in dichloromethane (5 mL) in an ice bath under nitrogen, and the mixture was stirred at room temperature for 2 hours. Water (50 mL) was added, and the mixture was extracted with dichloromethane (30 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 40/1) to give a yellow oil **22-3** (200.0 mg, yield 71.4%). LC-MS m/z (ESI): 877.0 [2M+Na]⁺.

### 22.4 Synthesis of intermediate 22-4

Trimethyltin hydroxide (252.6 mg) was added to a solution of compound **22-3** (200.0 mg) in 1,2-dichloroethane (5 mL), and the mixture was stirred at 70 °C for 2 hours. The mixture was cooled to room temperature, and water (50 mL) was added. The mixture was extracted with dichloromethane (20 mL × 3). The organic phase was washed with saturated sodium chloride solution (50 mL), dried over anhydrous sodium sulfate and filtered. The filtrate was concentrated under reduced pressure to give a yellow solid crude product **22-4** (180 mg). LC-MS m/z (ESI): 821.0 [2M+Na]⁺.

### 22.5 Synthesis of intermediate 22-5

**1-5** (140.6 mg), propylphosphonic anhydride (317.6 mg) and N,N-diisopropylethylamine (128.8 mg) were added to a solution of compound **22-4** (180.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 30/1) to give a yellow oil **22-5** (80.0 mg, yield 23.2%). LC-MS m/z (ESI): 664.0 [M+H]⁺.

### 22.6 Synthesis of intermediate 22

Trifluoroacetic acid (2 mL) was added to a solution of compound **22-5** (80.0 mg) in dichloromethane (6 mL), and the mixture was stirred at room temperature for 2 hours. The mixture was concentrated under reduced pressure, and water (10 mL) was added. The pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by silica gel column chromatography (eluent: dichloromethane/methanol = 20/1) to give a light yellow solid **22** (30.0 mg, yield 53.7%). LC-MS m/z (ESI): 464.1 [M+H]⁺. ¹H NMR (400 MHz, DMSO-*d*₆) δ ppm 10.77-10.69 (m, 1H), 8.86 - 8.71 (m, 3H), 8.17 - 8.10 (m, 1H), 8.09 - 7.92 (m, 1H), 7.75 - 7.64 (m, 1H), 7.50 - 7.39 (m, 1H), 7.41 - 7.34 (m, 1H), 6.12 - 6.10 (m, 2H), 5.75 - 5.72 (m, 1H), 5.17 - 4.56 (m, 2H), 1.67 - 1.52 (m, 3H).

### 23. Synthesis of example 23

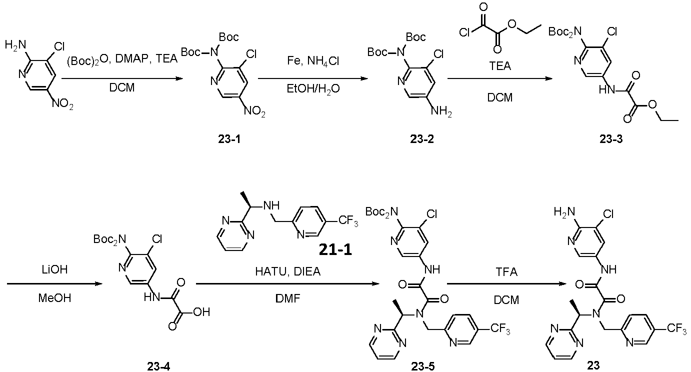

### 23.1 Synthesis of intermediate 23-1

2-Amino-3-chloro-5-nitropyridine (1.0 g) and di-tert-butyl dicarbonate (3.2 g) were added to a solution of 4-dimethylaminopyridine (140.0 mg) and triethylamine (2.4 g) in dichloromethane (20 mL), and the mixture was stirred at room temperature for 3 hours. Water (20 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: ethyl acetate/petroleum ether = 5/1) to give a white solid **23-1** (1.5 g, yield 69.4%). LC-MS m/z (ESI): 396.0 [M+Na]⁺.

### 23.2 Synthesis of intermediate 23-2

Iron powder (743.2 mg) and ammonium chloride (711.2 mg) were added to a mixed solution of **23-1** (500.0 mg) in ethanol (10 mL) and water (10 mL), and the mixture was stirred at 0 °C for 16 hours. The mixture was filtered, and water (50 mL) was added to the filtrate. The resulting mixture was extracted with ethyl acetate (30 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 10/1) to give a white solid **23-2** (220.0 mg, yield 47.8%). LC-MS m/z (ESI): 244.0 [M-100+H]⁺.

### 23.3 Synthesis of intermediate 23-3

Ethyl 2-chloro-2-oxoacetate (48.0 mg) was added to a solution of triethylamine (40.0 mg) and **23-2** (100.0 mg) in dichloromethane (5 mL) at 0 °C, and the mixture was stirred at room temperature for 2 hours. The organic phase was washed with saturated sodium chloride solution (3 mL), dried over anhydrous sodium sulfate and filtered. The filtrate was concentrated under reduced pressure. The residue was purified by silica gel column chromatography (eluent: petroleum ether/ethyl acetate = 2/1 - 1/1) to give a white solid **23-3** (100.0 mg, yield 77.4%). LC-MS m/z (ESI): 288.0 [M-156+H]⁺.

### 23.4 Synthesis of intermediate 23-4

Lithium hydroxide (20.0 mg) was added to a solution of **23-3** (100.0 mg) in methanol (3 mL) and water (1 mL), and the mixture was stirred at room temperature for 2 hours. The mixture was adjusted to acidity with 1 M hydrochloric acid, and filtered to give a white solid crude product **23-4** (100.0 mg). The crude product was directly used in the next step reaction without purification. LC-MS m/z (ESI): 437.9 [M+Na]⁺.

### 23.5 Synthesis of intermediate 23-5

**1-5** (70.0 mg), O-(7-azabenzotriazol-1-yl)-N,N,N',N'-tetramethyluronium hexafluorophosphate (142.0 mg), and N,N-diisopropylethylamine (65.0 mg) were added to a solution of **23-4** (100.0 mg) in N,N-dimethylformamide (1 mL). The mixture was stirred at room temperature for 12 hours under nitrogen. Water (1 mL) was added to the reaction solution, and the mixture was extracted with ethyl acetate (100 mL × 3). The organic phase was washed with saturated sodium chloride solution (1 mL), dried over anhydrous sodium sulfate and filtered. The filtrate was concentrated under reduced pressure to give a yellow solid crude product **23-5** (50.0 mg). LC-MS m/z (ESI): 680.0 [M+H]⁺.

### 23.6 Synthesis of intermediate 23

Trifluoroacetic acid (1 mL) was added to a solution of **23-5** (50.0 mg) in dioxymethane (2 mL) in an ice bath. The mixture was stirred at room temperature for 2 hours, and the pH was adjusted to 8 with saturated sodium carbonate solution. The mixture was extracted with dichloromethane (10 mL × 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of 23 (19.0 mg, yield 53.8%) as a yellow solid. LC-MS m/z (ESI): 480.1 [M+H]⁺. ¹H NMR (400 MHz, DMSO-*d*₆) δ ppm 10.77 - 10.60 (m, 1H), 8.83 - 8.74 (m, 3H), 8.20 - 8.06 (m, 2H), 7.92 - 7.34 (m, 3H), 6.24 - 6.18 (m, 2H), 5.77 - 5.70 (m, 1H), 5.21 - 4.56 (m, 2H), 1.65 - 1.47 (m, 3H).

### 24. Synthesis of example 24

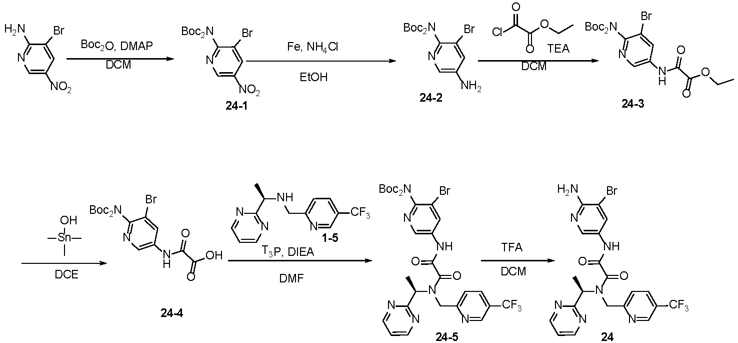

### 24.1 Synthesis of intermediate 24-1

Di-tert-butyl dicarbonate (12.5 g), triethylamine (9.3 g) and 4-dimethylaminopyridine (0.6 g) were added to a solution of 2-amino-3-bromo-5-nitropyridine (5 g) in dichloromethane (50 mL), and the mixture was stirred at room temperature for 2 hours. Water (30 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a white solid **24-1** (9.0 g, yield 97%). LC-MS m/z (ESI): 859.0 [2M+Na]⁺.

### 24.2 Synthesis of intermediate 24-2

Iron powder (664.5 mg) and ammonium chloride (636.4 mg) were added to a solution of compound **24-1** (500 mg) in ethanol (5 mL), and the mixture was stirred at 50 °C for 2 hours. Water (50 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid **24-2** (360.0 mg, yield 72.4%). LC-MS m/z (ESI): 799.0 [2M+Na]⁺.

### 24.3 Synthesis of intermediate 24-3

Ethyl 2-chloro-2-oxoacetate (82.9 mg) and triethylamine (61.4 mg) were added to a solution of compound **24-2** (200.0 mg) in dichloromethane (5 mL), and the mixture was stirred at room temperature for 2 hours. Water (50 mL) was added, and the mixture was extracted with dichloromethane (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid **24-3** (240 mg, yield 81.4%). LC-MS m/z (ESI): 998.8 [2M+Na]⁺.

### 24.4 Synthesis of intermediate 24-4

Trimethyltin hydroxide (276.6 mg) was added to a solution of compound **24-3** (240 mg) in 1,2-dichloroethane (5 mL), and the mixture was stirred at room temperature for 2 hours. Water (50 mL) was added, and the mixture was extracted with dichloromethane (50 mL × 3). The organic phase was washed with saturated sodium chloride solution (50 mL), dried over anhydrous sodium sulfate and filtered. The filtrate was concentrated under reduced pressure to give a yellow solid **24-4** (100 mg, yield 76.2%). LC-MS m/z (ESI): 942.8 [2M+Na]⁺.

### 24.5 Synthesis of intermediate 24-5

**1-5** (62.6 mg), propylphosphonic anhydride (55.9 mg) and N,N-diisopropylethylamine (55.8 mg) were added to a solution of compound **24-4** (100 mg) in N,N-dimethylformamide (3 mL), and the mixture was stirred at room temperature for 2 hours. Water (50 mL) was added, and the mixture was extracted with ethyl acetate (30 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow oil **24-5** (80 mg, yield 23.2%). LC-MS m/z (ESI): 723.9 [M+H]⁺.

### 24.6 Synthesis of compound 24

Trifluoroacetic acid (2 mL) was added to a solution of compound **24-5** (80.0 mg) in dichloromethane (6 mL), and the mixture was stirred at room temperature for 2 hours. The reaction solution was concentrated under reduced pressure. Water (10 mL) was added, and the pH was adjusted to weak alkalinity with saturated sodium bicarbonate solution. The mixture was extracted with dichloromethane (20 mL × 3). The organic phase was dried over anhydrous sodium sulfate and filtered. The filtrate was concentrated under reduced pressure. The residue was purified by silica gel column chromatography (eluent: dichloromethane/methanol = 20/1) to give a light yellow solid **24** (9.0 mg, yield 22.3%). LC-MS m/z (ESI): 524.0, 526.0 [M+H]⁺. ¹H NMR (400 MHz, DMSO-*d*₆) δ ppm 10.75 -10.25 (m, 1H), 8.86 - 8.73 (m, 3H), 8.15 - 8.10 (m, 2H), 7.68 - 7.35 (m, 3H), 6.17 - 6.11 (m, 2H), 5.71 - 5.70 (m, 1H), 5.19 - 4.56 (s, 2H), 1.59-1.52 (m, 3H).

### 25. Synthesis of example 25

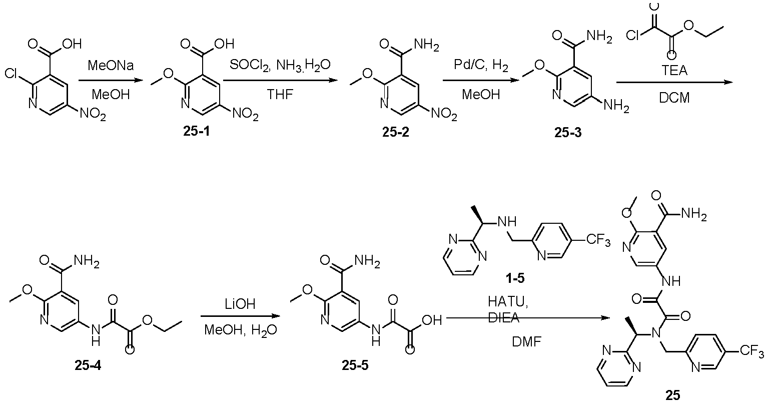

### 25.1 Synthesis of intermediate 25-1

2-Chloro-5-nitronicotinic acid (3.2 g) was added to a solution of sodium methoxide (4.3 g) in methanol (40 mL), and the mixture was stirred at 60 °C for 2 hours. The mixture was concentrated under reduced pressure, and the residue was diluted with added water (20 mL). The pH was adjusted to 7 with 1 M hydrochloric acid, and the mixture was filtered to give a light yellow solid **25-1** (1.8 g). The crude product was directly used in the next step reaction without purification. LC-MS m/z (ESI): 197.0 [M-H]⁺.

### 25.2 Synthesis of intermediate 25-2

25-1 (1.8 g) was added to a solution of thionyl chloride (20 mL), and the mixture was stirred at 80 °C for 4 hours. The mixture was concentrated under reduced pressure. Tetrahydrofuran (20 mL) was added to the residue, and the mixture was added dropwise to ammonia solution (9 mL) in an ice bath. The mixture was stirred at 0 °C for 1 hour. The mixture was warmed to room temperature, and water (50 mL) was added to dilute the mixture. The mixture was filtered to give a white solid crude product **25-2** (1.1 g). The crude product was directly used in the next step reaction without purification. LC-MS m/z (ESI): 198.1 [M+H]⁺.

### 25.3 Synthesis of intermediate 25-3

Palladium on carbon (0.1 g) was added to a solution of **25-2** (1.0 g) in methanol (12 mL) under nitrogen. The system was purged with introduced hydrogen three times, and the mixture was stirred at 25 °C for 2 hours. The mixture was filtered, and the filtrate was concentrated under reduced pressure to give an off-white solid crude product **25-3** (0.8 g). The crude product was directly used in the next step reaction without purification. LC-MS m/z (ESI): 168.1 [M+H]⁺.

### 25.4 Synthesis of intermediate 25-4

Ethyl 2-chloro-2-oxoacetate (270.0 mg) was added dropwise to a solution of crude product **25-3** (300.0 mg) and triethylamine (272.0 mg) in dichloromethane (8 mL) in an ice bath. The mixture was stirred at room temperature for 1 hour. Dichloromethane (30 mL) was added. The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = *5*/*1* - 4/1) to give a white solid **25-4** (340.0 mg, yield 63.8%). LC-MS m/z (ESI): 268.0 [M+H]⁺.

### 25.5 Synthesis of intermediate 25-5

Lithium hydroxide (34.4 mg) was added to a mixed solution of **25-5** (320.0 mg) in methanol and water (8 mL, V/V = 3:1). The mixture was stirred at room temperature for 1 hour. 1 M hydrochloric acid was added to adjust the pH to 7, and the mixture was vacuum filtered to give a white solid crude product **25-5** (200.0 mg). The crude product was directly used in the next step reaction without purification. LC-MS m/z (ESI): 239.9 [M+H]⁺.

### 25.6 Synthesis of compound 25

O-(7-azabenzotriazol-1-yl)-N,N,N',N'-tetramethyluronium hexafluorophosphate (254.4 mg) was added to a solution of N,N-diisopropylethylamine (108.1 mg), **25-5** (80.0 mg) and **1-5** (113.3 mg) in N,N-dimethylformamide (4 mL). The mixture was stirred at room temperature for 2 hours. Water (20 mL) was added, and the mixture was extracted with ethyl acetate (10 mL × 3). The organic phase was washed with saturated sodium chloride solution (10 mL), dried over anhydrous sodium sulfate and filtered. The filtrate was concentrated under reduced pressure. The residue was purified by high performance liquid chromatography (column: Xbridge-C18 150 × 19 mm, 5um; mobile phase: ACN--H₂O (0.1% FA); B%: 10%-60%, 20 min) to give the formate of **25** (27.8 mg, yield 15.7%) as a white solid. LC-MS m/z (ESI): 504.0 [M+H]⁺. ¹H NMR (400 MHz, DMSO-*d₆*) δ ppm 11.10 - 10.82 (m, 1H), 8.87 - 8.71 (m, 3H), 8.54 (dd, *J=* 14.8, 2.6 Hz, 1H), 8.48 - 8.39 (m, 1H), 8.21 - 8.07 (m, 1H), 7.80 - 7.45 (m, 3H), 7.45 - 7.34 (m, 1H), 5.78 - 5.66 (m, 1H), 5.22 - 4.91 (m, 1H), 4.90 - 4.49 (m, 1H), 3.97 - 3.91 (m, 3H), 1.67 - 1.52 (m, 3H).

### 26. Synthesis of example 26

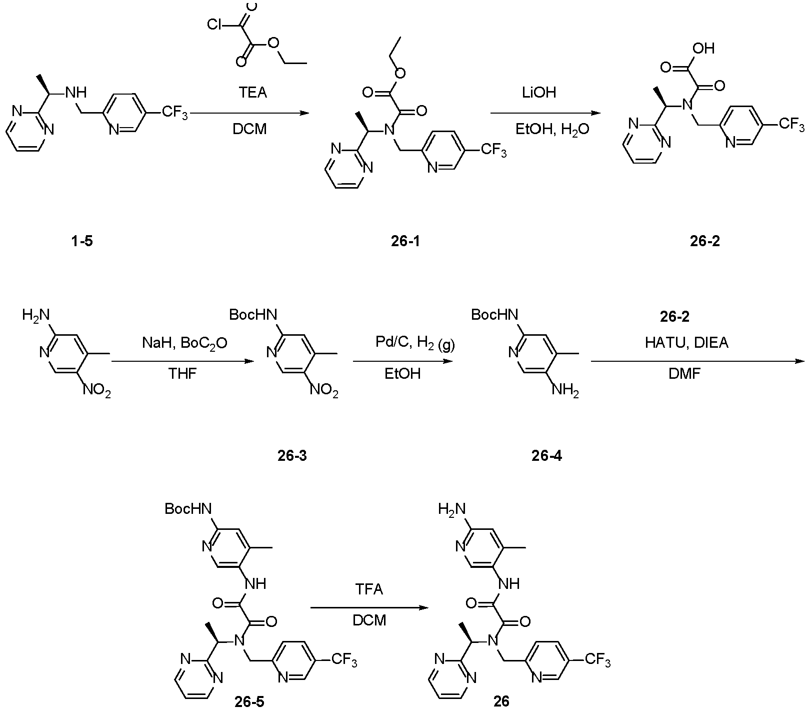

### 26.1 Synthesis of intermediate 26-1

Ethyl 2-chloro-2-oxoacetate (1.1 g) was slowly added dropwise to a solution of compound 1-5 (2.0 g) and triethylamine (0.9 g) in dichloromethane (20 mL) in an ice bath, and the mixture was warmed to room temperature and stirred for 16 hours. Water (20 mL) was added, and the mixture was extracted with dichloromethane (15 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 2/1) to give a yellow oil **26-1** (1.9 g, yield 70.1%). LC-MS m/z (ESI): 393.1 [M+H]⁺.

### 26.2 Synthesis of intermediate 26-2

Lithium hydroxide (238.0 mg) was added to a mixed solution of compound **26-1** (1.9 g) in ethanol and water (100 mL, V/V = 1:1) in an ice bath, and the mixture was warmed to room temperature and stirred for 2 hours. The mixture was concentrated under reduced pressure. Water (10 mL) was added, and the pH was adjusted to weak acidity with 2 M hydrochloric acid. The mixture was extracted with dichloromethane (15 mL × 3). The organic phase was washed with saturated sodium chloride solution (20 mL), dried over anhydrous sodium sulfate and filtered. The filtrate was concentrated under reduced pressure to give compound **26-2** (1.5 g, 85.2%). LC-MS m/z (ESI): 355.1 [M+H]⁺.

### 26.3 Synthesis of intermediate 26-3

Sodium hydride (200.0 mg) was added to a solution of 2-amino-5-nitro-4-methylpyridine (500.0 mg)in tetrahydrofuran (20 mL). The mixture was stirred at room temperature for 0.5 hours. Di-tert-butyl dicarbonate (900.0 mg) was added, and the mixture was stirred at room temperature for 2 hours. Water (50 mL) was added to the reaction solution, and the mixture was extracted with ethyl acetate (30 mL × 3). The organic phase was washed with saturated sodium chloride solution (20 mL × 2), dried over anhydrous sodium sulfate and filtered. The filtrate was concentrated under reduced pressure. The residue was purified by silica gel column chromatography (eluent: petroleum ether/ethyl acetate = 1/1) to give a yellow solid 26-3 (800.0 mg, yield 96.9%). LC-MS m/z (ESI): 198.1 [M-56+H]⁺.

### 26.4 Synthesis of intermediate 26-4

26-3 (800.0 mg) was added to a solution of palladium on carbon (200.0 mg) in ethanol (20 mL) under nitrogen. The system was purged with introduced hydrogen three times, and the mixture was stirred at room temperature for 5 hours. The mixture was filtered, and the filtrate was concentrated under reduced pressure. The residue was purified by silica gel column chromatography (eluent: petroleum ether/ethyl acetate = 1/2) to give a yellow solid 26-4 (300.0 mg, yield 40.8%). LC-MS m/z (ESI): 224.1 [M+H]⁺.

### 26.5 Synthesis of intermediate 26-5

26-4 (100.0 mg), N,N-diisopropylethylamine (173.5 mg), and O-(7-azabenzotriazol-1-yl)-N,N,N',N'-tetramethyluronium hexafluorophosphate (300.0 mg) were added to a solution of 26-2 (250.0 mg) in N,N-dimethylformamide (5 mL), and the mixture was stirred at room temperature for 2 hours. Water (10 mL) was added, and the mixture was extracted with ethyl acetate (5 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid 26-5 (35.0 mg, yield 14.8%). LC-MS m/z (ESI): 560.1 [M+H]⁺.

### 26.6 Synthesis of compound 26

Trifluoroacetic acid (1 mL) was added to a solution of 26-5 (35.0 mg) in dichloromethane (3 mL) in an ice bath. The mixture was stirred at room temperature for 1 hour, and concentrated under reduced pressure. The residue was purified by high performance liquid chromatography (column: Xbridge-C18 150 x 19 mm, 5um; mobile phase: ACN--H₂O (0.1% FA); B%: 5%-10%, 20 min) to give the formate of 26 (12.0 mg, yield 41.8%) as a white solid. LC-MS m/z (ESI): 460.0 [M+H]⁺. ¹H NMR (400 MHz, DMSO-*d*₆) δ ppm 10.10 - 9.96 (m, 1H), 8.83 - 8.74 (m, 3H), 8.14 - 8.10 (m, 1H), 7.70 - 7.63 (m, 1H), 7.46 - 7.35 (m, 2H), 6.32 - 6.25 (m, 1H), 5.95 - 5.88 (m, 3H), 5.25 - 4.97 (m, 1H), 4.92 - 4.59 (m, 1H), 2.01 - 1.82 (m, 3H), 1.66 - 1.52 (m, 3H).

### 27. Synthesis of example 27

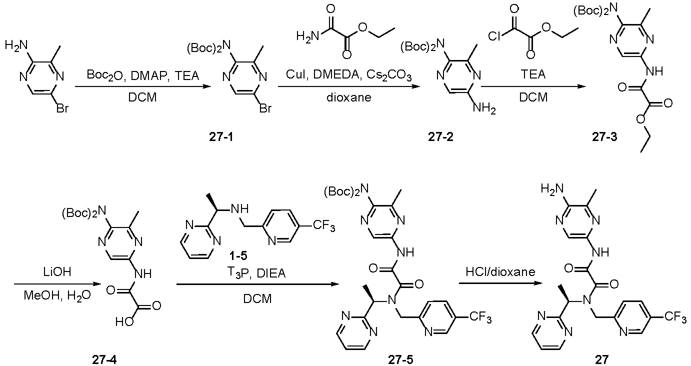

### 27.1 Synthesis of intermediate 27-1

Di-tert-butyl dicarbonate (2.9 g) was added to a solution of 4-dimethylaminopyridine (0.1 g), triethylamine (1.6 g) and 2-amino-5-bromo-3-methylpyrazine (1.0 g) in dichloromethane (20 mL), and the mixture was stirred at room temperature for 1 hour. The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 8/1 - 6/1) to give a white solid **27-1** (1.9 g, yield 83.0%). LC-MS m/z (ESI): 410.0, 411.9 [M+Na]⁺.

### 27.2 Synthesis of intermediate 27-2

Copper(I) iodide (0.1 g) was added to a solution of **27-1** (1.0 g), ethyl oxamate (0.5 g), N,N'-dimethylethylenediamine (0.1 g) and cesium carbonate (2.5 g) in 1,4-dioxane (10 mL). The mixture was stirred at 100 °C for 3 hours under nitrogen. The mixture was cooled to room temperature, and water (20 mL) was added. The mixture was extracted with ethyl acetate (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/1 - 2/3) to give a white solid **27-2** (600.0 mg, yield 65.4%). LC-MS m/z (ESI): 325.0 [M+H]⁺.

### 27.3 Synthesis of intermediate 27-3

Ethyl 2-chloro-2-oxoacetate (269.0 mg) was added dropwise to a solution of **27-2** (580.0 mg) and triethylamine (271.0 mg) in dichloromethane (8 mL) in an ice bath. The mixture was stirred at room temperature for 1 hour. Dichloromethane (10 mL) was added. The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 4/1 - 3/1) to give a white solid **27-3** (520.0 mg, yield 61.7%). LC-MS m/z (ESI): 425.0 [M+H]⁺.

### 27.4 Synthesis of intermediate 27-4

Lithium hydroxide (33.9 mg) was added to a mixed solution of **27-3** (500.0 mg) in methanol and water (5 mL, V/V = 3:1). The mixture was stirred at room temperature for 1 hour. Water (8 mL) was added, and the pH was adjusted to 7 with 1 M hydrochloric acid. The mixture was vacuum filtered to give a white solid crude product **27-4** (240.0 mg). The crude product was directly used in the next step reaction without purification. LC-MS m/z (ESI): 397.0 [M+H]⁺.

### 27.5 Synthesis of intermediate 27-5

Propylphosphonic anhydride (722.0 mg, 50% ethyl acetate solution) was added to a solution of N,N-diisopropylethylamine (244.5 mg), **27-4** (150.0 mg) and **1-5** (128.2 mg) in dichloromethane (4 mL). The mixture was stirred at room temperature for 2 hours. Iced water (20 mL) was added, and the mixture was extracted with ethyl acetate (15 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **27-5** (80.0 mg, yield 28.8%). LC-MS m/z (ESI): 661.0 [M+H]⁺.

### 27.6 Synthesis of compound 27

A solution of HCl in dioxane (2 mL, 4 M) was added to a solution of **27-5** (70.0 mg) in dioxane (1 mL) in an ice bath, and the mixture was stirred at room temperature for 4 hours. The mixture was concentrated under reduced pressure. The residue was purified by high performance liquid chromatography (column: Xbridge-C18 150 × 19 mm, 5um; mobile phase: ACN--H₂O (0.1% FA); B%: 10%-50%, 20 min) to give the formate of 27 (25.4 mg, yield 51.6%) as a white solid. LC-MS m/z (ESI): 461.2 [M+H]⁺. ¹H NMR (400 MHz, DMSO-*d*₆) δ ppm 11.03 - 10.58 (m, 1H), 8.80 - 8.71 (m, 3H), 8.41 - 8.06 (m, 2H), 7.69 - 7.54 (m, 1H), 7.39 - 7.33 (m, 1H), 6.15 - 6.11 (m, 2H), 5.71 - 5.47 (m, 1H), 5.12 - 4.65 (m, 2H), 2.28 - 2.22 (m, 3H), 1.64 - 1.53 (m, 3H).

### 28. Synthesis of example 28

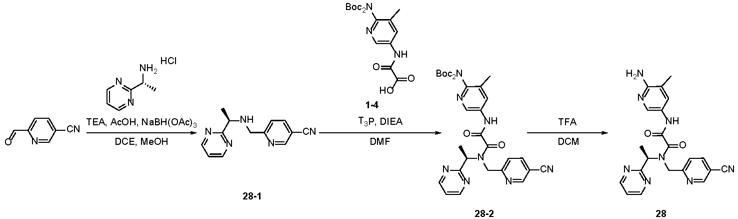

### 28.1 Synthesis of intermediate 28-1

Triethylamine (153.0 mg) was added to a mixed solution of (R)-1-(pyrimidin-2-yl)ethanamine hydrochloride (146.0 mg) in 1,2-dichloroethane/methanol (5 mL, V/V = 1:1), and the mixture was stirred at room temperature for 2 hours. Acetic acid (91.0 mg) and 5-cyanopyridine-2-carboxaldehyde (100.0 mg) were added, and the mixture was stirred at room temperature for 2 hours. Sodium triacetoxyborohydride (321.0 mg) was added, and the mixture was stirred at room temperature for 2 hours. Water (30 mL) was added, and the pH was adjusted to weak alkalinity with saturated sodium carbonate solution. The mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a yellow oil **28-1** (70.0 mg, yield 38.7%). LC-MS m/z (ESI): 240.0 [M+H]⁺.

### 28.2 Synthesis of intermediate 28-2

Compound **28-1** (45.0 mg), propylphosphonic anhydride (121.0 mg, 50% ethyl acetate solution) and N,N-diisopropylethylamine (33.0 mg) were added to a solution of compound **1-4** (50.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 50/1) to give a yellow solid **28-2** (30.0 mg, yield 38.2%). LC-MS m/z (ESI): 617.3 [M+H]⁺.

### 28.3 Synthesis of compound 28

Trifluoroacetic acid (1 mL) was added to a solution of compound **28-2** (30.0 mg) in dichloromethane (4 mL) in an ice bath, and the mixture was warmed to room temperature and stirred for 2 hours. The reaction solution was concentrated under reduced pressure. Water (10 mL) was added, and the pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: ACN--H₂O (0.1% FA); B%: 2%-35%, 14 min) to give the formate of **28** (1.9 mg, yield 9.3%) as a white solid. LC-MS m/z (ESI): 417.2 [M+H]⁺. ¹H NMR (400 MHz, MeOD-*d*₄) δ ppm 8.75 - 8.69 (m, 3H), 8.08 - 8.03 (m, 2H), 7.65 - 7.61 (m, 1H), 7.51 - 7.49 (m, 1H), 7.34 - 7.30 (m, 1H), 5.86 - 5.81 (m, 1H), 5.33 - 4.69 (m, 2H), 2.16 - 2.12 (m, 3H), 1.74 - 1.59 (m, 3H).

### 29. Synthesis of example 29

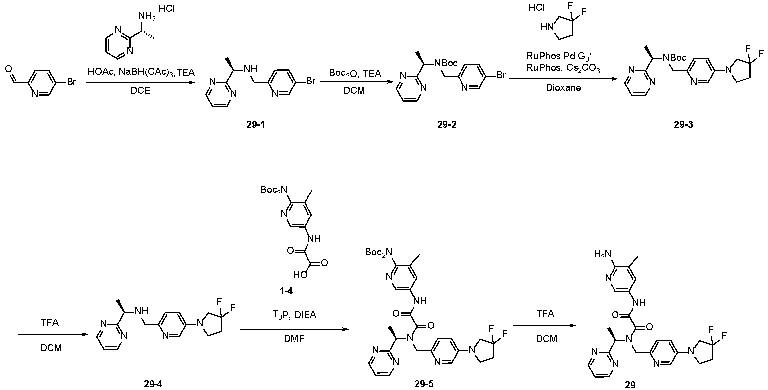

### 29.1 Synthesis of intermediate 29-1

Triethylamine (367.0 mg) was added to a solution of (R)-1-(pyrimidin-2-yl)ethanamine hydrochloride (558.0 mg) in 1,2-dichloroethane (10 mL), and the mixture was stirred at room temperature for 2 hours. Acetic acid (323.0 mg) and 5-bromopyridine-2-carbaldehyde (500.0 mg) were added, and the mixture was stirred at room temperature for 2 hours. Sodium triacetoxyborohydride (1140.0 mg) was added, and the mixture was stirred at room temperature for 2 hours. Water (100 mL)was added, and the pH was adjusted to weak alkalinity with saturated sodium carbonate solution. The mixture was extracted with dichloromethane (50 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: dichloromethane/methanol = 20/1) to give a yellow oil **29-1** (250.0 mg, yield 31.7%). LC-MS m/z (ESI): 293.1, 295.1 [M+H]⁺.

### 29.2 Synthesis of intermediate 29-2

Di-tert-butyl dicarbonate (280.0 mg) was added to a solution of compound **29-1** (250.0 mg) and triethylamine (175.0 mg) in dichloromethane (5 mL), and the mixture was stirred at room temperature for 16 hours. The mixture was concentrated under reduced pressure. The residue was purified by silica gel column chromatography (eluent: petroleum ether/ethyl acetate = 5/1) to give a yellow solid **29-2** (240 mg, yield 71.6%). LC-MS m/z (ESI): 392.9, 394.9 [M+H]⁺.

### 29.3 Synthesis of intermediate 29-3

3,3-Difluoropyrrolidine hydrochloride (168.0 mg), methanesulfonato(2-dicyclohexylphosphino-2',6'-di-i-propoxy-1,1'-biphenyl)(2'-amino-1,1'-biphenyl-2-yl)pa lladium(II) (98.0 mg), 2-dicyclohexylphosphino-2',6'-diisopropoxy-1,1'-biphenyl (109.0 mg) and cesium carbonate (760.0 mg) were added to a solution of compound **29-2** (230.0 mg) in 1,4-dioxane (10 mL), and the mixture was stirred at 90 °C for 16 hours. The mixture was cooled to room temperature and filtered. The filtrate was concentrated under reduced pressure. The residue was purified by silica gel column chromatography (eluent: petroleum ether/ethyl acetate = 2/1) to give a yellow oil **29-3** (150.0 mg, yield 56.2%). LC-MS m/z (ESI): 420.0 [M+H]⁺.

### 29.4 Synthesis of intermediate 29-4

Trifluoroacetic acid (2 mL) was added to a solution of compound **29-3** (150.0 mg) in dichloromethane (5 mL) in an ice bath, and the mixture was warmed to room temperature and stirred for 2 hours. The mixture was concentrated under reduced pressure, and water (20 mL) was added. The pH was adjusted to weak alkalinity with saturated sodium carbonate solution, and the mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a yellow oil **29-4** (90.0 mg, yield 78.9%). LC-MS m/z (ESI): 320.2 [M+H]⁺.

### 29.5 Synthesis of intermediate 29-5

Compound **29-4** (90.0 mg), propylphosphonic anhydride (359.0 mg, 50% ethyl acetate solution) and N,N-diisopropylethylamine (110.0 mg) were added to a solution of compound **1-4** (167.0 mg) in N,N-dimethylformamide (5 mL), and the mixture was stirred at room temperature for 16 hours. Water (50 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a yellow solid **29-5** (90.0 mg, yield 45.9%). LC-MS m/z (ESI): 697.3 [M+H]⁺.

### 29.6 Synthesis of compound 29

Trifluoroacetic acid (1 mL) was added to a solution of compound **29-5** (90.0 mg) in dichloromethane (3 mL) in an ice bath, and the mixture was warmed to room temperature and stirred for 2 hours. The reaction solution was concentrated under reduced pressure, and water (10 mL) was added. The pH was adjusted to weak alkalinity with saturated sodium carbonate solution. The mixture was extracted with dichloromethane (30 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: ACN--H₂O (0.1% FA); B%: 2%-30%, 15 min) to give the formate of **29** (11.0 mg, yield 17.2%) as a white solid. LC-MS m/z (ESI): 497.2 [M+H]⁺. ¹H NMR (400 MHz, MeOD-*d*₄) δ ppm 8.71 - 8.69 (m, 2H), 8.08 - 7.96 (m, 1H), 7.82 - 7.72 (m, 1H), 7.62 - 7.52 (m, 1H), 7.34 - 7.21 (m, 2H), 7.00 - 6.94 (m, 1H), 5.72 - 5.62 (m, 1H), 4.96 - 4.49 (m, 2H), 3.71 - 3.63 (m, 2H), 3.55 - 3.50 (m, 2H), 2.54 - 2.49 (m, 2H), 2.14 - 2.11 (m, 3H), 1.68 - 1.60 (m, 3H).

### 30. Synthesis of example 30

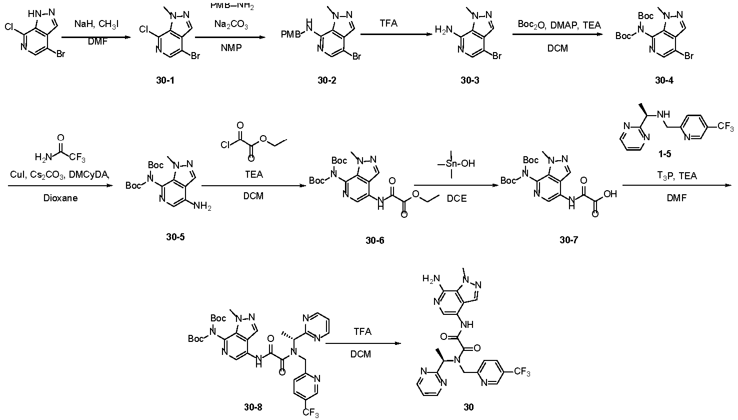

### 30.1 Synthesis of intermediate 30-1

Sodium hydride (2.0 g, 60%) was added to a solution of 4-bromo-7-chloro-1H-pyrazolo[3,4-C]pyridine (3.0 g) in N,N-dimethylformamide (30 mL) at 0 °C under nitrogen, and the mixture was stirred for 1 hour. Iodomethane (375.0 mg) was added dropwise to the reaction solution, and the mixture was warmed to room temperature and stirred for 2 hours. Water (50 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 2/1) to give a white solid 30-1 (1.9 g, yield 61.9%). LC-MS m/z (ESI): 245.9, 247.8 [M+H]⁺.

### 30.2 Synthesis of intermediate 30-2

Sodium carbonate (1.6 g) and p-anisidine (1.6 g) were added to a solution of **30-1** (1.9 g) in N-methylpyrrolidone (20 mL) under nitrogen, and the mixture was stirred at 130 °C for 2 hours. Water (60 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 2/1) to give a white solid **30-2** (1.6 g, yield 60.4%). LC-MS m/z (ESI): 346.9, 348.9 [M+H]⁺.

### 30.3 Synthesis of intermediate 30-3

**30-2** (1.6 g) was added to trifluoroacetic acid (20 mL) under nitrogen, and the mixture was stirred at 60 °C for 16 hours. Saturated sodium bicarbonate solution (60 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 2/1) to give a white solid **30-3** (800.0 mg, yield 76.9%). LC-MS m/z (ESI): 227.0, 229.1 [M+H]⁺.

### 30.4 Synthesis of intermediate 30-4

Triethylamine (1.5 g), 4-dimethylaminopyridine (80.0 mg), and di-tert-butyl dicarbonate (1.9 g) were added to a solution of **30-3** (800 mg) in dichloromethane (20 mL) under nitrogen, and the mixture was stirred at room temperature for 2 hours. Water (60 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 3/1) to give a white solid **30-4** (900.0 mg, yield 59.6%). LC-MS m/z (ESI): 449.0, 451.0 [M+Na]⁺.

### 30.5 Synthesis of intermediate 30-5

Trifluoroacetamide (250.0 mg), cesium carbonate (380.0 mg), copper(I) iodide (30.0 mg) and trans-N,N'-dimethyl-1,2-cyclohexanediamine (16.0 mg) were added to a solution of **30-4** (50.0 mg) in anhydrous dioxane (8 mL), and the mixture was stirred at 100 °C for 16 hours under nitrogen. The mixture was cooled to room temperature, and water (50 mL) was added. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 20/1) to give a yellow solid **30-5** (120.0 mg, yield 56.3%). LC-MS m/z (ESI): 364.2 [M+H]⁺.

### 30.6 Synthesis of intermediate 30-6

Ethyl 2-chloro-2-oxoacetate (45.0 mg) was added dropwise to a solution of triethylamine (35.0 mg) and 30-5 (110.0 mg) in anhydrous dichloromethane (5 mL) at 0 ° C under nitrogen, and the mixture was stirred at 0 ° C for 1 hour. The mixture was warmed to room temperature, and water (20 mL) was added. The mixture was extracted with dichloromethane (10 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 50/1) to give a yellow solid **30-6** (110 mg, yield 72.3%). LC-MS m/z (ESI): 464.0 [M+H]⁺.

### 30.7 Synthesis of intermediate 30-7

Trimethyltin hydroxide (78.0 mg) was added to a solution of **30-6** (40.0 mg) in 1,2-dichloroethane (6 mL) at room temperature, and the mixture was stirred at 50 °C for 2 hours. The mixture was cooled to room temperature. Dichloromethane (20 mL) and water (20 mL) were added, and the pH was adjusted to 4 to 5 with 1 M hydrochloric acid. The organic phase was washed with saturated sodium chloride solution (20 mL), dried over anhydrous sodium sulfate and filtered. The filtrate was concentrated under reduced pressure to give an off-white solid crude product **30-7** (40.0 mg). The crude product was directly used in the next step reaction. LC-MS m/z (ESI): 436.2 [M+H]⁺.

### 30.8 Synthesis of intermediate 30-8

Propylphosphonic anhydride (102.0 mg, 50% ethyl acetate solution) was added to a solution of triethylamine (32.0 mg), **1-5** (27.0 mg) and **30-7** (35.0 mg) in N,N-dimethylformamide (2 mL) at room temperature under nitrogen, and the mixture was stirred at room temperature for 16 hours. Ethyl acetate (30 mL) was added to dilute the mixture, and the mixture was washed with saturated sodium chloride solution (20 mL × 3). The organic phase was dried over anhydrous sodium sulfate and filtered. The filtrate was concentrated under reduced pressure. The residue was purified by silica gel column chromatography (eluent: dichloromethane/methanol = 100/1 - 30/1) to give an off-white solid **30-8** (30.0 mg, yield 53.5%). LC-MS m/z (ESI): 700.0 [M+H]⁺.

### 30.9 Synthesis of compound 30

Trifluoroacetic acid (1 mL) was added to a solution of **30-8** (30.0 mg) in dichloromethane (2 mL) in an ice bath under nitrogen. The mixture was stirred at room temperature for 1 hour, and concentrated under reduced pressure. The residue was purified by high performance liquid chromatography (column: Xbridge-C18 150 × 19 mm, 5um; mobile phase: ACN--H₂O (0.05% NH₄OH); B%: 20%-50%, 15 min) to give a white solid **30** (4.0 mg, yield 18.7%). LC-MS m/z (ESI): 500.0 [M+H]⁺. ¹H NMR (400 MHz, MeOD-*d*₄) δ ppm 8.73 - 8.69 (m, 3H), 8.05 - 7.82 (m, 2H), 7.75 - 7.66 (m, 1H), 7.64 - 7.55 (m, 1H), 7.33 - 7.29 (m, 1H), 5.91 - 5.82 (m, 1H), 5.35 - 5.12 (m, 1H), 5.05 - 4.78 (m, 1H), 4.34 - 4.30 (m, 3H), 1.76 - 1.63 (m, 3H).

### 31. Synthesis of example 31

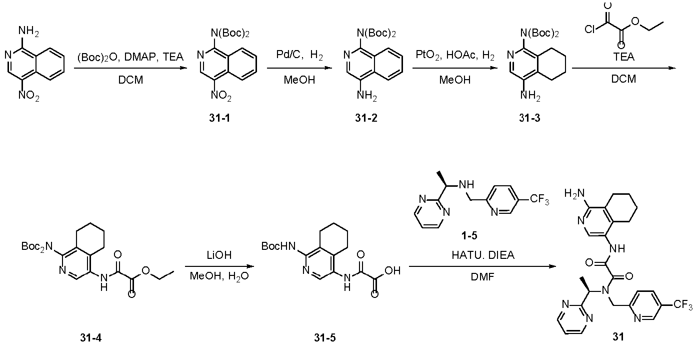

### 31.1 Synthesis of intermediate 31-1

Di-tert-butyl dicarbonate (1442.1 mg) was added to a solution of 4-dimethylaminopyridine (64.6 mg), triethylamine (802.4 mg) and 4-nitroisoquinolin-1-amine (500.0 mg) in dichloromethane (8 mL), and the mixture was stirred at room temperature for 1 hour. The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 1/1 - 2/3) to give a white solid **31-1** (830.0 mg, yield 72.5%). LC-MS m/z (ESI): 412.0 [M+Na]⁺.

### 31.2 Synthesis of intermediate 31-2

Palladium on carbon (88.5 mg) was added to a solution of **31-1** (810.0 mg) in methanol (10 mL) under nitrogen. The system was purged with hydrogen three times, and the mixture was stirred at 25 °C for 4 hours under hydrogen. The mixture was filtered, and the filtrate was concentrated under reduced pressure to give an off-white solid crude product **31-2** (600 mg). LC-MS m/z (ESI): 360.0 [M+H]⁺.

### 31.3 Synthesis of intermediate 31-3

Platinum dioxide (73.3 mg) was added to a solution of **31-2** (580.0 mg) and acetic acid (48.5 mg) in methanol (8 mL) under nitrogen. The system was purged with hydrogen three times, and the mixture was stirred at 25 °C for 16 hours under hydrogen. The mixture was filtered, and the filtrate was concentrated under reduced pressure. The residue was purified by silica gel column chromatography (eluent: dichloromethane/methanol = 12/1 - 10/1) to give an off-white solid **31-3** (500.0 mg, yield 76.7%). LC-MS m/z (ESI): 364.1 [M+H]⁺.

### 31.4 Synthesis of intermediate 31-4

Ethyl 2-chloro-2-oxoacetate (83.0 mg) was added to a solution of **31-3** (200.0 mg) and triethylamine (83.5 mg) in dichloromethane (4 mL) in an ice bath, and the mixture was stirred at room temperature for 1 hour. The mixture was diluted with dichloromethane (20 mL). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 4/1 - 3/1) to give a white solid **31-4** (240.0 mg, yield 84.7%). LC-MS m/z (ESI): 464.0 [M+H]⁺.

### 31.5 Synthesis of intermediate 31-5

Lithium hydroxide (13.6 mg) was added to a solution of **31-4** (220.0 mg) in methanol and water (5 mL, V/V = 4:1). The mixture was stirred at room temperature for 1 hour. 1 M hydrochloric acid was added to adjust the pH to 7, and the mixture was vacuum filtered to give a white solid crude product **31-5** (150.0 mg). The crude product was directly used in the next step reaction without purification. LC-MS m/z (ESI): 336.0 [M+H]⁺.

### 31.6 Synthesis of compound 31

O-(7-azabenzotriazol-1-yl)-N,N,N',N'-tetramethyluronium hexafluorophosphate (170.1 mg) was added to a solution of N,N-diisopropylethylamine (77.1 mg), **31-5** (100.0 mg) and **1-5** (101.0 mg) in N,N-dimethylformamide (2 mL). The mixture was stirred at room temperature for 1 hour. Iced water (10 mL) was added, and the mixture was extracted with ethyl acetate (10 mL × 3). The organic phase was washed with saturated sodium chloride solution (10 mL), dried over anhydrous sodium sulfate and filtered. The filtrate was concentrated under reduced pressure. The residue was purified by high performance liquid chromatography (column: Xbridge-C18 150 × 19 mm, 5um; mobile phase: ACN--H₂O (0.1% FA); B%: 10%-40%, 20 min) to give the formate of **31** (5.5 mg, yield 3.0%) as a white solid. LC-MS m/z (ESI): 500.0 [M+H]⁺. ¹H NMR (400 MHz, DMSO *-d*₆) δ ppm 10.00 - 9.89 (m, 1H), 8.85 - 8.73 (m, 3H), 8.14 - 8.11 (m, 1H), 7.65 - 7.59 (m, 1H), 7.47 - 7.35 (m, 2H), 5.69-5.64 (m, 2H), 5.26 - 4.59 (m, 2H), 2.49 - 2.05 (m, 4H), 1.70 - 1.45 (m, 8H).

### 32. Synthesis of example 32

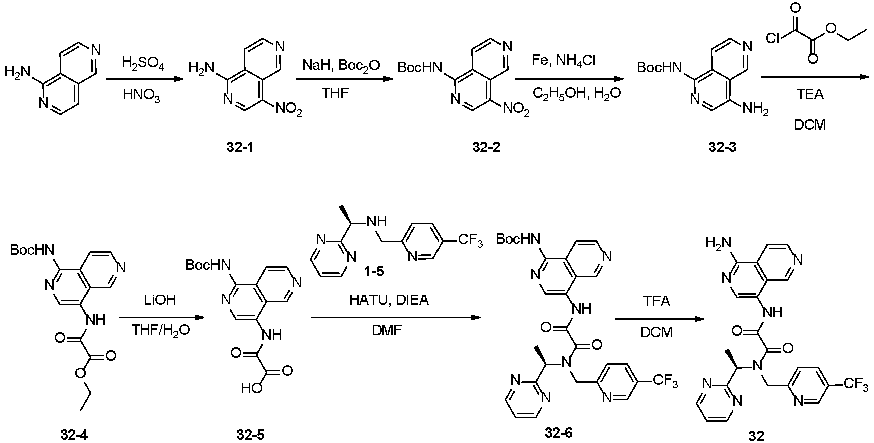

### 32.1 Synthesis of intermediate 32-1

Concentrated nitric acid (1.0 mL) was added to a solution of 2,6-naphthyridin-1-amine (1.0 g) in concentrated sulfuric acid (20 mL) at 0 °C, and the mixture was stirred for 16 hours. The reaction solution was slowly poured into saturated sodium bicarbonate solution, and the pH was adjusted to 8. The mixture was filtered, and the filter cake was dried to give a yellow solid **32-1** (1.0 g, yield 75.8%). LC-MS m/z (ESI): 191.1 [M+H]⁺.

### 32.2 Synthesis of intermediate 32-2

Sodium hydride (336.8 mg, 60%) was added to a solution of **32-1** (1.0 g) in tetrahydrofuran (500 mL) at 0 °C, and the mixture was stirred for 0.5 hours. Di-tert-butyl dicarbonate (10.2 g) was added, and the mixture was stirred at room temperature for 1 hour. Saturated ammonium chloride solution (10 mL) was added. The mixture was dried over anhydrous sodium sulfate and filtered. The filtrate was concentrated under reduced pressure. The residue was purified by silica gel column chromatography (eluent: petroleum ether/ethyl acetate = 2/1) to give a yellow solid **32-2** (1.0 g, yield 65.5%). LC-MS m/z (ESI): 291.1 [M+H]⁺.

### 32.3 Synthesis of intermediate 32-3

Reduced iron powder (1.9 g) was added to a mixed solution of **32-2** (1.0 g) and ammonium chloride (900.0 mg) in ethanol and water (30 mL, V/V = 1:9), and the mixture was stirred at 50 °C for 8 hours. Water (20 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a yellow oil **32-3** (600.0 mg, yield 66.9%). LC-MS m/z (ESI): 261.2 [M+H]⁺.

### 32.4 Synthesis of intermediate 32-4

Ethyl 2-chloro-2-oxoacetate (130.0 mg) was added to a solution of triethylamine (230.0 mg) and 32-3 (200.0 mg) in dichloromethane (10 mL) at 0 ° C. The mixture was stirred at room temperature for 2 hours. The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 1/1) to give a light yellow solid **32-4** (120.0 mg, yield 60.3%). LC-MS m/z (ESI): 233.0 [M-56+H]⁺.

### 32.5 Synthesis of intermediate 32-5

Lithium hydroxide (65.8 mg) was added to a mixed solution of **32-4** (120.0 mg) in tetrahydrofuran and water (10 mL, V/V = 5:1), and the mixture was stirred at 40 °C for 5 hours. The mixture was concentrated under reduced pressure. The residue was purified by high performance liquid chromatography (column: Daisogel-C18 30 x 250 mm, 10um; mobile phase: ACN--H₂O (0.1% FA); B%: 5%-60%, 80 min) to give the formate of **32-5** (60.0 mg, yield 54.6%) as a light yellow solid. LC-MS m/z (ESI): 333.0 [M+H]⁺.

### 32.6 Synthesis of compound 32-6

**32-5** (60.0 mg), N,N-diisopropylethylamine (69.9 mg), and O-(7-azabenzotriazol-1-yl)-N,N,N',N'-tetramethyluronium hexafluorophosphate (137.3 mg) were added to a solution of **1-5** (50.9 mg) in N,N-dimethylformamide (5 mL). The mixture was stirred at room temperature for 2 hours. Water (10 mL) was added, and the mixture was extracted with ethyl acetate (5 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/1) to give a light yellow solid **32-6** (42.7 mg, yield 11.9%). LC-MS m/z (ESI): 597.1 [M+H]⁺.

### 32.7 Synthesis of compound 32

Trifluoroacetic acid (1 mL) was added to a solution of **32-6** (42.7 mg) in dichloromethane (1 mL) in an ice bath. The mixture was stirred at room temperature for 1 hour, and concentrated under reduced pressure. The residue was purified by high performance liquid chromatography (column: Xbridge-C18 150 x 19 mm, 5um; mobile phase: ACN--H₂O (0.1% FA); B%: 5%-10%, 20 min) to give the formate of **32** (1.2 mg, yield 3.4%) as a white solid. LC-MS m/z (ESI): 497.2 [M+H]⁺. ¹H NMR (400 MHz, CD₃OD) δ ppm 9.23 - 8.98 (m, 1H), 8.69 - 8.60 (m, 3H), 8.49 - 8.31 (m, 3H), 7.97 - 7.89 (m, 3H), 7.52 - 7.44 (m, 1H), 7.27 - 7.20 (m, 1H), 5.83 - 5.81 (m, 1H), 5.31 - 5.11 (m, 1H), 4.93 - 4.89 (m, 1H), 1.67 - 1.53 (m, 3H).

### 33. Synthesis of example 33

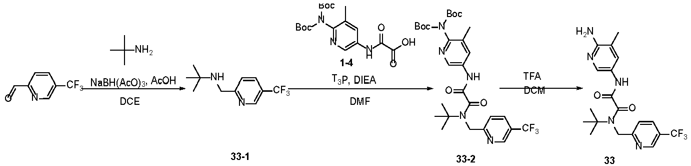

### 33.1 Synthesis of intermediate 33-1

5-(Trifluoromethyl)pyridine-2-carboxaldehyde (200.0 mg) and acetic acid (102.0 mg) were added to a solution of tert-butylamine (249.7 mg) in 1,2-dichloroethane (5 mL), and the mixture was stirred at room temperature for 2 hours. Sodium triacetoxyborohydride (721.6 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 40/1) to give a yellow oil **33-1** (220.0 mg, yield 72.3%). LC-MS m/z (ESI): 233.1 [M+H]⁺.

### 33.2 Synthesis of intermediate 33-2

Propylphosphonic anhydride (226.0 mg) and N,N-diisopropylethylamine (98.0 mg) were added to a solution of compound **33-1** (100.0 mg) and **1-4** (150.0 mg) in N,N-dimethylformamide (5 mL), and the mixture was stirred at room temperature for 5 hours. Water (50 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 30/1) to give a yellow solid **33-2** (50.0 mg, yield 25.6%). LC-MS m/z (ESI): 610.2 [M+H]⁺.

### 33.3 Synthesis of compound 33

Trifluoroacetic acid (1 mL) was added to a solution of compound **33-2** (50.0 mg) in dichloromethane (3 mL) in an ice bath, and the mixture was warmed to room temperature and stirred for 2 hours. The reaction solution was concentrated under reduced pressure. Water (10 mL) was added, and the pH was adjusted to weak alkalinity with saturated sodium carbonate solution. The mixture was extracted with dichloromethane (20 mL × 3). The organic phase was dried over anhydrous sodium sulfate and filtered. The filtrate was concentrated under reduced pressure. The residue was purified by high performance liquid chromatography (column: Gemini 5u C18 150 x 21.2 mm; mobile phase: [water(FA)-ACN]; B%: 2%-10%, 6min) to give the formate of **33** (2.0 mg, yield 10.3%) as a white solid. LC-MS m/z (ESI): 410.1 [M+H]⁺. ¹H NMR (400 MHz, MeOD-*d*₄) δ ppm 8.85 (s, 1H), 8.25 (s, 1H), 8.10 (d, *J =* 8.0, 1H), 8.01 (s, 1H), 7.71 (d, *J =* 8.0 Hz, 1H), 7.53 (s, 1H), 4.96 (s, 2H), 2.12 (s, 3H), 1.46 (s, 9H).

### 34. Synthesis of example 34

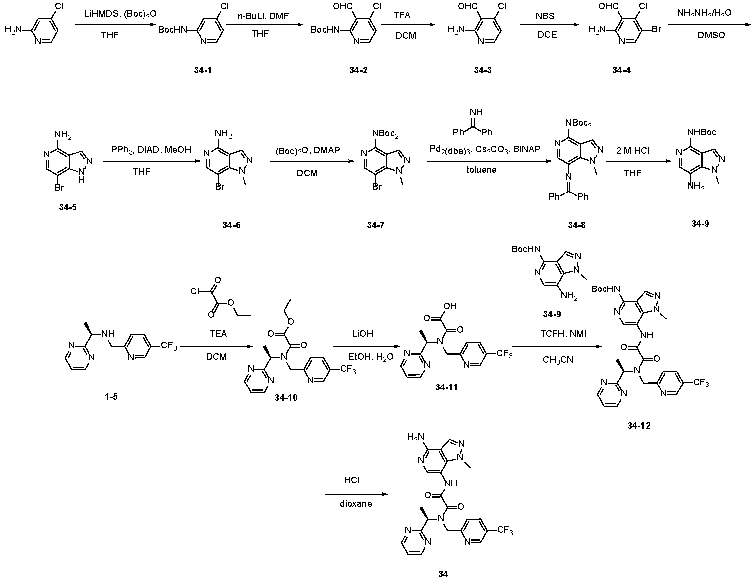

### 34.1 Synthesis of intermediate 34-1

A solution of lithium bis(trimethylsilyl)amide in tetrahydrofuran (77.8 mL, 1 M) was added to a solution of 2-amino-4-chloropyridine (5.0 g) in tetrahydrofuran (50 mL) at 0 °C, and the mixture was stirred for 0.5 hours. Di-tert-butyl dicarbonate (10.2 g) was slowly added, and the mixture was stirred at room temperature for 2 hours. Water (50 mL) was added, and the mixture was extracted with ethyl acetate (60 mL × 3). The organic phase was washed with saturated sodium chloride (50 mL), dried over anhydrous sodium sulfate and filtered. The filtrate was concentrated under reduced pressure. The residue was purified by silica gel column chromatography (eluent: petroleum ether/ethyl acetate = 2/1 - 1/1) to give a white solid **34-1** (6.0 g, yield 60.7%). LC-MS m/z (ESI): 173.0 [M-56+H]⁺.

### 34.2 Synthesis of intermediate 34-2

A solution of n-butyllithium in tetrahydrofuran (4.4 mL, 2.4 M) was added to a solution of **34-1** (1.0 g) in tetrahydrofuran (10 mL) at -78 °C. The mixture was stirred at -78 °C for 0.5 hours under nitrogen. Then, N,N-dimethylformamide (1.6 g) was added, and the mixture was stirred at room temperature for 1 hour. Saturated ammonium chloride solution (10 mL) was added to the reaction solution, and the mixture was extracted with ethyl acetate (15 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **34-2** (270.0 mg, yield 20.5%). LC-MS m/z (ESI): 156.9 [M-100+H]⁺.

### 34.3 Synthesis of intermediate 34-3

Trifluoroacetic acid (600.0 mg) was added to a solution of **34-2** (270.0 mg) in dichloromethane (3 mL) in an ice bath. The mixture was stirred at room temperature for 1 hour, and concentrated under reduced pressure. The pH was adjusted to weak alkalinity with saturated sodium bicarbonate solution. The mixture was extracted with dichloromethane (10 mL × 3). The organic phase was washed with saturated sodium chloride solution (10 mL), dried over anhydrous sodium sulfate and filtered. The filtrate was concentrated under reduced pressure to give a yellow solid crude product **34-3** (180.0 mg). The crude product was directly used in the next step reaction. LC-MS m/z (ESI): 157.0 [M+H]⁺.

### 34.4 Synthesis of intermediate 34-4

N-Bromosuccinimide (187.5 mg) was added to a solution of **34-3** (150.0 mg) in 1,2-dichloroethane (3 mL). The mixture was stirred at 50 °C for 2 hours and cooled to room temperature. The organic phase was washed with saturated sodium sulfite solution (3 mL), dried over anhydrous sodium sulfate and filtered. The filtrate was concentrated under reduced pressure to give a yellow solid **34-4** (180.0 mg). The crude product was directly used in the next step reaction. LC-MS m/z (ESI): 235.0, 237.0 [M+H]⁺.

### 34.5 Synthesis of intermediate 34-5

Hydrazine hydrate (171.9 mg) was added to a solution of **34-4** (160.0 mg) in dimethyl sulfoxide (2 mL), and the mixture was stirred at 120 °C for 2 hours. The mixture was cooled to room temperature, and water (5 mL) was added. The mixture was extracted with ethyl acetate (5 mL × 3). The organic phase was washed with saturated sodium chloride solution (5 mL), dried over anhydrous sodium sulfate and filtered. The filtrate was concentrated under reduced pressure. Then, dichloromethane (2 mL) was added, and the mixture was filtered to give a yellow solid **34-5** (60.0 mg, yield 35.2%). LC-MS m/z (ESI): 213.0, 215.0 [M+H]⁺.

### 34.6 Synthesis of intermediate 34-6

Diisopropyl azodicarboxylate (142.3 mg) was added to a solution of **34-5** (100.0 mg), triphenylphosphine (160.0 mg) and methanol (30.0 mg) in tetrahydrofuran (2 mL) at 0 °C under nitrogen, and the mixture was stirred at room temperature for 16 hours. Water (5 mL) was added, and the mixture was extracted with ethyl acetate (5 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: dichloromethane/ethyl acetate = 1/2) to give a white solid **34-6** (70.0 mg, yield 59.1%). LC-MS m/z (ESI): 226.9, 228.9 [M+H]⁺.

### 34.7 Synthesis of intermediate 34-7

Di-tert-butyl dicarbonate (148.0 mg) was added to a solution of **34-6** (70.0 mg), triethylamine (62.3 mg) and 4-dimethylaminopyridine (3.7 mg) in dichloromethane (2 mL), and the mixture was stirred at room temperature for 3 hours. The mixture was concentrated under reduced pressure. The residue was purified by silica gel column chromatography (eluent: petroleum ether/ethyl acetate = 1/1) to give a white solid **34-7** (100.0 mg, yield 89.2%). LC-MS m/z (ESI): 427.0, 429.0 [M+H]⁺.

### 34.8 Synthesis of intermediate 34-8

**34-7** (150.0 mg), benzophenone imine (127.2 mg), tris(dibenzylideneacetone)dipalladium(0) (32.1 mg), 2,2'-bis(diphenylphosphino)-1,1'-biphenyl (32.7 mg), and cesium carbonate (228.7 mg) were added to a toluene (10 mL) solution. The mixture was stirred at 110 °C for 16 hours under nitrogen. The mixture was filtered, and the filtrate was concentrated under reduced pressure. The residue was purified by silica gel column chromatography (eluent: petroleum ether/ethyl acetate = 1/3) to give a yellow solid **34-8** (100.0 mg, yield 48.6%). LC-MS m/z (ESI): 528.3 [M+H]⁺.

### 34.9 Synthesis of intermediate 34-9

Hydrochloric acid solution (1 mL, 2 M) was added to a solution of **34-8** (90.0 mg) in tetrahydrofuran (2 mL) at 0 °C, and the mixture was stirred at 0 °C for 1 hour. The mixture was concentrated under reduced pressure, and then freeze-dried to give a yellow solid **34-9** (20.0 mg). The crude product was directly used in the next step reaction without purification. LC-MS m/z (ESI): 264.1 [M+H]⁺.

### 34.10 Synthesis of intermediate 34-10

Ethyl 2-chloro-2-oxoacetate (1.1 g) was slowly added dropwise to a solution of compound **1-5** (2.0 g) and triethylamine (0.9 g) in dichloromethane (20 mL) in an ice bath, and the mixture was warmed to room temperature and stirred for 16 hours. Water (20 mL) was added, and the mixture was extracted with dichloromethane (15 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 2/1) to give a yellow oil 2 (1.9 g, yield 70.1%). LC-MS m/z (ESI): 393.1 [M+H]⁺.

### 34.11 Synthesis of intermediate 34-11

Lithium hydroxide (238.0 mg) was added to a solution of compound **34-10** (1.9 g) in a mixed solvent of ethanol and water (100 mL, V/V = 1:1) in an ice bath, and the mixture was warmed to room temperature and stirred for 2 hours. The mixture was concentrated under reduced pressure, and water (10 mL) was added. The pH was adjusted to weak acidity with 2 M hydrochloric acid, and the mixture was extracted with dichloromethane (15 mL × 3). The organic phase was washed with saturated sodium chloride solution (20 mL), dried over anhydrous sodium sulfate and filtered. The filtrate was concentrated under reduced pressure to give compound **34-11** (1.5 g, 85.2%). LC-MS m/z (ESI): 355.1 [M+H]⁺.

### 34.12 Synthesis of intermediate 34-12

N-methylimidazole (9.4 mg) was added to a solution of **34-11** (24.2 mg), **34-9** (15.0 mg) and chloro-N,N,N',N'-tetramethylformamidinium hexafluorophosphate (47.9 mg) in acetonitrile (2 mL). The mixture was stirred at room temperature for 2 hours, and water (5 mL) was added. The mixture was extracted with ethyl acetate (5 mL× 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **34-12** (25.0 mg). The crude product was directly used in the next step reaction. LC-MS m/z (ESI): 500.2 [M-100+H]⁺.

### 34.13 Synthesis of compound 34

A solution of HCl in dioxane (2 mL, 4 M) was added to a solution of **34-12** (25.0 mg) in dioxane (1 mL) in an ice bath. The mixture was stirred at room temperature for 1 hour, and the pH was adjusted to 8 with saturated sodium carbonate solution. The mixture was extracted with dichloromethane (5 mL × 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **34** (3.2 mg, yield 25.2%) as a white solid. LC-MS m/z (ESI): 500.2 [M+H]⁺. ¹H NMR (400 MHz, CD₃OD) δ ppm 8.66 - 8.61 (m, 3H), 8.14 - 8.10 (m, 1H), 7.94 - 7.91 (m, 1H), 7.57 - 7.42 (m, 2H), 7.26 - 7.20 (m, 2H), 5.87 - 5.76 (m, 1H), 5.41 - 5.05 (m, 1H), 4.03 - 3.97 (m, 3H), 1.67 - 1.50 (m, 3H).

### 35. Synthesis of example 35

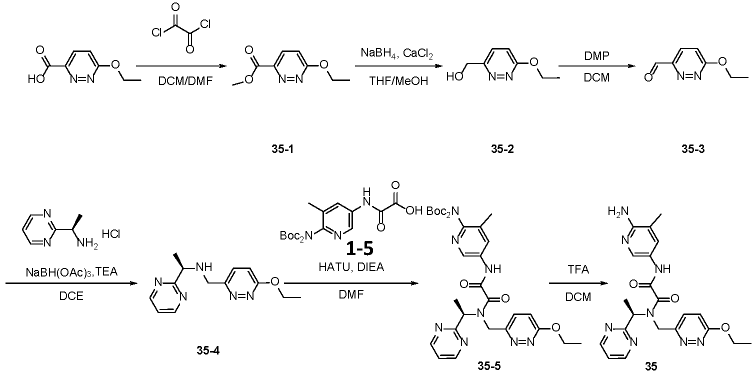

### 35.1 Synthesis of intermediate 35-1

Oxalyl chloride (755.0 mg) was added to a solution of 6-acetoxypyridazine-3-carboxylic acid (500.0 mg) in dichloromethane (10 mL) and N,N-dimethylformamide (0.1 mL) at 0 °C. The mixture was stirred at room temperature for 2 hours. Methanol (20 mL) was added, and the mixture was concentrated under reduced pressure. The residue was purified by silica gel column chromatography (eluent: petroleum ether/ethyl acetate = 3/1) to give a yellow oil **35-1** (500.0 mg, yield 92.3%). LC-MS m/z (ESI): 183.0 [M+H]⁺.

### 35.2 Synthesis of intermediate 35-2

Sodium borohydride (190.0 mg) and calcium chloride (280.0 mg) were added to a solution of **35-1** (480.0 mg) in tetrahydrofuran (5 mL) and methanol (1 mL), and the mixture was stirred at room temperature for 1 hour under nitrogen. Water (5 mL) was added to the reaction solution, and the mixture was extracted with ethyl acetate (10 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: dichloromethane/methanol = 10/1) to give a colorless oil **35-2** (400.0 mg, yield 98.5%). LC-MS m/z (ESI): 155.1 [M+H]⁺.

### 35.3 Synthesis of intermediate 35-3

Dess-Martin periodinane (1.1 g) was added to a solution of **35-2** (200.0 mg) in dichloromethane (10 mL) at 0 ° C. The mixture was stirred at room temperature for 2 hours and filtered. The filtrate was concentrated under reduced pressure. The residue was purified by silica gel column chromatography (eluent: petroleum ether/ethyl acetate = 1/1) to give a colorless oil **35-3** (190.0 mg, yield 96.3%). LC-MS m/z (ESI): 153.2 [M+H]⁺.

### 35.4 Synthesis of intermediate 35-4

(R)-1-(pyrimidine-2-amino)ethanamine hydrochloride (420.0 mg) and triethylamine (265.8 mg) were added to a solution of **35-3** (200.0 mg) in 1,2-chloroethane (10 mL). The mixture was stirred at room temperature for 1 hour under nitrogen. Then, sodium triacetoxyborohydride (557.0 mg) was added, and the mixture was stirred at room temperature for 1 hour. The organic phase was washed with saturated sodium chloride solution (5 mL), dried over anhydrous sodium sulfate and filtered. The filtrate was concentrated under reduced pressure to give a yellow oil **35-4** (140.0 mg). The crude product was directly used in the next step reaction without purification. LC-MS m/z (ESI): 260.2 [M+H]⁺.

### 35.5 Synthesis of intermediate 35-5

**1-5** (240.0 mg), O-(7-azabenzotriazol-1-yl)-N,N,N',N'-tetramethyluronium hexafluorophosphate (360.0 mg), and N,N-diisopropylethylamine (120.0 mg) were added to a solution of **35-4** (120.0 mg) in N,N-dimethylformamide (3 mL). The mixture was stirred at room temperature for 12 hours. Water (5 mL) was added to the reaction solution, and the mixture was extracted with ethyl acetate (5 mL × 3). The organic phase was washed with saturated sodium chloride solution (3 mL), dried over anhydrous sodium sulfate and filtered. The filtrate was concentrated under reduced pressure to give a white solid **35-5** (50.0 mg). The crude product was directly used in the next step reaction without purification. LC-MS m/z (ESI): 637.3 [M+H]⁺.

### 35.6 Synthesis of compound 35

Trifluoroacetic acid (0.5 mL) was added to a solution of **35-5** (50.0 mg) in dichloromethane (1 mL) in an ice bath. The mixture was stirred at room temperature for 2 hours, and the pH was adjusted to 8 with saturated sodium carbonate solution. The mixture was extracted with dichloromethane (10 mL× 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **35** (27.16 mg, yield 79.2%) as a white solid. LC-MS m/z (ESI): 437.2 [M+H]⁺. ¹H NMR (400 MHz, DMSO-*d*₆) δ ppm 10.52 - 10.44 (m, 1H), 8.76 - 8.73 (m, 2H), 8.03 - 7.96 (m, 1H), 7.77 - 7.52 (m, 1H), 7.47 - 7.43 (m, 1H), 7.40 - 7.34 (m, 1H), 7.13 - 7.10 (m, 1H), 5.68 - 5.54 (m, 3H), 5.13 - 4.59 (m, 2H), 4.42 - 4.38 (m, 2H), 2.03 - 1.99 (m, 3H) 1.65 - 1.52 (m, 3H), 1.38 - 1.32 (m, 3H).

### 36. Synthesis of example 36

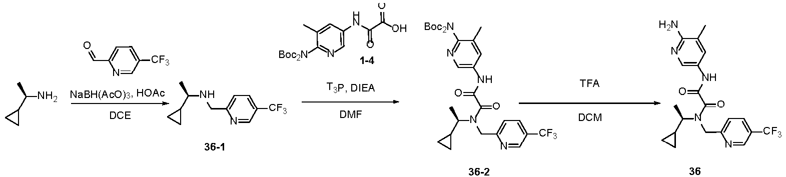

### 36.1 Synthesis of intermediate 36-1

5-(Trifluoromethyl)pyridine-2-carboxaldehyde (412.0 mg) was added to a solution of (R)-1-cyclopropyl-1-amine (200.0 mg) in 1,2-chloroethane (5 mL) and acetic acid (0.5 mL). The mixture was stirred at room temperature for 1 hour under nitrogen. Then, sodium triacetoxyborohydride (996.0 mg) was added, and the mixture was stirred at room temperature for 1 hour. Saturated sodium bicarbonate solution (10 mL) was added to the reaction solution, and the mixture was extracted with dichloromethane (15 mL × 3). The organic phase was washed with saturated sodium chloride solution (10 mL), dried over anhydrous sodium sulfate and filtered. The filtrate was concentrated under reduced pressure to give a light yellow oil 36-1 (200.0 mg). The crude product was directly used in the next step reaction without purification. LC-MS m/z (ESI): 245.2 [M+H]⁺.

### 36.2 Synthesis of intermediate 36-2

**1-4** (422.3 mg), propylphosphonic anhydride (522.3 mg), and N,N-diisopropylethylamine (223.0 mg) were added to a solution of **36-1** (200.0 mg) in N,N-dimethylformamide (2 mL). The mixture was stirred at room temperature for 12 hours. Water (5 mL) was added to the reaction solution, and the mixture was extracted with ethyl acetate (5 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 2/1) to give a white solid **36-2** (100.0 mg, yield 19.6%). LC-MS m/z (ESI): 622.2 [M+H]⁺.

### 36.3 Synthesis of compound 36

Trifluoroacetic acid (1 mL) was added to a solution of **36-2** (100.0 mg) in dichloromethane (2 mL) in an ice bath. The mixture was stirred at room temperature for 2 hours, and the pH was adjusted to 8 with saturated sodium carbonate solution. The mixture was extracted with dichloromethane (10 mL × 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **36** (43.21 mg, yield 63.7%) as a white solid. LC-MS m/z (ESI): 422.2 [M+H]⁺. ¹H NMR (400 MHz, DMSO-*d*₆) δ ppm 10.54 - 10.32 (m, 1H), 8.92 - 8.86 (m, 1H), 8.24 - 8.20 (m, 1H), 8.01 - 7.89 (m, 1H), 7.78 - 7.58 (m, 1H), 7.48 - 7.32 (m, 1H), 5.68 - 5.60 (m, 2H), 5.02 - 4.73 (m, 2H), 3.29 - 3.25 (m, 1H), 2.04 - 1.97 (m, 3H),1.29 - **1.14** (m, 3H), 0.99 - 0.96 (m, 1H), 0.50 - 0.48 (m, 1H), 0.31 - 0.11 (m, 3H).

### 37. Synthesis of example 37

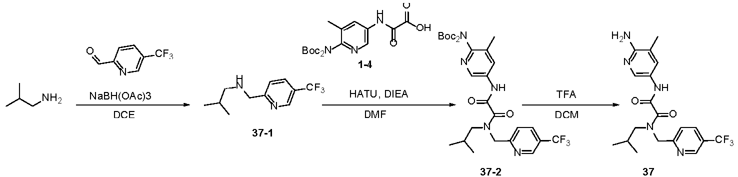

### 37.1 Synthesis of intermediate 37-1

Isobutylamine (200.0 mg) and sodium acetate borohydride (320.0 mg) were added to a solution of 5-(trifluoromethyl)-pyridine-2-carboxaldehyde (230.0 mg) in 1,2-dichloroethane (10 mL). The mixture was stirred at room temperature for 2 hours. Saturated sodium bicarbonate solution (10 mL) was added, and the mixture was extracted with dichloromethane (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/1) to give a light yellow solid **37-1** (180.0 mg, yield 28.6%). LC-MS m/z (ESI): 233.0 [M+H]⁺.

### 37.2 Synthesis of intermediate 37-2

**37-1** (100.0 mg), N,N-diisopropylethylamine (166.1 mg), and O-(7-azabenzotriazol-1-yl)-N,N,N',N'-tetramethyluronium hexafluorophosphate (320.0 mg) were added to a solution of **1-4** (170.0 mg) in N,N-dimethylformamide (5 mL). The mixture was stirred at room temperature for 2 hours. Water (10 mL) was added, and the mixture was extracted with ethyl acetate (5 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **37-2** (122.7 mg, yield 46.7%). LC-MS m/z (ESI): 610.2 [M+H]⁺.

### 37.3 Synthesis of compound 37

Trifluoroacetic acid (2 mL) was added to a solution of **37-2** (122.7 mg) in dichloromethane (2 mL) in an ice bath. The mixture was stirred at room temperature for 1 hour, and concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **37** (73.0 mg, yield 74.3%) as a white solid. LC-MS m/z (ESI): 410.1 [M+H]⁺. ¹H NMR (400 MHz, DMSO-*d*₆) δ ppm 10.48 - 10.34 (m, 1H), 8.95 - 8.91 (m, 1H), 8.26 - 8.20 (m, 1H), 8.03 - 7.93 (m, 1H), 7.61 - 7.55 (m, 1H), 7.49 - 7.39 (m, 1H), 5.67 - 5.62 (m, 2H), 4.93 - 4.75 (m, 2H), 3.39 (d, *J* = 8.0 Hz, 1H), 3.21 (d, *J* = 8.0 Hz, 1H), 2.04 - 1.96 (m, 3H), 1.96 - 1.94 (m, 1H), 0.89 - 0.82 (m, 6H).

### 38. Synthesis of example 38

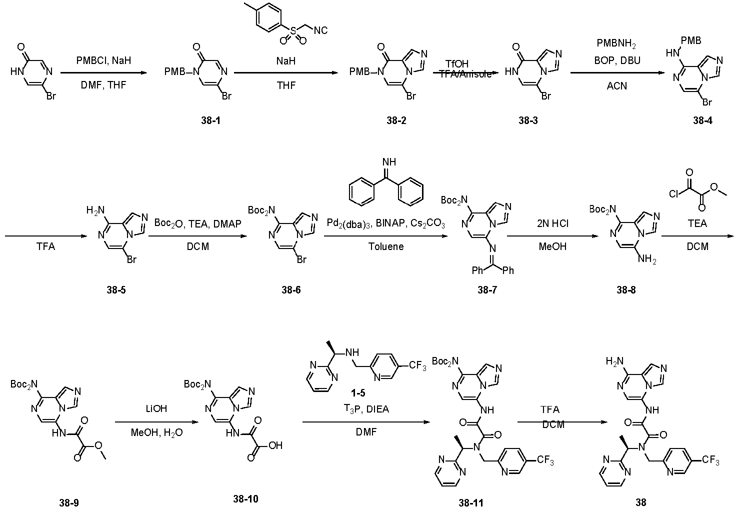

### 38.1 Synthesis of intermediate 38-1

A mixed solution of 2-bromo-5-hydroxypyrazine (10.0 g) in N,N-dimethylformamide (30 mL) and tetrahydrofuran (30 mL) was slowly added dropwise to a solution of sodium hydride (2.7 g, 60%) in N,N-dimethylformamide (30 mL) at 0 °C under nitrogen, and the mixture was stirred at 0 °C for 30 minutes. A solution of 4-methoxybenzyl chloride (10.0 g) in tetrahydrofuran (30 mL) was added, and the mixture was slowly warmed to room temperature and stirred for 16 hours. The mixture was cooled to 0 °C, and saturated ammonium chloride solution (10 mL) was added dropwise to quench the reaction. Water (500 mL) was added to dilute the mixture. The mixture was extracted with ethyl acetate (100 mL × 3). The organic phase was washed with saturated sodium chloride solution (50 mL × 3), dried over anhydrous sodium sulfate and filtered. The filtrate was concentrated under reduced pressure. The residue was purified by silica gel column chromatography (eluent: petroleum ether/ethyl acetate = 3/1 - 2/1) to give a white solid **38-1** (11.0 g, yield 65.5%). LC-MS m/z (ESI): 316.9, 318.9 [M+Na]⁺.

### 38.2 Synthesis of intermediate 38-2

A solution of p-toluenesulfonylmethyl isocyanide (8.9 g) and **38-1** (12.2 g) in tetrahydrofuran (60 mL) was slowly added dropwise to a solution of sodium hydride (2.3 g, 60%) in tetrahydrofuran (60 mL) at 0 °C under nitrogen, and the mixture was stirred at 0 °C for 30 minutes. The mixture was warmed to room temperature and stirred for 1 hour. The mixture was cooled to 0 °C, and saturated ammonium chloride solution (5 mL) was added dropwise to quench the reaction. Water (500 mL) was added to dilute the mixture. The mixture was extracted with ethyl acetate (100 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: petroleum ether/ethyl acetate = 2/3 - 1/4) to give a white solid **38-2** (10.0 g, yield 72.4%). LC-MS m/z (ESI): 333.9, 335.9 [M+H]⁺.

### 38.3 Synthesis of intermediate 38-3

Trifluoromethanesulfonic acid (25 mL) was added to a mixed solution of **38-2** (10.0 g) in trifluoroacetic acid (100 mL) and anisole (45 mL) at 0 °C under nitrogen. The mixture was warmed to room temperature and stirred for 30 minutes, and stirred at 40 °C for 1 hour. The mixture was concentrated under reduced pressure. The residue was slowly poured into an ice water mixture of sodium bicarbonate, and the mixture was concentrated at 60 °C under reduced pressure. The residue was slurried with dichloromethane and methanol (200 mL, V/V = 10:1), and the mixture was filtered. The filtrate was concentrated under reduced pressure. The residue was purified by silica gel column chromatography (eluent: dichloromethane/methanol = *100*/*5* - 100/6) to give a white solid **38-3** (4.5 g, yield 71.4%). LC-MS m/z (ESI): 213.9, 215.9 [M+H]⁺.

### 38.4 Synthesis of intermediate 38-4

4-Methoxybenzylamine (4.6 g), 1,8-diazacyclo[5.4.0]undec-7-ene (6.8 g) and benzotriazol-1-yloxytris(dimethylamino)phosphonium hexafluorophosphate (14.8 g) were added to a solution of **38-3** (4.5 g) in anhydrous acetonitrile (80 mL), and the mixture was stirred at 80 °C for 16 hours. The mixture was cooled to room temperature, concentrated under reduced pressure, and diluted with added water (100 mL). The mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = 3/1 - 1/1) to give a light yellow solid **38-4** (6.0 g, yield 85.7%). LC-MS m/z (ESI): 333.1, 335.0 [M+H]⁺.

### 38.5 Synthesis of intermediate 38-5

**38-4** (5.0 g) was added to trifluoroacetic acid (50 mL), and the mixture was stirred at 80 °C for 16 hours. The mixture was cooled to room temperature and concentrated under reduced pressure. The pH of the residue was adjusted to weak alkalinity with saturated sodium bicarbonate solution, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 100/3) to give a yellow solid **38-5** (2.3 g, yield 71.8%). LC-MS m/z (ESI): 213.0, 214.9 [M+H]⁺.

### 38.6 Synthesis of intermediate 38-6

Di-tert-butyl dicarbonate (5.9 g), triethylamine (3.2 g), and 4-dimethylaminopyridine (0.3 g) were added to a solution of **38-5** (2.3 g) in dichloromethane (30 mL), and the mixture was stirred at room temperature for 16 hours. Dichloromethane (100 mL) was added, and the mixture was washed with saturated sodium chloride solution (50 mL). The organic phase was dried over anhydrous sodium sulfate and filtered. The reaction solution was concentrated under reduced pressure. The residue was purified by silica gel column chromatography (eluent: petroleum ether/ethyl acetate = 3/1) to give a yellow solid **38-6** (2.5 g, yield 54.3%). LC-MS m/z (ESI): 412.9, 414.9 [M+H]⁺.

### 38.7 Synthesis of intermediate 38-7

Benzophenone imine (440.0 mg), cesium carbonate (790.0 mg), 2,2'-bis(diphenylphosphino)-1,1'-binaphthyl (150.0 mg) and tris(dibenzylideneacetone)dipalladium(0) (110.0 mg) were added to a solution of **38-6** (500.0 mg) in anhydrous toluene (20 mL), and the mixture was stirred at 100 °C for 16 hours under nitrogen. The mixture was cooled to room temperature, and diluted with added water (50 mL). The mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 4/1 - 3/1) to give a yellow solid **38-7** (500.0 mg, yield 80.3%). LC-MS m/z (ESI): 514.1 [M+H]⁺.

### 38.8 Synthesis of intermediate 38-8

Hydrochloric acid (5 mL, 2 M) was added to a solution of **38-7** (500.0 mg) in methanol (5 mL), and the mixture was stirred at room temperature for 30 minutes. The pH was adjusted to weak alkalinity with saturated sodium bicarbonate solution, and the mixture was extracted with ethyl acetate (30 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 100/31 - 100/3) to give an off-white solid **38-8** (300.0 mg, yield 88.3%). LC-MS m/z (ESI): 350.2 [M+H]⁺.

### 38.9 Synthesis of intermediate 38-9

Methyl 2-chloro-2-oxoacetate (83.0 mg) and triethylamine (80.0 mg) were added to a solution of **38-8** (200.0 mg) in dichloromethane (5 mL), and the mixture was stirred at room temperature for 3 hours. Water (10 mL) was added to dilute the mixture, and the mixture was extracted with ethyl acetate (20 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 1/1) to give an off-white solid **38-9** (100.0 mg, yield 40.2%). LC-MS m/z (ESI): 436.1 [M+H]⁺.

### 38.10 Synthesis of intermediate 38-10

Lithium hydroxide (15.0 mg) was added to a solution of **38-9** (90.0 mg) in methanol (3 mL) and water (3 mL), and the mixture was stirred at room temperature for 2 hours. Water (30 mL) was added, and the pH was adjusted to 6 with 1 M hydrochloric acid. The mixture was extracted with ethyl acetate (20 mL × 3). The organic phase was washed with saturated sodium chloride solution (30 mL), dried over anhydrous sodium sulfate and filtered. The filtrate was concentrated under reduced pressure to give a white solid 38-10 (50.0 mg). The crude product was directly used in the next step reaction. LC-MS m/z (ESI): 422.0 [M+H]⁺.

### 38.11 Synthesis of intermediate 38-11

Propylphosphonic anhydride (60.7 mg, 50% ethyl acetate solution) was added to a solution of N,N-diisopropylethylamine (25.0 mg), **38-10** (25.0 mg) and **1-5** (17.0 mg) in N,N-dimethylformamide (2 mL). The mixture was stirred at room temperature for 16 hours. The pH was adjusted to weak alkalinity with saturated sodium bicarbonate solution, and the mixture was extracted with ethyl acetate (20 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **38-11** (20.0 mg, yield 49.2%). LC-MS m/z (ESI): 686.0 [M+H]⁺.

### 38.12 Synthesis of compound 38

Trifluoroacetic acid (0.5 mL) was added to a solution of **38-11** (20.0 mg) in dichloromethane (2 mL) in an ice bath, and the mixture was stirred at room temperature for 1 hour. The mixture was concentrated under reduced pressure. The residue was purified by high performance liquid chromatography (column: Xbridge-C18 150 × 19 mm, 5um; mobile phase: ACN--H₂O (0.1% FA); B%: 10%-30%, 15 min) to give the formate of**38** (2.0 mg, yield 14.2%) as a white solid. LC-MS m/z (ESI): 486.1 [M+H]⁺. ¹H NMR (400 MHz, MeOD-*d*₄) δ ppm 8.77 (s, 1H), 8.73 - 8.51 (m, 3H), 8.18 - 8.13 (m, 1H), 7.95 - 7.68 (m, 3H), 7.31 - 7.22 (m, 1H), 5.27 - 4.97 (m, 2H), 4.65 - 4.55 (m, 1H), 1.77 - 1.58 (m, 3H).

### 39. Synthesis of example 39

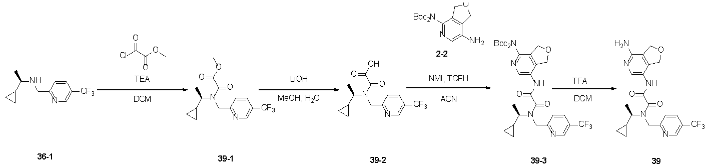

### 39.1 Synthesis of intermediate 39-1

Methyl 2-chloro-2-oxoacetate (60.0 mg) was added to a solution of triethylamine (60.0 mg) and **36-1** (100.0 mg) in dichloromethane (5 mL) in an ice bath, and the mixture was stirred at room temperature for 3 hours. Water (20 mL) was added to dilute the mixture, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1 - 2/1) to give a white solid **39-1** (120.0 mg, yield 88.9%). LC-MS m/z (ESI): 331.0 [M+H]⁺.

### 39.2 Synthesis of intermediate 39-2

Lithium hydroxide (30.0 mg) was added to a mixed solution of **39-1** (120.0 mg) in methanol (3 mL) and water (3 mL), and the mixture was stirred at room temperature for 2 hours. Water (20 mL) was added, and the pH was adjusted to 6 with 1 M hydrochloric acid. The mixture was extracted with ethyl acetate (10 mL × 3). The organic phase was washed with saturated sodium chloride solution (20 mL), dried over anhydrous sodium sulfate and filtered. The filtrate was concentrated under reduced pressure to give a white solid **39-2** (80.0 mg, yield 69.6%). The crude product was directly used in the next step reaction. LC-MS m/z (ESI): 317.0 [M+H]⁺.

### 39.3 Synthesis of intermediate 39-3

N-methylimidazole (23.0 mg) and chloro-N,N,N',N'-tetramethylformamidinium hexafluorophosphate (64.0 mg) were added to a solution of **2-2** (40.0 mg) and **39-2** (43.0 mg) in acetonitrile (2 mL), and the mixture was stirred at room temperature for 2 hours. Water (20 mL) was added to dilute the mixture, and the mixture was extracted with ethyl acetate (20 mL × 3). The organic phase was washed with saturated sodium chloride solution (2 mL), dried over anhydrous sodium sulfate and filtered. The filtrate was concentrated under reduced pressure. The residue was purified by silica gel column chromatography (eluent: petroleum ether/ethyl acetate = 3/1 - 1/1) to give a white solid **39-3** (20.0 mg, yield 22.7%). LC-MS m/z (ESI): 650.3 [M+H]⁺.

### 39.4 Synthesis of compound 39

Trifluoroacetic acid (1 mL) was added to a solution of **39-3** (20.0 mg) in dichloromethane (2 mL) in an ice bath, and the mixture was stirred at room temperature for 1 hour. The mixture was concentrated under reduced pressure. The residue was purified by high performance liquid chromatography (column: Xbridge-C18 150 × 19 mm, 5um; mobile phase: ACN--H₂O (0.1% FA); B%: 5%-30%, 80 min) to give the formate of **39** (1.64 mg, yield 12.6%) as a white solid. LC-MS m/z (ESI): 450.1 [M+H]⁺. ¹H NMR (400 MHz, MeOD-*d*₄) δ ppm 8.84 (d, *J=* 12.0 Hz, 1H), 8.10 (d, *J =* 8.0 Hz, 1H), 7.90 - 7.65 (m, 2H), 5.22 - 5.07 (m, 1H), 5.04 - 4.89 (m, 4H), 4.87 - 4.78 (m, 1H), 3.89 - 3.35 (m, 1H), 1.40 - 1.21 (m, 3H), 1.03 - 0.95 (m, 1H), 0.67 - 0.48 (m, 1H), 0.44 - 0.15 (m, 3H).

### 40. Synthesis of example 40

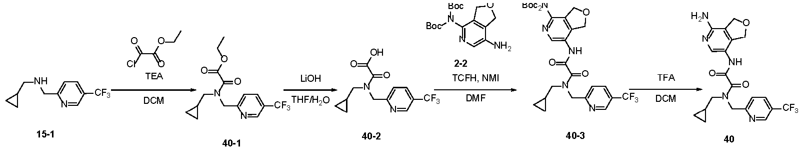

### 40.1 Synthesis of intermediate 40-1

Ethyl 2-chloro-2-oxoacetate (126.0 mg) was added to a solution of triethylamine (132.0 mg) and **15-1** (200.0 mg) in dichloromethane (5 mL) in an ice bath, and the mixture was warmed to room temperature and stirred for 2 hours. The organic phase was washed with saturated sodium chloride solution (2 mL), dried over anhydrous sodium sulfate and filtered. The filtrate was concentrated under reduced pressure. The residue was purified by silica gel column chromatography (eluent: petroleum ether/ethyl acetate = 5/1 - 1/1) to give a light yellow liquid **40-1** (200.0 mg, yield 69.7%). LC-MS m/z (ESI): 331.0 [M+H]⁺.

### 40.2 Synthesis of intermediate 40-2

Lithium hydroxide (29.0 mg) was added to a mixed solution of **40-1** (200.0 mg) in tetrahydrofuran (3 mL) and water (1 mL), and the mixture was stirred at room temperature for 2 hours. The mixture was adjusted to acidity with 1 M hydrochloric acid. The mixture was extracted with dichloromethane (5 mL × 3). The organic phase was washed with saturated sodium chloride solution (2 mL), dried over anhydrous sodium sulfate and filtered. The filtrate was concentrated under reduced pressure to give a light yellow solid **40-2** (160.0 mg). The crude product was directly used in the next step reaction without purification. LC-MS m/z (ESI): 303.0 [M+H]⁺.

### 40.3 Synthesis of intermediate 40-3

2-2 (30.0 mg), chloro-N,N,N',N'-tetramethylformamidinium hexafluorophosphate (50.0 mg), and N-methylimidazole (15.0 mg) were added to a solution of **40-2** (52.0 mg) in N,N-dimethylformamide (1 mL). The mixture was stirred at room temperature for 12 hours. Water (5 mL) was added to the reaction solution, and the mixture was extracted with ethyl acetate (5 mL × 3). The organic phase was washed with saturated sodium chloride solution (1 mL), dried over anhydrous sodium sulfate and filtered. The filtrate was concentrated under reduced pressure. The residue was purified by silica gel column chromatography (eluent: petroleum ether/ethyl acetate = 2/1 - 1/1) to give a light yellow solid **40-3** (30.0 mg, yield 55.3%). LC-MS m/z (ESI): 636.0 [M+H]⁺.

### 40.4 Synthesis of compound 40

Trifluoroacetic acid (0.5 mL) was added to a solution of **40-3** (30.0 mg) in dichloromethane (1 mL) in an ice bath. The mixture was stirred at room temperature for 2 hours, and concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of **40** (18.8 mg, yield 91.6%) as a yellow solid. LC-MS m/z (ESI): 436.2 [M+H]⁺. ¹H NMR (400 MHz, DMSO-*d*₆) δ ppm δ 10.56 - 10.37 (m, 1H), 8.93 (d, *J = 10.0* Hz, 1H), 8.23 (d, *J=* 8.0 Hz, 1H), 7.86 - 7.73 (m, 1H), 7.67 - 7.56 (m, 1H), 6.09 - 6.05 (m, 2H), 5.02 - 4.88 (m, 2H), 4.85 (s, 2H), 4.83 - 4.65 (m, 2H), 3.40 - 3.36 (m, 2H), 1.12 - 0.95 (m, 1H), 0.44 - 0.34 (m, 2H), 0.24 - 0.15 (m, 2H).

### 41. Synthesis of example 41

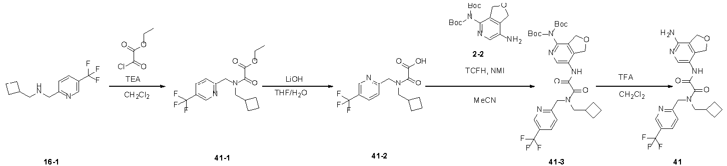

### 41.1 Synthesis of intermediate 41-1

Ethyl 2-chloro-2-oxoacetate (135.0 mg) was added to a solution of triethylamine (110.0 mg) and **16-1** (200.0 mg) in dichloromethane (5 mL) in an ice bath, and the mixture was warmed to room temperature and stirred for 2 hours. The organic phase was washed with saturated sodium chloride solution (1 mL), dried over anhydrous sodium sulfate and filtered. The filtrate was concentrated under reduced pressure. The residue was purified by silica gel column chromatography (eluent: petroleum ether/ethyl acetate = 5/1 - 1/1) to give a light yellow liquid **41-1** (200.0 mg, yield 70.9%). LC-MS m/z (ESI): 345.1 [M+H]⁺.

### 41.2 Synthesis of intermediate 41-2

Lithium hydroxide (30.0 mg) was added to a solution of **41-1** (200.0 mg) in tetrahydrofuran (3 mL) and water (1 mL), and the mixture was stirred at room temperature for 2 hours. The mixture was adjusted to acidity with 1 M hydrochloric acid. The mixture was extracted with dichloromethane (10 mL × 3). The organic phase was washed with saturated sodium chloride solution (5 mL), dried over anhydrous sodium sulfate and filtered. The filtrate was concentrated under reduced pressure to give a light yellow solid **41-2** (150.0 mg). The crude product was directly used in the next step reaction without purification. LC-MS m/z (ESI): 317.1 [M+H]⁺.

### 41.3 Synthesis of intermediate 41-3

Chloro-N,N,N',N'-tetramethylformamidinium hexafluorophosphate (54.0 mg) and N-methylimidazole (16.0 mg) were added to a solution of **41-2** (30.0 mg) and **2-2** (40.0 mg) in acetonitrile (2 mL). The mixture was stirred at room temperature for 5 hours. Water (10 mL) was added to dilute the mixture, and the mixture was extracted with ethyl acetate (10 mL × 3 mL). The organic phase was washed with saturated sodium chloride solution (5 mL), dried over anhydrous sodium sulfate and filtered. The filtrate was concentrated under reduced pressure. The residue was purified by silica gel column chromatography (eluent: dichloromethane/methanol = 20/1 - 10/1) to give a yellow solid **41-3** (24.0 mg, yield 34.9%). LC-MS m/z (ESI): 650.0 [M+H]⁺.

### 41.4 Synthesis of compound 41

Trifluoroacetic acid (2 mL) was added to a solution of **41-3** (24.0 mg) in dichloromethane (2 mL) in an ice bath. The mixture was stirred at room temperature for 1 hour, and concentrated under reduced pressure. The residue was purified by high performance liquid chromatography (column: Xbridge-C18 150 × 19 mm, 5um; mobile phase: ACN--H₂O (0.1% FA); B%: 10%-35%, 20 min) to give the formate of **41** (6.3 mg, yield 38.4%) as a white solid. LC-MS m/z (ESI): 450.1 [M+H]⁺. ¹H NMR (400 MHz, DMSO-*d*₆) δ ppm 10.51 - 10.33 (m, 1H), 8.94 - 8.92 (m, 1H), 8.24 (d, *J=* 8.0 Hz, 1H), 8.14 (s, 1H), 7.84 - 7.69 (m, 1H), 7.62 - 7.49 (m, 1H), 6.04 - 5.96 (m, 2H), 4.91 - 4.64 (m, 6H), 3.54 - 3.44 (m, 2H), 2.60 - 2.55 (m, 1H), 1.92 - 1.65 (m, 6H).

### 42. Synthesis of example 42

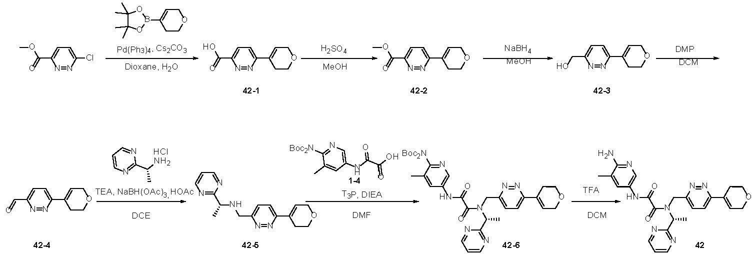

### 42.1 Synthesis of intermediate 42-1

3,6-Dihydro-2H-pyran-4-boronic acid pinacol ester (2.9 g), tetrakis(triphenylphosphine)palladium(0) (1.7 g) and cesium carbonate (7.6 g) were added to a mixed solution of methyl 6-chloropyridazine-3-carboxylate (2.0 g) in 1,4-dioxane (20 mL) and water (1 mL), and the mixture was stirred at 100 °C for 2 hours under nitrogen. The mixture was cooled to room temperature and concentrated under reduced pressure to give a brown solid **42-1** (3.0 g). The crude product was directly used in the next step reaction. LC-MS m/z (ESI): 207.1 [M+H]⁺.

### 42.2 Synthesis of intermediate 42-2

Concentrated sulfuric acid (6 mL) was added to a solution of compound **42-1** (3.0 g) in methanol (30 mL), and the mixture was stirred at 70 °C for 2 hours. The mixture was cooled to room temperature, and saturated sodium bicarbonate solution (100 mL) was added. The mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = 2/1) to give a yellow solid **42-2** (600.0 mg, two-step yield 24.2%). LC-MS m/z (ESI): 221.0 [M+H]⁺.

### 42.3 Synthesis of intermediate 42-3

Sodium borohydride (515.3 mg) was added to a solution of compound **42-2** (600 mg) in methanol (10 mL) at 0 °C, and the mixture was stirred at room temperature for 2 hours. Water (30 mL) was added, and the mixture was extracted with dichloromethane (30 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1) to give a yellow solid **42-3** (310.0 mg, yield 59.2%). LC-MS m/z (ESI): 193.1 [M+H]⁺.

### 42.4 Synthesis of intermediate 42-4

Dess-Martin periodinane (1.0 g) was added to a solution of compound **42-3** (310.0 mg) in dichloromethane (5 mL), and the mixture was stirred at room temperature for 2 hours. Water (50 mL) was added, and the mixture was extracted with dichloromethane (30 mL × 3). The organic phase was washed with saturated sodium chloride solution (50 mL), dried over anhydrous sodium sulfate and filtered. The filtrate was concentrated under reduced pressure to give a yellow solid **42-4** (150.0 mg, yield 49.2%). LC-MS m/z (ESI): 209.1 [M+H]⁺.

### 42.5 Synthesis of intermediate 42-5

Triethylamine (221.0 mg) was added to a solution of 1-(pyrimidin-2-yl)ethanamine hydrochloride (349.0 mg) in 1,2-dichloroethane (5 mL), and the mixture was stirred at room temperature for 0.5 hours. **42-4** (150.0 mg) and acetic acid (102.0 mg) were added, and the mixture was stirred at room temperature for 1 hour. Sodium triacetoxyborohydride (721.6 mg) was added, and the mixture was stirred at room temperature for 30 minutes. Water (30 mL) was added, and the pH was adjusted to weak alkalinity with saturated sodium carbonate solution. The mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a yellow oil **42-5** (90.0 mg, yield 58.0%). LC-MS m/z (ESI): 298.1 [M+H]⁺.

### 42.6 Synthesis of intermediate 42-6

Compound **42-5** (50.0 mg), propylphosphonic anhydride (113.0 mg) and N,N-diisopropylethylamine (38.0 mg) were added to a solution of compound **1-4** (150.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid **42-6** (50.0 mg, yield 25.6%). LC-MS m/z (ESI): 675.1 [M+H]⁺.

### 42.7 Synthesis of compound 42

Trifluoroacetic acid (1 mL) was added to a solution of compound **42-6** (50.0 mg) in dichloromethane (4 mL), and the mixture was stirred at room temperature for 1 hour. The mixture was concentrated under reduced pressure, and water (10 mL) was added. The pH was adjusted to weak alkalinity with saturated sodium bicarbonate solution. The mixture was extracted with dichloromethane (10 mL × 3). The organic phase was dried over anhydrous sodium sulfate and filtered. The filtrate was concentrated under reduced pressure. The residue was purified by high performance liquid chromatography (column: Gemini 5u C18 150 × 21.2 mm; mobile phase: [water(FA)-ACN]; B%: 2%-15%, 15 min) to give the formate of **42** (4.0 mg, yield 12.3%) as a white solid. LC-MS m/z (ESI): 475.0 [M+H]⁺. ¹H NMR (400 MHz, CD₃OD-*d*₄) δ ppm 8.74 - 8.67 (m, 2H), 8.08 - 8.06 (m, 1H), 7.81 - 7.59 (m, 1H), 7.62 - 7.52 (m, 2H), 7.28 - 7.25 (m, 1H), 6.95 - 6.75 (m, 1H), 5.85 - 5.75 (m, 1H), 5.75 - 4.95 (m, 2H), 4.46 - 4.26 (m, 2H), 4.02 - 3.78 (m, 2H), 2.82 - 2.58 (m, 2H), 2.25 - 2.13 (m, 3H), 1.65 - 1.78 (m, 3H).

### 43. Synthesis of example 43

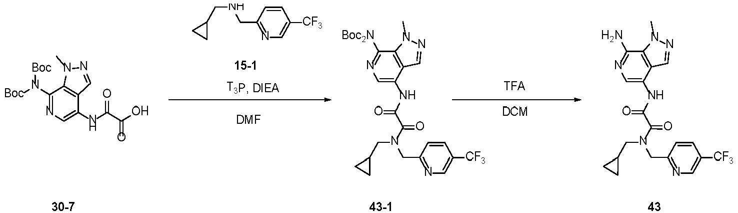

### 43.1 Synthesis of intermediate 43-1

Compound **30-7** (22.0 mg), propylphosphonic anhydride (73.0 mg) and N,N-diisopropylethylamine (45.0 mg) were added to a solution of compound **15-1** (50.0 mg) in N,N-dimethylformamide (3 mL), and the mixture was stirred at room temperature for 2 hours. Water (30 mL) was added, and the mixture was extracted with ethyl acetate (30 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a yellow solid **43-1** (46.0 mg, yield 62.2%). LC-MS m/z (ESI): 648.0 [M+H]⁺.

### 43.2 Synthesis of compound 43

Trifluoroacetic acid (1 mL) was added to a solution of compound **43-1** (46.0 mg) in dichloromethane (4 mL), and the mixture was stirred at room temperature for 1 hour. The mixture was concentrated under reduced pressure, and water (10 mL) was added. The pH was adjusted to weak alkalinity with saturated sodium bicarbonate solution. The mixture was extracted with dichloromethane (10 mL × 3). The organic phase was dried over anhydrous sodium sulfate and filtered. The filtrate was concentrated under reduced pressure. The residue was purified by high performance liquid chromatography (column: Gemini 5u C18 150 × 21.2 mm; mobile phase: [water(NH₃)-ACN]; B%: 10%-40%, 15 min) to give a white solid 43 (8.0 mg, yield 25.8%). LC-MS m/z (ESI): 448.0 [M+H]⁺. ¹H NMR (400 MHz, CD₃OD-*d*₄) δ ppm 8.94 - 8.53 (m, 1H), 8.35 - 8.25 (m, 1H), 8.24 -7.82 (m, 1H), 7.76 - 7.53 (m, 2H), 5.32 - 5.07 (m, 2H), 4.49 - 4.26 (m, 3H), 3.74 - 3.28 (m, 2H), 1.26 -1.03 (m, 1H), 0.52 - 0.35 (m, 2H), 0.28 - 0.19 (m, 2H).

### 44. Synthesis of example 44

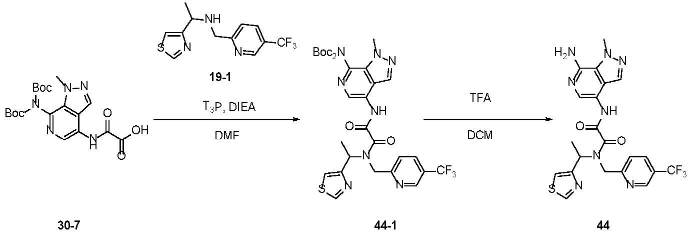

### 44.1 Synthesis of intermediate 44-1

Propylphosphonic anhydride (90.0 mg, 50% ethyl acetate solution) was added to a solution of N,N-diisopropylethylamine (27.0 mg), **30-7** (30.0 mg) and **19-1** (20.0 mg) in N,N-dimethylformamide (2 mL) at room temperature under nitrogen, and the mixture was stirred at room temperature for 16 hours. Ethyl acetate (30 mL) was added to dilute the mixture, and the mixture was washed with saturated sodium chloride solution (20 mL × 3). The organic phase was dried over anhydrous sodium sulfate and filtered. The filtrate was concentrated under reduced pressure. The residue was purified by silica gel column chromatography (eluent: dichloromethane/methanol = 100/1 - 30/1) to give an off-white solid **44-1** (28.0 mg, yield 58.3%). LC-MS m/z (ESI): 705.0 [M+H]⁺.

### 44.2 Synthesis of compound 44

Trifluoroacetic acid (1 mL) was added to a solution of **44-1** (28.0 mg) in dichloromethane (4 mL) in an ice bath under nitrogen. The mixture was stirred at room temperature for 1 hour and concentrated under reduced pressure. The residue was purified by high performance liquid chromatography (column: Xbridge-C18 150 × 19 mm, 5um; mobile phase: ACN--H₂O (0.05% NH₄OH); B%: 10%-40%, 13 min) to give a white solid **44** (10.0 mg, yield 50.1%). LC-MS m/z (ESI): 505.1 [M+H]⁺. ¹H NMR (400 MHz, MeOD-*d*₄) δ ppm 8.95 - 8.82 (m, 1H), 8.70 (d, *J =* 12.0 Hz, 1H), 8.05 - 7.88 (m, 2H), 7.77 - 7.67 (m, 1H), 7.62 - 7.55 (m, 1H), 7.46 - 7.36 (m, 1H), 6.16 - 5.75 (m, 1H), 5.04 - 4.60 (m, 2H), 4.36 - 4.31 (m, 3H), 1.78 - 1.60 (m, 3H).

### 45. Synthesis of example 45-P1 and example 45-P2

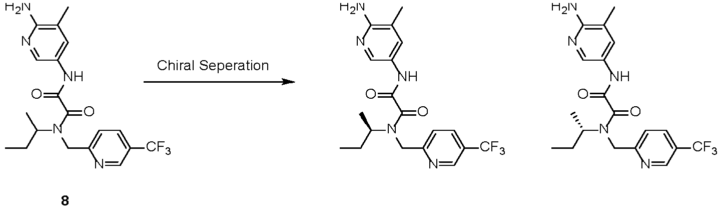

### 45.1 Synthesis of compound 45-P1 and compound 45-P2

Compound 8 (70.0 mg) was separated by SFC (column: CHIRALPAK AD-H 250 × 20 mm, 5um; mobile phase: 40% IPA (0.2% NH₄OH)) to give a white solid **45-P1** (25.8 mg, yield 36.8%). LC-MS m/z (ESI): 410.2 [M+H]⁺. ¹H NMR (400 MHz, DMSO-*d*₆) δ ppm 10.50 - 10.24 (m, 1H), 8.92 - 8.85 (m, 1H), 8.23 - 8.15 (m, 1H), 8.04 - 7.85 (m, 1H), 7.70 - 7.55 (m, 1H), 7.51 - 7.27 (m, 1H), 5.68 - 5.60 (m, 2H), 4.99 - 4.54 (m, 2H), 4.40 - 3.93 (m, 1H), 2.05 - 1.96 (m, 3H), 1.66 - 1.44 (m, 2H), 1.17 - 1.03 (m, 3H), 0.84 - 0.77 (m, 3H);
a white solid **45-P2** (29.5 mg, yield 42.1%) was obtained. LC-MS m/z (ESI): 410.2 [M+H]⁺. ¹H NMR (400 MHz, DMSO-*d*₆) δ ppm 10.57 - 10.32 (m, 1H), 8.91 - 8.85 (m, 1H), 8.23 - 8.15 (m, 1H), 8.07 - 7.89 (m, 1H), 7.70 - 7.55 (m, 1H), 7.55 - 7.34 (m, 1H), 5.89 (s, 2H), 5.00 - 4.54 (m, 2H), 4.39 - 3.93 (m, 1H), 2.06 - 1.98 (m, 3H), 1.66 - 1.44 (m, 2H), 1.17 - 1.03 (m, 3H), 0.84 - 0.77 (m, 3H).

### 46. Synthesis of example 159

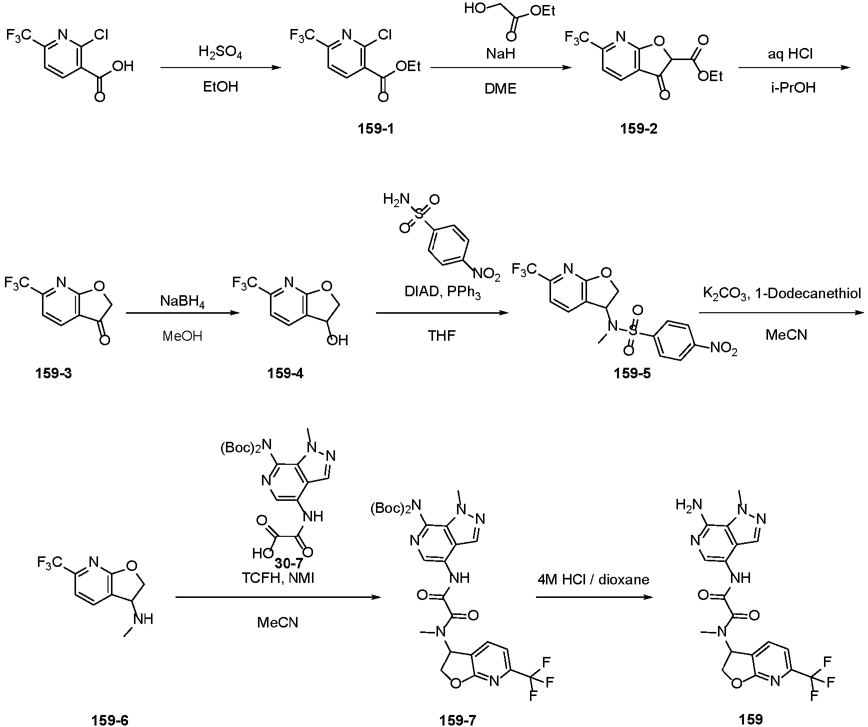

### 46.1 Synthesis of intermediate 159-1

Concentrated sulfuric acid (1.0 mL) was added to a solution of 2-chloro-6-(trifluoromethyl)nicotinic acid (5.0 g) in ethanol (50 mL), and the mixture was stirred at 80 °C for 10 hours. Water (100 mL) was added to dilute the mixture, and the mixture was extracted with ethyl acetate (200 mL × 3). The organic phase was washed with saturated sodium bicarbonate solution (100 mL), dried over anhydrous sodium sulfate and filtered. The filtrate was concentrated under reduced pressure. The residue was purified by silica gel column chromatography (eluent: petroleum ether/ethyl acetate = 5/1) to give a colorless oil **159-1** (5.0 g, yield 88.9%). LC-MS m/z (ESI): 254.0 [M+H]⁺.

### 46.2 Synthesis of intermediate 159-2

Sodium hydride (1.5 g, 60%wt) and ethyl glycolate (2.2 g) were successively added to a solution of **159-1** (4.5 g) in ethylene glycol dimethyl ether (50 mL) at 0 °C, and the mixture was stirred at room temperature for 10 hours. Water (100 mL) was added to dilute the mixture, and the mixture was extracted with ethyl acetate (50 mL × 3). The organic phase was washed with saturated sodium chloride (50 mL) solution, dried over anhydrous sodium sulfate and filtered. The filtrate was concentrated under reduced pressure. The residue was purified by silica gel column chromatography (eluent: dichloromethane/methanol = 20/1) to give a yellow solid **159-2** (4.0 g, yield 81.8%). LC-MS m/z (ESI): 276.1 [M+H]⁺.

### 46.3 Synthesis of intermediate 159-3

Hydrochloric acid (10 mL, 2 M) was added to a solution of compound **159-2** (2.0 g) in isopropyl alcohol (30 mL), and the mixture was stirred at 80 °C for 16 hours. The mixture was concentrated under reduced pressure. The residue was purified by silica gel column chromatography (eluent: dichloromethane/methanol = 20/1) to give a white solid **159-3** (0.5 g, yield 33.7%). LC-MS m/z (ESI): 204.0 [M+H]⁺.

### 46.4 Synthesis of intermediate 159-4

Sodium borohydride (1.0 g) was slowly added to a solution of compound **159-3** (0.5 g) in methanol (10 mL) at 0 °C, and the mixture was stirred at room temperature for 1 hour. Water (50 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: dichloromethane/methanol = 20/1) to give a light yellow solid **159-4** (0.4 g, yield 79.2%). LC-MS m/z (ESI): 206.1 [M+H]⁺.

### 46.5 Synthesis of intermediate 159-5

4-Nitrobenzenesulfonamide (0.5 g), triphenylphosphine (0.8 g) and diisopropyl azodicarboxylate (0.5 g) were added to a solution of compound **159-4** (0.4 g) in tetrahydrofuran (10 mL) at 0 °C, and the mixture was stirred at room temperature for 5 hours. Water (30 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 30/1) to give a white solid **159-5** (0.2 g, yield 28.0%). LC-MS m/z (ESI): 404.0 [M+H]⁺.

### 46.6 Synthesis of intermediate 159-6

Potassium carbonate (200.0 mg) and 1-dodecanethiol (200.0 mg) were added to a solution of compound **159-5** (220.0 mg) in acetonitrile (10 mL), and the mixture was stirred at 80 °C for 16 hours. The mixture was cooled to room temperature. (30 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 20/1) to give a white solid **159-6** (80.0 mg, 67.2%). LC-MS m/z (ESI): 219.1 [M+H]⁺.

### 46.7 Synthesis of intermediate 159-7

The intermediate **30-7** (80.0 mg), chloro-N,N,N',N'-tetramethylformamidinium hexafluorophosphate (100.0 mg) and N-methylimidazole (100.0 mg) were added to a solution of compound **159-6** (50.0 mg) in acetonitrile (5 mL), and the mixture was stirred at room temperature for 4 hours. Water (50 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1) to give a white solid **159-7** (40.0 mg, yield 27.5%). LC-MS m/z (ESI): 636.2 [M+H]⁺.

### 46.8 Synthesis of compound 159

A solution of HCl in dioxane (4 mL, 4 M) was added to a solution of the intermediate **159-7** (40.0 mg) in dichloromethane (2 mL), and the mixture was stirred at room temperature for 5 hours. The mixture was concentrated under reduced pressure. The residue was purified by high performance liquid chromatography (column: Gemini 5u C18 150 × 21.2 mm; mobile phase: ACN--H₂O (0.05% FA); B%: 5%-50%, 30 min) to give the formate of 159 (13.5 mg, yield 69.3%) as a white solid. LC-MS m/z (ESI): 436.2 [M+H]⁺. ¹H NMR (400 MHz, DMSO-*d*₆) δ ppm 10.88 - 10.80 (m, 1H), 8.15 - 7.81 (m, 3H), 7.59 - 7.51 (m, 1H), 6.27 (s, 2H), 6.24 - 5.95 (m, 1H), 4.94 - 4.84 (m, 1H), 4.78 - 4.73 (m, 1H), 4.27 (s, 3H), 2.90 - 2.63 (m, 3H).

### 47. Synthesis of example 160

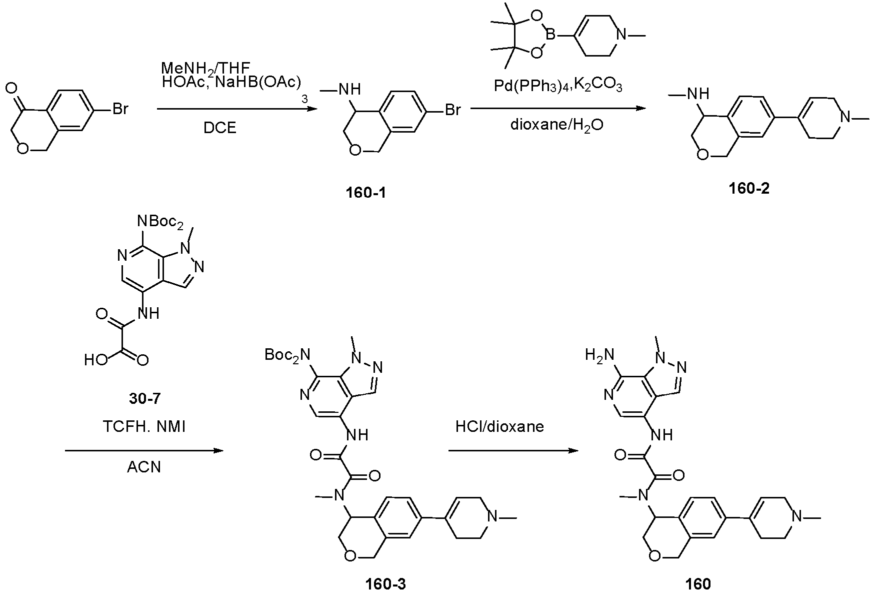

### 47.1 Synthesis of intermediate 160-1

A solution of methylamine in tetrahydrofuran (22.0 mL, 2.0 M) was added to a solution of 7-bromoisochroman-4-one (2.0 g) and acetic acid (1.1 g) in 1,2-dichloroethane (40 mL), and the mixture was stirred at room temperature for 2 hours. Sodium triacetoxyborohydride (3.7 g) was added, and the mixture was heated to 40 °C and stirred for 16 hours. Water (100 mL) was added, and the pH was adjusted to weak alkalinity with saturated sodium carbonate solution. The mixture was extracted with dichloromethane (100 mL × 3). The organic phase was washed with saturated sodium chloride solution (100 mL), dried over anhydrous sodium sulfate and filtered. The filtrate was concentrated under reduced pressure. The residue was purified by silica gel column chromatography (eluent: dichloromethane/methanol = 20/1) to give a yellow liquid **160-1** (1.0 g, yield 46.9%). LC-MS m/z (ESI): 242.1, 244.1 [M+H]⁺.

### 47.2 Synthesis of intermediate 160-2

**160-1** (100.0 mg) was added to a mixed solution of 1-methyl-1,2,3,6-tetrahydropyridine-4-boronic acid pinacol ester (111.0 mg), potassium carbonate (115.0 mg) and tetrakis(triphenylphosphine)palladium(0) (48.0 mg) in 1,4-dioxane (4 mL) and water (1 mL), and the mixture was stirred at 100 °C for 3 hours under nitrogen. The mixture was cooled to room temperature, and water (30 mL) was added. The mixture was extracted with dichloromethane (30 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1-30/1) to give a yellow liquid **160-2** (50.0 mg, yield 47.2%). LC-MS m/z (ESI): 259.2 [M+H]⁺.

### 47.3 Synthesis of intermediate 160-3

Chloro-N,N,N',N'-tetramethylformamidinium hexafluorophosphate (217.0 mg) was added to a solution of N-methylimidazole (48.0 mg), **30-7** (84.0 mg) and **160-2** (50.0 mg) in acetonitrile (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 10/1 - 5/1*)* to give a yellow liquid **160-3** (80.0 mg, yield 61.2%). LC-MS m/z (ESI): 676.4 [M+H]⁺.

### 47.4 Synthesis of compound 160

A solution of HCl in dioxane (4 mL, 4 M) was added to solution of **160-3** (80.0 mg) in dioxane (1 mL) in an ice bath, and the mixture was stirred at room temperature for 2 hours. The mixture was concentrated under reduced pressure. The pH of the residue was adjusted to 8 with saturated sodium carbonate solution. The mixture was extracted with dichloromethane (20 mL× 3). The organic phase was washed with saturated sodium chloride solution (20 mL), dried over anhydrous sodium sulfate and filtered. The filtrate was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give the formate of 160 (38.0 mg, yield 67.5%) as a white solid. LC-MS m/z (ESI): 476.0 [M+H]⁺. ¹H NMR (400 MHz, DMSO-*d*₆) δ ppm 10.88 - 10.64 (m, 1H), 7.99 - 7.83 (m, 2H), 7.50 - 7.19 (m, 3H), 6.25 (s, 2H), 6.23 - 6.17 (m, 1H), 5.56 - 4.86 (m, 1H), 4.85 - 4.76 (m, 1H), 4.70 - 4.57 (m, 1H), 4.32 - 4.24 (m, 3H), 4.16 - 3.96 (m, 2H), 3.16 - 3.05 (m, 2H), 2.81 - 2.66 (m, 3H), 2.65 - 2.11 (m, 2H), 2.55 - 2.51 (m, 2H), 2.40 - 2.28 (m, 3H).

### 48. Synthesis of example 163 (163-P1 and 163-P2)

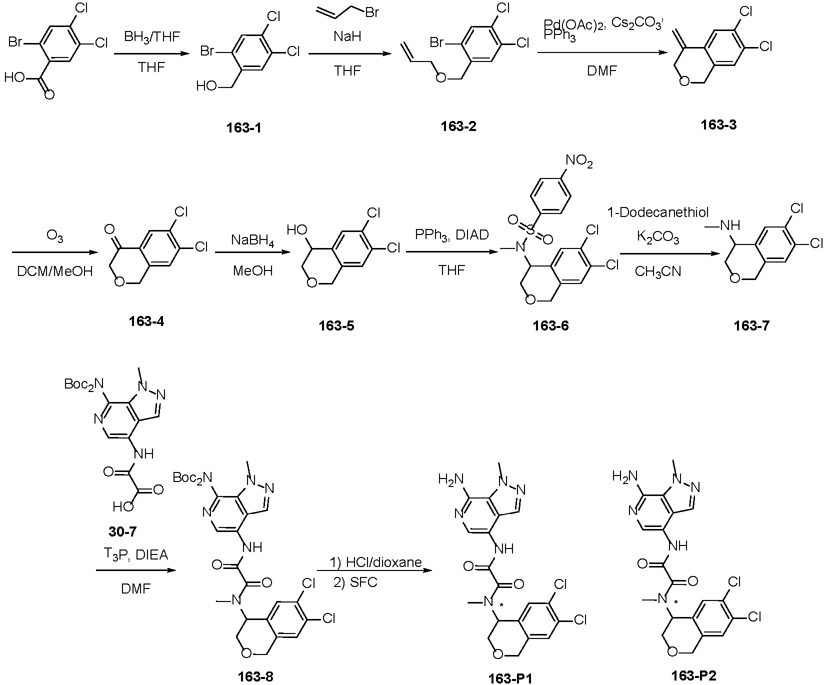

### 48.1 Synthesis of intermediate 163-1

A solution of borane in tetrahydrofuran (10.0 mL) was added dropwise to a solution of 2-bromo-4,5-dichlorobenzoic acid (1.0 g) in tetrahydrofuran (10 mL). The mixture was stirred at 50 °C for 3 hours under nitrogen. The mixture was cooled to room temperature, and water (10 mL) was slowly added. The mixture was extracted with dichloromethane (25 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **163-1** (800.0 mg, yield 84.4%). ¹H NMR (400 MHz, DMSO-*d*₆) δ ppm 7.94 (s, 1H), 7.66 (s, 1H), 5.68 (t, *J=* 5.6 Hz, 1H), 4.47 (d, *J=* 5.6 Hz, 2H).

### 48.2 Synthesis of intermediate 163-2

Sodium hydride (250.0 mg) was added to a solution of **163-1** (800.0 mg) in tetrahydrofuran (10 mL) in an ice bath. The mixture was stirred for 0.5 hours. 3-Bromopropene (760.0 mg) was added, and the mixture was stirred at room temperature overnight. Water (10 mL) was added to the reaction solution, and the mixture was extracted with ethyl acetate (20 mL × 3). The organic phase was washed with saturated sodium chloride solution (15 mL), dried over anhydrous sodium sulfate and filtered. The filtrate was concentrated under reduced pressure. The residue was purified by silica gel column chromatography (eluent: petroleum ether/ethyl acetate = 20/1 - 10/1) to give a colorless oil **163-2** (500 mg, yield 54.0%). ¹H NMR (400 MHz, DMSO-*d*₆) δ ppm 8.00 (s, 1H), 7.67 (s, 1H), 6.06 - 5.84 (m, 1H), 5.33 (d, *J=* 17.2 Hz, 1H), 5.21 (d, *J =* 10.0 Hz, 2H), 4.48 (s, 2H), 4.10 (d, *J = 4.0* Hz, 2H).

### 48.3 Synthesis of intermediate 163-3

Cesium carbonate (2.2 g), triphenylphosphine (184.0 mg) and palladium(II) acetate (84.0 mg) were added to a solution of **163-2** (1.0 g) in N,N-dimethylformamide (10 mL), and the mixture was stirred at 90 °C for 12 hours under nitrogen. The mixture was cooled to room temperature. Water (20 mL) was added to the reaction solution, and the mixture was extracted with ethyl acetate (25 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 20/1 - 10/1) to give a yellow liquid **163-3** (300.0 mg, yield 41.2%). ¹H NMR (400 MHz, DMSO-*d*₆) δ ppm 8.06 (s, 1H), 7.46 (s, 1H), 5.87 (s, 1H), 5.16 (s, 1H), 4.71 (s, 2H), 4.37 (s, 2H).

### 48.4 Synthesis of intermediate 163-4

Ozone was introduced into a solution of **163-3** (600.0 mg) in dichloromethane (5 mL) and methanol (5 mL) in an ice bath, and the mixture was stirred for 20 minutes. Dimethyl sulfide (1 mL) was added to the reaction solution, and the mixture was extracted with ethyl acetate (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 20/1 - 10/1) to give a white solid 163-4 (400.0 mg, yield 66.1%). ¹H NMR (400 MHz, DMSO-*d*₆) δ ppm 8.01 (s, 1H), 7.79 (s, 1H), 4.89 (s, 2H), 4.39 (s, 2H).

### 48.5 Synthesis of intermediate 163-5

Sodium borohydride (133.0 mg) was added to a solution of **163-4** (400.0 mg) in methanol (5 mL), and the mixture was stirred at room temperature for 2 hours. Water (10 mL) was added to the reaction solution, and the mixture was extracted with dichloromethane (15 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: dichloromethane/methanol = 20/1 - 10/1) to give a white solid **163-5** (300.0 mg, yield 74.3%). ¹H NMR (400 MHz, DMSO-*d*₆) δ ppm 7.63 (s, 1H), 7.40 (s, 1H), 5.60 (s, 1H), 4.69 - 4.52 (m, 3H), 3.90 - 3.86 (m, 1H), 3.61 - 3.56 (m, 1H).

### 48.6 Synthesis of intermediate 163-6

Triphenylphosphine (468.0 mg) and N-methyl-4-nitrobenzenesulfonamide (300.0 mg) were added to a solution of **163-5** (300.0 mg) in tetrahydrofuran (5 mL). Diisopropyl azodicarboxylate (420.0 mg) was added dropwise in an ice bath, and the mixture was warmed to room temperature and stirred for 2 hours. Water (10 mL) was added, and the mixture was extracted with ethyl acetate (15 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 20/1 - 10/1) to give a white solid 163-6 (300.0 mg, yield 52.4%). ¹H NMR (400 MHz, DMSO-*d*₆) δ ppm 8.45 - 8.42 (m, 2H), 8.18 - 8.15 (m, 2H), 7.47 (s, 1H), 6.98 (s, 1H), 5.00 (t, *J* = 3.6 Hz, 1H), 4.69 (d*, J= 15.6* Hz, 1H), 4.47 (d*, J=* 15.6 Hz, 1H), 3.72 - 3.61 (m, 2H), 2.61 (s, 3H).

### 48.7 Synthesis of intermediate 163-7

Potassium carbonate (300.0 mg) and 1-dodecanethiol (1.4 g) were added to a solution of 163-6 (300.0 mg) in acetonitrile (10 mL), and the mixture was stirred at 85 °C for 12 hours. The mixture was cooled to room temperature, and water (20 mL) was added to the reaction solution. The mixture was extracted with dichloromethane (20 mL × 3). The organic phase was washed with saturated sodium chloride solution (15 mL), dried over anhydrous sodium sulfate and filtered. The filtrate was concentrated under reduced pressure. The residue was purified by silica gel column chromatography (eluent: dichloromethane/methanol = 20/1 - 10/1) to give a colorless oil **163-7** (80.0 mg, yield 48.0%). LC-MS m/z (ESI): 231.9 [M+H]⁺.

### 48.8 Synthesis of intermediate 163-8

Propylphosphonic anhydride (440.0 mg) was added to a solution of **30-7** (152.0 mg), N,N-diisopropylethylamine (96.0 mg) and **163-7** (80.0 mg) in N,N-dimethylformamide (1 mL). The mixture was stirred at room temperature for 12 hours. Water (5 mL) was added to the reaction solution, and the mixture was extracted with ethyl acetate (10 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 2/1 - 1/1) to give a yellow solid **163-8** (110.0 mg, yield 49.0%). LC-MS m/z (ESI): 649.2 [M+H]⁺.

### 48.9 Synthesis of compound 163-P1 and compound 163-P2

A solution of HCl in 1,4-dioxane (5 mL, 4 M) was added to a solution of **163-8** (110.0 mg) in dichloromethane (5 mL) in an ice bath. The mixture was stirred at room temperature for 2 hours, and the pH was adjusted to 8 with saturated sodium carbonate solution. The mixture was extracted with dichloromethane (10 mL× 3), and the organic phase was concentrated under reduced pressure. The residue was purified by high performance liquid chromatography (column: Xbridge-C18 150 × 19 mm, 5um; mobile phase: ACN--H₂O (0.1% FA); B%: 5%-10%, 20 min) to give a white solid (56.1 mg, yield 73.7%), which was then separated by SFC (column: CHIRALPAK IC 250mm x 20 mm, 5um; mobile phase: ETOH (NH₄OH 0.2%); B%: 40% EtOH, 19 min) to give a white solid **163-P1** (ee value = 100%, 21.19 mg, RT = 2.68 min, yield 38.5%, which was an R isomer or S isomer). LC-MS m/z (ESI): 448.8 [M+H]⁺. ¹H NMR (400 MHz, DMSO-*d*₆) δ ppm 10.87 -10.71 (m, 1H), 8.00 - 7.94 (m, 2H), 7.84 - 7.77 (m, 1H), 7.54 - 7.51 (m, 1H), 6.30 (s, 2H), 5.55 - 4.87 (m, 2H), 4.64 - 4.55 (m, 1H), 4.28 - 4.26 (m, 3H), 4.10 - 4.04 (m, 2H), 2.82 - 2.70 (m, 3H); a white solid **163-P2** was obtained (ee value = 99.3%, 23.68 mg, RT = 4.9 min, yield 43.1%, which was an S isomer or R isomer). LC-MS m/z (ESI): 448.9 [M+H]⁺. ¹H NMR (400 MHz, DMSO-*d*₆) δ ppm 10.85 - 10.69 (m, 1H), 7.99 - 7.93 (m, 2H), 7.83 - 7.77 (m, 1H), 7.54 - 7.51 (m, 1H), 6.25 (s, 2H), 5.55 - 4.77 (m, 2H), 4.64 - 4.55 (m, 1H), 4.27 - 4.26 (m, 3H), 4.09 - 4.05 (m, 2H), 2.83 -2.70 (m, 3H).

### 49. Synthesis of example 173

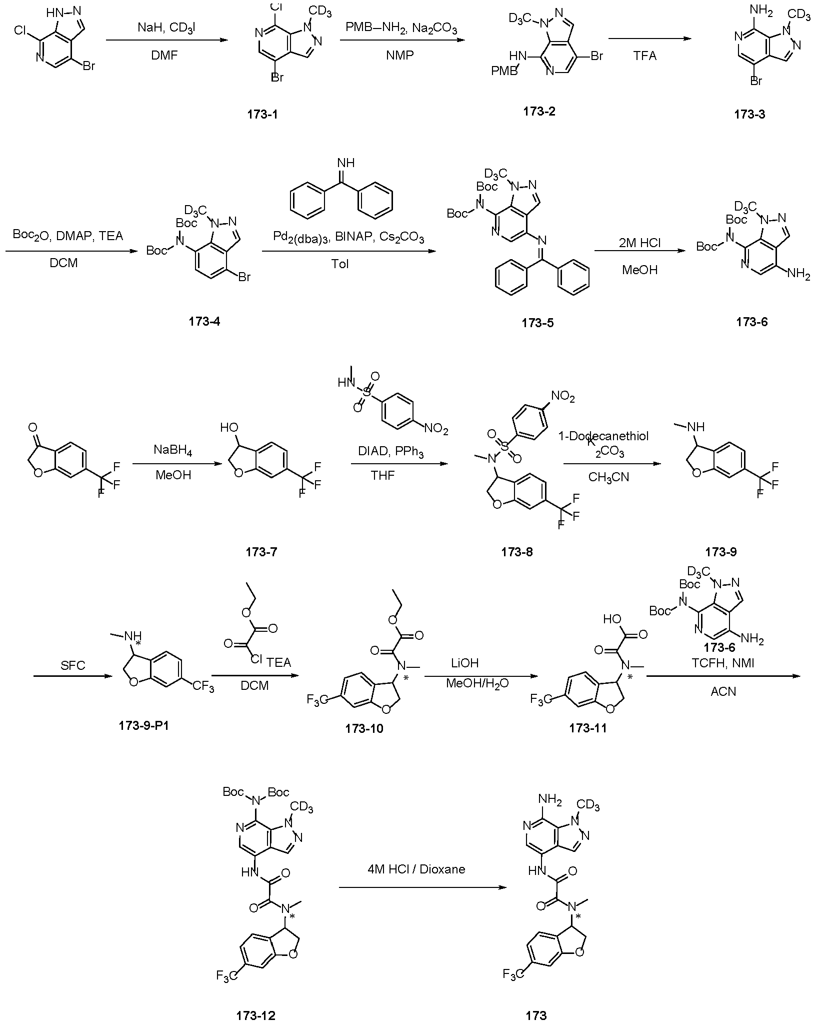

### 49.1 Synthesis of intermediate 173-1

Sodium hydride (0.9 g, 60%wt) was added to a solution of 4-bromo-7-chloro-1H-pyrazolo[3,4-c]pyridine (3.5 g) in N,N-dimethylformamide (40 mL) at 0 °C, and the mixture was stirred for 1 hour. Deuterated iodomethane (2.6 g) was added dropwise, and the mixture was warmed to room temperature and stirred for 2 hours. Water (200 mL) was added, and the mixture was extracted with ethyl acetate (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = 5/1) to give a white solid **173-1** (2.1 g, yield 55.6%). LC-MS m/z (ESI): 248.8, 250.8 [M+H]⁺.

### 49.2 Synthesis of intermediate 173-2

Sodium carbonate (1.8 g) and 4-methoxybenzylamine (1.7 g) were added to a solution of **173-1** (2.1 g) in N-methylpyrrolidone (20 mL), and the mixture was stirred at 130 °C for 2 hours. The mixture was cooled to room temperature, and water (100 mL) was added. The mixture was extracted with ethyl acetate (30 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1) to give a white solid **173-2** (2.5 g, yield 70.6%). LC-MS m/z (ESI): 350.0, 352.0 [M+H]⁺.

### 49.3 Synthesis of intermediate 173-3

**173-2** (2.5 g) was added to a solution of trifluoroacetic acid (30 mL), and the mixture was stirred at 80 °C for 16 hours. The mixture was cooled to room temperature and concentrated under reduced pressure. Saturated aqueous solution of sodium bicarbonate (60 mL) was added, and the mixture was filtered to give a white solid **173-3** (2.2 g). The crude product was directly used in the next step reaction. LC-MS m/z (ESI): 227.9, 229.9 [M+H]⁺.

### 49.4 Synthesis of intermediate 173-4

Triethylamine (3.9 g), 4-dimethylaminopyridine (240 mg), and di-tert-butyl dicarbonate (5.3 g) were added to a solution of **173-3** (2.2 g) in dichloromethane (50 mL), and the mixture was stirred at 40 °C for 16 hours. The mixture was cooled to room temperature, and water (100 mL) was added. The mixture was extracted with dichloromethane (50 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1) to give a yellow solid **174-4** (1.2 g, two-step yield 28.9%). LC-MS m/z (ESI): 429.9, 431.9 [M+H]⁺.

### 49.5 Synthesis of intermediate 173-5

Benzophenone imine (0.8 g), cesium carbonate (1.8 g), 2,2'-bis(diphenylphosphino)-1,1'-binaphthyl (0.4 g), and tris(dibenzylideneacetone)dipalladium(0) (0.3 g) were added to a solution of **173-4** (1.2 g) in toluene (20 mL), and the mixture was stirred at 100 °C for 16 hours. The mixture was cooled to room temperature and concentrated under reduced pressure. Water (60 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 3/1) to give a yellow solid **173-5** (1.3 g, yield 73.9%). LC-MS m/z (ESI): 531.0 [M+H]⁺.

### 49.6 Synthesis of intermediate 173-6

Hydrochloric acid (5 mL, 2 M) was added dropwise to a solution of **173-5** (1.3 g) in methanol (15 mL) at 0 °C, and the mixture was stirred at room temperature for 2 hours. Saturated sodium bicarbonate solution was added, and the pH was adjusted to weak alkalinity. The mixture was extracted with dichloromethane (50 mL × 3). The organic phase was washed with saturated sodium chloride solution (50 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **173-6** (0.7 g, yield 89.5%). LC-MS m/z (ESI): 367.0 [M+H]⁺.

### 49.7 Synthesis of intermediate 173-7

Sodium borohydride (56.1 mg) was added to a solution of 6-(trifluoromethyl)benzofuran-3(2H)-one (200.0 mg) in methanol (3 mL), and the mixture was stirred at room temperature for 2 hours. Water (10 mL) was added to the reaction solution, and the mixture was extracted with dichloromethane (10 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: petroleum ether/ethyl acetate = 1/2) to give a white solid **173-7** (150.0 mg, yield 66.8%).

### 49.8 Synthesis of intermediate 173-8

Diisopropyl azodicarboxylate (128.7 mg) was added to a solution of **173-7** (100.0 mg), N-methyl-4-nitrobenzenesulfonamide (127.1 mg) and triphenylphosphine (167.0 mg) in tetrahydrofuran (3 mL) in an ice bath. The mixture was warmed to room temperature and stirred for 3 hours under nitrogen. Water (5 mL) was added to the reaction solution, and the mixture was extracted with dichloromethane (10 mL × 3). The organic phase was washed with saturated sodium chloride solution (10 mL), dried over anhydrous sodium sulfate and filtered. The filtrate was concentrated under reduced pressure. The residue was purified by silica gel column chromatography (eluent: petroleum ether/ethyl acetate = 4/1) to give a white solid **173-8** (60.0 mg, yield 27.4%).

### 49.9 Synthesis of intermediate 173-9

**173-8** (280.0 mg), potassium carbonate (384.7 mg) and 1-dodecanethiol (563.4 mg) were added to a solution of acetonitrile (2 mL), and the mixture was stirred at 80 °C for 4 hours. The mixture was cooled to room temperature. Water (5 mL) was added, and the mixture was extracted with ethyl acetate (5 mL × 3). The organic phase was washed with saturated sodium chloride (5 mL). The residue was purified by silica gel column chromatography (eluent: dichloromethane/methanol = 20/1 - 10/1) to give a yellow solid **173-9** (100.0 mg, yield 52.9%).

### 49.10 Synthesis of intermediate 173-9-P1

**173-9** (400.0 mg) was purified by chiral chromatography (column: chiralpak AD-H 250 x 20 mm, 5um; mobile phase: 40% MeOH (0.2% NH₄OH) to give a yellow oil **173-9-P1** (220.0 mg, RT = 1.85 min, yield 55.0%).

### 49.11 Synthesis of intermediate 173-10

Ethyl 2-chloro-2-oxoacetate (408.6 mg) was added to a solution of **173-9-P1** (500.0 mg) and triethylamine (349.4 mg) in dichloromethane (5.0 mL) in an ice bath, and the mixture was warmed to room temperature and stirred for 2 hours. Water (5.0 mL) was added, and the layers were separated. Then, the organic phase was washed with saturated sodium chloride solution (2 mL), dried over anhydrous sodium sulfate and filtered. The filtrate was concentrated under reduced pressure. The residue was purified by column chromatography (silica, petroleum ether/ethyl acetate = 3/1) to give a yellow oil **173-10** (500.0 mg, yield 68.5%). LC-MS m/z (ESI): 340.1 [M+H]⁺.

### 49.12 Synthesis of intermediate 173-11

Lithium hydroxide (49.1 mg) was added to a mixed solution of **173-10** (500.0 mg) in methanol (3.0 mL) and water (1.0 mL). The mixture was stirred at 25 °C for 1 hour, and the pH was adjusted to 6 with dilute hydrochloric acid. The mixture was extracted with dichloromethane (10 mL × 3). The organic phase was dried over anhydrous sodium sulfate and filtered. The filtrate was concentrated under reduced pressure to give a crude product **173-11** (400.0 mg) as a colorless oil. The crude product was directly used in the next step reaction without purification. LC-MS m/z (ESI): 288.0 [M-H]⁻.

### 49.13 Synthesis of intermediate 173-12

Chloro-N,N,N',N'-tetramethylformamidinium hexafluorophosphate (776.1 mg) and N-methylimidazole (397.4 mg) were added to a solution of **173-11** (400.0 mg) and **173-6** (502.6 mg) in acetonitrile (10 mL), and the mixture was stirred at 25 °C for 2 hours. Water (20 mL) was added, and the mixture was extracted with ethyl acetate (15 mL × 3 mL). The organic phase was washed with saturated sodium chloride solution (5 mL), dried over anhydrous sodium sulfate and filtered. The filtrate was concentrated under reduced pressure. The residue was purified by silica gel column chromatography (eluent: dichloromethane/methanol = 20/1 - 10/1) to give a brown solid **173-12** (620.0 mg, yield 63.6%). LC-MS m/z (ESI): 634.9 [M+H]⁺.

### 49.14 Synthesis of compound 173

**173-12** (640.0 mg) was added to a solution of HCl in dioxane (8 mL, 4 M). The mixture was stirred at room temperature for 2 hours, and the pH was adjusted to 8 with saturated sodium carbonate solution. The mixture was extracted with dichloromethane (5 mL× 3), and the organic phase was concentrated under reduced pressure. The residue was purified by silica gel column chromatography (eluent: dichloromethane/methanol = 20/1 - 10/1) to give a greyish-white solid **173** (320.0 mg, yield 72.3%). LC-MS m/z (ESI): 438.1 [M+H]⁺. ¹H NMR (400 MHz, DMSO-*d*₆) δ ppm 10.92 - 10.59 (m, 1H), 8.01 - 7.81 (m, 2H), 7.72 - 7.52 (m, 1H), 7.39 - 7.33(m, 1H), 7.30 - 7.24 (m, 1H), 6.44 - 6.32 (m, 2H), 6.30 - 5.79 (m, 1H), 4.85 - 4.64 (m, 2H), 2.78 - 2.58 (m, 3H).

### 50. Synthesis of example 211

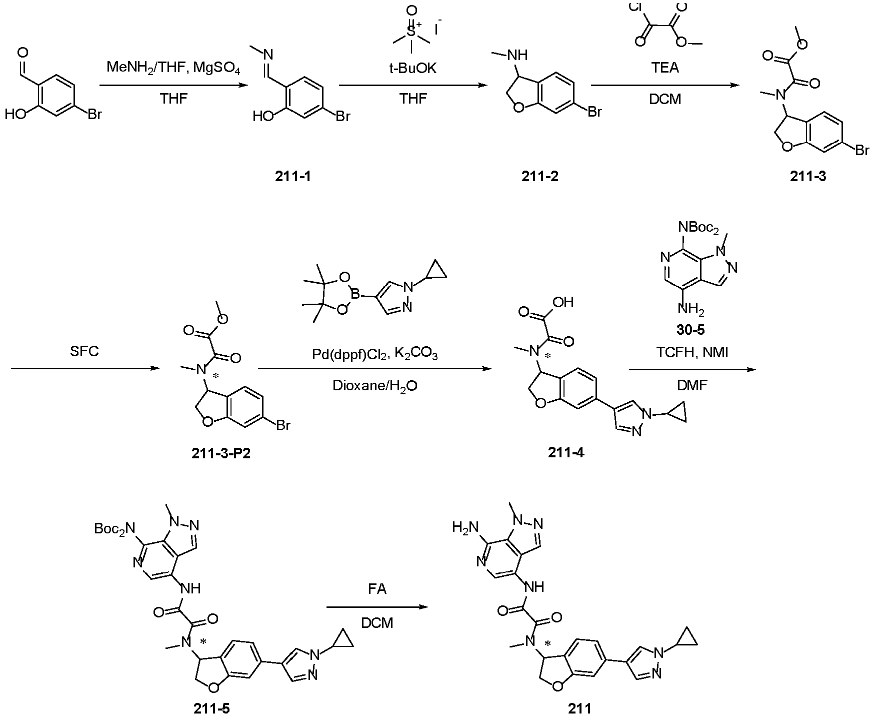

### 50.1 Synthesis of intermediate 211-1

A solution of methylamine in tetrahydrofuran (5.0 mL, 2.0 M) was added to a solution of 4-bromo-2-hydroxybenzaldehyde (1.0 g) and anhydrous magnesium sulfate (3.0 g) in dichloromethane (20 mL), and the mixture was stirred at room temperature for 16 hours. The mixture was filtered, and the filtrate was concentrated under reduced pressure to give a yellow solid **211-1** (0.6 g). The crude product was directly used in the next step.

### 50.2 Synthesis of intermediate 211-2

Potassium tert-butoxide (1.4 g) was added to a solution of trimethylsulfoxonium iodide (2.7 g) in tetrahydrofuran (20 mL), and the mixture was stirred at room temperature for 30 minutes. A solution of compound **211-1** (0.6 g) in tetrahydrofuran (5 mL) was added to the reaction solution, and the mixture was heated to 50 °C and stirred for 3 hours. The mixture was cooled to room temperature and filtered. The filtrate was concentrated under reduced pressure. The residue was purified by silica gel column chromatography (eluent: dichloromethane/methanol = 20/1) to give a yellow oil **211-2** (260.0 mg, yield 29.2%).

### 50.3 Synthesis of intermediate 211-3

Methyl 2-chloro-2-oxoacetate (168.0 mg) was added to a solution of compound **211-2** (260.0 mg) and triethylamine (173.0 mg) in dichloromethane (5 mL) in an ice bath, and the mixture was warmed to room temperature and stirred for 2 hours. Water (30 mL) was added, and the mixture was extracted with dichloromethane (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1) to give a yellow oil **211-3** (240.0 mg, yield 66.9%).

### 50.4 Synthesis of intermediate 211-3-P2

The intermediate **211-3** (6.0 g) was separated by chiral column (column: CHIRALPAK AS-H 250mm x 20 mm, 5µm; mobile phase: 40%EtOH (NH₄OH 0.2%): 60%CO₂) to give a white solid **211-3-P2** (2.6 g, RT = 3.22 min, ee value = 97.54%, yield 43.3%).

### 50.5 Synthesis of intermediate 211-4

[1,1'-Bis(diphenylphosphino)ferrocene]dichloropalladium(II) (46.0 mg) and sodium carbonate (135.0 mg) were added to a mixed solution of compound **211-3-P2** (200.0 mg) and 1-cyclopropyl-1H-pyrazole-4-boronic acid pinacol ester (224.0 mg) in 1,4-dioxane and water (5 mL, v/v = 4:1), and the mixture was stirred at 90 °C for 2 hours under nitrogen. The mixture was cooled to room temperature and filtered. The filtrate was diluted with sodium hydroxide solution (30 mL, 0.5 M), and washed with diethyl ether (30 mL × 3). The pH of the aqueous phase was adjusted to weak acidity with hydrochloric acid solution (1 M), and the mixture was extracted with dichloromethane (30 mL × 3). The organic phase was washed with saturated sodium chloride solution (30 mL), dried over anhydrous sodium sulfate and filtered. The filtrate was concentrated under reduced pressure and freeze-dried to give a yellow solid **211-4** (150 mg). The crude product was directly used in the next step. LC-MS m/z (ESI): 328.0 [M+H]⁺.

### 50.6 Synthesis of intermediate 211-5

Chloro-N,N,N',N'-tetramethylformamidinium hexafluorophosphate (171.0 mg) and N-methylimidazole (88.0 mg) were added to a solution of compound **211-4** (100.0 mg) and compound **30-5** (133.0 mg) in anhydrous N,N-dimethylformamide (3 mL), and the mixture was stirred at room temperature for 16 hours. Water (30 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **211-5** (140.0 mg, yield 68.0%). LC-MS m/z (ESI): 673.4 [M+H]⁺.

### 50.7 Synthesis of compound 211

Formic acid (4 mL) was added to a solution of compound **211-5** (130.0 mg) in dichloromethane (1 mL), and the mixture was stirred at room temperature for 16 hours. The mixture was concentrated under reduced pressure, and water (30 mL) was added. The pH was adjusted to weak alkalinity with saturated sodium carbonate solution. The mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 10/1) to give a white solid **211** (35.0 mg, yield 31.5%). LC-MS m/z (ESI): 473.0 [M+H]⁺. ¹H NMR (400 MHz, DMSO-*d*₆) δ ppm 10.78 - 10.62 (m, 1H), 8.26 - 8.25 (m, 1H), 8.04 - 7.83 (m, 3H), 7.42 - 7.28 (m, 1H), 7.24 - 7.21 (m, 1H), 7.16 - 7.14 (m, 1H), 6.24 (s, 2H), 6.22 - 5.70 (m, 1H), 4.75 - 4.67 (m, 1H), 4.61 - 4.53 (m, 1H), 4.28 (s, 3H), 3.74 - 3.70 (m, 1H), 2.72 - 2.60 (m, 3H), 1.08 - 1.04 (m, 2H), 1.00 - 0.95 (m, 2H).

### 51. Synthesis of example 212

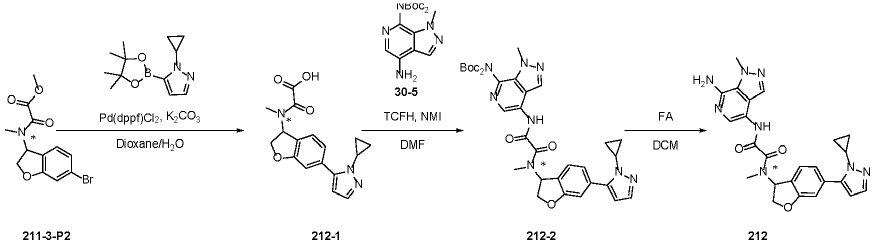

### 51.1 Synthesis of intermediate 212-1

[1,1'-Bis(diphenylphosphino)ferrocene]dichloropalladium(II) (46.0 mg) and sodium carbonate (135.0 mg) were added to a mixed solution of compound **211-3-P2** (200.0 mg) and 1-cyclopropylpyrazole-5-boronic acid pinacol ester (224.0 mg) in 1,4-dioxane and water (v/v = 4:1, 5 mL), and the mixture was stirred at 90 °C for 2 hours under nitrogen. The mixture was cooled to room temperature and filtered. The filtrate was diluted with sodium hydroxide solution (30 mL, 0.5 M) and washed with diethyl ether (30 mL × 3). The pH of the aqueous phase was adjusted to weak acidity with hydrochloric acid solution (1 M), and the mixture was extracted with dichloromethane (30 mL × 3). The organic phase was washed with saturated sodium chloride solution (30 mL), dried over anhydrous sodium sulfate and filtered. The filtrate was concentrated under reduced pressure and freeze-dried to give a crude product **212-1** (130.0 mg) as a yellow solid. The crude product was directly used in the next step reaction. LC-MS m/z (ESI): 328.0 [M+H]⁺.

### 51.2 Synthesis of intermediate 212-2

Chloro-N,N,N',N'-tetramethylformamidinium hexafluorophosphate (171.0 mg) and N-methylimidazole (88.0 mg) were added to a solution of compound **212-1** (100.0 mg) and compound **30-5** (133.0 mg) in anhydrous N,N-dimethylformamide (3 mL), and the mixture was stirred at room temperature for 16 hours. Water (30 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **212-2** (130.0 mg, yield 63.1%). LC-MS m/z (ESI): 673.3 [M+H]⁺.

### 51.3 Synthesis of compound 212

Formic acid (4 mL) was added to compound **212-2** (120.0 mg), and the mixture was stirred at room temperature for 16 hours. The mixture was concentrated under reduced pressure, and water (30 mL) was added. The pH was adjusted to weak alkalinity with saturated sodium carbonate solution, and the mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 10/1) to give a white solid **212** (35.0 mg, yield 41.5%). LC-MS m/z (ESI): 473.0 [M+H]⁺. ¹H NMR (400 MHz, DMSO-*d*₆) δ ppm 10.82 - 10.64 (m, 1H), 8.05 - 7.83 (m, 2H), 7.59 - 7.40 (m, 2H), 7.30 - 7.25 (m, 1H), 7.21 - 7.16 (m, 1H), 6.46 - 6.42 (m, 1H), 6.34 - 5.80 (m, 3H), 4.82 - 4.73 (m, 1H), 4.70 - 4.59 (m, 1H), 4.28 (s, 3H), 3.80 - 3.70 (m, 1H), 2.77 - 2.62 (m, 3H), 1.01 - 0.85 (m, 4H).

### 52. Synthesis of example 213

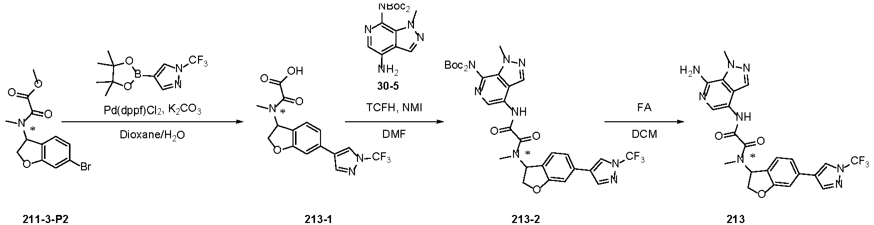

### 52.1 Synthesis of intermediate 213-1

[1,1'-Bis(diphenylphosphino)ferrocene]dichloropalladium(II) (46.0 mg) and sodium carbonate (135.0 mg) were added to a mixed solution of compound **211-3-P2** (200.0 mg) and 4-(4,4,5,5-tetramethyl-1,3,2-dioxaborolan-2-yl)-1-(trifluoromethyl)-1H-pyrazole (251.0 mg) in 1,4-dioxane and water (v/v = 4:1, 5 mL), and the mixture was stirred at 90 °C for 2 hours under nitrogen. The mixture was cooled to room temperature and filtered. The filtrate was diluted with sodium hydroxide solution (30 mL, 0.5 M) and washed with diethyl ether (30 mL × 3). The pH of the aqueous phase was adjusted to weak acidity with hydrochloric acid solution (1 M), and the mixture was extracted with dichloromethane (30 mL × 3). The organic phase was washed with saturated sodium chloride solution (30 mL), dried over anhydrous sodium sulfate and filtered. The filtrate was concentrated under reduced pressure and freeze-dried to give a crude product **213-1** (150.0 mg) as a yellow solid, which was directly used in the next step. LC-MS m/z (ESI): 355.9 [M+H]⁺.

### 52.2 Synthesis of intermediate 213-2

Chloro-N,N,N',N'-tetramethylformamidinium hexafluorophosphate (157.0 mg) and N-methylimidazole (81.0 mg) were added to a solution of compound **213-1** (100.0 mg) and compound **30-5** (122.0 mg) in anhydrous N,N-dimethylformamide (3 mL), and the mixture was stirred at room temperature for 16 hours. Water (30 mL) was added, and the mixture was extracted with ethyl acetate (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: petroleum ether/ethyl acetate = 1/2) to give a yellow solid **213-2** (140.0 mg, yield 63.1%). LC-MS m/z (ESI): 701.3 [M+H]⁺.

### 52.3 Synthesis of compound 213

Formic acid (4 mL) was added to compound **213-2** (120.0 mg), and the mixture was stirred at room temperature for 16 hours. The mixture was concentrated under reduced pressure, and water (30 mL) was added. The pH was adjusted to weak alkalinity with saturated sodium carbonate solution, and the mixture was extracted with dichloromethane (30 mL × 3). The organic phase was washed with saturated sodium chloride solution (30 mL), dried over anhydrous sodium sulfate and filtered. The filtrate was concentrated under reduced pressure. The residue was purified by silica gel column chromatography (eluent: dichloromethane/methanol = 10/1) to give a white solid **213** (42.0 mg, yield 49.0%). LC-MS m/z (ESI): 500.9 [M+H]⁺. ¹H NMR (400 MHz, DMSO-*d*₆) δ ppm 10.81 - 10.62 (m, 1H), 9.02 - 8.99 (m, 1H), 8.48 (s, 1H), 8.04 - 7.82 (m, 2H), 7.52 - 7.33 (m, 3H), 6.27 - 5.73 (m, 3H), 4.79 - 4.69 (m, 1H), 4.66 - 4.56 (m, 1H), 4.28 (s, 3H), 2.74 - 2.59 (m, 3H).

### 53. Synthesis of example 233

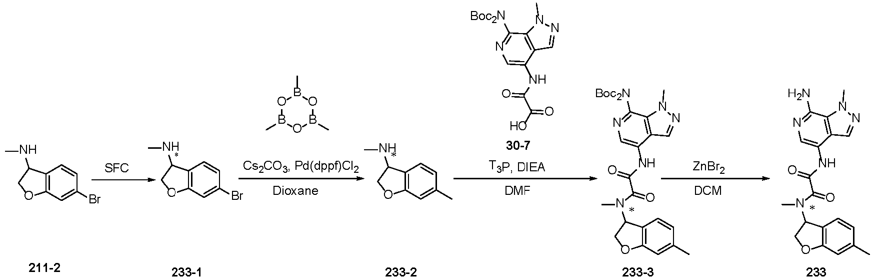

### 53.1 Synthesis of intermediate 233-1

Compound **211-2** (2.0 g) was separated by chiral column (column: CHIRALPAK AS-H 250 mm × 20 mm, 5µm; mobile phase: 40% MeOH (NH₄OH 0.2%): 60% CO₂) to give a white solid **233-1** (0.8 g, RT = 3.26 min, ee value = 99.15%, yield 40.0%).

### 53.2 Synthesis of intermediate 233-2

**233-1** (100.0 mg), trimethylboroxine (200.0 mg) and cesium carbonate (515.0 mg) were added to a mixed solution of [1,1'-bis(diphenylphosphino)ferrocene]dichloropalladium(II) (38.0 mg) in dioxane (5 mL) and water (1 mL). The mixture was stirred at 100 °C for 16 hours under nitrogen. The mixture was concentrated under reduced pressure. The residue was purified by silica gel column chromatography (eluent: petroleum ether/ethyl acetate = 10/1 - 5/1) to give an oily liquid **233-2** (60.0 mg, yield 83.3%).

### 53.3 Synthesis of intermediate 233-3

Propylphosphonic anhydride (120.0 mg) and N,N-diisopropylethylamine (75.0 mg) were added to a solution of **233-2** (60.0 mg) and **30-7** (70.0 mg) in N,N-dimethylformamide (3 mL) in an ice bath. The mixture was warmed to room temperature and stirred for 1 hour. Water (10 mL) was added, and the mixture was extracted with ethyl acetate (10 mL × 3). The organic phase was washed with saturated sodium chloride solution (5 mL), dried over anhydrous sodium sulfate and filtered. The filtrate was concentrated under reduced pressure. The residue was purified by silica gel column chromatography (eluent: dichloromethane/methanol = 20/1 - 10/1) to give a yellow solid **233-3** (80.0 mg, yield 37.6%). LC-MS m/z (ESI): 581.3 [M+H]⁺.

### 53.4 Synthesis of compound 233

Zinc bromide (130.0 mg) was added to a solution of **233-3** (80.0 mg) in dichloromethane (3 mL) in an ice bath. The mixture was warmed to room temperature and stirred for 2 hours. The mixture was filtered and concentrated under reduced pressure. The residue was purified by high performance liquid chromatography (column: Xbridge-C18 150 × 19 mm, 5um; mobile phase: ACN--H₂O (0.1% NH₄OH); B%: 5%-10%, 20 min) to give a white solid **233** (27.3 mg, yield 51.9%). LC-MS m/z (ESI): 381.0 [M+H]⁺. ¹H NMR (400 MHz, DMSO-*d*₆, 80°C) δ ppm 10.75 - 10.59 (m, 1H), 8.16 - 8.09 (m, 1H), 7.98 - 7.89 (m, 1H), 7.31 - 7.19 (m, 1H), 6.87 - 6.71 (m, 4H), 6.18 - 5.73 (m, 1H), 4.64 - 4.61 (m, 1H), 4.56 - 4.49 (m, 1H), 4.32 (s, 3H), 2.72 - 2.60 (m, 3H), 2.32 - 2.29 (m, 3H).

### 54. Synthesis of example 235

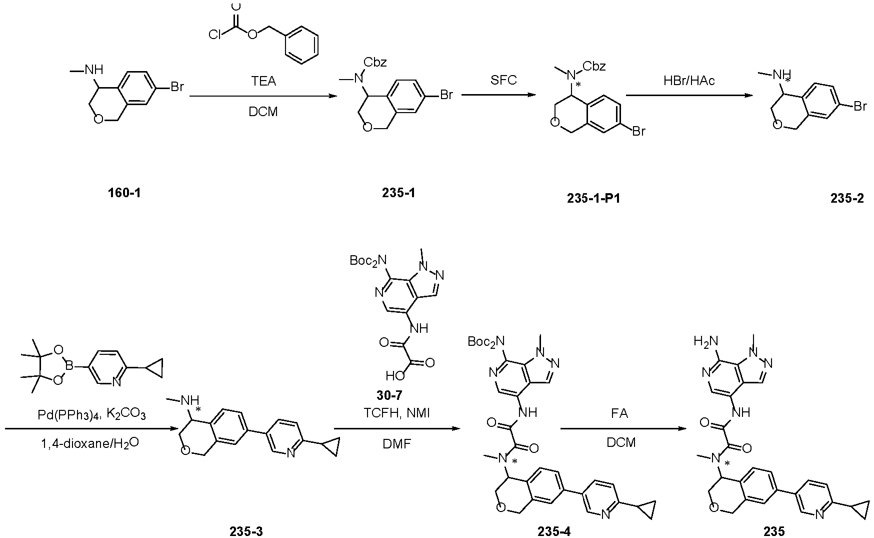

### 54.1 Synthesis of intermediate 235-1

Benzyl chloroformate (418.0 mg) was added to a solution of **160-1** (500.0 mg) and triethylamine (309.0 mg) in dichloromethane (5 mL) in an ice bath, and the mixture was stirred at 0 °C for 2 hours. Water (50 mL) was added, and the mixture was extracted with dichloromethane (30 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: petroleum ether/ethyl acetate = 5/1-2/1) to give a yellow liquid **235-1** (600.0 g, yield 77.1%). LC-MS m/z (ESI): 376.0 [M+H]⁺.

### 54.2 Synthesis of intermediate 235-1-P1

**235-1** (500.0 mg) was separated by SFC (column: CHIRALPAK IC 250mm × 4.6 mm, 5µm; mobile phase: i-PrOH (NH₄OH 0.2%); B%: 60% CO₂, 3mL/min) to give a white compound **235-1-P1** (ee=98.29 %, 200.0 mg, yield 40.0%, Rt=3.04 min).

### 54.3 Synthesis of intermediate 235-2

Hydrobromic acid solution (5 mL, 33%wt in HAc) was added to **235-1-P1** (150.0 mg), and the mixture was stirred at room temperature for 5 hours. The mixture was concentrated under reduced pressure. The pH of the residue was adjusted to 8 with saturated sodium carbonate solution. The mixture was extracted with dichloromethane (20 mL× 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 20/1 - 10/1) to give a yellow liquid **235-2** (50.0 mg, yield: 52.1%). LC-MS m/z (ESI): 242.0, 244.0 [M+H]⁺.

### 54.4 Synthesis of intermediate 235-3

Tetrakis(triphenylphosphine)palladium(0) (20.0 mg) and potassium carbonate (56.0 mg) were added to a mixed solution of **235-2** (50.0 mg) and 2-cyclopropyl-5-pyridineboronic acid ester (55.0 mg) in 1,4-dioxane and water (15 mL, v/v = 4:1), and the mixture was stirred at 90 °C for 2 hours under nitrogen. The mixture was cooled to room temperature and concentrated under reduced pressure. The residue was purified by silica gel column chromatography (eluent: dichloromethane/methanol = *50*/*1* - 20/1) to give a yellow liquid **235-3** (40.0 mg, yield: 68.9%). LC-MS m/z (ESI): 281.1 [M+H]⁺.

### 54.5 Synthesis of intermediate 235-4

Chloro-N,N,N',N'-tetramethylformamidinium hexafluorophosphate (78.0 mg) was added to a solution of N-methylimidazole (32.0 mg), **30-7** (60.0 mg) and **235-3** (40.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 10/1 - 5/1) to give a yellow liquid **235-4** (40.0 mg, yield 40.4%). LC-MS m/z (ESI): 698.1 [M+H]⁺.

### 54.6 Synthesis of compound 235

Anhydrous formic acid (4 mL) was added to a solution of **235-4** (40.0 mg) in dichloromethane (1 mL) in an ice bath, and the mixture was stirred at room temperature for 2 hours. The mixture was concentrated under reduced pressure. The pH of the residue was adjusted to 8 with saturated sodium carbonate solution. The mixture was extracted with dichloromethane (20 mL× 3). The organic phase was washed with saturated sodium chloride solution (20 mL), dried over anhydrous sodium sulfate and filtered. The filtrate was concentrated under reduced pressure. The residue was purified by high performance liquid chromatography (column: Xbridge-C18 150 x 19 mm, 5um; mobile phase: ACN--H₂O (0.1% FA); B%: 5%-10%, 20 min) to give the formate of **235** (21.4 mg, yield 73.7%) as a white solid. LC-MS m/z (ESI): 498.0 [M+H]⁺. ¹H NMR (400 MHz, DMSO-*d*₆, 353K) δ ppm 10.69 - 10.41 (m, 1H), 8.71 (s, 1H), 8.01 - 7.80 (m, 3H), 7.70 - 7.29 (m, 4H), 6.01 (s, 2H), 5.66 - 5.02 (m, 1H), 4.96 - 4.83 (m, 1H), 4.80 - 4.65 (m, 1H), 4.31- 4.25 (m, 3H), 4.20 - 4.03 (m, 2H), 2.92 - 2.70 (m, 3H), 2.19 - 2.10 (m, 1H), 1.02 - 0.93 (m, 4H).

### 55. Synthesis of example 236

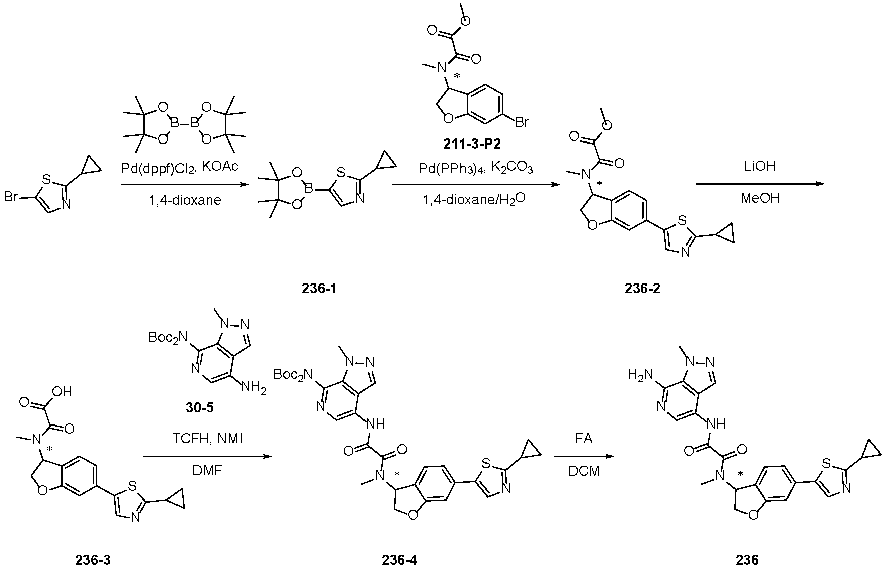

### 55.1 Synthesis of intermediate 236-1

[1,1'-Bis(diphenylphosphino)ferrocene]dichloropalladium(II) (200.0 mg), potassium acetate (720.0 mg) and bis(pinacolato)diboron (750.0 mg) were added to a solution of 5-bromo-2-cyclopropyl-thiazole (500.0 mg) in anhydrous 1,4-dioxane (15 mL), and the mixture was stirred at 90 °C for 16 hours under nitrogen. The mixture was cooled to room temperature and filtered. The filtrate was concentrated under reduced pressure to give a yellow liquid **236-1** (250.0 mg). The crude product was directly used in the next step reaction. LC-MS m/z (ESI): 170.1 [M-81]⁺.

### 55.2 Synthesis of intermediate 236-2

Tetrakis(triphenylphosphine)palladium(0) (115.0 mg) and potassium carbonate (206.0 mg) were added to a mixed solution of **236-1** (250.0 mg) and **211-3-P2** (300.0 mg) in 1,4-dioxane and water (15 mL, v/v = 4:1), and the mixture was stirred at 80 °C for 2 hours under nitrogen. The mixture was cooled to room temperature and concentrated under reduced pressure. The residue was purified by silica gel column chromatography (eluent: petroleum ether/ethyl acetate = 10/1 - 5/1) to give a yellow liquid **236-2** (200.0 mg, two-step yield: 22.7%). LC-MS m/z (ESI): 359.0 [M+H]⁺.

### 55.3 Synthesis of intermediate 236-3

Lithium hydroxide (27.0 mg) was added to a mixed solution of compound **236-2** (200.0 mg) in methanol and water (5 mL, v/v =1:1), and the mixture was stirred at room temperature for 2 hours. Water (20 mL) was added, and the mixture was washed with ethyl acetate (20 mL × 2). The pH of the aqueous phase was adjusted to weak acidity with hydrochloric acid (1 M). The mixture was extracted with dichloromethane (20 mL × 3). The organic phase was washed with saturated sodium chloride solution (30 mL), dried over anhydrous sodium sulfate and filtered. The filtrate was concentrated under reduced pressure to give a yellow solid **236-3** (120.0 mg). The crude product was directly used in the next step reaction. LC-MS m/z (ESI): 345.0 [M+H]⁺.

### 55.4 Synthesis of intermediate 236-4

Chloro-N,N,N',N'-tetramethylformamidinium hexafluorophosphate (82.0 mg) was added to a solution of N-methylimidazole (36.0 mg), **30-5** (53.0 mg) and **236-3** (50.0 mg) in N,N-dimethylformamide (3 mL), and the mixture was stirred at room temperature for 2 hours. Water (30 mL) was added, and the mixture was extracted with ethyl acetate (20 mL × 3). The organic phase was washed with saturated sodium chloride solution (20 mL), dried over anhydrous sodium sulfate and filtered. The filtrate was concentrated under reduced pressure. The residue was purified by silica gel column chromatography (eluent: dichloromethane/methanol = 10/1 - 5/1) to give a yellow liquid **236-4** (50.0 mg, yield 49.5%). LC-MS m/z (ESI): 690.4 [M+H]⁺.

### 55.5 Synthesis of compound 236

Anhydrous formic acid (4 mL) was added to a solution of **236-4** (50.0 mg) in dichloromethane (1 mL), and the mixture was stirred at room temperature for 2 hours. The mixture was concentrated under reduced pressure, and the pH of the residue was adjusted to 8 with saturated sodium carbonate solution. The mixture was extracted with dichloromethane (20 mL× 3). The organic phase was washed with saturated sodium chloride solution (20 mL), dried over anhydrous sodium sulfate and filtered. The filtrate was concentrated under reduced pressure. The residue was purified by high performance liquid chromatography (column: Xbridge-C18 150 x 19 mm, 5um; mobile phase: ACN--H₂O (0.1% FA); B%: 5%-10%, 20 min) to give the formate of **236** (23.3 mg, yield 65.8%) as a white solid. LC-MS m/z (ESI): 490.1 [M+H]⁺. ¹H NMR (400 MHz, DMSO-*d*₆, 80 °C) δ ppm 10.62 - 10.36 (m, 1H), 8.05 - 7.78 (m, 3H), 7.52 - 7.32 (m, 1H), 7.24 - 7.09 (m, 2H), 6.28 - 5.80 (m, 3H), 4.81 - 4.68 (m, 1H), 4.66 - 4.55 (m, 1H), 4.28 (s, 3H), 2.83- 2.60 (m, 3H), 2.44 - 2.34 (m, 1H), 1.17- 1.09 (m, 2H), 1.03 - 0.96 (m, 2H).

The compounds listed in the following table were synthesized by the same synthesis strategy as those of examples **4, 5, 159, 160, 163-P1, 163-P2, 173, 211** and **233:**

| Example | Structure | LC-MS [M+H]⁺ | ¹H NMR and SFC conditions |
|---|---|---|---|
| 46: 46-P1 (racemate: isomer) | 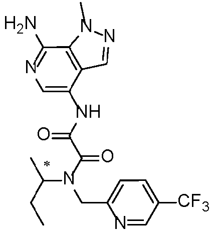 | 450.2 | ¹H NMR (400 MHz, DMSO-*d*₆, 80 °C) δ ppm 10.48 (s, 1H), 8.80 (s, 1H), 8.17 - 8.01 (m, 1H), 7.94 - 7.65 (m, 3H), 6.11 - 5.90 (m, 2H), 4.90 - 4.55 (m, 2H), 4.32 - 4.20 (m, 3H), 4.04 - 3.91 (m, 1H), 1.72 - 1.46 (m, 2H), 1.30 - 1.11 (m, 3H), 0.90 - 0.76 (m, 3H). |
| 46: 46-P2 | 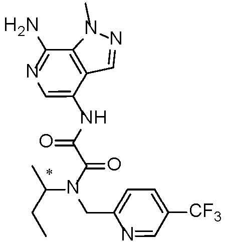 | 450.2 | ¹H NMR (400 MHz, DMSO-*d*₆, 80 °C) δ ppm 10.56 - 10.35 (m, 1H), 8.80 (s, 1H), 8.17 - 8.02 (m, 1H), 7.93 - 7.64 (m, 3H), 6.12 - 5.93 (m, 2H), 4.89 - 4.56 (m, 2H), 4.35 - 4.22 (m, 3H), 4.05 - 3.92 (m, 1H), 1.72 - 1.47 (m, 2H), 1.23 - 1.11 (m, 3H), 0.86 - 0.75 (m, 3H). |
| 47 | 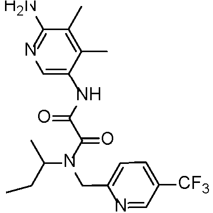 | 424.2 | ¹H NMR (400 MHz, DMSO-*d*₆, 80 °C) δ ppm 10.01 - 9.80 (m, 1H), 8.78 (s, 1H), 8.16 - 7.99 (m, 1H), 7.84 (d, J = 8.0 Hz, 1H), 7.70 - 7.41 (m, 1H), 5.44 - 5.30 (m, 2H), 4.89 - 4.55 (m, 2H), 4.22 - 3.94 (m, 1H), 2.10 - 1.89 (m, 6H), 1.69 - 1.46 (m, 2H), 1.23 - 1.10 (m, 3H), 0.83 (t, J = 7.4 Hz, 3H). |
| 48 | 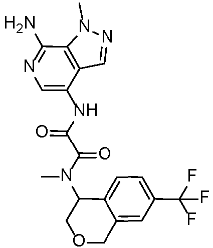 | 449.1 | ¹H NMR (400 MHz, DMSO-*d*₆, 80 °C) δ ppm 10.65 - 10.40 (m, 1H), 7.97 - 7.91 (m, 1H), 7.90 - 7.80 (m, 1H), 7.73 - 7.49 (m, 3H), 6.02 (s, 2H), 5.65 - 5.13 (m, 1H), 4.96 - 4.86 (m, 1H), 4.78 - 4.68 (m, 1H), 4.29 - 4.27 (m, 3H), 4.17 - 4.06 (m, 2H), 2.87 - 2.71 (m, 3H). |
| 49 | 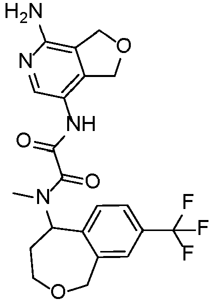 | 451.1 | ¹H NMR (400 MHz, DMSO-*d*₆, 80 °C) δ ppm 10.23 (s, 1H), 7.92 - 7.79 (m, 1H), 7.65 - 7.60 (m, 2H), 7.36 - 7.27 (m, 1H), 5.74 - 5.72 (m, 2H), 4.95 - 4.81 (m, 5H), 4.78 - 4.62 (m, 2H), 4.14 - 4.05 (m, 1H), 3.98 - 3.83 (m, 1H), 3.06 - 2.90 (m, 3H), 2.22 - 2.02 (m, 2H). |
| 50 | 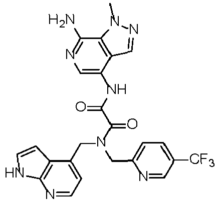 | 524.0 | ¹H NMR (400 MHz, CD₃OD-*d*₄) δ ppm 8.94 - 8.53 (m, 1H), 8.31 - 7.91 (m, 2H), 7.74 -7.89 (m, 2H), 7.73 - 7.27 (m, 2H), 7.24 -7.07 (m, 1H), 6.73 - 6.52 (m, 1H), 5.46 - 4.75 (m, 4H), 4.46 - 4.25 (m, 3H). |
| 51 | 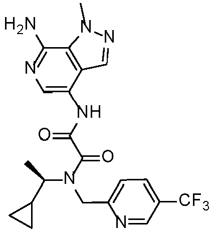 | 462.0 | ¹H NMR (400 MHz, CD₃OD-*d*₄) δ ppm 8.94 - 8.53 (m, 1H), 8.35 - 8.25 (m, 1H), 8.24 - 7.82 (m, 1H), 7.81 - 7.53 (m, 2H), 5.23 - 4.97 (m, 2H), 4.49 - 4.26 (m, 3H), 3.52 - 3.46 (m, 1H), 1.49 - 1.26 (m, 3H), 1.21 - 1.09 (m, 1H), 0.75 - 0.52 (m, 2H), 0.28 - 0.19 (m, 2H). |
| 52 | 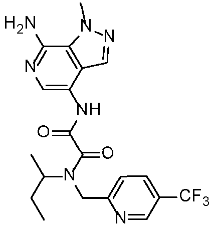 | 450.2 | ¹H NMR (400 MHz, MeOD-*d*₄) δ ppm 8.90 - 8.79 (m, 1H), 8.18 - 8.02 (m, 2H), 8.01 - 7.73 (m, 1H), 7.71 - 7.64 (m, 1H), 5.21 - 4.65 (m, 2H), 4.58 - 4.10 (m, 4H), 1.79 - 1.67 (m, 1H), 1.62 - 1.52 (m, 1H), 1.28 - 1.13 (m, 3H), 0.96 - 0.88 (m, 3H). |
| 53 | 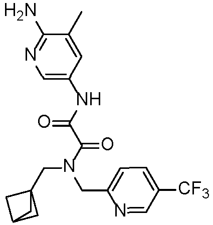 | 434.2 | ¹H NMR (400 MHz, DMSO-*d₆*) δ ppm 10.46 - 10.35 (m, 1H), 8.95 - 8.91 (m, 1H), 8.25 - 8.20 (m, 1H), 8.05 - 7.96 (m, 1H), 7.62 - 7.41 (m, 2H), 5.65 - 5.61 (m, 2H), 4.92 - 4.73 (m, 2H), 3.69 - 3.44 (m, 2H), 2.48 - 2.45 (m, 2H), 2.04 - 1.99 (m, 3H), 1.72 - 1.70 (m, 5H). |
| 54 | 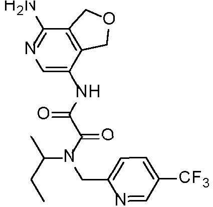 | 438.1 | ¹H NMR (400 MHz, DMSO-*d₆*) δ ppm 10.62 - 10.42 (m, 1H), 8.92 - 8.88 (m, 1H), 8.23 - 8.13 (m, 1H), 8.00 (s, 1H), 7.77 - 7.54 (m, 1H), 5.05 - 4.82 (m, 4H), 4.72 - 4.56 (m, 2H), 4.42 - 4.40 (m, 1H), 3.90 - 3.85 (m, 1H), 1.66 - 1.44 (m, 3H), 1.17 (d, *J* = 8.0 Hz, 1H), 1.02 (d, *J* = 8.0 Hz, 1H), 0.82 - 0.79 (m, 2H). |
| 55 | 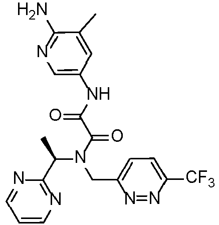 | 461.1 | ¹H NMR (400 MHz, MeOD-*d*₄) δ ppm 8.76 - 8.63 (m, 2H), 8.21 - 7.90 (m, 3H), 7.69 - 7.56 (m, 1H), 7.35 - 7.24 (m, 1H), 5.93 - 5.71 (m, 1H), 5.56 - 5.32 (m, 1H), 5.18 - 5.01 (m, 1H), 2.21 - 2.10 (m, 3H), 1.83 - 1.63 (m, 3H). |
| 56 | 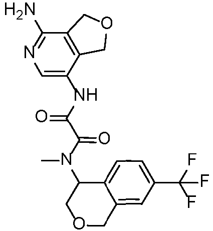 | 437.2 | ¹H NMR (400 MHz, MeOD-*d*₄) δ ppm 7.96 - 7.94 (m, 1H), 7.66 -7.44 (m, 3H), 5.70 - 5.28 (m, 1H), 5.07 - 5.05 (m, 2H), 4.98 - 4.96 (m, 2H), 4.95 - 4.73 (m, 2H), 4.25 - 4.07 (m, 2H), 2.94 - 2.79 (m, 3H). |
| 57 | 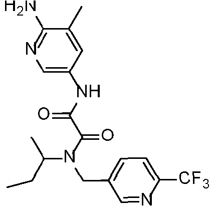 | 410.1 | ¹H NMR (400 MHz, MeOD-*d*₄) δ ppm 8.76 - 8.72 (m, 1H), 8.12 - 7.92 (m, 2H), 7.82 - 7.75 (m, 1H), 7.64 - 7.39 (m, 1H), 4.82 - 4.74 (m, 1H), 4.66 - 4.59 (m, 1H), 4.32 - 4.08 (m, 1H), 2.18 - 2.08 (m, 3H), 1.77 - 1.67 (m, 1H), 1.63 - 1.54 (m, 1H), 1.30 - 1.21 (m, 3H), 0.94 - 0.83 (m, 3H). |
| 58 | 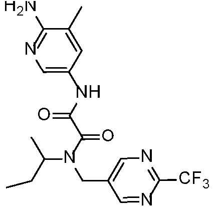 | 411.0 | ¹H NMR (400 MHz, MeOD-*d*₄) δ ppm 8.99 - 8.95 (m, 2H), 8.27 - 8.11 (m, 1H), 7.73 - 7.56 (m, 1H), 4.76 - 4.56 (m, 2H), 4.33 - 4.07 (m, 1H), 2.22 - 2.14 (m, 3H), 1.75 - 1.53 (m, 2H), 1.33 - 1.17 (m, 3H), 0.91- 0.83 (m, 3H). |
| 59 | 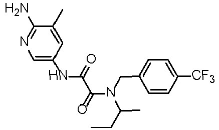 | 409.0 | ¹H NMR (400 MHz, DMSO-*d₆*) δ ppm 10.54 - 10.39 (m, 1H), 8.06 - 7.87 (m, 1H), 7.72 - 7.67 (m, 2H), 7.63 - 7.53 (m, 3H), 5.82 - 5.70 (m, 2H), 4.70 - 4.49 (m, 2H), 3.91 - 3.86 (m, 1H), 2.06 - 1.98 (m, 3H), 1.62 - 1.42 (m, 2H), 1.13 - 1.07 (m, 3H), 0.83 - 0.73 (m, 3H). |
| 60 | 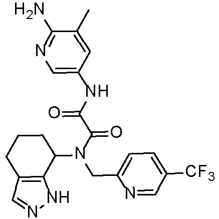 | 474.2 | ¹H NMR (400 MHz, MeOD-*d*₄) δ ppm 8.79 - 8.74 (m, 1H), 8.14 - 8.06 (m, 1H), 8.02 - 7.95 (m, 1H), 7.68 - 7.52 (m, 2H), 7.48 - 7.37 (m, 1H), 5.86 - 5.46 (m, 1H), 5.13 - 4.91 (m, 2H), 4.65 - 4.15 (m, 1H), 2.65 - 2.45 (m, 2H), 2.32 - 2.25 (m, 1H), 2.18 - 2.08 (m, 3H), 2.01 - 1.89 (m, 1H), 1.87 - 1.76 (m, 1H), 1.73 - 1.62 (m, 1H). |
| 61 | 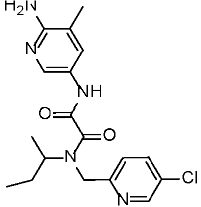 | 376.0 | ¹H NMR (400 MHz, MeOD-*d*₄) δ ppm 8.54 - 8.49 (m, 1H), 8.12 - 8.92 (m, 1H), 7.87 - 7.76 (m, 1H), 7.66 - 7.44 (m, 2H), 5.01 - 4.54 (m, 2H), 4.49 - 4.05 (m, 1H), 2.20 - 2.09 (m, 3H), 1.76 - 1.49 (m, 2H), 1.28 - 1.12 (m, 3H), 0.95 - 0.85 (m, 3H). |
| 62 | 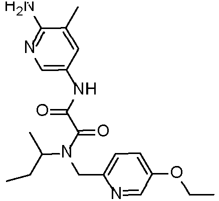 | 386.2 | ¹H NMR (400 MHz, MeOH-*d*₄) δ ppm 8.21 - 8.11 (m, 1H), 8.10 - 7.90 (m, 1H), 7.64 - 7.47 (m, 1H), 7.47 - 7.41 (m, 1H), 7.39 - 7.29 (m, 1H), 4.85 - 4.46 (m, 2H), 4.35 - 3.95 (m, 3H), 2.19 - 2.06 (m, 3H), 1.75 - 1.45 (m, 2H), 1.44 - 1.35 (m, 3H), 1.22 - 1.08 (m, 3H), 0.91 - 0.79 (m, 3H). |
| 63 | 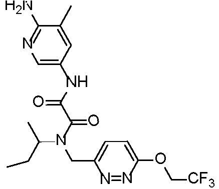 | 441.2 | ¹H NMR (400 MHz, DMSO-*d₆*) δ ppm 10.54 - 10.40 (m, 1H), 8.06 - 7.94 (m, 1H), 7.91 - 7.65 (m, 1H), 7.52 - 7.41 (m, 2H), 5.84 - 5.60 (m, 2H), 5.24 - 5.10 (m, 2H), 4.96 - 4.60 (m, 2H), 4.27 - 3.84 (m, 1H), 2.07 - 1.99 (m, 3H), 1.67 - 1.53 (m, 1H), 1.52 - 1.39 (m, 1H), 1.19 - 1.02 (m, 3H), 0.82 - 0.71 (m, 3H). |
| 64 | 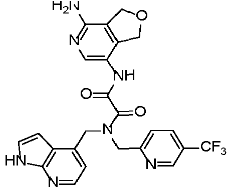 | 512.0 | ¹H NMR (400 MHz, CD₃OD) δ ppm 8.67 (s, 1H), 8.08 - 8.00 (m, 1H), 7.94 - 7.83 (m, 1H), 7.72 - 7.70 (m, 1H), 7.44 - 7.33 (m, 1H), 7.31 - 7.28 (m, 1H), 7.01 - 6.93 (m, 1H), 6.48 - 6.47 (s, 1H), 4.95 - 4.94 (m, 2H), 4.83 - 4.79 (m, 4H), 4.74 - 4.72 (m, 2H). |
| 65 | 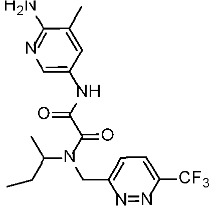 | 411.0 | ¹H NMR (400 MHz, DMSO-*d₆*) δ ppm 10.51 - 10.37 (m, 1H), 8.29 - 8.22 (m, 1H), 8.11 - 8.01 (m, 1H), 7.93 - 7.88 (m, 1H), 7.50 - 7.31 (m, 1H), 5.67 - 5.61 (m, 2H), 5.20 - 4.80 (m, 2H), 4.39 - 3.94 (m, 1H), 2.04 - 1.97 (m, 3H), 1.67 - 1.46 (m, 2H), 1.21 - 1.06 (m, 3H), 0.84 - 0.76 (m, 3H). |
| 66 | 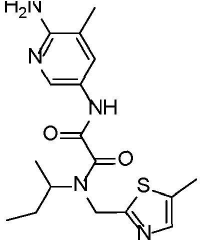 | 362.0 | ¹H NMR (400 MHz, DMSO-*d₆*) δ ppm 10.45 - 10.35 (m, 1H), 8.02 - 7.97 (m, 1H), 7.49 - 7.45 (m, 1H), 7.37 - 7.36 (m, 1H), 5.65 - 5.62 (m, 2H), 4.85 - 4.56 (m, 2H), 4.23 - 3.82 (m, 1H), 2.40 - 2.32 (m, 3H), 2.07 - 1.95 (m, 3H), 1.68 - 1.42 (m, 2H), 1.19 - 1.09 (m, 3H), 0.79 - 0.68 (m, 3H). |
| 67 | 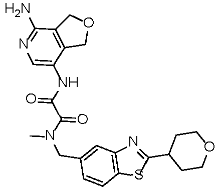 | 468.1 | ¹H NMR (400 MHz, MeOD-*d*₄) δ ppm 7.99 - 7.96 (m, 1H), 7.94 - 7.93 (m, 1H), 7.92 - 7.87 (m, 1H), 7.47 - 7.41 (m, 1H), 5.06 - 5.05 (m, 1H), 4.97 - 4.94 (m, 3H), 4.90 - 4.81 (m, 2H), 4.07 - 4.07 (m, 2H), 3.64 - 3.58 (m, 2H), 3.46 - 3.38 (m, 1H), 3.17 - 2.97 (m, 3H), 2.12 - 2.09 (m, 2H), 2.02 - 1.92 (m, 2H). |
| 68 | 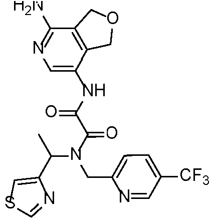 | 493.0 | ¹H NMR (400 MHz, MeOD-*d*₄) δ ppm 8.83 - 8.68 (m, 1H), 8.66 - 8.55 (m, 1H), 7.95 - 7.62 (m, 2H), 7.53 - 7.38 (m, 1H), 7.35 - 7.22 (m, 1H), 6.03 - 5.60 (m, 1H), 5.00 - 4.95 (m, 1H), 4.94 - 4.93 (m, 1H), 4.92 - 4.85 (m, 1H), 4.82 - 4.78 (m, 1H), 4.71 - 4.45 (m, 2H), 1.70 - 1.40 (m, 3H). |
| 69 | 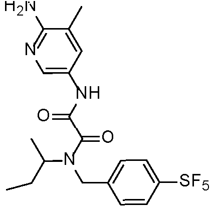 | 467.2 | ¹H NMR (400 MHz, DMSO-*d₆*) δ ppm 10.56 - 10.34 (m, 1H), 8.09 - 7.81 (m, 3H), 7.65 - 7.26 (m, 3H), 5.77 (s, 2H), 4.72 - 4.45 (m, 2H), 4.20 - 3.83 (m, 1H), 2.08 - 1.96 (m, 3H), 1.66 - 1.40 (m, 2H), 1.18 - 1.04 (m, 3H), 0.86 - 0.72 (m, 3H). |
| 70 | 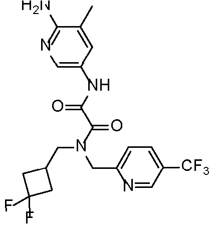 | 458.0 | ¹H NMR (400 MHz, MeOD-*d*₄) δ ppm 8.75 (s, 1H), 8.06 - 7.85 (m, 2H), 7.55 - 7.37 (m, 2H), 4.98 (s, 1H), 4.73 (s, 1H), 3.77 - 3.50 (m, 2H), 2.55 - 2.38 (m, 3H), 2.34 - 2.21 (m, 2H), 2.03 - 1.96 (m, 3H). |
| 71 | 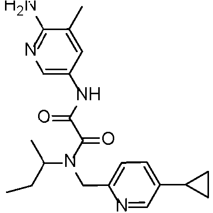 | 382.3 | ¹H NMR (400 MHz,CD₃OD) δ ppm 8.31 - 29 (m, 1H), 8.11 - 7.96 (m, 1H), 7.64 - 7.49 (m, 1H), 7.42 - 7.35 (m, 2H), 4.89 - 4.50 (m, 2H), 4.35 - 4.02 (m, 1H), 2.17 - 2.12 (m, 3H), 1.96 - 1.92 (m, 1H), 1.69 - 1.63 (m, 1H), 1.54 - 1.46 (m, 1H), 1.19 - 1.10 (m, 3H), 1.06 - 1.01 (m, 2H), 0.89 - 0.83 (m, 3H), 0.75 - 0.68 (m, 2H). |
| 72 | 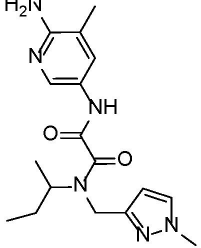 | 345.1 | ¹H NMR (400 MHz, DMSO-*d₆*) δ ppm 10.45 - 10.33 (m, 1H), 8.14 (s, 1H), 8.05 - 7.99 (m, 1H), 7.56 - 7.48 (m, 2H), 6.22 - 6.15 (m, 1H), 5.83 (s, 2H), 4.57 - 4.12 (m, 3H), 3.77 - 3.73 (m, 3H), 2.05 - 2.03 (m, 3H), 1.65 - 1.55 (m, 1H), 1.48 - 1.38 (m, 1H), 1.14 - 1.07 (m, 3H), 0.79 - 0.69 (m, 3H). |
| 73 | 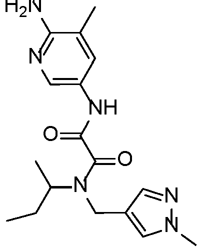 | 345.2 | ¹H NMR (400 MHz, CD₃OD) δ ppm 8.11 - 8.07 (m, 1H), 7.63 - 7.54 (m, 1H), 7.50 - 7.43 (m, 1H), 4.56 - 4.29 (m, 2H), 4.43 - 4.30 (m, 1H), 3.97 - 3.92 (m, 1H), 3.84 - 3.76 (m, 3H), 2.17 (s, 3H), 1.78 - 1.66 (m, 1H), 1.63 - 1.54 (m, 1H), 1.28 - 1.24 (m, 3H), 0.89 - 0.79 (m, 3H). |
| 74 | 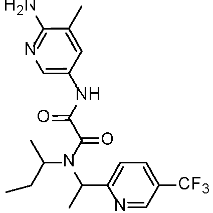 | 424.2 | ¹H NMR (400 MHz, DMSO-*d₆*) δ ppm 10.55 -10.32 (m, 1H), 9.00 - 8.89 (m, 1H), 8.31 - 8.12 (m, 1H), 8.09 - 7.97 (m, 1H), 7.87 - 7.68 (m, 1H), 7.59 - 7.45 (m, 1H), 5.73 (s, 2H), 5.30 - 4.69 (m, 1H), 3.91 - 3.75 (m, 1H), 2.10 - 2.00 (m, 3H), 1.89 - 1.45 (m, 5H), 1.41 - 1.21 (m, 3H), 0.96 - 0.76 (m, 3H). |
| 75 | 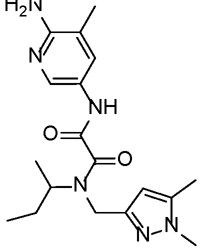 | 359.2 | ¹H NMR (400 MHz, MeOD-*d*₄) δ ppm 8.15 - 8.02 (m, 1H), 8.67 - 7. 58 (m, 1H), 6.09 (s, 1H), 4.64 - 4.32 (m, 2H), 4.28 - 3.89 (m, 1H), 3.76 - 3.61 (m, 3H), 2.31 - 2.20 (m, 3H), 2.21 - 2.12 (m, 3H), 1.79 - 1.46 (m, 2H), 1.26 - 1.14 (m, 3H), 0.93 - 0.74 (m, 3H). |
| 76 | 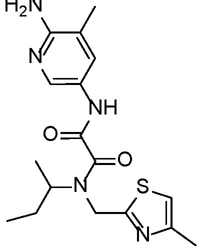 | 362.2 | ¹H NMR (400 MHz, DMSO-*d₆*) δ ppm 10.51 - 10.32 (m, 1H), 8.04 - 7.93 (m, 1H), 7.52 - 7.40 (m, 1H), 7.21 - 7.17 (m, 1H), 5.69 - 5.59 (m, 2H), 5.03 - 4.58 (m, 2H), 4.28 - 3.81 (m, 1H), 2.36 - 2.25 (m, 3H), 2.09 - 1.98 (m, 3H), 1.70 - 1.42 (m, 2H), 1.25 - 1.09 (m, 3H), 0.82 - 0.66 (m, 3H). |
| 77 | 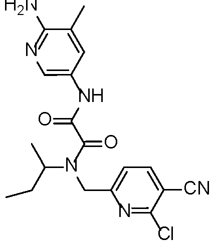 | 401.0 | ¹H NMR (400 MHz, DMSO-*d₆*) δ ppm 10.52 - 10.33 (m, 1H), 8.49 - 8.40 (m, 1H), 8.03 - 7.89 (m, 1H), 7.67 - 7.54 (m, 1H), 7.50 - 7.33 (m, 1H), 5.68 - 4.61 (m, 2H), 4.95 - 4.52 (m, 2H), 3.95 - 3.93 (m, 1H), 2.05 - 1.99 (m, 3H), 1.65 - 1.46 (m, 2H), 1.18 - 1.06 (m, 3H), 0.84 - 0.76 (m, 3H). |
| 78 | 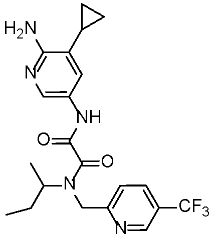 | 436.1 | ¹H NMR (400 MHz, CD₃OD) δ ppm 8.72 - 8.71 (m, 1H), 8.03 - 7.93 (m, 2H), 7.56 - 7.53 (m, 1H), 7.46 - 7.26 (m, 1H), 4.75 - 4.71 (m, 1H), 4.55 - 4.51 (m, 1H), 4.43 - 4.03 (m, 1H), 1.67 - 1.42 (m, 3H), 1.23 - 1.19 (m, 1H), 1.15 - 1.01 (m, 3H), 0.91 - 0.77 (m, 4H), 0.54 - 0.43 (m, 2H). |
| 79 | 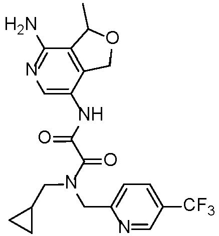 | 450.2 | ¹H NMR (400 MHz, DMSO-*d*₆, 80 °C) δ 10.53-10.35 (m, 1H), 8.88 (s, 1H), 8.16 (d, J = 8.1 Hz, 1H), 7.82 (m, 1H), 7.62 (m, 1H), 5.61 (m, 2H), 5.26 (s, 1H), 5.00 (m, 1H), 4.81 (m, 2H), 4.75 - 4.58 (m, 1H), 3.36 (m, 2H), 1.34 (m, 4H), 0.42 (m, 2H), 0.21 (m, 2H). |
| 80 | 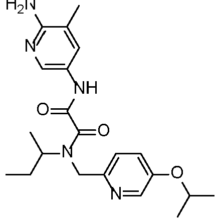 | 400.2 | ¹H NMR (400 MHz, DMSO-*d*₆ 80 °C) δ ppm 10.47 - 10.42 (m, 1H), 8.12 - 8.11 (m, 1H), 8.00 - 7.90 (m, 1H), 7.47 - 7.25 (m, 3H), 5.33 - 5.27 (m, 2H), 4.72 - 4.38 (m, 3H), 4.21 - 3.88 (m, 1H), 2.03 - 1.99 (m, 3H), 1.62 - 1.36 (m, 2H), 1.26 - 1.22 (m, 6H), 1.11 - 1.09 (m, 3H), 0.78 - 0.71 (m, 3H). |
| 81 | 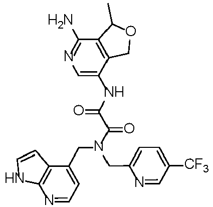 | 526.1 | ¹H NMR (400 MHz, DMSO-*d*₆, 80 °C) δ 11.47 (s, 1H), 10.21 - 10.29 (m, 1H), 8.84 (s, 1H), 8.17 - 8.13 (m, 1H), 8.11 - 8.07 (m, 1H), 7.80 (s, 1H), 7.54 - 7.51 (m, 1H), 7.45 - 7.35 (m, 1H), 7.01 - 6.93 (m, 1H), 6.49 (s, 1H), 6.00 (s, 2H), 5.21 (s, 1H), 5.15 (s, 1H), 4.93 (d, *J* = 3.6 Hz, 2H), 4.81 - 4.73 (m, 1H), 4.72 (s, 1H), 4.69 - 4.61 (m, 1H), 1.34 - 1.23 (m, 3H). |
| 82 | 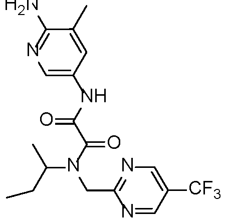 | 411.1 | ¹H NMR (400 MHz, DMSO-*d₆*) δ ppm 10.40 - 10.14 (m, 1H), 9.26 - 9.18 (m, 2H), 8.05 - 7.82 (m, 1H), 7.54 - 7.20 (m, 1H), 5.70 - 5.54 (m, 2H), 5.24 - 4.64 (m, 2H), 4.58 - 4.06 (m, 1H), 2.07 - 1.93 (m, 3H), 1.74 - 1.43 (m, 2H), 1.27 - 1.07 (m, 3H), 0.92 - 0.81 (m, 3H). |
| 83 | 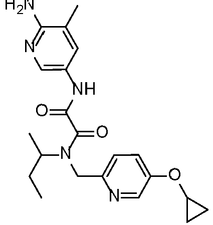 | 398.3 | ¹H NMR (400 MHz, DMSO-*d₆*) δ ppm 10.46 - 10.22 (m, 1H), 8.28 - 8.25 (m, 1H), 8.04 - 7.89 (m, 1H), 7.51 - 7.42 (m, 2H), 7.39 - 7.29 (m, 1H), 5.66 - 5.55 (m, 2H), 4.77 - 4.36 (m, 2H), 4.27 - 4.81 (m, 2H), 2.07 - 1.97 (m, 3H), 1.65 - 1.35 (m, 2H), 1.15 - 0.99 (m, 3H), 0.83 - 0.72 (m, 5H), 0.70 - 0.61 (m, 2H). |
| 84 | 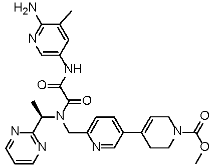 | 531.2 | ¹H NMR (400 MHz, DMSO-*d*₆, 80 °C) δ ppm 10.22 - 10.10 (m, 1H), 8.75 - 8.73 (m, 2H), 8.49 - 8.48 (m, 1H), 8.06 - 7.93 (m, 1H), 7.72 - 7.70 (m, 1H), 7.54 - 7.22 (m, 3H), 6.20 (s, 1H), 5.77 - 5.69 (m, 1H), 5.39 - 5.34 (m, 2H), 5.10 - 4.44 (m, 2H), 4.07 (s, 2H), 3.66 (s, 3H), 3.63 - 3.58 (s, 2H), 2.07 - 2.01 (m, 3H), 1.66 - 1.53 (m, 3H). |
| 85 | 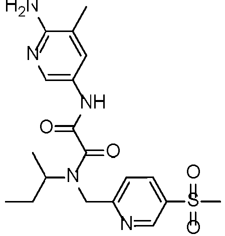 | 420.1 | ¹H NMR (400 MHz, DMSO-*d*₆, 80 °C) δ ppm 10.23 - 10.03 (m, 1H), 9.04 - 8.96 (m, 1H), 8.29 - 8.24 (m, 1H), 8.05 - 7.90 (m, 1H), 7.73 - 7.61 (m, 1H), 7.52 - 7.35 (m, 1H), 5.41 - 5.33 (m, 2H), 5.03 - 4.57 (m, 2H), 4.38 - 4.04 (m, 1H), 3.29 - 3.23 (m, 3H), 2.08 - 2.01 (m, 3H), 1.70 - 1.45 (m, 2H), 1.21 - 1.08 (m, 3H), 0.87 - 0.82 (m, 3H). |
| 86 | 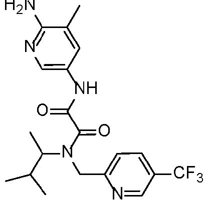 | 424.1 | ¹H NMR (400 MHz, DMSO-*d₆*) δ ppm 11.09 - 10.80 (m, 1H), 8.92 - 8.82 (m, 1H), 8.33 - 8.11 (m, 2H), 7.89 - 7.77 (m, 2H), 7.67 - 7.55 (m, 1H), 7.26 - 7.00 (m, 1H), 5.13 - 4.53 (m, 2H), 4.16 - 3.68 (m, 1H), 2.20 - 2.13 (m, 3H), 1.96 - 1.83 (m, 1H), 1.23 - 1.03 (m, 3H), 1.93 - 1.85 (m, 1H), 0.93 - 0.82 (m, 6H). |
| 87 | 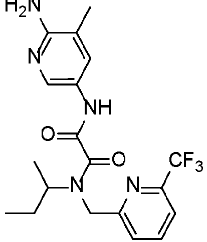 | 410.1 | ¹H NMR (400 MHz, DMSO-*d₆*) δ ppm 10.51 - 10.28 (m, 1H), 8.31 - 8.03 (m, 2H), 7.87 - 7.64 (m, 2H), 7.51 - 7.30 (m, 1H), 5.67 - 5.59 (m, 2H), 5.01 - 4.54 (m, 2H), 4.39 - 3.90 (m, 1H), 2.05 - 1.97 (m, 3H), 1.68 - 1.43 (m, 2H), 1.18 - 1.05 (m, 3H), 0.81 - 0.75 (m, 3H). |
| 88 | 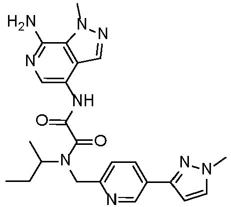 | 462.1 | ¹H NMR (400 MHz, DMSO-*d*₆, 80 °C) δ ppm 10.48 - 10.30 (m, 1H), 8.92 (s, 1H), 8.19 (s, 1H), 8.12 - 8.08 (m, 1H), 7.94 - 7.84 (m, 1H), 7.75 - 7.73 (m, 1H), 7.57 - 7.46 (m, 1H), 6.76 - 6.72 (m, 1H), 6.05 - 5.95 (m, 2H), 4.95 - 4.55 (m, 2H), 4.37 - 3.97 (m, 4H), 3.93 - 3.91 (m, 3H), 1.74 - 1.63 (m, 1H), 1.57 - 1.49 (m, H), 1.23 - 1.11 (m, 3H), 0.88 - 0.82 (m, 3H). |
| 89 | 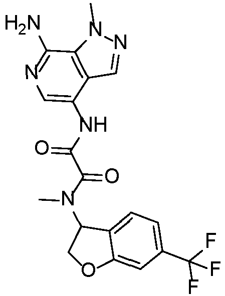 | 435.2 | ¹H NMR (400 MHz, DMSO-*d*₆, 80 °C) δ ppm 10.59 - 10.43 (m, 1H), 8.03 - 7.83 (m, 2H), 7.71 - 7.57 (m, 1H), 7.37 - 7.35 (m, 1H), 7.26 - 7.22 (m, 1H), 6.33 - 5.98 (m, 3H), 4.87 - 4.80 (m, 1H), 4.73 - 4.66 (m, 1H), 4.3 (s, 3H), 2.83 - 2.65 (m, 3H). |
| 90 | 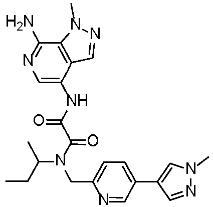 | 462.2 | ¹H NMR (400 MHz, DMSO-*d*₆, 80 °C) δ ppm 10.48 - 10.24 (m, 1H), 8.72 (s, 1H), 8.17 - 8.10 (m, 1H), 7.94 - 7.78 (m, 3H), 7.75 - 7.63 (m, 1H), 7.52 - 7.38 (m, 1H), 6.05 - 5.89 (m, 2H), 4.92 - 4.48 (m, 2H), 4.34 - 3.94 (m, 4H), 3.91 - 3.84 (m, 3H), 1.75 - 1.60 (m, 1H), 1.57 - 1.44 (m, 1H), 1.24 - 1.07 (m, 3H), 0.89 - 0.77 (m, 3H). |
| 91 | 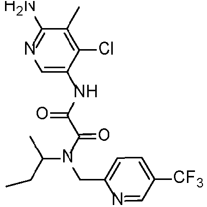 | 444.1 | ¹H NMR (400 MHz, DMSO-*d*₆, 80 °C) δ ppm 10.12 - 10.05 (m, 1H), 8.78 (s, 1H), 8.14 - 8.02 (m, 1H), 7.85 - 7.82 (m, 2H), 4.91 - 4.56 (m, 2H), 4.14 - 4.07 (m, 1H), 2.19 - 2.05 (m, 3H), 1.67 - 1.51 (m, 2H), 1.21 - 1.11 (m, 3H), 0.84 - 0.81 (m, 3H). |
| 92 | 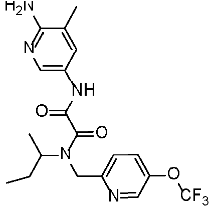 | 426.2 | ¹H NMR (400 MHz, DMSO-*d₆*) δ ppm 10.55 - 10.31 (m, 1H), 8.61 - 8.55 (m, 1H), 8.14 - 8.05 (m, 1H), 7.93 - 7.85 (m, 1H), 7.63 - 7.37 (m, 2H), 5.84 (s, 2H), 4.90 - 4.49 (m, 2H), 4.37 - 3.85 (m, 1H), 2.05 - 1.99 (m, 3H), 1.67 - 1.38 (m, 2H), 1.16 - 1.02 (m, 3H), 0.82 - 0.75 (m, 3H). |
| 93 | 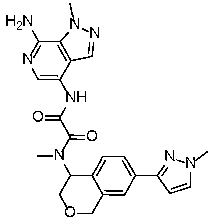 | 461.2 | ¹H NMR (400 MHz, DMSO-*d*₆, 80 °C) δ ppm 10.70 - 10.52 (m, 1H), 8.09 - 7.86 (m, 2H), 7.73 - 7.69 (m, 2H), 7.58 - 7.56 (m, 1H), 7.49 - 7.29 (m, 1H), 6.66 (s, 1H), 5.59 - 5.02 (m, 1H), 4.90 - 4.83 (m, 1H), 4.74 - 4.66 (m, 1H), 4.30 - 4.26 (m, 3H), 4.14 - 4.03 (m, 2H), 3.89 (s, 3H).2.87 - 2.78 (m, 3H). |
| 94 | 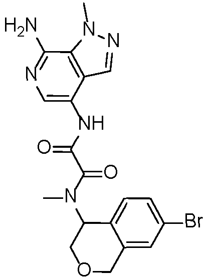 | 459.0 | ¹H NMR (400 MHz, DMSO-*d₆*) δ ppm 10.68 - 10.35 (m, 1H), 7.94 - 7.87 (m, 1H), 7.86 - 7.76 (m, 1H), 7.51 - 7.44 (m, 1H), 7.41 - 7.19 (m, 2H), 5.97 (s, 2H), 5.51 - 4.95 (m, 1H), 4.83 - 4.73 (m, 1H), 4.66 - 4.55 (m, 1H), 4.27 - 4.22 (m, 3H), 4.12 - 3.94 (m, 2H), 2.84 - 2.62 (m, 3H). |
| 95 | 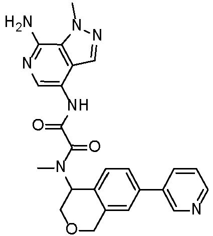 | 458.2 | ¹H NMR (400 MHz, DMSO-*d*₆, 80 °C) δ ppm 10.63 - 10.46 (m, 1H), 8.91 - 8.89 (m, 1H), 8.58 - 8.57 (m, 1H), 8.07 - 8.05 (m, 1H), 7.97 - 7.95 (m, 1H), 7.90 - 7.83 (m, 1H), 7.70 - 7.68 (m, 1H), 7.62 - 7.41 (m, 3H), 5.98 (s, 2H), 5.63 - 5.07 (m, 1H), 4.94 - 4.88 (m, 1H), 4.78 - 4.71 (m, 1H), 4.28 - 4.27 (m, 3H), 4.17 - 4.09 (m, 2H), 2.89 - 2.75 (m, 3H). |
| 96 | 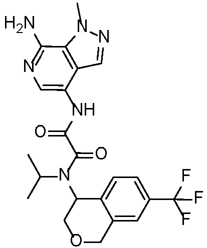 | 477.1 | ¹H NMR (400 MHz, DMSO-*d₆*) δ ppm 11.51 - 10.63 (m, 1H), 8.15 - 7.95 (m, 1H), 7.94 -7.82 (m, 1H), 7.82 - 7.63 (m, 1H), 7.62 - 7.49 (m, 1H), 7.48 - 7.43 (m, 1H), 7.36 - 6.61 (m, 2H), 4.89 - 4.61 (m, 2H), 4.32 - 4.25 (m, 2H), 4.23 - 4.95 (m, 2H), 3.38 - 3.15 (m, 3H), 1.43 - 1.25 (m, 3H), 1.23 - 0.82 (m, 3H). |
| 97 | 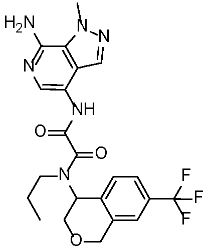 | 477.1 | ¹H NMR (400 MHz, DMSO-*d₆*) δ ppm 10.89 - 10.67 (m, 1H), 8.15 - 7.94 (m, 1H), 7.89 (s, 1H) 7.80 - 7.64 (m, 2H), 7.59 (s, 1H), 6.28 (s, 2H), 5.50 - 4.95 (m, 1H), 4.96 - 4.87 (m, 1H), 4.79 - 4.65 (m, 1H), 4.30 - 4.23 (m, 3H), 4.20 - 4.04 (m, 2H), 3.46 - 2.77 (m, 2H), 1.61 - 1.34 (m, 2H), 0.76 - 0.61 (m, 3H). |
| 98 | 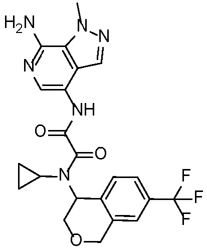 | 475.1 | ¹H NMR (400 MHz, DMSO-*d₆*) δ ppm 10.61 (s, 1H), 8.16 - 7.83 (m, 2H), 7.70 - 7.45 (m, 3H), 6.38 (s, 2H), 5.40 - 5.25 (m, 1H), 4.95 - 4.75 (m, 2H), 4.36 - 4.24 (m, 3H), 4.19 - 4.08 (m, 2H), 2.82 - 2.72 (m, 1H), 0.84 - 0.55 (m, 2H), 0.54 - 0.38 (m, 2H). |
| 99 | 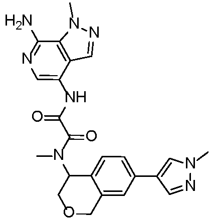 | 461.2 | ¹H NMR (400 MHz, DMSO-*d₆*) δ ppm 11.47 - 10.36 (m, 1H), 8.67 - 8.09 (m, 3H), 7.86 (s, 1H), 7.46 - 7.22 (m, 3H), 6.15 (s, 2H), 5.54 - 4.82 (m, 1H), 4.82 - 4.76 (m, 1H), 4.71 - 4.57 (m, 1H), 4.45 - 4.31 (m, 3H), 4.17 - 3.98 (m, 2H), 3.91 - 3.79 (m, 3H), 2.82 - 2.66 (m, 3H). |
| 100 | 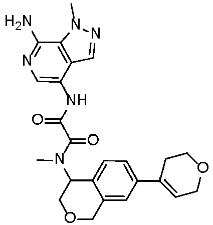 | 463.1 | ¹H NMR (400 MHz, DMSO-*d₆*) δ ppm 10.88 - 10.67 (m, 1H), 7.99 - 7.84 (m, 2H), 7.48 - 7.22 (m, 3H), 6.32 - 6.28 (m, 3H), 5.56 - 4.86 (m, 1H), 4.85 - 4.77 (m, 1H), 4.70 - 4.58 (m, 1H), 4.30 - 4.25 (m, 3H), 4.24 - 4.19 (m, 2H), 4.13 - 3.99 (m, 2H), 3.85 - 3.78 (m, 2H), 2.80 - 2.66 (m, 3H), 2.47 - 2.39 (m, 2H). |
| 101 | 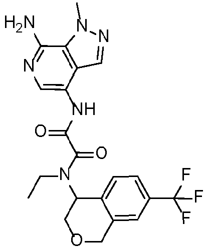 | 463.2 | ¹H NMR (400 MHz, DMSO-*d₆*) δ ppm 10.89 - 10.73 (m, 1H), 7.99 - 7.83 (m, 2H), 7.76 - 7.51 (m, 3H), 6.40 (s, 2H), 5.05 - 4.91 (m, 2H), 4.78 - 4.64 (m, 1H), 4.29 - 4.27 (m, 3H), 4.19 - 4.04 (m, 2H), 3.54 - 3.37 (m, 1H), 3.25 - 2.95 (m, 1H), 1.07 - 0.99 (m, 3H). |
| 102 | 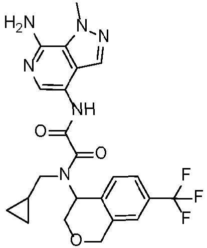 | 489.2 | ¹H NMR (400 MHz, DMSO-*d₆*) δ ppm 10.86 - 10.72 (m, 1H), 7.95 - 7.90 (m, 1H), 7.88 - 7.80 (m, 1H), 7.70 - 7.64 (m, 1H), 7.57 - 7.55 (m, 2H), 6.26 - 6.25 (m, 2H), 5.42 - 4.99 (m, 1H), 4.93 - 4.87 (m, 1H), 4.80 - 4.65 (m, 1H), 4.27 - 4.25 (m, 3H), 4.22 - 4.12 (m, 2H), 3.38 - 3.28 (m, 2H), 0.98 - 0.90 (m, 1H), 0.35 - 0.10 (m, 4H). |
| 103 | 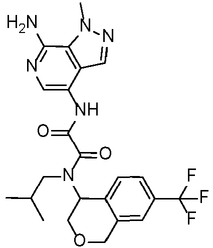 | 491.2 | ¹H NMR (400 MHz, DMSO-*d*₆, 80 °C) δ ppm 10.61 - 10.47 (m, 1H), 7.94 - 7.91 (m, 1H), 7.85 - 7.78 (m, 1H), 7.75 - 7.53 (m, 3H), 6.15 (s, 2H), 5.37 - 5.07 (m, 1H), 4.93 - 4.88 (m, 1H), 4.80 - 4.67 (m, 1H), 4.28 - 4.27 (m, 3H), 4.25 - 4.21 (m, 1H), 4.14 - 4.09 (m, 1H), 3.52 - 2.84 (m, 2H), 1.86 - 1.71 (m, 1H), 0.85 - 0.73 (m, 6H). |
| 104 | 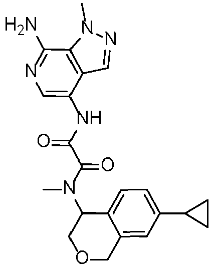 | 421.2 | ¹H NMR (400 MHz, DMSO-*d*₆, 80 °C) δ ppm 10.59 - 10.41 (m, 1H), 7.94 - 7.82 (m, 2H), 7.35 - 7.14 (m, 1H), 7.05 - 6.86 (m, 2H), 6.00 (s, 2H), 5.51 - 4.94 (m, 1H), 4.80 - 4.57 (m, 2H), 4.28 - 4.27 (m, 3H), 4.09 - 4.01 (m, 2H), 2.82 - 2.68 (m, 3H), 1.92 - 1.89 (m, 1H), 0.95 - 0.92 (m, 2H), 0.67 - 0.66 (m, 2H). |
| 105 | 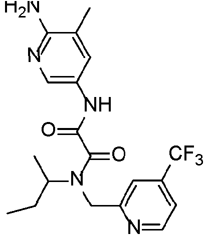 | 410.1 | ¹H NMR (400 MHz, DMSO-*d*₆, 80 °C) δ ppm 10.17 (s, 1H), 8.81 - 8.76 (m, 1H), 8.04 - 7.60 (m, 3H), 7.52 - 7.36 (m, 1H), 5.40 - 5.32 (m, 2H), 5.01 - 4.58 (m, 2H), 4.34 - 4.02 (m, 1H), 2.07 - 2.01 (m, 3H), 1.69 - 1.48 (m, 2H), 1.26 - 1.06 (m, 3H), 0.84 - 0.79 (m, 3H). |
| 106: 106-P1 | 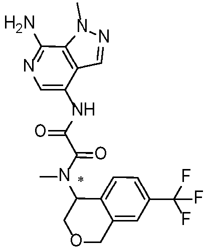 | 449.1 | ¹H NMR (400 MHz, DMSO-*d₆*) δ ppm 10.87 - 10.70 (m, 1H), 7.98 - 7.84 (m, 2H), 7.72 - 7.49 (m, 3H), 6.26 (s, 2H), 5.63 - 5.00 (m, 1H), 4.94 - 4.88 (m, 1H), 4.76 - 4.68 (m, 1H), 4.28 - 4.27 (m, 3H), 4.14 - 4.07 (m, 2H), 2.81 - 2.68 (m, 3H). |
| | | | SFC (column: Thar prep 80, CHIRALPAK AD-H 250mm x 20 mm, 5µm; mobile phase: 40%IPA (0.2% NH₄OH), RT (retention time) = 4.62 min. |
| 106: 106-P2 | 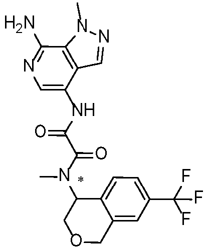 | 449.1 | ¹H NMR (400 MHz, DMSO-*d₆*) δ ppm 10.87 - 10.70 (m, 1H), 7.98 - 7.84 (m, 2H), 7.74 - 7.49 (m, 3H), 6.27 (s, 2H), 5.63 - 5.00 (m, 1H), 4.94 - 4.88 (m, 1H), 4.76 - 4.68 (m, 1H), 4.28 - 4.27 (m, 3H), 4.13 - 4.04 (m, 2H), 2.81 - 2.68 (m, 3H). |
| | | | SFC (column: Thar prep 80, CHIRALPAK AD-H 250mm x 20 mm, 5µm; mobile phase: 40%IPA (0.2% NH₄OH), RT = 5.26 min. |
| 107 | 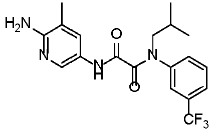 | 395.1 | ¹H NMR (400 MHz, DMSO-*d*₆, 80 °C)) δ 10.43 (s, 1H), 7.74 (s, 1H), 7.70 - 7.64 (m, 4H), 7.14 (s, 1H), 5.61 (s, 2H), 3.69 (d, *J* = 7.4 Hz, 2H), 1.95 (s, 3H), 1.69 (dt, *J =* 13.6, 6.8 Hz, 1H), 0.88 (d, *J* = 6.5 Hz, 6H). |
| 108 | 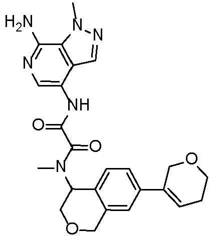 | 463.0 | ¹H NMR (400 MHz, DMSO-*d₆*) δ ppm 10.93 - 10.62 (m, 1H), 8.07- 7.83 (m, 2H), 7.47 -7.13 (m, 3H), 6.36 - 6.31 (m, 1H), 6.29 (s, 2H), 5.56 - 4.85 (m, 1H), 4.84 - 4.75 (m, 1H), 4.68 - 4.56 (m, 1H), 4.44 - 4.39 (m, 2H), 4.32 - 4.25 (m, 3H), 4.12 - 3.97 (m, 2H), 3.76 - 3.67 (m, 2H), 2.81 - 2.64 (m, 3H), 2.29 - 2.20 (m, 2H). |
| 109 | 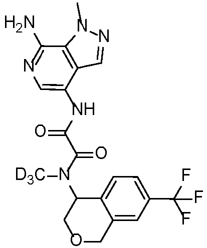 | 452.0 | ¹H NMR (400 MHz, DMSO-*d₆*) δ ppm 10.89 - 10.66 (m, 1H), 7.99 - 7.83 (m, 2H), 7.74 -7.46 (m, 3H), 6.29 - 6.18 (m, 2H), 5.68 - 4.97 (m, 1H), 4.96 - 4.83 (m, 1H), 4.76 - 4.65 (m, 1H), 4.31 - 4.26 (m, 3H), 4.16 - 4.08 (m, 2H). |
| 110: 110-P1 | 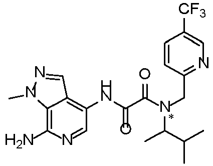 | 464.1 | ¹H NMR (400 MHz, DMSO-*d₆*) δ ppm 10.73 - 10.42 (m, 1H), 8.92 - 8.85 (m, 1H), 8.24 - 8.14 (m, 1H), 7.87 - 7.79 (m, 1H), 7.72 - 7.51 (m, 2H), 6.29 - 6.18 (m, 2H), 5.14 - 4.53 (m, 2H), 4.28 - 4.21 (m, 3H), 3.71 - 3.63 (m, 1H), 1.93 - 1.85 (m, 1H), 1.22 - 1.05 (m, 3H), 0.93 - 0.86 (m, 6H). |
| | | | SFC (column: chiralpak-IC 250 x 4.6 mm, 5um; mobile phase: 40% IPA (0.2% NH₄OH), RT = 2.91 min. |
| 110: 110-P2 | 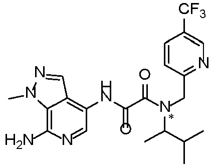 | 464.1 | ¹H NMR (400 MHz, DMSO-*d₆*) δ ppm 10.74 - 10.42 (m, 1H), 8.93 - 8.85 (m, 1H), 8.24 - 8.14 (m, 1H), 7.87 - 7.80 (m, 1H), 7.72 - 7.52 (m, 2H), 6.29 - 6.18 (m, 2H), 5.15 - 4.53 (m, 2H), 4.28 - 4.22 (m, 3H), 3.78 - 3.63 (m, 1H), 1.93 - 1.86 (m, 1H), 1.22 - 1.03 (m, 3H), 0.93 - 0.86 (m, 6H). |
| | | | SFC (column: chiralpak-IC 250 x 4.6 mm, 5um; mobile phase: 40% IPA (0.2% NH₄OH), RT = 3.42 min. |
| 111 | 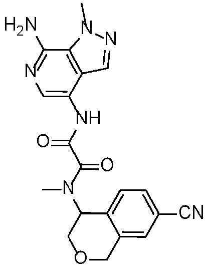 | 406.0 | ¹H NMR (400 MHz, DMSO-*d*₆, 80 °C) δ ppm 10.71 - 10. 39 (m, 1H), 8.15 - 7.95 (m, 1H), 7.99 -7.91 (m, 1H), 7.99 - 7.73 (m, 1H), 7.72 - 7.43 (m, 2H), 6.05 (s, 2H), 5.69 - 5.06 (m, 1H), 4.93 - 4.77 (m, 1H), 4.75 - 4.62 (m, 1H), 4.32 - 4.26 (m, 3H), 4.17 - 4.05 (m, 2H), 2.91 - 2.66 (m, 3H). |
| 112: 112-P1 | 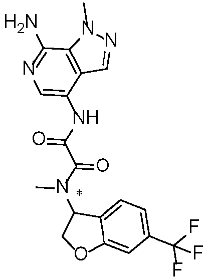 | 435.1 | ¹H NMR (400 MHz, DMSO-*d*₆, 80 °C) δ ppm 10.82 - 10.66 (m, 1H), 8.19 - 7.90 (m, 2H), 7.68 - 7.54 (m, 1H), 7.35 - 7.33 (m, 1H), 7.23 - 7.22 (m, 1H), 6.31 - 5.93 (m, 1H), 4.86 - 4.77 (m, 1H), 4.71 - 4.65 (m, 1H), 4.30 (s, 3H), 2.81 - 2.64 (m, 3H). |
| | | | SFC (column: chiralpak OJ-H 250 x 20 mm, 5um; mobile phase: 40% IPA (0.2% NH₄OH), RT = 2.22 min. |
| 112: 112-P2 | 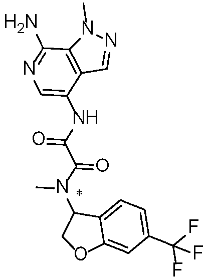 | 435.1 | ¹H NMR (400 MHz, DMSO-*d₆*) δ ppm 10.94 - 10.68 (m, 1H), 8.20 - 8.12 (m, 1H), 7.99 - 7.90 (m, 1H), 7.68 - 7.55 (m, 1H), 7.35 - 7.33 (m, 1H), 7.24 - 7.22 (m, 1H), 6.30 - 5.93 (m, 1H), 4.86 - 4.77 (m, 1H), 4.71 - 4.65 (m, 1H), 4.30 (s, 3H), 2.81 - 2.64 (m, 3H). |
| | | | SFC (column: chiralpak OJ-H 250 x 20 mm, 5um; mobile phase: 40% IPA (0.2% NH₄OH), RT = 2.67 min. |
| 113 | 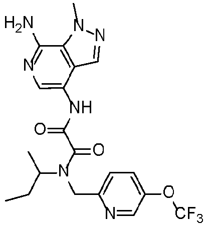 | 466.2 | ¹H NMR (400 MHz, DMSO-*d₆*) δ ppm 10.73 - 10.47 (m, 1H), 8.62 - 8.56 (m, 1H), 7.95 - 7.90 (m, 1H), 7.89 - 7.81 (m, 1H), 7.69 - 7.50 (m, 2H), 6.28 - 6.20 (m, 2H), 4.96 - 4.53 (m, 2H), 4.28 - 4.23 (m, 3H), 3.92 - 3.87 (m, 1H), 1.68 - 1.43 (m, 2H), 1.19 - 1.05 (m, 3H), 0.84 - 0.78 (m, 3H). |
| 114 | 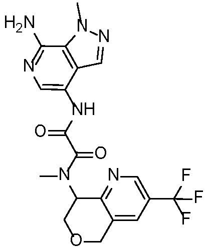 | 450.1 | ¹H NMR (400 MHz, DMSO-*d₆*) δ ppm 10.80 - 10.65 (m, 1H), 8.98 - 8.92 (m, 1H), 8.18 - 8.16 (m, 1H), 7.96 - 7.92 (m, 1H), 7.85- 7.80 (m, 1H), 6.28 (s, 2H), 5.60 - 5.20 (m, 1H), 5.00 - 4.90 (m, 1H), 4.85 - 4.70 (m, 1H), 4.32 - 4.25 (m, 3H), 4.23 - 4.11 (m, 2H), 2.86 - 2.64 (m, 3H). |
| 115 | 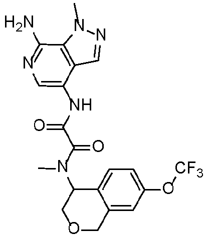 | 465.1 | ¹H NMR (400 MHz, DMSO-*d₆*) δ ppm 10.87 - 10.68 (m, 1H), 7.99 - 7.83 (m, 2H), 7.64 - 7.58 (m, 1H), 7.44 - 7.32 (m, 1H), 7.29 - 7.21 (m, 1H), 6.27 (s, 2H), 5.61 - 4.90 (m, 1H), 4.96 - 4.80 (m, 1H), 4.74 - 4.62 (m, 1H), 4.31 - 4.23 (m, 3H), 4.17 - 4.00 (m, 2H), 2.84 - 2.64 (m, 3H). |
| 116 | 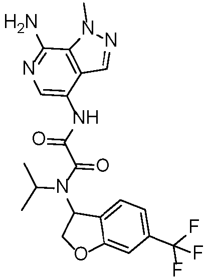 | 463.1 | ¹H NMR (400 MHz, DMSO-*d₆*) δ ppm 10.56 (s, 1H),8.17 (s, 1H), 8.86 - 8.84 (m, 1H) 7.38 - 7.32 (m, 1H), 7.26 - 7.20 (m, 1H), 7.14 - 7.09 (m, 1H), 6.30 - 6.17 (m, 2H), 5.53 - 5.38 (m, 1H), 4.86 - 4.74 (m, 1H), 4.66 - 4.58 (m, 1H), 4.29 - 7.23 (m, 3H), 4.14 - 4.05 (m, 1H), 1.17 - 1.13 (m, 3H), 1.13 - 1.12 (m, 3H). |
| 117 | 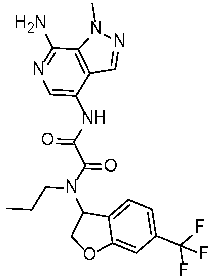 | 463.1 | ¹H NMR (400 MHz, DMSO-*d₆*) δ ppm 10.85 - 10.62 (m, 1H), 8.02 - 7.75 (m, 2H), 7.72 - 7.52 (m, 1H), 7.37 - 7.31 (m, 1H), 7.29 - 7.21 (m, 1H), 6.33 - 6.19 (m, 2H), 5.92 - 5.78 (m, 1H), 4.89 - 4.77 (m, 1H), 4.69 - 4.62 (m, 1H), 4.27 (s, 3H), 3.34 - 3.26 (m, 1H), 3.10 - 2.90 (m, 1H), 1.49 - 1.39 (m, 1H), 1.22 - 1.07 (m, 1H), 0.73 - 0.62 (m, 3H). |
| 118 | 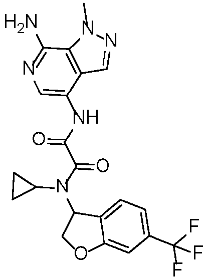 | 461.1 | ¹H NMR (400 MHz, DMSO-*d₆*) δ ppm 10.51 (s, 1H), 7.96 - 7.89 (m, 1H), 7.84 - 7.79 (m, 1H) 7.61 - 7.52 (m, 1H), 7.34 - 7.26 (m, 1H), 7.25 - 7.19 (m, 1H), 6.24 (s, 2H), 5.96 - 5.88 (m, 1H), 4.90 - 4.79 (m, 1H), 4.71 - 4.62 (m, 1H), 4.27 (s, 3H), 2.85 - 2.30 (m, 1H), 0.76 - 0.56 (m, 2H), 0.55 - 0.39 (m, 2H). |
| 119 | 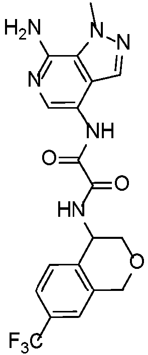 | 434.9 | ¹H NMR (400 MHz, DMSO-*d₆*) δ ppm 10.75 - 10.63 (s, 1H), 9.27 - 9.16 (m, 1H), 7.87 (s, 1H), 7.53 (s, 1H), 7.65 - 7.59 (m, 1H), 7.56 - 7.46 (m, 2H), 6.27 (s, 2H), 5.19 - 5.11 (m, 1H), 4.89 - 4.76 (m, 2H), 4.27 (s, 3H), 4.07 - 3.83 (m, 2H). |
| 120 | 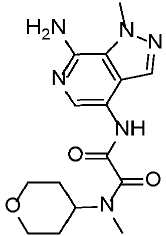 | 333.1 | ¹H NMR (400 MHz, DMSO-*d₆*) δ ppm 10.73 - 10.49 (m, 1H), 8.02 - 7.75 (m, 2H), 6.25 (s, 2H), 4.49 - 3.72 (m, 6H), 3.47 - 3.37 (m, 1H), 3.29 - 3.18 (m, 1H), 2.98 - 2.75 (m, 3H), 1.95 - 1.73 (m, 2H), 1.70 - 1.49 (m, 2H). |
| 121 | 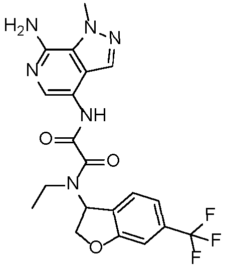 | 448.9 | ¹H NMR (400 MHz, DMSO-*d₆*) δ ppm 10.8 - 10.66 (m, 1H), 8.02 - 7.80 (m, 2H), 7.73 - 7.55 (m, 1H), 7.37 - 7.26 (m, 2H), 6.27 (s, 2H), 5.95 - 5.77 (m, 1H), 4.87 - 4.78 (m, 1H), 4.68 - 4.63 (m, 1H), 4.28 (s, 3H), 3.42 - 3.36 (m, 1H), 3.17 - 3.04 (m, 1H), 1.06 - 0.87 (m, 3H). |
| 122 | 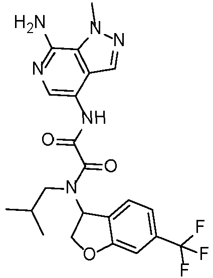 | 477.1 | ¹H NMR (400 MHz, DMSO-*d*₆, 80 °C) δ ppm 10.46 - 10.41 (m, 1H), 7.99 - 7.49 (m, 3H), 7.31 - 7.26 (m, 1H), 7.21 - 7.14 (m, 1H), 6.03 (s, 2H), 5.89 - 5.69 (s, 1H), 4.86 - 4.67 (m, 2H), 4.27 (s, 3H), 3.39 - 3.31 (m, 1H), 1.76 - 1.61 (m, 2H), 0.94 - 0.61 (m, 6H). |
| 123 | 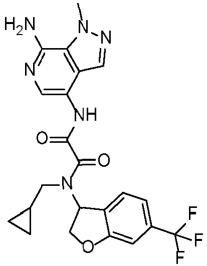 | 475.2 | ¹H NMR (400 MHz, DMSO-*d₆*) δ ppm 10.84 - 10.75 (m, 1H), 8.06 - 7.80 (m, 2H), 7.72 - 7.55 (m, 1H), 7.36 - 7.22 (m, 2H), 6.48 (s, 2H), 5.92 - 5.83 (m, 1H), 4.93 - 4.78 (m, 1H), 4.77 - 4.68 (m, 1H), 4.29 - 4.27 (m, 3H), 3.22 - 3.18 (m, 1H), 3.08 - 2.90 (m, 1H), 1.02 - 0.75 (m, 1H), 0.46 - 0.34 (m, 2H), 0.26 - 0.12 (m, 2H). |
| 124: 124-P1 | 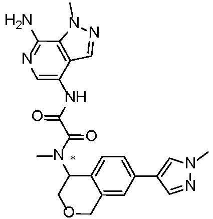 | 461.1 | ¹H NMR (400 MHz, DMSO-*d₆*) δ ppm 10.95 - 10.73 (m, 1H), 8.15 (s, 1H), 8.06 - 7.99 (m, 1H), 7.98 - 7.87 (m, 1H), 7.86 (s, 1H), 7.57 - 7.50 (m, 1H), 7.47 - 7.21 (m, 2H), 6.52 (s, 2H), 5.56 - 4.86 (m, 1H), 4.85 - 4.77 (m, 1H), 4.72 - 4.59 (m, 1H), 4.33 - 4.25 (m, 3H), 4.15 - 4.00 (m, 2H), 3.90 - 3.82 (m, 3H), 2.83 - 2.68 (m, 3H). |
| | | | SFC (column: CHIRALPAK IC 250 mm × 20 mm, 5µm; mobile phase: ETOH (0.2% NH₄OH); B%: 40% EtOH, 18 min), RT = 6.96 min). |
| 124: 124-P2 | 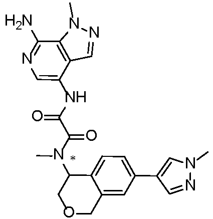 | 461.1 | ¹H NMR (400 MHz, DMSO-*d₆*) δ ppm 10.90 - 10.66 (m, 1H), 8.15 (s, 1H), 8.01 - 7.84 (m, 3H), 7.57 - 7.50 (m, 1H), 7.48 - 7.22 (m, 2H), 6.26 (s, 2H), 5.56 - 4.85 (m, 1H), 4.85 - 4.77 (m, 1H), 4.72 - 4.60 (m, 1H), 4.32 - 4.24 (m, 3H), 4.15 - 4.00 (m, 2H), 3.91 - 3.83 (m, 3H), 2.85 - 2.68 (m, 3H). |
| | | | SFC (column: CHIRALPAK IC 250 mm × 20 mm, 5µm; mobile phase: ETOH (0.2% NH₄OH); B%: 40% EtOH, 18 min), RT = 9.05 min). |
| 125: 125-P1 | 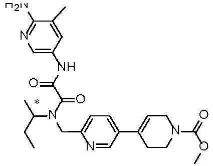 | 481.3 | ¹H NMR (400 MHz, DMSO-*d₆*) δ ppm 10.50 - 10.27 (m, 1H), 8.59 - 8.55 (m, 1H), 8.04 - 7.89 (m, 1H), 7.84 - 7.77 (m, 1H), 7.51 - 7.43 (m, 1H), 7.33 - 7.31 (m, 1H), 6.27 (s, 1H), 5.69 - 5.62 (m, 2H), 4.84 - 4.60 (m, 2H), 4.47 - 4.43 (m, 1H), 4.31 - 3.87 (m, 3H), 3.64 - 3.63 (m, 3H), 3.61 - 3.56 (m, 2H), 2.50 - 2.46 (m, 1H), 2.05 - 1.98 (m, 3H), 1.61 - 1.41 (m, 2H), 1.13 - 1.01 (m, 3H), 0.81 - 0.74 (m,3H). |
| | | | SFC (column: CHIRALPAK AS-H 250 mm x 20 mm, 5 µm; mobile phase: 40% IPA (0.2% NH₄OH), RT = 3.11 min. |
| 125: 125-P2 | 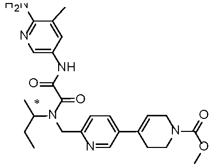 | 481.3 | ¹H NMR (400 MHz, DMSO-*d₆*) δ ppm 10.53 - 10.28 (m, 1H), 8.59 - 8.54 (m, 1H), 8.04 - 7.89 (m, 1H), 7.84 - 7.78 (m, 1H), 7.51 - 7.44 (m, 1H), 7.33 - 7.31 (m, 1H), 6.27 (s, 1H), 5.67 - 5.59 (m, 2H), 4.80 - 4.43 (m, 2H), 4.33 - 3.82 (m, 4H), 3.64 - 3.63 (m, 3H), 3.60 - 3.56 (m, 2H), 2.50 - 2.46 (m, 1H), 2.05 - 1.97 (m, 3H), 1.63 - 1.39 (m, 2H), 1.13 - 1.01 (m, 3H), 0.81 - 0.74 (m, 2H). |
| | | | SFC (column: CHIRALPAK AS-H 250 mm x 20 mm, 5 µm; mobile phase: 40% IPA (0.2% NH₄OH), RT = 3.52 min. |
| 126 | 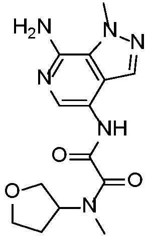 | 319.2 | ¹H NMR (400 MHz, DMSO-*d₆*) δ ppm 10.60 - 10.56 (m, 1H), 7.97 - 7.85 (m, 2H), 6.24 (s, 2H), 5.12 - 4.48 (m, 1H), 4.27 (s, 3H), 4.00 - 3.91 (m, 1H), 3.82 - 3.77 (m, 1H), 3.73 - 3.66 (m, 1H), 3.63 - 3.52 (m, 1H), 2.96 - 2.85 (m, 3H), 2.26 - 2.15 (m, 1H), 2.03 - 1.89 (m, 1H). |
| 127 | 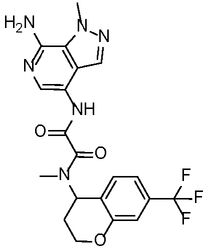 | 449.1 | ¹H NMR (400 MHz, DMSO-*d₆*) δ ppm 10.83 - 10.75 (m, 1H), 8.02 - 7.82 (m, 2H), 7.58 - 7.25 (m, 2H) 7.19 - 7.11 (m, 1H), 6.35 - 6.15 (m, 2H), 5.91 - 5.20 (m, 1H), 4.50 - 4.28 (m, 2H), 4.27 - 4.21 (m, 3H), 2.85 - 2.60 (m, 3H), 2.42 - 1.98 (m, 2H). |
| 128 | 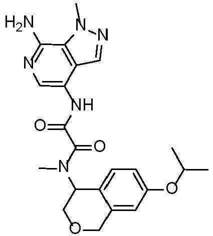 | 439.1 | ¹H NMR (400 MHz, DMSO-*d*₆, 80 °C) δ ppm 10.84 - 10.66 (m, 1H), 8.16 - 8.08 (m, 1H), 7.96 - 7.91 (m, 1H), 7.89 (s, 2H), 7.32 - 7.11 (m, 1H), 6.85 - 6.82 (m, 1H), 6.67 - 6.65 (m, 1H), 5.45 - 4.84 (m, 1H), 4.74 - 4.71 (m, 1H), 4.61 - 4.51 (m, 2H), 4.31 - 4.29 (m, 3H), 4.07 - 3.93 (m, 2H), 2.77 - 2.67 (m, 3H), 1.26 - 1.22 (m, 6H). |
| 129 | 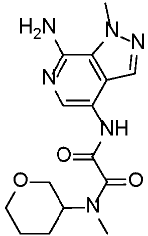 | 333.0 | ¹H NMR (400 MHz, DMSO-*d*₆, 80 °C) δ ppm 10.35 - 10.27 (m, 1H), 7.88 (s, 1H), 7.79 - 7.77 (m, 1H), 5.96 (s, 2H), 4.24 (s, 3H), 3.73 - 3.68 (m, 2H), 3.49 - 3.42 (m, 2H), 3.27 - 3.21 (m, 1H), 2.96 - 2.83 (m, 3H), 1.87 - 1.81 (m, 2H), 1.73 - 1.66 (m, 2H). |
| 130 | 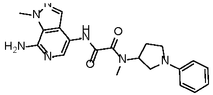 | 394.1 | ¹H NMR (400 MHz, DMSO-*d*₆, 80 °C) δ ppm 10.42 - 10.35 (m, 1H), 7.97 - 7.83 (m, 2H), 7.19 - 7.16 (m, 2H), 6.67 - 6.58 (m, 3H), 5.99 (s, 2H), 5.14 - 4.70 (m, 1H), 4.28 (s, 3H), 3.52 - 3.33 (m, 3H), 3.32 - 3.19 (m, 1H), 3.04 - 2.92 (m, 3H), 2.33 - 2.19 (m, 2H). |
| 131 | 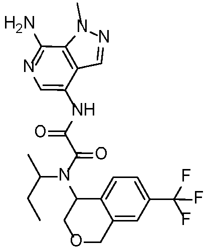 | 491.1 | ¹H NMR (400 MHz, DMSO-*d₆*) δ ppm 10.75 - 10.58 (m, 1H), 7.92 - 7.85 (m, 1H), 7.82 - 7.67 (m, 2H), 7.59 - 7.56 (m, 1H), 7.50 - 7.46 (m, 1H), 6.26 - 6.24 (m, 2H), 4.95 - 4.65 (m, 3H), 4.27 - 4.25 (m, 3H), 4.09 - 3.99 (m, 1H), 3.50 - 3.45 (m, 2H), 1.94 - 1.80 (m, 1H), 1.70 - 1.54 (m, 1H), 1.43 - 1.23 (m, 3H), 0.93 - 0.81 (m, 3H). |
| 132: 132-P1 | 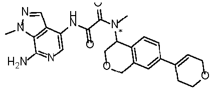 | 463.0 | ¹H NMR (400 MHz, CD₃OD-*d*₄) δ ppm 7.99 - 7.97 (m, 1H), 7.90 - 7.85 (m, 1H), 7.46 - 7.28 (m, 2H), 7.25 - 7.20 (m, 1H), 6.27 - 6.21 (m, 1H), 5.66 - 5.15 (m, 1H), 4.91 - 4.83 (m, 1H), 4.75 - 4.65 (m, 1H), 4.37 - 4.33 (m, 3H), 4.32 - 4.28 (m, 2H), 4.26 - 4.18 (m, 1H), 4.17 - 4.05 (m, 1H), 3.96 - 3.89 (m, 2H), 3.02 - 2.79 (m, 3H), 2.56 - 2.46 (m, 2H). |
| | | | SFC (column: CHIRALPAK AS-H 250 mm × 20 mm, 5 µm; mobile phase: 40% IPA (0.2% NH₄OH)), RT = 3.73 min). |
| 132: 132-P2 | 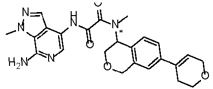 | 463.0 | ¹H NMR (400 MHz, CD₃OD-*d*₄) δ ppm 8.00 - 7.96 (m, 1H), 7.90 - 7.85 (m, 1H), 7.45 - 7.27 (m, 2H), 7.25 - 7.19 (m, 1H), 6.26 - 6.18 (m, 1H), 5.66 - 5.16 (m, 1H), 4.91 - 4.83 (m, 1H), 4.77 - 4.65 (m, 1H), 4.37 - 4.32 (m, 3H), 4.33 - 4.27 (m, 2H), 4.20 - 4.18 (m, 1H), 4.17 - 4.02 (m, 1H), 3.96 - 3.88 (m, 2H), 2.99 - 2.79 (m, 3H), 2.56 - 2.46 (m, 2H). |
| | | | SFC (column: CHIRALPAK AS-H 250 mm × 20 mm, 5 µm; mobile phase: 40% IPA (0.2% NH₄OH)), RT = 4.87 min). |
| 133 | 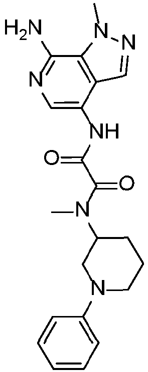 | 408.3 | ¹H NMR (400 MHz, DMSO-*d*₆, 80 °C) δ ppm 10.43 - 10.32 (m, 1H), 7.95 (s, 1H), 7.88 - 7.84 (m, 1H), 7.25 - 7.21 (m, 1H), 7.07 - 7.03 (m, 1H), 6.98 - 6.86 (m, 2H), 6.79 - 6.65 (m, 1H), 5.98 (s, 2H), 4.28 - 4.26 (m, 3H), 3.87 - 3.60 (m, 4H), 3.05 - 2.94 (m, 3H), 2.67 - 2.62 (m, 1H), 1.95 - 1.82 (m, 4H). |
| 134 | 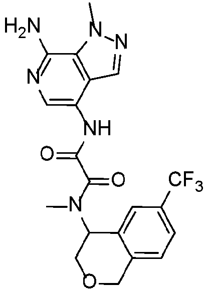 | 449.1 | ¹H NMR (400 MHz, DMSO-*d₆*) δ ppm 10.94 - 10.74 (m, 1H), 8.00 (s, 1H), 7.97 - 7.95 (m, 1H), 7.89 - 7.85 (m, 1H), 7.72 - 7.67 (m, 1H), 7.46 - 7.41 (m, 1H), 6.31 (s, 2H), 5.64 - 4.88 (m, 2H), 4.76 - 4.66 (m, 1H), 4.28 - 4.26 (m, 3H), 4.18 - 4.03 (m, 2H), 2.80 - 2.69 (m, 3H). |
| 135 | 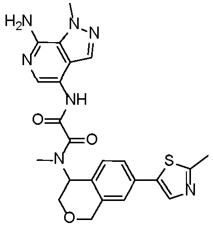 | 478.1 | ¹H NMR (400 MHz, DMSO-*d₆*) δ ppm 10.95 - 10.65 (m, 1H), 8.08 - 8.04 (m, 1H), 8.01 - 7.84 (m, 2H), 7.66 - 7.57 (m, 1H), 7.56 - 7.28 (m, 2H), 6.26 (s, 2H), 5.60 - 4.90 (m, 1H), 4.89 - 4.80 (m, 1H), 4.75 - 4.61 (m, 1H), 4.35 - 4.21 (m, 3H), 4.17 - 3.98 (m, 2H), 2.84 - 2.69 (m, 3H), 2.68 - 2.64 (m, 3H). |
| 136 | 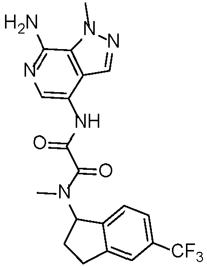 | 433.0 | ¹H NMR (400 MHz, DMSO-*d₆*) δ ppm 10.86 - 10.66 (m, 1H), 8.02 - 7.82 (m, 2H), 7.73 - 7.36 (m, 3H), 6.33 - 6.19 (m, 2H), 6.15 - 5.43 (m, 1H), 4.33 - 4.21 (m, 3H), 3.18 - 2.86 (m, 2H), 2.78 - 2.59 (m, 3H), 2.45 - 2.35 (m, 1H), 2.26 - 2.09 (m, 1H). |
| 137 | 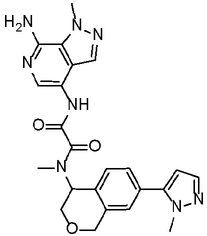 | 461.2 | ¹H NMR (400 MHz, DMSO-*d₆*) δ ppm 10.90 - 10.68 (m, 1H), 8.01 - 7.83 (m, 2H), 7.64 - 7.33 (m, 4H), 7.46 - 7.40 (m, 1H), 6.26 (s, 2H), 5.65 - 4.93 (m, 1H), 4.92 - 4.83 (m, 1H), 4.76 - 4.64 (m, 1H), 4.34 - 4.22 (m, 3H), 4.20 - 4.01 (m, 2H), 3.92 - 3.79 (m, 3H), 2.88 - 2.68 (m, 3H). |
| 138 | 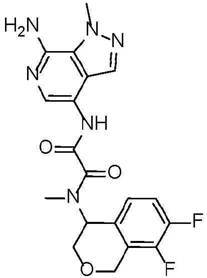 | 417.1 | ¹H NMR (400 MHz, DMSO-*d₆*) δ ppm 10.84 - 10.68 (m, 1H), 7.97 - 7.84 (m, 2H), 7.53 - 7.43 (m, 1H), 7.39 - 7.36 (m, 1H), 6.27 (s, 2H), 5.46 - 4.89 (m, 2H), 4.75 - 4.66 (m, 1H), 4.27 - 4.26 (m, 3H), 4.14 - 4.00 (m, 2H), 2.80 - 2.68 (m, 3H). |
| 139 | 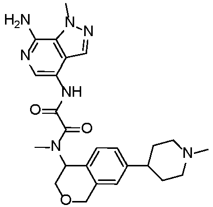 | 478.0 | ¹H NMR (400 MHz, DMSO-*d₆*) δ ppm 10.86 - 10.64 (m, 1H), 8.00 - 7.81 (m, 2H), 7.45 - 7.22 (m, 1H), 7.21 - 7.18 (m, 1H), 7.05 - 7.00 (m, 1H), 6.26 (s, 2H), 5.55 - 4.83 (m, 1H), 4.83 - 4.74 (m, 1H), 4.68 - 4.55 (m, 1H), 4.32 - 4.22 (m, 3H), 4.14 - 3.94 (m, 2H), 3.04 - 2.92 (m, 2H), 2.81 - 2.64 (m, 3H), 2.55 - 2.51 (m, 1H), 2.35 - 2.25 (m, 3H), 2.23 - 2.09 (m, 2H), 1.82 - 1.59 (m, 4H). |
| 140: 140-P1 | 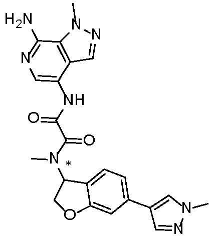 | 447.0 | ¹H NMR (400 MHz, DMSO-*d*₆, 80 °C) δ ppm 10.55 - 10.39 (m, 1H), 8.07 (s, 1H), 8.01 -7.81 (m, 3H), 7.41 - 7.28 (m, 1H), 7.19 - 7.17 (m, 1H), 7.09 - 7.07 (m, 1H), 6.21 - 5.79 (m, 3H), 4.75 - 4.69 (m, 1H), 4.60 - 4.53 (m, 1H), 4.28 (s, 3H), 3.86 (s, 3H), 2.77 - 2.63 (m, 3H). |
| | | | SFC (column: CHIRALPAK IC 250 mm × 20 mm, 5 µm; mobile phase: ETOH (0.2% NH₄OH); B%: 40% EtOH, 19 min), RT = 6.48 min. |
| 140: 140-P2 | 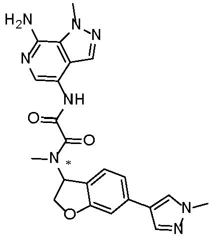 | 447.0 | ¹H NMR (400 MHz, DMSO-*d*₆, 80 °C) δ ppm 10.56 - 10.39 (m, 1H), 8.07 - 7.81 (m, 4H), 7.41 - 7.28 (m, 1H), 7.19 - 7.17 (m, 1H), 7.09 - 7.07 (m, 1H), 6.21 - 5.79 (m, 3H), 4.75 - 4.67 (m, 1H), 4.60 - 4.53 (m, 1H), 4.28 (s, 3H), 3.86 (s, 3H), 2.77 - 2.63 (m, 3H). |
| | | | SFC (column: CHIRALPAK IC 250 mm × 20 mm, 5 µm; mobile phase: ETOH (0.2% NH₄OH); B%: 40% EtOH, 19 min), RT = 9.20 min. |
| 141: 141-P1 | 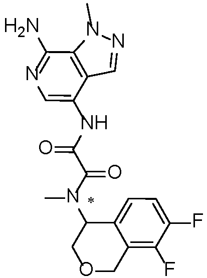 | 417.0 | ¹H NMR (400 MHz, DMSO-*d₆*) δ ppm 10.84 - 10.68 (m, 1H), 7.96 - 7.84 (m, 2H), 7.50 - 7.14 (m, 2H), 6.26 (s, 2H), 5.55 - 4.88 (m, 2H), 4.74 - 4.66 (m, 1H), 4.27 - 4.26 (m, 3H), 4.14 - 3.98 (m, 2H), 2.80 - 2.68 (m, 3H). |
| | | | SFC (column: CHIRALPAK IC 250 mm x 20 mm, 5um; mobile phase: ETOH (0.2% NH₄OH); B%: 40% EtOH, 19 min), RT = 2.32 min. |
| 141: 141-P2 | 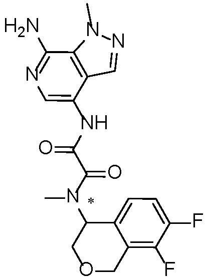 | 417.0 | ¹H NMR (400 MHz, DMSO-*d₆*) δ ppm 10.85 - 10.70 (m, 1H), 7.97 - 7.84 (m, 2H), 7.50 - 7.15 (m, 2H), 6.27 (s, 2H), 5.54 - 4.91 (m, 2H), 4.74 - 4.66 (m, 1H), 4.28 - 4.26 (m, 3H), 4.15 - 3.99 (m, 2H), 2.80 - 2.68 (m, 3H). |
| | | | SFC (column: CHIRALPAK IC 250 mm x 20 mm, 5um; mobile phase: ETOH (0.2% NH₄OH); B%: 40% EtOH, 19 min), RT = 3.70 min. |
| 142 | 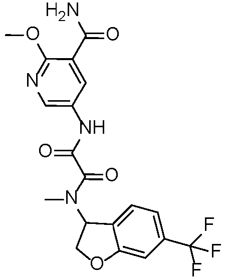 | 439.1 | ¹H NMR (400 MHz, DMSO-*d*₆, 80 °C) δ ppm 10.91 - 10.75 (m, 1H), 8.57 - 8.47 (m, 2H), 7.66 - 7.53 (m, 3H), 7.33 - 7.32 (m, 1H), 7.22 (s, 1H), 6.27 - 6.03 (m, 1H), 4.84 - 4.76 (m, 1H), 4.69 - 4.63 (m, 1H), 4.00 (s, 3H), 2.81 - 2.62 (m, 3H). |
| 143 | 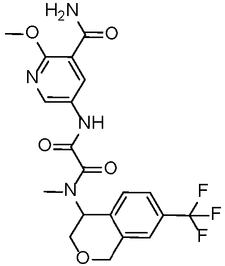 | 453.1 | ¹H NMR (400 MHz, DMSO-*d*₆, 80 °C) δ ppm δ 10.96 - 10.79 (m, 1H), 8.58 - 8.49 (m, 2H), 7.66 - 7.47 (m, 5H), 5.62 - 5.21 (m, 1H), 4.94 - 4.88 (m, 1H), 4.76 - 4.69 (m, 1H), 4.14 - 4.00 (m, 2H), 4.01 - 4.00 (m, 3H), 2.86 - 2.70 (m, 3H). |
| 144 | 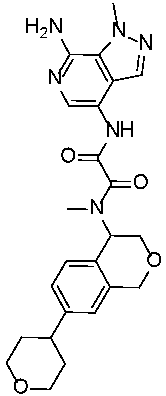 | 465.2 | ¹H NMR (400 MHz, DMSO-*d₆*) δ ppm 10.91 - 10.62 (m, 1H), 8.42 - 7.84 (m, 2H), 7.44 - 6.99 (m, 3H), 6.33 - 6.20 (m, 2H), 5.56 - 4.86 (m, 1H), 4.85 - 4.77 (m, 1H), 4.69 - 4.55 (m, 1H), 4.31 - 4.24 (m, 3H), 4.13 - 3.88 (m, 4H), 3.53 - 3.41 (m, 4H), 2.82 - 2.63 (m, 4H), 1.75 - 1.55 (m, 4H). |
| 145 | 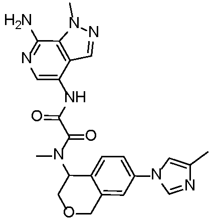 | 461.0 | ¹H NMR (400 MHz, DMSO-*d₆*) δ ppm 10.90 - 10.67 (m, 1H), 8.18 - 8.07 (m, 1H), 8.02 - 7.82 (m, 2H), 7.66 - 7.32(m, 4H), 6.26 (s, 2H), 5.63 - 4.90 (m, 1H), 4.89 - 4.79 (m, 1H), 4.74 - 4.60 (m, 1H), 4.32 - 4.20 (m, 3H), 4.17 - 3.98 (m, 2H), 2.85 - 2.64 (m, 3H), 2.23 - 2.09 (m, 3H). |
| 146: 146-P1 | 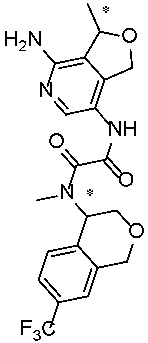 | 450.9 | ¹H NMR (400 MHz, DMSO-*d₆*) δ ppm 10.57 - 10.39 (m, 1H), 7.99 - 7.85 (m, 1H), 7.74 - 7.41 (m, 3H), 6.05 - 5.89 (m, 2H), 5.63 - 5.16 (m, 1H), 4.98 - 4.86 (m, 3H), 4.84 - 4.76 (m, 1H), 4.75 - 4.66 (m, 1H), 4.17 - 4.01 (m, 2H), 2.81 - 2.61 (m, 3H), 1.38 - 1.27 (m, 3H). |
| | | | SFC (column: CHIRALPAK AS-H 250 mm × 20 mm, 5 µm; mobile phase: 40% IPA (0.2% NH₄OH)), RT = 2.53 min. |
| 146: 146-P2 | 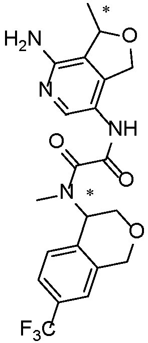 | 450.9 | ¹H NMR (400 MHz, DMSO-*d₆*) δ ppm 10.63 - 10.41 (m, 1H), 8.02 - 7.85 (m, 1H), 7.74 - 7.43 (m, 3H), 5.98 - 5.86 (m, 2H), 5.65 - 5.16 (m, 1H), 5.08 - 4.86 (m, 3H), 4.84 - 4.76 (m, 1H), 4.76 - 4.65 (m, 1H), 4.16 - 3.98 (m, 2H), 2.81 - 2.57 (m, 3H), 1.37 - 1.25 (m, 3H). |
| | | | SFC (column: CHIRALPAK AS-H 250 mm × 20 mm, 5 µm; mobile phase: 40% IPA (0.2% NH₄OH)), RT = 2.83 min. |
| 146: 146-P3 | 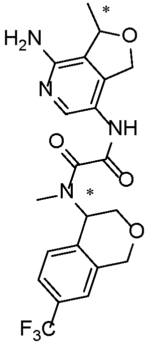 | 450.9 | ¹H NMR (400 MHz, DMSO-*d₆*) δ ppm 10.62 - 10.39 (m, 1H), 7.97 - 7.84 (m, 1H), 7.76 - 7.37 (m, 3H), 5.98 - 5.88 (m, 2H), 5.62 - 5.18 (m, 1H), 4.99 - 4.87 (m, 3H), 4.82 - 4.75 (m, 1H), 4.73 - 4.65 (m, 1H), 4.17 - 4.02 (m, 2H), 2.80 - 2.60 (m, 3H), 1.37 - 1.29 (m, 3H). |
| | | | SFC (column: CHIRALPAK AS-H 250 mm × 20 mm, 5µm; mobile phase: 40% IPA (0.2% NH₄OH)), RT = 3.55 min. |
| 147 | 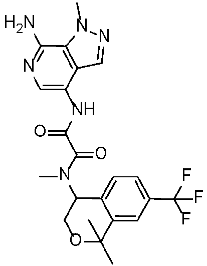 | 476.9 | ¹H NMR (400 MHz, DMSO-*d₆*) δ ppm 10.90 - 10.68 (m, 1H), 8.03 - 7.81 (m, 2H), 7.79 - 7.42 (m, 3H), 6.26 (s, 2H), 5.60 - 4.87 (m, 1H), 4.34 - 4.22 (m, 3H), 4.21 - 3.96 (m, 2H), 2.86 - 2.65 (m, 3H), 1.63 - 1.40 (m, 6H). |
| 148 | 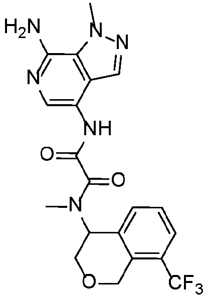 | 449.1 | ¹H NMR (400 MHz, DMSO-*d₆*) δ ppm 10.86 - 10.70 (m, 1H), 8.19 - 7.98 (s, 1H), 7.94 - 7.58 (m, 4H), 6.27 (s, 2H), 5.64 - 5.01 (m, 1H), 4.98 - 4.94 (m, 1H), 4.80 - 4.72 (m, 1H), 4.29 - 4.27 (m, 3H), 4.18 - 4.15 (m, 1H), 4.13 - 4.09 (m, 1H), 2.81 - 2.69 (m, 3H). |
| 149 | 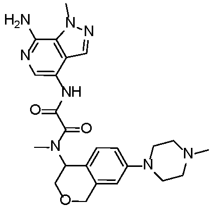 | 479.0 | ¹H NMR (400 MHz, DMSO-*d₆*) δ ppm 10.87 - 10.62 (m, 1H), 8.02 - 7.82 (m, 2H), 7.35 - 7.05 (m, 1H), 7.01 - 6.87 (m, 1H), 6.75 - 6.62 (m, 1H), 6.24 (s, 2H), 5.50 - 4.75 (m, 1H), 4.74 - 4.66 (m, 1H), 4.62 - 4.49 (m, 1H), 4.34 - 4.22 (m, 3H), 4.12 - 3.90 (m, 2H), 3.25 - 3.05 (m, 4H), 2.83 - 2.62 (m, 3H), 2.48 - 2.35 (m, 4H), 2.30 - 2.15 (m, 3H). |
| 150 | 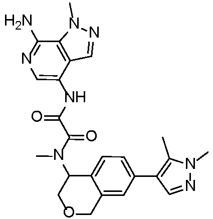 | 475.2 | ¹H NMR (400 MHz, DMSO-*d₆*) δ ppm 10.91 - 10.66 (m, 1H), 8.01 - 7.84 (m, 2H), 7.64 - 7.57 (m, 1H), 7.53 - 7.24 (m, 2H), 7.22 - 7.15 (m, 1H), 6.26 (s, 2H), 5.60 - 4.88 (m, 1H), 4.87 - 4.80 (m, 1H), 4.75 - 4.60 (m, 1H), 4.32 - 4.23 (m, 3H), 4.17 - 3.98 (m, 2H), 3.81 - 3.74 (m, 3H), 2.84 - 2.67 (m, 3H), 2.41 - 2.33 (m, 3H). |
| 151 | 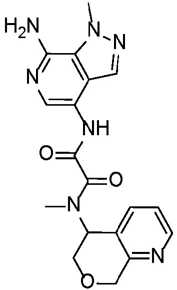 | 382.1 | ¹H NMR (400 MHz, DMSO-*d₆*) δ ppm 10.84 - 10.69 (m, 1H), 8.52 - 8.24 (m, 1H), 7.98 - 7.68 (m, 3H), 7.43 - 7.39 (m, 1H), 6.27 (s, 2H), 5.63 - 5.00 (m, 1H), 4.82 - 4.77 (m, 1H), 4.69 - 4.60 (m, 1H), 4.28 - 4.27(m, 3H), 4.15 - 4.08 (m, 2H), 2.82 - 2.70 (m, 3H). |
| 152: 152-P1 | 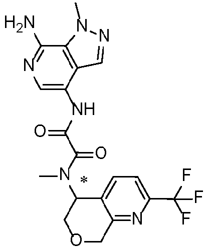 | 450.1 | ¹H NMR (400 MHz, DMSO-*d*₆ 80 °C) δ ppm 10.83 - 10.72 (m, 1H), 8.21 - 7.85 (m, 4H), 7.17 (s, 2H), 5.72 - 5.21 (m, 1H), 4.91 - 4.86 (m, 1H), 4.79 - 4.70 (m, 1H), 4.32 - 3.91 (m, 3H), 4.21 - 4.14 (m, 2H), 2.91 - 2.74 (m, 3H). |
| | | | SFC (column: CHIRALPAK IC 250 mm x 20 mm, 5um; mobile phase: 40% MeOH (NH₄OH 0.2%); 60% CO₂,), RT = 4.67 min. |
| 152: 152-P2 | 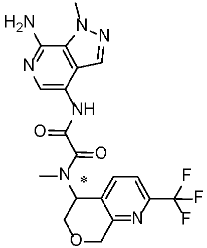 | 450.1 | ¹H NMR (400 MHz, DMSO-*d*₆, 80 °C) δ ppm 10.52 (s, 1H), 8.20 - 7.82 (m, 4H), 6.03 (s, 2H), 5.72 - 5.22 (m, 1H), 4.91 - 4.86 (m, 1H), 4.79 - 4.70 (m, 1H), 4.29 - 4.27 (m, 3H), 4.21 - 4.15 (m, 2H), 2.92 - 2.74 (m, 3H). |
| | | | SFC (column: CHIRALPAK IC 250 mm x 20 mm, 5um; mobile phase: 40% MeOH (NH₄OH 0.2%); 60% CO₂,), RT = 5.21 min. |
| 153: 153-P1 | 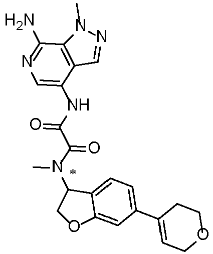 | 449.1 | ¹H NMR (400 MHz, DMSO-*d*₆, 80 °C) δ ppm 10.62 - 10.46 (m, 1H), 8.12 - 7.83 (m, 2H), 7.42 - 7.28 (m, 1H), 7.09 - 7.07 (m, 1H), 6.96 - 6.94 (m, 1H), 6.40 - 5.80 (m, 4H), 4.74 - 4.66 (m, 1H), 4.60 - 4.53 (m, 1H), 4.29 (s, 3H), 4.24 - 4.19 (m, 2H), 3.85 - 3.81 (m, 2H), 2.77 - 2.59 (m, 3H), 2.45 - 2.40 (m, 2H). |
| | | | SFC (column: CHIRALPAK IC 250 mm × 20 mm, 5 µm; mobile phase: ETOH (NH₄OH 0.2%); B%: 40% EtOH, 19 min), RT = 5.19 min. |
| 153: 153-P2 | 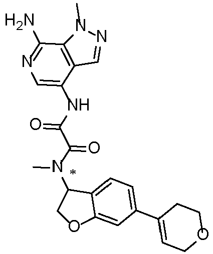 | 449.1 | ¹H NMR (400 MHz, DMSO-*d*₆, 80 °C) δppm 10.51 - 10.36 (m, 1H), 7.97 - 7.77 (m, 2H), 7.38 - 7.28 (m, 1H), 7.25 - 7.05 (m, 1H), 6.92 - 6.90 (m, 1H), 6.21 - 5.76 (m, 4H), 4.70 - 4.63 (m, 1H), 4.56 - 4.50 (m, 1H), 4.24 (s, 3H), 4.18 (s, 2H), 3.79 - 3.77 (m, 2H), 2.74 - 2.56 (m, 3H), 2.45 - 2.40 (m, 2H). |
| | | | SFC (column: CHIRALPAK IC 250 mm × 20 mm, 5 µm; mobile phase: ETOH (NH₄OH 0.2%); B%: 40% EtOH, 19 min), RT = 6.99 min. |
| 154 | 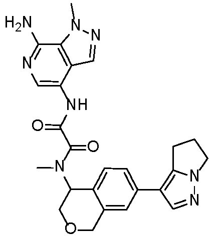 | 487.2 | ¹H NMR (400 MHz, DMSO-*d*₆, 80 °C) δ ppm 10.61 - 10.44 (m, 1H), 7.96 - 7.83 (m, 4H), 7.46 - 7.41 (m, 1H), 7.27 - 7.26 (m, 1H), 6.02 (s, 2H), 5.56 - 5.01 (m, 1H), 4.87 - 4.81 (m, 1H), 4.71 - 4.63 (m, 1H), 4.29 - 4.27 (m, 3H), 4.13 - 4.05 (m, 4H), 3.01 - 2.98 (m, 2H), 2.86 - 2.73 (m, 3H), 2.67 - 2.59 (m, 2H). |
| 155: 155-P1 | 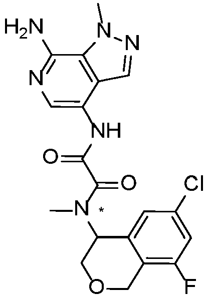 | 432.9 | ¹H NMR (400 MHz, DMSO-*d₆*) δ ppm 10.93 - 10.65 (m, 1H), 8.07 - 7.79 (m, 2H), 7.54 - 7.18 (m, 2H), 6.34 - 6.19 (m, 2H), 5.59 - 4.78 (m, 2H), 4.72 - 4.86 (m, 1H), 4.32 - 4.22 (m, 3H), 3.38 - 3.26 (m, 2H), 2.88 - 2.66 (m, 3H). |
| | | | SFC (column: CHIRALPAK AS-H 250 mm × 20 mm, 5 µm; mobile phase: 40% IPA (0.2% NH₄OH)), RT = 2.09 min. |
| 155: 155-P2 | 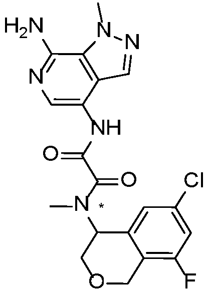 | 432.9 | ¹H NMR (400 MHz, DMSO-*d₆*) δ ppm 11.03 - 10.69 (m, 1H), 8.12 - 7.78 (m, 2H), 7.52 - 7.19 (m, 2H), 6.32 - 6.21 (m, 2H), 5.62 - 4.81 (m, 2H), 4.69 - 4.88 (m, 1H), 4.31 - 4.26 (m, 3H), 3.36 - 3.25 (m, 2H), 2.86 - 2.68 (m, 3H). |
| | | | SFC (column: CHIRALPAK AS-H 250 mm × 20 mm, 5 µm; mobile phase: 40% IPA (0.2% NH₄OH)), RT = 4.42 min. |
| 156: 156-P1 | 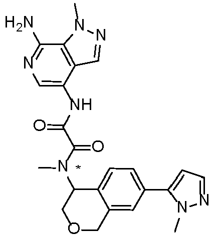 | 461.2 | ¹H NMR (400 MHz, DMSO-*d₆*) δ ppm 10.90 - 10.67 (m, 1H), 8.01 - 7.83 (m, 2H), 7.64 - 7.31 (m, 4H), 6.47 - 6.40 (m, 1H), 6.26 (s, 2H), 5.65 - 4.93 (m, 1H), 4.92 - 4.83 (m, 1H), 4.78 - 4.62 (m, 1H), 4.36 - 4.22 (m, 3H), 4.20 - 4.01 (m, 2H), 3.93 - 3.79 (m, 3H), 2.88 - 2.68 (m, 3H). |
| | | | SFC (column: CHIRALPAK AS-H 250 mm × 20 mm, 5 µm; mobile phase: 40% IPA (0.2% NH₄OH)), RT =2.76 min. |
| 156: 156-P2 | 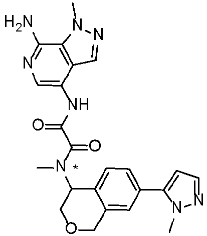 | 461.1 | ¹H NMR (400 MHz, DMSO-*d₆*) δ ppm 10.92 - 10.65 (m, 1H), 8.03 - 7.84 (m, 2H), 7.64 - 7.32 (m, 4H), 6.46 - 6.40 (m, 1H), 6.30 (s, 2H), 5.65 - 4.93 (m, 1H), 4.92 - 4.82 (m, 1H), 4.79 - 4.64 (m, 1H), 4.36 - 4.21 (m, 3H), 4.20 - 4.01 (m, 2H), 3.92 - 3.79 (m, 3H), 2.90 - 2.68 (m, 3H). |
| | | | SFC (column: CHIRALPAK AS-H 250 mm × 20 mm, 5 µm; mobile phase: 40% IPA (0.2% NH₄OH)), RT = 3.73 min. |
| 157 | 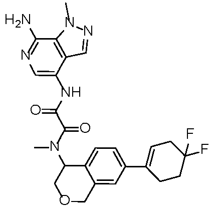 | 497.0 | ¹H NMR (400 MHz, DMSO-*d₆*) δ ppm 11.01 - 10.72 (m, 1H), 8.10 - 8.00 (m, 1H), 7.99 - 7.88 (m, 1H), 7.49 - 7.21 (m, 3H), 6.67 (s, 2H), 6.05 (s, 1H), 5.57 - 4.86 (m, 1H), 4.85 - 4.76 (m, 1H), 4.71 - 4.56 (m, 1H), 4.35 - 4.25 (m, 3H), 4.15 - 3.98 (m, 2H), 2.82 - 2.73 (m, 2H), 2.71 - 2.65 (m, 2H), 2.58 - 2.52 (m, 3H), 2.26 - 2.09 (m, 2H). |
| 158 | 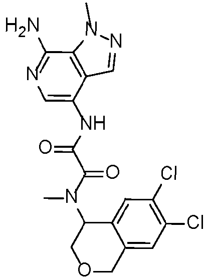 | 448.8 | ¹H NMR (400 MHz, DMSO-*d*₆, 80 °C) δ ppm 10.64 - 10.46 (m, 1H), 7.97 - 7.48 (m, 4H), 6.03 (s, 2H), 5.55 - 4.98 (m, 1H), 4.84 - 4.78 (m, 1H), 4.66 - 4.58 (m, 1H), 4.28 - 4.27 (m, 3H), 4.10 - 4.03 (m, 2H), 2.87 - 2.72 (m, 3H). |
| 161 | 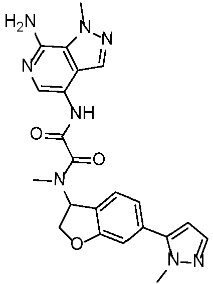 | 447.2 | ¹H NMR (400 MHz, DMSO-*d*₆, 80 °C) δ ppm 10.65 - 10.40 (m, 1H), 8.06 - 7.80 (m, 2H), 7.59 - 7.41 (m, 2H), 7.21 - 7.01 (m, 2H), 6.45 - 6.35 (m, 1H), 6.34 - 5.85 (m, 3H), 4.89 - 4.52 (m, 2H), 4.35 - 4.22 (m, 3H), 3.92 - 3.75 (m, 3H), 2.86 - 2.61 (m, 3H). |
| 162: 162-P1 | 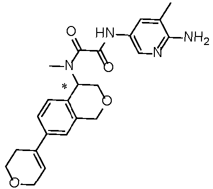 | 423.1 | ¹H NMR (400 MHz, DMSO-*d*₆, 80 °C) δ ppm 10.48 - 10.13 (m, 1H), 8.13 - 8.02 (m, 1H), 7.58 - 7.49 (m, 1H), 7.47 - 7.18 (m, 3H), 6.29 - 6.22 (m, 1H), 5.59 - 5.00 (m, 3H), 4.88 - 4.76 (m, 1H), 4.71 - 4.59 (m, 1H), 4.27 - 4.19 (m, 2H), 4.15 - 3.95 (m, 2H), 3.87 - 3.44 (m, 2H), 2.84 - 2.63 (m, 3H), 2.47 - 2.37 (m, 2H), 2.09 - 2.02 (m, 3H). |
| | | | SFC (column: CHIRALPAK IC 250 mm x 20 mm, 5um; mobile phase: 40%IPA (0.2% NH₄OH)), RT = 7.76 min. |
| 162: 162-P2 | 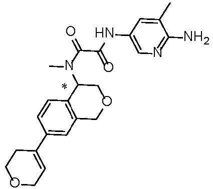 | 423.1 | ¹H NMR (400 MHz, DMSO-*d*₆, 80 °C) δ ppm 10.48 - 10.13 (m, 1H), 8.13 - 8.02 (m, 1H), 7.58 - 7.49 (m, 1H), 7.47 - 7.18 (m, 3H), 6.29 - 6.22 (m, 1H), 5.59 - 5.00 (m, 3H), 4.88 - 4.76 (m, 1H), 4.71 - 4.59 (m, 1H), 4.27 - 4.19 (m, 2H), 4.15 - 3.95 (m, 2H), 3.87 - 3.44 (m, 2H), 2.84 - 2.63 (m, 3H), 2.47 - 2.37 (m, 2H), 2.09 - 2.02 (m, 3H). |
| | | | SFC (column: CHIRALPAK IC 250 mm x 20 mm, 5um; mobile phase: 40%IPA (0.2% NH₄OH)), RT = 9.53 min. |
| 164 | 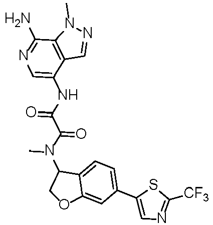 | 518.1 | ¹H NMR (400 MHz, DMSO-*d₆*) δ ppm 10.82 - 10.67 (m, 1H), 8.61 - 8.57 (m, 1H), 8.04 - 7.83 (m, 2H), 7.60 - 7.58 (m, 1H), 7.45 - 7.42 (m, 2H), 6.26 (s, 2H), 5.85 - 5.77 (m, 1H), 4.80 - 4.75 (m, 1H), 4.70 - 4.62 (m, 1H), 4.27 (s, 3H), 2.75 - 2.62 (m, 3H). |
| 165 | 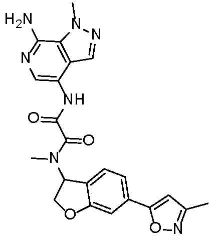 | 448.1 | ¹H NMR (400 MHz, DMSO-*d₆*) δ ppm 10.82 - 10.67 (m, 1H), 8.04 - 7.83 (m, 2H), 7.61 - 7.47 (m, 2H), 7.38 - 7.36 (m, 1H), 6.93 - 6.92 (m, 1H), 6.29 (s, 2H), 5.83 - 5.80 (m, 1H), 4.82 - 4.74 (m, 1H), 4.68 - 4.61 (m, 1H), 4.27 (s, 3H), 2.74 - 2.61 (m, 3H), 2.29 - 2.27 (m, 3H). |
| 166 | 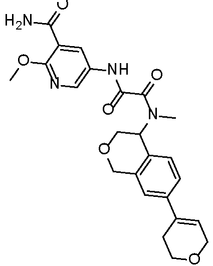 | 467.2 | ¹H NMR (400 MHz, DMSO-*d*₆, 80 °C) δ ppm 10.93 -10.76 (m, 1H), 8.54 - 8.45 (m, 2H), 7.27 - 7.36 (m, 2H), 7.38 - 7.18 (m, 3H), 6.26 (s, 1H), 5.55 - 5.06 (m, 1H), 4.81 - 4.76 (m, 1H), 4.66 - 4.58 (m, 1H), 4.25 - 4.20 (m, 2H), 4.06 - 4.01 (m, 2H), 3.97 - 3.96 (m, 3H), 3.81 - 3.78 (m, 2H), 2.79 - 2.66 (m, 3H), 2.44 - 2.38 (m, 2H). |
| 167 | 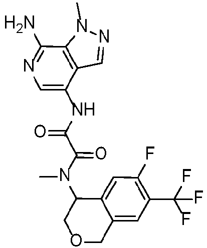 | 467.1 | ¹H NMR (400 MHz, DMSO-*d₆*) δ ppm 10.85 - 10.68 (m, 1H), 7.99 - 7.81 (m, 2H), 7.73 - 7.38 (m, 2H), 6.27 (s, 2H), 5.63 - 4.97 (m, 1H), 4.92 - 4.83 (m, 1H), 4.74 - 4.62 (m, 1H), 4.30 - 4.25 (m, 3H), 4.13 - 4.04 (m, 2H), 2.88 - 2.70 (m, 3H). |
| 168: 168-P1 | 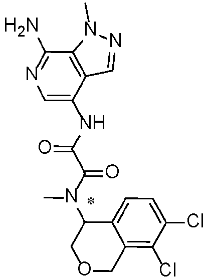 | 449.1 | ¹H NMR (400 MHz, DMSO-*d₆*) δ ppm 10.87 - 10.66 (m, 1H), 7.99 - 7.83 (m, 2H), 7.72 - 7.65 (m, 1H), 7.55 - 7.29 (m, 1H), 6.26 (s, 2H), 5.58 - 4.92 (m, 1H), 4.92 - 4.83 (m, 1H), 4.70 - 4.59 (m, 1H), 4.31 - 4.24 (m, 3H), 4.16 - 3.97 (m, 2H), 2.83 - 2.66 (m, 3H). |
| | | | SFC (column: CHIRALPAK IC 250mm × 20 mm, 5 µm; mobile phase: ETOH (NH₄OH 0.2%); B%: 40% EtOH, 19 min), RT = 2.85min. |
| 168: 168-P2 | 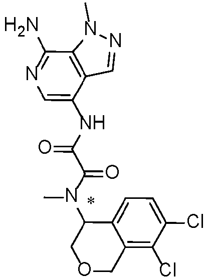 | 449.1 | ¹H NMR (400 MHz, DMSO-*d₆*) δ ppm 10.86 - 10.67 (m, 1H), 7.99 - 7.83 (m, 2H), 7.72 - 7.65 (m, 1H), 7.55 - 7.29 (m, 1H), 6.26 (s, 2H), 5.58 - 4.92 (m, 1H), 4.92 - 4.83 (m, 1H), 4.70 - 4.59 (m, 1H), 4.32 - 4.24 (m, 3H), 4.18 - 3.97 (m, 2H), 2.85 - 2.65 (m, 3H). |
| | | | SFC (column: CHIRALPAK IC 250mm × 20 mm, 5 µm; mobile phase: ETOH (NH₄OH 0.2%); B%: 40% EtOH, 19 min), RT = 4.75 min. |
| 169 | 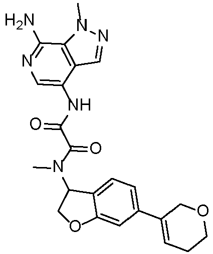 | 449.0 | ¹H NMR (400 MHz, DMSO-*d*₆, 80 °C) δ ppm 10.63 - 10.33 (m, 1H), 8.05 - 7.76 (m, 2H), 7.46 - 7.23 (m, 1H), 7.04 - 6.38 (m, 2H), 6.34 - 6.26 (m, 1H), 6.25 - 5.76 (m, 3H), 4.76 - 4.46 (m, 2H), 4.45 - 4.34 (m, 2H), 4.28 (s, 3H), 3.84 - 3.64 (m, 2H), 2.78 - 2.58 (m, 3H), 2.31 - 2.21 (m, 2H). |
| 170 | 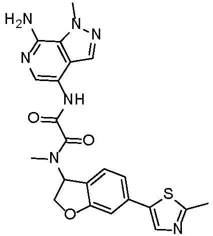 | 464.0 | ¹H NMR (400 MHz, DMSO-*d₆*) δ ppm 10.82 - 10.63 (m, 1H), 8.09 - 7.82 (m, 3H), 7.52 - 7.34 (m, 1H), 7.26 - 7.18 (m, 2H), 6.30 - 5.74 (m, 3H), 4.80 - 4.70 (m, 1H), 4.67 - 4.57 (m, 1H), 4.31 - 4.24 (m, 3H), 2.76 - 2.58 (m, 6H). |
| 171 | 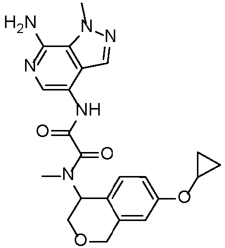 | 437.0 | ¹H NMR (400 MHz, DMSO-*d₆*) δ ppm 10.92 - 10.64 (m, 1H), 7.99 - 7.81 (m, 2H), 7.45 - 7.13 (m, 1H), 7.03 - 6.93 (m, 1H), 6.91 - 6.83 (m, 1H), 6.26 (s, 2H), 5.53 - 4.82 (m, 1H), 4.79 - 4.72 (m, 1H), 4.67 - 4.52 (m, 1H), 4.29 - 4.25 (m, 3H), 4.13 - 3.92 (m, 2H), 3.89 - 3.77 (m, 1H), 2.79 - 2.64 (m, 3H), 0.82 - 0.73 (m, 2H), 0.69 - 0.58 (m, 2H). |
| 172: 172-P1 | 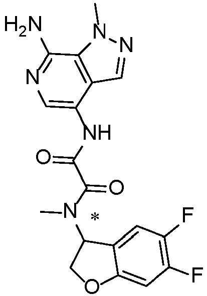 | 402.9 | ¹H NMR (400 MHz, DMSO-*d₆*) δ ppm 10.75 - 10.59 (m, 1H), 8.02 - 7.81 (m, 2H), 7.54 - 7.40 (m, 1H), 7.15 - 7.10 (m, 1H), 6.25 (s, 2H), 6.22 - 5.76 (m, 1H), 4.81 - 4.61 (m, 2H), 4.27 (s, 3H), 2.75 - 2.61 (m, 3H). |
| | | | SFC (column: CHIRALPAK AS-H 250mm × 20 mm, 5 µm; phase: 40%MeOH (NH₄OH 0.2%): 60%CO₂;), RT = 1.84 min. |
| 172: 172-P2 | 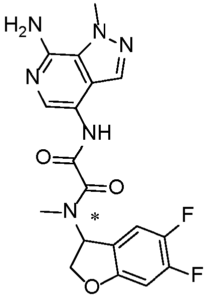 | 402.9 | ¹H NMR (400 MHz, DMSO-*d₆*) δ ppm 10.74 - 10.58 (m, 1H), 8.02 - 7.81 (m, 2H), 7.54 - 7.40 (m, 1H), 7.14 - 7.09 (m, 1H), 6.25 (s, 2H), 6.22 - 5.77 (m, 1H), 4.81 - 4.61 (m, 2H), 4.28 (s, 3H), 2.75 - 2.61 (m, 3H). |
| | | | SFC (column: CHIRALPAK AS-H 250mm × 20 mm, 5 µm; phase: 40%MeOH (NH₄OH 0.2%): 60%CO₂;), RT = 2.65 min. |
| 174: 174-P1 | 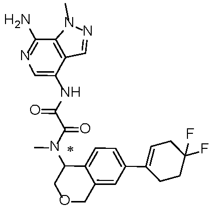 | 497.1 | ¹H NMR (400 MHz, DMSO-*d*₆, 80 °C) δ ppm 10.65 - 10.38 (m, 1H), 7.98 - 7.91 (m, 1H), 7.89 - 7.79 (m, 1H), 7.48 - 7.16 (m, 3H), 6.10 - 5.96 (m, 3H), 5.61 - 4.96 (m, 1H), 4.86 - 4.76 (m, 1H), 4.71 - 4.59 (m, 1H), 4.31 - 4.25 (m, 3H), 4.17 - 3.98 (m, 2H), 2.86 - 2.72 (m, 3H), 2.72 - 2.69 (m, 2H), 2.67 - 2.62 (m, 2H), 2.25 - 2.09 (m, 2H). |
| | | | SFC (column: CHIRALPAK AS-H 250mm × 20 mm, 5 µm; mobile phase: 40%IPA (0.2% NH₄OH)), RT = 3.82 min. |
| 174: 174-P2 | 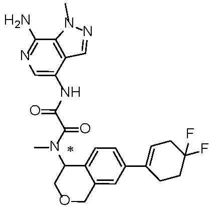 | 497.1 | ¹H NMR (400 MHz, DMSO-*d*₆, 80 °C) δ ppm 10.70 - 10.35 (m, 1H), 7.97 - 7.93 (m, 1H), 7.87 - 7.81 (m, 1H), 7.49 - 7.16 (m, 3H), 6.10 - 5.96 (m, 3H), 5.60 - 4.98 (m, 1H), 4.88 - 4.77 (m, 1H), 4.71 - 4.59 (m, 1H), 4.32 - 4.26 (m, 3H), 4.16 - 3.98 (m, 2H), 2.88 - 2.73 (m, 3H), 2.73 - 2.69 (m, 2H), 2.68 - 2.63 (m, 2H), 2.24 - 2.08 (m, 2H). |
| | | | SFC (column: CHIRALPAK AS-H 250mm × 20 mm, 5µm; mobile phase: 40%IPA (0.2% NH₄OH)), RT = 3.82 min. |
| 175 | 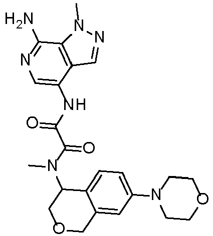 | 466.3 | ¹H (400 MHz, DMSO-*d₆*) δ 10.81 - 10.64 (m, 1H), 7.93 - 7.81 (m, 2H), 7.29 - 7.06 (m, 1H), 6.98 - 6.93 (m, 1H), 6.70 - 6.66 (m, 1H), 6.24 (s, 2H), 5.47 - 4.71 (m, 2H), 4.62 - 4.57 m, 1H), 4.27 - 4.26 (m, 3H), 4.11 - 3.93 (m, 2H), 3.73 - 3.70 (m, 4H), 3.13 - 3.09 (m, 4H), 2.77 - 2.67 (m, 3H). |
| 176 | 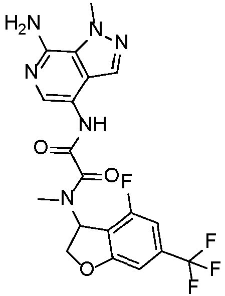 | 453.0 | ¹H NMR (400 MHz, DMSO-*d*₆, 80 °C) δ ppm 10.62 - 10.42 (m, 1H), 8.02 - 7.92 (m, 1H), 7.89 - 7.79 (m, 1H), 7.19 (d, *J* = 8.0 Hz, 1H), 7.14 (s, 1H), 6.41 - 6.28 (m, 1H), 6.13 (s, 2H), 4.95 - 4.85 (m, 1H), 4.79 - 4.69 (m, 1H), 4.28 (s, 3H), 2.92 - 2.68 (m, 3H). |
| 177 | 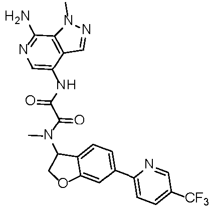 | 511.9 | ¹H NMR (400 MHz, DMSO-*d*₆, 80 °C) δ ppm 11.01 - 10.55 (m, 1H), 9.08 - 9.01 (m, 1H), 8.40 - 8.15 (m, 5H), 7.72 - 7.66 (m, 1H), 7.65 - 7.47 (m, 1H), 6.35 - 5.81 (m, 3H), 4.84 - 4.73 (m, 1H), 4.71 - 4.61 (m, 1H), 4.31 - 4.24 (m, 3H), 2.79 - 2.61 (m, 3H). |
| 178 | 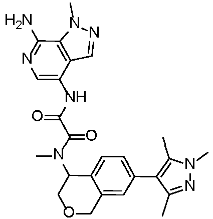 | 489.1 | ¹H NMR (400 MHz, DMSO-*d*₆, 80 °C) δ ppm 10.69 - 10.35 (m, 1H), 8.00 - 7.92 (m, 1H), 7.91 - 7.81 (m, 1H), 7.55 - 7.28 (m, 1H), 7.27 -7.14 (m, 1H), 7.09 - 6.99 (m, 1H), 6.15 - 5.95 (m, 2H), 5.67 - 4.99 (m, 1H), 4.91 - 4.79 (m, 1H), 4.75 - 4.63 (m, 1H), 4.31 - 4.24 (m, 3H), 4.19 - 4.01 (m, 2H), 3.70 (s, 3H), 2.95 - 2.68 (m, 3H), 2.25 - 2.16 (m, 3H), 2.15 - 2.08 (m, 3H). |
| 179 | 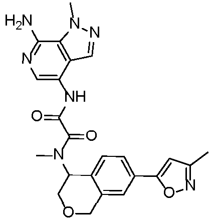 | 461.9 | ¹H NMR (400 MHz, DMSO-*d*₆, 80 °C) δ ppm 10.86 - 10.43 (m, 1H), 8.07 - 7.97 (m, 1H), 7.95 - 7.83 (m, 1H), 7.81 - 7.73 (m, 1H), 7.68 - 7.38 (m, 2H), 6.87 - 6.76 (m, 1H), 6.56 - 6.15 (m, 2H), 5.68 - 5.02 (m, 1H), 4.97 - 4.82 (m, 1H), 4.78 - 4.66 (m, 1H), 4.34 - 4.24 (m, 3H), 4.21 - 4.02 (m, 2H), 2.88 - 2.69 (m, 3H), 2.34 - 2.24 (m, 3H). |
| 180: 180-P1 | 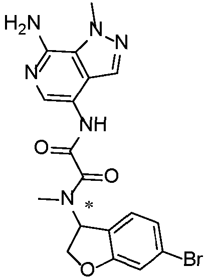 | 445.0, 447.0 | ¹H NMR (400 MHz, DMSO-*d₆*) δ ppm 10.79 - 10.64 (m, 1H), 8.03 - 7.83 (m, 2H), 7.42 - 7.27 (m, 1H), 7.20 - 7.16 (m, 2H), 6.31 - 5.73 (m, 3H), 4.79 - 4.71 (m, 1H), 4.65 - 4.58 (m, 1H), 4.28 (s, 3H), 2.72 -2.59 (m, 3H). |
| | | | SFC (column: CHIRALPAK AS-H 250mm × 20 mm, 5 µm; mobile phase: 40%MEOH (NH₄OH 0.2%): 60%CO₂), RT = 3.03 min. |
| 180: 180-P2 | 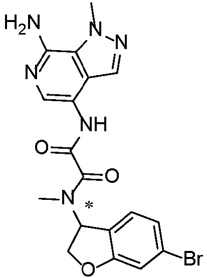 | 445.0, 447.0 | ¹H NMR (400 MHz, DMSO-*d₆*) δ ppm 10.79 - 10.64 (m, 1H), 8.02 - 7.83 (m, 2H), 7.43 - 7.27 (m, 1H), 7.20 - 7.16 (m, 2H), 6.26 (s, 2H), 6.22 - 5.73 (m, 1H), 4.79 - 4.71 (m, 1H), 4.65 - 4.58 (m, 1H), 4.27 (s, 3H), 2.72 - 2.58 (m, 3H). |
| | | | SFC (column: CHIRALPAK AS-H 250mm × 20 mm, 5 µm; mobile phase: 40%MEOH (NH₄OH 0.2%): 60%CO₂), RT = 3.03 min. |
| 181: 181-P1 | 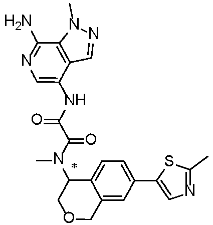 | 478.1 | ¹H NMR (400 MHz, DMSO-*d₆*) δ ppm 10.85 - 10.68 (m, 1H), 8.05 (s, 1H), 7.98 - 7.85 (m, 2H), 7.62 - 7.31 (m, 3H), 6.25 (s, 2H), 5.56 - 4.91 (m, 1H), 4.89 - 4.82 (m, 1H), 4.71 - 4.63 (m, 1H), 4.28 - 4.27 (m, 3H), 4.12 - 4.04 (m, 2H), 2.82 - 2.70 (m, 3H), 2.69 - 2.67 (m, 3H). |
| | | | SFC (column: CHIRALPAK OJ-H 250mm x 20 mm, 5 µm; mobile phase: 40% EtOH (NH₄OH 0.2%): 60%CO₂), RT = 8.80 min. |
| 181: 181-P2 | 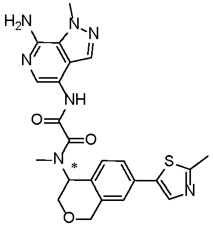 | 478.1 | ¹H NMR (400 MHz, DMSO-*d₆*) δ ppm 10.85 - 10.68 (m, 1H), 8.05 (s, 1H), 7.98 - 7.85 (m, 2H), 7.62 - 7.31 (m, 3H), 6.25 (s, 2H), 5.57 - 4.90 (m, 1H), 4.89 - 4.82 (m, 1H), 4.71 - 4.63 (m, 1H), 4.28 - 4.27 (m, 3H), 4.12 - 4.04 (m, 2H), 2.82 - 2.70 (m, 3H), 2.69 - 2.67 (m, 3H). |
| | | | SFC (column: CHIRALPAK OJ-H 250mm x 20 mm, 5µm; mobile phase: 40% EtOH (NH₄OH 0.2%): 60%CO₂), RT = 10.90 min. |
| 182: 182-P1 | 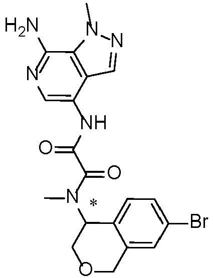 | 459.0, 461.0 | ¹H NMR (400 MHz, 353K, DMSO-*d₆*) δ ppm 10.56 - 10.39 (m, 1H), 7.90 - 7.89 (m, 1H), 7.84 - 7.77 (m, 1H), 7.49 -7.46 (m, 1H), 7.41 - 7.36 (m, 2H), 5.97 (s, 2H), 5.50 - 4.94 (m, 1H), 4.81 - 4.74 (m, 1H), 4.64 - 4.56 (m, 1H), 4.24 - 4.23 (m, 3H), 4.11 - 3.98 (m, 2H), 2.80 - 2.65 (m, 3H). |
| | | | SFC (column: CHIRALPAK IC 250mm x 20 mm, 5um; mobile phase: ETOH (NH₄OH 0.2%); B%: 40% EtOH, 19 min), RT = 3.96 min. |
| 182: 182-P2 | 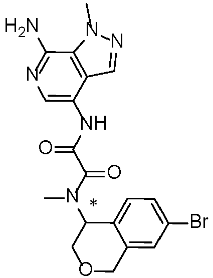 | 459.0, 461.0 | ¹H NMR (400 MHz, DMSO-*d*₆, 80 °C) δ ppm 10.56 - 10.39 (m, 1H), 7.90 - 7.89 (m, 1H), 7.84 - 7.77 (m, 1H), 7.49 -7.46 (m, 1H), 7.41 - 7.18 (m, 2H), 5.97 (s, 2H), 5.50 - 4.94 (m, 1H), 4.81 - 4.74 (m, 1H), 4.64 - 4.56 (m, 1H), 4.24 - 4.23 (m, 3H), 4.07 - 3.98 (m, 2H), 2.80 - 2.65 (m, 3H). |
| | | | SFC (column: CHIRALPAK IC 250mm x 20 mm, 5um; mobile phase: ETOH (NH₄OH 0.2%); B%: 40% EtOH, 19 min), RT = 5.17 min. |
| 184 | 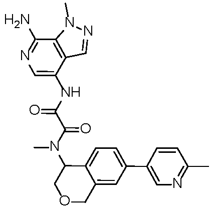 | 472.0 | ¹H NMR (400 MHz, DMSO-*d*₆, 80 °C) δ ppm 10.69 - 10.43 (m, 1H), 8.81- 8.71 (m, 1H), 8.02 - 7.93 (m, 2H), 7.91 - 7.81(m, 1H), 7.72 - 7.60 (m, 1H), 7.61 - 7.29 (m, 3H), 6.14 - 5.97 (m, 2H), 5.67 - 5.01 (m, 1H), 4.95 - 4.82 (m, 1H), 4.80 - 4.66 (m, 1H), 4.31 - 4.26 (m, 3H), 4.19 - 4.03 (m, 2H), 2.91 - 2.72 (m, 3H), 2.53 - 2.50 (m, 3H). |
| 185 | 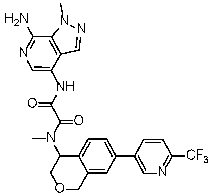 | 525.8 | ¹H NMR (400 MHz, DMSO-*d*₆, 80 °C) δ ppm 10.73 - 10.39 (m, 1H), 9.09 (s, 1H), 8.42 - 8.29 (m, 1H), 8.02 - 7.93 (m, 2H), 7.92 - 7.82 (m, 1H), 7.81 - 7.73 (m, 1H), 7.71 - 7.41 (m, 2H), 6.04 (s, 2H), 5.71 - 5.05 (m, 1H), 4.97 - 4.84 (m, 1H), 4.82 - 4.71 (m, 1H), 4.32 - 4.27 (m, 3H), 4.24 - 3.99 (m, 2H), 2.96 - 2.66 (m, 3H). |
| 186: 186-P1 | 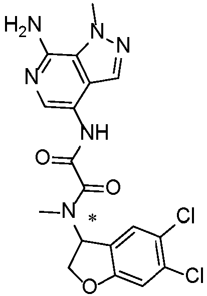 | 434.8 | ¹H NMR (400 MHz, DMSO-*d*₆, 80 °C) δ ppm 10.55 - 10.38 (m, 1H), 8.01 - 7.91 (m, 2H), 7.79 - 7.56 (m, 1H), 7.22 (s, 1H), 6.21 - 5.87 (m, 3H), 4.83 - 4.76 (m, 1H), 4.70 - 4.64 (m, 1H), 4.28 (s, 3H), 2.84 - 2.65 (m, 3H). |
| | | | SFC (column: CHIRALPAK AS-H 250mm x 20 mm, 5um; mobile phase: 40% MeOH (NH₄OH 0.2%); 60% CO₂,), RT = 3.83 min. |
| 186: 186-P2 | 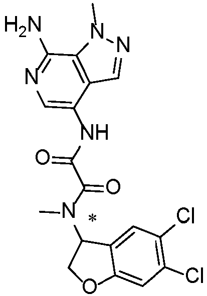 | 434.8 | ¹H NMR (400 MHz, DMSO-*d*₆, 80 °C) δ ppm 10.64 (s, 1H), 8.01 - 7.91 (m, 2H), 7.79 - 7.56 (m, 1H), 7.22 (s, 1H), 6.22 - 5.86 (m, 3H), 4.83 - 4.76 (m, 1H), 4.69 - 4.63 (m, 1H), 4.28 (s, 3H), 2.84 - 2.65 (m, 3H). |
| | | | SFC (column: CHIRALPAK AS-H 250mm x 20 mm, 5um; mobile phase: 40% MeOH (NH₄OH 0.2%); 60% CO₂,), RT = 5.18 min. |
| 187: 187-P1 | 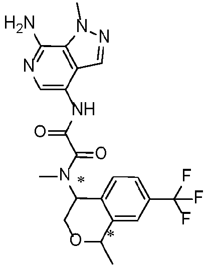 | 463.2 | ¹H NMR (400 MHz, DMSO-*d*₆, 80 °C) δ ppm 10.59 - 10.44 (m, 1H), 7.93 - 7.91 (m, 1H), 7.85 - 7.78 (m, 1H), 7.65 - 7.41 (m, 3H), 5.99 (s, 2H), 5.66 - 5.12 (m, 1H), 5.01 - 4.92 (m, 1H), 4.25 - 4.23 (m, 3H), 4.21 - 4.14 (m, 1H), 3.98 - 3.86 (m, 1H), 2.80 - 2.62 (m, 3H), 1.50 - 1.43 (m, 3H). |
| 187: 187-P2 | 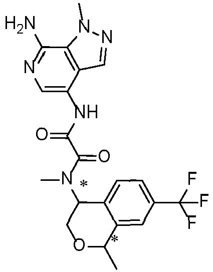 | 463.2 | ¹H NMR (400 MHz, DMSO-*d*₆, 80 °C) δ ppm 10.60 - 10.40 (m, 1H), 7.93 - 7.78 (m, 2H), 7.70 - 7.46 (m, 3H), 5.97 (s, 2H), 5.53 - 4.97 (m, 1H), 4.82 - 4.72 (m, 1H), 4.24 - 4.23 (m, 3H), 4.18 - 4.15 (m, 1H), 4.06 - 3.99 (m, 1H), 2.84 - 2.71 (m, 3H), 1.56 - 1.54 (m, 3H). |
| 188 | 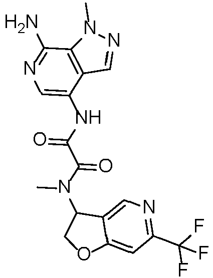 | 435.9 | ¹H NMR (400 MHz, DMSO-*d*₆, 80 °C) δ ppm 10.58 - 10.40 (m, 1H), 8.70 - 8.62 (m, 2H), 8.02 - 7.79 (m, 2H), 7.46 (s, 1H), 6.30 - 6.04 (m, 3H), 4.98 - 4.90 (m, 1H), 4.85 - 4.80 (m, 1H), 4.28 (s, 3H), 2.90 - 2.66 (m, 3H). |
| 189 | 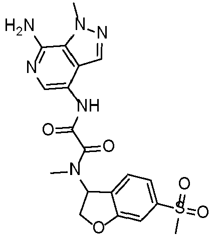 | 445.1 | ¹H NMR (400 MHz, DMSO-*d₆*) δ 10.81-10.67 (m, 1H), 8.22 - 8.03 (m, 1H), 7.93 - 7.83 (m, 1H), 7.72 - 7.54 (m, 2H), 7.43 - 7.32 (m, 1H), 6.32 - 5.90 (m, 3H), 4.89 - 4.76 (m, 1H), 4.75 -4.64 (m, 1H), 4.29 - 4.26 (m, 3H), 3.27 - 3.21 (m, 3H), 2.77 - 2.61 (m, 3H). |
| 190 | 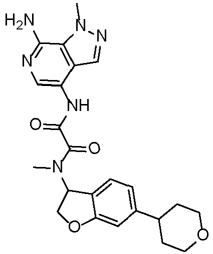 | 451.2 | ¹H NMR (400 MHz, DMSO-*d₆*) δ ppm 10.75 - 10.61 (m, 1H), 7.97 - 7.79 (m, 2H), 7.34 - 7.20 (m, 1H), 6.86 - 6.84 (m, 1H), 6.78 - 6.76 (m, 1H), 6.21 (s, 2H), 6.17 - 5.63 (m, 1H), 4.67 - 4.47 (m, 2H), 4.23 (s, 3H), 3.91 - 3.87 (m, 2H), 3.40 - 3.34 (m, 2H), 2.73 - 2.53 (m, 3H), 2.29 - 2.22 (m, 1H), 1.67 - 1.54 (m, 4H). |
| 191 | 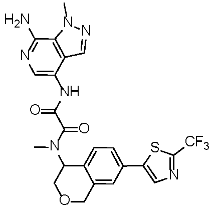 | 532.1 | ¹H NMR (400 MHz, DMSO-*d*₆, 80 °C) δ ppm 10.60 - 10.34 (m, 1H), 8.46 (s, 1H), 7.93 - 7.91 (m, 1H), 7.86 - 7.80 (m, 1H), 7.73 - 7.70 (m, 1H), 7.59 - 7.37 (m, 2H), 5.98 (s, 2H), 5.59 - 5.04 (m, 1H), 4.89 - 4.82 (m, 1H), 4.73 - 4.65 (m, 1H), 4.25 - 4.24 (m, 3H), 4.12 - 4.05 (m, 2H), 2.85 - 2.70 (m, 3H). |
| 192: 192-P1 | 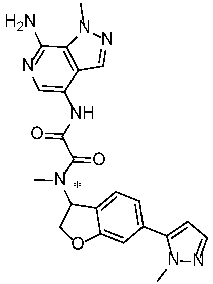 | 447.0 | ¹H NMR (400 MHz, DMSO-*d*₆, 80 °C) δ ppm 10.65 - 10.37 (m, 1H), 8.05 - 7.80 (m, 2H), 7.62 - 7.39 (m, 2H), 7.16 - 7.01 (m, 2H), 6.39 (s, 1H), 6.32 - 5.87 (m, 3H), 4.83 - 4.72 (m, 1H), 4.69 - 4.58 (m, 1H), 4.28 (s, 3H), 3.89 - 3.81 (m, 3H), 2.85 - 2.64 (m, 3H). |
| | | | SFC (column: CHIRALPAK IC 250mm × 20 mm, 5 µm; mobile phase: ETOH (NH₄OH 0.2%); B%: 40% EtOH, 18 min), RT = 2.98 min. |
| 192: 192-P2 | 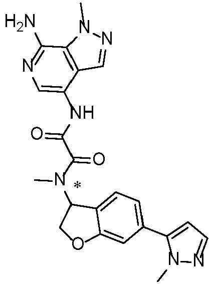 | 447.0 | ¹H NMR (400 MHz, DMSO-*d*₆, 80 °C) δ ppm 10.62 - 10.38 (m, 1H), 8.04 - 7.80 (m, 2H), 7.60 - 7.41 (m, 2H), 7.16 - 7.00 (m, 2H), 6.39 (s, 1H), 6.32 - 5.87 (m, 3H), 4.83 - 4.72 (m, 1H), 4.70 - 4.57 (m, 1H), 4.28 (m, 3H), 3.87 - 3.82 (m, 3H), 2.84 - 2.64 (m, 3H). |
| | | | SFC (column: CHIRALPAK IC 250mm × 20 mm, 5 µm; mobile phase: ETOH (NH₄OH 0.2%); B%: 40% EtOH, 18 min), RT = 4.56 min. |
| 193 | 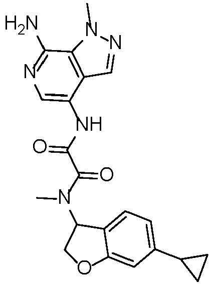 | 407.2 | ¹H NMR (400 MHz, DMSO-*d₆*) δ ppm 10.84 - 10.63 (m, 1H), 8.04 - 7.82 (m, 2H), 7.34 - 7.15 (m, 1H), 6.77 - 6.71 (m, 1H), 6.62 - 6.57 (m, 1H), 6.25 (s, 2H), 6.18 - 5.63 (m, 1H), 4.72 - 4.60 (m, 1H), 4.58 - 4.48 (m, 1H), 4.27 (s, 3H), 2.70 - 2.54 (m, 3H), 1.96 - 1.85 (m, 1H), 0.98 - 0.9 (m, 2H), 0.72 - 0.62 (m, 2H) |
| 194 | 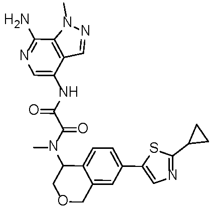 | 504.2 | ¹H NMR (400 MHz, DMSO-*d*₆, 80 °C) δ ppm 10.69 - 10.36 (m, 1H), 7.99 - 7.81 (m, 3H), 7.59 - 7.29 (m, 3H), 6.01 (s, 2H), 5.62 - 4.99 (m, 1H), 4.90 - 4.63 (m, 2H), 4.32 - 4.23 (m, 3H), 4.16 - 4.04 (m, 2H), 2.89 - 2.71 (m, 3H), 2.45 - 2.35 (m, 1H), 1.18 - 1.12 (m, 2H), 1.03 - 0.96 (m, 2H). |
| 195 | 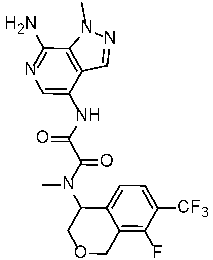 | 467.1 | ¹H NMR (400 MHz, DMSO-*d*₆, 80 °C) δ ppm 10.61 - 10.45 (m, 1H), 7.92 - 7.78 (m, 2H), 7.73 - 7.68 (m, 1H), 7.53 - 7.31 (m, 1H), 5.98 (s, 2H), 5.60 - 5.11 (m, 1H), 4.96 - 4.89 (m, 1H), 4.76 - 4.68 (m, 1H), 4.25 -4.24 (m, 3H), 4.12 - 4.05 (m, 2H), 2.86 - 2.69 (m, 3H). |
| 196 | 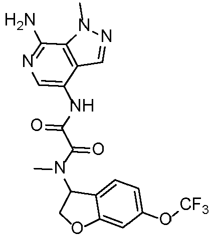 | 451.2 | ¹H NMR (400 MHz, DMSO-*d*₆, 80 °C) δ ppm 10.57 - 10.42 (m, 1H), 7.81 - 7.76 (m, 2H), 7.43 - 7.37 (m, 1H), 6.96 - 6.90 (m, 2H), 6.22 - 5.83 (m, 3H), 4.81 - 4.73 (m, 1H), 4.66 - 4.59 (m, 1H), 4.28 (s, 3H), 2.79 - 2.63 (m, 3H). |
| 197 | 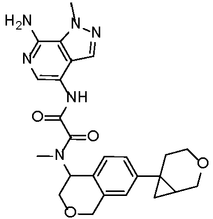 | 477.3 | ¹H NMR (400 MHz, DMSO-*d*₆, 80 °C) δ ppm 10.55 - 10.38 (m, 1H), 7.90 - 7.89 (m, 1H), 7.83 - 7.78 (m, 1H), 7.36 - 7.17 (m, 2H), 7.03 - 7.01 (m, 1H), 5.96 (s, 2H), 5.49 - 4.93 (m, 1H), 4.78 - 4.72 (m, 1H), 4.63 - 4.55 (m, 1H), 4.24 - 4.23 (m, 3H), 4.09 - 4.02 (m, 2H), 3.97 - 3.93 (m, 1H), 3.77 - 3.75 (m, 1H), 3.43 - 3.29 (m, 2H), 2.78 - 2.63 (m, 3H), 2.00 - 1.94 (m, 2H), 1.32 - 1.30 (m, 1H), 0.97 - 0.95 (m, 1H), 0.85 - 0.81 (m, 1H). |
| 198 | 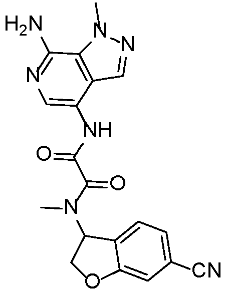 | 392.1 | ¹H NMR (400 MHz, DMSO-*d₆*) δ ppm 10.80 - 10.66 (m, 1H), 8.02 - 7.82 (m, 2H), 7.68 - 7.51 (m, 1H), 7.49 - 7.43 (m, 2H), 6.31 - 5.89 (m, 3H), 4.84 - 4.74 (m, 1H), 4.72 - 4.64 (m, 1H), 4.27 (s, 3H), 2.75 - 2.59 (m, 3H). |
| 199 | 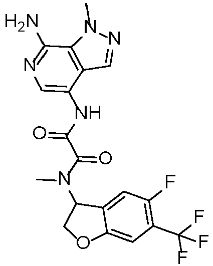 | 453.2 | ¹H NMR (400 MHz, DMSO-*d*₆, 80 °C) δ ppm 10.50 - 10.34 (m, 1H), 7.96 - 7.75 (m, 2H), 7.54 - 7.42 (m, 1H), 7.21 - 7.20 (m, 1H), 6.26 - 5.93 (m, 3H), 4.82 -4.75 (m, 1H), 4.69 - 4.62 (m, 1H), 4.25 (s, 3H), 2.83 - 2.63 (m, 3H). |
| 200 | 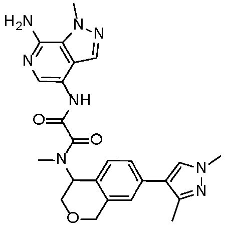 | 475.3 | ¹H NMR (400 MHz, DMSO-*d*₆, 80 °C) δ ppm 10.57 - 10.40 (m, 1H), 7.92 - 7.79 (m, 3H), 7.42 - 7.15 (m, 3H), 5.96 (s, 2H), 5.54 - 4.97 (m, 1H), 4.83 - 4.60 (m, 2H), 4.25 - 4.24 (m, 3H), 4.10 - 4.02 (m, 2H), 3.75 - 3.74 (m, 3H), 2.83 - 2.70 (m, 3H), 2.27 - 2.26 (m, 3H). |
| 201 | 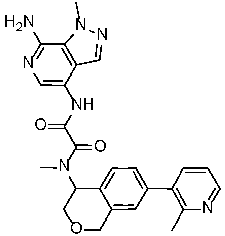 | 472.2 | ¹H NMR (400 MHz, DMSO-*d*₆, 80 °C) δ ppm 10.59 - 10.42 (m, 1H), 8.44 - 8.42 (m, 1H), 7.94 - 7.80 (m, 2H), 7.60 - 7.52 (m, 1H), 7.42 - 7.22 (m, 3H), 7.16 - 7.15 (m, 1H), 5.98 (s, 2H), 5.61 - 5.05 (m, 1H), 4.87 - 4.80 (m, 1H), 4.72 - 4.64 (m, 1H), 4.25 - 4.24 (m, 3H), 4.16 - 4.02 (m, 2H), 2.86 - 2.73 (m, 3H), 2.42 - 2.39 (m, 3H). |
| 202 | 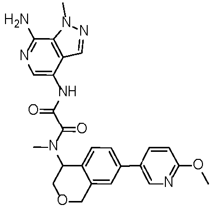 | 488.2 | ¹H NMR (400 MHz, DMSO-*d*₆, 80 °C) δ ppm 10.63 - 10.45 (m, 1H), 8.51 - 8.47 (m, 1H), 8.05 - 7.98 (m, 2H), 7.90 - 7.83 (m, 1H), 7.64 - 7.34 (m, 3H), 6.90 - 6.88 (m, 1H), 6.02 (s, 2H), 5.62 - 5.05 (m, 1H), 4.95 - 4.85 (m, 1H), 4.78 - 4.65 (m, 1H), 4.29 - 4.28 (m, 3H), 4.18 - 4.04 (m, 2H), 3.93 - 3.91 (m, 3H), 2.88 - 2.74 (m, 3H). |
| 203 | 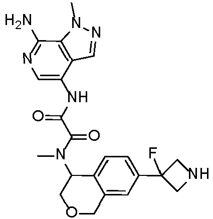 | 454.1 | ¹H NMR (400 MHz, DMSO-*d₆*) δ ppm 10.86 - 10.64 (m, 1H), 7.96 - 7.81 (m, 2H), 7.55 - 7.29 (m, 3H), 6.22 (s, 2H), 5.56 - 4.87 (m, 1H), 4.86 - 4.78 (m, 1H), 4.69 - 4.59 (m, 1H), 4.25 - 4.22 (m, 3H), 4.09 - 3.99 (m, 2H), 3.98 - 3.88 (m, 2H), 3.87 - 3.75 (m, 2H), 2.79 - 2.63 (m, 3H). |
| 204 | 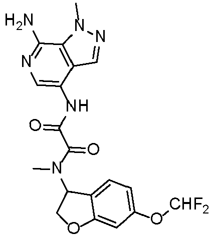 | 433.2 | ¹H NMR (400 MHz, DMSO-*d*₆, 80°C) δ ppm 10.61 - 10.34 (m, 1H), 8.02 - 7.78 (m, 2H), 7.51 - 6.96 (m, 2H), 6.82 - 6.71 (m, 2H), 6.25 (s, 2H), 6.22 - 5.80 (m, 1H), 4.83 - 4.71 (m, 1H), 4.69 - 4.56 (m, 1H), 4.28 (s, 3H), 2.81 - 2.58 (m, 3H). |
| 205 | 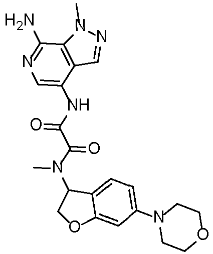 | 452.1 | ¹H NMR (400 MHz, DMSO-*d₆*) δ ppm 10.82 - 10.58 (m, 1H), 8.03 - 7.82 (m, 2H), 7.29 - 7.12 (m, 1H), 6.61 - 6.55 (m, 1H), 6.50 - 6.44 (m, 1H), 6.23 (s, 2H), 6.14 - 5.56 (m, 1H), 4.71 - 4.60 (m, 1H), 4.57 - 4.47 (m, 1H), 4.27 (s, 3H), 3.77 - 3.66 (m, 4H), 3.15 - 3.05 (m, 4H), 2.69 - 2.55 (m, 3H). |
| 206 | 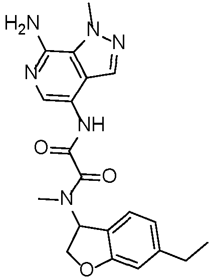 | 395.2 | ¹H NMR (400 MHz, DMSO-*d*₆, 80 °C) δ ppm 10.76 - 10.61 (m, 1H), 8.15 - 8.08 (m, 1H), 7.96 - 7.87 (m, 1H), 7.30 - 7.16 (m, 1H), 6.80 - 6.78 (m, 2H), 6.18 - 5.67 (m, 1H), 4.68 - 4.46 (m, 2H), 4.29 (s, 3H), 2.69 - 2.56 (m, 5H), 1.18 - 1.13 (m, 3H). |
| 207 | 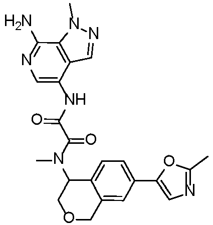 | 462.2 | ¹H NMR (400 MHz, DMSO-*d*₆, 80 °C) δ ppm 10.62 - 10.44 (m, 1H), 7.96 - 7.94 (m, 1H), 7.90 - 7.83 (m, 1H), 7.62 - 7.60 (m, 1H), 7.55 - 7.36 (m, 3H), 6.01 (s, 2H), 5.59 - 5.03 (m, 1H), 4.90 - 4.83 (m, 1H), 4.75 - 4.65 (m, 1H), 4.29 - 4.26 (m, 3H), 4.15 - 4.03 (m, 2H), 2.86 - 2.72 (m, 3H), 2.48 - 2.47 (m, 3H). |
| 208 | 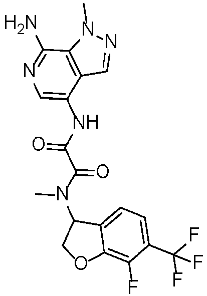 | 452.9 | ¹H NMR (400 MHz, DMSO-*d₆*) δ ppm 10.84 - 10.67 (m, 1H), 8.05 - 7.83 (m, 2H), 7.51 - 7.37 (m, 2H), 6.39 (s, 2H), 6.36 - 5.97 (m, 1H), 4.97 - 4.88 (m, 1H), 4.84 - 4.78 (m, 1H), 4.28 (s, 3H), 2.81 - 2.64 (m, 3H). |
| 209: 209-P1 | 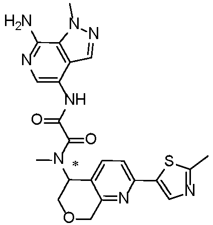 | 479.1 | ¹H NMR (400 MHz, DMSO-*d*₆, 80°C) δ ppm 10.65 - 10.42 (m, 1H), 8.28 (s, 1H), 7.99 - 7.71 (m, 4H), 6.02 (s, 2H), 5.67 - 5.09 (m, 1H), 4.84 - 4.59 (m, 2H), 4.32 - 4.25 (m, 3H), 4.18 - 4.08 (m, 2H), 2.92 - 2.73 (m, 3H), 2.68 (s, 3H). |
| | | | SFC (column: CHIRALPAK AS-H 250mm × 20 mm, 5 µm; mobile phase: 40%IPA (0.2% NH₄OH)), RT = 14.11 min. |
| 209: 209-P2 | 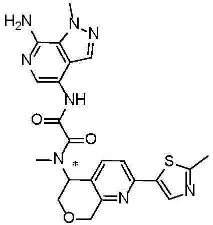 | 479.2 | ¹H NMR (400 MHz, DMSO-*d*₆, 80°C) δ ppm 10.68 - 10.46 (m, 1H), 8.28 (s, 1H), 8.03 - 7.71 (m, 4H), 6.20 (s, 2H), 5.67 - 5.09 (m, 1H), 4.84 - 4.59 (m, 2H), 4.32 - 4.26 (m, 3H), 4.18 - 4.09 (m, 2H), 2.92 - 2.73 (m, 3H), 2.68 (s, 3H). |
| | | | SFC (column: CHIRALPAK AS-H 250mm × 20 mm, 5 µm; mobile phase: 40%IPA (0.2% NH₄OH)), RT = 14.11 min. |
| 210 | 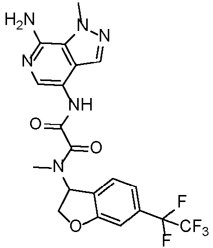 | 485.0 | ¹H NMR (400 MHz, DMSO-*d*₆, 80°C) δ ppm 10.62 - 10.38 (m, 1H), 8.05 - 7.78 (m, 2H), 7.75 - 7.55 (m, 1H), 7.35 - 7.27 (m, 1H), 7.17 (s, 1H), 6.38 - 5.94 (m, 3H), 4.88 - 4.77 (m, 1H), 4.73 - 4.63 (m, 1H), 4.28 (s, 3H), 2.85 - 2.58 (m, 3H). |
| 214 | 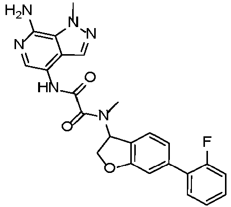 | 461.2 | ¹H NMR (400 MHz, DMSO-*d₆*) δ ppm 10.84 - 10.66 (m, 1H), 8.06 - 7.83 (m, 2H), 7.59 - 7.39 (m, 3H), 7.35 - 7.26 (m, 2H), 7.20 - 7.15 (m, 1H), 7.10 - 7.05 (m, 1H), 6.33 - 5.78 (m, 3H), 4.82 - 4.71 (m, 1H), 4.69 - 4.58 (m, 1H), 4.28 (s, 3H), 2.79 - 2.61 (m, 3H). |
| 215 | 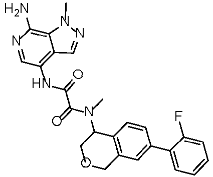 | 475.2 | ¹H NMR (400 MHz, DMSO-*d₆*) δ ppm 10.90 - 10.70 (m, 1H), 8.01 - 7.84 (m, 2H), 7.62 - 7.28 (m, 7H), 6.26 (s, 2H), 5.65 - 4.94 (m, 1H), 4.93 - 4.83 (m, 1H), 4.77 - 4.65 (m, 1H), 4.31- 4.24 (m, 3H), 4.20 - 3.98 (m, 2H), 2.87 - 2.71 (m, 3H). |
| 216 | 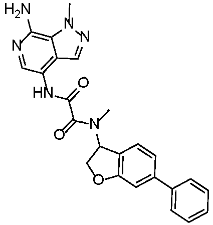 | 443.1 | ¹H NMR (400 MHz, DMSO-*d*₆, 80 °C) δ ppm 10.83 - 10.68 (m, 1H), 8.23 - 8.14 (m, 1H), 8.02 - 7.93 (m, 1H), 7.68 - 7.62 (m, 2H), 7.54 - 7.14 (m, 8H), 6.27 - 5.84 (m, 1H), 4.81 - 4.60 (m 2H), 4.34 (s, 3H), 2.80 - 2.68 (m, 3H). |
| 217 | 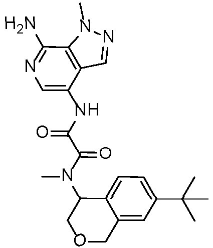 | 437.3 | ¹H NMR (400 MHz, DMSO-*d*₆, 80 °C) δ ppm 10.75 - 10.36 (m, 1H), 8.03 - 7.79 (m, 2H), 7.50 - 7.13 (m, 3H), 6.03 (s, 2H), 5.59 - 4.95 (m, 1H), 4.88 - 4.76 (m, 1H), 4.73 - 4.58 (m, 1H), 4.37 - 4.21 (m, 3H), 4.16 - 3.98 (m, 2H), 2.87 - 2.65 (m, 3H), 1.35 - 1.27 (m, 9H). |
| 218 | 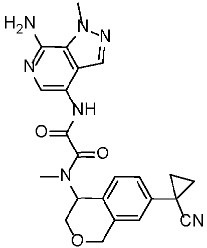 | 446.2 | ¹H NMR (400 MHz, DMSO-*d*₆, 80 °C) δ ppm 10.62 - 10.40 (m, 1H), 7.99 - 7.82 (m, 2H), 7.50 - 7.13 (m, 3H), 6.01 (s, 2H), 5.58 - 4.98 (m, 1H), 4.87 - 4.60 (m, 2H), 4.31 - 4.25 (m, 3H), 4.12 - 3.99 (m, 2H), 2.85 - 2.68 (m, 3H), 1.77 - 1.67 (m, 2H), 1.54 - 1.47 (m, 2H). |
| 219 | 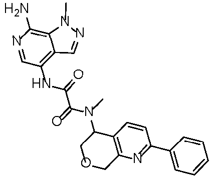 | 458.1 | ¹H NMR (400 MHz, DMSO-*d₆*) δ ppm 10.87 - 10.67 (m, 1H), 8.12 - 8.05 (m, 2H), 8.02 - 7.93 (m, 3H), 7.87 - 7.75 (m, 1H), 7.54 - 7.39 (m, 3H), 6.25 (s, 2H), 5.70 - 5.02 (m, 1H), 4.92 - 4.82 (m, 1H), 4.78 - 4.65 (m, 1H), 4.31 - 4.25 (m, 3H), 4.21 - 4.07 (m, 2H), 2.90 - 2.71 (m, 3H). |
| 220 | 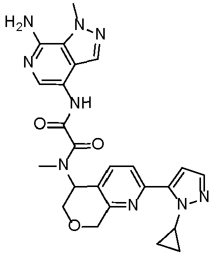 | 488.1 | ¹H NMR (400 MHz, DMSO-*d₆*) δ ppm 10.87 - 10.67 (m, 1H), 8.02 - 7.93 (m, 2H), 7.87 - 7.77 (m, 2H), 7.47 - 7.43 (m, 1H), 6.79 - 6.76 (m, 1H), 6.25 (s, 2H), 5.70 - 5.03 (m, 1H), 4.91 - 4.81 (m, 1H), 4.78 - 4.65 (m, 1H), 4.45 - 4.35 (m, 1H), 4.31 - 4.24 (m, 3H), 4.22 - 4.08 (m, 2H), 2.90 - 2.71 (m, 3H), 1.12 - 0.88 (m, 4H). |
| 221: 221-P1 | 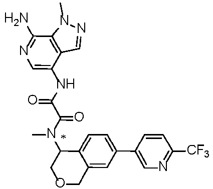 | 526.0 | ¹H NMR (400 MHz, DMSO-*d*₆, 80 °C) δ ppm 10.69 - 10.42 (m, 1H), 9.09 (s, 1H), 8.41 - 8.31 (m, 1H), 8.02 - 7.92 (m, 2H), 7.91 - 7.82 (m, 1H), 7.81 - 7.73 (m, 1H), 7.71 - 7.41 (m, 2H), 6.05 (s, 2H), 5.72 - 5.05 (m, 1H), 4.98 - 4.83 (m, 1H), 4.82 - 4.71 (m, 1H), 4.31 - 4.28 (m, 3H), 4.21 - 4.01 (m, 2H), 2.94 - 2.71 (m, 3H). |
| | | | SFC (column: CHIRALPAK AS-H 250mm × 20 mm, 5µm; mobile phase: 40%IPA (0.2% NH₄OH)), RT = 2.71 min. |
| 221: 221-P2 | 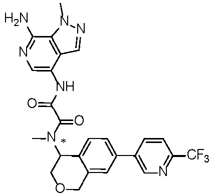 | 526.0 | ¹H NMR (400 MHz, DMSO-*d*₆, 80 °C) δ ppm 10.78 - 10.46 (m, 1H), 9.17 - 9.06 (m, 1H), 8.45 - 8.31 (m, 1H), 8.05 - 7.91 (m, 2H), 7.90 - 7.83 (m, 1H), 7.82 - 7.71 (m, 1H), 7.70 - 7.43 (m, 2H), 6.19 (s, 2H), 5.69 - 5.04 (m, 1H), 4.96 - 4.82 (m, 1H), 4.81 - 4.71 (m, 1H), 4.36 - 4.25 (m, 3H), 4.22 - 4.03 (m, 2H), 2.93 - 2.72 (m, 3H). |
| | | | SFC (column: CHIRALPAK AS-H 250mm × 20 mm, 5µm; mobile phase: 40%IPA (0.2% NH₄OH)), RT = 4.28 min. |
| 222 | 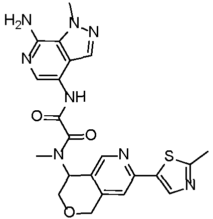 | 479.1 | ¹H NMR (400 MHz, DMSO-*d₆*) δ ppm 10.89 - 10.71 (m, 1H), 8.61 - 8.46 (m, 1H), 8.28 - 8.27 (m, 1H), 8.04 - 7.85 (m, 2H), 7.81 - 7.79 (m, 1H), 6.51 (s, 2H), 5.62 - 5.01 (m, 1H), 4.91 - 4.84 (m, 1H), 4.73 - 4.64 (m, 1H), 4.30 - 4.28 (m, 3H), 4.18 - 4.04 (m, 2H), 2.86 - 2.74 (m, 3H), 2.68 - 2.67 (m, 3H). |
| 223: 223-P1 | 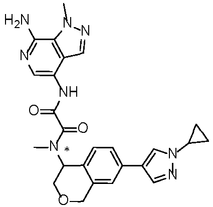 | 487.0 | ¹H NMR (400 MHz, DMSO-*d*₆, 80 °C) δ ppm 10.63 - 10.46 (m, 1H), 7.97 - 7.83 (m, 2H), 7.60 - 7.41 (m, 4H), 6.46 - 6.38 (m, 1H), 6.03 (s, 2H), 5.64 - 5.08 (m, 1H), 4.93 - 4.86 (m, 1H), 4.77 - 4.69 (m, 1H), 4.29 - 4.28 (m, 3H), 4.19 - 4.10 (m, 2H), 3.76 - 3.66 (m, 1H), 2.90 - 2.76 (m, 3H), 1.02 - 0.93 (m, 4H). |
| | | | SFC (column: CHIRALPAK IC 250 mm x 20 mm, 5um; mobile phase: ETOH (NH₄OH 0.2%); B%: 40% EtOH, 19 min), RT = 3.87 min. |
| 223: 223-P2 | 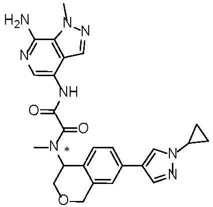 | 487.0 | ¹H NMR (400 MHz, DMSO-*d*₆, 80 °C) δ ppm 10.62 - 10.45 (m, 1H), 7.97 - 7.83 (m, 2H), 7.60 - 7.39 (m, 4H), 6.40 - 6.37 (m, 1H), 6.02 (s, 2H), 5.64 - 5.08 (m, 1H), 4.92 - 4.86 (m, 1H), 4.79 - 4.66 (m, 1H), 4.31 - 4.23 (m, 3H), 4.20 - 4.06 (m, 2H), 3.74 - 3.68 (m, 1H), 2.89 - 2.75 (m, 3H), 1.03 - 0.93 (m, 4H). |
| | | | SFC (column: CHIRALPAK IC 250 mm x 20 mm, 5um; mobile phase: ETOH (NH₄OH 0.2%); B%: 40% EtOH, 19 min), RT = 5.86 min. |
| 225 | 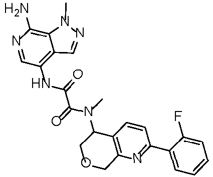 | 476.2 | ¹H NMR (400 MHz, DMSO-*d₆*) δ ppm 10.84 - 10.68 (m, 1H), 8.01 - 7.99 (m, 1H), 7.95 - 7.94 (m, 1H), 7.92 - 7.88 (m, 1H), 7.84 - 7.79 (m, 2H), 7.52 - 7.48 (m, 1H), 7.37 - 7.32 (m, 2H), 6.24 (s, 2H), 5.70 - 5.05 (m, 1H), 4.91 - 4.83 (m, 1H), 4.77 - 4.68 (m, 1H), 4.28 - 4.27 (m, 3H), 4.21 - 4.12 (m, 2H), 2.89 - 2.76 (m, 3H). |
| 226 | 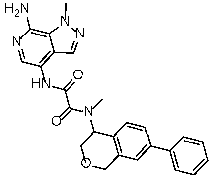 | 457.0 | ¹H NMR (400 MHz, DMSO-*d₆*) δ ppm 10.85 - 10.68 (m, 1H), 7.99 - 7.85 (m, 2H), 7.70 - 7.56 (m, 4H), 7.50 - 7.45 (m, 3H), 7.39 - 7.35 (m, 1H), 6.24 (s, 2H), 5.60 - 4.96 (m, 1H), 4.90 - 4.87 (m, 1H), 4.76 - 4.68 (m, 1H), 4.28 - 4.27(m, 3H), 4.15 - 4.07 (m, 2H), 2.84 - 2.73 (m, 3H). |
| 227 | 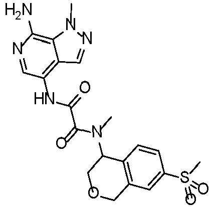 | 459.2 | ¹H NMR (400 MHz, DMSO-*d*₆, 80 °C) δ ppm 10.60 - 10.42 (m, 1H), 7.99 - 7.47 (m, 5H), 5.98 (s, 2H), 5.61- 5.10 (m, 1H), 4.95 - 4.66 (m, 2H), 4.27 - 4.25 (m, 3H), 4.15 - 4.04 (m, 2H), 3.16 - 3.15 (m, 3H), 2.84 - 2.68 (m, 3H). |
| 228 | 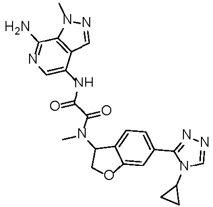 | 474.2 | ¹H NMR (400 MHz, DMSO-*d₆*) δ ppm 10.83 - 10.66 (m, 1H), 8.62 - 8.60 (m, 1H), 8.06 - 7.83 (m, 2H), 7.65 - 7.40 (m, 3H), 6.35 - 5.84 (m, 3H), 4.86 - 4.62 (m, 2H), 4.28 (s, 3H), 3.70 - 3.62 (m, 1H), 2.80 - 2.62 (m, 3H), 1.04 - 0.95 (m, 2H), 0.93 - 0.82 (m, 2H). |
| 229 | 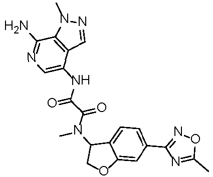 | 449.0 | ¹H NMR (400 MHz, DMSO-*d₆*) δ ppm 10.83 - 10.65 (m, 1H), 8.05 - 7.82 (m, 2H), 7.68 - 7.42 (m, 3H), 6.34 - 5.83 (m, 3H), 4.84 - 4.75 (m, 1H), 4.71 - 4.62 (m, 1H), 4.28 (s, 3H), 2.77 - 2.60 (m, 6H). |
| 230 | 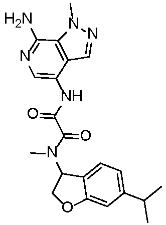 | 409.0 | ¹H NMR (400 MHz, DMSO-*d*₆, 80°C) δ ppm 10.81 - 10.46 (m, 1H), 8.18 - 8.02 (m, 1H), 7.99 - 7.81 (m, 1H), 7.41 - 7.17 (m, 1H), 6.92 - 6.82 (m, 1H), 6.81 - 6.73 (m, 1H), 6.63 (s, 2H), 6.26 - 5.68 (m, 1H), 4.77 - 4.43 (m, 2H), 4.31 (s, 3H), 2.96 - 2.83 (m, 1H), 2.78 - 2.56 (m, 3H), 1.27 - 1.21 (m, 3H), 1.21 - 1.13 (m, 3H). |
| 231: 231-P1 | 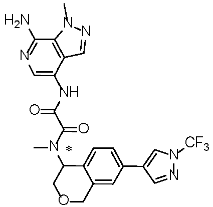 | 515.3 | ¹H NMR (400 MHz, DMSO-*d*₆, 80 °C) δ ppm 10.63 - 10.45 (m, 1H), 8.87 (s, 1H), 8.40 (s, 1H), 7.97 - 7.83 (m, 2H), 7.70 - 7.67 (m, 1H), 7.56 - 7.33 (m, 2H), 6.01 (s, 2H), 5.61 - 5.02 (m, 1H), 4.88 - 4.82 (m, 1H), 4.74 - 4.66 (m, 1H), 4.29 - 4.27 (m, 3H), 4.15 - 4.07 (m, 2H), 2.87 - 2.73 (m, 3H). |
| | | | SFC (column: CHIRALPAK AS-H 250 mm x 20 mm, 5um; mobile phase: MEOH (NH₄OH 0.2%); B%: 60% MeOH, 19 min), RT = 2.50 min. |
| 231: 231-P2 | 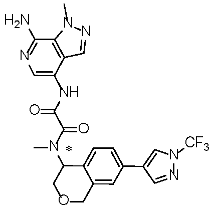 | 515.2 | ¹H NMR (400 MHz, DMSO-*d*₆, 80 °C) δ ppm 10.63 - 10.45 (m, 1H), 8.87 (s, 1H), 8.40 (s, 1H), 7.97 - 7.83 (m, 2H), 7.70 - 7.68 (m, 1H), 7.56 - 7.32 (m, 2H), 6.02 (s, 2H), 5.61 - 5.01 (m, 1H), 4.89 - 4.83 (m, 1H), 4.74 - 4.66 (m, 1H), 4.29 - 4.28 (m, 3H), 4.15 - 4.07 (m, 2H), 2.87 - 2.73 (m, 3H). |
| | | | SFC (column: CHIRALPAK AS-H 250 mm x 20 mm, 5um; mobile phase: MEOH (NH₄OH 0.2%); B%: 60% MeOH, 19 min), RT = 4.27 min. |
| 232 | 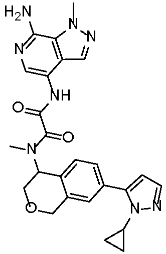 | 487.3 | ¹H NMR (400 MHz, DMSO-*d*₆, 80 °C) δ ppm 10.62 - 10.45 (m, 1H), 7.96 - 7.83 (m, 2H), 7.66 - 7.38 (m, 4H), 6.44 - 6.39 (m, 1H), 6.02 (s, 2H), 5.70 - 5.06 (m, 1H), 4.88 - 4.83 (m, 1H), 4.76 - 4.69 (m, 1H), 4.28- 4.27 (m, 3H), 4.21 - 4.07 (m, 2H), 3.77 - 3.64 (m, 1H), 2.89 - 2.75 (m, 3H), 1.04 - 0.93 (m, 4H). |
| 234 | 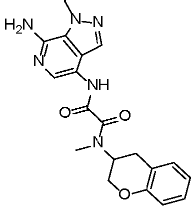 | 381.2 | ¹H NMR (400 MHz, DMSO-*d₆*) δ ppm 10.72 - 10.59 (m, 1H), 7.96 - 7.91 (m, 1H), 7.88 - 7.85 (m, 1H), 7.18 - 7.06 (m, 2H), 6.93 - 6.76 (m, 2H), 6.29 - 6.21 (m, 2H), 4.72 - 4.28 (m, 1H), 4.28 - 4.15 (m, 5H), 3.28 - 3.12 (m, 1H), 3.02 - 2.89 (m, 4H). |
| 237 | 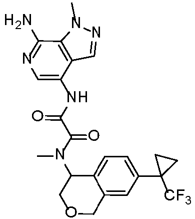 | 489.3 | ¹H NMR (400 MHz, DMSO-*d*₆, 80 °C) δ 10.58 - 10.42 (m, 1H), 7.91 - 7.78 (m, 2H), 7.46 - 7.22 (m, 3H), 5.97 (s, 2H), 5.53 - 4.98 (m, 1H), 4.82 - 4.58 (m, 2H), 4.25 - 4.23 (m, 3H), 4.13 - 3.97 (m, 2H), 2.84 - 2.70 (m, 3H), 1.36 - 1.33 (m, 2H), 1.16 - 1.11 (m, 2H). |
| 238 | 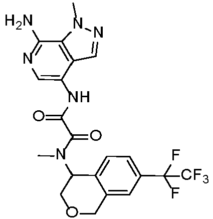 | 499.0 | ¹H NMR (400 MHz, DMSO-*d*₆, 80°C) δ ppm 10.64 - 10.47 (m, 1H), 7.96 - 7.94 (m, 1H), 7.89 - 7.82 (m, 1H), 7.75 - 7.54 (m, 3H), 6.03 (s, 2H), 5.65 -5.13 (m, 1H), 4.96 - 4.89 (m, 1H), 4.79 - 4.71 (m, 1H), 4.29 - 4.27 (m, 3H), 4.16 - 4.09 (m, 2H), 2.08 - 2.72 (m, 3H). |
| 239 | 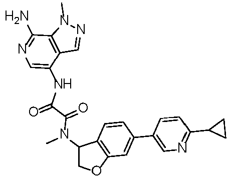 | 484.0 | ¹H NMR (400 MHz, DMSO-*d*₆, 80°C) δ ppm 10.59 - 10.43 (m, 1H), 8.72 - 8.67 (m, 1H), 8.02 - 7.82 (m, 3H), 7.55 - 7.41 (m, 1H), 7.33 - 7.27 (m, 2H), 7.21 - 7.19 (m, 1H), 6.28 - 5.87 (m, 3H), 4.79 - 4.72 (m, 1H), 4.65 - 4.58 (m, 1H), 4.28 (s, 3H), 2.80 - 2.65 (m, 3H), 2.17 - 2.11 (m, 1H), 0.98 - 0.96 (m, 4H). |
| 240 | 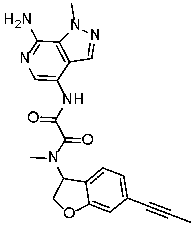 | 405.0 | ¹H NMR (400 MHz, DMSO-*d*₆, 80°C) δ ppm 10.55 - 10.40 (m, 1H), 8.00 - 7.81 (m, 2H), 7.41 - 7.27 (m, 1H), 7.01 - 6.87 (m, 2H), 6.23 - 5.81 (m, 3H), 4.75 - 4.53 (m, 2H), 4.28 (s, 3H), 2.76 - 2.60 (m, 3H), 2.03 (s, 3H). |
| 241: 241-P1 | 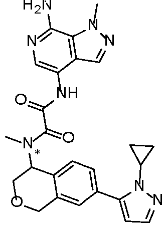 | 487.0 | ¹H NMR (400 MHz, DMSO-*d*₆, 80 °C) δ ppm 10.63 - 10.45 (m, 1H), 7.96 - 7.95 (m, 1H), 7.90 - 7.83 (m, 1H), 7.60 - 7.39 (m, 4H), 6.42 - 6.40 (m, 1H), 6.02 (s, 2H), 5.65 - 5.08 (m, 1H), 4.92 - 4.86 (m, 1H), 4.77 - 4.69 (m, 1H), 4.31 - 4.27 (m, 3H), 4.18 - 4.10 (m, 2H), 3.73 - 3.69 (m, 1H), 2.89 - 2.75 (m, 3H), 1.02 - 0.94 (m, 4H). |
| | | | SFC (column: CHIRALPAK IC 250mm x 20 mm, 5um; mobile phase: ETOH (NH₄OH 0.2%); B%: 40% EtOH,19 min), r.t. = 3.24 min. |
| 241: 241-P2 | 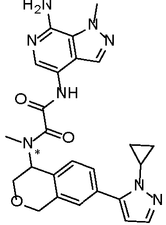 | 487.0 | ¹H NMR (400 MHz, DMSO-*d*₆, 80 °C) δ ppm 10.63 - 10.46 (m, 1H), 7.97 - 7.95 (m, 1H), 7.90 - 7.83 (m, 1H), 7.60 - 7.39 (m, 4H), 6.41 - 6.40 (m, 1H), 6.02 (s, 2H), 5.65 - 5.08 (m, 1H), 4.92 - 4.86 (m, 1H), 4.77 - 4.69 (m, 1H), 4.31 - 4.27 (m, 3H), 4.18 - 4.10 (m, 2H), 3.73 - 3.69 (m, 1H), 2.89 - 2.75 (m, 3H), 1.02 - 0.94 (m, 4H). |
| | | | SFC (column: CHIRALPAK IC 250mm x 20 mm, 5um; mobile phase: ETOH (NH₄OH 0.2%); B%: 40% EtOH,19 min), r.t. = 3.46 min. |
| 242 | 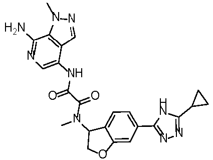 | 474.2 | ¹H NMR (400 MHz, DMSO-*d₆*) δ ppm 10.83 - 10.66 (m, 1H), 8.05 - 7.82 (m, 2H), 7.63 - 7.37 (m, 3H), 6.30 - 5.76 (m, 3H), 4.79 - 4.70 (m, 1H), 4.66 - 4.57 (m, 1H), 4.27 (s, 3H), 2.74 - 2.58 (m, 3H), 2.10 - 2.01 (m, 1H), 1.05 - 0.98 (m, 2H), 0.96 - 0.90 (m, 2H). |
| 243 | 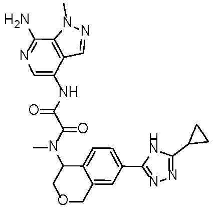 | 488.2 | ¹H NMR (400 MHz, DMSO-*d₆*) δ ppm 10.87 - 10.71 (m, 1H), 8.42 (m, 1H), 7.98 - 7.84 (m, 3H), 7.74 - 7.72 (m, 1H), 7.54 - 7.32 (m, 1H),6.24 (s, 2H), 5.57 - 4.92 (m, 1H), 4.88 - 4.85 (m, 1H), 4.73 - 4.69 (m, 1H), 4.27 - 4.26 (m, 3H), 4.12 - 4.08 (m, 2H), 2.81 - 2.66 (m, 3H), 2.07 - 2.06 (m, 1H), 1.02 - 1.00 (m, 2H), 0.94 - 0.92 (m, 2H). |
| 244 | 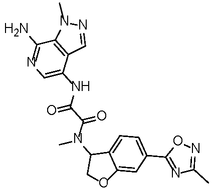 | 449.2 | ¹H NMR (400 MHz, DMSO-*d₆*) δ ppm 10.81 - 10.66 (m, 1H), 8.04 - 7.83 (m, 2H), 7.77 - 7.54 (m, 3H), 6.35 - 5.89 (m, 3H), 4.86 - 4.78 (m, 1H), 4.73 - 4.66 (m, 1H), 4.27 (s, 3H), 2.77 - 2.62 (m, 3H), 2.42 - 2.41 (m, 3H). |
| 245 | 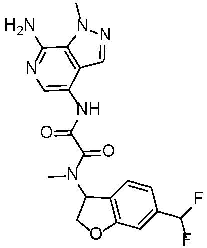 | 417.2 | ¹H NMR (400 MHz, DMSO-*d₆*) δ ppm 10.83 - 10.67 (m, 1H), 8.05 - 7.81 (m, 2H), 7.63 - 7.44 (m, 1H), 7.23 - 7.08 (m, 2H), 7.02 - 6.86 (m, 1H), 6.33 - 5.79 (m, 3H), 4.81 - 4.72 (m, 1H), 4.69 - 4.60 (m, 1H), 4.27 (s, 3H), 2.74 - 2.57 (m, 3H). |

### Assay example 1: Method of anti-proliferation assay on cells

### Materials and cells

HCT116^{MTAP Deletion} cells were from Pharmaron (China); HCT116 cells were from ATCC; McCoy's 5A medium was purchased from Invitrogen (USA); penicillin-streptomycin and fetal bovine serum were purchased from Gibco (USA); a 384-well plate was purchased from PerkinElmer (USA); and Cell-Titer Glo reagent was purchased from Promega (USA).

### Storage of compound

Compound dissolution: All the compounds were dissolved in dimethyl sulfoxide (DMSO) and then stored. The compounds were stored at room temperature in a dry condition for 3 months for short term storage or at -20 °C for long term storage.

### Cell culture and passaging

The cells were cultured strictly in accordance with ATCC requirements. The cell culture medium (McCoy's 5A medium + 10% fetal bovine serum + 1% penicillin + streptomycin) was transferred to a 15 mL centrifuge tube and centrifuged at 1000 rpm for 5 min. The supernatant was removed. The cells were resuspended with complete culture medium, inoculated into a culture dish at the required density, and cultured in an incubator at 37 °C, 95% humid air, and 5% carbon dioxide. The culture medium was replenished or the cells were passaged every 2-3 days depending on the cell growth condition.

### Cell proliferation activity assay

When the confluency of cells in the culture dish reached about 90%, the cells were washed twice with PBS, and then treated with trypsin to become single cells. The cells were counted, and 40µl of the cells was inoculated into each well of a 384-well plate (50 cells/well). The plate was equilibrated at 37 °C and 5% carbon dioxide for 10 to 15 minutes. The compounds were injected into the 384-well plate using Echo550, and positive control and negative control (0.1% DMSO) were set up. Each compound was subjected to a 3-fold dilution, resulting in 10 concentration gradients, in duplicate, and the content of DMSO was 0.1%. The 384-well plate was incubated in a cell culture incubator for 10 days.

Then, 40 µl of cell proliferation assay reagent (CTG) was added to the 384-well plate, and the plate was shaken to mix well. Then, the 384-well plate was incubated at 37 °C and 5% carbon dioxide in the dark for 30 minutes. The data were read using the Envision multifunctional microplate reader. The formula for calculating the percentage of inhibition rate of a compound was as follows: percentage of inhibition rate = 100-(signal value _{drug treatment group}-signal value _{positive control group})/(signal value _{negative control group}-signal value positive _{control group})×100. According to the different drug concentrations and their corresponding inhibition rates, the GI₅₀ curve was drawn using GraphPad 8.0 software. The data were analyzed, and the final IC₅₀ value was obtained. The results are shown in Table 1, wherein GI₅₀ ≤ 0.01 µM is AA, 0.01 µM < GI₅₀ ≤ 0.1 µM is A, 0.1 µM < GI₅₀ ≤ 1 µM is B, 1 µM < GI₅₀ ≤ 10 µM is C, and 10 µM < GI₅₀ is D.

**Table 1 Assay results of the anti-proliferative activity of the compounds of the present disclosure on cells**

| Compound No. | HCT116^{MTAP Deletion} GI₅₀ (µM) | Compound No. | HCT116^{MTAP Deletion} GI₅₀ (µM) |
|---|---|---|---|
| 1 | B | 2 | A |
| 3 | c | 4 | A |
| 5-P1/5-P2 | A/A | 6 | D |
| 7 | D | 8 | A |
| 9 | A | 10 | A |
| 11 | B | 12 | B |
| 13 | B | 14-P1 | B |
| 14-P2 | B | 15 | A |
| 16 | A | 17 | A |
| 18 | B | 19-P1 | A |
| 19-P2 | A | 20 | A |
| 21 | B | 22 | C |
| 23 | C | 24 | B |
| 25 | A | 26 | B |
| 27 | B | 28 | C |
| 29 | B | 30 | A |
| 31 | B | 32 | B |
| 33 | C | 34 | B |
| 35 | A | 36 | A |
| 37 | A | 38 | D |
| 39 | B | 40 | B |
| 41 | B | 42 | B |
| 43 | AA | 44 | A |
| 45-P1 | A | 45-P2 | A |
| 46-P1 | A | 46-P2 | A |
| 48 | AA | 49 | B |
| 50 | AA | 51 | A |
| 52 | AA | 53 | B |
| 54 | A | 55 | B |
| 56 | A | 57 | B |
| 58 | D | 59 | A |
| 60 | B | 61 | B |
| 62 | A | 63 | B |
| 64 | A | 65 | B |
| 66 | C | 67 | C |
| 68 | A | 69 | B |
| 70 | B | 71 | A |
| 72 | C | 73 | D |
| 74 | C | 75 | B |
| 76 | B | 77 | A |
| 78 | B | 79 | B |
| 80 | A | 81 | AA |
| 82 | B | 83 | A |
| 84 | A | 85 | B |
| 86 | A | 87 | B |
| 88 | AA | 89 | AA |
| 90 | AA | 92 | A |
| 93 | A | 94 | AA |
| 95 | AA | 96 | A |
| 97 | AA | 98 | AA |
| 99 | AA | 100 | AA |
| 101 | AA | 102 | AA |
| 103 | AA | 104 | A |
| 105 | B | 106-P1 | A |
| 106-P2 | AA | 107 | D |
| 108 | A | 109 | AA |
| 110-P1 | AA | 110-P2 | AA |
| 111 | AA | 112-P1 | AA |
| 112-P2 | A | 113 | AA |
| 114 | B | 115 | AA |
| 116 | A | 117 | A |
| 118 | AA | 119 | C |
| 120 | C | 121 | AA |
| 122 | A | 123 | AA |
| 124-P1 | AA | 124-P2 | A |
| 125-P1 | AA | 125-P2 | AA |
| 126 | C | 127 | A |
| 128 | A | 129 | C |
| 130 | B | 131 | A |
| 132-P1 | AA | 132-P2 | A |
| 133 | C | 134 | A |
| 135 | AA | 136 | A |
| 137 | AA | 138 | A |
| 139 | A | 140-P1 | AA |
| 140-P2 | A | 141-P1 | A |
| 141-P2 | B | 142 | C |
| 143 | B | 144 | A |
| 145 | A | 146-P1 | B |
| 146-P2 | A | 147 | A |
| 148 | A | 149 | A |
| 150 | AA | 151 | A |
| 152-P1 | B | 152-P2 | AA |
| 153-P1 | AA | 153-P2 | B |
| 154 | AA | 155 | A |
| 156 | AA | 157 | AA |
| 158 | AA | 159 | B |
| 160 | A | 161 | AA |
| 162 | B | 163 | AA |
| 164 | A | 165 | A |
| 166 | B | 167 | AA |
| 168 | AA | 169 | A |
| 170 | AA | 171 | A |
| 172 | A | 173 | A |
| 174 | AA | 175 | A |
| 176 | A | 177 | A |
| 178 | AA | 179 | A |
| 180 | AA | 181 | AA |
| 182 | AA | 184 | AA |
| 185 | AA | 186 | AA |
| 187 | AA | 188 | A |
| 189 | A | 190 | A |
| 191 | AA | 192 | AA |
| 193 | A | 194 | AA |
| 195 | AA | 196 | A |
| 197 | AA | 198 | A |
| 199 | A | 200 | AA |
| 201 | AA | 202 | AA |
| 203 | A | 204 | A |
| 205 | A | 206 | AA |
| 207 | AA | 208 | A |
| 209 | AA | 210 | AA |
| 211 | AA | 212 | AA |
| 213 | AA | 214 | AA |
| 215 | AA | 216 | A |
| 217 | AA | 218 | AA |
| 219 | AA | 220 | AA |
| 221 | AA | 222 | A |
| 223 | AA | 225 | AA |
| 226 | AA | 227 | A |
| 228 | B | 229 | A |
| 230 | AA | 231 | AA |
| 232 | AA | 233 | A |
| 234 | B | 235 | AA |
| 236 | AA | 237 | AA |
| 238 | AA | 239 | AA |
| 240 | A | 241 | AA |

In addition, the compounds of the present disclosure also have selectivity for mutant HCT116 cells. For example, the GI₅₀ of compound 33 and compound 34 for HCT116^{MTAP Deletion} is less than or equal to 0.1 µM, but the GI₅₀ for HCT116 is 1-10 µM, with a selectivity of more than 300 times. The selectivity data of representative compounds are shown in Table 2:

**Table 2 Selectivity data of representative compounds**

| Compound No. | Selectivity multiple of HCT116^{MTAP WT} GI₅₀/ HCT116^{MTAP Deletion} GI₅₀ | Compound No. | Selectivity multiple of HCT116^{MTAP WT} GI₅₀/ HCT116^{MTAP Deletion} GI₅₀ |
|---|---|---|---|
| 88 | 161 | 89 | 160 |
| 90 | 119 | 93 | 244 |
| 94 | 144 | 95 | 224 |
| 98 | 106 | 99 | 285 |
| 100 | 179 | 104 | 144 |
| 106-P2 | 169 | 110-P1 | 146 |
| 111 | 354 | 112-P1 | 385 |
| 113 | 125 | 124-P1 | 215 |
| 132-P1 | 266 | 135 | 285 |
| 156-P | 138 | 157 | 193 |
| 161 | 169 | 163-P1 | 94 |
| 164 | 337 | 165 | 295 |
| 167 | 61 | 168-P1 | 147 |
| 178 | 116 | 181-P1 | 247 |
| 184 | 221 | 185 | 240 |
| 186-P1 | 210 | 187-P1 | 78 |
| 187-P2 | 59 | 192-P1 | 252 |
| 194 | 429 | 195 | 226 |
| 201 | 128 | | |

### Assay example 2: Method of In-Cell Western Assay of cellular SDMA

### 2.1 Materials and cells

HCT116^{MTAP Deletion} cells were from Horizon; HCT116 cells were from ATCC; RPMI-1640 medium was purchased from ATCC (USA); penicillin-streptomycin was purchased from Gibco (USA); fetal bovine serum was purchased from ExCell; 96-well plate was purchased from Corning (USA); Symmetric Di-Methyl Arginine Motif [sdme-RG] antibody was purchased from CST (USA); IRDye 800CW Goat anti-Rabbit IgG secondary antibody was purchased from LI-COR (USA), and CellTag 700 stain was purchased from LI-COR (USA).

### 2.2 Storage of compound

Compound dissolution: All the compounds were dissolved in dimethyl sulfoxide (DMSO) and then stored. The compounds were stored at -20°C for long term storage.

### 2.3 Cell culture and passaging

The cells were cultured strictly in accordance with ATCC requirements. The cell culture medium (RPMI-1640 medium + 10% serum + 1% penicillin + streptomycin) was transferred to a 15 mL centrifuge tube and centrifuged at 1000 rpm for 5 min. The supernatant was removed. The cells were resuspended with complete culture medium, inoculated into a culture dish at the required density, and cultured in an incubator at 37 °C, 95% humid air, and 5% carbon dioxide. The culture medium was replenished or the cells were passaged every 2-3 days depending on the cell growth condition.

### 2.4 In-Cell Western Assay of SDMA

When the confluency of cells in the culture dish reached about 90%, the cells were washed twice with PBS, and then digested with trypsin. 100 µl of the cells was inoculated into each well of a 96-well plate (800 cells/well). The plate was incubated overnight in a 37 °C and 5% CO₂ incubator. 50 µL of 3x final **concentration** of a compound was added to the cell culture plate. Each compound was subjected to a 3-fold dilution, resulting in 10 concentration gradients, in duplicate, and the final concentration of DMSO was 0.5%. The plate was incubated in a 37 °C and 5% CO₂ incubator for 96 hours.

150µL of 8% paraformaldehyde was added to each well, and the plate was incubated at room temperature for 15 minutes. The supernatant was discarded. The plate was washed with PBS, and 200µL of freshly prepared 1x Permeabilization buffer was added to each well. The plate was incubated at room temperature for 30 minutes. The supernatant was discarded. 200µL of 1x Blocking buffer was added to each well, and the plate was incubated at room temperature for 2 hours. The supernatant was discarded. 50µL of primary antibody (CST, 1x Incubation buffer 1:500 dilution) was added to each well, and the plate was incubated at 4°C overnight. The supernatant was discarded. 250µL of 1x Washing buffer was added to each well, and the plate was washed three times. 50µL of secondary antibody (IRDye 800CW Goat anti-Rabbit IgG and CellTag 700 dye, 1:800 and 1:500 diluted in Incubation buffer) was added to each well, and the plate was incubated at room temperature for 1 hour. The supernatant was discarded. The plate was washed with 1x Washing buffer. Signals were detected using Licor Odyssey CLX. The formula for calculating the percentage of inhibition rate of a compound was as follows: percentage of inhibition rate = 100-(signal value _{drug treatment} group-signal value background ᵥₐₗᵤₑ)/(signal value _{0.5% DMSO treatment group}-signal value _{background value}) × 100. According to the different drug concentrations and their corresponding inhibition rates, the IC₅₀ curve was drawn using GraphPad 8.0 software. The data were analyzed, and the final IC₅₀ value was obtained. The results are shown in Table 3, wherein IC₅₀ ≤ 0.01 µM is AA, 0.01 µM < IC₅₀ ≤ 0.1 µM is A, 0.1 µM < IC₅₀ ≤ 1 µM is B, and 1 µM < IC₅₀ ≤ 10 µM is C.

**Table 3 Inhibitory activity on cellular SDMA**

| Compound No. | SDMA inhibition IC₅₀ (µM) in HCT116^{MTAP Deletion} | SDMA inhibition IC₅₀ (µM) in HCT116 |
|---|---|---|
| 2 | AA | |
| 106-P2 | AA | A |
| 112-P1 | AA | B |
| 132-P1 | AA | |
| 135 | AA | |
| 152-P2 | A | |
| 156-P1 | AA | |
| 192-P1 | AA | |

### Assay example 3 In vivo pharmacodynamic study in LU99 CDX model

3.1 Assay materials: LU99 cells were from JCRB (Japan); RPMI-1640 and 0.25% trypsin-EDTA were purchased from Gibco (USA); fetal bovine serum was purchased from Dongling (China); PBS was purchased from Hyclone (USA); penicillin-streptomycin was purchased from Meilun Biotechnology (China); Matrigel was purchased from Corning (USA).

3.2 Information of animals: BALB/c Nude mice, female, 6-8 weeks old, weighing 18-22 g, were purchased from Beijing Vital River Laboratory Animal Technology Co., Ltd. The mice were housed in an SPF environment with individual ventilation for each cage. All the animals had free access to standard certified commercial laboratory diets and drinking water.

### 3.3 Assay methods:

3.3.1 Cell culture: Human lung giant cell carcinoma cells LU99 were cultured in monolayer in vitro in RPMI-1640 medium supplemented with 10% fetal bovine serum in a 37 °C and 5% CO₂ incubator. The cells were routinely digested with trypsin-EDTA and passaged twice a week. When the number of cells reached the required level, the cells were harvested and counted.

3.3.2 Cell inoculation: 0.2 mL (5×10⁶) of LU99 cells (Matrigel was added at a volume ratio of 1:1) were subcutaneously inoculated into the right back of each mouse. When the average tumor volume reached about 150 mm³, the mice were divided into groups and administered with drugs according to the tumor volume and animals' body weight using random stratified sampling method.

3.3.3 Administration: The dosage of compound 106-P2 and compound 112-P1 was 30 mpk, PO, once daily (QD) × 3 weeks. There were 6 mice in each group.

### 3.3.4 Tumor measurement and assay indicators:

The diameter of a tumor was measured with a vernier caliper twice a week. The calculation formula for the tumor volume was: V = 0.5a × b², wherein a and b represent the long diameter and short diameter of the tumor, respectively. The body weight of a mouse was measured twice a week.

The antitumor efficacy of a compound was evaluated by tumor growth inhibition rate TGI(%).TGI (%) = [(1-(average tumor volume at the end of administration of a treatment group - average tumor volume at the beginning of administration of the treatment group))/(average tumor volume at the end of treatment of the solvent control group - average tumor volume at the beginning of treatment of the solvent control group)] × 100%.

The assay results are shown in Fig. 1, Fig. 2, Fig. 3 and Fig. 4. No mice became sick or died during the assay.

### Assay example 4 Investigation of brain permeability in CD1 mice after oral gavage administration

### 4.1 Assay design

### 4.1.1 Information of administration

| Group | Name of the test sample | Dosage (mg/kg) | Volume (mL/kg) | Concentration (mg/mL) | Administration method | Number of animals (male) |
|---|---|---|---|---|---|---|
| 1 | 112-P1 | 30 | 10 | 3 | PO | 3 at each time point |

### 4.1.2 Collection time:

| | |
|---|---|
| Group | PK collection time point |
| PO | Plasma, cerebrospinal fluid, brain: 0.25, 1, 2, 4, 8 hours |

### 4.1.3 Collection and processing of blood sample

| Collection site | Blood collection by cardiac puncture |
|---|---|
| Amount of the sample collected | Approximately 0.5 mL of whole blood per animal at each time point |
| Anticoagulant | EDTA-K2 |
| Processing and storage of plasma sample | 1) Approximately 0.2 mL of blood was collected into a pre-chilled tube at each time point. All the blood samples should be placed on wet ice immediately before processing. |
| | 2) The blood samples were centrifuged at 4 °C and 4000 g for 5 minutes to give plasma as quickly as possible. |
| | 3) For each plasma sample collected, 0.1 mL of the sample was transferred to a pre-chilled 2 mL polypropylene screw-cap tube, and 10 µL of frozen 0.1 mg/mL dichlorvos (DDV)* was added on wet ice. |
| | 4) All the plasma samples should be immediately placed on dry ice and then stored at -80°C for final storage. |
| Processing and storage of cerebrospinal fluid sample | 1) After anesthesia, the cisterna magna was exposed and cerebrospinal fluid samples were collected with a syringe. |
| | 2) The cerebrospinal fluid (CSF) samples were stored in polypropylene tubes and stored in a -75±15 °C freezer before analysis. |
| Processing and storage of brain sample | The mice needed to be completely bled out before collecting the brain samples. |
| | After the brain samples were collected, the samples were rinsed with a small amount of saline to remove residual blood on the surface, and then the saline on the surface was absorbed with a filter paper. |
| | 2) The brain samples were collected at the specified time points, and the samples were frozen in a -75±15 °C freezer for later use. |
| | 3) Before analysis, all the brain samples were weighed and slurried with PBS at a ratio of 1:4 of brain weight (g) to PBS volume (mL). |
| | The actual concentration was the test value multiplied by the dilution factor. |

### 4.1.4 Evaluation during the in vivo experiment

| Index | Frequency |
|---|---|
| Cage observation | Twice a day |
| Detailed clinical observation | Before the random grouping and on the day of administration |
| Body weight | Before administration |

### 4.1.5 Test system

| | |
|---|---|
| Species/Strain/Sex: | Mouse/CD1/Male |
| Source: | Qualified suppliers |
| Age on the day of administration: | About 6-8 weeks |
| Weight on the day of administration: | About 20-30g |

### 4.2 Test sample and preparation

### 4.2.1 Information of test sample

| Name | Lot # | MW | FW | Purity | Weight (mg) | Storage condition |
|---|---|---|---|---|---|---|
| 112-P1 | NA | 434.38 | 434.38 | 99.7% | 15.16 | Room temperature |

### 4.2.2 Preparation

| | |
|---|---|
| Preparation frequency: | Freshly prepared on the day of administration |
| Solvent composition: | PO: 2% DMA/10% Solutol HS-15/88% saline |
| Preparation process: | Room temperature |
| Storage condition: | To be determined |

### 4.3 Sample analysis

All the samples were analyzed by LC-MS/MS, and the concentration of the drug to be tested in the samples was determined by the standard curve method.

For the plasma assay results, the pharmacokinetic parameters were calculated using WinNonlin (Phoenix^{™}) or other similar software.

### 4.4 Assay results

**Table 4 Brain permeability data of the compound of the present disclosure**

| Group | Plasma AUCₗₐₛₜ/D (h*mg/mL) | CSF AUCₗₐₛₜ/D (h*mg/mL) | Brain AUCₗₐₛₜ/D (h*mg/mL) |
|---|---|---|---|
| **112-P1** | 464 | 6.15 | 428 |

## Claims

1. A compound of formula (X), or a pharmaceutically acceptable salt, an isotopic variant, a tautomer, a stereoisomer, a prodrug, a polymorph, a hydrate or a solvate thereof: 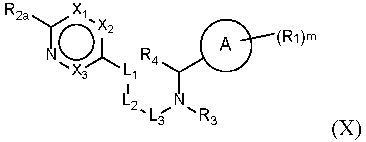 wherein,
ring A is C₆₋₁₀ aryl or 5- to 10-membered heteroaryl;
L₁ is NH or CHRₓ;
L₂ is CRₓR_{y};
L₃ is -C(O)-, -S(O)- or -S(O)₂-;
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene; R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)(=NRₐ)Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
R₄ is selected from H, D, C₁₋₆ alkyl and C₁₋₆ haloalkyl;
or one R₁ is connected to R₄ to form -(CRₛₛRₛₛ)_{q1}-X-(CRₛₛRₛₛ)_{q2}- (alternatively -(CH₂)_{q1}-X-(CH₂)_{q2}-); wherein X is O, S, NH or CH₂, q1=0, 1 or 2, q2=1, 2 or 3, Rₛₛ is independently selected from H, D, C₁₋₆ alkyl and C₁₋₆ haloalkyl, or two Rₛₛ and the carbon atom to which they are attached are taken together to form C₃₋₁₀ cycloalkyl; X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, CN, ORₐ, NR_{b}R_{c}, C(O)OR_{A} and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₃ is selected from -C₁₋₆ alkylene-ORₐ, -C₁₋₆ alkylene-NR_{b}R_{c}, C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl, which is optionally substituted with 1, 2 or 3 R₃ₛ₁ and 1 R₃ₛ₂;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, CN, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; or two R₁ₛ and the atom(s) to which they are attached are taken together to form C₃₋₁₀ cycloalkyl or 3- to 10-membered heterocyclyl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ; or, R₃ₛ₁, R₃ₛ₂ and the carbon atom(s) to which they are attached are taken together to form C₃₋₁₀ cycloalkyl; R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the groups in the above ring A, L₁, L₂, Rₓ, R_{y}, R₁, R₂ₐ, R_{2b}, R_{2c}, R_{2d}, R₃, R₄, L, R, R₁ₛ, R₂ₛ, R₃ₛ, R₃ₛ₁, R₃ₛ₂, Rₐ, R_{b} and R_{c} is optionally deuterated, up to fully deuterated;
with the proviso that, when L₂ is CRₓR_{y} and L₃ is -S(O)- or -S(O)₂-, R_{y} and R_{2d} are combined to form vinylene.

2. The compound according to claim 1, or a pharmaceutically acceptable salt, an isotopic variant, a tautomer, a stereoisomer, a prodrug, a polymorph, a hydrate or a solvate thereof, wherein, ring A is phenyl or 5- to 6-membered heteroaryl; alternatively phenyl, pyridyl, pyridazinyl, pyrimidinyl or pyrazinyl.

3. The compound according to claim 1 or 2, or a pharmaceutically acceptable salt, an isotopic variant, a tautomer, a stereoisomer, a prodrug, a polymorph, a hydrate or a solvate thereof, wherein, L₁ is NH, and L₂ is CRₓR_{y}; -L₁-L₂- is alternatively 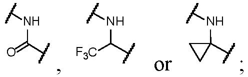 -L₁-L₂- is alternatively

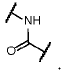

4. The compound according to any one of claims 1 to 3, or a pharmaceutically acceptable salt, an isotopic variant, a tautomer, a stereoisomer, a prodrug, a polymorph, a hydrate or a solvate thereof, wherein, L₃ is -C(O)-.

5. The compound according to any one of claims 1 to 4, or a pharmaceutically acceptable salt, an isotopic variant, a tautomer, a stereoisomer, a prodrug, a polymorph, a hydrate or a solvate thereof, wherein, R₁ and R₄ are connected to form -(CH₂)_{q1}-X-(CH₂)_{q2}-; wherein X is O, S, NH or CH₂, q1=0 or 1, q2=1 or 2; alternatively, R₁ and R₄ are connected to form -(CH₂)_{q1}-X-(CH₂)_{q2}-; wherein X is O, S or NH, q1=0 or 1, q2=1 or 2.

6. The compound according to any one of claims 1 to 5, or a pharmaceutically acceptable salt, an isotopic variant, a tautomer, a stereoisomer, a prodrug, a polymorph, a hydrate or a solvate thereof, wherein, 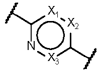 is selected from 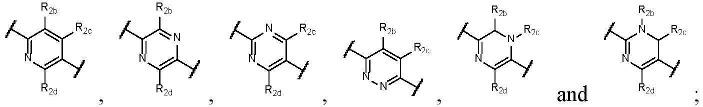 alternatively

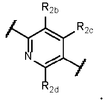

7. The compound according to any one of claims 1 to 6, or a pharmaceutically acceptable salt, an isotopic variant, a tautomer, a stereoisomer, a prodrug, a polymorph, a hydrate or a solvate thereof, wherein, R₂ₐ is selected from H, D, CN, ORₐ and NR_{b}R_{c}; alternatively selected from ORₐ and NR_{b}R_{c}; alternatively selected from OMe and NH₂; alternatively NH₂.

8. The compound according to any one of claims 1 to 7, or a pharmaceutically acceptable salt, an isotopic variant, a tautomer, a stereoisomer, a prodrug, a polymorph, a hydrate or a solvate thereof, wherein, X₁, X₂ and their substituents are taken together to form C₅₋₁₀ cycloalkyl, 5- to 10-membered heterocyclyl, C₆₋₁₀ aryl or 5- to 10-membered heteroaryl; alternatively form C₅₋₇ cycloalkyl, 5- to 7-membered heterocyclyl, phenyl or 5- to 6-membered heteroaryl; alternatively form 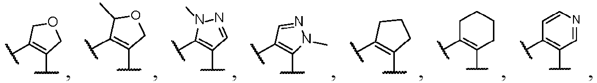 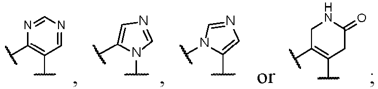 alternatively form 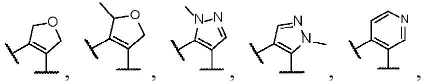 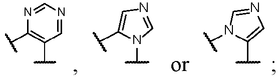 alternatively form 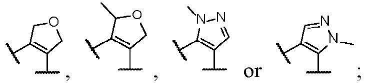 alternatively form

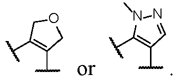

9. The compound according to any one of claims 1 to 8, or a pharmaceutically acceptable salt, an isotopic variant, a tautomer, a stereoisomer, a prodrug, a polymorph, a hydrate or a solvate thereof, wherein, R₃ is selected from C₁₋₆ alkyl, C₁₋₆ haloalkyl, C₂₋₆ alkenyl, C₂₋₆ alkynyl, -L-C₃₋₁₀ cycloalkyl, -L-3- to 10-membered heterocyclyl, -L-C₆₋₁₀ aryl and -L-5- to 10-membered heteroaryl; alternatively selected from C₁₋₆ alkyl, C₁₋₆ haloalkyl, -L-C₃₋₁₀ cycloalkyl, -L-3- to 10-membered heterocyclyl, -L-C₆₋₁₀ aryl and -L-5- to 10-membered heteroaryl; alternatively selected from C₁₋₆ alkyl, C₁₋₆ haloalkyl, C₅₋₆ cycloalkyl, 5- to 6-membered heterocyclyl, phenyl and 5- to 6-membered heteroaryl; alternatively selected from Me, Et, iPr, cyclopropyl, cyclobutyl,

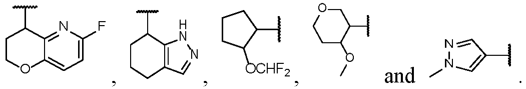

10. The compound according to any one of claims 1 to 9, or a pharmaceutically acceptable salt, an isotopic variant, a tautomer, a stereoisomer, a prodrug, a polymorph, a hydrate or a solvate thereof, wherein the compound has the following structural formula: 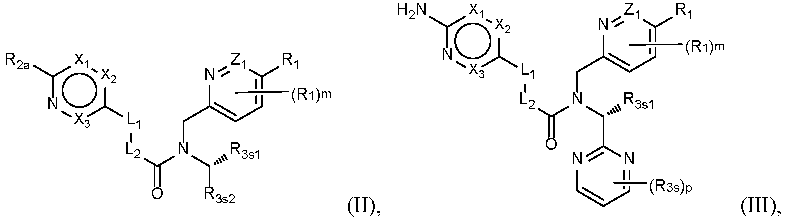 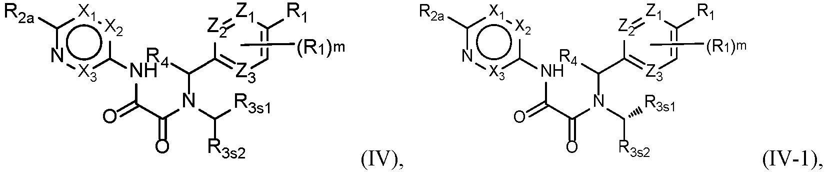 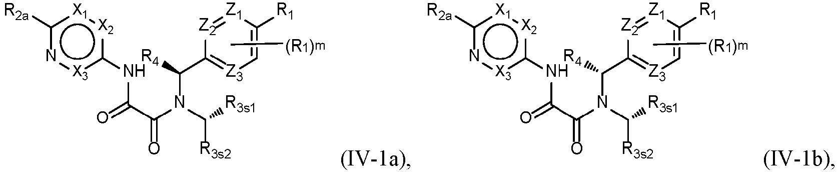 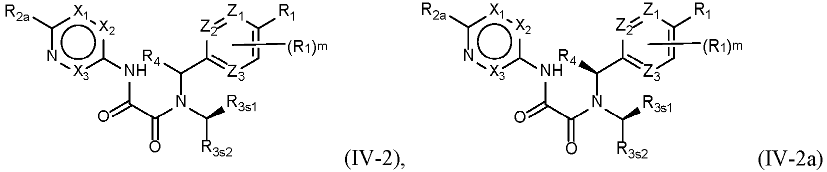 or 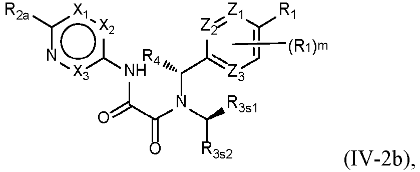 wherein each group is as defined according to claims 1 to 9.

11. The compound according to claim 10, or a pharmaceutically acceptable salt, an isotopic variant, a tautomer, a stereoisomer, a prodrug, a polymorph, a hydrate or a solvate thereof, which is a compound of formula (II), wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene; Z₁ is CH or N;
R₁ is selected from H, D, halogen, CN, ORₐ, SRₐ, NR_{b}R_{c}, SF₅, C₁₋₆ alkyl, C₁₋₆ haloalkyl, C₅₋₇ cycloalkyl and 5- to 7-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is selected from H, D, ORₐ and NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
R₁ₛ is selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl;
R₂ₛ is selected from H, D, halogen, =O, C₁₋₆ alkyl and C₁₋₆ haloalkyl;
R₃ₛ₁ is selected from H, D, halogen, C₁₋₆ alkyl, C₁₋₆ haloalkyl, C₃₋₆ cycloalkyl and 3- to 6-membered heterocyclyl;
R₃ₛ₂ is selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl, which is optionally substituted with 1, 2 or 3 R₃ₛ;
L is a chemical bond or C₁₋₆ alkylene, which is optionally substituted with 1, 2 or 3 R;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom to which they are attached are taken together to form 5- to 10-membered heterocyclyl;
wherein each of the groups in the above L₁, L₂, Rₓ, R_{y}, Z₁, R₁, R₂ₐ, R_{2b}, R_{2c}, R_{2d}, R₁ₛ, R₂ₛ, R₃ₛ₁, R₃ₛ₂, L, R, R₃ₛ, Rₐ, R_{b} and R_{c} is optionally deuterated, up to fully deuterated.

12. The compound according to claim 11, or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₄ cycloalkylene;
or when L₁ is CHRₓ and L₂ is CRₓR_{y}, the two Rₓ groups may combine to form a chemical bond or C₁₋₃ alkylene; Z₁ is CH or N;
R₁ is selected from H, D, halogen, CN, ORₐ, NR_{b}R_{c}, C₁₋₆ alkyl, C₁₋₆ haloalkyl and 5- to 6-membered heterocyclyl, wherein the heterocyclyl is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is NH₂;
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H, D, C₁₋₆ alkyl and C₁₋₆ haloalkyl;
R₁ₛ is selected from H, D, halogen, C(O)ORₐ, C(O)NR_{b}R_{c}, C₁₋₆ alkyl and C₁₋₆ haloalkyl;
R₂ₛ is selected from H, D, =O, C₁₋₆ alkyl and C₁₋₆ haloalkyl;
R₃ₛ₁ is selected from H, D, C₁₋₆ alkyl, C₁₋₆ haloalkyl, C₃₋₆ cycloalkyl and 3- to 6-membered heterocyclyl;
R₃ₛ₂ is selected from H, D, -L-ORₐ, -L-NR_{b}R_{c}, C₁₋₆ alkyl, C₁₋₆ haloalkyl, C₅₋₇ cycloalkyl, 5- to 7-membered heterocyclyl, C₆₋₁₀ aryl and 5- to 10-membered heteroaryl, which is optionally substituted with 1, 2 or 3 R₃ₛ;
L is a chemical bond or C₁₋₃ alkylene, which is optionally substituted with 1, 2 or 3 R;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl.

13. The compound according to claim 11, or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₃ alkyl or C₁₋₃ haloalkyl;
or CRₓR_{y} are taken together to form C=O or cyclopropylene;
or when L₁ is CHRₓ and L₂ is CRₓR_{y}, the two Rₓ groups may combine to form a chemical bond or C₁₋₂ alkylene; Z₁ is CH or N;
R₁ is selected from H, D, CN, ORₐ, C₁₋₆ alkyl, C₁₋₆ haloalkyl and 5- to 6-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R₂ₐ is NH₂;
R_{2b} is selected from H, D, halogen, C(O)NR_{b}R_{c}, C₁₋₆ alkyl, C₁₋₆ haloalkyl and 4- to 6-membered heterocyclyl; R_{2c} is selected from H, D, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ; alternatively form 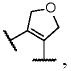 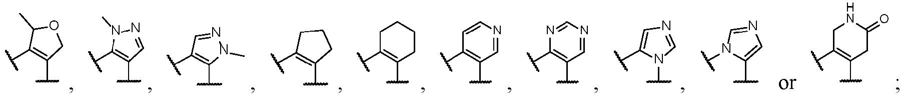 R_{2d} is selected from H and D;
R₁ₛ is selected from H, D, halogen, C(O)ORₐ, C₁₋₆ alkyl and C₁₋₆ haloalkyl, alternatively halogen or C(O)OCH₃; R₂ₛ is selected from H, D, =O and Me;
R₃ₛ₁ is selected from H, D, C₁₋₆ alkyl, C₁₋₆ haloalkyl and C₃₋₆ cycloalkyl;
R₃ₛ₂ is selected from H, D, -L-ORₐ, C₁₋₆ alkyl, C₁₋₆ haloalkyl, C₅₋₇ cycloalkyl, phenyl and 5- to 10-membered heteroaryl, which is optionally substituted with 1, 2 or 3 R₃ₛ; alternatively, R₃ₛ₂ is selected from H, D, Me, CF₃, CH₂OCH₃, cyclopropyl, 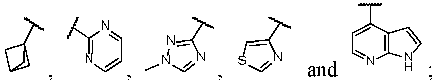
L is a chemical bond or C₁₋₃ alkylene, alternatively methylene, which is optionally substituted with 1, 2 or 3 R; R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl.

14. The compound according to claim 10, or a pharmaceutically acceptable salt, an isotopic variant, a tautomer, a stereoisomer, a prodrug, a polymorph, a hydrate or a solvate thereof, which is a compound of formula (II), wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3 or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl (alternatively 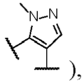 which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ; R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl, alternatively H, D, C₁₋₆ alkyl or C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

15. The compound according to claim 14, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl (alternatively 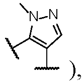 which is optionally substituted with 1, 2 or 3 R₂ₛ;
R_{2d} is selected from H, D, halogen, CN, NO₂, ORₐ, NR_{b}R_{c}, C₁₋₆ alkyl and C₁₋₆ haloalkyl;
Z₁ is CH;
L is a chemical bond, C₁₋₆ alkylene, C₂₋₆ alkenylene or C₂₋₆ alkynylene;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₆ cycloalkyl, 3- to 6-membered heterocyclyl, phenyl and 5- to 6-membered heteroaryl, alternatively H, D, C₁₋₆ alkyl or C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

16. The compound according to claim 14, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl (alternatively 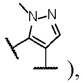 which is optionally substituted with 1, 2 or 3 R₂ₛ;
R_{2d} is selected from H, D, halogen, CN, NO₂, C₁₋₆ alkyl and C₁₋₆ haloalkyl;
Z₁ is CH;
L is a chemical bond or C₁₋₆ alkylene;
R₁ₛ and R₂ₛ are independently selected from H, D, halogen, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl and C₁₋₆ haloalkyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₆ cycloalkyl and 3- to 6-membered heterocyclyl, alternatively H, D, C₁₋₆ alkyl or C₁₋₆ haloalkyl; or R_{b}, R_{c} and the atom to which they are attached are taken together to form 5- to 10-membered heterocyclyl;
wherein each of the above groups is optionally deuterated, up to fully deuterated.

17. The compound according to claim 14, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR_{b}R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl (alternatively 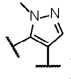 ),which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ; R and R₃ₛ are independently selected from H, D, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₆ cycloalkyl and 3- to 6-membered heterocyclyl, alternatively H, D, C₁₋₆ alkyl or C₁₋₆ haloalkyl; or R_{b}, R_{c} and the atom(s) to which they are attached are taken together to form 5- to 10-membered heterocyclyl;
wherein each of the above groups is optionally deuterated, up to fully deuterated.

18. The compound according to claim 17, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, -L-ORₐ, -L-SRₐ, -L-NR_{b}R_{c}, C(O)Rₐ, C(O)ORₐ, C(O)NR_{b}R_{c}, S(O)Rₐ, S(O)₂Rₐ, S(O)ORₐ, S(O)₂ORₐ, S(O)NR_{b}R_{c}, S(O)₂NR_{b}R_{c}, C₁₋₆ alkyl, C₁₋₆ haloalkyl, C₂₋₆ alkenyl, C₂₋₆ alkynyl, -L-C₃₋₇ cycloalkyl and -L-5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from H, D, CN, ORₐ and NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl (alternatively 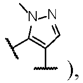 which is optionally substituted with 1, 2 or 3 R₂ₛ;
R_{2d} is selected from H, D, halogen and CN;
Z₁ is CH;
L is a chemical bond, C₁₋₆ alkylene, C₂₋₆ alkenylene or C₂₋₆ alkynylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl;
Rₐ, R_{b} and R_{c} are independently selected from H, D, C₁₋₆ alkyl, C₁₋₆ haloalkyl, C₃₋₆ cycloalkyl and 3- to 6-membered heterocyclyl, alternatively H, D, C₁₋₆ alkyl or C₁₋₆ haloalkyl; or R_{b}, R_{c} and the atom to which they are attached are taken together to form 5- to 10-membered heterocyclyl;
wherein each of the above groups is optionally deuterated, up to fully deuterated.

19. The compound according to claim 17, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from ORₐ and NR_{b}R_{c};
R_{2b} is selected from C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl (alternatively 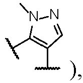 which is optionally substituted with 1, 2 or 3 R₂ₛ;
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H, D, halogen, C₁₋₄ alkyl and C₁₋₄ haloalkyl;
R₃ₛ₂ is selected from -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl;
Rₐ, R_{b} and R_{c} are independently selected from H, D, C₁₋₆ alkyl, C₁₋₆ haloalkyl, C₃₋₆ cycloalkyl and 3- to 6-membered heterocyclyl, alternatively H, D, C₁₋₆ alkyl or C₁₋₆ haloalkyl; or R_{b}, R_{c} and the atom to which they are attached are taken together to form 5- to 10-membered heterocyclyl;
wherein each of the above groups is optionally deuterated, up to fully deuterated.

20. The compound according to claim 17, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 10-membered heteroaryl, which is optionally substituted with 1, 2 or 3 R₁ₛ;
m = 0, 1, 2, 3, or 4;
X₁ is C atom, which is substituted with R_{2b};
X₂ is C atom, which is substituted with R_{2c};
X₃ is C atom, which is substituted with R_{2d};
R₂ₐ is selected from ORₐ and NR_{b}R_{c};
R_{2b} is selected from C(O)ORₐ, C(O)NR_{b}R_{c} and C₁₋₆ alkyl;
R_{2c} is selected from H, D, halogen, C₁₋₆ alkyl and C₁₋₆ haloalkyl;
or X₁, X₂ and their substituents are taken together to form 5- to 10-membered heterocyclyl, C₆₋₁₀ aryl or 5- to 10-membered heteroaryl (alternatively 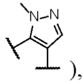 which is optionally substituted with 1, 2 or 3 R₂ₛ;
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl; R₃ₛ₁ is Me;
R₃ₛ₂ is Et;
Rₐ, R_{b} and R_{c} are independently selected from H, D, C₁₋₆ alkyl, C₁₋₆ haloalkyl, C₃₋₆ cycloalkyl and 3- to 6-membered heterocyclyl, alternatively H, D, C₁₋₆ alkyl or C₁₋₆ haloalkyl; or R_{b}, R_{c} and the atom to which they are attached are taken together to form 5- to 10-membered heterocyclyl;
wherein each of the above groups is optionally deuterated, up to fully deuterated.

21. The compound according to claim 17, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 6-membered heteroaryl, which is optionally substituted with 1, 2 or 3 R₁ₛ;
m = 0, 1, 2, 3, or 4;
X₃ is C atom, which is substituted with R_{2d};
R₂ₐ is selected from ORₐ and NR_{b}R_{c};
X₁, X₂ and their substituents are taken together to form 5- to 6-membered heteroaryl (alternatively 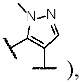 which is optionally substituted with 1, 2 or 3 R₂ₛ;
R_{2d} is selected from H, D, halogen and CN;
Z₁ is CH;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from H and D;
R₃ₛ₂ is selected from C₃₋₆ cycloalkyl, 5- to 6-membered heterocyclyl, phenyl and 5- to 6-membered heteroaryl; Rₐ, R_{b} and R_{c} are independently selected from H, D, C₁₋₆ alkyl and C₁₋₆ haloalkyl; or R_{b}, R_{c} and the atom to which they are attached are taken together to form 5- to 10-membered heterocyclyl;
wherein each of the above groups is optionally deuterated, up to fully deuterated.

22. The compound according to claim 17, or a pharmaceutically acceptable salt, an isotopic variant, a tautomer, a stereoisomer, a prodrug, a polymorph, a hydrate or a solvate thereof, wherein,
L₁ is NH;
L₂ is CRₓR_{y}; wherein CRₓR_{y} are taken together to form C=O or C=S;
R₁ is selected from H, D, ORₐ, SRₐ, NR_{b}R_{c}, C(O)Rₐ, S(O)Rₐ, S(O)₂Rₐ, C₁₋₆ alkyl, C₁₋₆ haloalkyl, C₃₋₇ cycloalkyl and 5- to 6-membered heteroaryl, which is optionally substituted with 1, 2 or 3 R₁ₛ;
m = 0, 1, 2, 3, or 4;
X₃ is C atom, which is substituted with R_{2d};
R₂ₐ is selected from ORₐ and NR_{b}R_{c};
X₁, X₂ and their substituents are taken together to form 5- to 6-membered heteroaryl (alternatively which is optionally substituted with 1, 2 or 3 R₂ₛ; 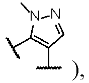
R_{2d} is selected from H, D, halogen and CN;
Z₁ is CH;
L is a chemical bond or C₁₋₆ alkylene;
R₁ₛ and R₂ₛ are independently selected from H, D, C₁₋₆ alkyl, C₁₋₆ haloalkyl, C₂₋₆ alkenyl and C₂₋₆ alkynyl;
R₃ₛ₁ is selected from C₁₋₄ alkyl and C₁₋₄ haloalkyl;
R₃ₛ₂ is selected from -L-ORₐ, -L-NR_{b}R_{c}, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom to which they are attached are taken together to form 5- to 10-membered heterocyclyl;
wherein each of the above groups is optionally deuterated, up to fully deuterated.

23. The compound according to claim 10, or a pharmaceutically acceptable salt, an isotopic variant, a tautomer, a stereoisomer, a prodrug, a polymorph, a hydrate or a solvate thereof, which is a compound of formula (III), wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₆ cycloalkylene;
or when L₁ is CHRₓ and L₂ is CRₓR_{y}, the two Rₓ groups may combine to form a chemical bond or C₁₋₄ alkylene; Z₁ is CH or N;
R₁ is selected from H, D, halogen, CN, ORₐ, SRₐ, NR_{b}R_{c}, SF₅, C₁₋₆ alkyl, C₁₋₆ haloalkyl, C₅₋₇ cycloalkyl and 5- to 7-membered heterocyclyl, which is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C(O)NR_{b}R_{c}, C₁₋₆ alkyl, C₁₋₆ haloalkyl, C₅₋₇ cycloalkyl and 5- to 7-membered heterocyclyl;
R₂ₛ is selected from H, D, halogen, =O, C₁₋₆ alkyl and C₁₋₆ haloalkyl;
R₃ₛ₁ is selected from H, D, halogen, C₁₋₆ alkyl, C₁₋₆ haloalkyl, C₃₋₆ cycloalkyl and 3- to 6-membered heterocyclyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl; or R_{b}, R_{c} and the atom to which they are attached are taken together to form 5- to 10-membered heterocyclyl;
wherein each of the groups in the above L₁, L₂, Rₓ, R_{y}, Z₁, R₁, R_{2b}, R_{2c}, R₁ₛ, R₂ₛ, R₃ₛ₁, R₃ₛ₁ Rₐ, R_{b} and R_{c} is optionally deuterated, up to fully deuterated.

24. The compound according to claim 23, or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₆ alkyl or C₁₋₆ haloalkyl;
or CRₓR_{y} are taken together to form C=O, C=S or C₃₋₄ cycloalkylene;
or when L₁ is CHRₓ and L₂ is CRₓR_{y}, the two Rₓ groups may combine to form a chemical bond or C₁₋₃ alkylene; Z₁ is CH or N;
R₁ is selected from H, D, halogen, CN, ORₐ, NR_{b}R_{c}, C₁₋₆ alkyl, C₁₋₆ haloalkyl and 5- to 6-membered heterocyclyl, wherein the heterocyclyl is optionally substituted with 1, 2 or 3 R₁ₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)ORₐ, C(O)NR_{b}R_{c}, C₁₋₆ alkyl, C₁₋₆ haloalkyl, C₃₋₆ cycloalkyl and 3- to 6-membered heterocyclyl;
R_{2c} is selected from H, D, halogen, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C(O)NR_{b}R_{c}, C₁₋₆ alkyl and C₁₋₆ haloalkyl;
R₂ₛ is selected from H, D, =O, C₁₋₆ alkyl and C₁₋₆ haloalkyl;
R₃ₛ₁ is selected from H, D, C₁₋₆ alkyl, C₁₋₆ haloalkyl, C₃₋₆ cycloalkyl and 3- to 6-membered heterocyclyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl.

25. The compound according to claim 23, or a pharmaceutically acceptable salt, an isotopic variant, a tautomer, a stereoisomer, a prodrug, a polymorph, a hydrate or a solvate thereof, wherein,
L₁ is NH or CHRₓ;
L₂ is CRₓR_{y};
wherein Rₓ and R_{y} are independently H, D, C₁₋₃ alkyl or C₁₋₃ haloalkyl;
or CRₓR_{y} are taken together to form C=O or cyclopropylene;
or when L₁ is CHRₓ and L₂ is CRₓR_{y}, the two Rₓ groups may combine to form a chemical bond or C₁₋₂ alkylene; Z₁ is CH or N;
R₁ is selected from H, D, CN, ORₐ, C₁₋₆ alkyl, C₁₋₆ haloalkyl and 5- to 6-membered heterocyclyl, which is optionally substituted with 1, 2 or 3 Rₗₛ;
m = 0, 1, 2 or 3, alternatively m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is selected from H, D, halogen, C(O)NR_{b}R_{c}, C₁₋₆ alkyl, C₁₋₆ haloalkyl and 4- to 6-membered heterocyclyl; R_{2c} is selected from H, D, C₁₋₆ alkyl and C₁₋₆ haloalkyl;
or X₁, X₂ and their substituents are taken together to form C₅₋₇ cycloalkyl, 5- to 7-membered heterocyclyl, phenyl or 5- to 6-membered heteroaryl, which is optionally substituted with 1, 2 or 3 R₂ₛ;
R_{2d} is selected from H and D;
R₁ₛ is selected from H, D, halogen, C(O)ORₐ, C₁₋₆ alkyl and C₁₋₆ haloalkyl, alternatively halogen or C(O)OCH₃; R₂ₛ is selected from H, D, =O and Me;
R₃ₛ₁ is selected from H, D, C₁₋₆ alkyl, C₁₋₆ haloalkyl and C₃₋₆ cycloalkyl;
R₃ₛ is selected from H, D, halogen, C₁₋₆ alkyl and C₁₋₆ haloalkyl;
p=0, 1, 2 or 3, alternatively p=0;
Rₐ, R_{b} and R_{c} are independently selected from H, D, C₁₋₆ alkyl and C₁₋₆ haloalkyl.

26. The compound according to claim 23, or a pharmaceutically acceptable salt, an isotopic variant, a tautomer, a stereoisomer, a prodrug, a polymorph, a hydrate or a solvate thereof, wherein,
L₁-L₂ is: 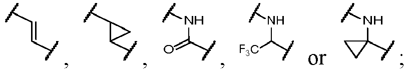
Z₁ is CH or N;
R₁ is CF₃, CN, OCF₃, OCH₂CH₃, 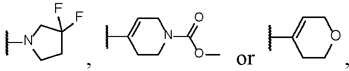 alternatively CF₃;
m = 0;
X₁ is C atom or N atom, which is optionally substituted with R_{2b};
X₂ is C atom or N atom, which is optionally substituted with R_{2c};
X₃ is C atom or N atom, which is optionally substituted with R_{2d};
R_{2b} is H, F, Cl, Br, Me, CH₂CH₃, C(O)NH₂ or 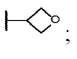
R_{2c} is H or Me;
or X₁, X₂ and their substituents are taken together to form: 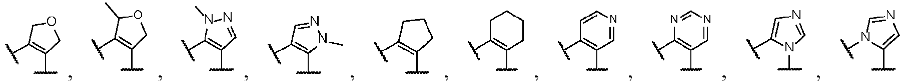
or 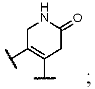
R_{2d} is H;
R₃ₛ₁ is H, Me or cyclopropyl;
R₃ₛ is H;
p=0.

27. The compound according to claim 10, or a pharmaceutically acceptable salt, an isotopic variant, a tautomer, a stereoisomer, a prodrug, a polymorph, a hydrate or a solvate thereof, which is a compound of formula (IV), (IV-1), (IV-1a), (IV-1b), (IV-2), (IV-2a) or (IV-2b), wherein,
R₁ is selected from H, D, halogen, CN, NO₂, -L-ORₐ, -L-SRₐ, -L-NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(O)₂ORₐ, S(O)NR_{b}R_{c}, S(O)₂NR₃R_{c}, P(O)(Rₐ)₂, C₁₋₆ alkyl, C₁₋₆ haloalkyl, C₂₋₆ alkenyl, C₂₋₆ alkynyl, -L-C₃₋₇ cycloalkyl, -L-3- to 7-membered heterocyclyl, -L-C₆₋₁₀ aryl and -L-5- to 10-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ, NR_{b}R_{c}, C(O)ORₐ and C(O)NR_{b}R_{c};
R_{2b} is selected from H, D, halogen, C(O)ORₐ, C(O)NR_{b}R_{c}, C₁₋₆ alkyl, C₁₋₆ haloalkyl, C₃₋₁₀ cycloalkyl and 3- to 10-membered heterocyclyl;
R_{2c} is selected from H, D, halogen, C₁₋₆ alkyl and C₁₋₆ haloalkyl;
or X₁, X₂ and their substituents are taken together to form C₅₋₁₀ cycloalkyl, 5- to 10-membered heterocyclyl, C₆₋₁₀ aryl or 5- to 10-membered heteroaryl (alternatively 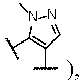 which is optionally substituted with 1, 2 or 3 R₂ₛ;
R_{2d} is selected from H, D, halogen, CN, NO₂, ORₐ, NR_{b}R_{c}, C₁₋₆ alkyl, C₁₋₆ haloalkyl, C₅₋₁₀ cycloalkyl, 5- to 10-membered heterocyclyl, C₆₋₁₀ aryl and 5- to 10-membered heteroaryl;
Z₁ is CH;
Z₂ is CR₁ or N;
Z₃ is CR₁ or N;
when Z₂ or Z₃ is CR₁, any R₁ is connected to R₄ to form -(CRₛₛRₛₛ)_{q1}-X-(CRₛₛRₛₛ)_{q2}- (alternatively -(CH₂)_{q1}-X-(CH₂)_{q2}-); wherein X is O, S, NH or CH₂, q1=0, 1 or 2, q2=1, 2 or 3, Rₛₛ is independently selected from H, D, C₁₋₆ alkyl and C₁₋₆ haloalkyl, or two Rₛₛ and the carbon atom to which they are attached are taken together to form C₃₋₁₀ cycloalkyl;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, CN, =O, ORₐ, NR_{b}R_{c}, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; or two R₁ₛ and the atom(s) to which they are attached are taken together to form C₃₋₁₀ cycloalkyl or 3- to 10-membered heterocyclyl; alternatively R₁ₛ and R₂ₛ are independently selected from H, D, halogen, CN, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
or, R₃ₛ₁, R₃ₛ₂ and the carbon atom to which they are attached are taken together form C₃₋₁₀ cycloalkyl;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the above groups is optionally deuterated, up to fully deuterated.

28. The compound according to claim 27, or a pharmaceutically acceptable salt, an isotopic variant, a tautomer, a stereoisomer, a prodrug, a polymorph, a hydrate or a solvate thereof, wherein,
R₁ is selected from H, D, halogen, CN, NO₂, ORₐ, SRₐ, NR_{b}R_{c}, SF₅, C(O)Rₐ, C(O)ORₐ, C(O)NR_{b}R_{c}, OC(O)Rₐ, NR_{b}C(O)R_{c}, S(O)Rₐ, S(O)₂Rₐ, S(O)ORₐ, S(L)₂ORₐ, S(O)NR_{b}R_{c}, S(O)₂NR₃R_{c}, P(O)(Rₐ)₂, C₁₋₆ alkyl, C₁₋₆ haloalkyl, C₂₋₆ alkenyl, C₂₋₆ alkynyl, C₃₋₇ cycloalkyl, 3- to 7-membered heterocyclyl, C₆₋₁₀ aryl and 5- to 10-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from H, D, CN, ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₆ cycloalkyl, 5- to 6-membered heterocyclyl, phenyl
or 5- to 6-membered heteroaryl (alternatively 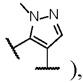 which is optionally substituted with 1, 2 or 3 R₂ₛ;
R_{2d} is selected from H, D, halogen, CN, NO₂, ORₐ, NR_{b}R_{c}, C₁₋₆ alkyl and C₁₋₆ haloalkyl;
Z₁ is CH;
Z₂ is CR₁, and the R₁ and R₄ are connected to form -(CRₛₛRₛₛ)_{q1}-X-(CRₛₛRₛₛ)_{q2}- (alternatively -(CH₂)_{q1}-X-(CH₂)_{q2}-); wherein X is O, S, NH or CH₂, q1=0 or 1, q2=1 or 2, Rₛₛ is independently selected from H, D, C₁₋₆ alkyl and C₁₋₆ haloalkyl, or two Rₛₛ and the carbon atom to which they are attached are taken together to form C₃₋₁₀ cycloalkyl;
Z₃ is CR₁ or N;
L is a chemical bond, C₁₋₆ alkylene, C₂₋₆ alkenylene or C₂₋₆ alkynylene, which is optionally substituted with 1, 2 or 3 R;
R₁ₛ and R₂ₛ are independently selected from H, D, halogen, =O, C(O)ORₐ, C(O)NR_{b}R_{c}, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl; or two R₁ₛ and the atom(s) to which they are attached are taken together to form C₃₋₆ cycloalkyl or 3-to 6-membered heterocyclyl;
R₃ₛ₁ is selected from H, D, halogen, C₁₋₆ alkyl, C₁₋₆ haloalkyl, C₂₋₆ alkenyl, C₂₋₆ alkynyl, C₃₋₁₀ cycloalkyl, 3- to 10-membered heterocyclyl, C₆₋₁₀ aryl and 5- to 10-membered heteroaryl;
R₃ₛ₂ is selected from H, D, -L-ORₐ, -L-NR_{b}R_{c}, C₁₋₆ alkyl, C₁₋₆ haloalkyl, C₃₋₁₀ cycloalkyl, 5- to 10-membered heterocyclyl, C₆₋₁₀ aryl and 5- to 10-membered heteroaryl, which is optionally substituted with 1, 2 or 3 R₃ₛ;
or, R₃ₛ₁, R₃ₛ₂ and the carbon atom to which they are attached are taken together to form C₃₋₁₀ cycloalkyl;
R and R₃ₛ are independently selected from H, D, halogen, C₁₋₆ alkyl and C₁₋₆ haloalkyl;
Rₐ, R_{b} and R_{c} are independently selected from H, D, C₁₋₆ alkyl, C₁₋₆ haloalkyl, C₃₋₁₀ cycloalkyl, 3- to 10-membered heterocyclyl, C₆₋₁₀ aryl and 5- to 10-membered heteroaryl; or R_{b}, R_{c} and the atom(s) to which they are attached are taken together to form 5- to 10-membered heterocyclyl;
wherein each of the above groups is optionally deuterated, up to fully deuterated.

29. The compound according to claim 27, or a pharmaceutically acceptable salt, an isotopic variant, a tautomer, a stereoisomer, a prodrug, a polymorph, a hydrate or a solvate thereof, wherein,
R₁ is selected from H, D, halogen, CN, NO₂, ORₐ, SRₐ, NR_{b}R_{c}, SF₅, C(O)Rₐ, S(O)Rₐ, S(O)₂Rₐ, C₁₋₆ alkyl, C₁₋₆ haloalkyl, C₂₋₆ alkenyl, C₂₋₆ alkynyl, C₄₋₆ cycloalkyl, 5- to 6-membered heterocyclyl, phenyl and 5- to 6-membered heteroaryl, which is optionally substituted with 1, 2 or 3 R₁ₛ;
m = 0, 1 or 2;
R₄ is selected from H and D;
X₁ is C atom, which is substituted with R_{2b};
X₂ is C atom or N atom, which is optionally substituted with R_{2c};
X₃ is C atom, which is substituted with R_{2d};
R₂ₐ is selected from ORₐ and NR_{b}R_{c};
R_{2b} is selected from H, D, halogen, C(O)ORₐ, C(O)NR_{b}R_{c}, C₁₋₆ alkyl and C₁₋₆ haloalkyl;
R_{2c} is selected from H, D, halogen, C₁₋₆ alkyl and C₁₋₆ haloalkyl;
or X₁, X₂ and their substituents are taken together to form C₅₋₆ cycloalkyl, 5- to 6-membered heterocyclyl, phenyl or 5- to 6-membered heteroaryl (alternatively 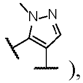 which is optionally substituted with 1, 2 or 3 R₂ₛ;
R_{2d} is selected from H, D, halogen, CN, NO₂, C₁₋₆ alkyl and C₁₋₆ haloalkyl;
Z₁ is CH;
Z₂ is CR₁, and the R₁ and R₄ are connected to form -(CRₛₛRₛₛ)_{q1}-X-(CRₛₛRₛₛ)_{q2}- (alternatively -(CH₂)_{q1}-X-(CH₂)_{q2}-); wherein X is O, S or NH, q1=0 or 1, q2=1 or 2, Rₛₛ is independently selected from H, D, C₁₋₆ alkyl and C₁₋₆ haloalkyl, or two Rₛₛ and the carbon atom to which they are attached are taken together to form C₃₋₆ cycloalkyl;
Z₃ is CR₁ or N;
L is a chemical bond or C₁₋₆ alkylene;
R₁ₛ and R₂ₛ are independently selected from H, D, halogen, C₁₋₆ alkyl, C₁₋₆ haloalkyl, C₂₋₆ alkenyl and C₂₋₆ alkynyl; or two R₁ₛ and the atom(s) to which they are attached are taken together to form C₃₋₆ cycloalkyl or 3- to 6-membered heterocyclyl;
R₃ₛ₁ is selected from H, D, halogen, C₁₋₆ alkyl, C₁₋₆ haloalkyl, C₂₋₆ alkenyl and C₂₋₆ alkynyl;
R₃ₛ₂ is selected from H, D, -L-ORₐ, -L-NR_{b}R_{c}, C₁₋₆ alkyl, C₁₋₆ haloalkyl, C₃₋₆ cycloalkyl, 5- to 6-membered heterocyclyl, phenyl and 5- to 6-membered heteroaryl, which is optionally substituted with 1, 2 or 3 R₃ₛ;
or, R₃ₛ₁, R₃ₛ₂ and the carbon atom to which they are attached are taken together to form C₃₋₆ cycloalkyl;
R₃ₛ is independently selected from H, D, halogen, C₁₋₆ alkyl and C₁₋₆ haloalkyl;
Rₐ, R_{b} and R_{c} are independently selected from H, D, C₁₋₆ alkyl and C₁₋₆ haloalkyl; or R_{b}, R_{c} and the atom to which they are attached are taken together to form 5- to 10-membered heterocyclyl;
wherein each of the above groups is optionally deuterated, up to fully deuterated.

30. The compound of formula (X) according to claim 1, or a pharmaceutically acceptable salt, an isotopic variant, a tautomer, a stereoisomer, a prodrug, a polymorph, a hydrate or a solvate thereof, wherein, the compound is selected from compound 1 to compound 245.

31. A pharmaceutical composition, comprising the compound according to any one of claims 1 to 30, or a pharmaceutically acceptable salt, an isotopic variant, a tautomer, a stereoisomer, a prodrug, a polymorph, a hydrate or a solvate thereof, and a pharmaceutically acceptable carrier, adjuvant or vehicle, and optionally other therapeutic agents.

32. The pharmaceutical composition according to claim 31, wherein the other therapeutic agents are selected from the following targeted drugs/cell activity modulators, including CDK4/6 inhibitors, MAT2A inhibitors, MAPK1/MAPK3 inhibitors, Type I PRMT inhibitors, EGFR inhibitors, SHP2 inhibitors, pan-KRAS inhibitors, KRASG12C inhibitors, RAF inhibitors, MEK inhibitors, ERK inhibitors, Bcl-2 inhibitors, SOS1 inhibitors, PARP inhibitors, MALT1 inhibitors, MALT2 inhibitors, BTK inhibitors, PI3K inhibitors, AKT inhibitors, FGFR inhibitors, DNA methyltransferase (DNMT) inhibitors, EZH1/2 inhibitors, EZH2 inhibitors, Menin-MLL inhibitors, IDH1 inhibitors, IDH2 inhibitors, IDH1/2 inhibitors, chemotherapy drugs, radiotherapy, STING agonists and immune checkpoint inhibitors/regulators.

33. Use of the compound according to any one of claims 1 to 30, or a pharmaceutically acceptable salt, an isotopic variant, a tautomer, a stereoisomer, a prodrug, a polymorph, a hydrate or a solvate thereof, in the manufacture of a medicament for the treatment or prevention of a PRMT5-mediated disease.

34. A method of treating or preventing a PRMT5-mediated disease in a subject, comprising administering the compound according to any one of claims 1 to 30, or a pharmaceutically acceptable salt, an isotopic variant, a tautomer, a stereoisomer, a prodrug, a polymorph, a hydrate or a solvate thereof, or the pharmaceutical composition according to claim 31 or 32 to the subject.

35. The compound according to any one of claims 1 to 30, or a pharmaceutically acceptable salt, an isotopic variant, a tautomer, a stereoisomer, a prodrug, a polymorph, a hydrate or a solvate thereof, or the pharmaceutical composition according to claim 31 or 32, for use in the treatment or prevention of a PRMT5-mediated disease.

36. The use according to claim 33 or the method according to claim 34 or the compound or pharmaceutical composition for use according to claim 35, wherein the disease is a cancer.

37. The use according to claim 33 or the method according to claim 34 or the compound or pharmaceutical composition for use according to claim 35, wherein the PRMT5-mediated disease is selected from the following cancers: heart: sarcoma (angiosarcoma, fibrosarcoma, rhabdomyosarcoma, liposarcoma), myxoma, rhabdomyoma, fibroma, lipoma, and teratoma; lung: bronchogenic carcinoma (squamous cell carcinoma, small cell undifferentiated carcinoma, large cell undifferentiated carcinoma, adenocarcinoma), alveolar (bronchiolar) carcinoma, bronchial adenoma, sarcoma, lymphoma, chondroma, hamartoma, mesothelioma; gastrointestinal tract: esophagus (squamous cell carcinoma, adenocarcinoma, leiomyosarcoma, lymphoma), stomach (cancer, lymphoma, leiomyosarcoma), pancreas (ductal adenocarcinoma, insulinoma, glucagonoma, gastrinoma, carcinoid tumor, hemangioma), small intestine (adenocarcinoma, lymphoma, carcinoid tumor, Kaposi's sarcoma, leiomyoma, hemangioma, lipoma, neurofibroma, fibroma), large intestine (adenocarcinoma, tubular adenoma, villous adenoma, hamartoma, leiomyoma); genitourinary tract: kidney (adenocarcinoma, Wilms tumor (nephroblastoma), lymphoma, leukemia), bladder and urethra (squamous cell carcinoma, transitional cell carcinoma, adenocarcinoma), prostate (adenocarcinoma, sarcoma), testicle (seminoma, teratoma, embryonal carcinoma, teratocarcinoma, choriocarcinoma, sarcoma, stromal cell carcinoma, fibroma, fibroadenoma, adenomatoid tumor, lipoma); liver: hepatoma (hepatocellular carcinoma), cholangiocarcinoma, hepatoblastoma, angiosarcoma, hepatocellular adenoma, hemangioma; biliary tract: gallbladder cancer, ampullary cancer, cholangiocarcinoma; bone: osteosarcoma, fibrosarcoma, malignant fibrous histiocytoma, chondrosarcoma, Ewing sarcoma, malignant lymphoma (reticulum cell sarcoma), multiple myeloma, malignant giant cell tumor, chordoma, osteocartilaginous exostoses, benign enchondroma, chondroblastoma, fibrochondroma, chondromyxoid fibroma, osteoid osteoma, and giant cell tumor; nervous system: skull (osteoma, hemangioma, granuloma, xanthoma, osteitis deformans), meninges (meningioma, meningeal sarcoma, glioma), brain (astrocytoma, medulloblastoma, glioma, ependymoma, germinoma (pineal tumor), glioblastoma multiforme, oligodendroglioma, schwannoma, retinoblastoma, congenital tumor), spinal neurofibroma, meningioma, glioma, sarcoma; gynaecology: uterus (endometrial cancer), cervix (cervical cancer, preneoplastic cervical dysplasia, etc.), ovary (ovarian cancer, serous cystadenocarcinoma, mucinous cystadenocarcinoma, unclassified cancer), granulosa cell tumor, Sertoli-Leydig cell tumor, dysgerminoma, malignant teratoma), vulva (squamous cell carcinoma, intraepithelial carcinoma, adenocarcinoma, fibrosarcoma, melanoma), vagina (clear cell carcinoma, squamous cell carcinoma, sarcoma botryoides (embryonal rhabdomyosarcoma)), fallopian tube (cancer); hematology: blood (myeloid leukemia (acute and chronic), acute lymphoblastic leukemia, chronic lymphocytic leukemia, diffuse large B-cell lymphoma, mantle cell lymphoma (MCL), follicular lymphoma, myeloproliferative disorder, multiple myeloma, myelodysplastic syndrome), Hodgkin disease, non-Hodgkin lymphoma (malignant lymphoma); skin: malignant melanoma, basal cell carcinoma, squamous cell carcinoma, Kaposi sarcoma, dysplastic nevus, lipoma, hemangioma, dermatofibroma, keloma, psoriasis; and adrenal gland: neuroblastoma.

38. The use according to claim 33 or the method according to claim 34 or the compound or pharmaceutical composition for use according to claim 35, wherein the PRMT5-mediated disease is selected from the following cancers: malignant peripheral nerve sheath tumor, mesothelioma, glioblastoma multiforme, pancreatic cancer, cholangiocarcinoma, prostate cancer, breast cancer, brain cancer, skin cancer, cervical cancer, bladder cancer, astrocytoma, colorectal cancer, endometrial cancer, esophageal cancer, gastric cancer, thymoma, head and neck cancer, hepatocellular carcinoma, laryngeal cancer, squamous cell carcinoma of the lung, lung adenocarcinoma, oral cancer, ovarian cancer, kidney cancer, and thyroid cancer and sarcoma.

39. The use according to claim 33 or the method according to claim 34 or the compound or pharmaceutical composition for use according to claim 35, wherein the PRMT5-mediated disease is selected from MTAP-related cancers, such as hepatocellular carcinoma, breast cancer, skin cancer, bladder cancer, liver cancer, pancreatic cancer and head and neck cancer.
